(19) Europäisches Patentamt
European Patent Office
Office européen des brevets

(11) EP 1 746 100 A1

(12) EUROPEAN PATENT APPLICATION
published in accordance with Art. 158(3) EPC

(43) Date of publication:
**24.01.2007 Bulletin 2007/04**

(21) Application number: **05750690.9**

(22) Date of filing: **29.04.2005**

(51) Int Cl.:
***C07D 487/04*** *(2006.01)*

(86) International application number:
**PCT/RU2005/000235**

(87) International publication number:
**WO 2005/105805 (10.11.2005 Gazette 2005/45)**

(84) Designated Contracting States:
**AT BE BG CH CY CZ DE DK EE ES FI FR GB GR HU IE IS IT LI LT LU MC NL PL PT RO SE SI SK TR**

(30) Priority: **29.04.2004 RU 2004113251**

(71) Applicant: **"Chemical diversity Research Institute", Ltd.**
**Moskovskaya obl., 141700 (RU)**

(72) Inventors:
- **IVASHCHENKO, Alexander Vasilievich**
  **Encinitas, CA 92024 (US)**
- **VVEDENSKY, Vladimir Yurievich**
  **Irkutsk, 664003 (RU)**
- **ILYN, Aleksei Petrovich**
  **Moscow, 125565 (RU)**
- **KYSEL, Volodymyr Mikhailovich**
  **Kiev, 021140 (UA)**
- **KHVAT, Alexander Viktorovich**
  **San Diego, CA 92131 (US)**
- **KUZOVKOVA, Yulia Aleksandrovna**
  **Moscow, 125566 (RU)**
- **KUTEPOV, Sergey Aleksandrovich**
  **Ryazan 117654 (RU)**
- **DMITRIEVA, Irina Gennadievna**
  **Moscow, 125340 (RU)**
- **ZOLOTAREV, Denis Anatolievich**
  **Moscow, 107065 (RU)**
- **Tkachenko, Sergey Yevgenievich**
  **Moskovskaya obl., 142432 (RU)**
- **OKUN, Ilya Matusovich**
  **San-Diego, CA 92121 (US)**
- **Kravchenko, Dmitri Vladimirovich**
  **Moskovskaya obl., 141100 (RU)**
- **Kobak, Vladimir Vasilievich**
  **Moskovskaya obl., 140014 (RU)**
- **TRIFILENKOV, Andrei Sergeevich**
  **Moscow 125473 (RU)**
- **Mishunina, Yulia Serafimova**
  **Moskovskskaya obl., 141700 (RU)**
- **LOSEVA, Marina Vasilievna**
  **Moscow, 125502 (RU)**
- **RIZHOVA, Elena Alexandrovna**
  **Moscow, 129226 (RU)**
- **PARCHINSKY, Vladislav Zenonovich**
  **Moscow, 125649 (RU)**
- **TSIRULNIKOV, Sergey Alexandrovich**
  **Moscow, 123585 (RU)**
- **Kyselev, Alexandr Sergeevich**
  **San Diego, CA 92130 (US)**

(74) Representative: **Jeck, Anton**
**Patentanwalt,**
**Klingengasse 2/1**
**71665 Vaihingen/Enz (DE)**

# (54) ANNELATED CARBAMOYLASE-HETEROCYCLES, FOCUSED LIBRARY, PHARMACEUTICAL COMPOSITIONS AND METHODS FOR THE PRODUCTION THEREOF

(57) This invention relates to novel annellated carbamyl-aza-heterocyclic compounds that are potential physiologically active compounds (agonists, antagonists, and receptor modulators, enzyme inhibitors, oncolytics, antibacterial and antiparasitic agents, and so on), to a focused library comprising annellated carbamyl-aza-heterocyclic compounds, a pharmaceutical composition comprising annellated carbamyl-aza-heterocyclic compounds as the active ingredient, and to methods of producing and using the same.

The invention relates to annellated carbamyl-aza-heterocyclic compounds of general formula 1:

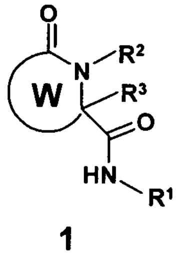

1

EP 1 746 100 A1

wherein:
W is 6-oxopiperazine, [1,4]diazepan, [1,4]thiazepan or [1,4]oxazepan compound annellated to at least one optionally substituted and optionally condensed heterocyclic compound **Q**;
**$R^1$, $R^2$** and **$R^3$** are, independently of one another, a hydrogen atom, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl; and
Q is a pyrrole, pyrazole, imidazole, thiazole, pyrrolidine, indole, benzofuran, 4,5,6,7-tetrahydrobenzothiophene, thieno[3,2-b]pyrrole, furo[3,2-b]pyrrole, thieno[2,3-b]pyrrole, benzimidazole, pyridine, quinoline, or 1,2,3,4-tetrahydroisoquinoline cyclic compound.

EP 1 746 100 A1

## Description

### Field of the Invention

This invention relates to synthesis of new chemical compounds, a search for new physiologically active compounds, leader compounds, and drug candidates produced by screening combinatorial or focused libraries of compounds, and to a pharmaceutical composition, and to methods of producing and using the same.

More specifically, this invention relates to novel annellated carbamyl-aza-heterocyclic compounds that are interesting as potential physiologically active compounds (agonists, antagonists, and receptor modulators, enzyme inhibitors, oncolytics, antibacterial and antiparasitic agents, and so on), to a focused library comprising annellated carbamyl-aza-heterocyclic compounds, to a pharmaceutical composition comprising annellated carbamyl-aza-heterocyclic compounds as an active ingredient, and to method of preparing and using the same.

### Background of the Invention

There is a large number of natural and synthetic physiologically active compounds having molecules that are annellated aza-heterocyclic compounds. Among natural compounds, the more significant are anti-neoplastic and antibacterial alkaloids such as longamide, longamide B, and phakellstatins derived from sea sponges *Agelas genus, Homaxinella sp.* and *Phakellia mauritiana* [Cafieri, F., et al., J. Nat. Prod. 1998, 61: 122. Umeyama, A., et al., J. Nat. Prod. 1998, 61: 1433. Poullennec, K., Romo, D. J. Am. Chem. Soc. 2003, 125: 6344]

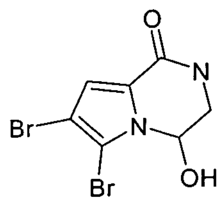

(+/-)-Longamide

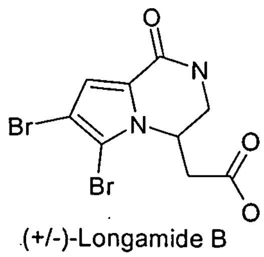

(+/-)-Longamide B

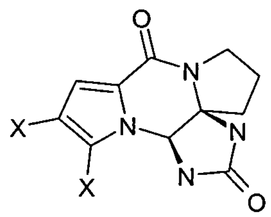

X = H, (-)-Phakellstatin
X = Br, (-)-Dibromophakellstatin

Synthetic annellated carbamyl-aza-heterocyclic compounds have an extraordinarily broad spectrum of physiological activity. For example, 4-methyl-2-(3-trifluoromethyl phenyl)-hexahydropyrido[1,2-a]pyrazin-3-one exhibits the properties of an anxiolytic and antidepressant [Silvestrini, B., Baiocchi, L. ACRAF SpA. WO 8705022, 1987], 3-[1-oxo-2-[2-(piperidin-1-yl)ethyl]-1,2,3,4-tetrahydro-pyrrolo[1,2-*a*]pyrazin-7-yl-carboxamido]propionic acid is an antagonist of the fibrinogenic gpIIb/IIIa receptor and, as a result, effectively inhibits aggregation of thrombocytes [Askew, B.C., et al., J. Med. Chem. 1997, 40(12):1779*],* while [6*S*-[6α[R*(trans),8aα]]-*N*-[1-(4-aminocyclohexyl)-2-(2-benzothiazolyl)-2-oxoethyl]-1,4-dioxo-2-(3-phenylpropyl)perhydro-pyrrolo[1,2-*a*]pyrazine-6-carboxamide is an anticoagulant, which inhibits serine thrombin and factor Xa proteases [Berryman, K.A.; Doherty, A.M.; Edmunds, J.J.; Siddiqui, M.A. Pfizer Inc. WO 9748706, 1997].

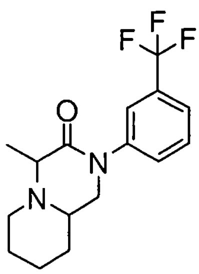

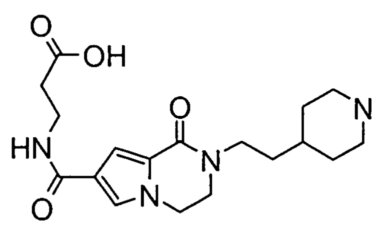

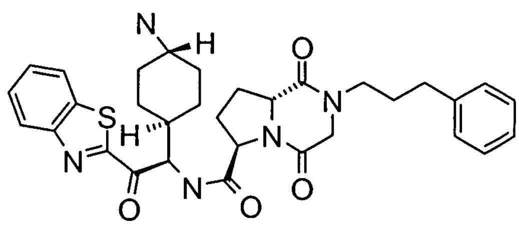

Antibacterial properties are exhibited by 3-isopropyl-hexahydropyrrolo[1,2-*a*]pyrazine-1,4-dione [Kwon O., et. al. J. Antibiot. 2001, 54(2): 179], and *N*-[4-(3-oxo-3,5-dihydro-1,4,8b-triaza-acenaphthylen-4-yl)butyl]trifluoromethanesulfonamide is an inhibitor of receptor PDGF kinase and, for this reason, a potential drug to treat renal insufficiency, hyperlipidemia, and hypertension [Ikemoto, T., et al. Tetrahedron 2000, 56(40): 7915]. High antiprotozoic activity is exhibited by 3,4,7,8-tetrahydro-2*H*-pyrazino[1,2-*a*]indole-1,6,9-trione [Tapia, R., et al. Eur. J. Org. Chem. 2002, 23:

4005], and 2-{4-[4-(3-trifluoromethylphenyl)piperazin-1-yl]butyl}hexahydropyrido[1,2-*a*]pyrazine-1,4-dione is a highly effective ligand of serotonin 5-HT1a receptor [Lopez-Rodriguez, M., et al. J. Med. Chem. 2001, 44(2): 186].

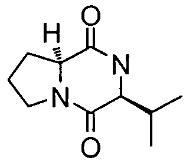

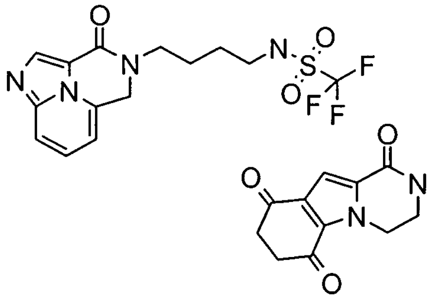

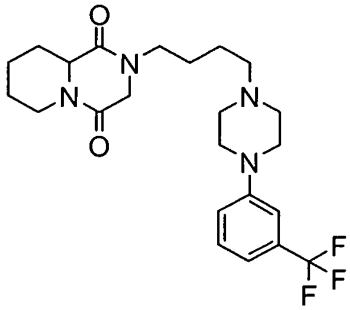

Considering the high potential and broad spectrum of physiological activity of annellated aza-heterocyclic compounds, there is increasing urgency to develop new compounds of this type, focused libraries, and pharmaceutical compositions including these compounds, and also methods of preparing and using the same.

As a result of studies conducted to find new physiologically active compounds and leader compounds, these inventors have produced previously unknown annellated carbamyl-aza-heterocyclic compounds exhibiting physiological activity, a focused library, and a pharmaceutical composition containing these compounds, and developed methods of preparing and using the same.

**Disclosure of the Invention**

The terms used in the description of the invention are defined below.

"Combinatorial library" is a collection of compounds produced by parallel synthesis and intended for searching a hit or leader compound, and also for optimizing the physiological activity of the hit or leader, each compound in the library corresponding to the common scaffold, the library being a collection of related homologues or analogues.

"Focused library" is a combinatorial library or a combination of several combinatorial libraries, or a combination of libraries and compounds organized in a special way to enhance the probability of finding hits and leaders or to improve the efficiency of their optimization. The design of focused libraries is, as a rule, linked to the direction in which a search is conducted for effectors (inhibitors, activators, agonists, antagonists, and so on) of specific bio-targets (enzymes, receptors, ion channels, and so on).

"Hit compound" or "hit" is a compound that exhibited desired physiological activity during primary screening.

"Leader compound" or "leader" is a compound of an outstanding (maximum) physiological activity associated with a specific bio-target related to a particular (or several) pathology or disease.

"Parallel synthesis" is a method of conducting combinatorial chemical synthesis of the library.

"Scaffold" is a general structural formula or molecular framework, or an invariant region of compounds typical of all compounds comprising the combinatorial library.

"Nucleophilic" means electron-excessive reagent.

"Electrophilic" means an electron-deficient reagent.

"Substituent" means a chemical radical that is attached to the scaffold or synthesis intermediate during the synthesis thereof, for example, "nucleophilic substituent," "electrophilic substituent," NH-protective substituent," or "inert substituent."

"Optionally substituted radical" means a radical without substituents or a radical containing one or several substituents, including "nucleophilic substituent," "electrophilic substituent," NH-protective substituent," and/or "inert substituent."

"Nucleophilic substituent" is a chemical radical that is attached to the scaffold as a result of reaction with a nucleophilic reagent, for example, one selected from a group of primary or secondary amines, alcohols, phenols, mercaptans, and thiophenols.

"Electrophilic substituent" means a chemical radical that is attached to the scaffold as a result of reaction with an electrophilic reagent, for example, one selected from a group of organic halides (optionally substituted $C_1$-$C_7$ alkyl halides, optionally substituted aryl $C_1$-$C_7$ alkyl halides, optionally substituted heterocyclyl-$C_1$-$C_7$ alkyl halides, optionally substituted aryl halides, optionally substituted heterocyclyl halides), organic acids or their derivatives (anhydrides, imidazolides, halo-anhydrides), esters of organic sulfo acids or organic sulfochlorides, organic haloformates, organic isocyanates, and organic isothio-cyanate aminomethyls.

"NH-protective substituent" means a chemical radical that is attached to the scaffold or synthesis intermediate

for temporary protection of the amino group in multifunctional compounds, including, but not limited to, an amide substituent, such as formyl, optionally substituted acetyl (for example, trichloroacetyl, trifluoroacetyl 3-phenylpropionyl, etc.), optionally substituted benzoyl, etc.; carbamate substituent, such as optionally substituted $C_1$-$C_7$ alkyloxycarbonyl, for example, methyloxycarbonyl, ethyloxycarbonyl, tertbutyloxycarbonyl, 9-fluorenyl-methyloxycarbonyl (Fmoc), etc.; optionally substituted $C_1$-$C_7$ alkyl substituent, for example, tert-butyl, benzyl, 2,4-dimethyloxybenzyl, 9-phenylfluorenyl, etc.; sulfonyl substituent, for example, benzene sulfonyl, n-toluene sulfonyl, etc. NH-protective substituents are described in more detail in "Protective Groups in Organic Synthesis," Third Edition Greene, T.W. and Wuts, P.G.M. 1999, pp. 494-653. Published by John Wiley & Sons, Inc., New York, Chichester, Weinheim, Brisbane, Toronto, Singapore.

"Inert substituent" (or "non-interfering substituent") means a low-reactive or non-reactive radical, including, but not limited to, $C_1$-$C_7$ alkyl, $C_2$-$C_7$ alkenyl, $C_2$-$C_7$ alkynyl, $C_1$-$C_7$ alkoxy, $C_7$-$C_{12}$ aralkyl, aralkyl substituted with inert substituents, $C_7$-$C_{12}$ heterocyclyl alkyl, heterocyclyl alkyl substituted with inert substituents, $C_7$-$C_{12}$ alkaryl, $C_3$-$C_{10}$ cycloalkyl, $C_3$-$C_{10}$ cycloalkenyl, phenyl, substituted phenyl, toluene, xylenyl, biphenyl, $C_2$-$C_{12}$ alkoxyalkyl, $C_2$-$C_{10}$ alkyl sulfinyl, $C_2$-$C_{10}$ alkyl sulfonyl, $(CH_2)_m$-O-($C_1$-$C_7$ alkyl), -$(CH_2)_m$-N($C_1$-$C_7$ alkyl)$_n$, aryl, aryl substituted with inert substituents, alkoxy substituted with inert substituents, fluoroalkyl, aryloxyalkyl, heterocyclyl, heterocyclyl substituted with inert substituents, and nitroalkyl; wherein m and n vary from 1 to 7. Preferred "inert substituents" are $C_1$-$C_7$ alkyl, $C_2$-$C_7$ alkenyl, $C_2$-$C_7$ alkynyl, $C_1$-$C_7$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, $C_1$-$C_7$ alkyl substituted with inert substituents, phenyl, phenyl substituted with inert substituents, $(CH_2)_m$-O-($C_1$-$C_7$ alkyl), -$(CH_2)_m$-N ($C_1$-$C_7$ alkyl)$_n$, aryl, aryl substituted with inert substituents, heterocyclyl, and heterocyclyl substituted with inert substituents.

"Aryl" means one or more aromatic cyclic compounds, each including 5 or 6 carbon atoms. "Aryl" may be a condensed polycyclic compound, for example, naphthalene, or uncondensed, for example, biphenyl. "Substituted aryl" has one or more substituents.

"Halogen" means an atom of fluorine, chlorine, bromine or iodine.

"Heterocylie compound" means one or several saturated, unsaturated, or aromatic cyclic compounds having 5, 6 or 7 atoms, at least one of which is a heteroatom. Preferred heteroatoms are sulfur, oxygen, and nitrogen. A "heterocyclic compound" may be a condensed polycyclic compound, for example, benzimidazole, benzoxazole, benzothiazole, and quinoline, or uncondensed, for example, bipyridyl.

"Azoheterocyclic compound" means a heterocyclic compound containing at least one nitrogen atom, for example, piperidine, morpholine, pyrrole, benzimidazole, benzoxazole, benzothiazole, and quinoline.

"Substituted heterocyclic compound" means a heterocyclic compound having one or several substituents.

"Substituted azoheterocyclic compound" means an azoheterocyclic compounds having one or several substituents.

An object of this invention is to produce novel annellated carbamyl-aza-heterocyclic compounds.

This object is achieved by using annellated carbamyl-aza-heterocyclic compounds of general formula **1:**

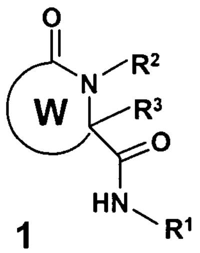

**1**

wherein: **W** is a 6-oxopiperazine, [1,4]diazepan, [1,4]thiazepan or [1,4]oxazepan cyclic ring annellated with at least one optionally substituted and one optionally condensed heterocyclic compound **Q**;

**Q** is a pyrrole, pyrazole, imidazole, thiazole, pyrrolidine, indole, benzofuran, 4,5,6,7-tetrahydrobenzothiophene, thieno [3,2-b]pyrrole, furo[3,2-b]pyrrole, thieno[2,3-b]pyrrole, benzimidazole, pyridine, quinoline or 1,2,3,4-tetrahydro-isoquinoline cyclic compound; and

$\mathbf{R^1}$, $\mathbf{R^2}$ and $\mathbf{R^3}$ are, independently of one another, a hydrogen atom, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl.

According to the invention, the preferred annellated carbamyl-aza-heterocyclic compounds are substituted 1-oxo-1,2,3,4-tetrahydropyrrolo[1,2-a]pyrazine-3-carboxamides of general formula **1.1,** 3-oxo-2,3,4,6-tetrahydro-1*H*-pyrrolo[1,2-a]pyrazine-1-carboxamides of general formula **1.2,** 6-oxo-5,6-dihydro-4*H*-benzo[f]pyrrolo[1,2-a][1,4]diazepine-4-carboxamides of general formula **1.3,** and 5-oxo-1,3,4,5,6,7,8,9-octahydro-2*H*-10-thia-6,9-diaza-benzo[a]-cyclopenta [e]azulene-7-carboxamides of general formula **1.4:**

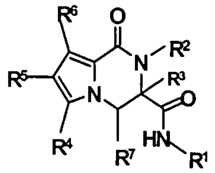

1.1

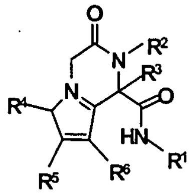

1.2

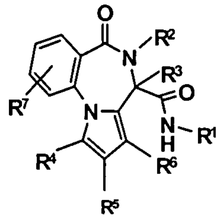

1.3

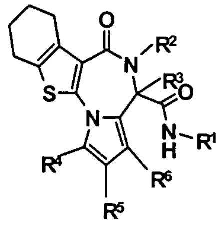

1.4

wherein: $R^1$, $R^2$ and $R^3$ are as above;
$R^4$, $R^5$ and $R^6$ are, independently of one another, a hydrogen atom, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl; and
$R^7$ is a hydrogen atom or an optionally substituted $C_1$-$C_6$ alkyl.

According to the invention, the preferred annellated carbamyl-aza-heterocyclic compounds also are substituted 4-oxo-4,5,6,7-tetrahydropyrazolo[1,5-a]pyrazine-6-carboxamides of general formula **1.5,** 4-oxo-1,4,5,6-tetrahydro-1,2,5,9a-tetra-azacyclopenta[e]azulene-6-carboxamides of general formula **1.6,** and 6-oxo-4,5,6,7-tetrahydro-1*H*-8-thia-1,2,5-triaza-azulene-4-carboxamides of general formula **1.7:**

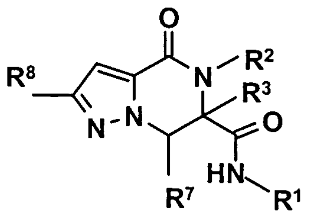

1.5

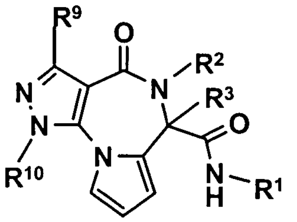

1.6

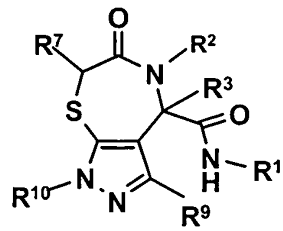

1.7

wherein: $R^1$, $R^2$, $R^3$, and $R^7$ are as above;
$R^8$ and $R^9$ are, independently of one another, an inert hydrogen atom, carboxyalkyl, carboxy, or an optionally substituted carbamyl; and
$R^8$, $R^9$ and $R^{10}$ are, independently from one another, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl.

According to the invention, the preferred annellated carbamyl-aza-heterocyclic compounds also are substituted 8-oxo-5,6,7,8-tetrahydro-imidazo[1,5-a]pyrazine-6-carboxamides of general formula **1.8:**

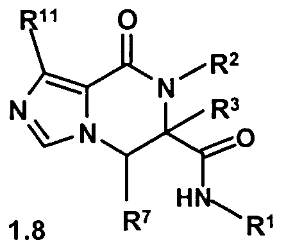

1.8

wherein: $R^1$, $R^2$, $R^3$ and $R^7$ are as above; and
$R^{11}$ is a hydrogen atom, carboxyalkyl, carboxy, or an optionally substituted carbamyl.

According to the invention, the preferred annellated carbamyl-aza-heterocyclic compounds also are substituted 8-oxo-5,6,7,8-tetrahydro-4-oxa-1-thia-3,7-diaza-azulene-6-carboxamides of general formula 1.9:

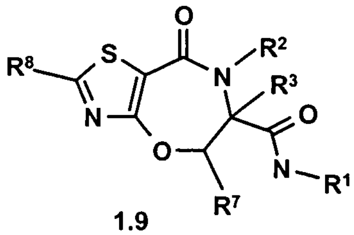

1.9

wherein: $R^1$, $R^2$, $R^3$, $R^7$ and $R^8$ are as above.

According to the invention, the preferred annellated carbamyl-aza-heterocyclic compounds also are substituted 1-oxo-1,2,3,4-tetrahydropyrazino[1,2-a]indole-3-carboxamides of general formula **1.10,** 7-oxo-5,7,8,10-tetrahydro-6*H*-9-thia-6,10-diaza-benzo[a]azulene-5-carboxamides of general formula **1.11,** 7-oxa-7,8,9,10-tetrahydro-6*H*-5-thia-8,10-diazabenzo[a]azulene-9- carboxamides of general formula **1.12,** and 9-oxo-7,8,9,10- tetrahydro-6*H*-5-thia-8,10-diaza-benzo[a]azulene-7-carboxamides of general formula **1.13:**

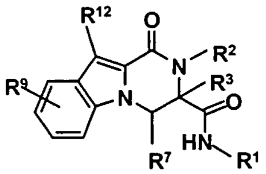 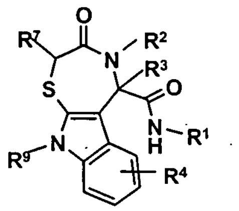 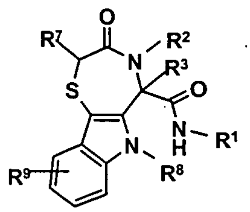 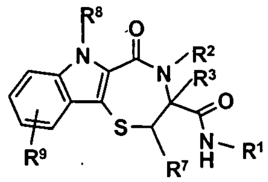

**1.10** **1.11** **1.12** **1.13**

wherein: $R^1$, $R^2$, $R^3$, $R^7$, $R^8$ and $R^9$ are as above; and

$R^{12}$ is a hydrogen atom, an inert substituent, a substituted amino group, or pyrrol-1-yl.

According to the invention, the preferred annellated carbamyl-aza-heterocyclic compounds also are substituted 9-oxo-6,7,8,9-tetrahydro-10-oxa-5-thia-8-aza-benzo[a]azulene-7-carboxamides of general formula **1.14:**

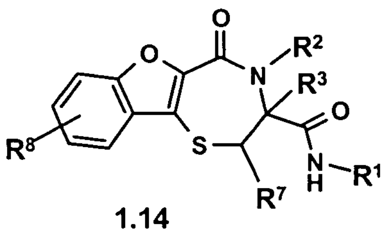

**1.14**

wherein: $R^1$, $R^2$, $R^3$, $R^7$ and $R^8$ are as above.

According to the invention, the preferred annellated carbamyl-aza-heterocyclic compounds also are substituted 1-oxo-1,2,3,4-tetrahydro-benzo[4,5]imidazo[1,2-a]pyrazine-3-carboxamides of general formula **1.15**:

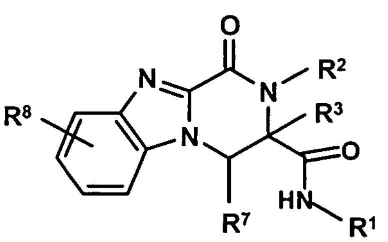

**1.15**

wherein: $R^1$, $R^2$, $R^3$, $R^7$ and $R^8$ are as above.

According to the invention, the preferred annellated carbamyl-aza-heterocyclic compounds also are substituted 7-oxo-4,5,6,7-tetrahydro-1-thia-3b,6-diazacyclopenta[a]indene-5-carboxamides of general formula **1.16** and 7-oxo-4,5,6,7-tetrahydro-1-oxa-3b,6-diaza-cyclopenta[a]indene-5-carboxamides of general formula **1.17:**

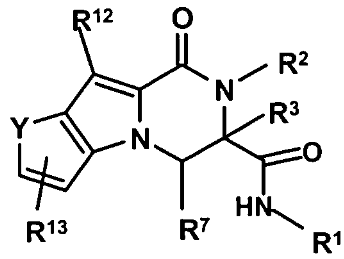

**1.16 (Y=S), 1.17 (Y=O)**

wherein: **$R^1$, $R^2$, $R^3$, $R^7$** and **$R^{12}$** are as above; and

**$R^{13}$** is a hydrogen atom or an optionally substituted $C_1$-$C_6$ alkyl.

According to the invention, the preferred annellated carbamyl-aza-heterocyclic compounds also are substituted 7-oxo-4,5,6,7-tetrahydro-3-thia-3b,6-diazacyclopenta[a]indene-5-carboxamides of general formula 1.18:

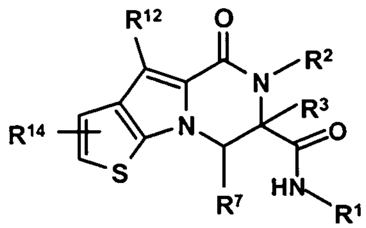

**1.18**

wherein: **$R^1$, $R^2$, $R^3$, $R^7$** and **$R^{12}$** are as above; and

**$R^{14}$** is a hydrogen atom or an optionally substituted $C_1$-$C_6$ alkyl.

According to the invention, the preferred annellated carbamyl-aza-heterocyclic compounds also are substituted 7-oxo-5,6,7,8-tetrahydro-9-thia-1,6-diaza-benzocycloheptane-5-carboxamides of general formula **1.19** and 8-oxo-7,8,9,10-tetrahydro-6-thia-5,9-diazacyclopenta[b]naphthalene-10-carboxamides of general formula **1.20:**

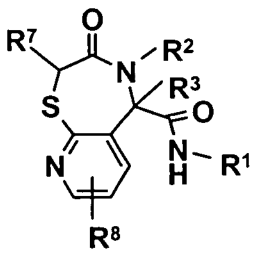

**1.19**

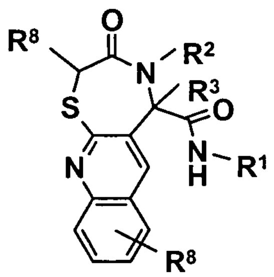

**1.20**

wherein: **$R^1$, $R^2$, $R^3$, $R^7$** and **$R^8$** are as above.

According to the invention, the preferred annellated carbamyl-aza-heterocyclic compounds also are substituted 4-oxo-2,3,3a,4,5,6-hexahydro-1*H*-benzo[f]pyrrolo[1,2-a][1,4]diazepine-6-carboxamides of general formula **1.21,** (3a*S*)-4-oxo-2,3,3a,4,5,6-hexahydro-1*H*-benzo[f]pyrrolo[1,2-a][1,4]diazepine-6-carboxamides of general formula **1.22,** (3a*S*,6*S*)-4-oxo-2,3,3a,4,5,6-hexahydro-1*H*-benzo[f]pyrrolo[1,2-a][1,4]diazepine-6-carboxamides of general formula **1.22a,** and (3a*S*,6*R*)-4-oxo-2,3,3a,4,5,6-hexahydro-1*H*-benzo[f]pyrrolo[1,2-a][1,4]diazepine-6-carboxamides of general formula **1.22b:**

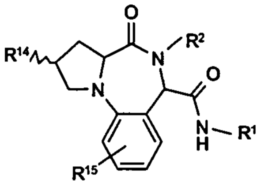

1.21

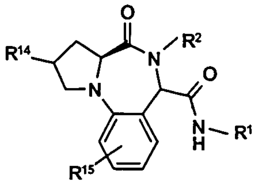

1.22

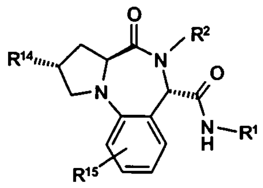

1.22a

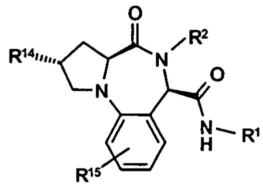

1.22b

wherein: $\mathbf{R^1}$ and $\mathbf{R^2}$ are as above;
$\mathbf{R^{14}}$ is a hydrogen atom or an optionally substituted hydroxyl group; and
$\mathbf{R^{15}}$ is a hydrogen atom, $NO_2$, $CF_3$, CN, carboxyalkyl, carboxy, an optionally substituted carbamyl, or an optionally substituted amino group.

According to the invention, the preferred annellated carbamyl-aza-heterocyclic compounds also are substituted 1-oxo-1,3,4,6,11,11a-hexahydro-2*H*-pyrazino[1,2-b]isoquinoline-3- carboxamides of general formula **1.23**

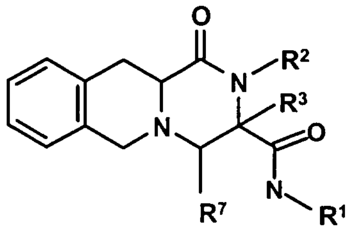

**1.23**

wherein: $\mathbf{R^1}$, $\mathbf{R^2}$, $\mathbf{R^3}$ and $\mathbf{R^7}$ are as above.

It is an object of this invention to develop a method for producing novel annellated carbamyl-aza-heterocyclic compounds of general formula **1.**

This object is achieved by a method to produce novel annellated carbamyl-aza-heterocyclic compounds of general formula **1** by three-component condensation of a suitable isonitrile of general formula **2,** a suitable primary amine of general formula 3, and a suitable heterocyclic bifunctional reagent of general formula **4:**

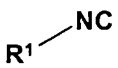

**2**

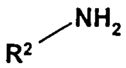

**3**

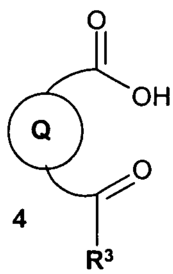

**4**

wherein: **Q,** $\mathbf{R^1}$, $\mathbf{R^2}$ and $\mathbf{R^3}$ are as above.

According to the invention, preferred bifunctional reagents **4** are heterocyclic reagents containing a carboxyl and carbonyl groups of general formulas **4.1-4.23**

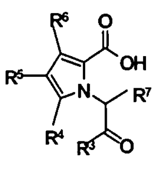

4.1

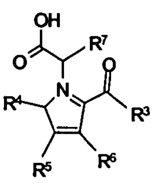

4.2

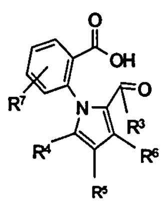

4.3

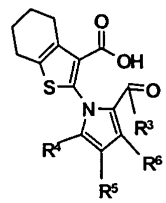

4.4

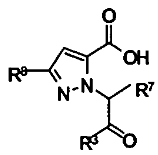

4.5

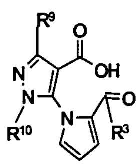

4.6

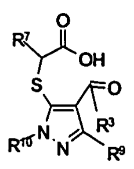
4.7

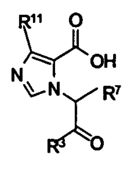
4.8

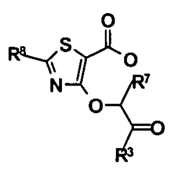
4.9

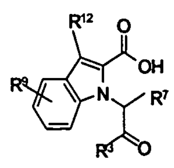
4.10

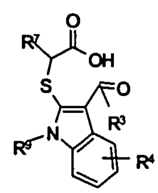
4.11

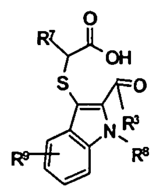
4.12

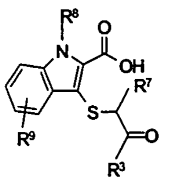
4.13

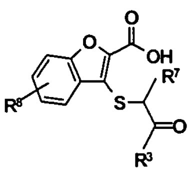
4.14

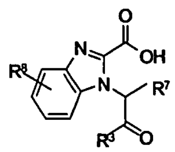
4.15

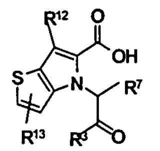
4.16

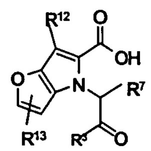
4.17

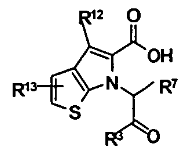
4.18

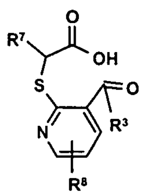
4.19

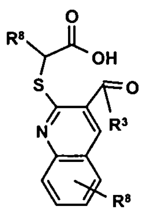
4.20

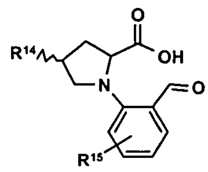
4.21

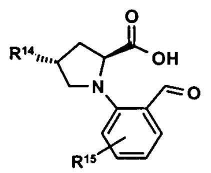
4.22

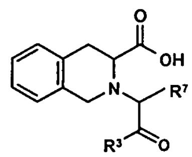
4.23

wherein: $\mathbf{R^3}$ to $\mathbf{R^{15}}$ are as above.

Another object of this invention is to develop a novel focused library for determining leader compounds.

This object is achieved by developing a focused library designed to determine leader compounds and comprising at least one annellated carbamyl-aza-heterocyclic compound of general formula **1** as claimed in claim 1.

A further object of this invention is to produce a novel pharmaceutical composition exhibiting anticancer activity.

This object is achieved by producing a pharmaceutical composition exhibiting anticancer activity, which comprises at least one annellated carbamyl-aza-heterocyclic compound of general formula **1** or a pharmaceutically acceptable salt thereof.

**Best Embodiment of the Invention**

The invention is described below with reference to examples, which illustrate, but do not limit, the scope of this invention.

**Example 1.** A general method of producing annellated carbamyl-aza-heterocyclic compounds of general formula **1.**

A mixture of 3 mmol of a suitable isonitrile of general formula 2, 3 mmol of a suitable primary amine of general formula **3,** and 3.1 mmol of a suitable bifunctional reagent of general formula **4** was stirred in 5 ml of dry methanol or another suitable solvent at room temperature for 48 to 56 hours. The resulting residue was filtered off and, if necessary, was recrystallized from a suitable solvent. This yielded compound 1 in the form of a mixture of enantiomers at a yield of 40% to 95% (Tables 1.01 to 1.23). In need, compound 1 was separated chromatographically into optical isomers (Tables 1.21-22).

**Table 1.01(1).** 1-oxo-6-(2-furyl)-1,2,3,4-tetrahydropyrrolo[1,2-a]pyrazine-3-carboxamides of general formula 1.1. Substituted 1-oxo-6-(2-furyl)-1,2,3,4-tetrahydropyrrolo[1,2-a]pyrazine-3-carboxamides of general formula **1.1.**
**Table 1.01(2).** Substituted 1-oxo-6-(2-phenyl)-1,2,3,4-tetrahydropyrrolo[1,2-a]pyrazine-3-carboxamides of general formula **1.1.**
**Table 1.02.** Substituted 3-oxo-1,2,3,4-tetrahydropyrazino[1,2-a]indole-1-carboxamides of general formula 1.1.
**Table 1.03.** Substituted 6-oxo-5,6-dihydro-4*H*-benzo[f]pyrrolo[1,2-a][1,4]diazepine-4-carboxamides of general formula **1.3.**

**Table 1.04.** Substituted 5-oxo-1,3,4,5,6,7,8,9-octahydro-2*H*-10-thia-6,9-diaza-benzo[a]-cyclopenta[e]azulene-7-carboxamides of general formula **1.4.**

**Table 1.05(1).** Substituted 5-aryl-2-*tert*-butyl-4-oxo-4,5,6,7-tetrahydropyrazolo[1,5-a]pyrazine-6-carboxamides of general formula **1.5.**

**Table 1.05(2).** Substituted 5-benzyl-2-*tert*-butyl-4-oxo-4,5,6,7-tetrahydropyrazolo[1,5-a]pyrazine-6-carboxamides of general formula **1.5.**

**Table 1.05(3).** Substituted 2-aryl-4-oxo-4,5,6,7-tetrahydropyrazolo[1,5-a]pyrazine-6-carboxamides of general formula **1.5.**

**Table 1.05(4).** Substituted 4-oxo-2-(2-furyl)-4,5,6,7-tetrahydropyrazolo[1,5-a]pyrazine-6-carboxamides of general formula **1.5.**

**Table 1.05(5).** Substituted 2-carboxyalkyl-4-oxo-4,5,6,7-tetrahydropyrazolo[1,5-a]pyrazine-6-carboxamides of general formula **1.5.**

**Table 1.05(6).** Substituted 2-carbamino-4-oxo-4,5,6,7-tetrahydropyrazolo[1,5-a]pyrazine-6-carboxamides of general formula **1.5.**

**Table 1.06.** Substituted 4-oxo-1,4,5,6-tetrahydro-1,2,5,9a-tetra-aza-cyclopenta[e]azulene-6-carboxamides of general formula **1.6.**

**Table 1.07.** Substituted 6-oxo-4,5,6,7-tetrahydro-1*H*-8-thia-1,2,5-triaza-azulene-4-carboxamides of general formula **1.7.**

**Table 1.08.** Substituted 8-oxo-5,6,7,8-tetrahydroimidazo[1,5-a]pyrazine-6-carboxamides of general formula 1.8.

**Table 1.09.** Substituted 8-oxo-5,6,7,8-tetrahydro-4-oxa-1-thia-3,7-diaza-azulene-6-carboxamides of general formula **1.9.**

**Table 1.10(1).** Substituted 7-methoxy- and 6,9-dimethoxy-1-oxo-1,2,3,4-tetrahydropyrazino[1,2 -a]indole-3-carboxamides of general formula **1.10.**

**Table 1.10(2).** Substituted 10-acetylamino-1-oxo-1,2,3,4-tetrahydropyrazino[1,2-a]indole-3-carboxamides of general formula **1.10.**

**Table 1.10(3).** Substituted 1-oxo-10-pyrrol-1-yl)-1,2,3,4-tetrahydropyrazino[1,2-a]indole-3-carboxamides of general formula **1.10.**

**Table 1.11.** Substituted 7-oxo-5,7,8,10-tetrahydro-6*H*-9-thia-6,10-diaza-benzo[a]azulene-5-carboxamides of general formula **1.11.**

**Table 1.12.** Substituted 7-oxo-7,8,9,10-tetrahydro-6*H*-5-thia-8,10-diaza-benzo[a]azulene-9-carboxamides of general formula **1.12.**

**Table 1.13.** Substituted 9-oxo-7,8,9,10-tetrahydro-6*H*-5-thia-8,10-diaza-benzo[a]azulene-7-carboxamides of general formula **1.13.**

**Table 1.14.** Substituted 9-oxo-6,7,8,9-tetrahydro-10-oxa-5-thia-8-aza-benzo[a]azulene-7-carboxamides of general formula **1.14.**

**Table 1.15.** Substituted 1-oxo-1,2,3,4-tetrahydro-benzo[4,5]imidazo[1,2-a]pyrazine-3-carboxamides of general formula **1.15.**

**Table 1.16(1).** Substituted 6-alkyl- and 6-cycloalkyl-7-oxo-4,5,6,7-tetrahydo-1-thia-3b,6-diazacyclopenta[a]indene-5-carboxamides of general formula **1.16.**

**Table 1.16(2).** Substituted 6-aryl-7-oxo-4,5,6,7-tetrahydro-1-thia-3b,6-diazacyclopenta[a]indene-5-carboxamides of general formula **1.16.**

**Table 1.16(3).** Substituted 6-benzyl-7-oxo-4,5,6,7-tetrahydro-1-thia-3b,6-diazacyclopenta[a]indene-5-carboxamides of general formula **1.16.**

**Table 1.16(4).** Substituted 6-(2-hydroxyethyl)- and 6-(2-aminoethyl)-7-oxo-4,5,6,7-tetrahydro-1-thia-3b,6-diaza-cyclopenta[a]indene-5-carboxamides of general formula **1.16.**

**Table 1.16(5).** Substituted 6-(3-hydroxypropyl)- and 6-(3-aminopropyl)-7-oxo-4,5,6,7-tetrahydro-1-thia-3b,6-diaza-cyclopenta[a]indene-5-carboxamides of general formula **1.16.**

**Table 1.16(6).** Substituted 7-oxo-5-phenyl-4,5,6,7-tetrahydro-1-thia-3b,6-diazacyclopenta[a]indene-5-carboxamides of general formula **1.16.**

**Table 1.17(1).** Substituted 6-alkyl- and 6-cycloalkyl-7-oxo-4,5,6,7-tetrahydro-1-oxa-3b,6-diaza-cyclopenta[a]indene-5-carboxamides of general formula **1.17**

**Table 1.17(2).** Substituted 6-aryl-7-oxo-4,5,6,7-tetrahydro-1-oxa-3b,6-diazacyclopenta[a]indene-5-carboxamides of general formula **1.17**

**Table 1.17(3).** Substituted 6-benzyl-7-oxo-4,5,6,7-tetrahydro-1-oxa-3b,6-diazacyclopenta[a]indene-5-carboxamides of general formula **1.17**

**Table 1.17(4).** Substituted 6-(2-hydroxyethyl)- and 6-(2-aminoethyl)-7-oxo-4,5,6,7-tetrahydro-1-oxa-3b,6-diaza-cyclopenta[a]indene-5-carboxamides of general formula **1.17**

**Table 1.17(5).** Substituted 6-(3-hydroxypropyl)- and 6-(3-aminopropyl)-7-oxo-4,5,6,7-tetrahydro-1-oxa-3b,6-diaza-cyclopenta[a]indene-5-carboxamides of general formula **1.17**

**Table 1.17(6).** Substituted 7-oxo-5-phenyl-4,5,6,7-tetrahydro-1-oxa-3b,6-diazacyclopenta[a]indene-5-carboxamides of general formula **1.17**

**Table 1.18.** Substituted 7-oxo-4,5,6,7-tetrahydro-3-thia-3b,6-diaza-cyclopenta[a]indene-5-carboxamides of general formula **1.18.**

**Table 1.19-20.** Substituted 7-oxo-5,6,7,8-tetrahydro-9-thia-1,6-diaza-benzocycloheptane-5-carboxamides of general formula **1.19** and 8-oxo-7,8,9,10-tetrahydro-6-thia-5,9-diazacyclopenta[b]naphthalene-10-carboxamides of general formula **1.20.**

**Table 1.21-22.** Substituted 4-oxo-2,3,3a,4,5,6-hexahydro-1*H*-benzo[f]pyrrolo[1,2-a][1,4]diazepine-6-carboxamides of general formula **1.21** and **1.22.**

**Table 1.23.** Substituted 1-oxo-1,3,4,6,11,11a-hexahydro-2*H*-pyrazino[1,2-b]isoquinoline-3-carboxamides of general formula **1.23.**

**Example 2.** Tests of biological activity of compounds of general formula 1.

A focused library was compiled to include compounds of general formula 1, as shown in Tables 1.01 to 1.23, and tested for anticancer activity. Anticancer activity of the compounds was measured by their capacity to kill cancerous cells in a tissue culture. Three cell lines representing three types of cancerous tumors were chosen, in particular: DLD-1 (adenocarcinoma of the rectum), DU-145 (carcinoma of the brain), and T-47D (tumor of the mammary gland). All the cell cultures were received from the American Tissue Culture Collection (ATCC). The efficiency of the compounds was assessed by measuring the number of dead cells following treatment with the test compounds over 48 hours. The cells were seeded at a concentration of $5*10^3$ cells per hole of a standard 96-hole plate and left overnight for cells to be attached to the hole bottom. Whereupon the test compounds were added to the cells at an ultimate concentration of 30 μM, and the plates were left for 48 hours in an incubator at 37°C maintaining the 5%$CO_2$/95% air atmosphere at 100% humidity. At the end of the experiment, the medium with the compounds was sucked out of all the holes, and the holes were rinsed twice with physiological solution. 50 μM of Alamar Blue solution was added to each hole, and fluorescence (λex = 531 nm, λem = 589 nm) was measured immediately after addition and 2 hours thereafter. The rate of fluorescence growth was proportional to the number of cells that survived incubation with the test compounds [John O'Brien, Ian Wilson, Terry Orton and François Pognan, "Investigation of the Alamar Blue (resazurin) Fluorescent Dye for the Assessment of Mammalian Cell Cytotoxicity." Eur. J. Biochem. 2000,b 267, 5421-5426]. The proportion of cell growth suppression was found from the following equation:

$$\%\text{ of growth inhibition} = ((\Delta\Phi_\kappa - \Delta\Phi_i)/\Delta\Phi_\kappa)*100\%,$$

wherein: ΔΦ means increase in fluorescence over two years following addition of the Alamar Blue solution, and the subscripts κ and *i* mean, respectively, control cells (grown without any compounds added) and experimental cells (grown in the presence of test compounds). Data for some of the test compounds are given in Table 24. It follows from Table 24 that test compounds exhibit a high activity in suppressing tumor cell growth. What is more, compounds **1.5**(3){44}, **1.5**(3){50}, **1.10** (1){18}, and **1.16** (2){28} are equally good at suppressing cell growth in all three cancer tumors, while the remaining compounds show a varying degree of selectivity toward different tumors.

**Example 3.** A method of producing a pharmaceutical composition in tablets.

A mixture is prepared from 800 mg of starch, 800 mg of ground lactose, 200 mg of talc, and 500 mg of cyclohexylamide of 6-methyl-4-oxo-5-(2-pyrrolidin-1-yl-ethyl)-2-(4-ethylphenyl)-4,5,6,7-tetrahydropyrazolo[1,5-a]pyrazine-6-carbonic acid **1.5**(3){50} and pressed into a bar. The bar thus produced is crushed into granules and screened so that 14 to 16 mesh granules could be collected. The granules are then compressed into tablets of suitable shape weighing 280 mg each. Similarly, pharmaceutical compositions are obtained in the form of tablets containing other 1,3-dioxo-2,3-dihydro-1*H*-pyrrolo[3,4-*c*]quinolines of general formula 1 as the active ingredient.

**Example 4.** A method of producing a pharmaceutical composition in capsules.

Compound **1.5**(3){50} is carefully mixed with powdered lactose at a ratio of 2:1, and the resulting mixture is packed into gelatin capsule of suitable size 300 mg to a capsule.

**Example 5.** A method of producing a pharmaceutical composition for intramuscular, intraperitoneal, or subcutaneous injections.

A hydrochloride of compound **1.5**(3){50} is mixed with 300 mg of chlorobutanol, 2 ml of propylene glycol, and 100 ml of injection water. The resultant solution is filtered and placed in 1 ml ampoules, which are sealed and sterilized in an autoclave.

**Commercial Applicability**

This invention may be used in medicine, veterinary, biochemistry, and general chemistry.

| | Table 1.01(1) | | | |
|---|---|---|---|---|
| № | Formula | Mol. Weight | LC MS, m/z (M+1) | 1H NMR |
| 1 | 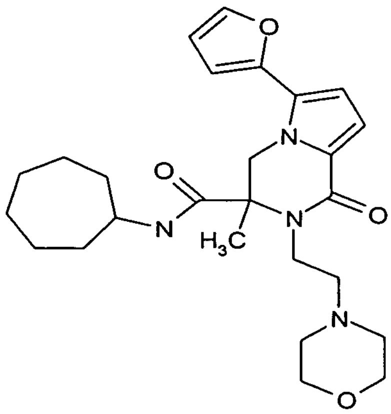 | 468,60122 | 469 | m. 1.27-1.71 (15H, CH and CH3 of alkyl), m. 2.35-2.62 (6H, CH of alkyl), m. 3.50-3.71 (7H, CH of alkyl and CH of cycloheptyl), dd. 4.00, 4.87 (J=12.7Hz, 2H, CH2 of cycle), d. 6.37 (J=4.0Hz, 1 H, CH of furan ring), br.s. 6.52 (1 H, CH of pyrrol ring), ... |
| 2 | 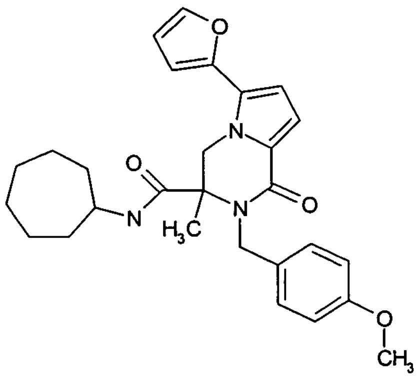 | 475,59291 | 476 | 0.89 (10; 6H; m.), 1.05 - 1.45 (8, 9, 14; 5H; m.), 1.45 (4; 3H, s.), 1.50 - 1.91, 2.50 - 2.89, 3.05 - 3.28 (7, 11, 12, 13; 11H; m.m.), 3.95 + 4.41 (3; 2H; AB; 12.4 Hz), 6.45 (2; 1H; d., 1.4 Hz), 6.52 (5; 1H; s.), 7.42 (1; 1 H; d., 1.4 Hz), 8.15 (6; 1H... |
| 3 | 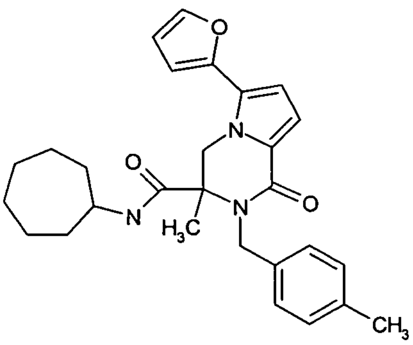 | 459,59351 | 460 | |

(continued)

| | Table 1.01(1) | | | |
|---|---|---|---|---|
| № | Formula | Mol. Weight | LC MS, m/z (M+1) | 1H NMR |
| 4 | 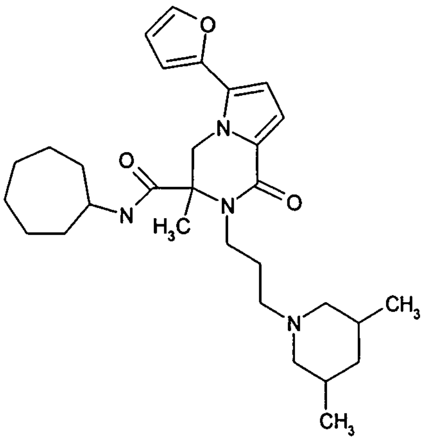 | 508,71018 | 509 | |

| | Table 1.01 (2) | | |
|---|---|---|---|
| № | Formula | Mol. Weight | LC MS, m/z (M+1) |
| 1 | 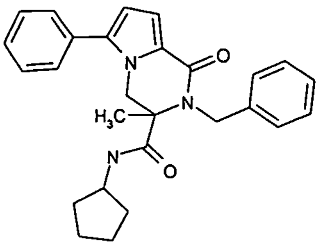 | 427,55108 | 428 |
| 2 | 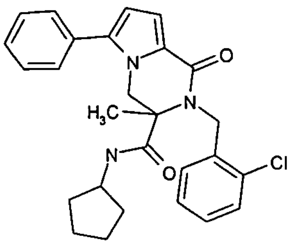 | 461,99611 | 462 |
| 3 | 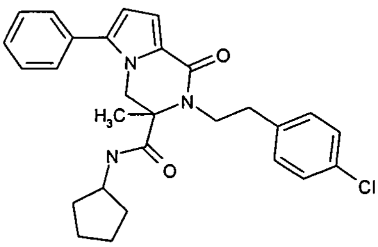 | 476,0232 | 477 |

(continued)

| | Table 1.01 (2) | | |
|---|---|---|---|
| № | Formula | Mol. Weight | LC MS, m/z (M+1) |
| 4 | 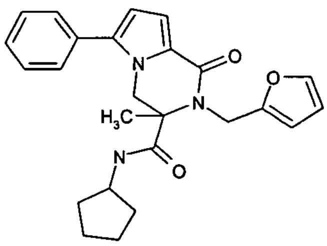 | 417,51224 | 418 |
| 5 | 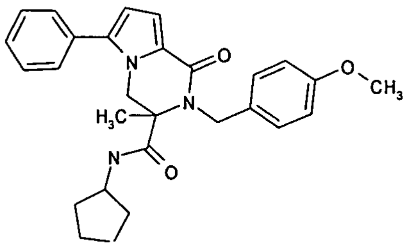 | 457,57757 | 458 |
| 6 | 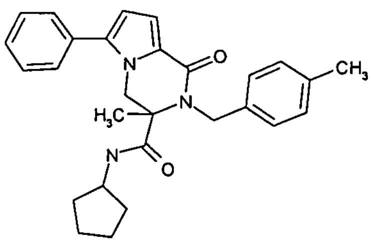 | 441,57817 | 442 |
| 7 | 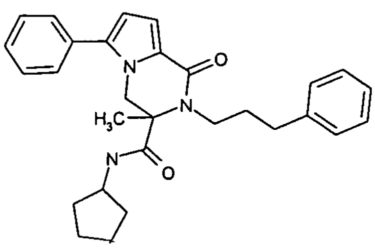 | 455,60526 | 456 |
| 8 | 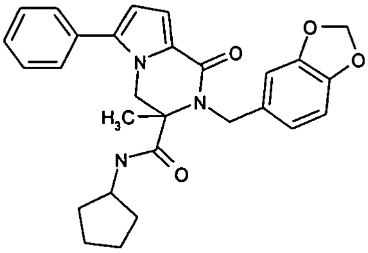 | 471,56103 | 472 |
| 9 | 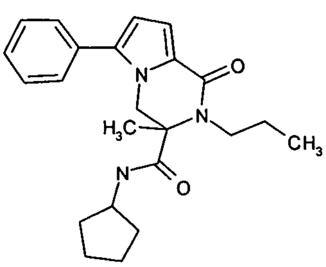 | 379,50648 | 380 |

(continued)

| | Table 1.01 (2) | | |
|---|---|---|---|
| № | Formula | Mol. Weight | LC MS, m/z (M+1) |
| 10 | 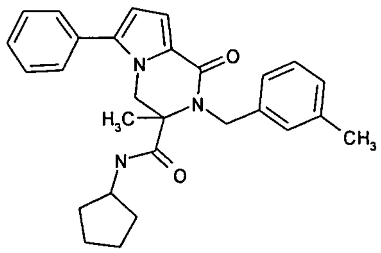 | 441,57817 | 442 |
| 11 | 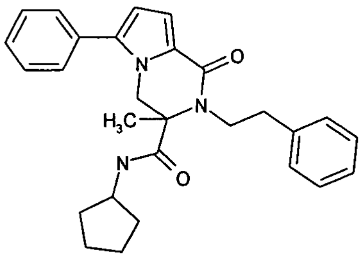 | 441,57817 | 442 |
| 12 | 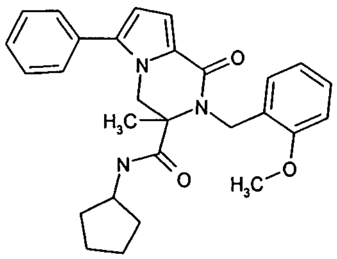 | 457,57757 | 458 |
| 13 | 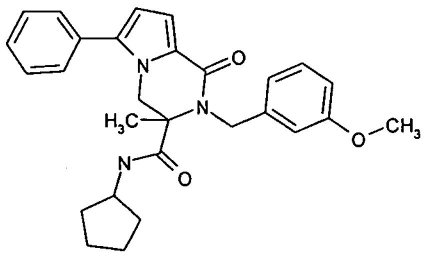 | 457,57757 | 458 |
| 14 | 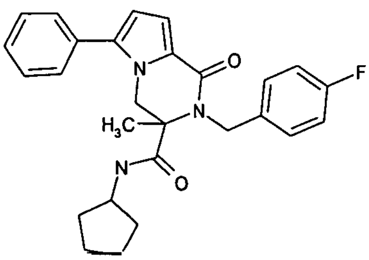 | 445,54151 | 446 |

(continued)

| | Table 1.01 (2) | | |
|---|---|---|---|
| № | Formula | Mol. Weight | LC MS, m/z (M+1) |
| 15 | 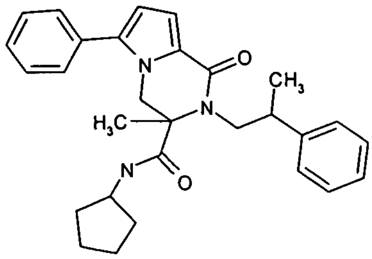 | 455,60526 | 456 |
| 16 | 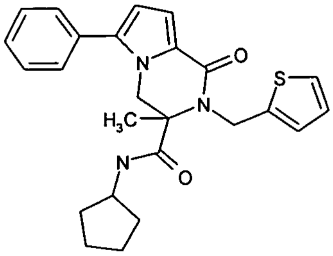 | 433,57684 | 434 |
| 17 | 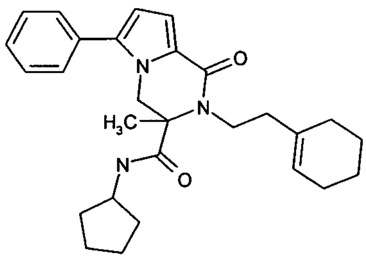 | 445,61005 | 446 |
| 18 | 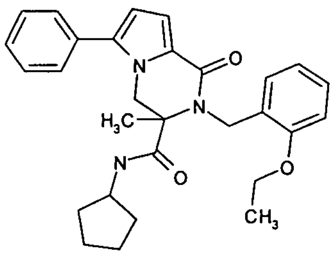 | 471,60466 | 472 |
| 19 | 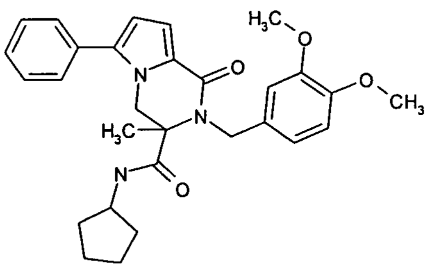 | 487,60406 | 488 |

(continued)

| | Table 1.01 (2) | | |
|---|---|---|---|
| № | Formula | Mol. Weight | LC MS, m/z (M+1) |
| 20 | 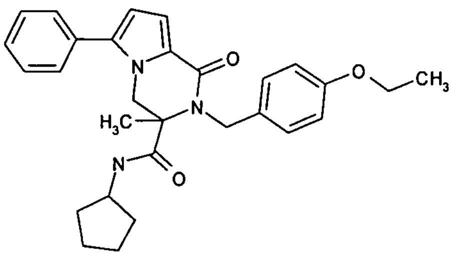 | 471,60466 | 472 |
| 21 | 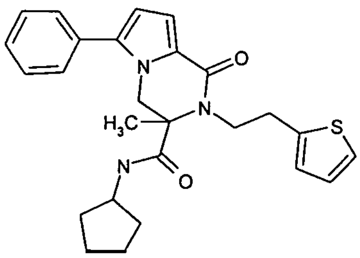 | 447,60393 | 448 |
| 22 | 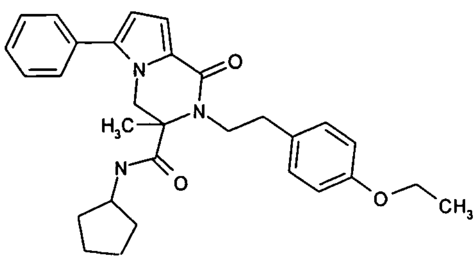 | 485,63175 | 486 |
| 23 | 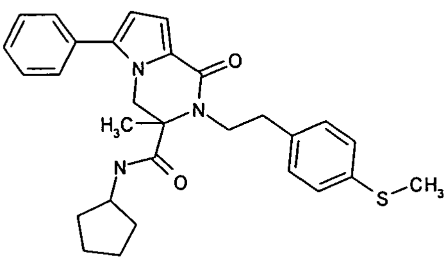 | 487,66926 | 488 |
| 24 | 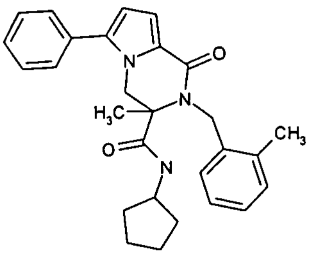 | 441,57817 | 442 |

(continued)

| | Table 1.01 (2) | | |
|---|---|---|---|
| № | Formula | Mol. Weight | LC MS, m/z (M+1) |
| 25 | 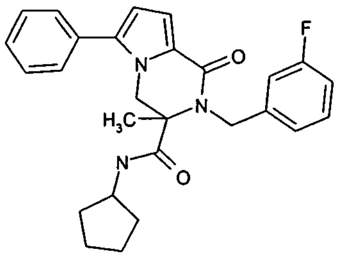 | 445,54151 | 446 |
| 26 | 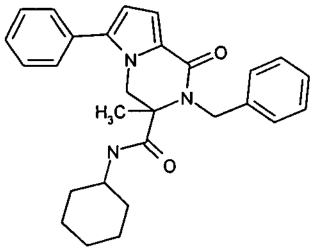 | 441,57817 | 442 |
| 27 | 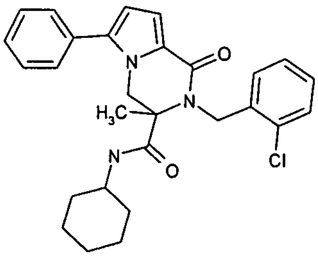 | 476,0232 | 477 |
| 28 | 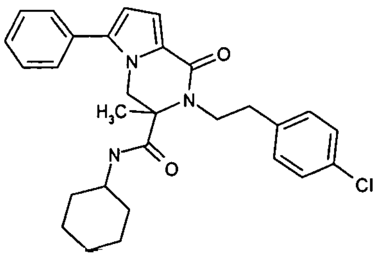 | 490,05029 | 491 |
| 29 | 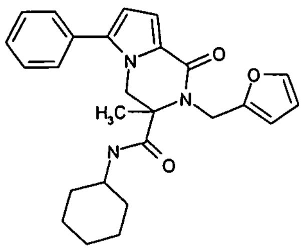 | 431,53933 | 432 |

(continued)

| | Table 1.01 (2) | | |
|---|---|---|---|
| № | Formula | Mol. Weight | LC MS, m/z (M+1) |
| 30 | 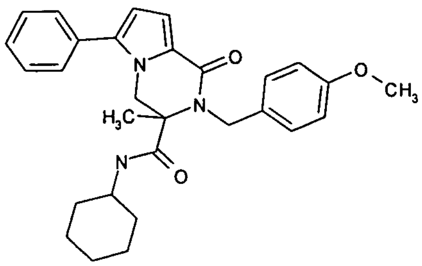 | 471,60466 | 472 |
| 31 | 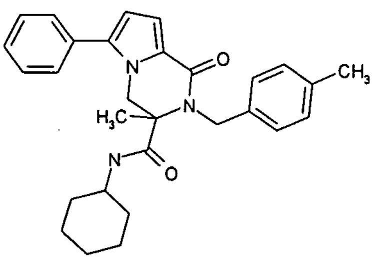 | 455,60526 | 456 |
| 32 | 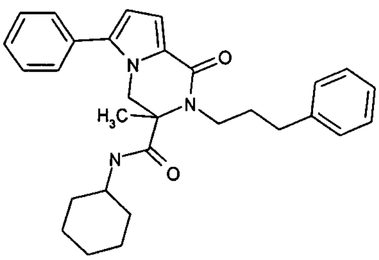 | 469,63235 | 470 |
| 33 | 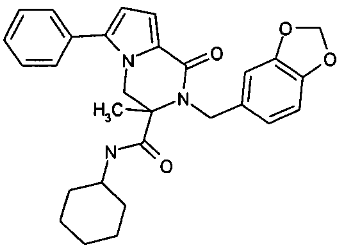 | 485,58812 | 486 |
| 34 | 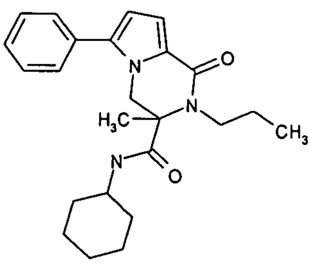 | 393,53357 | 394 |

(continued)

| | Table 1.01 (2) | | |
|---|---|---|---|
| № | Formula | Mol. Weight | LC MS, m/z (M+1) |
| 35 | 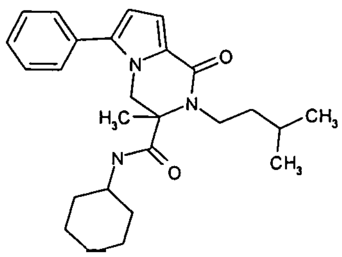 | 421,58775 | 422 |
| 36 | 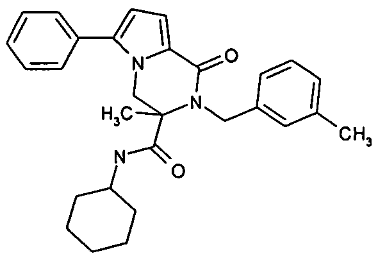 | 455,60526 | 456 |
| 37 | 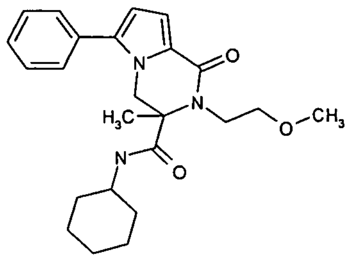 | 409,53297 | 410 |
| 38 | 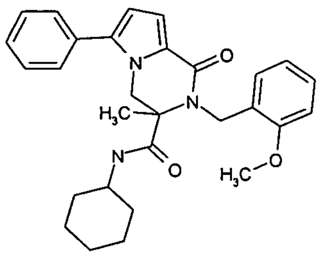 | 471,60466 | 472 |
| 39 | 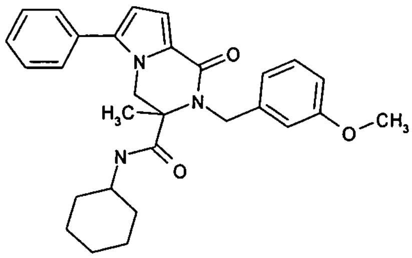 | 471,60466 | 472 |

(continued)

| | Table 1.01 (2) | | |
|---|---|---|---|
| № | Formula | Mol. Weight | LC MS, m/z (M+1) |
| 40 | 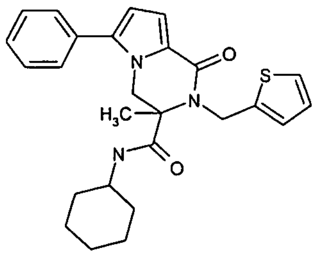 | 447,60393 | 448 |
| 41 | 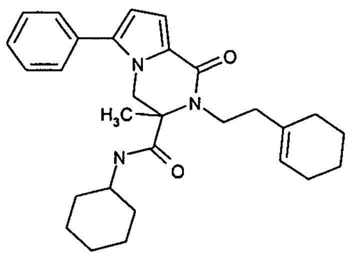 | 459,63714 | 460 |
| 42 | 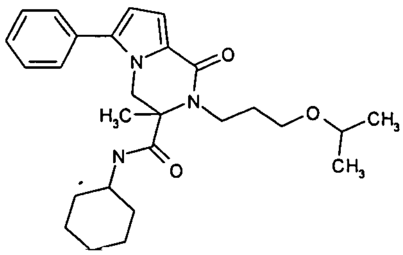 | 451,61424 | 452 |
| 43 | 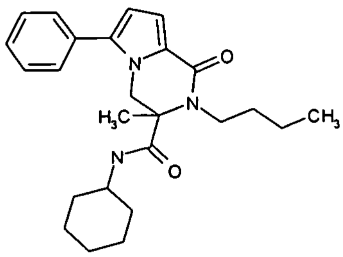 | 407,56066 | 408 |
| 44 | 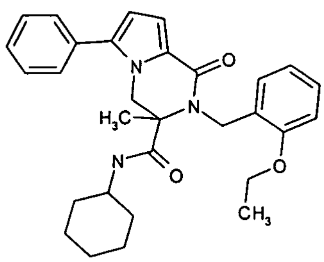 | 485,63175 | 486 |

(continued)

| | Table 1.01 (2) | | |
|---|---|---|---|
| № | Formula | Mol. Weight | LC MS, m/z (M+1) |
| 45 | 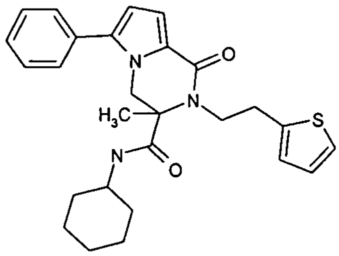 | 461,63102 | 462 |
| 46 | 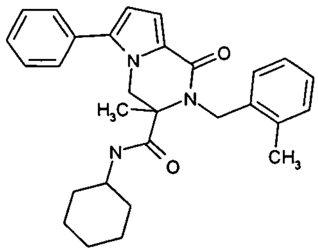 | 455,60526 | 456 |
| 47 | 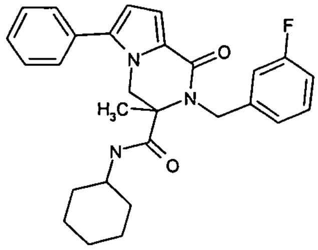 | 459,5686 | 460 |
| 48 | 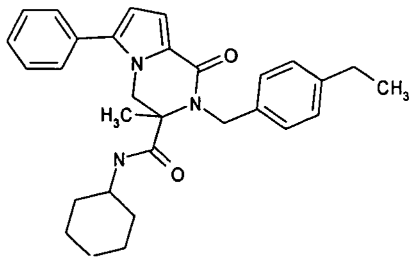 | 469,63235 | 470 |
| 49 | 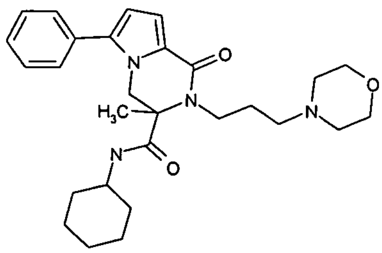 | 478,64006 | 479 |
| 50 | 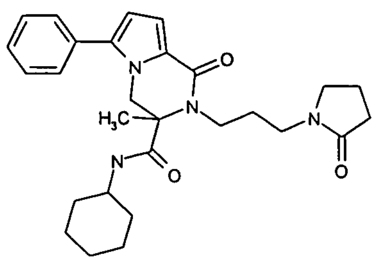 | 476,62412 | 477 |

(continued)

| | Table 1.01 (2) | | |
|---|---|---|---|
| № | Formula | Mol. Weight | LC MS, m/z (M+1) |
| 51 | 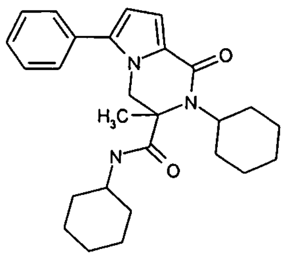 | 433,5989 | 434 |
| 52 | 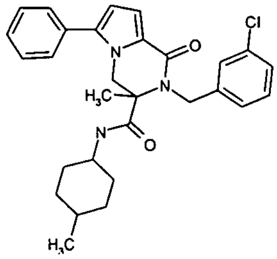 | 490,05029 | 491 |
| 53 | 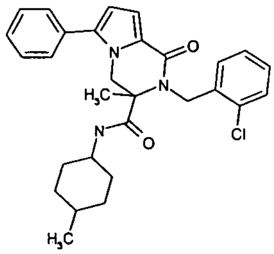 | 490,05029 | 491 |
| 54 | 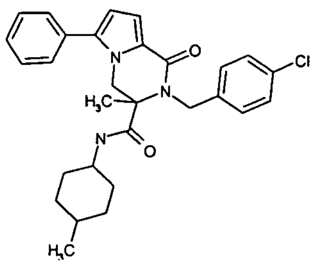 | 490,05029 | 491 |
| 55 | 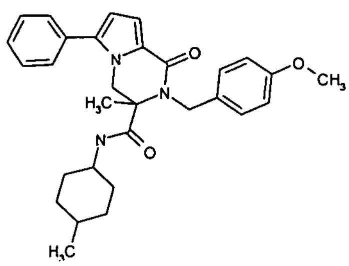 | 485,63175 | 486 |
| 56 | 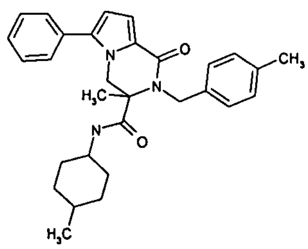 | 469,63235 | 470 |

(continued)

| | Table 1.01 (2) | | |
|---|---|---|---|
| № | Formula | Mol. Weight | LC MS, m/z (M+1) |
| 57 | 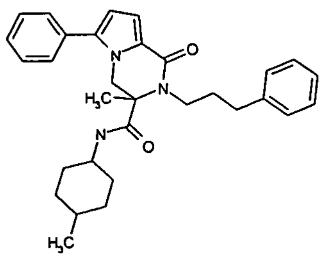 | 483,65944 | 484 |
| 58 | 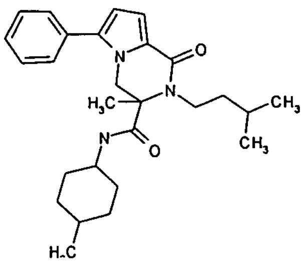 | 435,61484 | 436 |
| 59 | 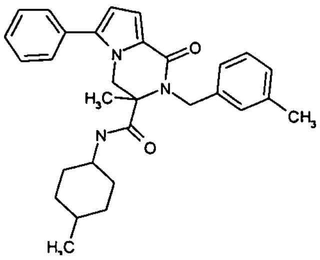 | 469,63235 | 470 |
| 60 | 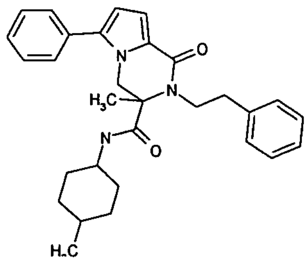 | 469,63235 | 470 |
| 61 | 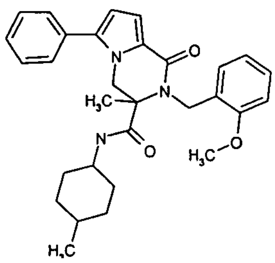 | 485,63175 | 486 |

(continued)

| | Table 1.01 (2) | | |
|---|---|---|---|
| № | Formula | Mol. Weight | LC MS, m/z (M+1) |
| 62 | 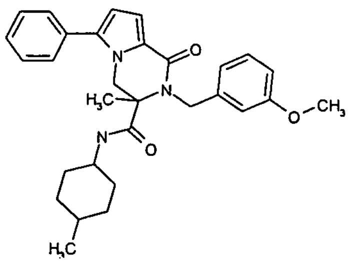 | 485,63175 | 486 |
| 63 | 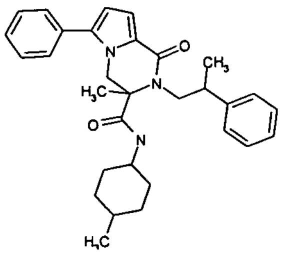 | 483,65944 | 484 |
| 64 | 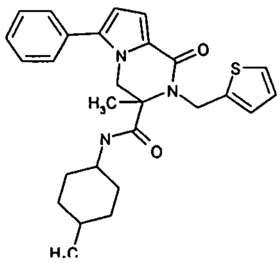 | 461,63102 | 462 |
| 65 | 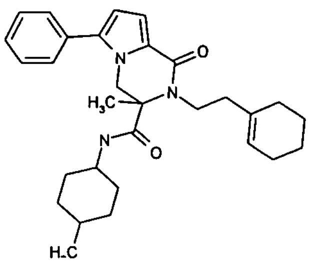 | 473,66423 | 474 |
| 66 | 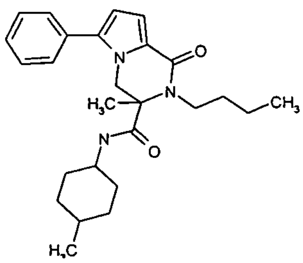 | 421,58775 | 422 |

(continued)

| | Table 1.01 (2) | | |
|---|---|---|---|
| № | Formula | Mol. Weight | LC MS, m/z (M+1) |
| 67 | 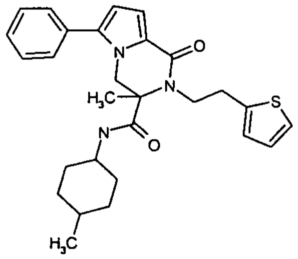 | 475,65811 | 476 |
| 68 | 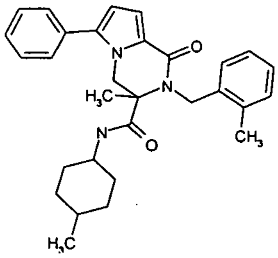 | 469,63235 | 470 |
| 69 | 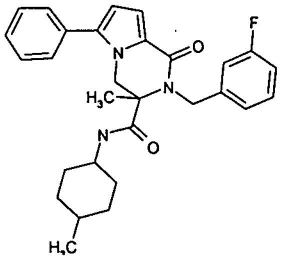 | 473,59569 | 474 |
| 70 | 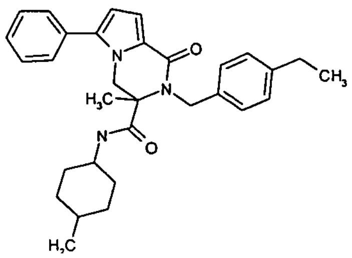 | 483,65944 | 484 |
| 71 | 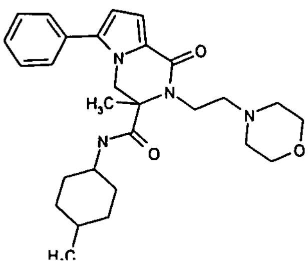 | 478,64006 | 479 |

(continued)

| | Table 1.01 (2) | | |
|---|---|---|---|
| № | Formula | Mol. Weight | LC MS, m/z (M+1) |
| 72 | 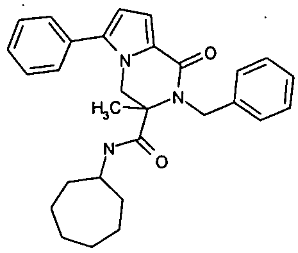 | 455,60526 | 456 |
| 73 | 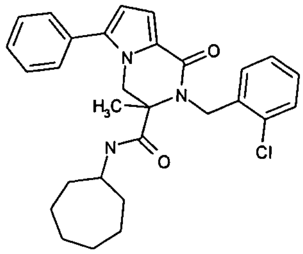 | 490,05029 | 491 |
| 74 | 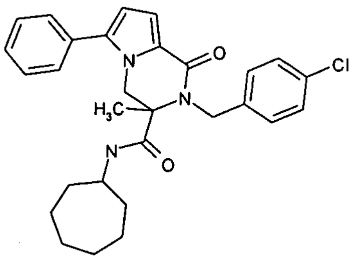 | 490,05029 | 491 |
| 75 | 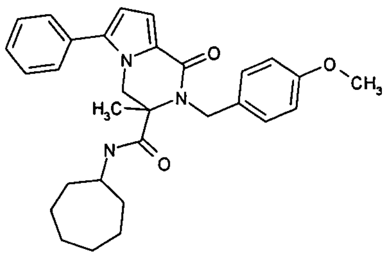 | 485,63175 | 486 |
| 76 | 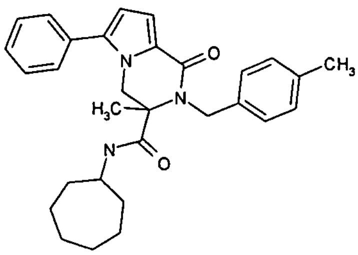 | 469,63235 | 470 |

(continued)

| | Table 1.01 (2) | | |
|---|---|---|---|
| № | Formula | Mol. Weight | LC MS, m/z (M+1) |
| 77 | 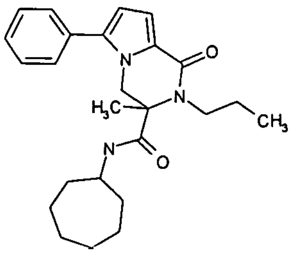 | 407,56066 | 408 |
| 78 | 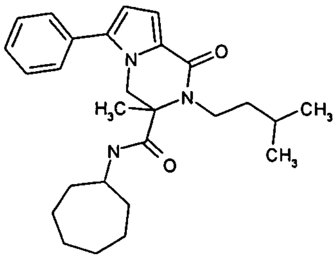 | 435,61484 | 436 |
| 79 | 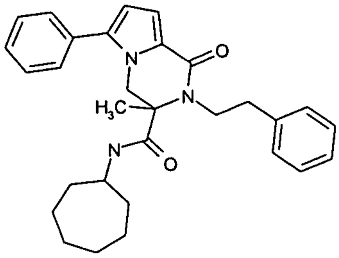 | 469,63235 | 470 |
| 80 | 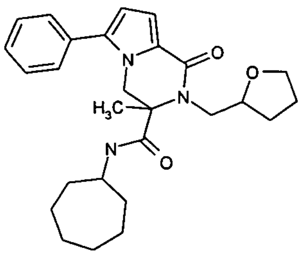 | 449,5983 | 450 |
| 81 | 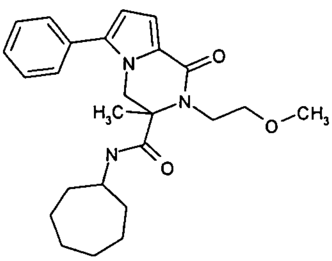 | 423,56006 | 424 |

(continued)

| | Table 1.01 (2) | | |
|---|---|---|---|
| № | Formula | Mol. Weight | LC MS, m/z (M+1) |
| 82 | 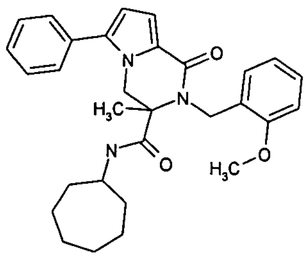 | 485,63175 | 486 |
| 83 | 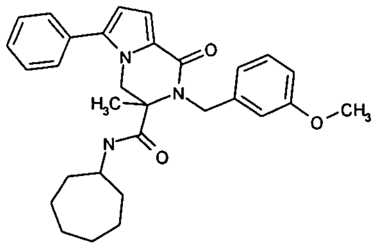 | 485,63175 | 486 |
| 84 | 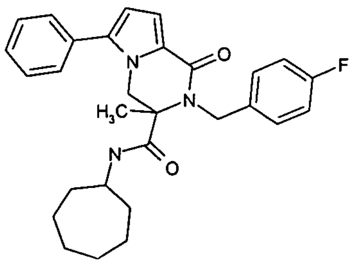 | 473,59569 | 474 |
| 85 | 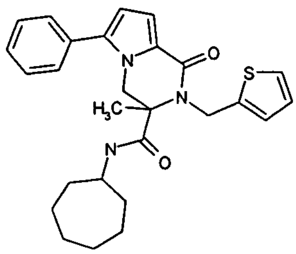 | 461,63102 | 462 |
| 86 | 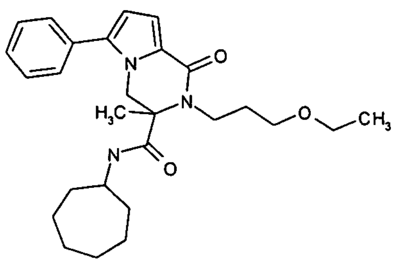 | 451,61424 | 452 |

(continued)

| | Table 1.01 (2) | | |
|---|---|---|---|
| № | Formula | Mol. Weight | LC MS, m/z (M+1) |
| 87 | 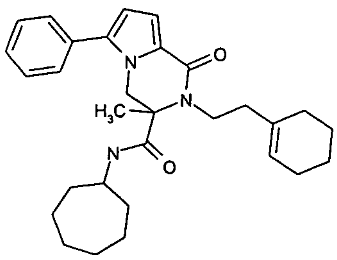 | 473,66423 | 474 |
| 88 | 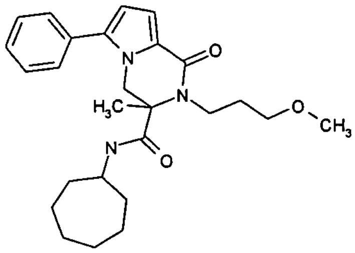 | 437,58715 | 438 |
| 89 | 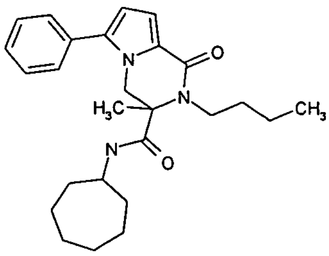 | 421,58775 | 422 |
| 90 | 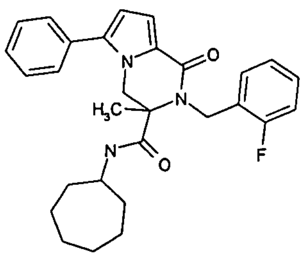 | 473,59569 | 474 |
| 91 | 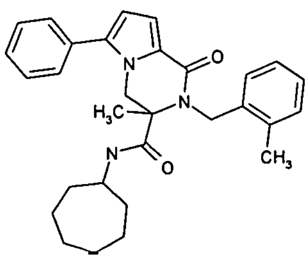 | 469,63235 | 470 |

(continued)

| | Table 1.01 (2) | | |
|---|---|---|---|
| № | Formula | Mol. Weight | LC MS, m/z (M+1) |
| 92 | 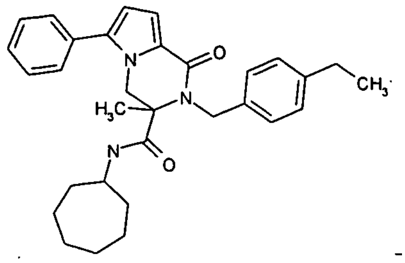 | 483,65944 | 484 |
| 93 | 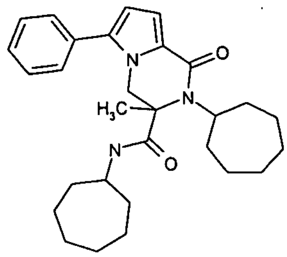 | 461,65308 | 462 |
| 94 | 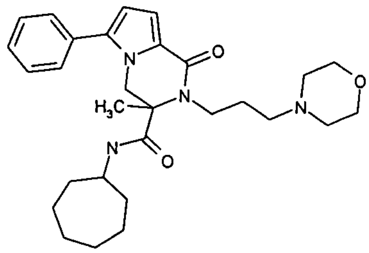 | 492,66715 | 493 |
| 95 | 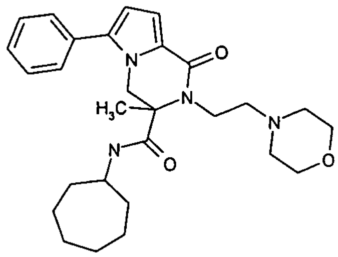 | 478,64006 | 479 |
| 96 | 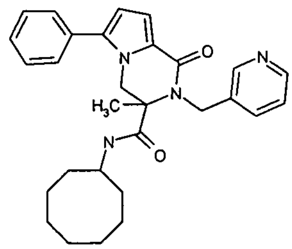 | 470,61993 | 471 |

(continued)

| | Table 1.01 (2) | | |
|---|---|---|---|
| № | Formula | Mol. Weight | LC MS, m/z (M+1) |
| 97 | 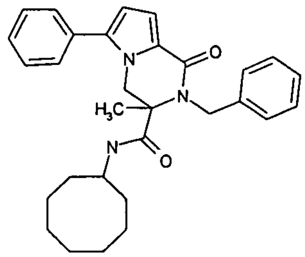 | 469,63235 | 470 |
| 98 | 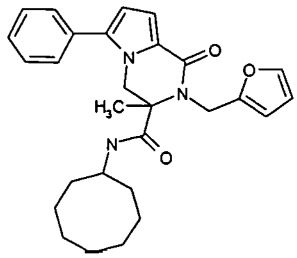 | 459,59351 | 460 |
| 99 | 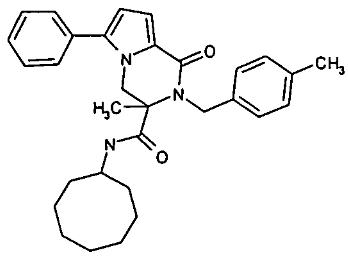 | 483,65944 | 484 |
| 100 | 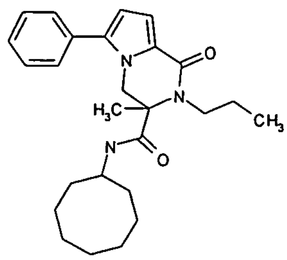 | 421,58775 | 422 |
| 101 | 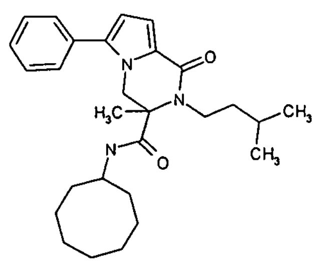 | 449,64193 | 450 |

(continued)

| | Table 1.01 (2) | | |
|---|---|---|---|
| № | Formula | Mol. Weight | LC MS, m/z (M+1) |
| 102 | 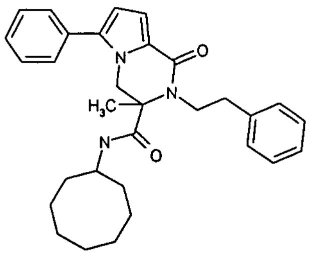 | 483,65944 | 484 |
| 103 | 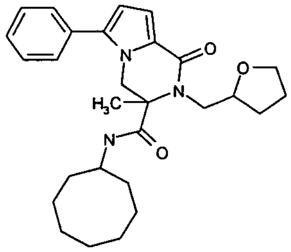 | 463,62539 | 464 |
| 104 | 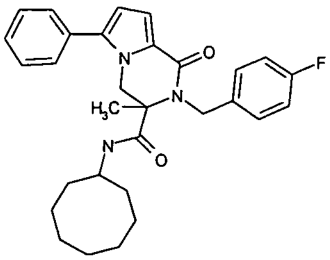 | 487,62278 | 488 |
| 105 | 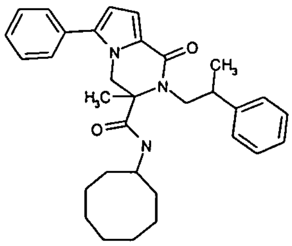 | 497,68653 | 498 |
| 106 | 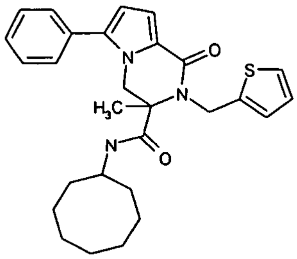 | 475,65811 | 476 |

(continued)

| | Table 1.01 (2) | | |
|---|---|---|---|
| № | Formula | Mol. Weight | LC MS, m/z (M+1) |
| 107 | 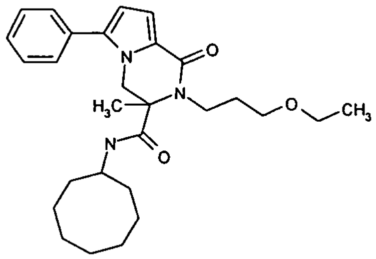 | 465,64133 | 466 |
| 108 | 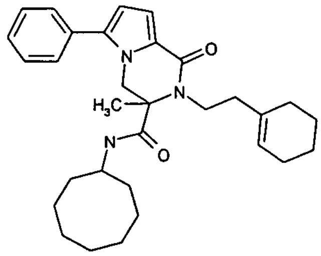 | 487,69132 | 488 |
| 109 | 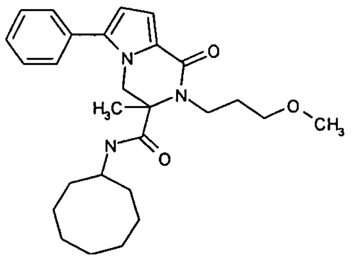 | 451,61424 | 452 |
| 110 | 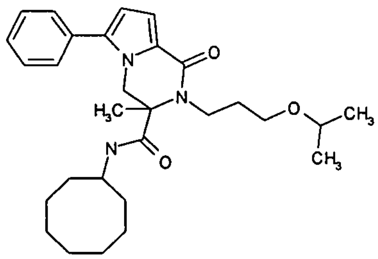 | 479,66842 | 480 |
| 111 | 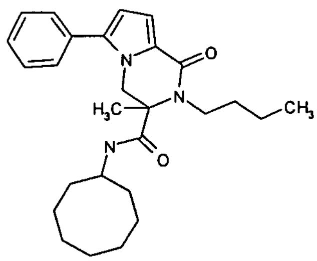 | 435,61484 | 436 |

(continued)

| | Table 1.01 (2) | | |
|---|---|---|---|
| № | Formula | Mol. Weight | LC MS, m/z (M+1) |
| 112 | 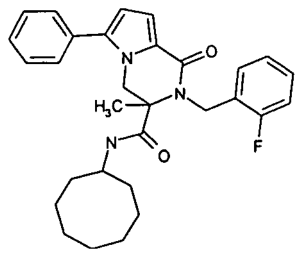 | 487,62278 | 488 |
| 113 | 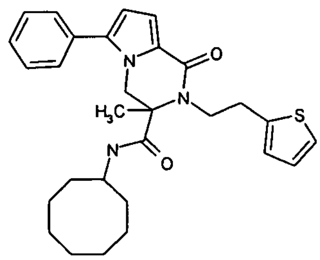 | 489,6852 | 490 |
| 114 | 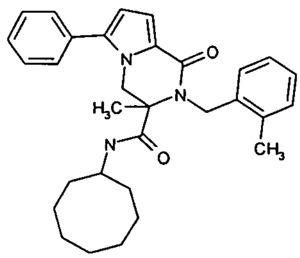 | 483,65944 | 484 |
| 115 | 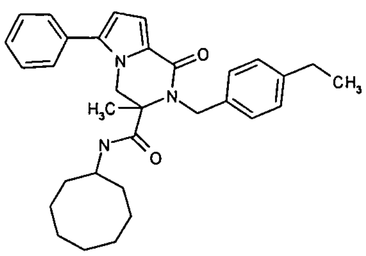 | 497,68653 | 498 |
| 116 | 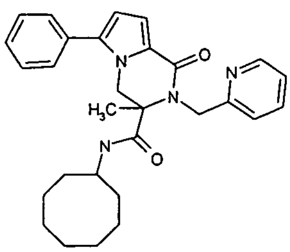 | 470,61993 | 471 |

(continued)

| | Table 1.01 (2) | | |
|---|---|---|---|
| № | Formula | Mol. Weight | LC MS, m/z (M+1) |
| 117 | 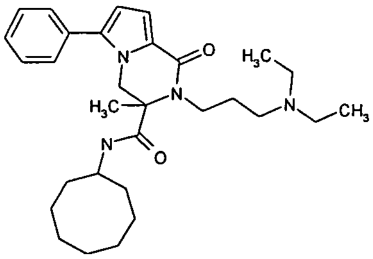 | 492,71078 | 493 |
| 118 | 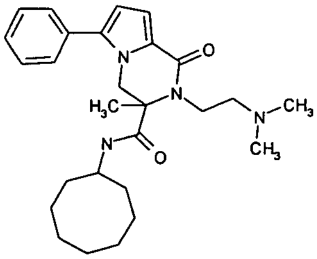 | 450,62951 | 451 |
| 119 | 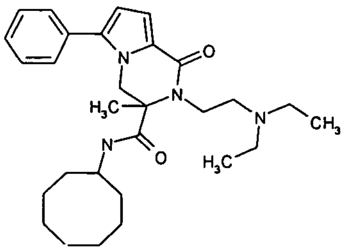 | 478,68369 | 479 |
| 120 | 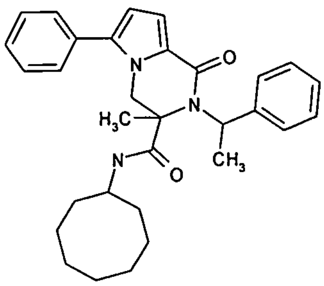 | 483,65944 | 484 |
| 121 | 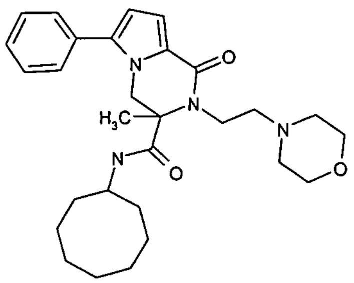 | 492,66715 | 493 |

(continued)

| | Table 1.01 (2) | | |
|---|---|---|---|
| No | Formula | Mol. Weight | LC MS, m/z (M+1) |
| 122 | 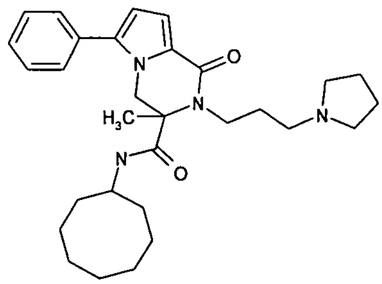 | 490,69484 | 491 |
| 123 | 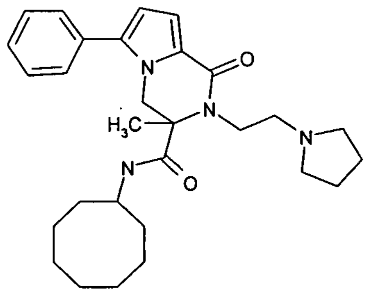 | 476,66775 | 477 |

Table 1.02

| No | Formula | Spectral data |
|---|---|---|
| 1 | 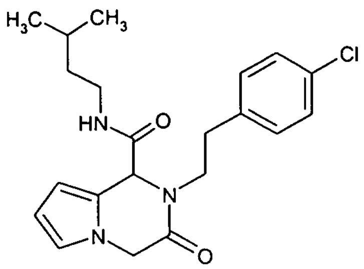 | $^{1}$H-NMR (400 MHz, DMSO-$d_6$+$CCl_4$): δ 8.1-8.0 (br s, 1H), 7.3-7.1 (m, 4H), 6.55 (s, 1H), 6.0-5.9 (d, *J* = 8.6 Hz, 2H), 5.06 (s, 1H), 4.7 (d, *J* = 5.4 Hz, 1H), 4.5 (d, *J* = 5.4 Hz, 1H), 3.81-3.74 (m, 1H), 3.2-3.08 (m, 3H), 3.0-2.8 (m, 1H), 2.8-2.6 (m, 1H), 1.65-1.5 (m, 1H), 1.38-1.22 (q, *J* = 4.6 Hz, 2H); HRMS (*m/z*): [M]$^+$ calcd for $C_{21}H_{26}ClN_3O_2$, 387.9133; found, 387.9138. |
| 2 | 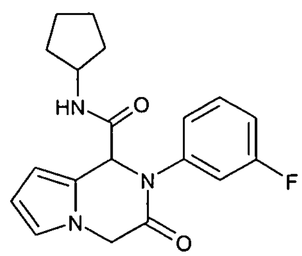 | $^{1}$H-NMR (400 MHz, DMSO-$d_6$+$CCl_4$): δ 8.16 (d, *J* = 7.8 Hz, 1H), 7.4 ($q_{(F)}$, *J*= 7.6 Hz, 1H), 7.1-6.9 (m, 3H), 6.63 (s, 1H), 6.0-5.9 (d, *J* = 9.4 Hz, 2H), 5.4 (s, 1H), 4.9 (d, *J* = 5.4 Hz, 1H), 4.7 (d, *J* = 5.4 Hz, 1H), 4.01-3.85 (m, 1H), 2.0-1.2 (m, 8H); HRMS (*m/z*): [M]$^+$ calcd for $C_{19}H_{20}FN_3O_2$, 341.3886; found, 341.3879. |
| 3 | 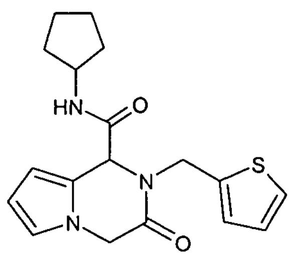 | $^{1}$H-NMR (400 MHz, DMSO-$d_6$+$CCl_4$): δ 8.12 (d, *J* = 7.6 Hz, 1H), 7.24 (d, *J* = 8.2 Hz, 1H), 7.0 (d, *J* = 8.2 Hz, 1H), 7.0-6.82 (t, *J* = 8.0 Hz, 1H), 6.58 (s, 1H), 6.0-5.9 (d, *J* = 5.9 Hz, 2H), 5.3 (d, *J* = 5.6 Hz, 1H), 5.1 (s, 1H), 4.75 (d, *J* = 5.6 Hz, 1H), 4.6 (d, *J* = 5.6 Hz, 1H), 4.1 (d, *J* = 5.6 Hz, 1H), 4.02-3.87 (m, 1H), 2.0-1.1 (m, 8H); HRMS (m/z): [M]$^+$ calcd for $C_{18}H_{21}N_3O_2S$, 343.4510; found, 343.4516. |

(continued)

| № | Formula | Spectral data |
|---|---|---|
| 4 | 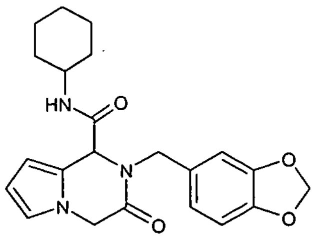 | $^1$H-NMR (400 MHz, DMSO-$d_6$+$CCl_4$): δ 8.2 (br s, 1H), 7.2 (d, $J$ = 7.8 Hz, 1H), 7.0 (s, 1H), 6.7 (d, $J$ = 7.8 Hz, 1H), 6.55 (s, 1H), 6.0-5.9 (d, $J$= 8.6 Hz, 2H), 5.1 (s, 1H), 4.8 (d, $J$ = 5.4 Hz, 1H), 4.51 (d, $J$ = 5.4 Hz, 1H), 3.83-3.79 (m, 2H), 3.2-3.03 (m, 2H), 3.0-2.8 (m, 1H), 1.9-1.0 (m, 10H); HRMS (*m/z*): $[M]^+$ calcd for $C_{22}H_{25}N_3O_4$, 395.4623; found, 395.4620. |
| 5 | 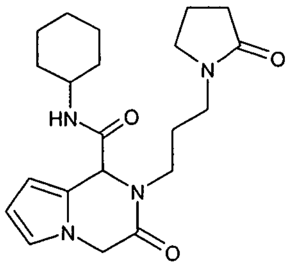 | $^1$H-NMR (400 MHz, DMSO-$d_6$+$CCl_4$): δ 8.02 (d, $J$ = 7.6 Hz, 1H), 6.55 (s, 1H), 6.0-5.8 (d, $J$ = 8.2 Hz, 2H), 5.1 (s, 1H), 4.72 (d, $J$ = 5.4 Hz, 1H), 4.45 (d, $J$ = 5.4 Hz, 1H), 3.6-3.5 (m, 1H), 3.5-3.4 (m, 1H), 3.4-3.3 (m, 2H), 3.3-3.15 (m, 2H), 3.1-2.9 (m, 1H), 2.22-1.13 (m, 16H); HRMS (*m/z*): $[M]^+$ calcd for $C_{21}H_{30}N_4O_2$, 386.4982; found, 386.4978. |
| 6 | 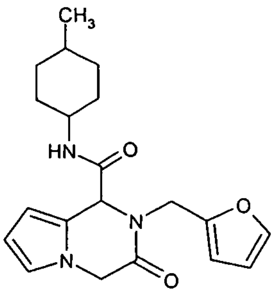 | $^1$H-NMR (400 MHz, DMSO-$d_6$+-$CCl_4$): δ 8.10 (d, $J$ = 7.4 Hz, 1H), 7.26 (d, $J$ = 8.4 Hz, 1H), 7.1 (d, $J$ = 8.4 Hz, 1H), 7.1-6.9 (t, $J$ = 8.2 Hz, 1H), 6.53 (s, 1H), 6.0-5.88 (d, $J$= 5.8 Hz, 2H), 5.31 (d, $J$= 5.6 Hz, 1H), 5.11 (s, 1H), 4.73 (d, $J$ = 5.6 Hz, 1H), 4.63 (d, $J$ = 5.6 Hz, 1H), 4.1 (d, $J$= 5.6 Hz, 1H), 391-3.82 (m, 1H), 2.1-1.0 (m, 9H); 0.98 (d, $J$= 4.6 Hz, 2H), 0.93 (d, $J$ = 4.6 Hz, 1H); HRMS (*m/z*): $[M]^+$ calcd for $C_{20}H_{25}N_2O_3$, 355.4423; found, 355.4416. |
| 7 | 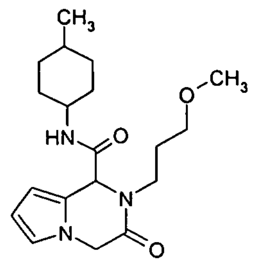 | $^1$H-NMR (400 MHz, DMSO-$d_6$+$CCl_4$): δ 7.83 (br s, 1H), 6.52 (s, 1H), 6.0-5.89 (d, $J$= 7.4 Hz, 2H), 5.2 (s, 1H), 4.65 (d, $J$ = 5.6 Hz, 1H), 4.46 (d, $J$ = 5.6 Hz, 1H), 3.88-3.71 (m, 2H), 3.51-3.3 (m, 2H), 3.22 (s, 3H), 3.1-3.0 (m, 1H), 1.92-1.0 (m, 9H), 0.99 (d, $J$ = 4.6 Hz, 2H), 0.95 (d, $J$= 4.6 Hz, 1H); HRMS (*m/z*): $[M]^+$ calcd for $C_{18}H_{27}N_3O_3$, 333.4342; found, 333.4340. |
| 8 | 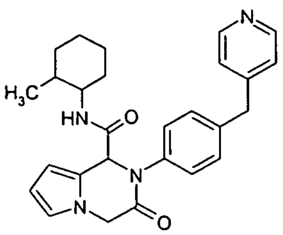 | $^1$H-NMR (400 MHz, DMSO-$d_6$+$CCl_4$): δ 8.4 (s, 2H), 7.9-7.69 (m, 1H), 7.3-7.0 (m, 6H), 6.60 (s, 1H), 6.08-6.0 (d, $J$ = 8.4 Hz, 2H), 5.4 (s, 1H), 4.8 (d, $J$ = 5.6 Hz, 1H), 4.62 (d, $J$ = 5.6 Hz, 1H), 4.0 (s, 2H), 3.18-3.03 (m, 1H), 1.8-1.0 (m, 9H), 0.8-0.63 (m, 3H); HRMS (*m/z*): $[M]^+$ calcd for $C_{27}H_{30}N_4O_2$, 442.5658; found, 442.5653. |
| 9 | 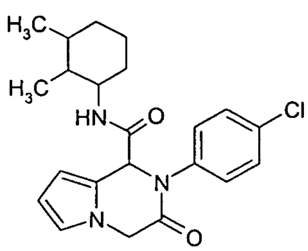 | $^1$H-NMR (400 MHz, DMSO-$d_6$+$CCl_4$): δ 7.8-7.68 (d, $J$= 6.4 Hz, 1H), 7.3 (d, $J$ = 7.8 Hz, 2H), 7.2 (d, $J$ = 7.8 Hz, 2H), 6.56 (s, 1H), 6.1-5.95 (d, $J$ = 7.6 Hz, 2H), 5.23 (s, 1H), 4.72 (d, $J$= 5.4 Hz, 1H), 4.55 (d, $J$ = 5.4 Hz, 1H), 3.2-3.11 (m, 1H), 2.0-0.6 (m, 8H), 0.8 (s, 3H), 0.68 (s, 3H); HRMS (*m/z*): $[M]^+$ calcd for $C_{22}H_{26}ClN_3O_2$, 399.9244; found, 399.9248. |

(continued)

| № | Formula | Spectral data |
|---|---|---|
| 10 | 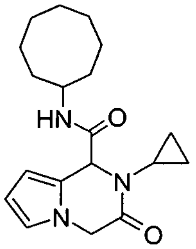 | $^1$H-NMR (400 MHz, DMSO-$d_6$+$CCl_4$): δ 8.03 (d, *J* = 7.8 Hz, 1H), 6.51 (s, 1H), 6.0-5.92 (d, *J* = 7.6 Hz, 2H), 5.1 (s, 1H), 4.68 (d, *J*= 5.4 Hz, 1H), 4.48 (d, *J* = 5.4 Hz, 1H), 4.1-3.92 (m, 1H), 3.81-3.67 (m, 1H), 1.91-1.31 (m, 14H), 1.00-0.5 (m, 4H); HRMS (*m*/*z*): $[M]^+$ calcd for $C_{19}H_{27}N_3O_2$, 329.4459; found, 329.4463. |

**Table 1.03**

| № | Formula | Mol. Weight | HRMS *m*/*z* | Spectral data |
|---|---|---|---|---|
| 1 | 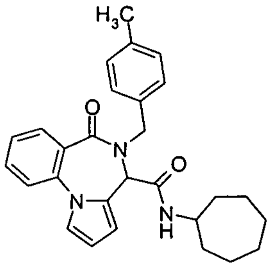 | 476.2099 | 476.2091 | $^1$HNMR (400MHz, DMSO+$CCl_4$): δ 0.75-1.6 (m, 12 H, cyclooctane), 2.3 (s, 3 H, $CH_3$), 3.3-3.48 (m, 1 H, CH), 4.6-4.7 (q, *J* = 5.6 Hz, 2 H, $CH_2$), 5.15 (s, 1 H, CH), 5.9 (d, *J* = 2.2 Hz, 1 H, NH), 6.0-6.08 (s, 1 H, ArH), 6.18-6.2 (s, 1 H, ArH), 7.0-7.4 (m, 7 H, ArH), 7.78 (d, *J*= 7.7 Hz, 1 H, ArH) |
| 2 | 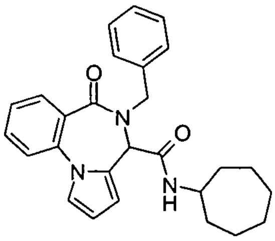 | 462.1943 | 462.1935 | $^1$HNMR (400MHz, DMSO+$CCl_4$): δ 0.73-1.58 (m, 12 H, cyclooctane), 3.28-3.47 (m, 1 H, CH), 4.61-4.72 (d, *J*= 5.9 Hz, 2 H, $CH_2$), 4.8-4.96 (d, *J*= 5.9 Hz, 2 H, $CH_2$), 5.12 (s, 1 H, CH), 5.9-6.0 (m, 2 H, ArH, NH), 6.18 (s, 1 H, ArH), 7.0-7.4 (m, 8 H, ArH), 7.77 (d, *J* = 7.8 Hz, 1 H, ArH) |

**Table 1.04.** Substituted 5-oxo-1,3,4,5,6,7,8,9-octahydro-2*H*-10-thia-6,9-diaza-benzo[a]-cyclopenta[e]azulene-7-carboxamides of general formula **1.4.**

| № | Formula | Mol. weight | HRMS *m*/*z* | Spectral data |
|---|---|---|---|---|
| 1 | 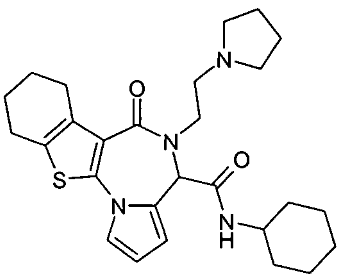 | 481.2632 | 481.2633 | $^1$HNMR (400MHz, DMSO+$CCl_4$): δ 0.75-3.45 (m, 30 H, cyclohexane, pyrrolidine, tetrahydrobenzothiophene, $2CH_2$), 4.37-4.52 (t, *J* = 3.1 Hz, 1 H, CH), 5.15 (s, 1 H, CH), 6.17 (s, 2 H, ArH), 6.8 (s, 1 H, ArH), 8.15-8.3 (br s, 1 H, NH) |

(continued)

| № | Formula | Mol. weight | HRMS *m/z* | Spectral data |
|---|---|---|---|---|
| 2 | 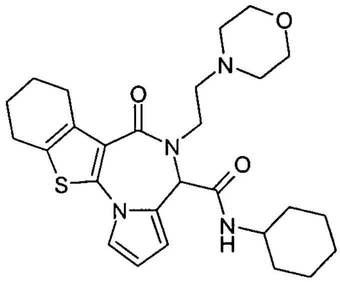 | 497.2581 | 497.2576 | $^1$HNMR (400MHz, DMSO+$CCl_4$): δ 0.85-3.77 (m, 30 H, cyclohexane, morpholine, tetrahydrobenzothiophene, $2CH_2$), 4.2-4.4 (t, *J*= 3.4 Hz, 1 H, CH), 5.2 (s, 1 H, CH), 6.19 (s, 2 H, ArH), 6.8 (s, 1 H, ArH), 7.1-7.2 (br s, 1 H, NH) |
| 3 | 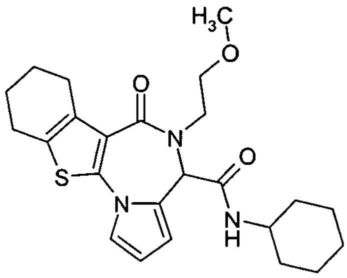 | 442.2159 | 442.2152 | $^1$HNMR (400MHz, DMSO+$CCl_4$): δ 0.76-3.73 (m, 22 H, cyclohexane, tetrahydrobenzothiophene, $2CH_2$), 3.33 (s, 3 H, $CH_3$), 4.13 (s, 1 H, CH), 5.17 (s, 1 H, CH), 6.18 (s, 2 H, ArH), 6.84 (s, 1 H, ArH), 6.5-6.69 (br s, 1 H, NH) |
| 4 | 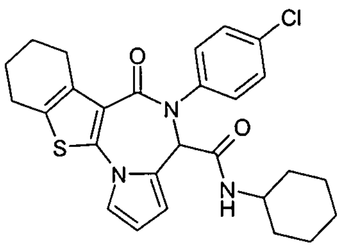 | 494.1663 | 494.1661 | $^1$HNMR (400MHz, DMSO+$CCl_4$): δ 0.8-3.0 (m, 18 H, cyclohexane, tetrahydrobenzothiophene), 3.31-3.49 (s, 1 H, CH), 5.35 (s, 1 H, CH), 6.8-6.0 (br s, 1 H, NH), 6.23 (s, 2 H, ArH), 6.91 (s, 1 H, ArH), 7.2-7.4 (m, 4 H, ArH) |
| 5 | 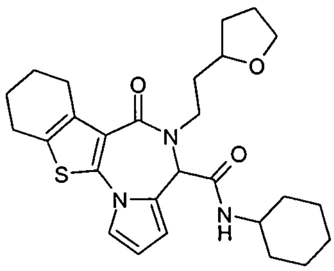 | 482.2315 | 482.2474 | $^1$HNMR (400MHz, DMSO+$CCl_4$): δ 1.0-3.2 (m, 24 H, cyclohexane, tetrahydrofuran, tetrahydrobenzothiophene), 3.5-3.6 (m, 1 H, CH), 3.7-4.0 (m, 2 H, $CH_2$), 4.2-4.5 (q, *J*= 1.8 Hz, 1 H, CH), 5.26 (s, 1 H, NH), 6.15 (s, 2 H, ArH), 6.87 (d, *J*= 7.7 Hz, 1 H, ArH), 7.3 (br s, 1 H, NH) |
| 6 | 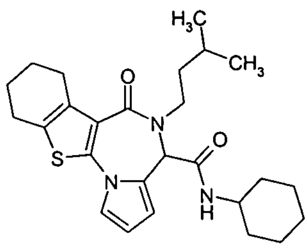 | 458.2523 | 458.2521 | $^1$HNMR (400MHz, DMSO+$CCl_4$): δ 0.6-2.0 (m, 18 H, cyclohexane, tetrahydrobenzothiophene), 1.5 (s, 6 H, $2CH_3$), 2.3-3.0 (m, 4 H, $2CH_2$), 3.3-3.47 (m, 1 H, CH), 5.07 (s, 1 H, CH), 5.9-6.0 (m, 1 H, NH), 6.23 (d, *J* = 7.8 Hz, 2 H, ArH), 6.88 (s, 1 H, ArH) |

(continued)

| № | Formula | Mol. weight | HRMS *m/z* | Spectral data |
|---|---|---|---|---|
| 7 | 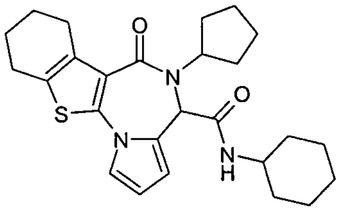 | 466.2366 | 466.2513 | $^1$HNMR (400MHz, DMSO+$CCl_4$): δ 1.1-2.0 (m, 26 H, cyclohexane, cyclopentane, tetrahydrobenzothiophene), 2.8-3.0 (m, 1 H, CH), 3.47-3.59 (m, 1 H, CH), 5.1 (s, 1 H, CH), 5.7-5.9 (br s, 1 H, NH), 6.16-6.3 (d, *J* = 7.6 Hz, 2 H, ArH), 6.9 (s, 1 H, ArH) |
| 8 | 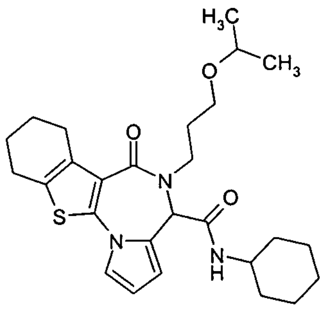 | 498.2628 | 498.2763 | $^1$HNMR (400MHz, DMSO+$CCl_4$): δ 1.1 (2s, 3 H, $2CH_3$), 1.04-2.1 (m, 24 H, cyclohexane, tetrahydrobenzothiophene), 2.65-2.7 (m, 2 H, $CH_2$), 3.25-3.32 (m, 2 H, $CH_2$), 3.39-3.58 (m, 2 H, $CH_2$), 3.5-3.61 (m, 1 H, CH), 3.62-3.73 (m, 1 H, CH), 5.12 (s, 1 H, CH), 5.9-6.05 (br s, 1 H, NH), 6.21-6.3 (d, *J* = 7.9 Hz, 2 H, ArH), 6.8 (s, 1 H, ArH) |

| | Table1.05(1) | | |
|---|---|---|---|
| № | Formula | Mol.w. | LC MS, m/z (M+1), 1H NMR |
| 1 | 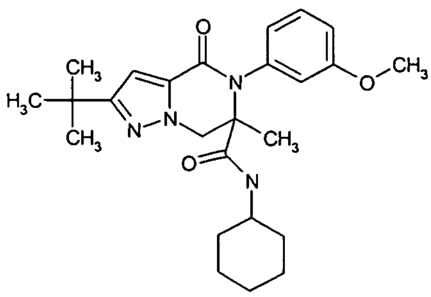 | 438,5747 | LC MS, m/z: 439 (M+1). 1H NMR: 1.00-1.75 (m, 24H, Alk), 3.46-3.58 (br s, 1H, NHCH), 3.81 (s, 3H, OMe), 4.29-4.35, 4.93-5.00 (dd, 2H, NCH2, J=13.9), 6.52 (s, 1H, CH(pyrazol)), 6.83-6.90 (m, 2H, 2CH(PhOMe)), 6.96 (s, 1H, CH(PhOMe)), 7.23-7.30 (m, 2H, 2CH(PhOMe)), 7.55-7.61 (m, 1H, NH) |
| 2 | 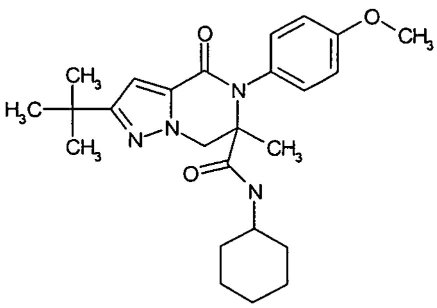 | 438,5747 | LC MS, m/z: 439 (M+1). 1 H NMR: 1.00-1.76 (m, 24H, Alk), 3.45-3.55 (m, 1H, NHCH), 3.82 (s, OMe), 4.28-4.34, 4.93-5.00 (dd, 2H, NCH2, J=13.9), 6.5 (s, 1H, CH(pyrazol)), 6.85-6.90, 7.22-7.27 (dd, 4H, 4CH(p-PhOMe), J=9.3)), 7.53-7.58 (m, 1H, NH) |

(continued)

| | Table1.05(1) | | |
|---|---|---|---|
| № | Formula | Mol.w. | LC MS, m/z (M+1), 1H NMR |
| 3 | 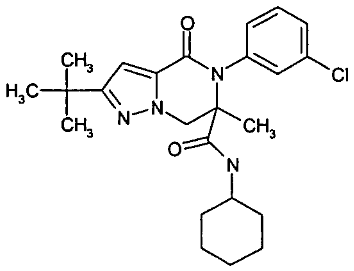 | 442,9933 | 443 |
| 4 | 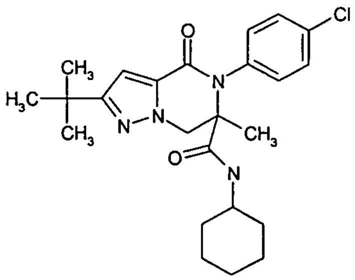 | 442,9933 | 443 |
| 5 | 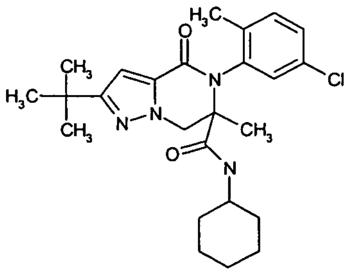 | 457,0204 | 458 |
| 6 | 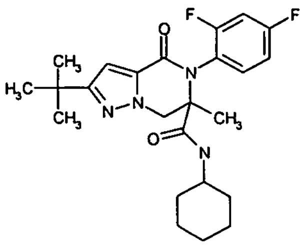 | 444,5291 | 445 |
| 7 | 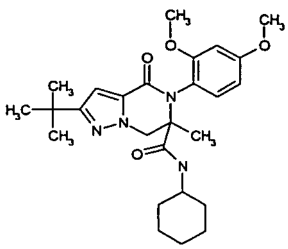 | 468,6012 | 469 |

(continued)

| | Table1.05(1) | | |
|---|---|---|---|
| № | Formula | Mol.w. | LC MS, m/z (M+1), 1H NMR |
| 8 | 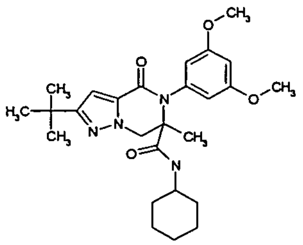 | 468,6012 | 469 |
| 9 | 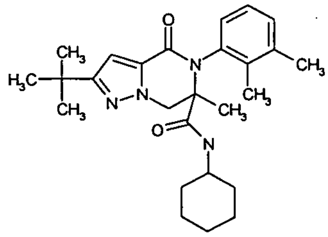 | 436,6024 | 437 |
| 10 | 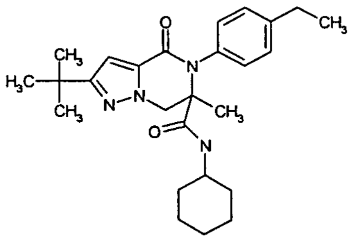 | 436,6024 | 437 |
| 11 | 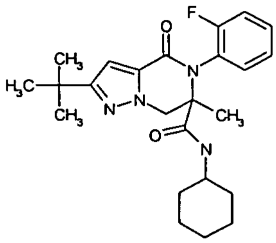 | 426,5387 | 427 |
| 12 | 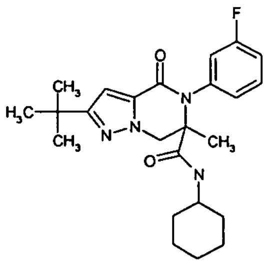 | 426,5387 | 427 |
| 13 | 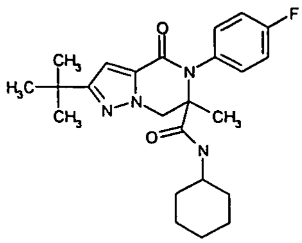 | 426,5387 | 427 |

(continued)

| | Table1.05(1) | | |
|---|---|---|---|
| № | Formula | Mol.w. | LC MS, m/z (M+1), 1H NMR |
| 14 | 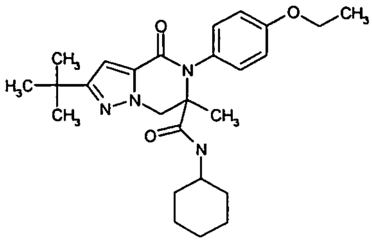 | 452,6018 | 453 |
| 15 | 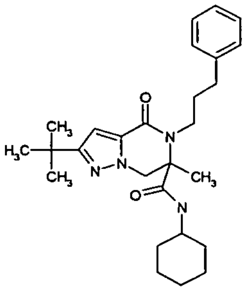 | 450,6295 | 451 |
| 16 | 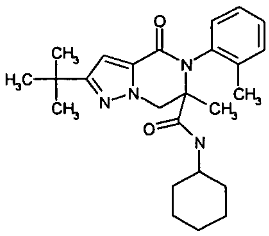 | 422,5753 | 423 |
| 17 | 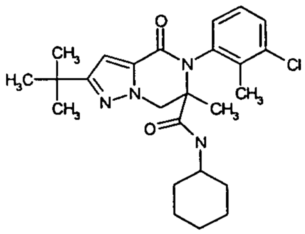 | 457,0204 | 458 |
| 18 | 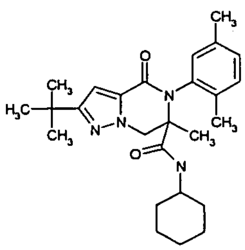 | 436,6024 | 437 |
| 19 | 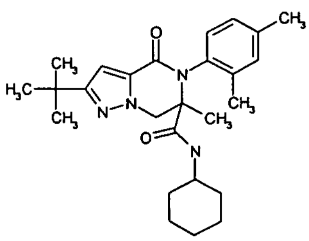 | 436,6024 | 437 |

(continued)

| | Table1.05(1) | | |
|---|---|---|---|
| № | Formula | Mol.w. | LC MS, m/z (M+1), 1H NMR |
| 20 | 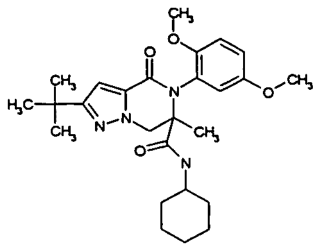 | 468,6012 | 469 |
| 21 | 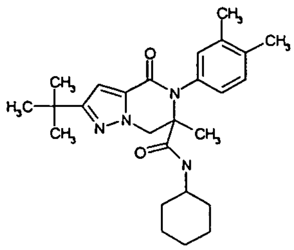 | 436,6024 | 437 |
| 22 | 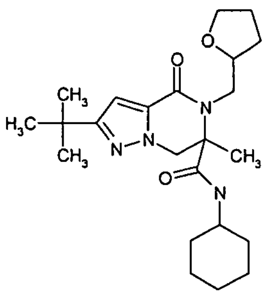 | 416,5684 | 417 |
| 23 | 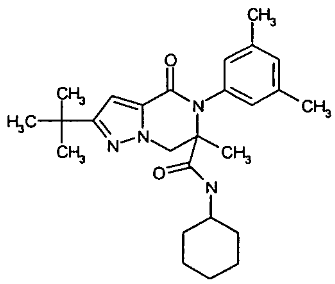 | 436,6024 | 437 |
| 24 | 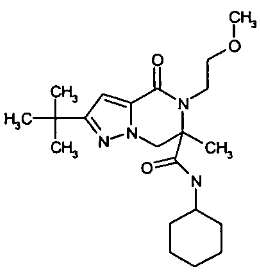 | 390,5301 | |

(continued)

| | Table1.05(1) | | |
|---|---|---|---|
| № | Formula | Mol.w. | LC MS, m/z (M+1), 1H NMR |
| 25 | 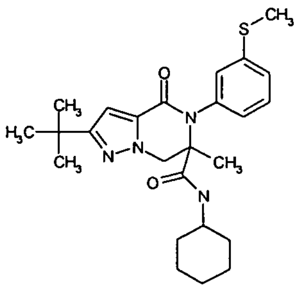 | 454,6393 | 455 |
| 26 | 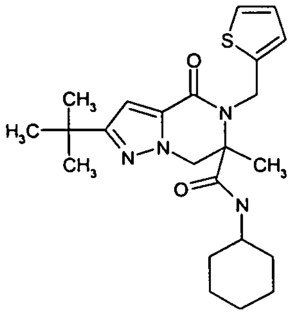 | 428,6011 | 429 |
| 27 | 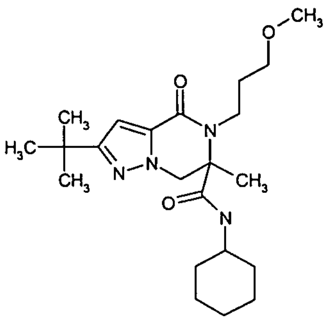 | 404,5572 | LC MS, m/z: 405 (M+1). 1 H NMR: 1.03-1.32 (m, 14H, Alk), 1.50-1.86 (m, 10H, Alk), 3.18-3.26 (m, 2H, Alk), 3.27 (s, 3H, OMe), 3.33-3.40 (m, 2H, Alk), 3.41-3.60 (m, 2H, Alk), 4.01-4.08, 4.71-4.77 (dd,2H, NCH2, J=3.17), 6.45 (s, 1H, CH (pyrazol)), 7.42-7.49 (m, 1H, NH) |
| 28 | 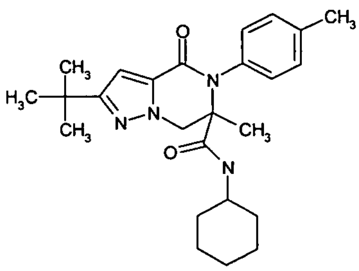 | 422,5753 | 423 |
| 29 | 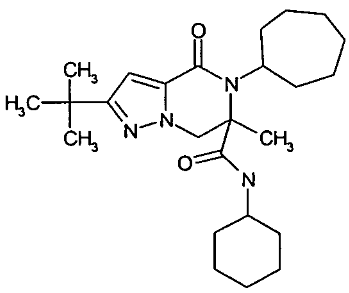 | 428,6232 | 429 |

(continued)

| | Table1.05(1) | | |
|---|---|---|---|
| № | Formula | Mol.w. | LC MS, m/z (M+1), 1H NMR |
| 30 | 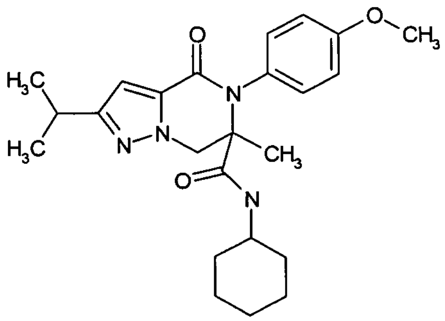 | 424,5476 | LC MS, m/z: 425 (M+1). 1H NMR: 1.00-1.74 (m, 24H, Alk), 3.43-3.52 (m, 1 H, NHCH), 3.8 (s, 3H, OMe), 4.25-4.32, 4.87-4.94 (dd, 2H, NCH2, J=13.8), 6.44 (s, 1H, CH(pyrazol)), 6.82-6.89, 7.19-7.25 (dd,2H, (p-OMePh)), J=8), 7.53-7.59 (m, 1H, NH) |
| 31 | 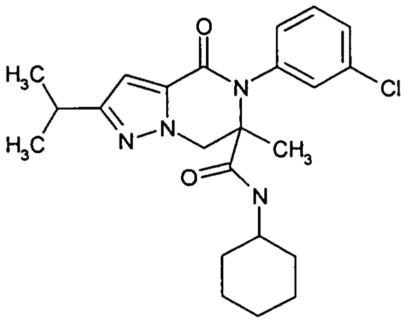 | 428,9662 | 429 |
| 32 | 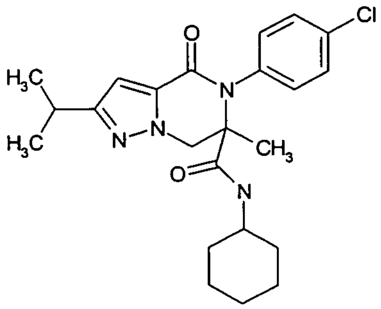 | 428,9662 | LC MS, m/z: 429 (M+1). 1H NMR: 1.00-1.78 (m, 22H, Alk), 3.43-3.55 (m, 1H, NHCH), 4.31-4.37, 4.91-4.98 (dd, 2H, NCH2, J=12,7), 6.48 (s, 1H, CH(pyrazol)), 7.30-7.45 (m, 4H, Ar), 7.63-7.69 (m, 1H, NH) |
| 33 | 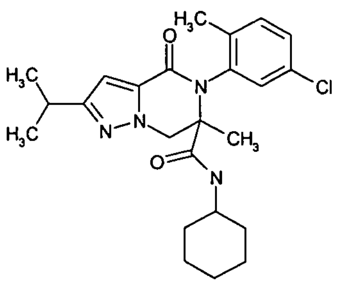 | 442,9933 | 443 |
| 34 | 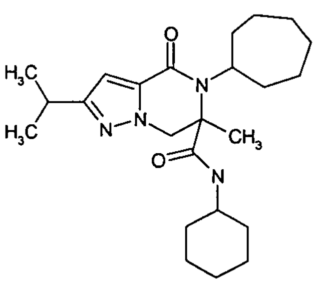 | 414,5961 | 415 |

(continued)

| | Table1.05(1) | | |
|---|---|---|---|
| № | Formula | Mol.w. | LC MS, m/z (M+1), 1H NMR |
| 35 | 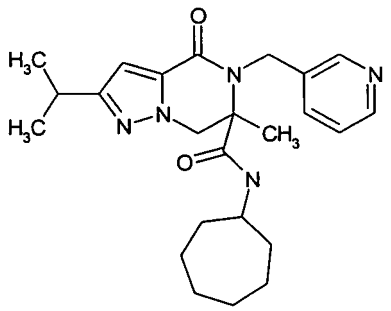 | 423,5629 | 424 |
| 36 | 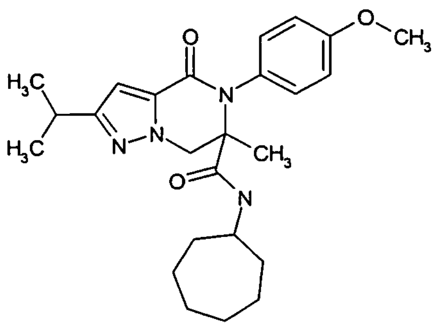 | 438,5747 | LC MS, m/z: 439 (M+1). 1 H NMR: 1.00-1.75 (m, 25H, Alk), 2.86-3.00 (m, 9H, (H2O+Alk)), 3.59-3.70 (m, 1H, NHCH), 3.8 (s, 3H, OMe), 4.24-4.32, 4.87-4.95 (dd,2H, NHCH,J=13.7), 6.44 (s,1 H, CH(pyrazol)), 6.83-6.89, 7.19-7.26 (dd,4H, p-OMePh, J=9), 7.58-7.65 (m, 1H, NH) |
| 37 | 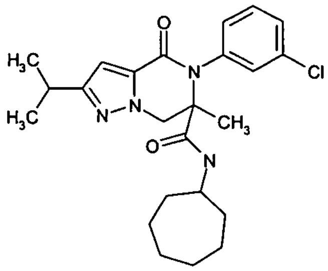 | 442,9933 | 443 |
| 38 | 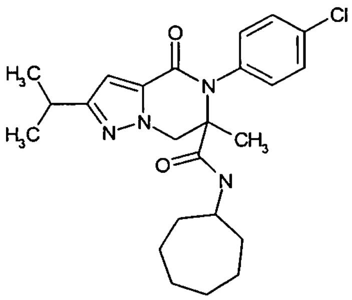 | 442,9933 | 443 |
| 39 | 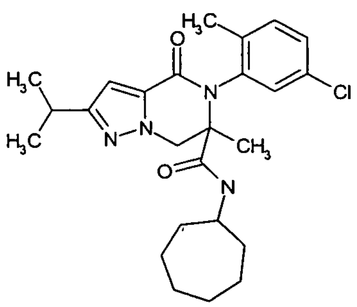 | 457,0204 | 458 |

(continued)

| | Table1.05(1) | | |
|---|---|---|---|
| № | Formula | Mol.w. | LC MS, m/z (M+1), 1H NMR |
| 40 | 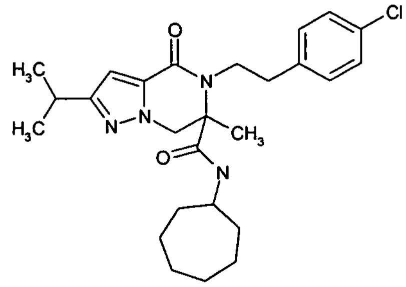 | 471,0475 | 472 |
| 41 | 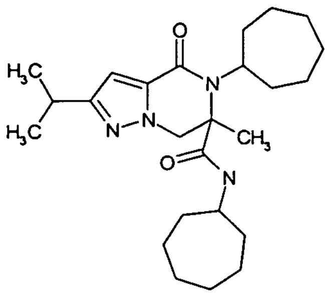 | 428,6232 | LC MS, m/z: 429 (M+1). 1 H NMR: .18-2.00 (m, 36H, Alk), 2.3-2.47 (m, 2H, Alk), 2.79-2.99 (m, 10H, (H2O+Alk)), 3.76-3.89 (m, 1H, CHNH), 3.95-4.03, 4.55-4.62 (dd, 2H, NCH2, J=13.3), 6.44 (s, 1H, CH(pyrazol)), 7.94-8.00 (m, 1H, NH) |
| 42 | 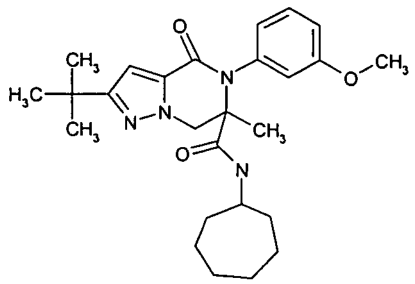 | 452,6018 | 453 |
| 43 | 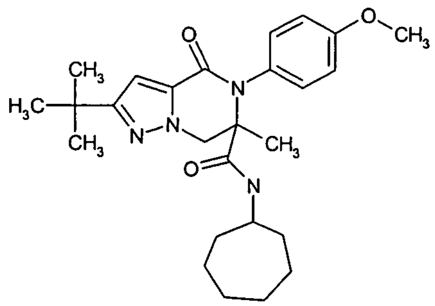 | 452,6018 | LC MS, m/z: 453 (M+1). 1 H NMR: 1.10-1.76 (m, 32H, Alk), 3.62-3.73 (m, 1H, NHCH), 3.83 (s,3H, OMe), 4.28-4.35, 4.93-5.00 (dd,2H, NCH2, J=13.4), 6.49 (s,1H, CH(pyrazol)), 6.86-6.91, 7.22-7.28 (dd, 4H, Ar, J=9.2), 7.62-7.67 (m, 1H, NH) |
| 44 | 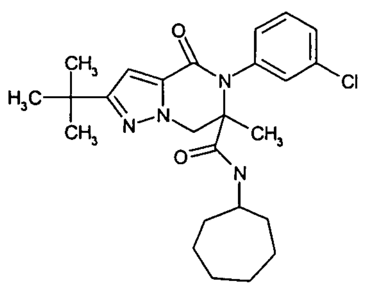 | 457,0204 | LC MS, m/z: 458 (M+1). 1H NMR: 1.53-1.80 (m, 31 H, Alk), 3.63-3.71 (m, 1H, NHCH), 4.35-4.41, 4.97-5.04 (dd, 2H, NCH2, J=13.1), 6.53 (s, 1H, CH(pyrazol)), 7.3-7.5 (m, 4H, ClPh), 7.6-7.81 (m, 1H, NH) |

(continued)

| | Table1.05(1) | | |
|---|---|---|---|
| № | Formula | Mol.w. | LC MS, m/z (M+1), 1H NMR |
| 45 | 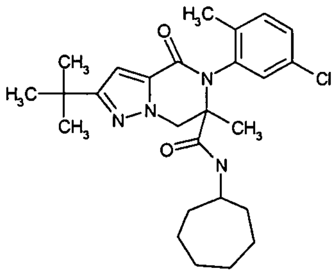 | 471,0475 | 472 |
| 46 | 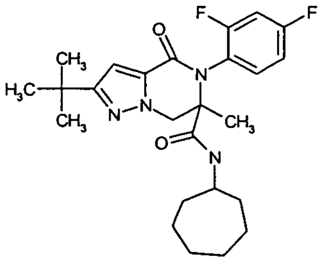 | 458,5562 | 459 |
| 47 | 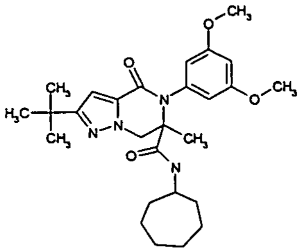 | 482,6283 | 483 |
| 48 | 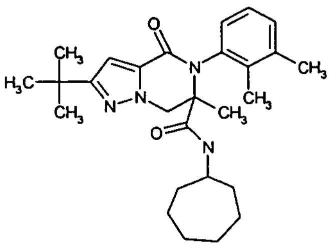 | 450,6295 | 451 |
| 49 | 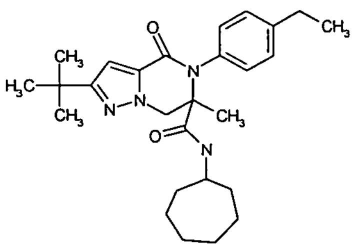 | 450,6295 | 451 |

(continued)

| | Table1.05(1) | | |
|---|---|---|---|
| № | Formula | Mol.w. | LC MS, m/z (M+1), 1H NMR |
| 50 | 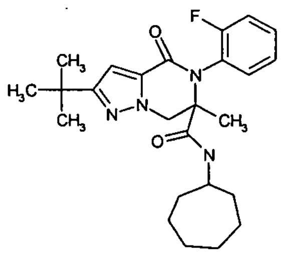 | 440,5658 | 441 |
| 51 | 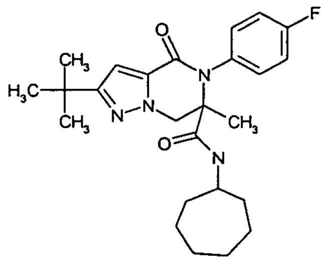 | 440,5658 | 441 |
| 52 | 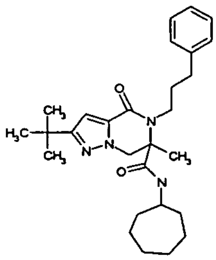 | 464,6566 | 465 |
| 53 | 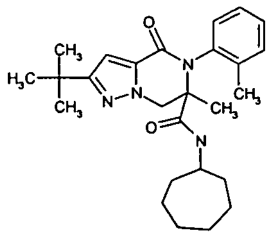 | 436,6024 | 437 |
| 54 | 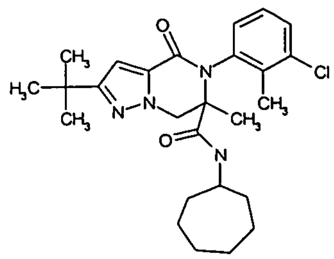 | 471,0475 | 472 |

(continued)

| | Table1.05(1) | | |
|---|---|---|---|
| № | Formula | Mol.w. | LC MS, m/z (M+1), 1H NMR |
| 55 | 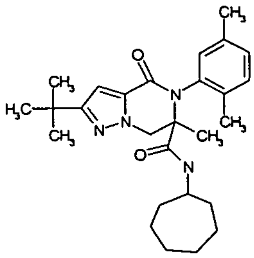 | 450,6295 | 451 |
| 56 | 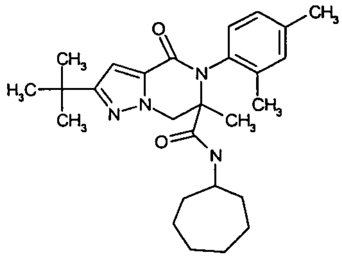 | 450,6295 | 451 |
| 57 | 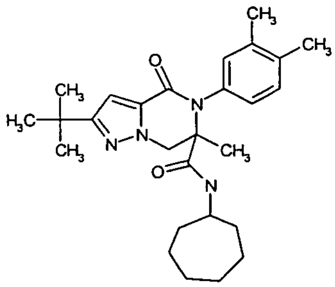 | 450,6295 | 451 |
| 58 | 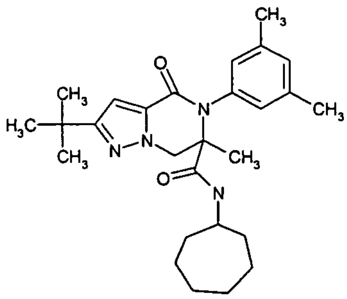 | 450,6295 | 451 |
| 59 | 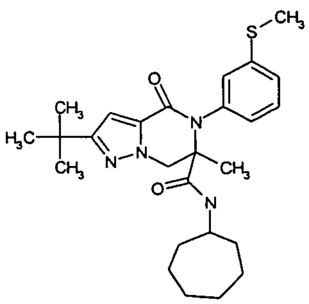 | 468,6664 | 469 |

(continued)

| | Table1.05(1) | | |
|---|---|---|---|
| № | Formula | Mol.w. | LC MS, m/z (M+1), 1H NMR |
| 60 | 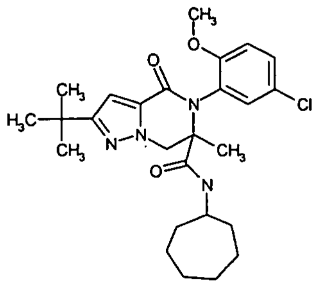 | 487,0469 | 488 |
| 61 | 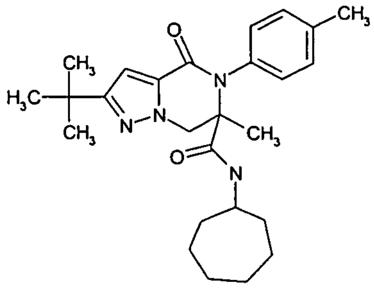 | 436,6024 | 437 |
| 62 | 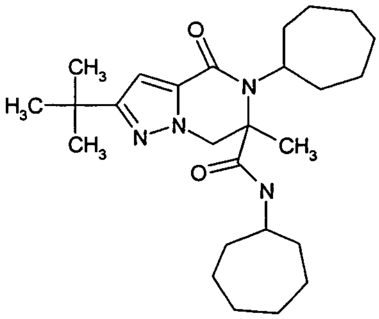 | 442,6502 | LC MS, m/z: 443 (M+1). 1 H NMR: 1.20-2.00 (m, 35H, Alk), 2.30-2.46 (m, 3H, Alk), 2.79-2.89 (m, 2H, Alk), 3.77-3.86 (m, 1H, NHCH), 3.95-4.01, 4.55-4.62 (dd,2H, CH2N, J=13.3), 6.46 (s, 1 H, CH(pyrazol)), 7.91-7.97 (m, 1H, NH) |
| 63 | 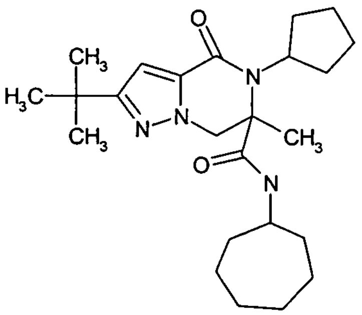 | 414,5961 | LC MS, m/z: 415 (M+1). 1 H NMR: 1.08-1.96 (m, 36H, Alk), 2.01-2.12 (m, 1H, Alk), 2.24-2.32 (m, 1H, Alk), 3.20-3.31 (m, 1H, Alk), 3.75-3.86 (m, 1H, NHCH), 3.98-4.05, 4.51-4.58 (dd, 2H, NCH2, J=12.3), 6.48 (s, 1H, pyrazol), 7.91-7.98 (m, 1H, NH) |
| 64 | 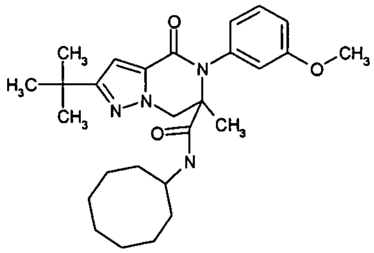 | 466,6289 | 467 |

(continued)

| | Table1.05(1) | | |
|---|---|---|---|
| № | Formula | Mol.w. | LC MS, m/z (M+1), 1H NMR |
| 65 | 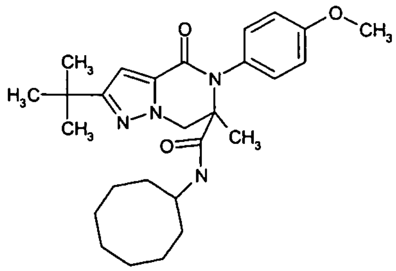 | 466,6289 | 467 |
| 66 | 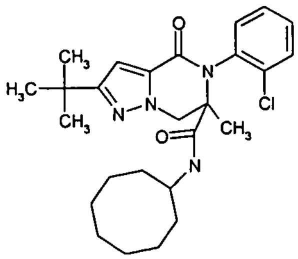 | 471,0475 | 472 |
| 67 | 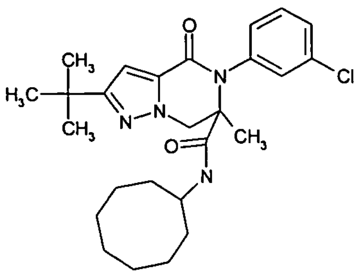 | 471,0475 | 472 |
| 68 | 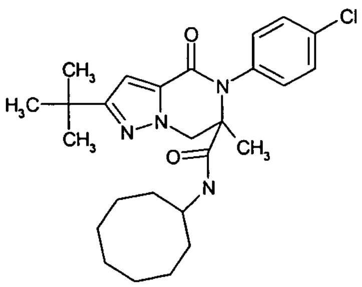 | 471,0475 | LC MS, m/z: 472 (M+1). 1H NMR: 1.14-1.69 (m, 30H, Alk), 3.69-3.79 (m, 1 H, Alk), 4.32-4.38, 4.98-5.04 (dd, 2H, NCH2, J=13.6), 6.25 (s, 1H, CH(pyrazol)), 7.34-7.41 (m, 4H, p-ClPh), 7.66-7.72 (m, 1H, NH) |
| 69 | 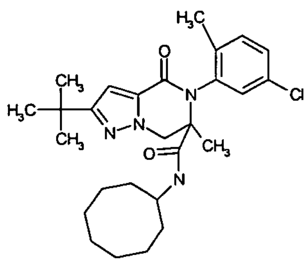 | 485,0745 | 486 |

(continued)

| | Table1.05(1) | | |
|---|---|---|---|
| Nº | Formula | Mol.w. | LC MS, m/z (M+1), 1H NMR |
| 70 | 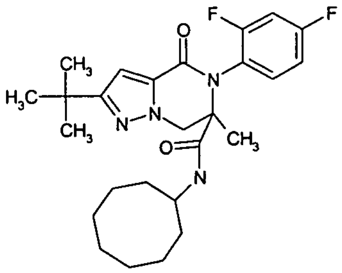 | 472,5833 | 473 |
| 71 | 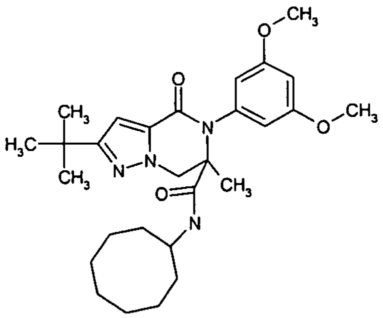 | 496,6554 | 497 |
| 72 | 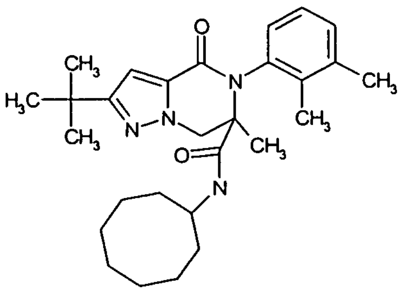 | 464,6566 | 465 |
| 73 | 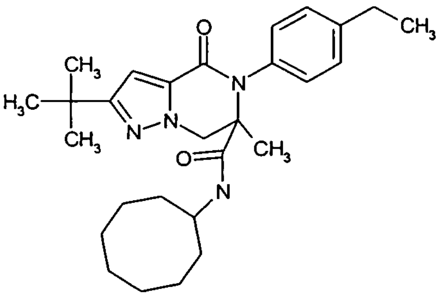 | 464,6566 | LC MS, m/z: 465 (M+1). 1H NMR: 1.23-1.70 (m, 33H, Alk), 2.66-2.74 (q, 2H, p-EtPh, J=7.2), 3.70-3.79 (m, 1H, NHCH), 4.29-4.38, 4.96-5.03 (dd, 2H, NCH2, J=14.5), 6.53 (s, 1H, CH (pyrazol)), 7.18-7.23, 7.23-7.28 (dd, 4H, Ar, J=7.2), 7.60 7.69 (m, 1H, NH) |
| 74 | 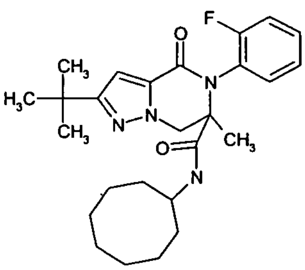 | 454,5929 | 455 |

(continued)

| | Table1.05(1) | | |
|---|---|---|---|
| № | Formula | Mol.w. | LC MS, m/z (M+1), 1H NMR |
| 75 | 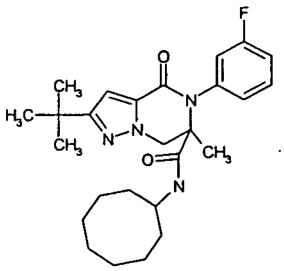 | 454,5929 | 455 |
| 76 | 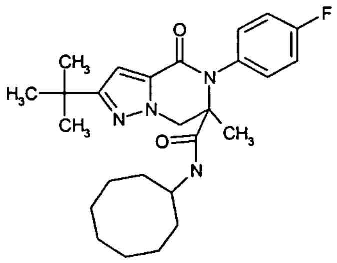 | 454,5929 | 455 |
| 77 | 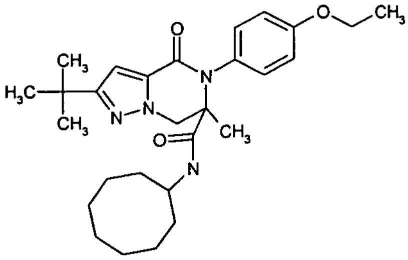 | 480,656 | 481 |
| 78 | 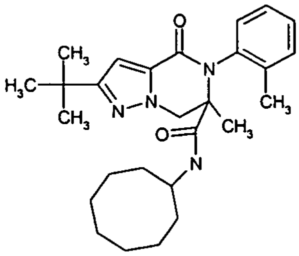 | 450,6295 | 451 |
| 79 | 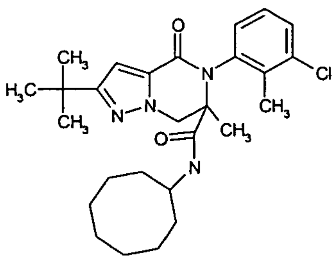 | 485,0745 | 486 |

(continued)

| | Table1.05(1) | | |
|---|---|---|---|
| № | Formula | Mol.w. | LC MS, m/z (M+1), 1H NMR |
| 80 | 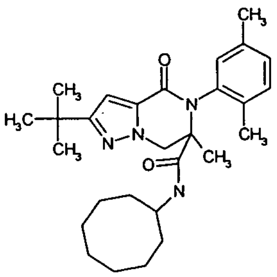 | 464,6566 | 465 |
| 81 | 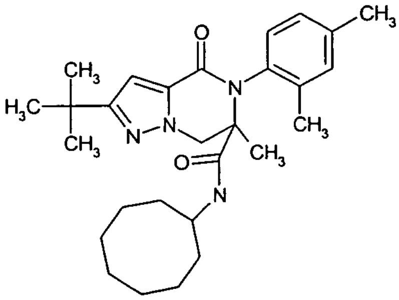 | 464,6566 | 465 |
| 82 | 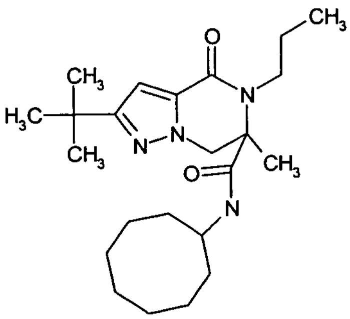 | 402,5849 | LC MS, m/z: 403 (M+1). 1 H NMR: 1.20-163 (m, 30H, Alk), 1.78-1.87 (m, 2H, Alk), 3.21-3.28 (m, 1H, Alk), 3.30 (s, 3H, CH3oMe), 3.36-3.42 (m, 2H, Alk), 3.53-3.63 (m, 1H, Alk), 3.70-3.79 (m, 1H, CHNH), 4.03-4.1, 4.75-4.82 (dd, 2H, NCH2, J=13.5), 6.47 (s, 1H, CH(pyrazol)), 7.41-7.49 (m, 1H, |
| 83 | 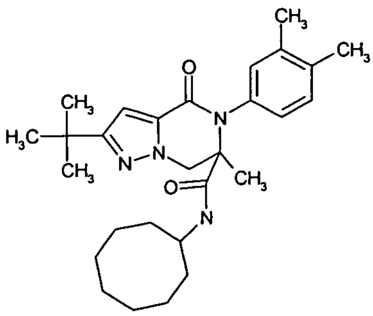 | 464,6566 | 465 |
| 84 | 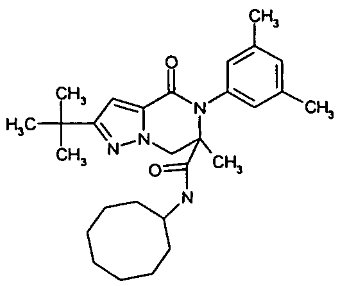 | 464,6566 | 465 |

(continued)

| | Table1.05(1) | | |
|---|---|---|---|
| № | Formula | Mol.w. | LC MS, m/z (M+1), 1H NMR |
| 85 | 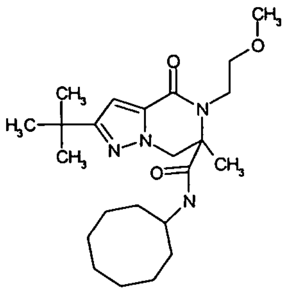 | 418,5843 | LC MS, m/z: 419 (M+1). 1H NMR: 1.21-1.64 (m, 28H, Alk), 3.31 (s, 3H, OMe), 3.42-3.73 (m, 5H, Alk), 4.01-4.08, 4.75-4.82 (dd,2H, NCH2, J=12.6), 6.46 (s, 1 H, CH(pyrazol)), 7.43-7.49 (m, 1H, NH) |
| 86 | 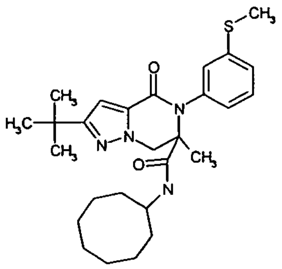 | 482,6935 | 483 |
| 87 | 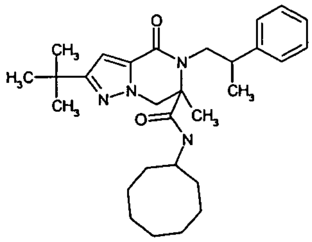 | 478,6837 | 479 |
| 88 | 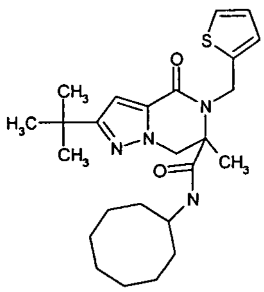 | 456,6553 | 457 |
| 89 | 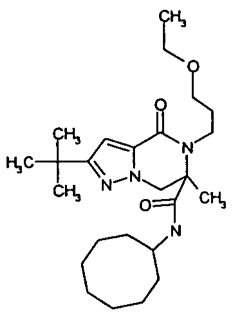 | 446,6385 | 447 |

(continued)

| | Table1.05(1) | | |
|---|---|---|---|
| № | Formula | Mol.w. | LC MS, m/z (M+1), 1H NMR |
| 90 | 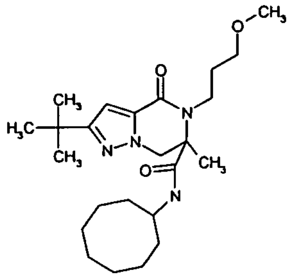 | 432,6114 | 433 |
| 91 | 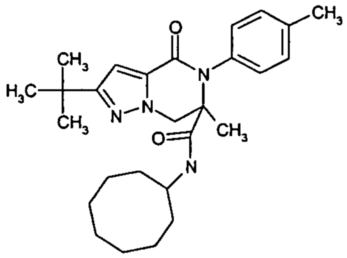 | 450,6295 | 451 |
| 92 | 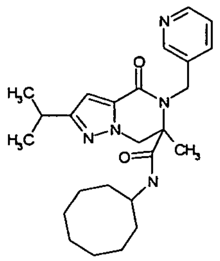 | 437,59 | 438 |
| 93 | 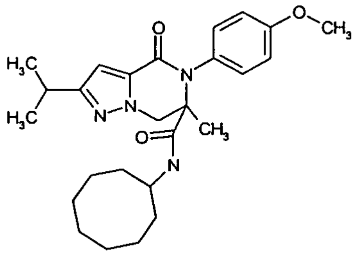 | 452,6018 | 453 |
| 94 | 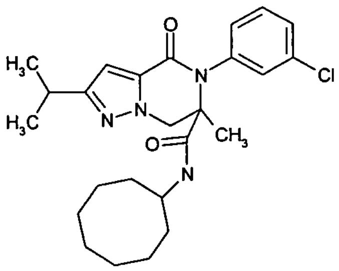 | 457,0204 | 458 |

(continued)

| | Table1.05(1) | | |
|---|---|---|---|
| № | Formula | Mol.w. | LC MS, m/z (M+1), 1H NMR |
| 95 | 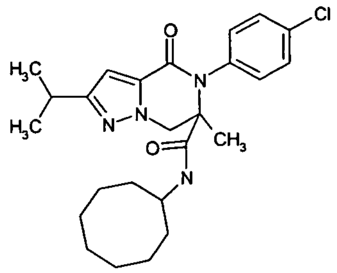 | 457,0204 | 458 |
| 96 | 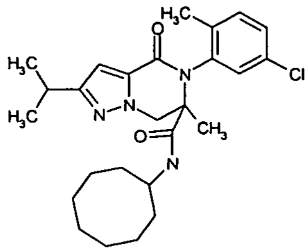 | 471,0475 | 472 |
| 97 | 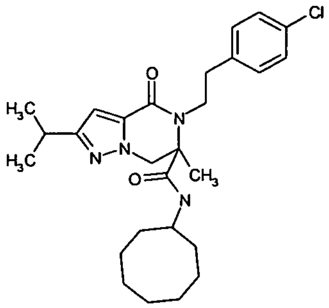 | 485,0745 | 486 |
| 98 | 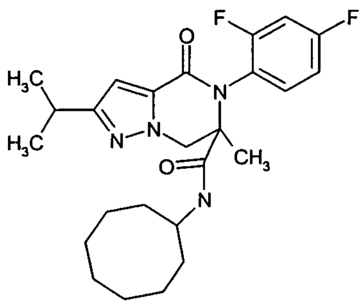 | 458,5562 | LC MS, m/z: 459 (M+1). 1 H NMR: 1.16-1.68 (m, 28H, Alk), 3.67-3.77 (m, 1 H, NHCH), 4.24-4.31 , 5.02-5.10 (dd, 2H, NCH2, J=13.6), 6.5 (s, 1H, pyrazol), 6.94-7.06 (m, 2H, 2,4-F2Ph), 7.68-7.82 (m, 2H, NH, CH(2,4-F2Ph) |
| 99 | 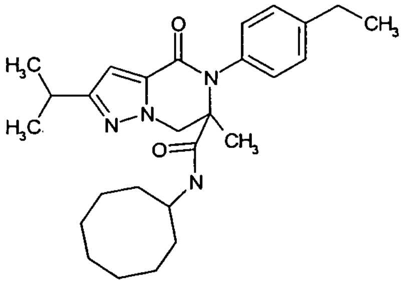 | 450,6295 | LC MS, m/z: 451 (M+1). 1 H NMR: 1.66 (m, 30H, Alk), 2.63-2.72 (q, 2H, CH2(p-EtPh), J=7.9)), 2.87-2.97 (m, 4H, (H2O+Alk)), 3.68-3.78 (m, 1H, NHCH), 4.24-4.32, 4.89-4.97 (dd, 2H, NCH2, J=13.8), 6.46 (s, 1H, CH(pyrazol)), 7.13-7.19, 7.19-7.25 (dd, 4H,, 4CH(p- |

(continued)

| | Table1.05(1) | | |
|---|---|---|---|
| № | Formula | Mol.w. | LC MS, m/z (M+1), 1H NMR |
| 100 | 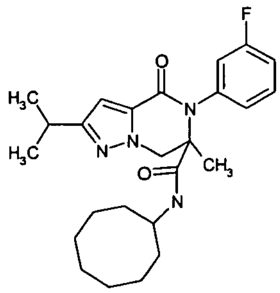 | 440,5658 | 441 |
| 101 | 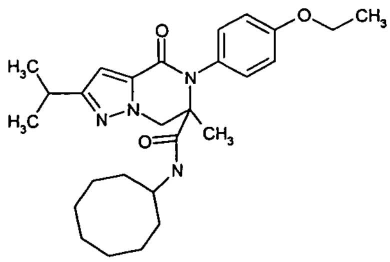 | 466,6289 | 467 |
| 102 | 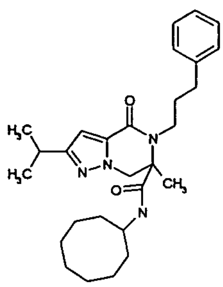 | 464,6566 | 465 |
| 103 | 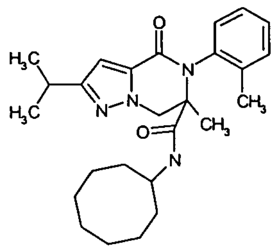 | 436,6024 | 437 |
| 104 | 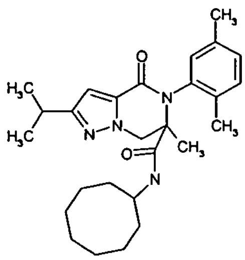 | 450,6295 | 451 |

(continued)

| | Table1.05(1) | | |
|---|---|---|---|
| № | Formula | Mol.w. | LC MS, m/z (M+1), 1H NMR |
| 105 | 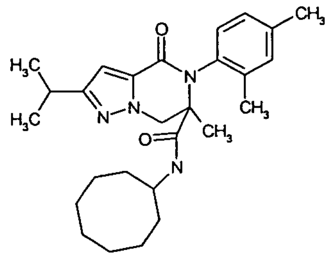 | 450,6295 | 451 |
| 106 | 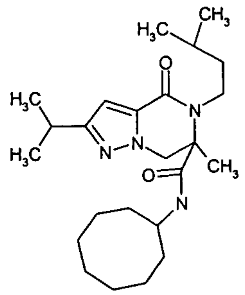 | 416,612 | 417 |
| 107 | 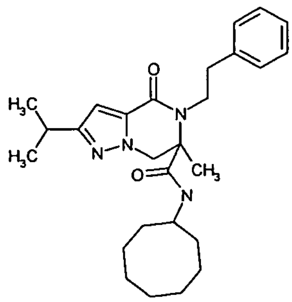 | 450,6295 | 451 |
| 108 | 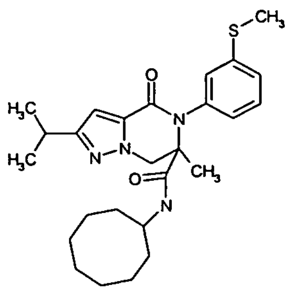 | 468,6664 | 469 |
| 109 | 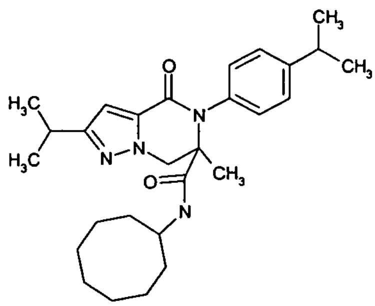 | 464,6566 | 465 |

(continued)

| | Table1.05(1) | | |
|---|---|---|---|
| № | Formula | Mol.w. | LC MS, m/z (M+1), 1H NMR |
| 110 | 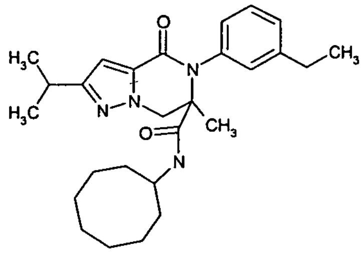 | 450,6295 | 451 |
| 111 | 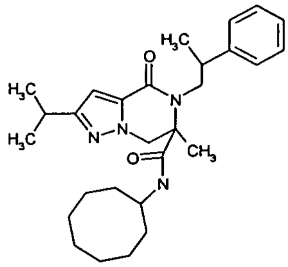 | 464,6566 | 465 |
| 112 | 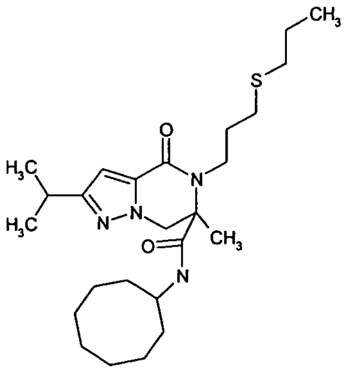 | 462,7031 | 463 |
| 113 | 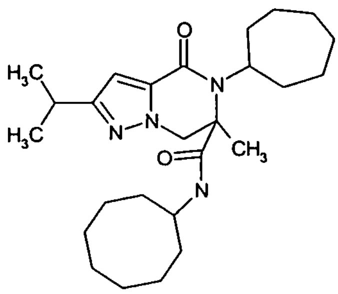 | 442,6502 | LC MS, m/z: 443 (M+1). 1 H NMR: 1.16-1.80 (m, 35H, Alk), 1.88-2.00 (m, 1H, Alk), 2.30-2.47 (m, 2H, Alk), 2.82-3.00 (m, 3H, Alk), 3.80-3.92 (m, 1H, NHCH), 4.00-4.07, 4.56-4.63 (dd, 2H, NCH2, J=13.8), 6.45 (s, 1H, CH(pyrazol)), 7.96-8.04 (m, 1H, NH) |
| 114 | 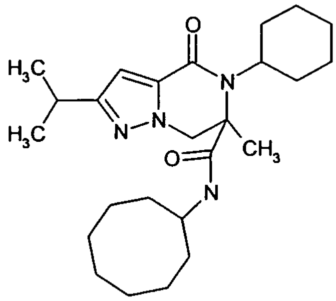 | 428,6232 | LC MS, m/z: 429 (M+1). 1 H NMR: 1.09-1.90 (m, 36H, Alk), 2.38-2.54 (m, 4H, DMSO+Alk), 2.60-2.71 (m, 1H, Alk), 2.85-2.96 (m, 6H, H2O+Alk), 1.84-1.93 (m, 1H, NHCH), 3.94-4.00, 4.54-4.61 (dd, 2H, NCH2, J=12.1), 6.44 (s, 1H, CH(pyrazol)), 7.96-8.04 (m, 1H, NH) |

(continued)

| | Table1.05(1) | | |
|---|---|---|---|
| № | Formula | Mol.w. | LC MS, m/z (M+1), 1H NMR |
| 115 | 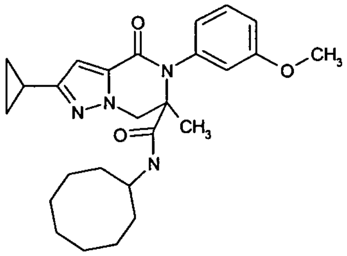 | 450,5859 | 451 |
| 116 | 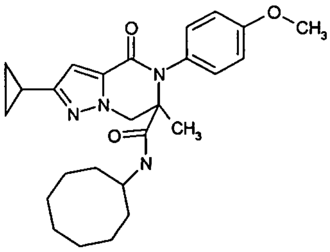 | 450,5859 | 451 |
| 117 | 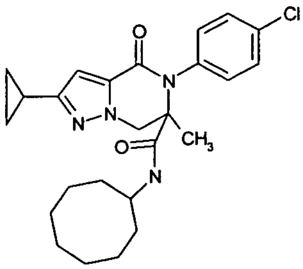 | 455,0044 | 456 |
| 118 | 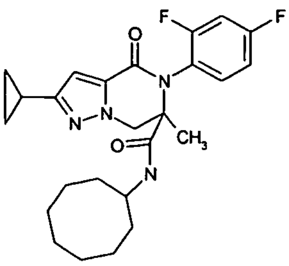 | 456,5403 | 457 |
| 119 | 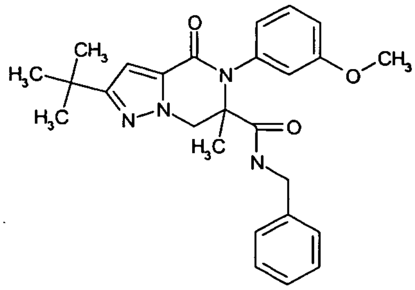 | 446,554 | 447 |

(continued)

| | Table1.05(1) | | |
|---|---|---|---|
| № | Formula | Mol.w. | LC MS, m/z (M+1), 1H NMR |
| 120 | 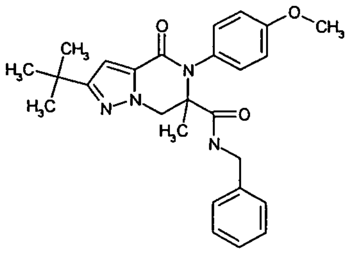 | 446,554 | 447 |
| 121 | 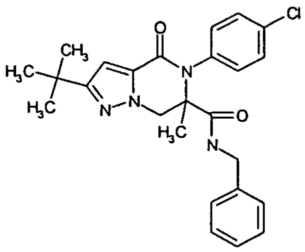 | 450,9725 | 451 |
| 122 | 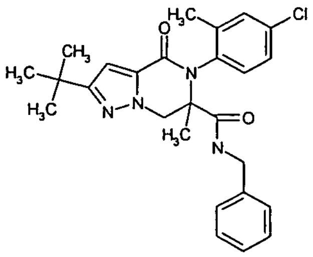 | 464,9996 | 465 |
| 123 | 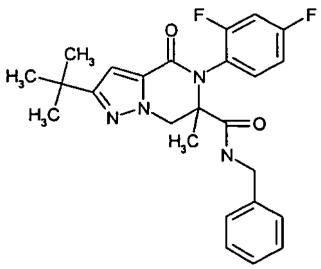 | 452,5084 | 453 |
| 124 | 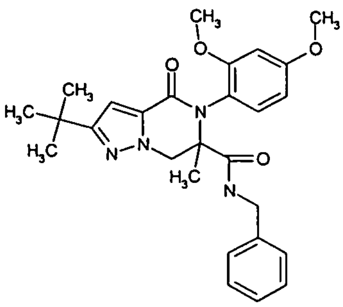 | 476,5805 | 477 |

(continued)

| | Table1.05(1) | | |
|---|---|---|---|
| № | Formula | Mol.w. | LC MS, m/z (M+1), 1H NMR |
| 125 | 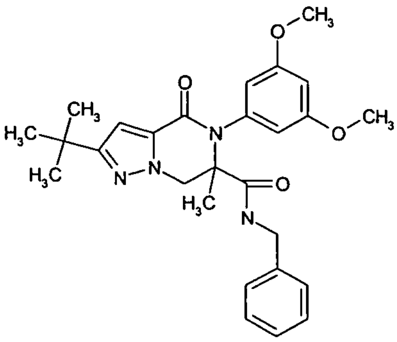 | 476,5805 | 477 |
| 126 | 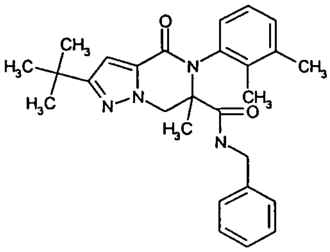 | 444,5817 | 445 |
| 127 | 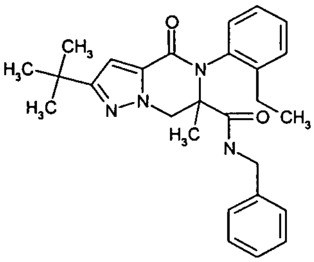 | 444,5817 | 445 |
| 128 | 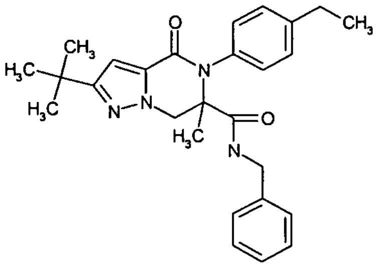 | 444,5817 | 445 |
| 129 | 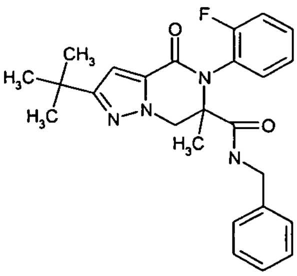 | 434,5179 | 435 |

(continued)

| | Table1.05(1) | | |
|---|---|---|---|
| № | Formula | Mol.w. | LC MS, m/z (M+1), 1H NMR |
| 130 | 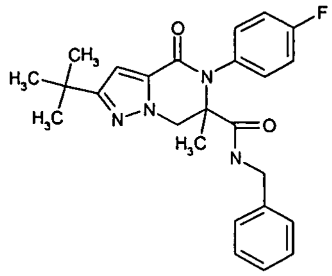 | 434,5179 | 435 |
| 131 | 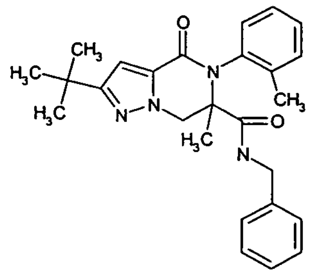 | 430,5546 | 431 |
| 132 | 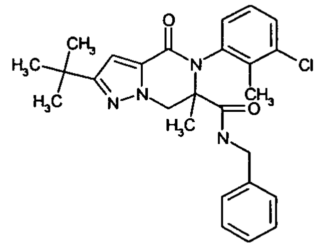 | 464,9996 | 465 |
| 133 | 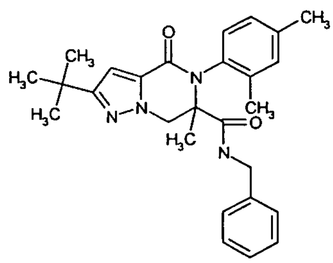 | 444,5817 | 445 |
| 134 | 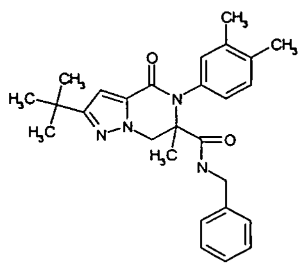 | 444,5817 | 445 |

(continued)

| | Table1.05(1) | | |
|---|---|---|---|
| No | Formula | Mol.w. | LC MS, m/z (M+1), 1H NMR |
| 135 | 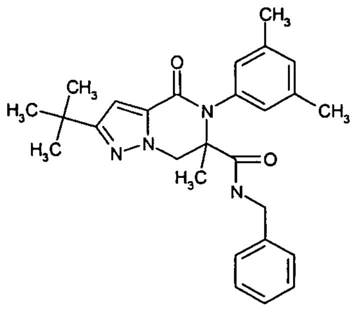 | 444,5817 | 445 |
| 136 | 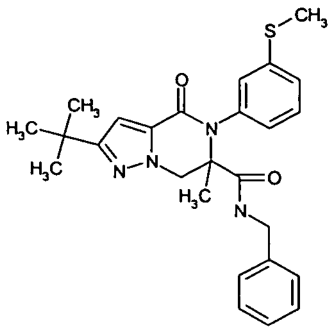 | 462,6186 | 463 |
| 137 | 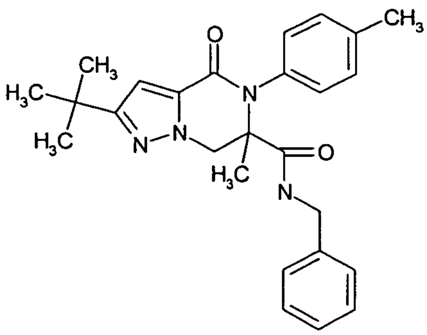 | 430,5546 | 431 |

| | Table 1.05(2) | | |
|---|---|---|---|
| No | Formula | Mol.w. | LC MS, m/z (M+1), 1H NMR |
| 1 | 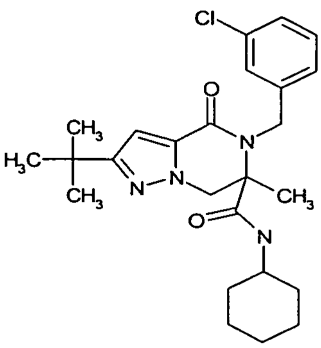 | 457,0204 | 458 |

(continued)

| | Table 1.05(2) | | |
|---|---|---|---|
| № | Formula | Mol.w. | LC MS, m/z (M+1), 1H NMR |
| 2 | 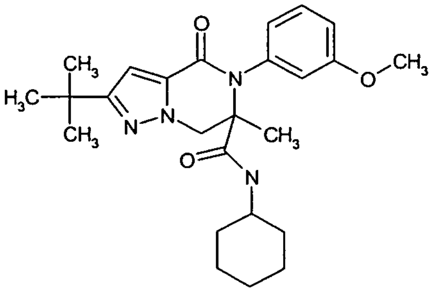 | 438,5747 | LC MS, m/z: 439 (M+1). 1H NMR: 1.00-1.75 (m, 24H, Alk), 3.46-3.58 (br s, 1H, NHCH), 3.81 (s, 3H, OMe), 4.29-4.35, 4.93-5.00 (dd, 2H, NCH2, J=13.9), 6.52 (s, 1H, CH(pyrazol)), 6.83-6.90 (m, 2H, 2CH(PhOMe)), 6.96 (s, 1 H, CH(PhOMe)), 7.23-7.30 (m, 2H, 2CH(PhOMe)), 7.55-7.61 (m, 1H, NH... |
| 3 | 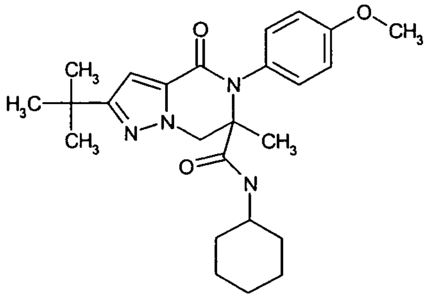 | 438,5747 | LC MS, m/z: 439 (M+1). 1H NMR: 1.00-1.76 (m, 24H, Alk), 3.45-3.55 (m, 1H, NHCH), 3.82 (s, OMe), 4.28-4.34, 4.93-5.00 (dd, 2H, NCH2, J=13.9), 6.5 (s, 1H, CH(pyrazol)), 6.85-6.90, 7.22-7.27 (dd, 4H, 4CH(p-PhOMe), J=9.3)), 7.53-7.58 (m, 1 H, NH) |
| 4 | 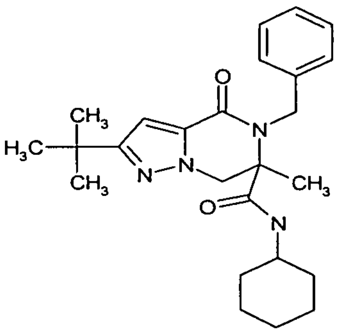 | 422,5753 | 423 |
| 5 | 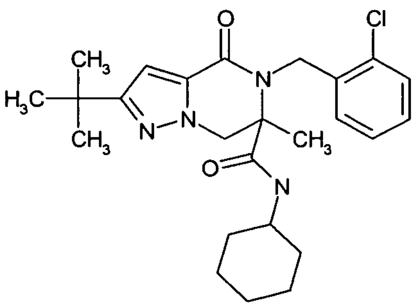 | 457,0204 | 458 |

(continued)

| | Table 1.05(2) | | |
|---|---|---|---|
| № | Formula | Mol.w. | LC MS, m/z (M+1), 1H NMR |
| 6 | 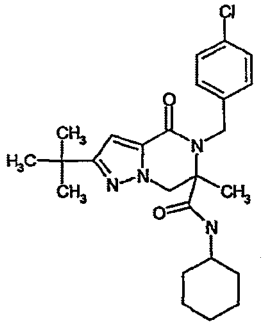 | 457,0204 | LC MS, m/z: 458 (M+1). 1H NMR: 0.93-1.72 (m, 24H, Alk), 3.40-3.50 (m, 1H, NHCH), 4.07-4.11, 5.19-5.28 (dd, 2H, NCH2, J=12.9), 4.11-4.16, 4.84-4.9 (dd,2H, CH2Ph, J=14.2), 6.53 (s, 1H, CH (pyrazol)), 7.21-7.25, 7.25-7.21 (dd, 4H, 4CH(p-ClPh), J=8.9)), 7.39-7.44 (m, 1H, NH) |
| 7 | 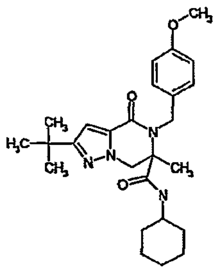 | 452,6018 | 453 |
| 8 | 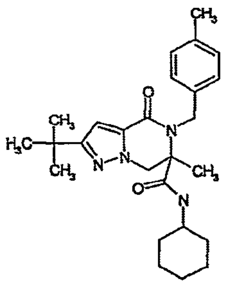 | 436,6024 | 437 |
| 9 | 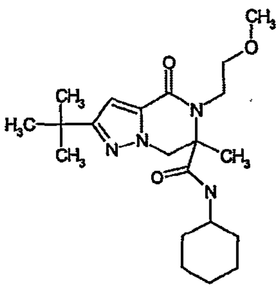 | 390,5301 | LC MS, m/z: 391 (M+1). 1H NMR: 1.04-1.3 (m, 17H, Alk), 1.55-1.69 (m, 8H, Alk), 3.33 (s, 3H, OMe), 3.43-3.62 (m, 5H, Alk), 4.02-4.08, 4.75-4.81 (dd,2H, NCH2, J=13.2), 6.47 (s, 1H, CH(pyrazol)), 7.38-7.44 (m, 1H, NH) |
| 10 | 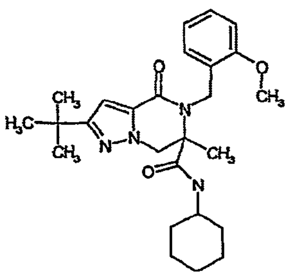 | 452,6018 | 453 |

(continued)

| | Table 1.05(2) | | |
|---|---|---|---|
| № | Formula | Mol.w. | LC MS, m/z (M+1), 1H NMR |
| 11 | 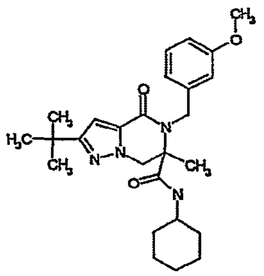 | 452,6018 | 453 |
| 12 | 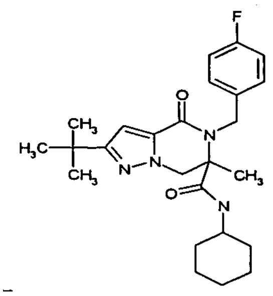 | 440,5658 | 441 |
| 13 | 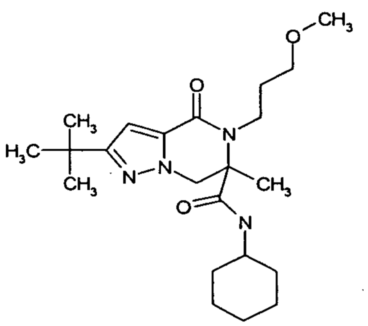 | 404,5572 | LC MS, m/z: 405 (M+1). 1H NMR: 1.03-1.32 (m, 14H, Alk), 1.50-1.86 (m, 10H, Alk), 3.18-3.26 (m, 2H, Alk), 3.27 (s, 3H, OMe), 3.33-3.40 (m, 2H, Alk), 3.41-3.60 (m, 2H, Alk), 4.01-4.08, 4.71-4.77 (dd, 2H, NCH2, J=3.17), 6.45 (s, 1H, CH(pyrazol)), 7.42-7.49 (m, 1H, NH) |
| 14 | 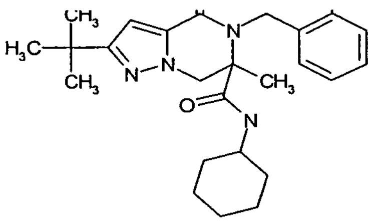 | 440,5658 | 441 |
| 15 | 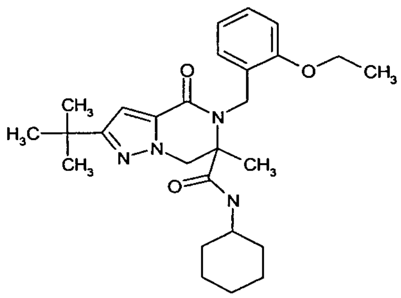 | 466,6289 | 467 |

(continued)

| | Table 1.05(2) | | |
|---|---|---|---|
| № | Formula | Mol.w. | LC MS, m/z (M+1), 1H NMR |
| 16 | 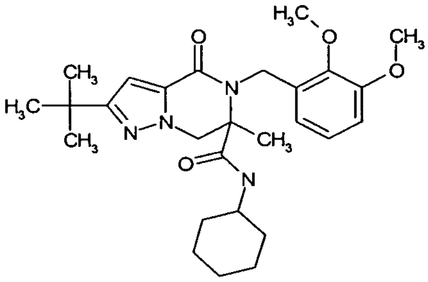 | 482,6283 | 483 |
| 17 | 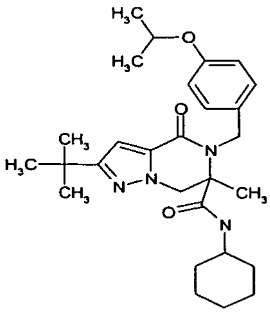 | 480,656 | 481 |
| 18 | 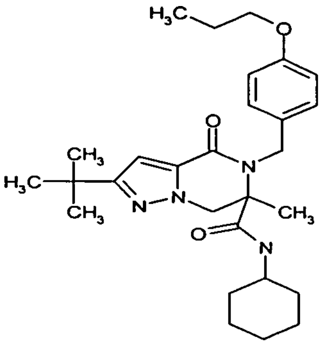 | 480,656 | 481 |
| 19 | 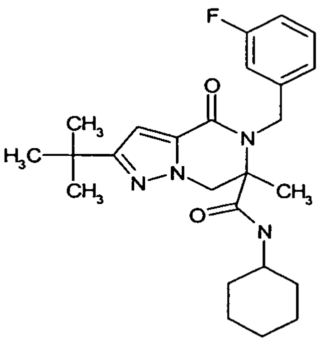 | 440,5658 | 441 |

(continued)

| | Table 1.05(2) | | |
|---|---|---|---|
| № | Formula | Mol.w. | LC MS, m/z (M+1), 1H NMR |
| 20 | 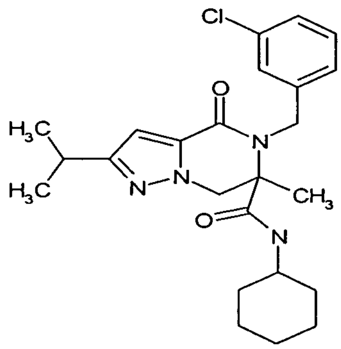 | 442,9933 | LC MS, m/z: 443 (M+1). 1H NMR: 0.97-1.75 (m, 24H, Alk), 3.41-3.51 (m, 1H, NHCH), 4.11-4.18 (m, 2H, Alk), 4.85-4.91, 5.19-5.26 (dd, 2H, NCH2, J=3.5), 6.52 (s, 1H, CH(pyrazol)), 7.16-7.33 (m, 5H, Ar), 7.47-7.54 (m, 1H, NH) |
| 21 | 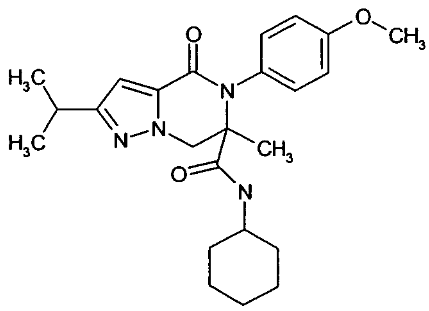 | 424,5476 | LC MS, m/z: 425 (M+1). 1H NMR: 1.00-1.74 (m, 24H, Alk), 3.43-3.52 (m, 1H, NHCH), 3.8 (s, 3H, OMe), 4.25-4.32, 4.87-4.94 (dd, 2H, NCH2, J=13.8), 6.44 (s, 1H, CH(pyrazol)), 6.82-6.89, 7.19-7.25 (dd,2H, (p-OMePh)), J=8), 7.53-7.59 (m, 1H, NH) |
| 22 | 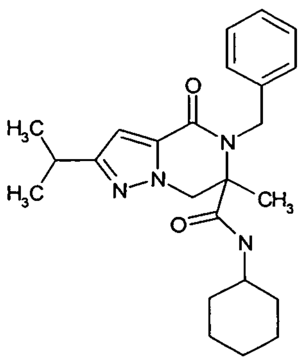 | 408,5482 | 409 |
| 23 | 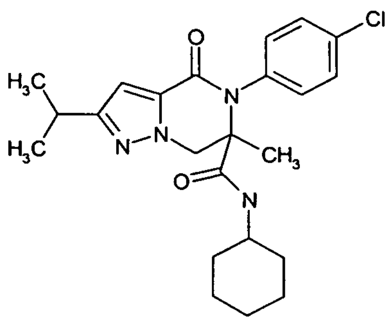 | 428,9662 | LC MS, m/z: 429 (M+1). 1H NMR: 1.00-1.78 (m, 22H, Alk), 3.43-3.55 (m, 1H, NHCH), 4.31-4.37, 4.91-4.98 (dd, 2H, NCH2, J=12,7), 6.48 (s, 1H, CH (pyrazol)), 7.30-7.45 (m, 4H, Ar), 7.63-7.69 (m, 1H, NH) |

(continued)

| | Table 1.05(2) | | |
|---|---|---|---|
| № | Formula | Mol.w. | LC MS, m/z (M+1), 1H NMR |
| 24 | 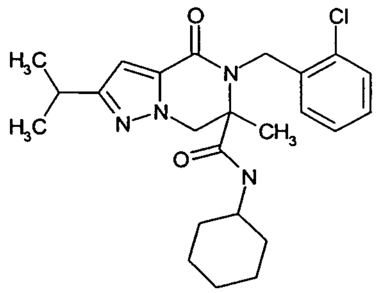 | 442,9933 | 443 |
| 25 | 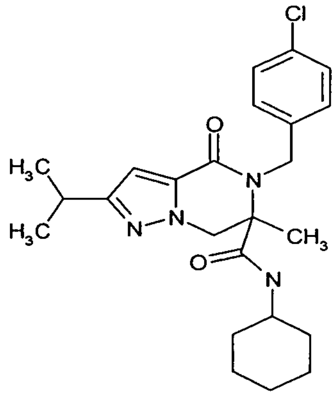 | 442,9933 | LC MS, m/z: 443 (M+1). 1H NMR: 0.93-1.74 (m, 22H, Alk), 2.86-2.95 (m, 1H, Alk), 3.37-3.50 (m , 1H, NHCH), 4.04-4.14 (m, 2H, CH(CH2Ph), CH (NCH2)), 4.80-4.88 (d, 1H, J=13.8), 5.20-5.28 (d, 1H, CH, CH2Ph, J=16.7) 6.46-6.51 (m, 1H, CH (pyrazol)), 7.20-7.26, 7.26-7.31 (dd, 4H, p-ClPh, J=7.9), 7.43-7.48 (m, 1H, NH) |
| 26 | 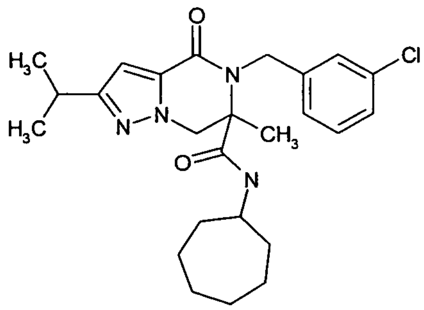 | 457,0204 | 458 |
| 27 | 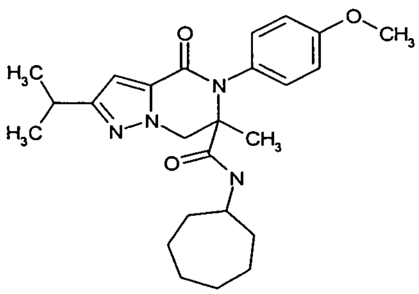 | 438,5747 | LC MS, m/z: 439 (M+1). 1H NMR: 1.00-1.75 (m, 25H, Alk), 2.86-3.00 (m, 9H, (H2O+Alk)), 3.59-3.70 (m, 1H, NHCH), 3.8 (s, 3H, OMe), 4.24-4.32, 4.87-4.95 (dd,2H, NHCH,J=13.7), 6.44 (s,1H, CH(pyrazol)), 6.83-6.89, 7.19-7.26 (dd,4H, p-OMePh, J=9), 7.58-7.65 (m, 1H, NH) |

(continued)

| | Table 1.05(2) | | |
|---|---|---|---|
| № | **Formula** | **Mol.w.** | **LC MS, m/z (M+1), 1H NMR** |
| 28 | 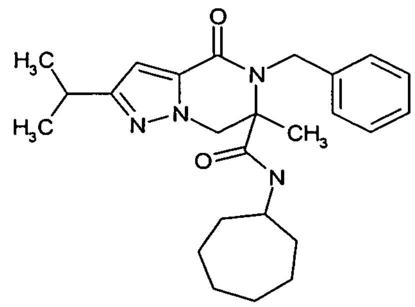 | 422,5753 | LC MS, m/z: 423 (M+1). 1H NMR: 1.08-1.69 (m, 25H, Alk), 2.77-3.00 (m, 7H, (H2O+Alk)), 3.55-3.66 (m, 1H, CHNH), 4.00-4.14 (m, 2H, CH (CH2Ph), CH(NCH2), 4.79-4.88 (d, 1H, CH2Ph, J=13.4), 5.23-5.33 (d, 1H, CHNCH2, J=15.9), 6.47 (s,1H, CH(pyrazol)), 7.09-7.13 (m, 5H, Ar), 7.35-7.43 (m, 1H, NH) |
| 29 | 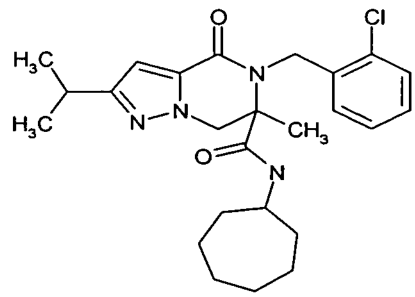 | 457,0204 | LC MS, m/z: 458 (M+1). 1H NMR: 1.20-1.75 (m, 21H, Alk), 2.90-2.99 (m, 1H, CH(i-Pr)), 3.62-3.72 (m, 1H, CONHCH), 4.16-4.26 (m, 2H, CH2Ph), 4.89-4.97, 5.18 5.26 (dd, 2H, CH2-cycle, J=13.3), 6.51 (s, CH(pyrazol)), 7.15-7.24 (m, 2H, o-ClPh), 7.29-7.35 (m, 2H, o-ClPh), 7.53-7.58 (m, 1H, NH) |
| 30 | 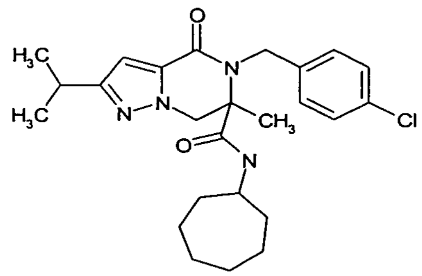 | 457,0204 | 458 |
| 31 | 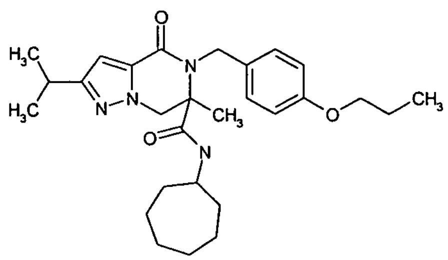 | 480,656 | 481 |
| 32 | 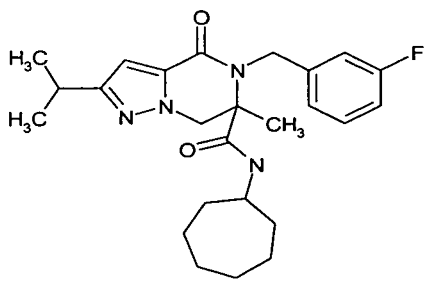 | 440,5658 | 441 |

(continued)

| | Table 1.05(2) | | |
|---|---|---|---|
| № | Formula | Mol.w. | LC MS, m/z (M+1), 1H NMR |
| 33 | 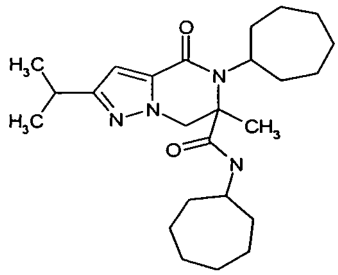 | 428,6232 | LC MS, m/z: 429 (M+1). 1H NMR: 1.18-2.00 (m, 36H, Alk), 2.3-2.47 (m, 2H, Alk), 2.79-2.99 (m, 10H, (H2O+Alk)), 3.76-3.89 (m, 1H, CHNH), 3.95-4.03, 4.55-4.62 (dd, 2H, NCH2, J=13.3), 6.44 (s, 1H, CH(pyrazol)), 7.94-8.00 (m, 1H, NH) |
| 34 | 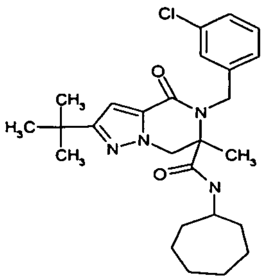 | 471,0475 | LC MS, m/z: 472 (M+1). 1H NMR: 1.16-1.74 (m, 33H, Alk), 3.58-3.69 (m, 1H, NHCH), 4.12-4.21 (m, 2H, CH(NCH2), CH(CH2Ph)), 4.88-4.95 (d,1H, CH(CH2N), J=12.6)), 5.17-5.25 (d,1H,CH (CH2Ph), J=16.3)), 6.56 (s,1H,CH(pyrazol)), 7.16-7.34 (m, 5H, |
| 35 | 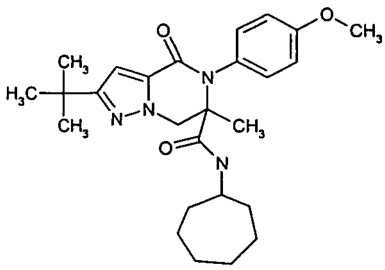 | 452,6018 | LC MS, m/z: 453 (M+1). 1H NMR: 1.10-1.76 (m, 32H, Alk), 3.62-3.73 (m, 1H, NHCH), 3.83 (s,3H, OMe), 4.28-4.35, 4.93-5.00 (dd,2H, NCH2, J=13.4), 6.49 (s,1H, CH(pyrazol)), 6.86-6.91, 7.22-7.28 (dd, 4H, Ar, J=9.2), 7.62-7.67 (m, 1H, NH) |
| 36 | 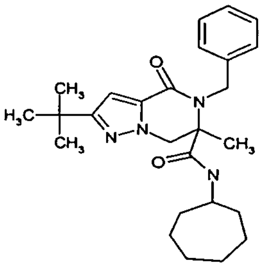 | 436,6024 | 437 |
| 37 | 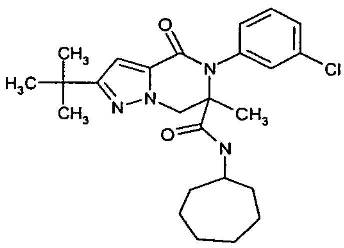 | 457,0204 | LC MS, m/z: 458 (M+1). 1H NMR: 1.53-1.80 (m, 31 H, Alk), 3.63-3.71 (m, 1 H, NHCH), 4.35-4.41, 4.97-5.04 (dd, 2H, NCH2, J=13.1), 6.53 (s, 1H, CH (pyrazol)), 7.3-7.5 (m, 4H, ClPh), 7.6-7.81 (m, 1H, NH) |

(continued)

| | Table 1.05(2) | | |
|---|---|---|---|
| № | Formula | Mol.w. | LC MS, m/z (M+1), 1H NMR |
| 38 | 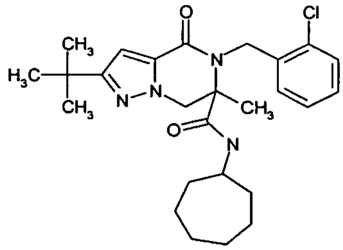 | 471,0475 | 472 |
| 39 | 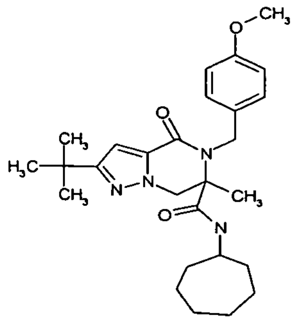 | 466,6289 | 467 |
| 40 | 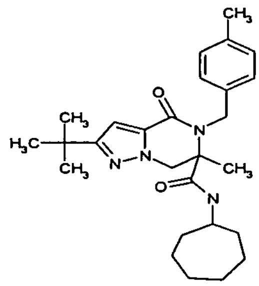 | 450,6295 | 451 |
| 41 | 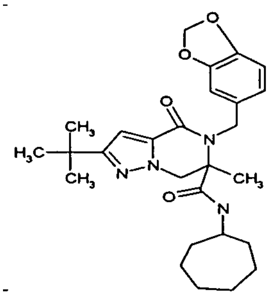 | 480,6124 | 481 |
| 42 | 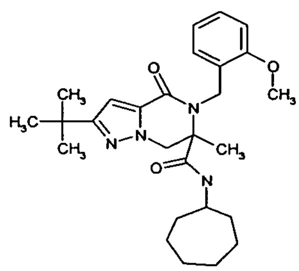 | 466,6289 | LC MS, m/z: 467 (M+1). 1H NMR: 1.55-1.68 (m, 30H, Alk), 3.6-3.69 (m, 1H, NHCH), 3.87 (s, 3H, OMe), 4.13-4.24 (m, 2H, CH(CH2Ph)CH(NCH2)), 4.87-4.93 (d, 1H, CHNCH2, J=12.9), 5.02-5.09 (d, 1H, CHCH2Ph, J=16.8), 6.51 (s,1H, CH(pyrazol)), 6.80-6.89 (m, 2H, Ar), 7.12-7.20 (m, 2H. Ar), 7.35-7.43 (m, 1H, NH) |

(continued)

| | Table 1.05(2) | | |
|---|---|---|---|
| № | Formula | Mol.w. | LC MS, m/z (M+1), 1H NMR |
| 43 | 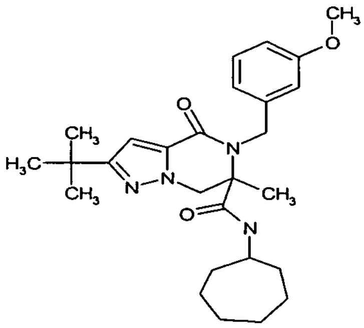 | 466,6289 | 467 |
| 44 | 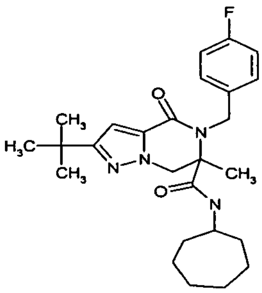 | 454,5929 | 455 |
| 45 | 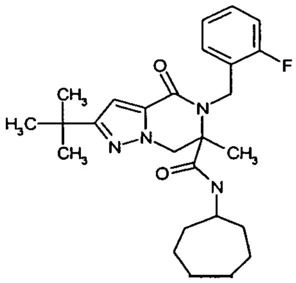 | 454,5929 | 455 |
| 46 | 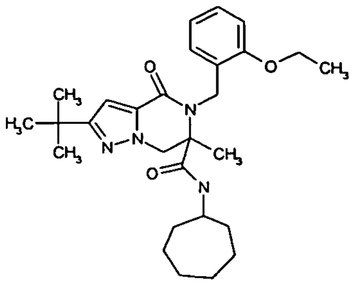 | 480,656 | 481 |
| 47 | 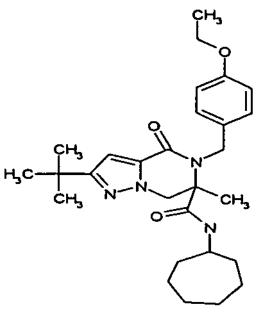 | 480,656 | 481 |

(continued)

| | Table 1.05(2) | | |
|---|---|---|---|
| № | Formula | Mol.w. | LC MS, m/z (M+1), 1H NMR |
| 48 | 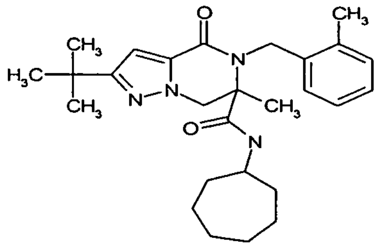 | 450,6295 | 451 |
| 49 | 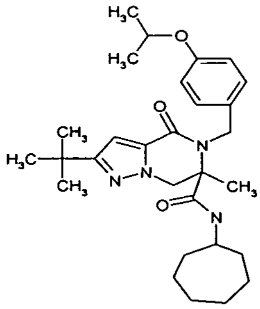 | 494,6831 | 495 |
| 50 | 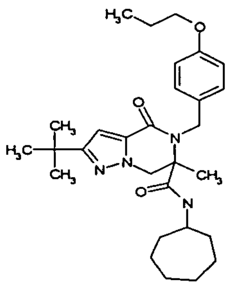 | 494,6831 | 495 |
| 51 | 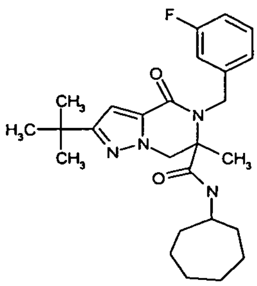 | 454,5929 | 455 |
| 52 | 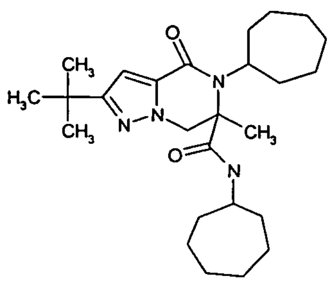 | 442,6502 | LC MS, m/z: 443 (M+1). 1H NMR: 1.20-2.00 (m, 35H, Alk), 2.30-2.46 (m, 3H, Alk), 2.79-2.89 (m, 2H, Alk), 3.77-3.86 (m, 1H, NHCH), 3.95-4.01, 4.55-4.62 (dd,2H, CH2N, J=13.3), 6.46 (s, 1H, CH (pyrazol)), 7.91-7.97 (m, 1H, NH) |

(continued)

| | Table 1.05(2) | | |
|---|---|---|---|
| № | Formula | Mol.w. | LC MS, m/z (M+1), 1H NMR |
| 53 | 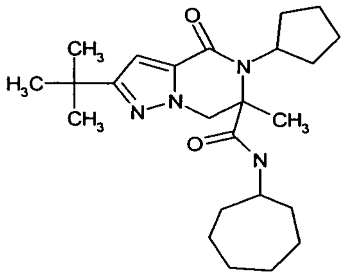 | 414,5961 | LC MS, m/z: 415 (M+1). 1H NMR: 1.08-1.96 (m, 36H, Alk), 2.01-2.12 (m, 1H, Alk), 2.24-2.32 (m, 1H, Alk), 3.20-3.31 (m, 1H, Alk), 3.75-3.86 (m, 1H, NHCH), 3.98-4.05, 4.51-4.58 (dd, 2H, NCH2, J=12.3), 6.48 (s, 1H, pyrazol), 7.91-7.98 (m, 1H, NH) |
| 54 | 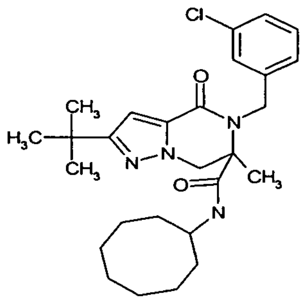 | 485,0745 | 486 |
| 55 | 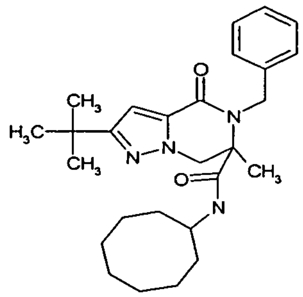 | 450,6295 | 451 |
| 56 | 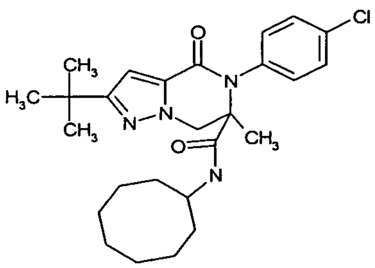 | 471,0475 | LC MS, m/z: 472 (M+1). 1H NMR: 1.14-1.69 (m, 30H, Alk), 3.69-3.79 (m, 1H, Alk), 4.32-4.38, 4.98-5.04 (dd, 2H, NCH2, J=13.6), 6.25 (s, 1H, CH (pyrazol)), 7.34-7.41 (m, 4H, p-ClPh), 7.66-7.72 (m, 1H, NH) |
| 57 | 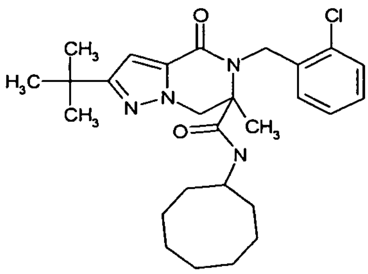 | 485,0745 | 486 |

(continued)

| | Table 1.05(2) | | |
|---|---|---|---|
| № | Formula | Mol.w. | LC MS, m/z (M+1), 1H NMR |
| 58 | 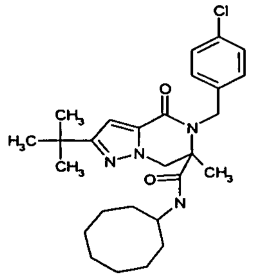 | 485,0745 | LC MS, m/z: 486 (M+1). 1H NMR: 1.20-1.65 (m, 30H, Alk), 3.65-3.75 (m, 1H, NHCH), 4.10-4.20 (m, 2H, CHN(CH2)CH(CH2Ph)), 4.87-4.94 (d, 1H, CH (NCH2), J=13.7)), 5.2-5.3 (d, 1H, CH(CH2Ph), J=16.9)), 6.54 (s, 1H, CH(pyrazol)), 7.23-7.33 (m, 4H, Ar), 7.48- |
| 59 | 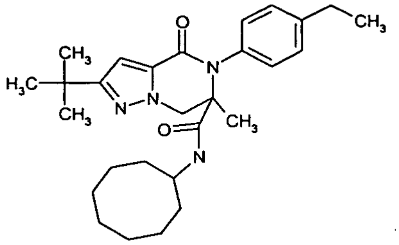 | 464,6566 | LC MS, m/z: 465 (M+1). 1H NMR: 1.23-1.70 (m, 33H, Alk), 2.66-2.74 (q, 2H, p-EtPh, J=7.2), 3.70-3.79 (m, 1H, NHCH), 4.29-4.38, 4.96-5.03 (dd, 2H, NCH2, J=14.5), 6.53 (s, 1H, CH(pyrazol)), 7.18-7.23, 7.23-7.28 (dd, 4H, Ar, J=7.2), 7.60-7.69 (m, 1H, NH) |
| 60 | 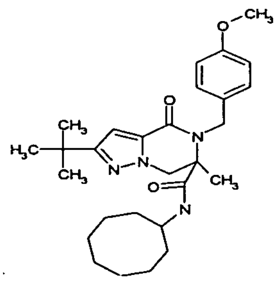 | 480,656 | 481 |
| 61 | 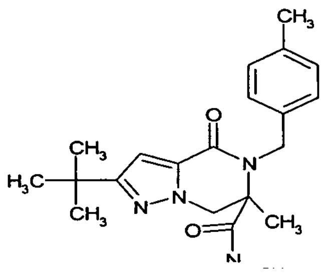 | 464,6566 | 465 |
| 62 | 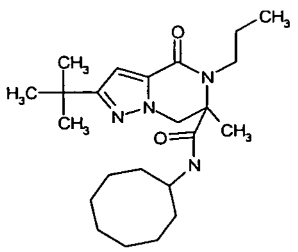 | 402,5849 | LC MS, m/z: 403 (M+1). 1H NMR: 1.20-163 (m, 30H, Alk), 1.78-1.87 (m, 2H, Alk), 3.21-3.28 (m, 1H, Alk), 3.30 (s, 3H, CH3oMe), 3.36-3.42 (m, 2H, Alk), 3.53-3.63 (m, 1H, Alk), 3.70-3.79 (m, 1H, CHNH), 4.03-4.1, 4.75-4.82 (dd, 2H, NCH2, J=13.5), 6.47 (s, 1H, CH(pyrazol)), 7.41-7.49 (m,... |

(continued)

| | Table 1.05(2) | | |
|---|---|---|---|
| № | Formula | Mol.w. | LC MS, m/z (M+1), 1H NMR |
| 63 | 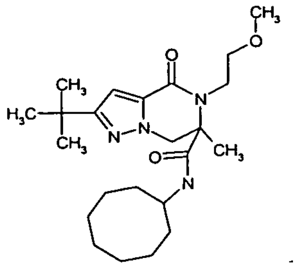 | 418,5843 | LC MS, m/z: 419 (M+1). 1H NMR: 1.21-1.64 (m, 28H, Alk), 3.31 (s, 3H, OMe), 3.42-3.73 (m, 5H, Alk), 4.01-4.08, 4.75-4.82 (dd,2H, NCH2, J=12.6), 6.46 (s, 1H, CH(pyrazol)), 7.43-7.49 (m, 1H, NH) |
| 64 | 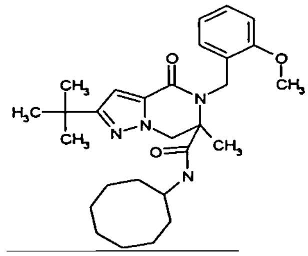 | 480,656 | 481 |
| 65 | 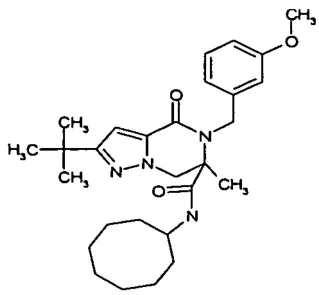 | 480,656 | 481 |
| 66 | 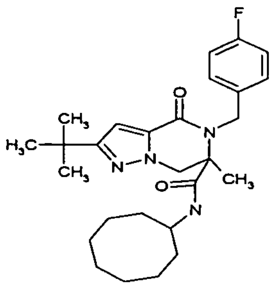 | 468,6199 | 469 |
| 67 | 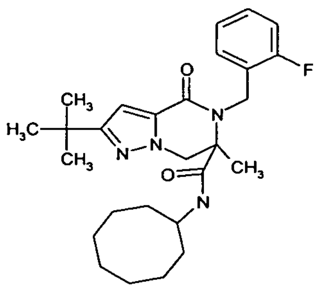 | 468,6199 | 469 |

(continued)

| | Table 1.05(2) | | |
|---|---|---|---|
| № | Formula | Mol.w. | LC MS, m/z (M+1), 1H NMR |
| 68 | 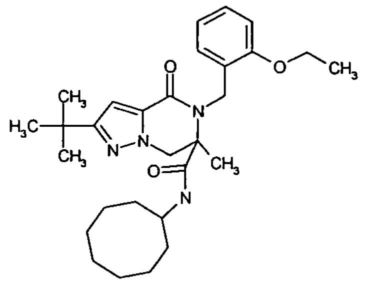 | 494,6831 | 495 |
| 69 | 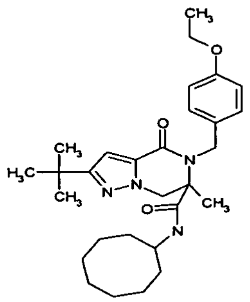 | 494,6831 | LC MS, m/z: 495 (M+1). 1H NMR: 1.20-1.60 (m, 31H, Alk), 3.63-3.73 (m, 1H, NHCH), 3.95-4.03 (q, 2H, OCH2CH3, J=6.9), 4.03-4.10, 4.83-4.90 (dd, 2H, NHCH, J=12.8), 4.10-4.17, 5.13-5.22 (dd, 2H, CH2Ph, J=16.2), 6.54 (s, 1H, CH(pyrazol)), 6.74-6.79, 7.18-7.23 (dd, 4H, p-EtOPh, J=8.9), 7.23-7.28 (m, 1H, NH) |
| 70 | 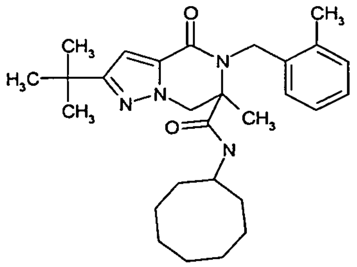 | 464,6566 | 465 |
| 71 | 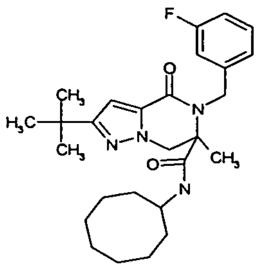 | 468,6199 | 469 |
| 72 | 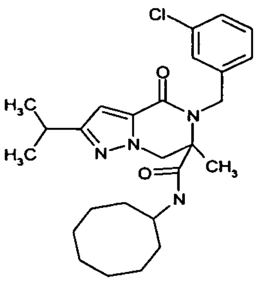 | 471,0475 | 472 |

(continued)

| | Table 1.05(2) | | |
|---|---|---|---|
| № | Formula | Mol.w. | LC MS, m/z (M+1), 1H NMR |
| 73 | 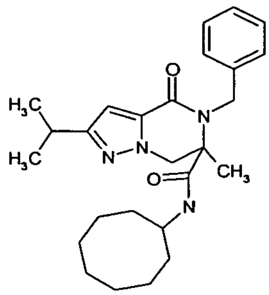 | 436,6024 | 437 |
| 74 | 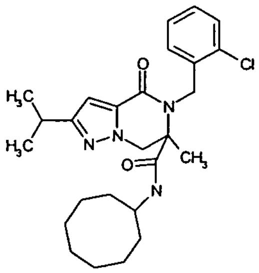 | 471,0475 | 472 |
| 75 | 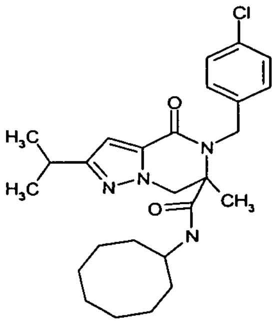 | 471,0475 | LC MS, m/z: 472 (M+1). 1H NMR: 1.18-1.70 (m, 24H, Alk), 2.89-3.00 (m, 2H, (H2O+Alk)), 3.65-3.77 (m, 1H, NHCH), 4.08-4.18 (m, 2H, CH (NCH2), CH(CH2Ph)), 4.84-4.93 (d, 1H, CH (NCH), J=12.8)), 5.21-5.29 (d, 1H, CH(CH2Ph), J=17.1)), 6.5 (s, 1H, CH(pyrazol)), 7.25-7.28, 7.28-7.33 (dd, 4H, (p-ClPh), J=8.5)), 7.45-7.50 (m, 1H, NH) |
| 76 | 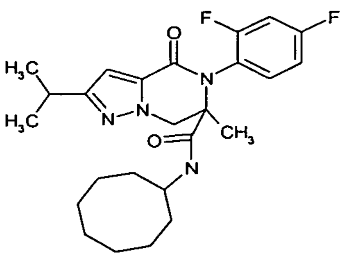 | 458,5562 | LC MS, m/z: 459 (M+1). 1H NMR: 1.16-1.68 (m, 28H, Alk), 3.67-3.77 (m, 1H, NHCH), 4.24-4.31, 5.02-5.10 (dd, 2H, NCH2, J=13.6), 6.5 (s, 1H, pyrazol), 6.94-7.06 (m, 2H, 2,4-F2Ph), 7.68-7.82 (m, 2H, NH, CH(2,4-F2Ph)) |
| 77 | 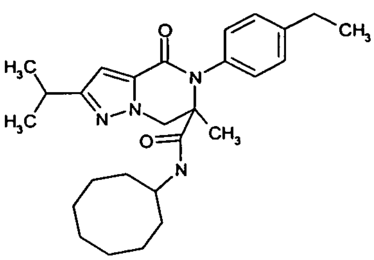 | 450,6295 | LC MS, m/z: 451 (M+1). 1H NMR: 1.66 (m, 30H, Alk), 2.63-2.72 (q, 2H, CH2(p-EtPh), J=7.9)), 2.87-2.97 (m, 4H, (H2O+Alk)), 3.68-3.78 (m, 1H, NHCH), 4.24-4.32, 4.89-4.97 (dd, 2H, NCH2, J=13.8), 6.46 (s, 1H, CH(pyrazol)), 7.13-7.19, 7.19-7.25 (dd, 4H,, 4CH(p-EtPh), J=7.9)), 7.53-7.61 (... |

(continued)

| | Table 1.05(2) | | |
|---|---|---|---|
| № | Formula | Mol.w. | LC MS, m/z (M+1), 1H NMR |
| 78 | 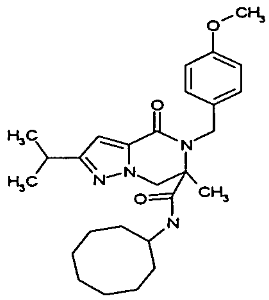 | 466,6289 | 467 |
| 79 | 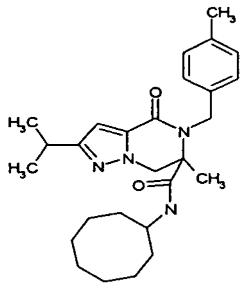 | 450,6295 | LC MS, m/z: 451 (M+1). 1H NMR: 1.09-1.64 (m, 26H, Alk), 2.31 (s, 3H, MePh), 2.86-2.97 (m, 2H, (H2O+Alk)), 3.62-3.73 (m, 1H, (NHCH)), 4.00-4.13 (m, 2H, CH(NCH2), CH(CH2Ph)), 4.81-4.88 (d, 2H, CH(NCH2), J=13.7)), 5.20-5.28 (d, 1H, CH(CH2Ph), J=16.6, 6.48 (s, 1H, CH (pyrazol)), 7.01-7.07. 7.11-7.18 (dd. |
| 80 | 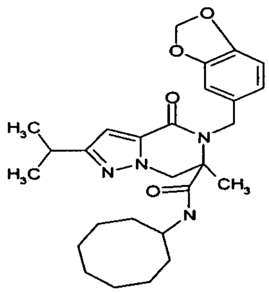 | 480,6124 | 481 |
| 81 | 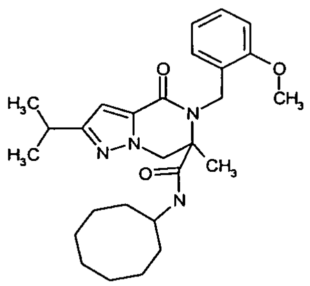 | 466,6289 | 467 |
| 82 | 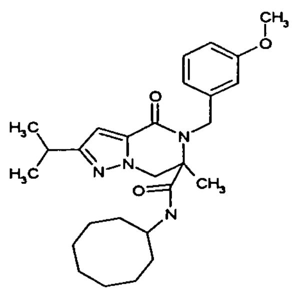 | 466,6289 | 467 |

(continued)

| | Table 1.05(2) | | |
|---|---|---|---|
| № | Formula | Mol.w. | LC MS, m/z (M+1), 1H NMR |
| 83 | 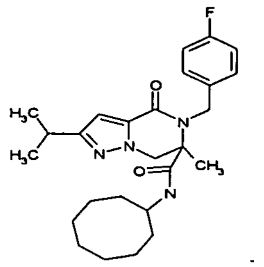 | 454,5929 | 455 |
| 84 | 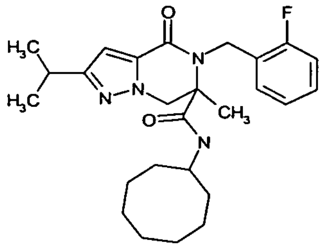 | 454,5929 | 455 |
| 85 | 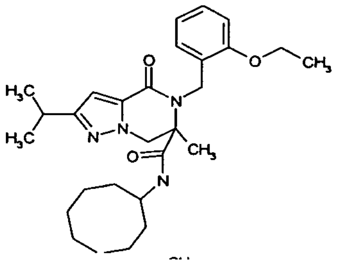 | 480,656 | 481 |
| 86 | 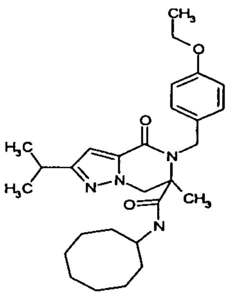 | 480,656 | 481 |
| 87 | 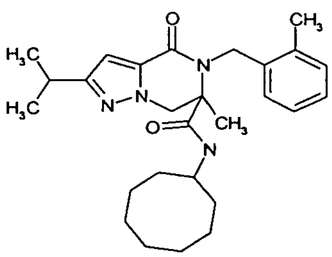 | 450,6295 | 451 |

(continued)

| | Table 1.05(2) | | |
|---|---|---|---|
| № | Formula | Mol.w. | LC MS, m/z (M+1), 1H NMR |
| 88 | 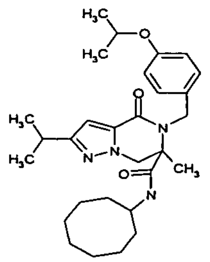 | 494,6831 | 495 |
| 89 | 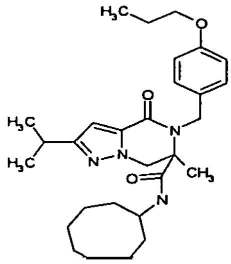 | 494,6831 | 495 |
| 90 | 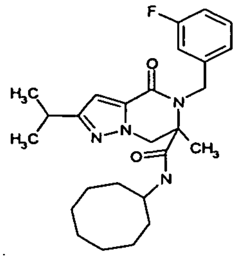 | 454,5929 | 455 |
| 91 | 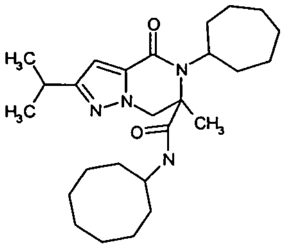 | 442,6502 | LC MS, m/z: 443 (M+1). 1H NMR: 1.16-1.80 (m, 35H, Alk), 1.88-2.00 (m, 1 H, Alk), 2.30-2.47 (m, 2H, Alk), 2.82-3.00 (m, 3H, Alk), 3.80-3.92 (m, 1H, NHCH), 4.00-4.07, 4.56-4.63 (dd, 2H, NCH2, J=13.8), 6.45 (s, 1H, CH(pyrazol)), 7.96-8.04 (m, 1H, NH) |
| 92 | 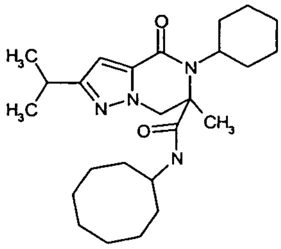 | 428,6232 | LC MS, m/z: 429 (M+1). 1H NMR: 1.09-1.90 (m, 36H, Alk), 2.38-2.54 (m, 4H, DMSO+Alk), 2.60-2.71 (m, 1H, Alk), 2.85-2.96 (m, 6H, H2O+Alk), 1.84-1.93 (m, 1H, NHCH), 3.94-4.00, 4.54-4.61 (dd, 2H, NCH2, J=12.1), 6.44 (s, 1H, CH (pyrazol)), 7.96-8.04 (m, 1H, NH) |
| 93 | 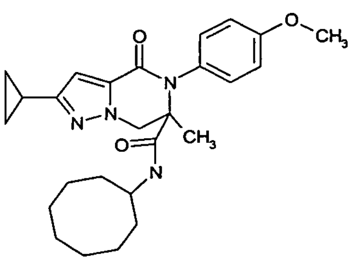 | 450,5859 | LC MS, m/z: 451 (M+1). 1H NMR: 065-0.92 (m, 4H, Alk), 1.18-1.70 (m, 31H, Alk), 1.83-1.94 (m, 1H, Alk), 3.68-3.78 (m, 1H, NHCH), 3.82 (s, 3H, OMe), 4.22-4.31, 4.85-4.93 (dd, 2H, NCH2, J=13.5), 6.35 (s, 1H, CH(pyrazol)), 6.83-6.90, 7.20-7.28 (dd, 4H, Ar, J=8.6), 7.56-7.63 (m, 1H, NH) |

(continued)

| | Table 1.05(2) | | |
|---|---|---|---|
| № | Formula | Mol.w. | LC MS, m/z (M+1), 1H NMR |
| 94 | 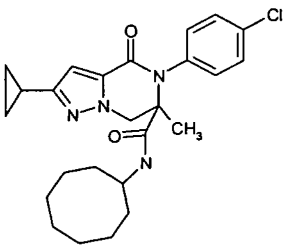 | 455,0044 | LC MS, m/z: 456 (M+1). 1H NMR: 0.63-0.94 (m, 4H, Alk), 1.23-1.70 (m, 20H, Alk), 1.83-1.95 (m, 1H, Alk), 3.67-3.80 (m, 1H, NHCH), 4.27-4.36, 4.88-4.97 (dd, 2H, NCH2, J=12.9), 6.38 (s, 1H, CH (pyrazol)), 7.37 (s, 4H, Ar), 7.66-7.72 (m, 1H, NH) |
| 95 | 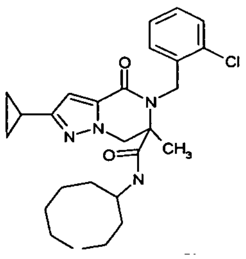 | 469,0315 | 470 |
| 96 | 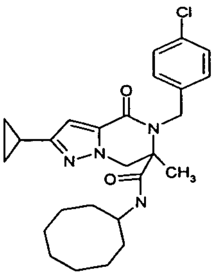 | 469,0315 | 470 |
| 97 | 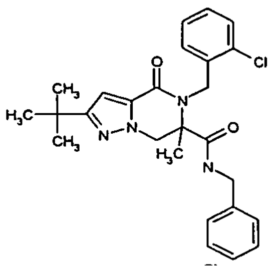 | 464,9996 | 465 |
| 98 | 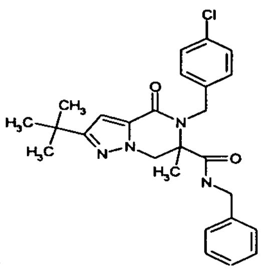 | 464,9996 | 465 |

(continued)

| | Table 1.05(2) | | |
|---|---|---|---|
| № | **Formula** | **Mol.w.** | **LC MS, m/z (M+1), 1H NMR** |
| 99 | 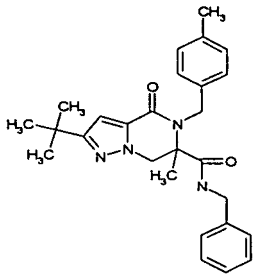 | 444,5817 | 445 |
| 100 | 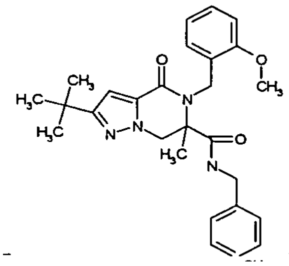 | 460,5811 | 461 |
| 101 | 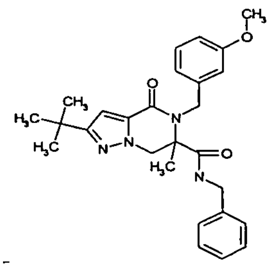 | 460,5811 | 461 |
| 102 | 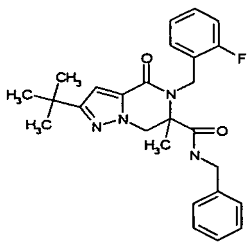 | 448,545 | 449 |
| 103 | 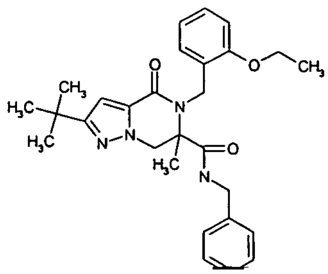 | 474,6082 | 475 |

(continued)

| | Table 1.05(2) | | |
|---|---|---|---|
| № | Formula | Mol.w. | LC MS, m/z (M+1), 1H NMR |
| 104 | 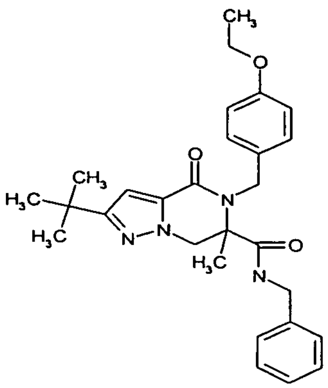 | 474,6082 | 475 |
| 105 | 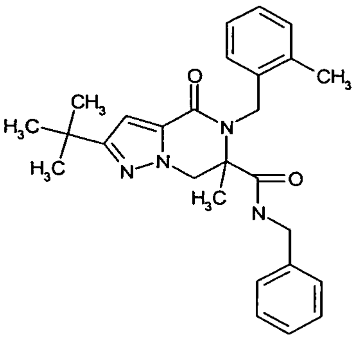 | 444,5817 | 445 |
| 106 | 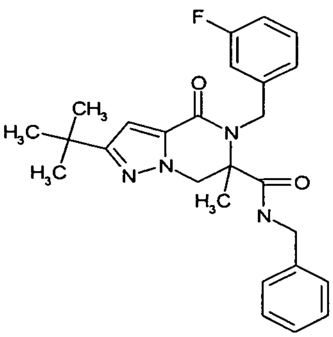 | 448,545 | 449 |
| 107 | 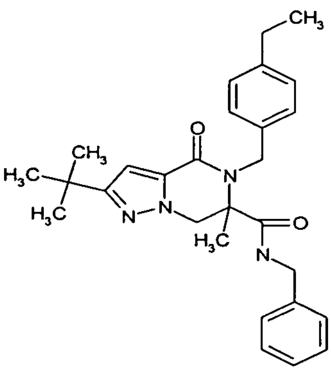 | 458,6088 | 459 |

| | Table 1.05(3) | | |
|---|---|---|---|
| № | Formula | Mol.w. | LC MS, m/z (M+1), 1H NMR |
| 1 | 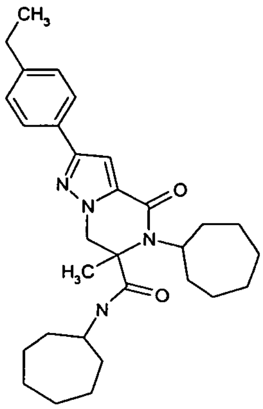 | 490,69484 | LC MS, m/z: 491 (M+1). 1H NMR: 1.26 (t, 3H, CH3 (EtPh)); 1,44-2,00 (m, 2CH, Alk); 2.34-2.475 (m, 2H, Alk); 2.6-2.7 (m, 2H, CH2 (EtPh)); 2.93 (s, 1H, N-CH); 3.84 (brs, 1H, CO-N-CH); 4.08-4.14 , 4.67-4.72 (dd, 2H, CH2, I =12.9); 6.97 (s, 1H, CH (pyrazol)); 7.15-7.19 , 7.66-7.70 (dd, 4H, Ar, I = 8.4); 8.062 (brs, 1 H, NH); |
| 2 | 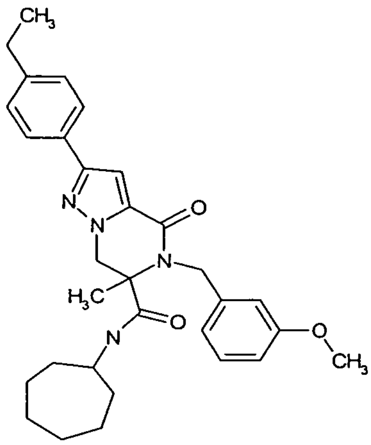 | 514,67351 | LC MS, m/z: 515 (M+1). 1H NMR: 1.26 (t, 3H, CH3 (EtPh)); 1.30-1.72 (m, 13H, Alk); 2.6-2.7 (m, 2H, CH2(EtPh)); 3.63 (brs,1H, N-CH); 3.77 (s, 3H, CH3); 4.103-4.17 , 5.21-5.24 (dd, 2H, N- CH2_Ph, I = 16.5); 4.21-4.27 , 4.94-4.99 (dd,2H, CH2, I = 13.5); 6,69-6,75 (m,1H, CH (Ar)); 6.83-6.89 (m, 2H, 2CH (Ar)); 7.05 (s, 1H, CH (pyrazol)); 17.14-7.20 (m, 3H, 3CH (Ar)); 7.45-7.53 (m, 1H, Ar); 7.69 (d, 1H, NH) |
| 3 | 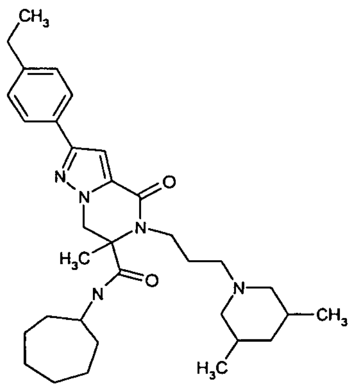 | 547,79078 | 548 |

(continued)

| | Table 1.05(3) | | |
|---|---|---|---|
| № | Formula | Mol.w. | LC MS, m/z (M+1), 1H NMR |
| 4 | 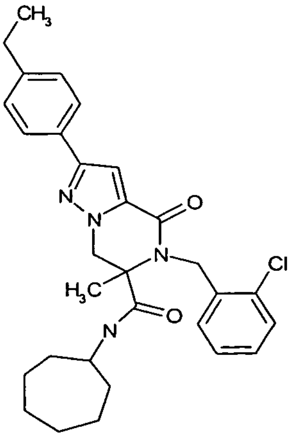 | 519,09205 | LC MS, m/z: 520 (M+1). 1 H NMR: 1.27 (t, 3H, CH3 (EtPh)); 1.31-1.79 (m,13H, Alk); 2.6-2.7 (m, 2H, CH2(EtPh)); 3.66 (brs, 1 H, N-CH); 4.19-4.29 , 5.204-5.28 (dd, 2H, CH2 (öèêë) I = 17.85); 4.31-4.37 , 4.99-5.06 (dd, 2H, CH2, I = 13.3); 7.04 (s, 1H, CH (pyrazol)); 7.15-7.29 (m, 4H, 2CH (pEtPh), 2CH (-o-ClPh)); 7.31-7.41 (m, 2H, 2CH (-o-ClPh)); 7.64-7.75 (m,3H, 2CH (pEtPh), NH) |
| 5 | 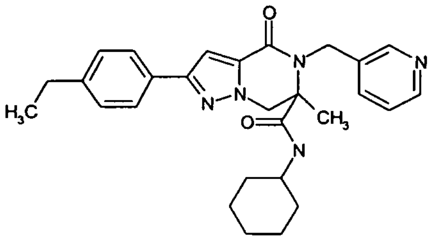 | 471,60751 | 472 |
| 6 | 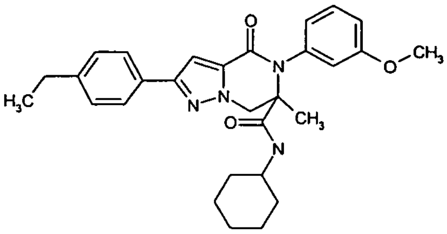 | 486,61933 | 487 |
| 7 | 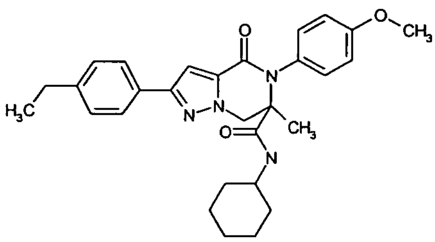 | 486,61933 | 487 |
| 8 | 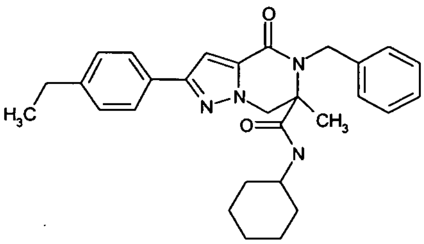 | 470,61993 | 471 |

(continued)

| | Table 1.05(3) | | |
|---|---|---|---|
| № | Formula | Mol.w. | LC MS, m/z (M+1), 1H NMR |
| 9 | 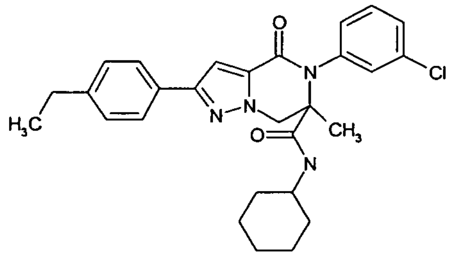 | 491,03787 | LC MS, m/z: 492 (M+1). 1H NMR: 1.00-1.78 (m, 16H, Alk), 2.66 (q, 2H, p-EtPh, J=7.8), 3.44-3.55 (m, 1 H, CONHCH), 4.45-4.53, 5.00-5.08 (dd, 2H, CH2-cycle, J=12.9), 7.06 (s, CH(pyrazol)), 7.15-7.20, 7.68-7.73 (dd, 4H, p-EtPh, J=8.1), 7.30-7.41 (m, 3H, m-ClPh), 7.47 (s, 1H, CH(m-ClPh)), 7.73-7.78 (m, 1H, NH) |
| 10 | 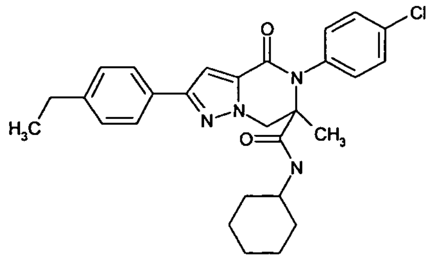 | 491,03787 | 492 |
| 11 | 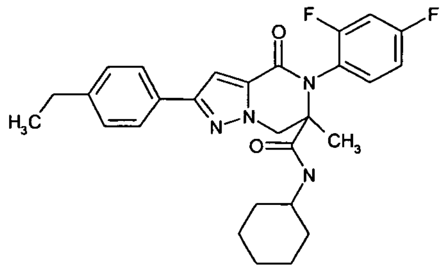 | 492,5737 | 493 |
| 12 | 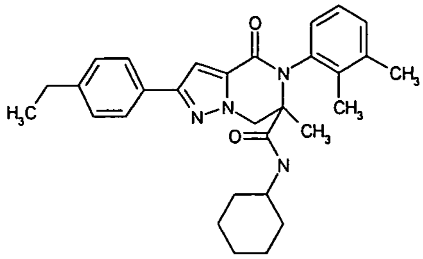 | 484,64702 | 485 |
| 13 | 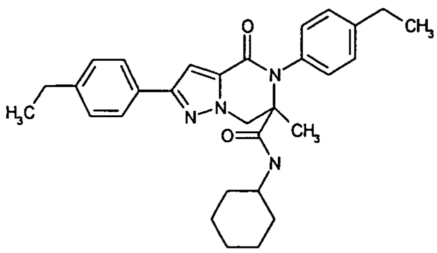 | 484,64702 | LC MS, m/z: 485 (M+1). 1 H NMR: 1.03-1.74 (m, 18H, Alk), 2.61-2.74 (m, 4H, 2CH2(p-EtPh)), 3.44-3.55 (m, 1H, CONHCH), 4.39-4.49, 4.98-5.05 (dd,2H, CH2-cycle, J=13.3), 7.026 (s, 1h, CH(pyrazol)), 7.15-7.28 (m, 6H, 6CH(p-EtPh)), 7.59-7.64 (m, 1H, NH), 7.27-7.67 (d, 2H, 2CH, p-EtPh, J=7.3) |

(continued)

| | Table 1.05(3) | | |
|---|---|---|---|
| № | Formula | Mol.w. | LC MS, m/z (M+1), 1H NMR |
| 14 | 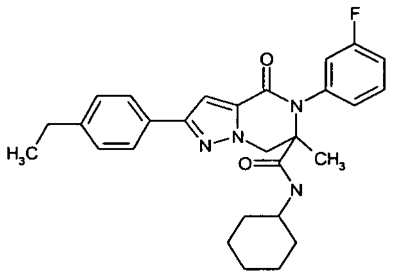 | 474,58327 | 475 |
| 15 | 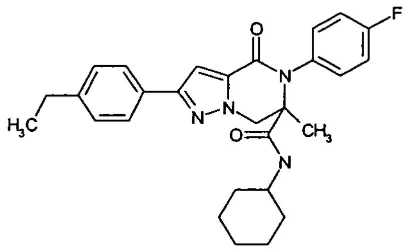 | 474,58327 | 475 |
| 16 | 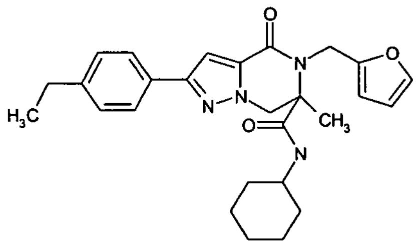 | 460,58109 | 461 |
| 17 | 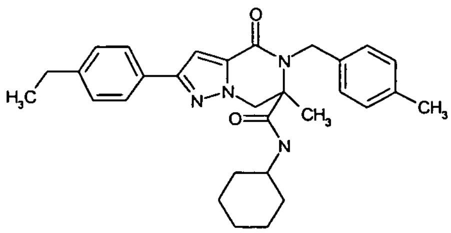 | 484,64702 | LC MS, m/z: 485 (M+1). 1H NMR: 095-1.72 (m, 16H, Alk), 2.32 (s, 3H, p-MePh), 2.66 (q, 2H, CH2(p-EtPh), J=8.9)), 3.39-3.51 (m, 1H, CONHCH), 4.08-4.16, 5.24-5.33 (dd, 2H, CH2-cycle, J=13.2), 7.02-7.10 (m, 3H, CH (pyrazol) 2CH(p-EtPh)), 7.14-7.22 (m, 4hH, 4CH(p-MePh)), 7.32-7.39 (m, 1H, NH), 7.69 (d, 2H, 2CH(p-EtPh), J=8.0)) |
| 18 | 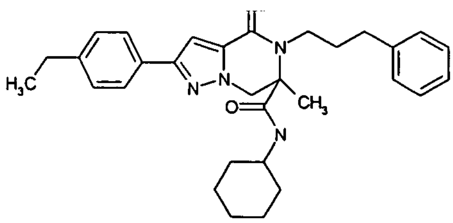 | 498,67411 | 499 |

(continued)

| | Table 1.05(3) | | |
|---|---|---|---|
| № | Formula | Mol.w. | LC MS, m/z (M+1), 1H NMR |
| 19 | 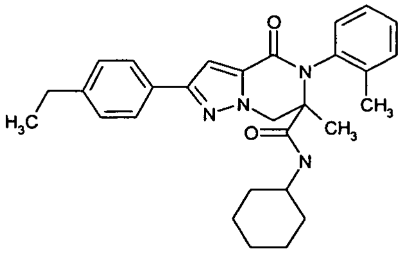 | 470,61993 | 471 |
| 20 | 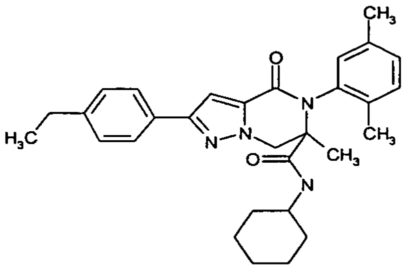 | 484,64702 | 485 |
| 21 | 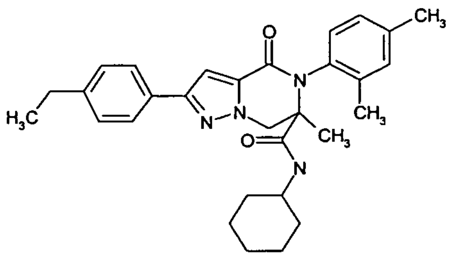 | 484,64702 | 485 |
| 22 | 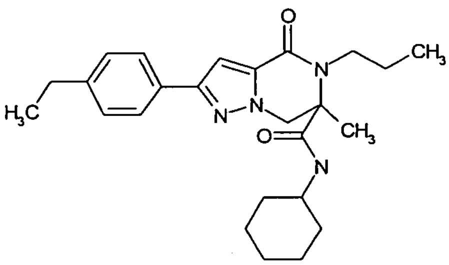 | 422,57533 | 423 |
| 23 | 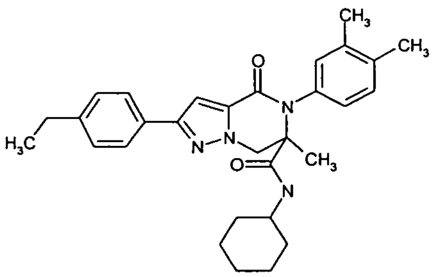 | 484,64702 | 485 |

(continued)

| | Table 1.05(3) | | |
|---|---|---|---|
| № | Formula | Mol.w. | LC MS, m/z (M+1), 1H NMR |
| 24 | 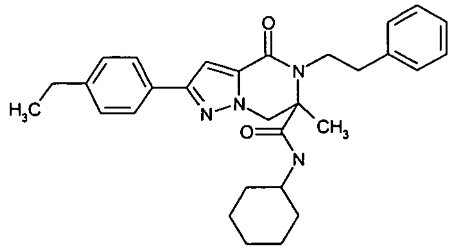 | 484,64702 | 485 |
| 25 | 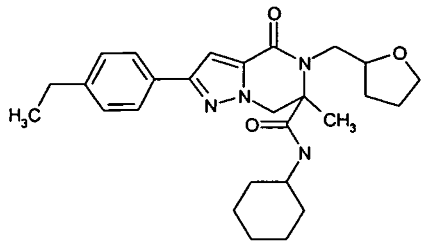 | 464,61297 | 465 |
| 26 | 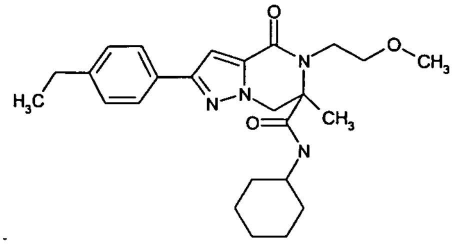 | 438,57473 | 439 |
| 27 | 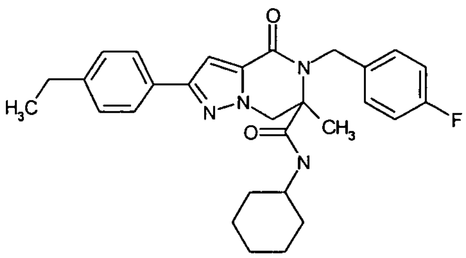 | 488,61036 | LC MS, m/z: 489 (M+1). 1H NMR: 1.00-1.74 (m, 16H, Alk), 2.66 (q, 2H, CH2(p-EtPh), J=8.1)), 3.41-3.53 (m, 1H, CONHCH), 4.09-4.19, 5.23-5.32 (dd, 2H, CH2-Ph, J=17), 4.20-4.27, 4.92-4.99 (dd, 2H, CH2-cycle, J=13.8), 6.95-7.06 (m, 3H, 1CH(pyrazol), 2CH(p-EtPh)), 7.15-7.21, 7.66-7.73 (dd, 4H, 4CH(p-EtPh), J=8.2)), 7.30-7.37 (m, 2H, 2CH (p-FPh)), 7.45-7.54 (m 1H NH) |
| 28 | 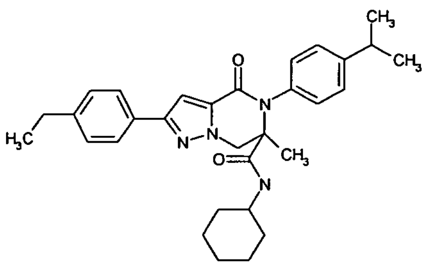 | 498,67411 | 499 |
| 29 | 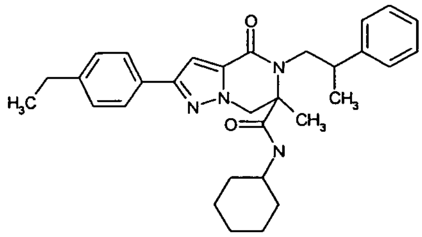 | 498,67411 | 499 |

(continued)

| | Table 1.05(3) | | |
|---|---|---|---|
| № | Formula | Mol.w. | LC MS, m/z (M+1), 1H NMR |
| 30 | 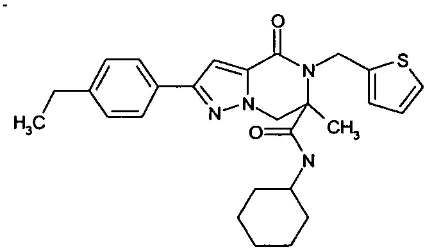 | 476,64569 | 477 |
| 31 | 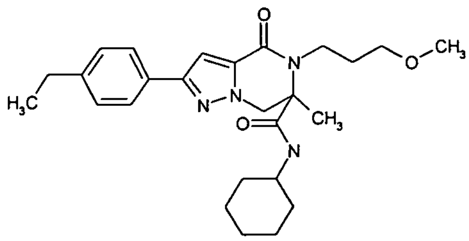 | 452,60182 | LC MS, m/z: 453 (M+1). 1 H NMR: 1.04-1.90 (m, 18 H, Alk), 2.65 (q, 2H, CH2(p-EtPh), J=8.4)), 3.30 (s, 3H, o-CH3), 3.35-3.66 (m, 5H, Alk), 4.14-4.20, 4.82-4.88 (dd, 2H, CH2-cycle, J=13.5), 6.97 (s, 1 H, CH(pyrazol)), 7.14-7.19, 7.64-7.69 (dd, 4H, 4CH(p-EtPh), J=8.8)), 7.47-7.55 (m, 1H, NH) |
| 32 | 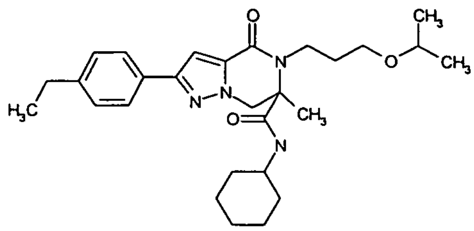 | 480,656 | 481 |
| 33 | 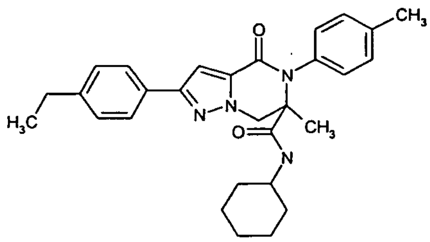 | 470,61993 | 471 |
| 34 | 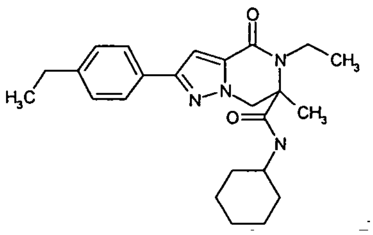 | 408,54824 | 409 |
| 35 | 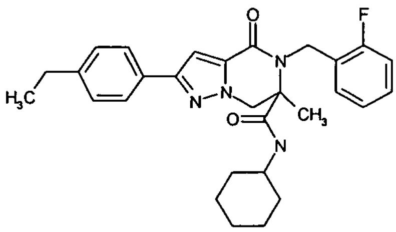 | 488,61036 | 489 |

(continued)

| | Table 1.05(3) | | |
|---|---|---|---|
| № | Formula | Mol.w. | LC MS, m/z (M+1), 1H NMR |
| 36 | 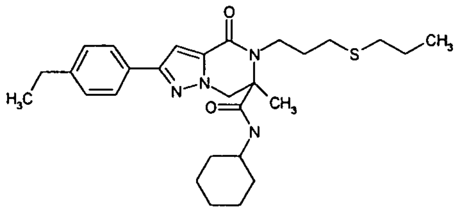 | 496,7206 | 497 |
| 37 | 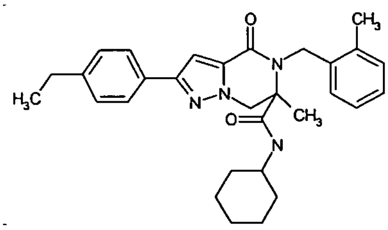 | 484,64702 | 485 |
| 38 | 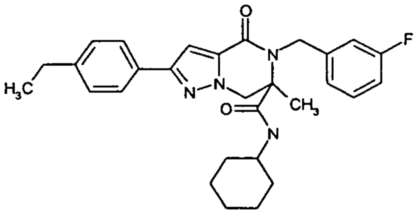 | 488,61036 | 489 |
| 39 | 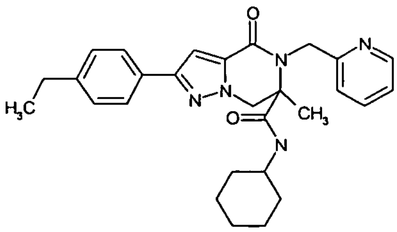 | 471,60751 | 472 |
| 40 | 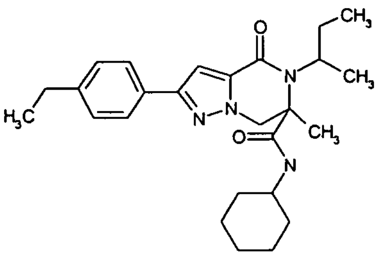 | 436,60242 | 437 |
| 41 | 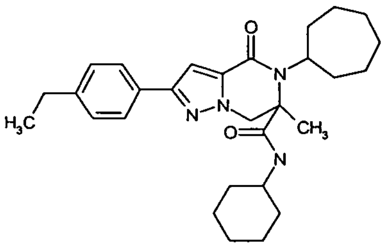 | 476,66775 | 477 |

(continued)

| | Table 1.05(3) | | |
|---|---|---|---|
| № | Formula | Mol.w. | LC MS, m/z (M+1), 1H NMR |
| 42 | 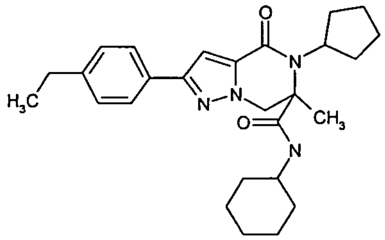 | 448,61357 | 449 |
| 43 | 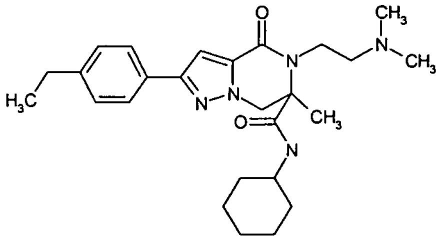 | 451,61709 | 452 |
| 44 | 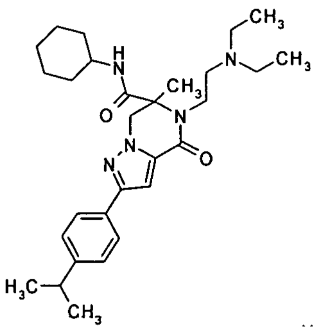 | 493,70 | 494 |
| 45 | 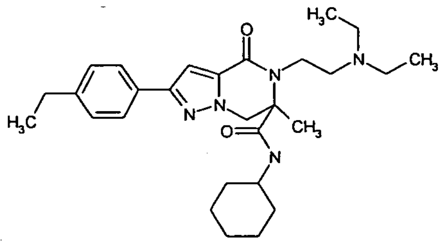 | 479,67127 | 480 |
| 46 | 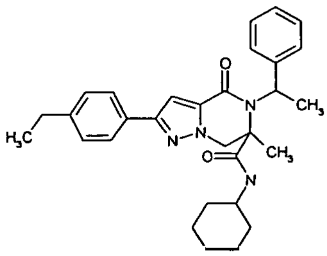 | 484,64702 | 485 |

(continued)

| | Table 1.05(3) | | |
|---|---|---|---|
| № | Formula | Mol.w. | LC MS, m/z (M+1), 1H NMR |
| 47 | 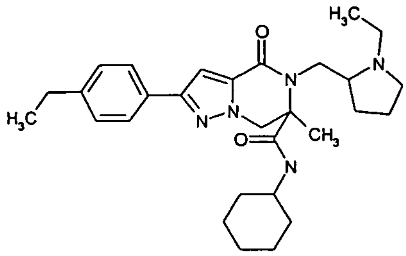 | 491,68242 | 492 |
| 48 | 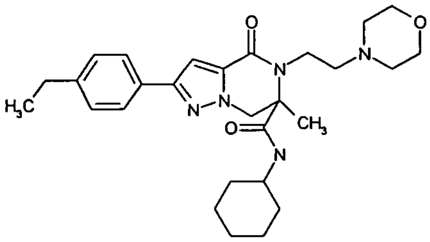 | 493,65473 | 494 |
| 49 | 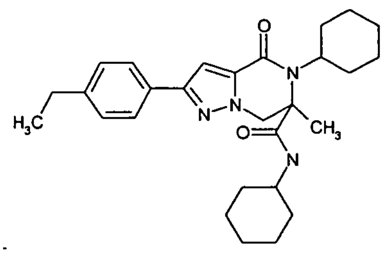 | 462,64066 | 463 |
| 50 | 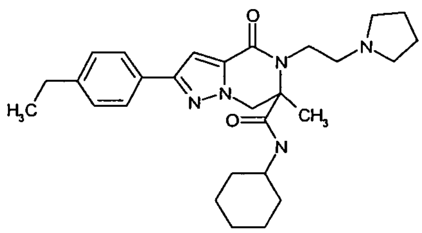 | 477,65533 | 478 |
| 51 | 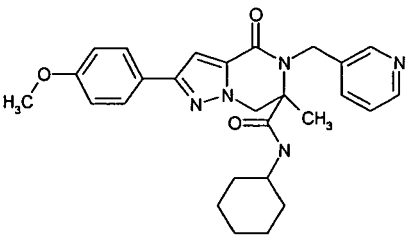 | 473,57982 | 474 |
| 52 | 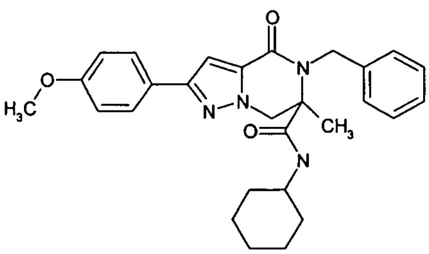 | 472,59224 | LC MS, m/z: 473 (M+1). 1H NMR: 1.00-1.72 (m, 13H, Alk), 3.4-3.52 (m, 1H, CONHCH), 3.82 (s, 3H, o-CH3), 4.11-4.18, 5.30-5.39 (dd,2H, CH2-Ph, J=8.9), 4.18- 4.24, 4.93-4.99 (dd, 2H, CH2-cycle, J=14.0), 6.85-6.90, 7.68-7.74 (dd, 4H, 4CH(p-OMePh), J=8.8)), 7.00-7.02 (m, 1H, CH (pyrazol)), 7.16-7.33(m, 5H, Ph), 7.41-7.47 (m, 1H, NH) |

(continued)

| | Table 1.05(3) | | |
|---|---|---|---|
| № | Formula | Mol.w. | LC MS, m/z (M+1), 1H NMR |
| 53 | 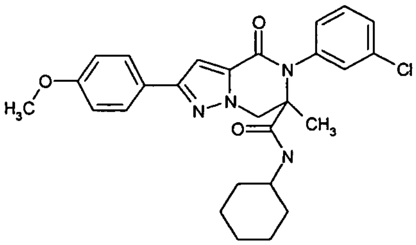 | 493,01018 | 1.02-1.88 (m, 13H, Alk), 3.46-3.56 (m, 1H, CONHCH), 3.82 (s, 3H, OCH3), 4.44-4.49, 5.01-5.07 (dd, 2H, CH2-cycle, J=12.5), 6.86 6.91, 7.70-7.75 (dd, 4H, 4CH(p-MeOPh), J=8.4)), 7.02 (s, 1H, CH(pyrazol)), 7.31-7.42 (m, 3H, 3CH(m-ClPh)), 7.48 (s, 1H, CH(m-ClPh)), 7.74-7.77 (m, 1H, NH) |
| 54 | 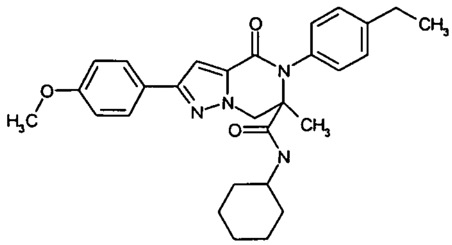 | 486,61933 | LC MS, m/z: 487 (M+1). 1 H NMR: 1.03-1.76 (m, 18H, Alk), 2.69 (q, 2H, PhCH2Ch3, J=7.5), 3.82 (s, 3H, MeO), 3.44-3.55 (m, 1H, CONHCH), 4.37-4.43, 4.97-5.03 (dd, 2H, CH2-cycle, J=12.5), 6.85-6.91, 7.69-7.74 (dd, 4H, 4CH(p-OMePh), J=8.6)), 6.98 (s, 1H, CH(pyrazol)), 7.16-7.21, 7.21-7.27 (dd, 4H, 4CH(p-EtPh), J=8.0)), 7.57-7.62 |
| 55 | 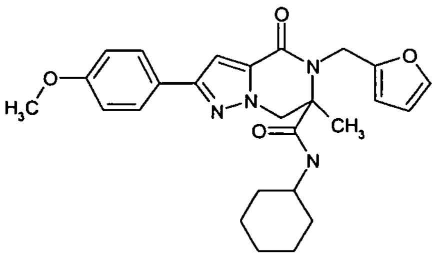 | 462,5534 | 463 |
| 56 | 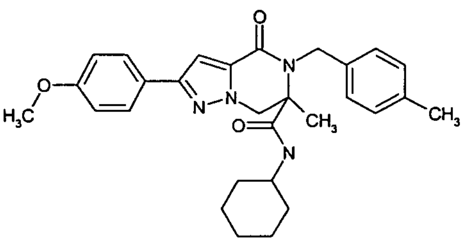 | 486,61933 | LC MS, m/z: 487 (M+1). 1 H NMR: 0,97-1.72 (m, 14H, Alk), 2.33 (s, #h, CH3(p-MePh00, 3.39-3.50 (m, 1 H, CONHCH), 3.82 (s, 3H, OMe), 4.08-4.15, 5.24-5.33 (dd, 2H, CH2-cycle, J=12.9), 6.86-6.92, 7.68-7.74 (dd, 4H, 4CH(OMePh), J=8.2)), 7.00 (s, 1H, CH (pyrazol)), 7.01-7.09, 7.15-7.21 (dd, 4H, 4CH (p-MePh), J=8.3)), 7.34-7.41 |
| 57 | 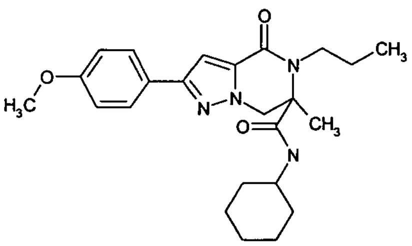 | 424,54764 | 425 |
| 58 | 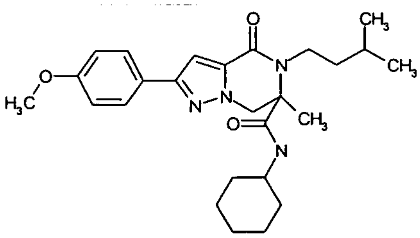 | 452,60182 | 453 |

(continued)

| | Table 1.05(3) | | |
|---|---|---|---|
| № | Formula | Mol.w. | LC MS, m/z (M+1), 1H NMR |
| 59 | 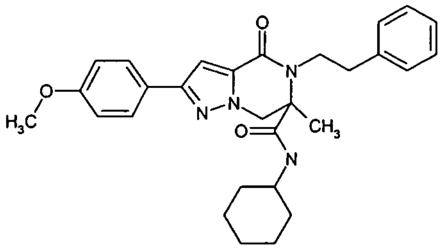 | 486,61933 | 487 |
| 60 | 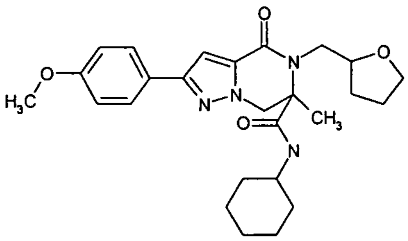 | 466,58528 | 467 |
| 61 | 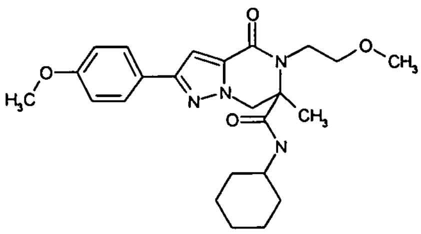 | 440,54704 | LC MS, m/z: 441 (M+1). 1 H NMR: 1.05-1.33 (m, 5H, Alk), 1.54-1.72 (m, 9H, Alk), 3.39 (s, 3H, CH2OMe), 3.43-3.67 (m, 5H, Alk), 3.81 (s, 3H, MeOPh), 4.10-4.16, 4.83-4.89 (dd, 2H, CH2-cycle, J=13.5), 6.83-6.89, 7.66-7.72 (dd, 4H, 4CH(p-OMePh), J=8.5)), 6.93 (s, 1H, CH(pyrazol)), 7.42-7.48 (m, 1H, |
| 62 | 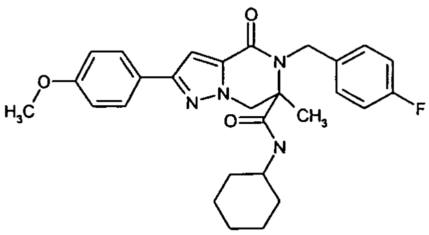 | 490,58267 | 491 |
| 63 | 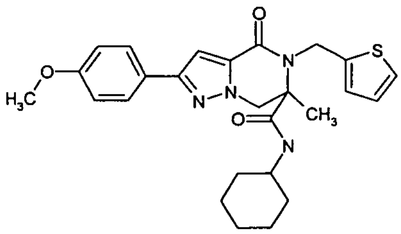 | 478,618 | 479 |
| 64 | 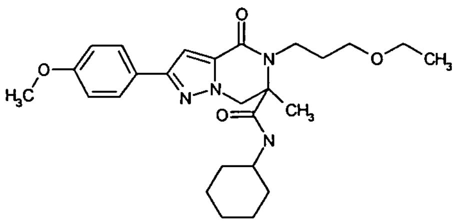 | 468,60122 | 469 |

(continued)

| | Table 1.05(3) | | |
|---|---|---|---|
| № | Formula | Mol.w. | LC MS, m/z (M+1), 1H NMR |
| 65 | 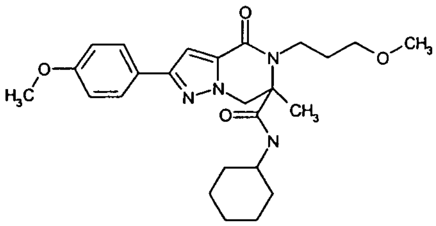 | 454,57413 | 455 |
| 66 | 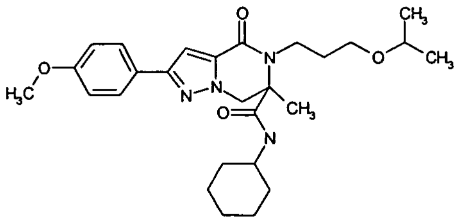 | 482,62831 | 483 |
| 67 | 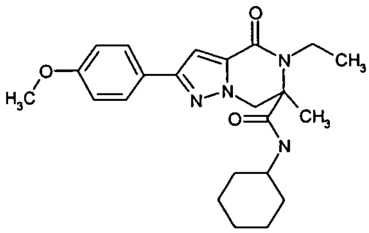 | 410,52055 | 411 |
| 68 | 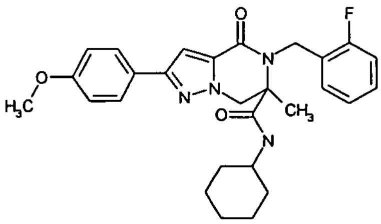 | 490,58267 | 491 |
| 69 | 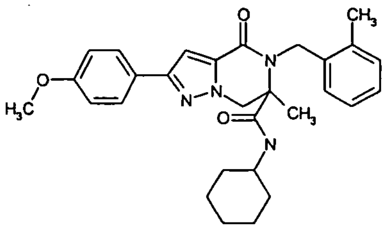 | 486,61933 | 487 |
| 70 | 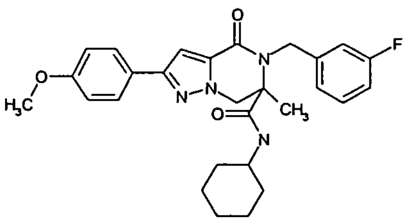 | 490,58267 | 491 |

(continued)

| | Table 1.05(3) | | |
|---|---|---|---|
| № | Formula | Mol.w. | LC MS, m/z (M+1), 1H NMR |
| 71 | 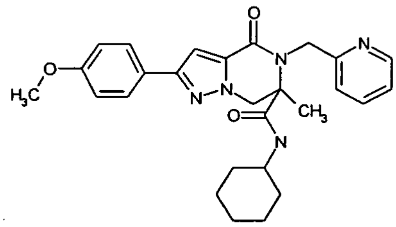 | 473,57982 | 474 |
| 72 | 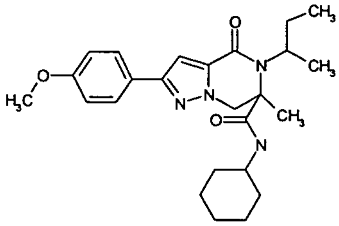 | 438,57473 | 439 |
| 73 | 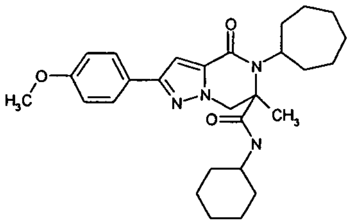 | 478,64006 | 479 |
| 74 | 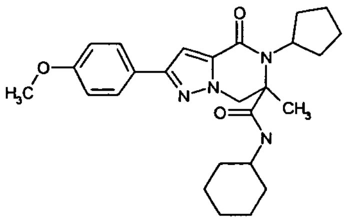 | 450,58588 | 451 |
| 75 | 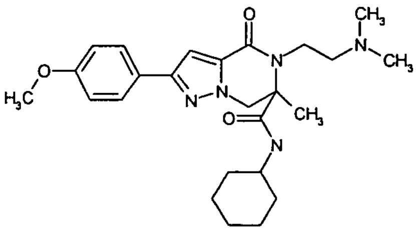 | 453,5894 | 454 |
| 76 | 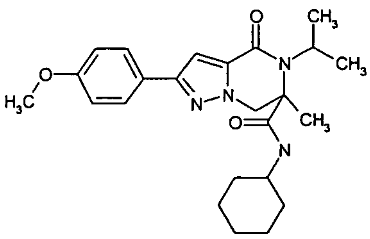 | 424,54764 | 425 |

(continued)

| | Table 1.05(3) | | |
|---|---|---|---|
| № | Formula | Mol.w. | LC MS, m/z (M+1), 1H NMR |
| 77 | 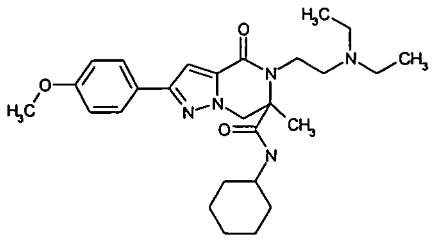 | 481,64358 | 482 |
| 78 | 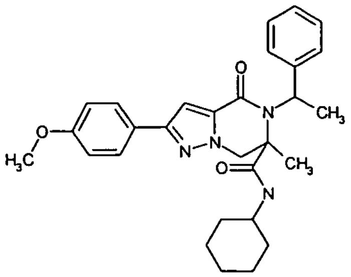 | 486,61933 | 487 |
| 79 | 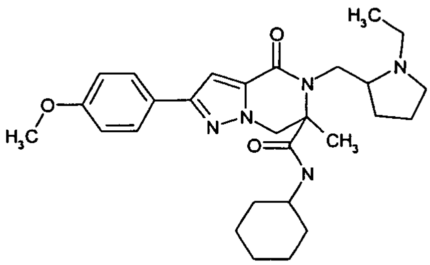 | 493,65473 | 494 |
| 80 | 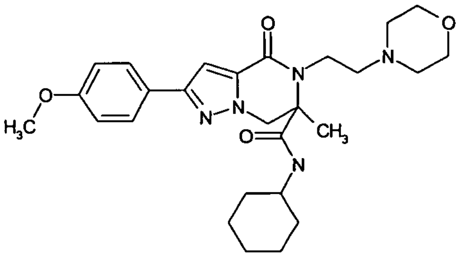 | 495,62704 | LC MS, m/z: 496 (M+1). 1H NMR: 1.04-1.33 (m, 5H, Alk), 1.55-1.75 (m, 8H, Alk), 2.39-2.59 (m, 7H, DMSO, 3NCH2), 3.37-3.69 (m, 7H, Alk), 3.80 (s, 3H, MeO), 4.12-4.18, 4.79-4.88 (dd, 2H, CH2-cycle, J=13.8), 6.84-6.89, 7.66-7.72 (dd, 4H, 4CH(OMePh), J=8.5)), 6.93 (s, 1H, CH(pyrazol)), 7.57 (d, 1H, NH, J=8.0) |
| 81 | 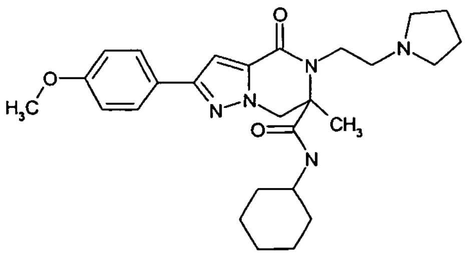 | 179,62764 | 480 |

(continued)

| | Table 1.05(3) | | |
|---|---|---|---|
| № | Formula | Mol.w. | LC MS, m/z (M+1), 1H NMR |
| 82 | 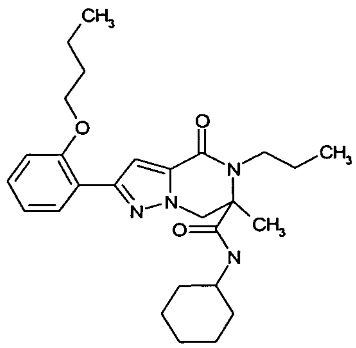 | 466,62891 | 467 |
| 83 | 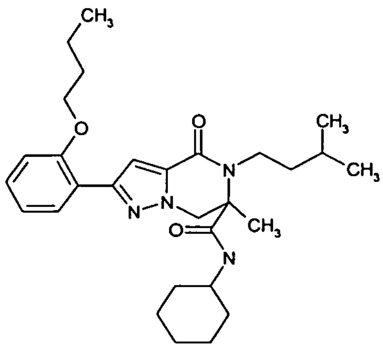 | 494,68309 | 495 |
| 84 | 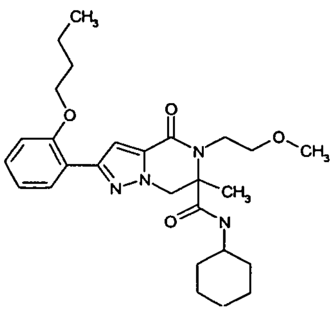 | 482,62831 | LC MS, m/z: 483 (M+1). 1 H NMR: 1.00-1.34 (m, 9H, Alk), 1.50-1.74 (m, 12H, Alk), 1.85-1.96 (m, 2H, Alk), 2.88-3.01 (m, 2H, (H2O+Alk)), 3.36 (s, 3H, OMe), 3.43-3.70 (m, 6H, Alk), 4.05-4.12 (m, 2H, Alk), 4.15-4.22, 4.85-4.93 (dd, 2H, NCH2, J=13.5), 6.88-7.00 (m, 2H, Ar), 7.14-7.25 (m, 2H, Ar), 7.44-7.54 (m, 1H, NH), 7.91-7.96 (m, 1H, Ar) |
| 85 | 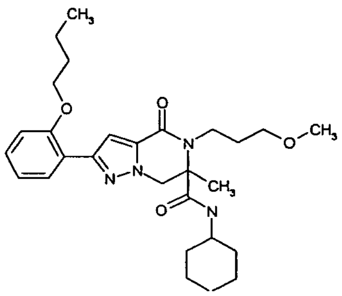 | 496,6554 | LC MS, m/z: 497 (M+1). 1 H NMR: 1.00-1.34 (m, 8H, Alk), 1.50-1.95 (m, 16H, Alk), 2.87-3.00 (m, 2H, (H2O+Alk)), 3.29 (s,3H, Alk), 3.37-3.66 (m, 4H, Alk), 4.04-4.11 (m, 2H, Alk), 4.15-4.22, 4.82-4.89 (dd, 2H, NCH2, J=13.6), 6.87-6.98 (m, 2H, Ar), 7.14-7.25 (m, 2H, Ar), 7.47-7.57 (m, 1H, Ar0, 7.89-7.95 (m, 1H, NH) |

(continued)

| | Table 1.05(3) | | |
|---|---|---|---|
| № | Formula | Mol.w. | LC MS, m/z (M+1), 1H NMR |
| 86 | 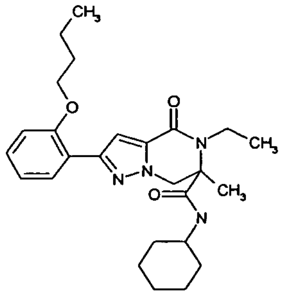 | 452,60182 | 453 |
| 87 | 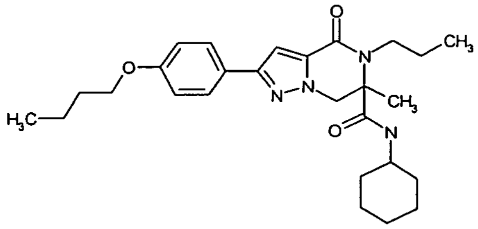 | 466,62891 | LC MS, m/z: 467 (M+1). 1H NMR: 0.89-1.34 (m, 12H, Alk), 1.45-1.81 (m, 16H, Alk), 3.02-3.14 (m, 1 H, Alk), 3.43-3.56 (m, 2H, Alk), 3.92-4.00 (m, 2H, Alk), 4.11-4.18, 4.78 4.85 (dd, 2H, NCH2,J=13.2), 6.81-6.87, 7.64-7.71 (dd, 4H, Ar, J=8.3), 6.91-6.94 (m, 1H, CH(pyrazol)), 7.52-7.59 (m, 1H, NH) |
| 88 | 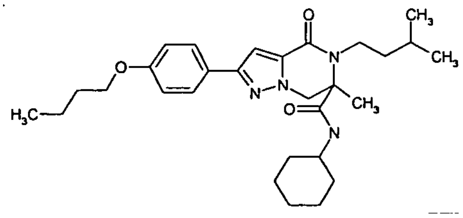 | 494,68309 | 495 |
| 89 | 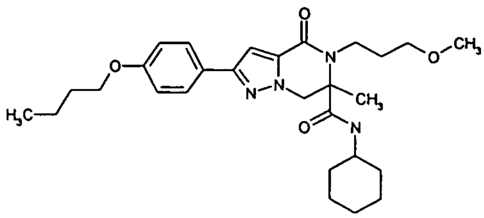 | 196,6554 | LC MS, m/z: 497 (M+1). 1 H NMR: 0.93-1.33 (m, 9H, Alk), 1.46-1.89 (m, 16H, Alk), 3.21-3.65 (m, 9H, Alk), 3.92-3.99 (m, 2H, Alk), 4.13-4.20, 4.79-4.86 (dd, 2H, NCH2, J=13.8), 6.81-6.88, 7.64-7.70 (dd, 4H, Ar, J=8.9), 6.93 (s, 1H, CH(pyrazol)), 7.52-7.59 (m, 1 H, NH) |
| 90 | 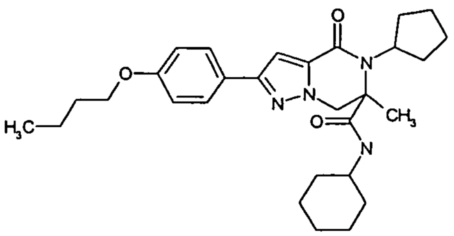 | 492,66715 | LC MS, m/z: 493 (M+1). 1 H NMR: 0.95-2.00 (m, 31 H, Alk), 2.04-2.17 (m, 1H, Alk), 2.28-2.40 (m, 1H, Alk), 2.25-2.36 (m, 1 H, Alk), 3.56-3.67 (m, 1H, NHCH), 3.93-4.00 (m, 2H, OCH2), 4.09-4.17, 4.60-4.68 (dd, 2H, NCH2, J=13.9), 6.82-6.89, 7.63-7.71 (dd, 4H, Ar, J=8.7), 6.92-6.96 (m, 1H, CH(pyrazol)), 7.97-8.05 (m, 1H, NH) |
| 91 | 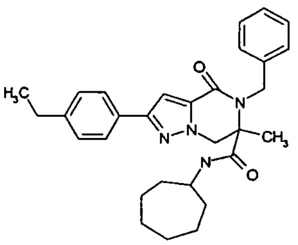 | 484,64702 | 485 |

(continued)

| | Table 1.05(3) | | |
|---|---|---|---|
| № | Formula | Mol.w. | LC MS, m/z (M+1), 1H NMR |
| 92 | 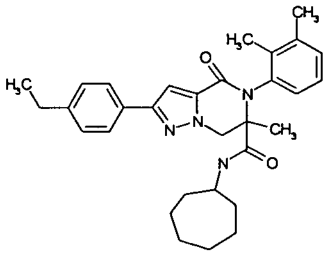 | 498,67411 | LC MS, m/z: 499 (M+1). 1H NMR: 1.15-1.83 (m, 22H, Alk), 2.10 (s, 3H, MePh), 2.34 (s, 3H, MePh), 2.62-2.71 (m, 2H, CH2(p-EtPh)), 3.64-3.75 (m, 1H, NHCH), 4.42-4.50, 5.02-5.09 (dd, 2H, NCH2, J=13.6), 7.00-7.14 (m, 4H, Ar), 7.15-7.21, 7.67-7.75 (dd, 4H, Ar, J=7.8), 7.30-7.35 (m, 1H, NH) |
| 93 | 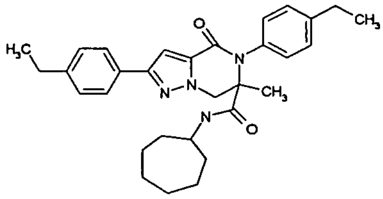 | 498,67411 | 499 |
| 94 | 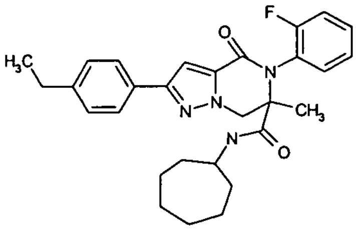 | 488,61036 | 489 |
| 95 | 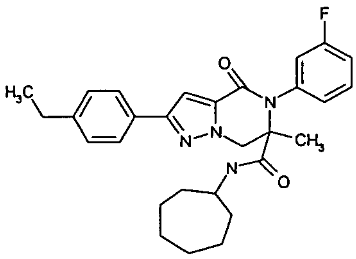 | 488,61036 | 489 |
| 96 | 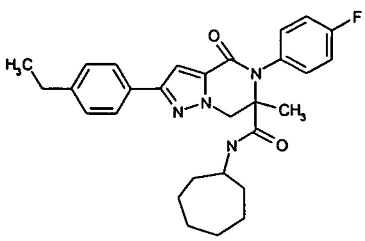 | 488,61036 | LC MS, m/z: 489 (M+1). 1 H NMR: 1.10-1.79 (m, 20H, Alk), 2.60-2.70 (q, 2H, OCH2,J=7.9), 3.61-3.72 (m, 1H, NHCH), 4.43-4.51, 5.00-5.08 (dd, 2H, NCH2, J=13.9), 7.00-7.15 (m, 3H, Ar), 7.15-7.21, 7.67-7.73 (dd,4H, Ar, J=7.4), 7.36-7.46 (m, 2H, Ar), 7.74-7.80 (m, 1H, NH) |
| 97 | 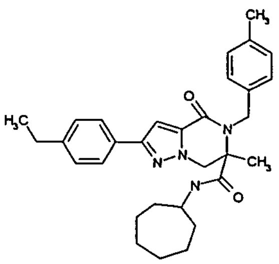 | 498,67411 | 499 |

(continued)

| | Table 1.05(3) | | |
|---|---|---|---|
| № | Formula | Mol.w. | LC MS, m/z (M+1), 1H NMR |
| 98 | 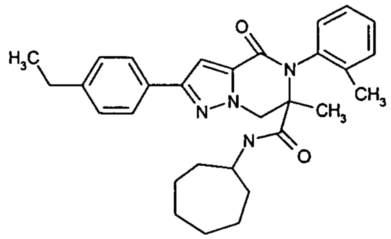 | 484,64702 | 485 |
| 99 | 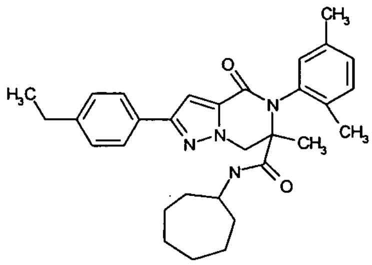 | 498,67411 | 499 |
| 100 | 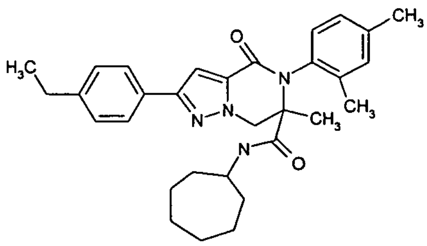 | 498,67411 | 499 |
| 101 | 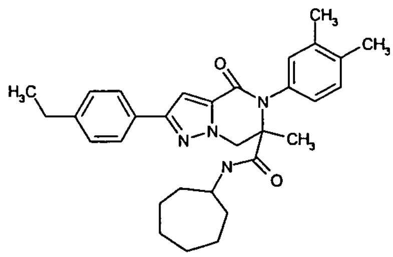 | 498,67411 | 499 |
| 102 | 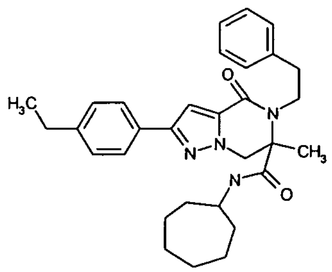 | 498,67411 | 499 |
| 103 | 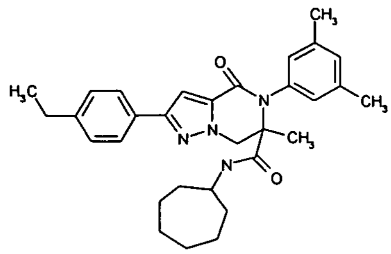 | 498,67411 | 499 |

(continued)

| | Table 1.05(3) | | |
|---|---|---|---|
| № | Formula | Mol.w. | LC MS, m/z (M+1), 1H NMR |
| 104 | 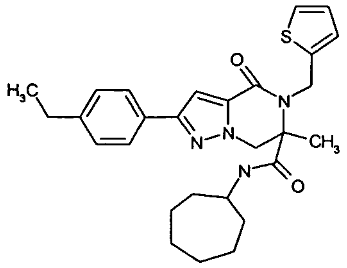 | 490,67278 | 491 |
| 105 | 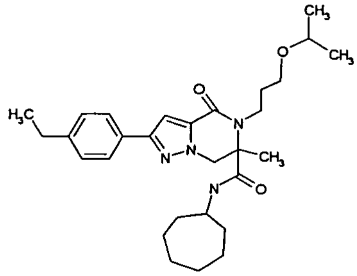 | 494,68309 | LC MS, m/z: 495 (M+1). 1 H NMR: 1.00-1.89 (m, 30H, Alk), 2.60-2.69 (q,2H, OCH2, J=6.7), 3.18-3.76 (m, 8H, Alk), 4.13-4.20, 4.80-4.89 (dd, 2H, NCH2, J=12.4), 6.97 (s, 1H, CH(pyrazol)), 7.13-7.19, 7.64-7.70 (dd, 4H, Ar, J=7.6), 7.53-7.60 (m, 1H, NH) |
| 106 | 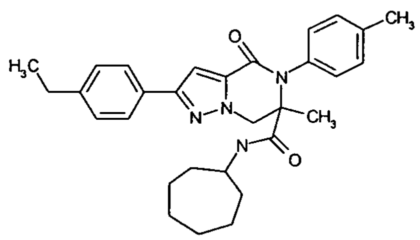 | 484,64702 | 485 |
| 107 | 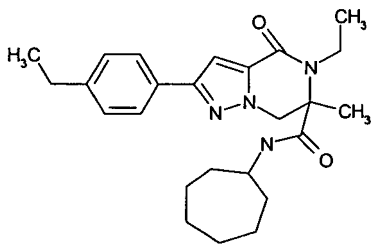 | 422,57533 | 423 |
| 108 | 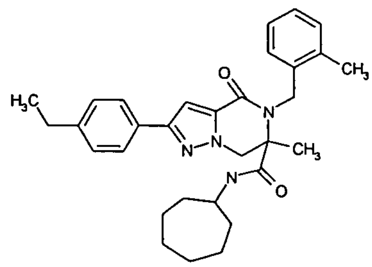 | 498,67411 | 499 |
| 109 | 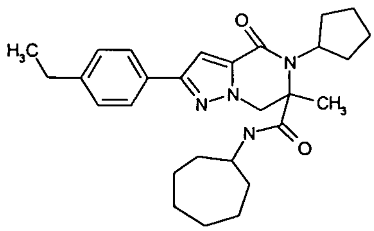 | 462,64066 | 463 |

(continued)

| | Table 1.05(3) | | |
|---|---|---|---|
| № | Formula | Mol.w. | LC MS, m/z (M+1), 1H NMR |
| 110 | 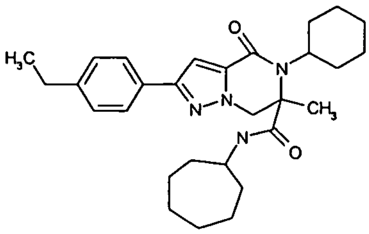 | 476,66775 | 477 |
| 111 | 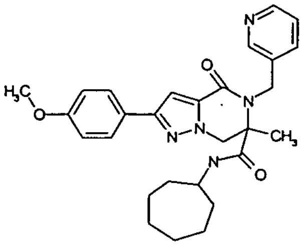 | 487,60691 | 488 |
| 112 | 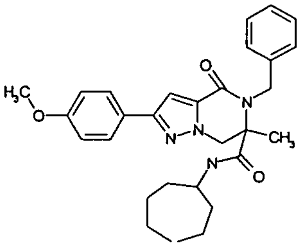 | 486,61933 | 487 |
| 113 | 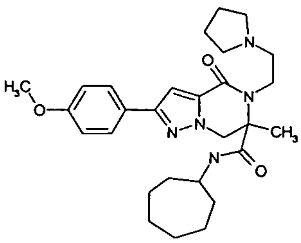 | 493,65473 | 494 |
| 114 | 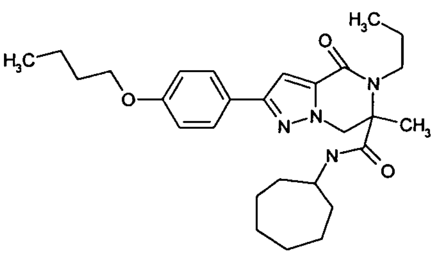 | 480,656 | 481 |
| 115 | 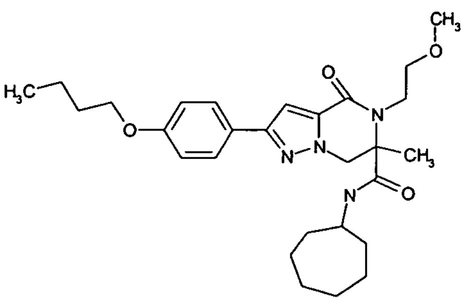 | 496,6554 | LC MS, m/z: 497 (M+1). 1H NMR: 0.96-1.82 (m, 20H, Alk), 3.33 (s, 3H, OMe), 3.48-3.70 (m, 5H, Alk), 3.92-4.00 (m, 2H, OCH2), 4.10-4.18, 4.81-4.89 (dd, 2H, NCH2, J=13.3), 6.81-6.88, 7.63-7.70 (dd, 4H, Ar, J=7.8), 6.92 (s, 1 H, CH(pyrazol)), 7.51-7.57 (m, 1H, NH) |

(continued)

| | Table 1.05(3) | | |
|---|---|---|---|
| № | Formula | Mol.w. | LC MS, m/z (M+1), 1H NMR |
| 116 | 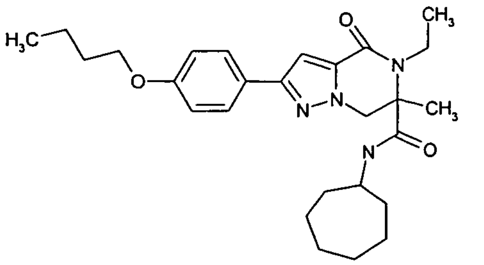 | 466,62891 | LC MS, m/z: 467 (M+1). 1H NMR: 0.90-1.82 (m, 30H, Alk), 3.20-3.32 (m, 1H, Alk), 3.53-3.66 (m, 1H, Alk), 3.66-3.75 (m, 1H, CHNH), 3.92-4.00 (m, 2H, OCH2), 4.10-4.85, 4.77-4.85 (dd, 2H, NCH2, J=13.9), 6.82-6.89 (d, 2H, Ar, J=8.2), 6.94 (s,1H, CH(pyrazol)), 7.62-7.73 (m, 3H, (Ar)NH)) |
| 117 | 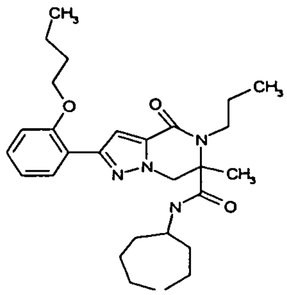 | 480,656 | LC MS, m/z: 481 (M+1). 1H NMR: 0.90-0.98 (m, 3H, CH3(Alk)), 1.00-1.06 (m, 3H, CH3 (Alk)), 1.33-1.73 (m, 24H, Alk), 1.83-1.93 (m, 2H, Alk), 3.03-3.13 (m, 1 H, Alk), 3.44-3.55 (m, 1H, Alk), 3.65-3.75 (m, 1H, NHCH), 4.04-4.11 (m, 2H, OCH2), 4.13-4.19, 4.81-4.87 (dd, 2H, NCH2, J=13.3), 6.87-6.96 (m, 2H, Ar), 7.14-7.24 (m, 2H, Ar). 7.52-7.57 (m. 1H. NH), 7.89-7.94 (d. |
| 118 | 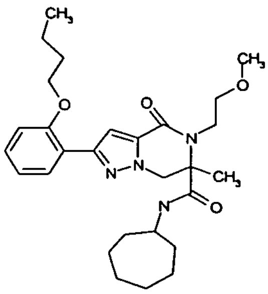 | 496,6554 | LC MS, m/z: 497 (M+1). 1H NMR: 1.00-1.08 (m, 3H, CH3(Alk)), 1.30-1.94 (m, 23H, Alk), 3.34 (s, 3H, OMe), 3.50-3.60 (m, 6H, Alk), 4.04-4.11 (m, 2H, OCH2), 4.13-4.20, 4.84-4.90 (dd, 2H, NCH2, J=12.6), 6.87-6.97 (m, 2H, Ar), 7.14-7.24 (m, 2H, Ar), 7.50-7.56 (m, 1H, NH), 7.89-7.95 (m, 1 H, Ar) |
| 119 | 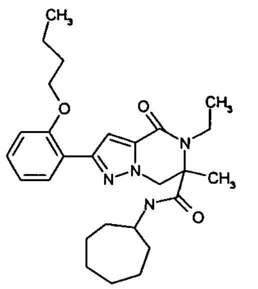 | 466,62891 | 467 |
| 120 | 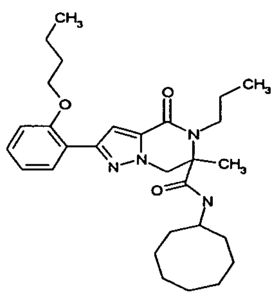 | 494,68309 | LC MS, m/z: 495 (M+1). 1H NMR: 0.90-098 (m, 3H, CH3(Alk)), 1.00-1.08 (m, 3H, Alk), 1.37-1.73 (m, 28H, Alk), 1.84-1.94 (m, 2H, Alk), 3.03-3.14 (m, 1 H, Alk), 3.46-3.57 (m, 2H, Alk), 3.72-3.82 (m, 1H, NHCH), 4.03-4.11 (m, 2H, OCH2), 4.13-4.21, 4.80-4.89 (dd, 2H, NCH2, J=12.2), 6.87-6.98 (m, 2H, Ar), 7.14-7.24 (m, 2H, Ar), 7.50-7.58 (m, 1H, NH), 7.89-7.95 (d,1H, Ar, J=8.1) |

(continued)

| | Table 1.05(3) | | |
|---|---|---|---|
| № | Formula | Mol.w. | LC MS, m/z (M+1), 1H NMR |
| 121 | 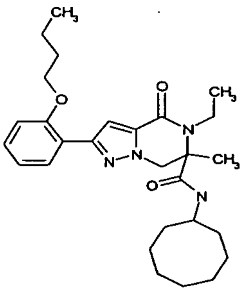 | 480,656 | LC MS, m/z: 481 (M+1). 1H NMR: 1.00-1.06 (m, 3H, CH3(Alk)), 1.20-1.27 (m, 2H, Alk), 1.40-1.69 (m, 21 H, Alk), 1.83-1.93 (m, 2H, Alk), 3.22-3.32 (m, 1H, Alk), 3.56-3.65 (m, 1H, Alk), 3.73-3.82 (m, 1H, NHCH), 4.04-4.11 (m, 2H, OCH2), 4.14-4.21, 4.80-4.87 (dd, 2H, NCH2, J=13.1), 6.87-6.97 (m, 2H, Ar), 7.15-7.24 (m, 2H, Ar), 7.57-7.61 (m, 1H, NH), 7.90-7.94 (m, 1H, Ar) |
| 122 | 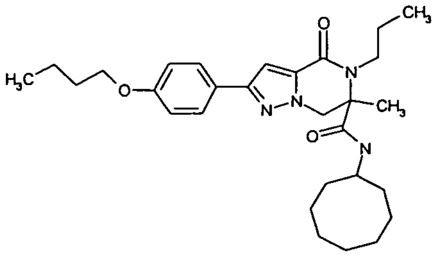 | 494,68309 | LC MS, m/z: 495 (M+1). 1 H NMR: 0.88-1.04 (m, 6H, Alk), 1.37-1.81 (m, 26H, Alk), 3.00-3.14 (m, 1H, Alk), 3.45-3.56 (m, 1H, Alk), 3.71-3.81 (m, 1H, CHNH), 3.92-4.00 (m, 2H, OCH2), 4.10-4.18, 4.79-4.87 (dd, 2H, NCH2, J=13.8), 6.80-6.87, 7.64-7.70 (dd, 4H, Ar, J=7.5), 6.93 (s, 1H, CH(pyrazol)), 7.52-7.59 (m, 1H, NH) |
| 123 | 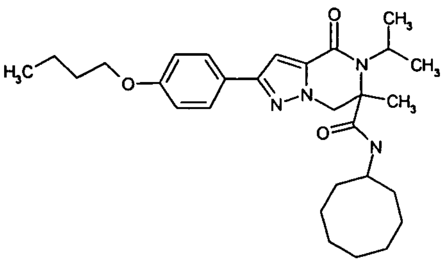 | 494,68309 | LC MS, m/z: 495 (M+1). 1H NMR: 0.95-1.04 (m, 3H, Alk), 1.34-1.82 (m, 30H, Alk), 3.17-3.28 (m, 1 H, Alk), 3.84-3.93 (m, 1 H, NHCH), 3.94-4.00 (m, 2H, OCH2), 4.05-4.13, 4.65-4.72 (dd,2H, NCH2, J=12.5), 6083-6089, 7.64-7.71 (dd, 4H, Ar, J=9.6), 6.94 (s, 1H, CH(pyrazol)), 8.06-8.14 (m, 1H, NH) |
| 124 | 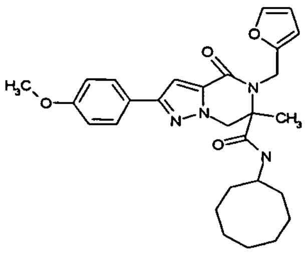 | 490,60758 | 491 |
| 125 | 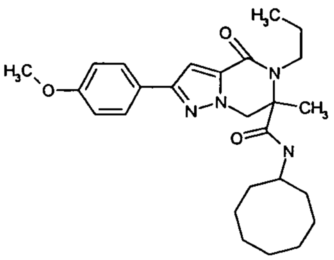 | 452,60182 | 453 |

(continued)

| | Table 1.05(3) | | |
|---|---|---|---|
| № | Formula | Mol.w. | LC MS, m/z (M+1), 1H NMR |
| 126 | 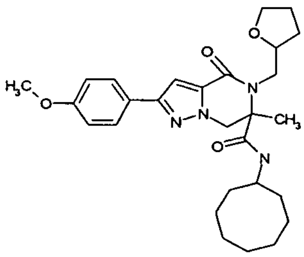 | 494,63946 | 495 |
| 127 | 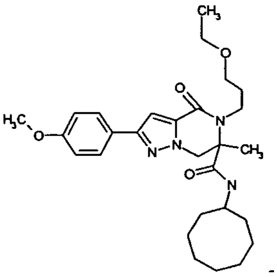 | 496,6554 | 497 |
| 128 | 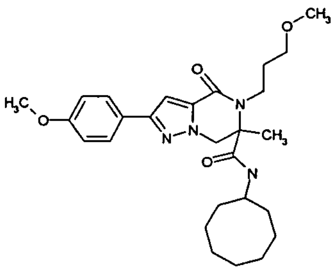 | 482,62831 | 483 |
| 129 | 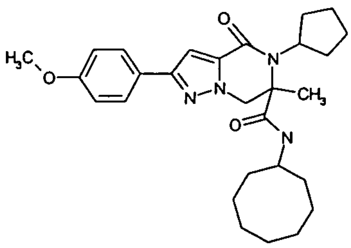 | 478,64006 | 479 |
| 130 | 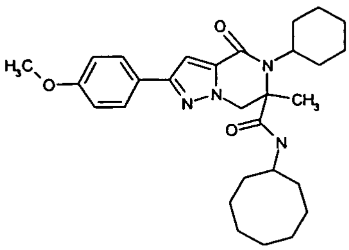 | 492,66715 | 493 |

(continued)

| | Table 1.05(3) | | |
|---|---|---|---|
| № | Formula | Mol.w. | LC MS, m/z (M+1), 1H NMR |
| 131 | 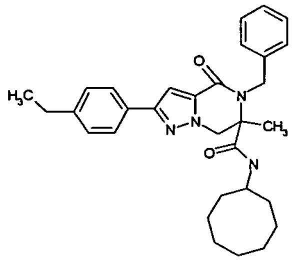 | 498,67411 | 499 |
| 132 | 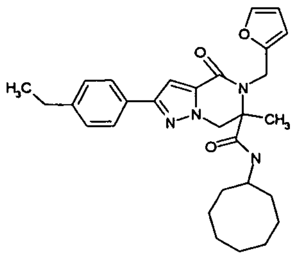 | 488,63527 | 489 |
| 133 | 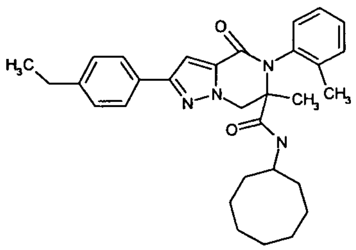 | 498,67411 | 499 |
| 134 | 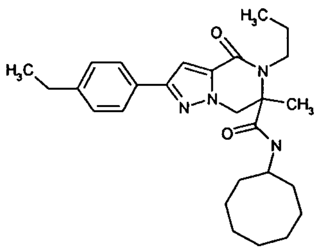 | 450,62951 | 451 |
| 135 | 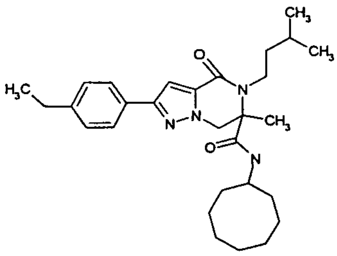 | 478,68369 | 479 |
| 136 | 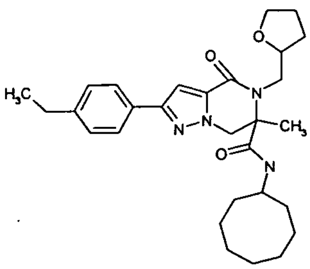 | 492,66715 | 493 |

(continued)

| | Table 1.05(3) | | |
|---|---|---|---|
| № | Formula | Mol.w. | LC MS, m/z (M+1), 1H NMR |
| 137 | 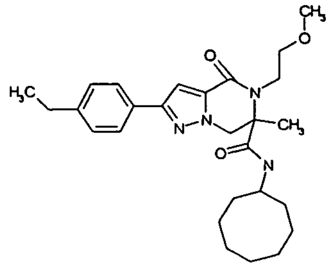 | 466,62891 | 467 |
| 138 | 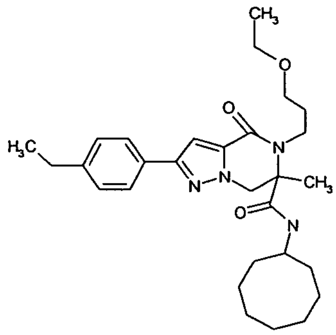 | 494,68309 | 495 |
| 139 | 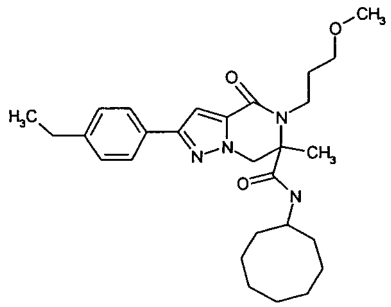 | 480,656 | 481 |
| 140 | 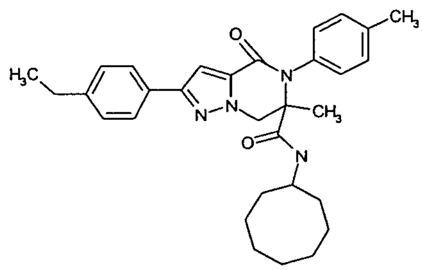 | 498,67411 | LC MS, m/z: 499 (M+1). 1H NMR: 1.22-1.68 (m, 22H, Alk), 2.39 (s, 3H, CH3(MePh)), 2.6-2.7 (m, 2H, CH2(EtPh)), 3.69-3.80 (m, 1H, NHCH), 4.38-4.46, 5.00-5.07 (dd,2H, NCH2, J=13.1), 7.01 (s, 1H, CH(pyrazol)), 7.13-7.27 (m, 6H, Ar), 7.62-7.79 (m, 3H, NH, Ar) |
| 141 | 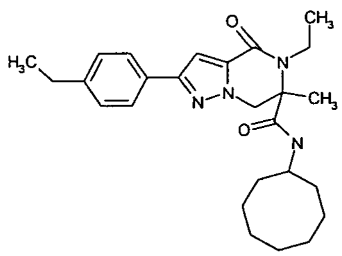 | 436,60242 | LC MS, m/z: 437 (M+1). 1H NMR: 1.15-1.68 (m, 30H, Alk), 2.59-2.69 (m, 2H, CH2,(EtPh)), 2.87-2.98 (m, 4H, (H2O+Alk)), 3.20-3.30 (m, 1H, Alk), 3.55-3.66 (m, 1H, Alk), 3.73-3.82 (m, 1H, NHCH), 4.12-4.21, 4.79-4.87 (dd, 2H, NCH2, J=13.5), 6.98 (s, 1H, pyrazol), 7.13-7.22, 7.66-7.71 (dd, 4H, Ar, J=8.3), 7.6-7.65 (m, 1H, NH) |

(continued)

| | Table 1.05(3) | | |
|---|---|---|---|
| № | Formula | Mol.w. | LC MS, m/z (M+1), 1H NMR |
| 142 | 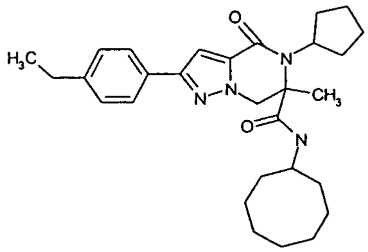 | 476,66775 | 477 |
| 143 | 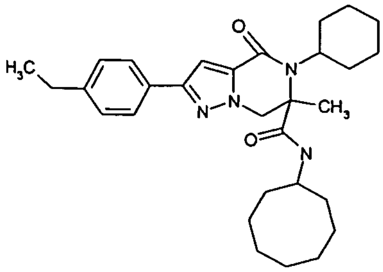 | 490,69484 | 491 |

| | Table 1.05(4) | | |
|---|---|---|---|
| № | Formula | Mol.w. | LC MS, m/z (M+1), 1H NMR |
| 1 | 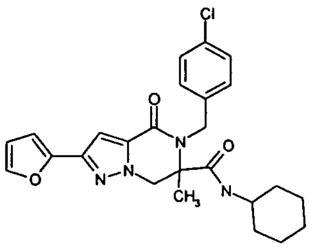 | 466,9719 | 467 |
| 2 | 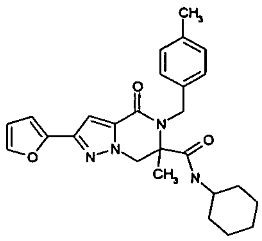 | 446,554 | 447 |
| 3 | 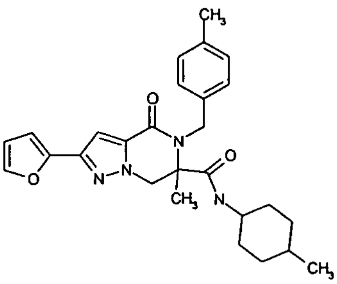 | 460,5811 | 461 |

(continued)

| | Table 1.05(4) | | |
|---|---|---|---|
| № | Formula | Mol.w. | LC MS, m/z (M+1), 1H NMR |
| 4 | 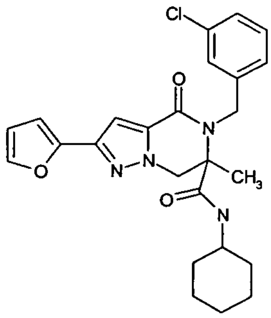 | 466,9719 | LC MS, m/z: 467 (M+1). 1H NMR:1.00-1.36 (m, 6H, Alk), 1.50-1.77 (m, 9H, Alk), 3.42-3.54 (br s, 1H, NHCH), 4.15-4.22, 5.23-5.33 (dd, 2H, CH2N, J=13.9), 4.25-4.33, 4.95-5.02 (dd,2H, CH2Ph, J=16.5), 6.46 (s, 1H, CH(pyrazol)), 6.67-6.71 (m, 1 H, CH(furan)), 6.94 (s, 1H, CH(furan)), 7.17-7.31 (m, 4H, 4CH(ClPh)), 7.35 (s, 1H, CH(ClPh)), 7.50 (s, 1H, CH(furan)), 7.52-7.57 (m, 1H, NH) |
| 5 | 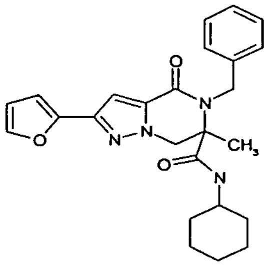 | 432,5269 | 433 |
| 6 | 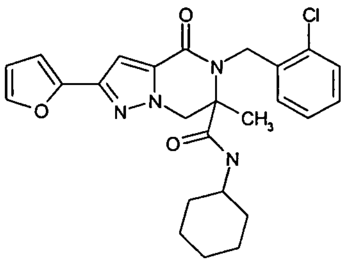 | 466,9719 | 467 |
| 7 | 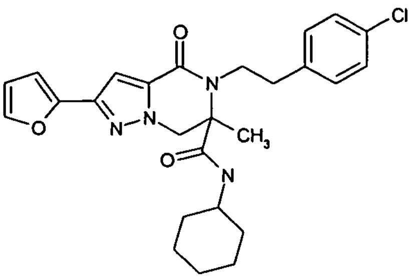 | 480,999 | 481 |
| 8 | 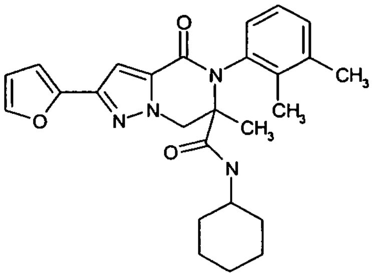 | 446,554 | LC MS, m/z: 447 (M+1). 1H NMR:0.91-1.35 (m, 11H, Alk), 1.55-1.75 (m, 10H, Alk), 2.65 (q, 2H, CH2(p-EtPh), J=7.4)), 3.05-3.16 (m, 1H, CONHCH), 3.44-3.58 (m, 2H, Alk), 4.13-4.20, 4.81-4.86 (dd, 2H, CH2-cycle, J=12.8), 6.98 (s, 1H, CH(pyrazol)), 7.14-7.19, 7.65-7.68 (dd, 4H, 4CH (p-EtP... |

(continued)

| | Table 1.05(4) | | |
|---|---|---|---|
| № | Formula | Mol.w. | LC MS, m/z (M+1), 1H NMR |
| 9 | 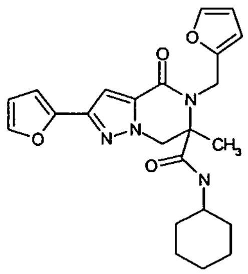 | 422,4881 | 422 |
| 10 | 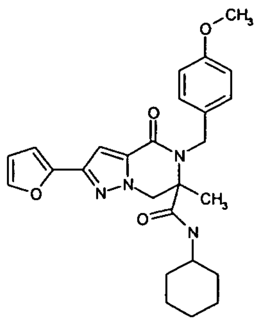 | 462,5534 | LC MS, m/z: 463 (M+1). 1H NMR:1.71-1.94 (m, 15H, Alk), 3.39-3.50 (m, 1H, NHCH), 3.76 (s, 3H, OMe), 4.09-4.15, 5.18 5.26 (dd, 2H, NCH2, J=14.2), 4.15-4.21,4.88-4.96 (dd, 2H, CH2Ph, J=16.9), 6.44 (s, 1 H, CH(pyrazol)), 6.45-6.68 (m, 1 H, CH(furan)), 6.76-6.82, 7.2-7.25 (dd, 4H, 4CH (p-PhOMe)), 6.90 (s, 1H, CH(furan)), 7.32-7.36 (m, 1H, NH), 7.48 (s, 1H, CH(furan)) |
| 11 | 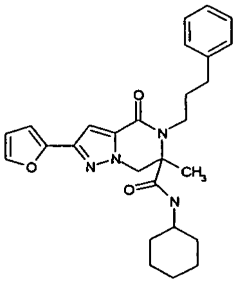 | 460,5811 | 461 |
| 12 | 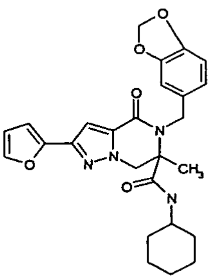 | 476,5369 | 477 |
| 13 | 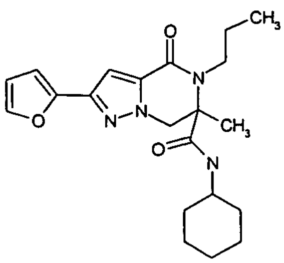 | 384,4823 | LC MS, m/z: 384 (M+1). 1 H NMR:0.89-1.76 (m, 21 H, Alk), 3.04-3.15 (m, 1H, Alk), 3.45-3.57 (m, 2H, Alk), 4.12-4.2, 4.79-4.86 (dd,2H, NCH2, J=13.8), 6.44 (br s, 1H, CH(pyrazol)), 6.62-6.66 (m, 1H, CH(furan)), 6.85 (s,1H, CH(furan)), 7.47 (s, 1H, CH(furan)), 7.49-7.56 (m, 1H, NH), |

(continued)

| | Table 1.05(4) | | |
|---|---|---|---|
| Nº | Formula | Mol.w. | LC MS, m/z (M+1), 1H NMR |
| 14 | 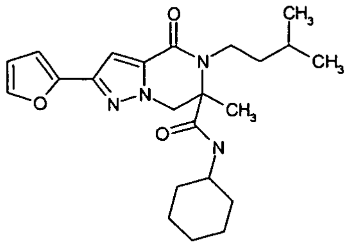 | 412,5365 | LC MS, m/z: 413 (M+1). 1H NMR:092-1.77 (m, 28H, Alk), 3.08-3.2 (m, 1H, Alk), 3.47-3.63 (m, 2H, Alk), 4.11-4.18, 4.8-4.87 (dd,2H,NCH2, J=14.2), 6.44 (s,1H, CH(pyrazol)), 6.62-6.67 (m, 1 H, CH (furan)), 6.84 (s, 1H, CH(furan)), 7.47 (s,1H, CH (furan)), 7.5-7.56 (m, 1H, NH) |
| 15 | 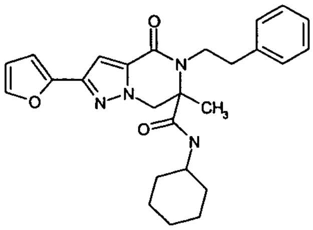 | 446,554 | 447 |
| 16 | 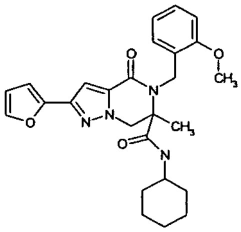 | 462,5534 | 463 |
| 17 | 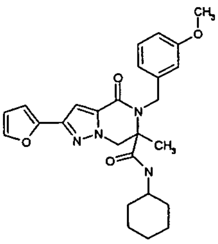 | 462,5534 | 463 |
| 18 | 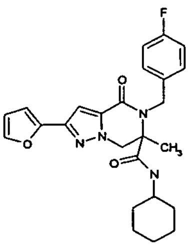 | 450,5173 | 451 |
| 19 | 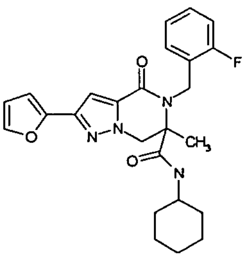 | 450,5173 | 451 |

(continued)

| | Table 1.05(4) | | |
|---|---|---|---|
| № | Formula | Mol.w. | LC MS, m/z (M+1), 1H NMR |
| 20 | 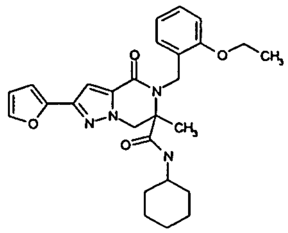 | 476,5805 | 477 |
| 21 | 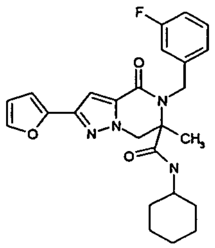 | 450,5173 | 451 |
| 22 | 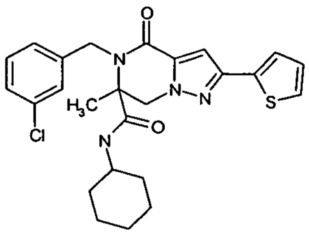 | 483,0365 | 484 |
| 23 | 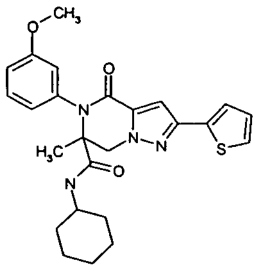 | 464,5909 | 465 |
| 24 | 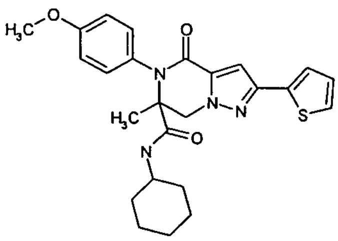 | 464,5909 | 465 |
| 25 | 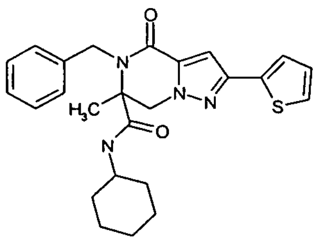 | 448,5915 | 449 |

(continued)

| | Table 1.05(4) | | |
|---|---|---|---|
| № | Formula | Mol.w. | LC MS, m/z (M+1), 1H NMR |
| 26 | 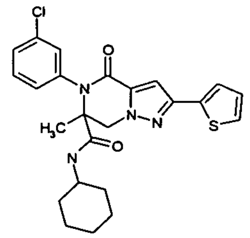 | 469,0095 | 470 |
| 27 | 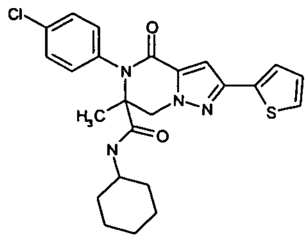 | 469,0095 | 470 |
| 28 | 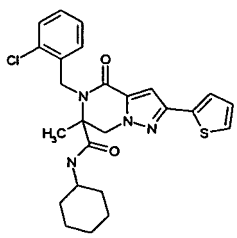 | 483,0365 | 484 |
| 29 | 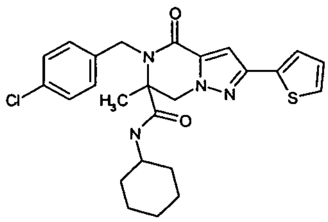 | 483,0365 | 484 |
| 30 | 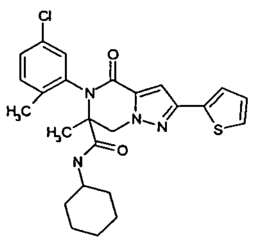 | 483,0365 | 484 |
| 31 | 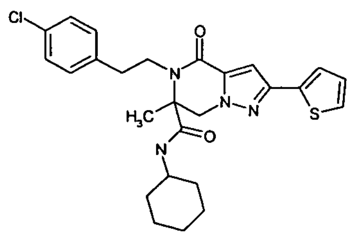 | 497,0636 | 498 |

(continued)

| | Table 1.05(4) | | |
|---|---|---|---|
| № | Formula | Mol.w. | LC MS, m/z (M+1), 1H NMR |
| 32 | 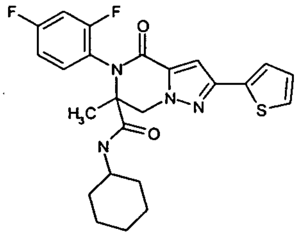 | 470,5453 | 471 |
| 33 | 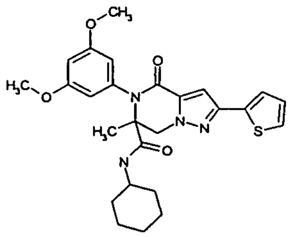 | 494,6174 | 495 |
| 34 | 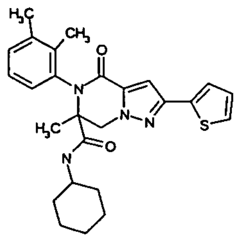 | 462,6186 | 463 |
| 35 | 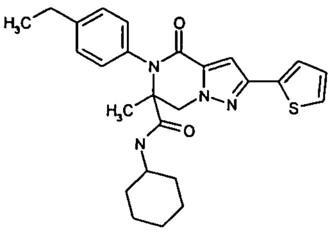 | 462,6186 | 463 |
| 36 | 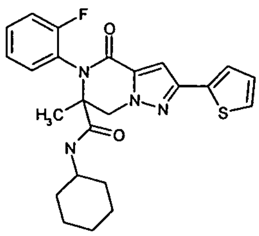 | 452,5549 | 453 |
| 37 | 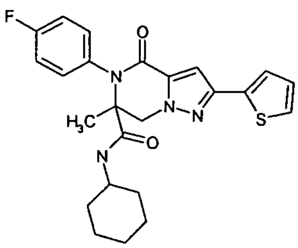 | 452,5549 | 453 |

(continued)

| | Table 1.05(4) | | |
|---|---|---|---|
| № | Formula | Mol.w. | LC MS, m/z (M+1), 1H NMR |
| 38 | 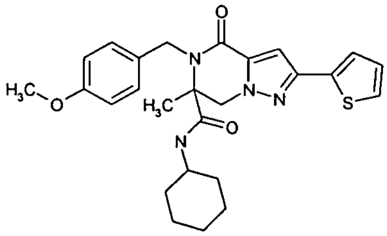 | 478,618 | 479 |
| 39 | 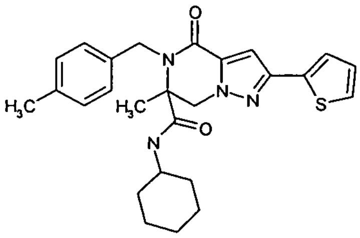 | 462,6186 | 463 |
| 40 | 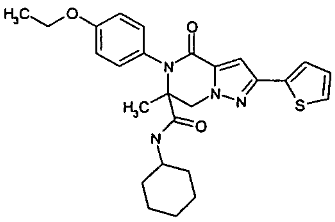 | 478,618 | 479 |
| 41 | 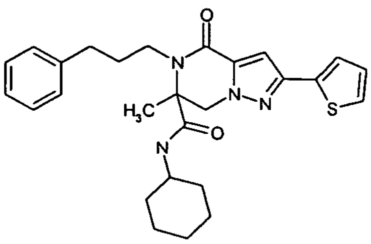 | 476,6457 | 477 |
| 42 | 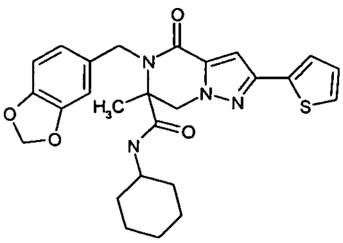 | 492,6015 | 493 |
| 43 | 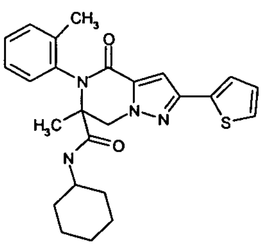 | 448,5915 | 449 |

(continued)

| | Table 1.05(4) | | |
|---|---|---|---|
| № | Formula | Mol.w. | LC MS, m/z (M+1), 1H NMR |
| 44 | 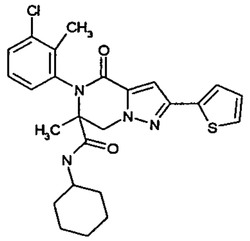 | 483,0365 | 484 |
| 45 | 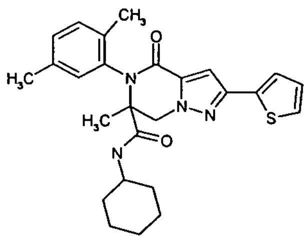 | 462,6186 | 463 |
| 46 | 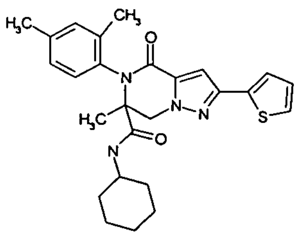 | 462,6186 | 463 |
| 47 | 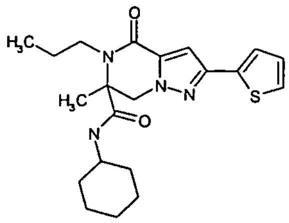 | 400,5469 | 401 |
| 48 | 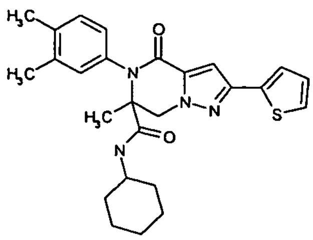 | 462,6186 | 463 |
| 49 | 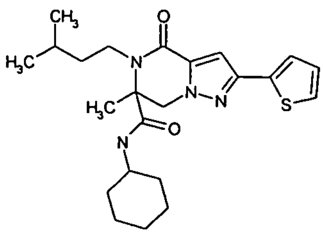 | 428,6011 | 429 |

(continued)

| | Table 1.05(4) | | |
|---|---|---|---|
| № | Formula | Mol.w. | LC MS, m/z (M+1), 1H NMR |
| 50 | 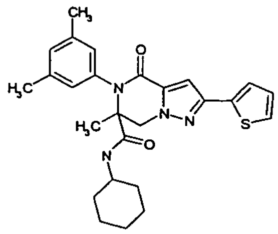 | 462,6186 | 463 |
| 51 | 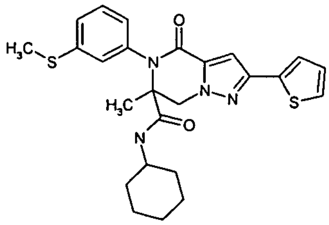 | 480,6555 | 481 |
| 52 | 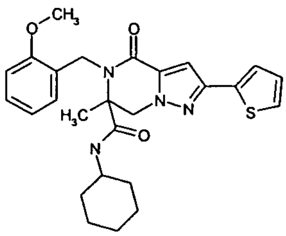 | 478,618 | 479 |
| 53 | 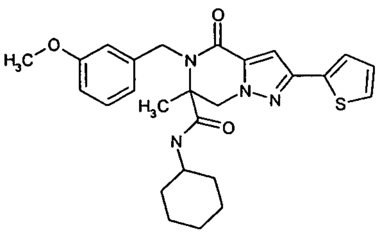 | 478,618 | 479 |
| 54 | 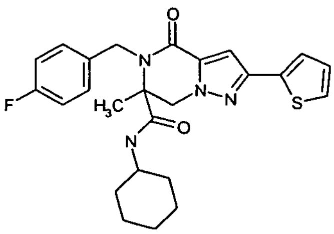 | 466,5819 | 467 |
| 55 | 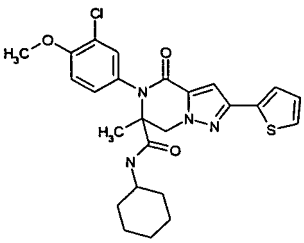 | 499,0359 | 500 |

(continued)

| | Table 1.05(4) | | |
|---|---|---|---|
| Nº | Formula | Mol.w. | LC MS, m/z (M+1), 1H NMR |
| 56 | 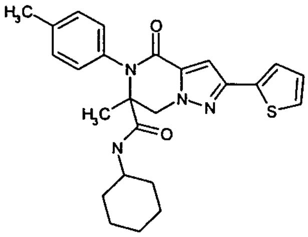 | 448, 5915 | 449 |
| 57 | 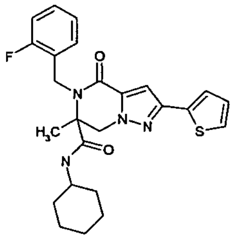 | 466,5819 | 467 |
| 58 | 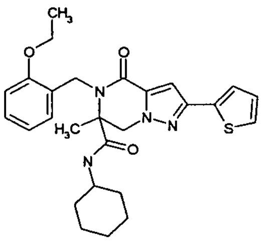 | 492,6451 | 493 |
| 59 | 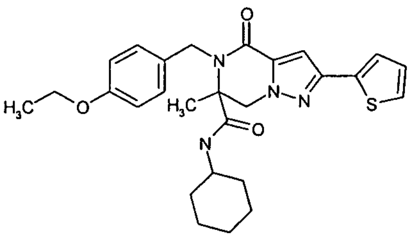 | 492,6451 | 493 |
| 60 | 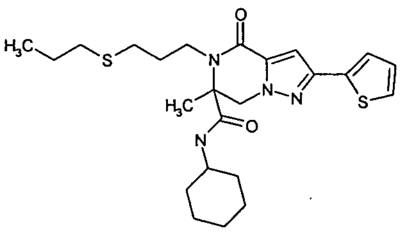 | 474,6922 | 475 |
| 61 | 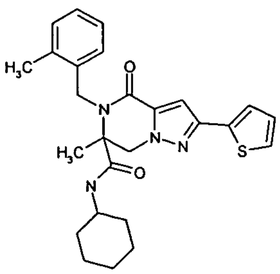 | 462,6186 | 463 |

(continued)

| | Table 1.05(4) | | |
|---|---|---|---|
| № | Formula | Mol.w. | LC MS, m/z (M+1), 1H NMR |
| 62 | 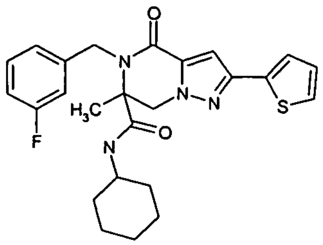 | 466,5819 | 467 |
| 63 | 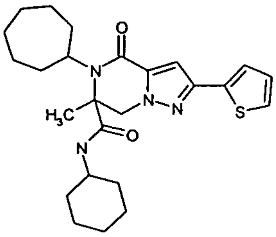 | 454,6393 | 455 |
| 64 | 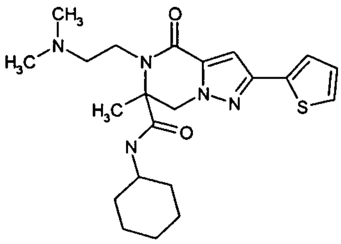 | 429,5887 | 430 |
| 65 | 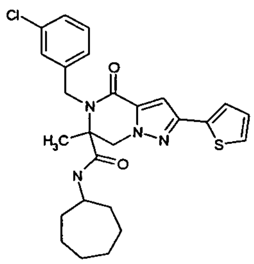 | 497,0636 | 498 |
| 66 | 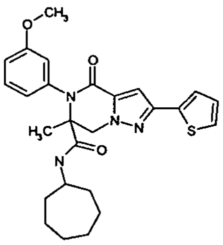 | 478,618 | 479 |
| 67 | 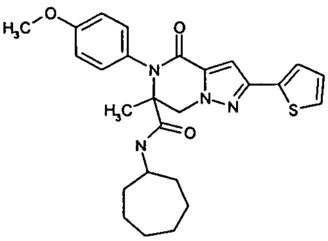 | 478,618 | 479 |

(continued)

| | Table 1.05(4) | | |
|---|---|---|---|
| № | Formula | Mol.w. | LC MS, m/z (M+1), 1H NMR |
| 68 | 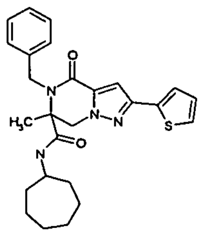 | 462,6186 | 463 |
| 69 | 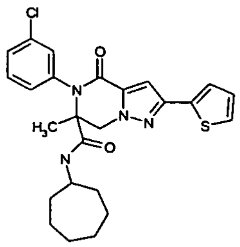 | 483,0365 | 484 |
| 70 | 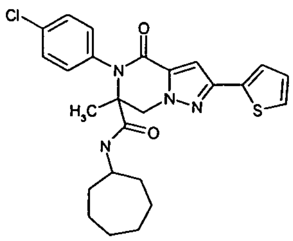 | 483,0365 | 484 |
| 71 | 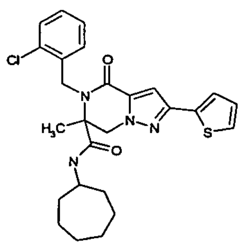 | 497,0636 | 484 |
| 72 | 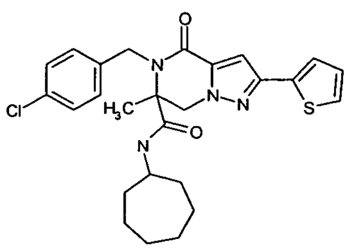 | 497,0636 | 484 |
| 73 | 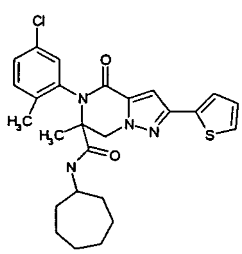 | 497,0636 | 498 |

(continued)

| | Table 1.05(4) | | |
|---|---|---|---|
| № | Formula | Mol.w. | LC MS, m/z (M+1), 1H NMR |
| 74 | 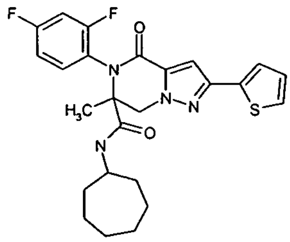 | 484,5724 | 485 |
| 75 | 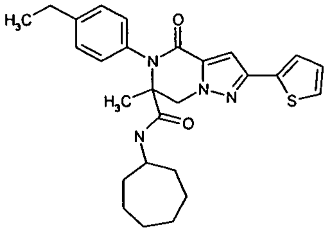 | 476,6457 | 477 |
| 76 | 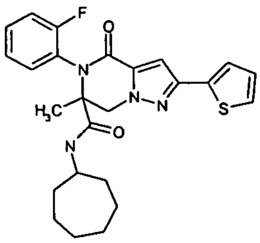 | 466,5819 | 467 |
| 77 | 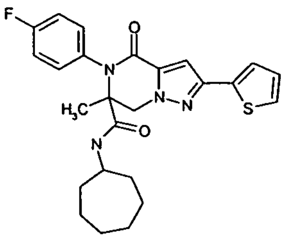 | 466,5819 | 467 |
| 78 | 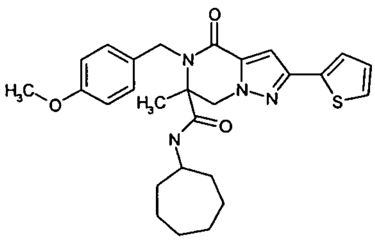 | 492,6451 | 493 |
| 79 | 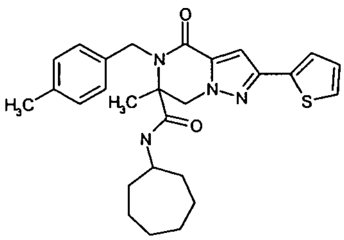 | 476,6457 | 477 |

(continued)

| | Table 1.05(4) | | |
|---|---|---|---|
| № | Formula | Mol.w. | LC MS, m/z (M+1), 1H NMR |
| 80 | 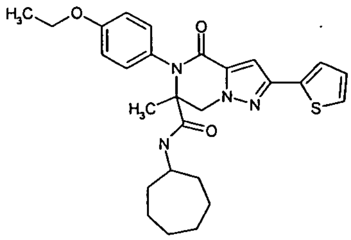 | 492,6451 | 493 |
| 81 | 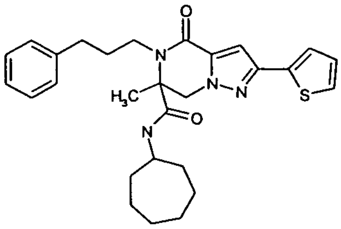 | 490,6728 | 491 |
| 82 | 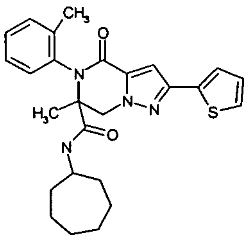 | 462,6186 | 463 |
| 83 | 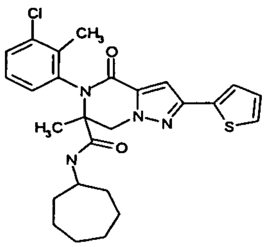 | 497,0636 | 498 |
| 84 | 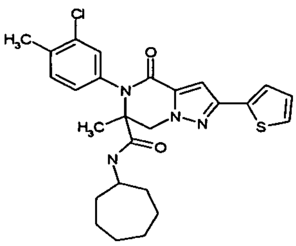 | 497,0636 | 498 |
| 85 | 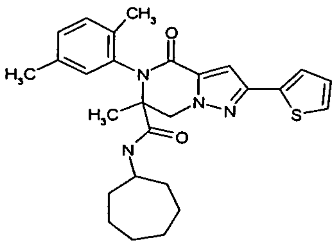 | 476,6457 | 477 |

(continued)

| | Table 1.05(4) | | |
|---|---|---|---|
| № | Formula | Mol.w. | LC MS, m/z (M+1), 1H NMR |
| 86 | 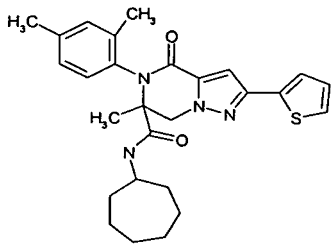 | 476,6457 | 477 |
| 87 | 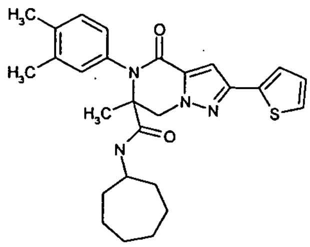 | 476,6457 | 477 |
| 88 | 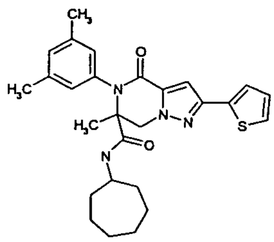 | 476,6457 | 477 |
| 89 | 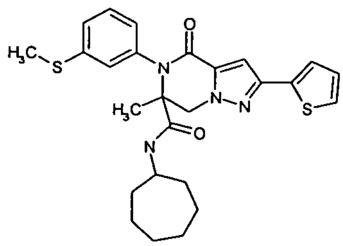 | 494,6826 | 495 |
| 90 | 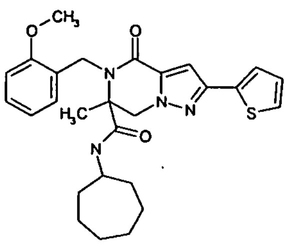 | 492,6451 | 493 |
| 91 | 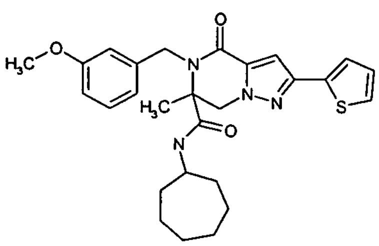 | 492,6451 | 493 |

(continued)

| | Table 1.05(4) | | |
|---|---|---|---|
| № | Formula | Mol.w. | LC MS, m/z (M+1), 1H NMR |
| 92 | 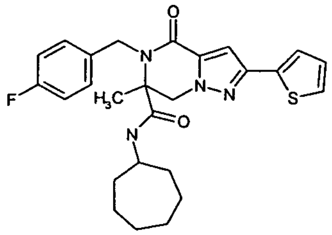 | 480,609 | 481 |
| 93 | 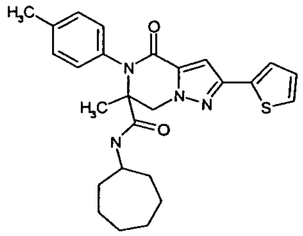 | 462,6186 | 463 |
| 94 | 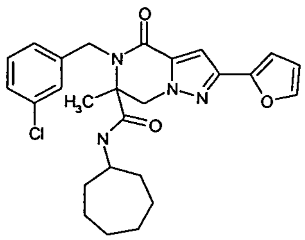 | 480,999 | 481 |
| 95 | 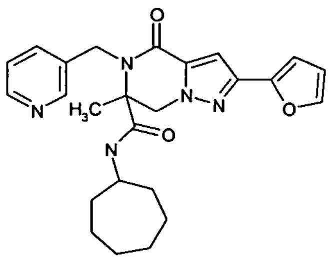 | 447,5416 | 448 |
| 96 | 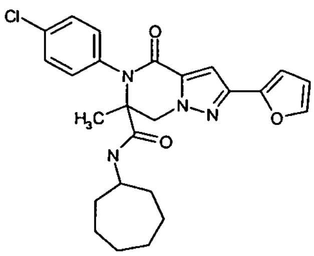 | 466,9719 | LC MS, m/z: 467 (M+1). 1H NMR:1.23-1.78 (m, 18H, Alk), 3.63-3.72 (m, 1H, NHCH), 4.45-4.51, 5.01-5.08 (dd, 2H, CH2N, J=14.4), 6.45-6.48 (m, 1H, CH(pyrazol)), 6.66-6.70 (m, 1H, CH(furan)), 6.91 (s, 1 H, CH(furan)), 7.36-7.44 (m, 4H, 4CH (ClPh)), 7.5 (s, 1H, CH(furan)), 7.74-7.79 (m, 1H, NH) |
| 97 | 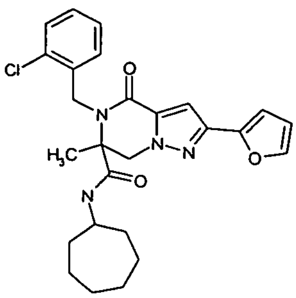 | 480,999 | LC MS, m/z: 481 (M+1). 1 H NMR:1.28-1.77 (m, 16H, Alk); 3.62-3.72 (m, 1H, CO-NH-CH); 4.19-4.27 , 5.22-5.30 (dd, 2H, N-CH2-Ph , I = 18); 4.33-4.4 , 4.99-5.06 (dd, 2H, CH2 (öèêë), I = 13.2); 6.46 (t, 1H, CH (furan)); 6.69 (d,1H, CH (furan)); 6.93 (s,1H, CH (pyrazol)); 7.17-7.26 (m, 2H, 2CH (-o-ClPh)); 7.32-7.396 (m, 2H, 2CH (-o-ClPh)); 7.50 (s, 1H, (furan)); 7.7 (d, 1H, CONH) |

(continued)

| | Table 1.05(4) | | |
|---|---|---|---|
| № | Formula | Mol.w. | LC MS, m/z (M+1), 1H NMR |
| 98 | 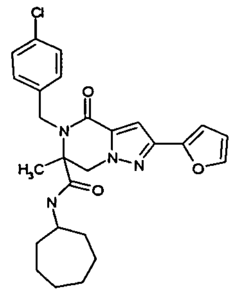 | 480,999 | 481 |
| 99 | 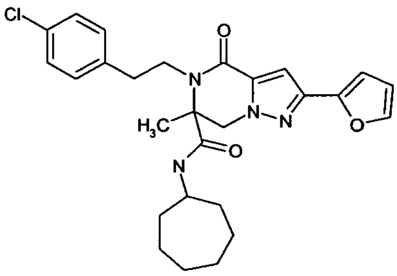 | 495,0261 | 496 |
| 100 | 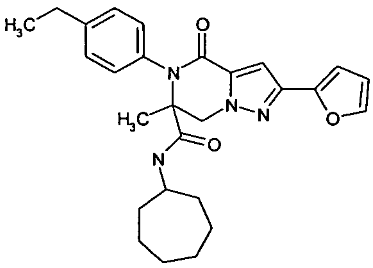 | 460,5811 | LC MS, m/z: 461 (M+1). 1H NMR:1.22-1.78 (m, 21 H, Alk), 2.69 (q, 2H, CH2(p-EtPh)), 3.62-3.74 (m, 1H, NHCH), 4.38-4.46, 4.96-5.04 (dd, 2H, NCH2, J=13.6), 6.45 (s, 1 H, CH(pyrazol)), 6.64-6.68 (m, 1H, CH(furan)), 6.19 (s, 1 H< CH (furan)), 7.16-7.22, 7.22-7.27 (dd, 4H, 4CH(p-EtPh), J=8.1)), 7.48 (s,1H, CH(furan)), 7.63-769 (m, 1H, NH) |
| 101 | 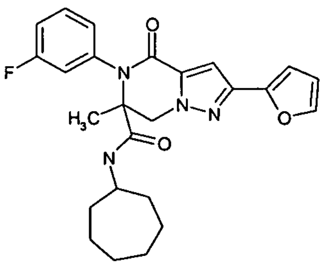 | 450,5173 | 451 |
| 102 | 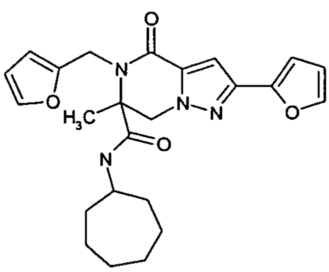 | 436,5152 | 437 |

(continued)

| | Table 1.05(4) | | |
|---|---|---|---|
| № | Formula | Mol.w. | LC MS, m/z (M+1), 1H NMR |
| 103 | 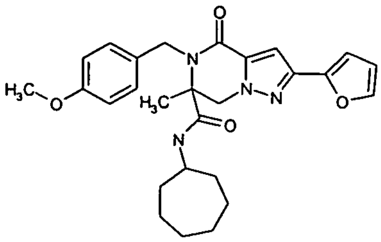 | 476,5805 | 477 |
| 104 | 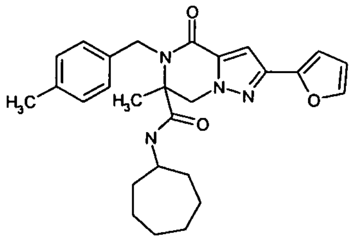 | 460,5811 | 461 |
| 105 | 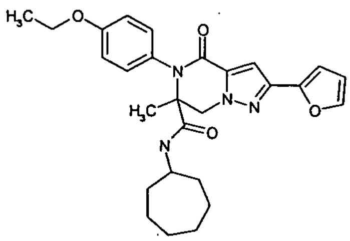 | 476,5805 | 477 |
| 106 | 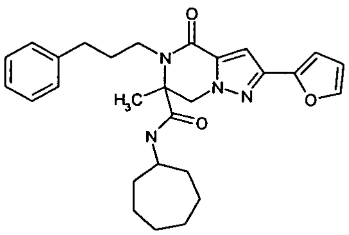 | 474,6082 | 475 |
| 107 | 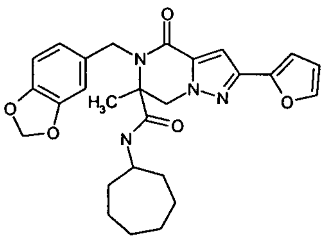 | 490,564 | 491 |
| 108 | 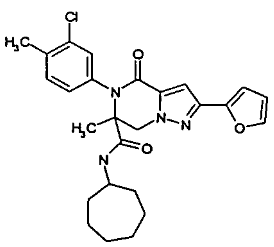 | 480,999 | 481 |

(continued)

| | Table 1.05(4) | | |
|---|---|---|---|
| № | Formula | Mol.w. | LC MS, m/z (M+1), 1H NMR |
| 109 | 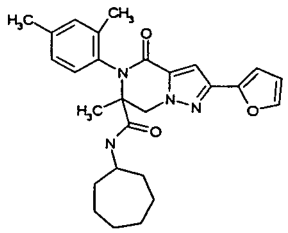 | 460,5811 | 461 |
| 110 | 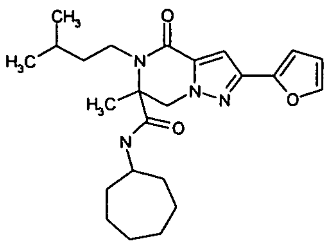 | 426,5636 | 427 |
| 111 | 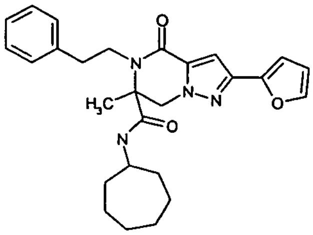 | 460,5811 | 461 |
| 112 | 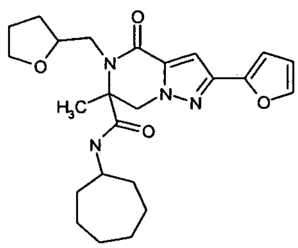 | 440,547 | 441 |
| 113 | 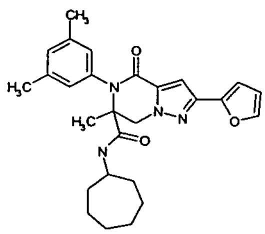 | 460,5811 | 461 |
| 114 | 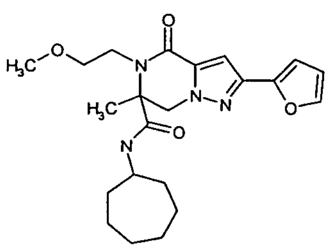 | 414,5088 | 415 |

(continued)

| | Table 1.05(4) | | |
|---|---|---|---|
| № | Formula | Mol.w. | LC MS, m/z (M+1), 1H NMR |
| 115 | 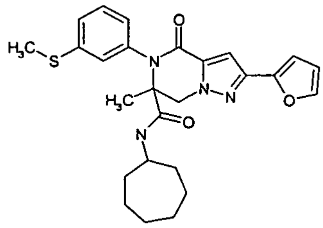 | 478,618 | 479 |
| 116 | 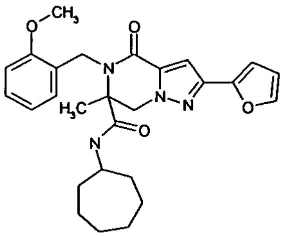 | 476,5805 | 477 |
| 117 | 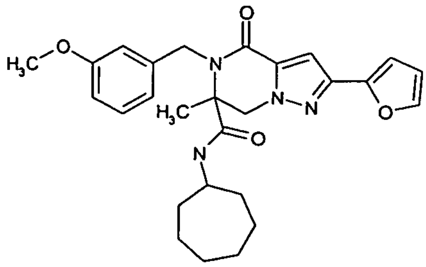 | 476,5805 | LC MS, m/z: 477 (M+1). 1H NMR:1.22-1.72 (m, 15H, Alk); 3.58-3.67 (m,1H, CO-NH-CH); 3.77 (s, 3H, CH3-O); 4.1-4.17 , 5.25-5.32 (dd,2H, N-CH2-Ph, I = 17.8); 4.21-4.28 , 4.92-4.98 (dd, 2H,CH2 (öèêë), I = 13.5); 6.46 (t, 1 H, CH (furan)); 6.67 (d, 1H,CH (furan)); 6.71 (d, 1 H, CH (-m-OMePh)); 6.91 (s,1H,CH (pyrazol)); 7.14-7.19 (m,1H, Ar); 7.5 (s, 1H, CO-NH) |
| 118 | 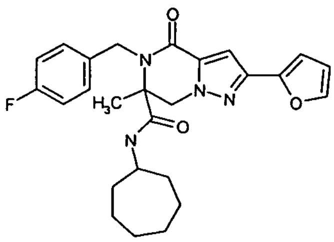 | 464,5444 | 465 |
| 119 | 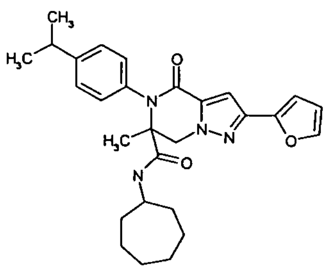 | 474,6082 | 475 |
| 120 | 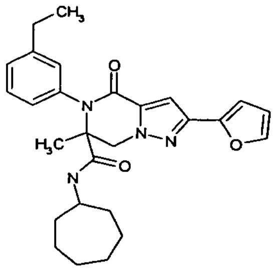 | 460,5811 | 461 |

(continued)

| | Table 1.05(4) | | |
|---|---|---|---|
| № | Formula | Mol.w. | LC MS, m/z (M+1), 1H NMR |
| 121 | 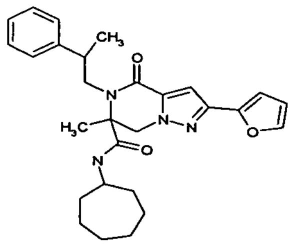 | 474,6082 | 475 |
| 122 | 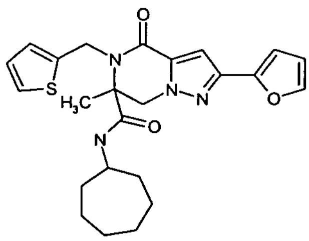 | 452,5798 | 453 |
| 123 | 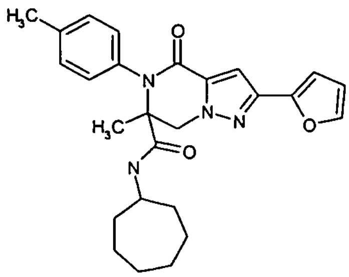 | 446,554 | 447 |
| 124 | 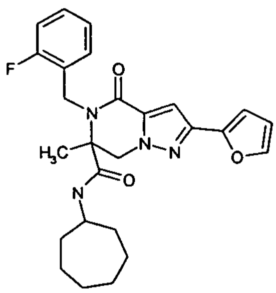 | 464,5444 | LC MS, m/z: 465 (M+1). 1 H NMR:1.25-1.75 (m, 15H, Alk), 3.60-3.70 (m, 1H, CONHCH), 4.27 (t, 2H, CH2Ph, J=16.5), 4.96-5.03, 5.21-5.29(dd, 2H, CH2-cycle, J=14.3), 6.44-6.47 (m, 1H,CH(furan)), 6.66 6.68 (m,1H, CH(furan)), 6.92 (s, 1 H, CH (pyrazol)), 6.99-7.13 (m, 2H, 2CH (o-FPh)), 7.18-7.67 (m, 4H, 2CH(o-FPh)), 1CH(furan), NH)) |
| 125 | 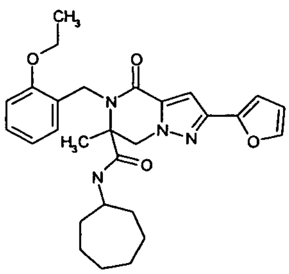 | 490,6076 | 491 |

(continued)

| | Table 1.05(4) | | |
|---|---|---|---|
| № | Formula | Mol.w. | LC MS, m/z (M+1), 1H NMR |
| 126 | 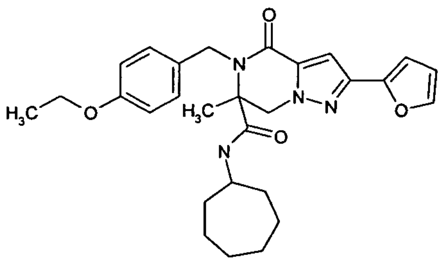 | 490,6076 | LC MS, m/z: 491 (M+1). 1H NMR:1.19-1.69 (m, 18H, Alk), 3.57-3.67 (m, 1 H, CONHCH), 3.99 (q, 2H, CH3CH2O, J=7.1), 4.12-4.20 (m, 2H,CH2Ph), 4.90-4.95, 5.16-5.25 (dd, 2H,CH2-cycle, J=13.5), 6.43-6.46 (m, 1 H, CH(furan)), 6.65-6.68 (m, 1H, CH(furan)), 6.75-6.80, 7.20-7.24 (dd,4H,4CH(p-OEtPh), J=8.5)), 6.91 (s, 1H, CH(pyrazol)), 7.36 (br, s, 1H, CH(furan)), 7.49 (s,1H, NH) |
| 127 | 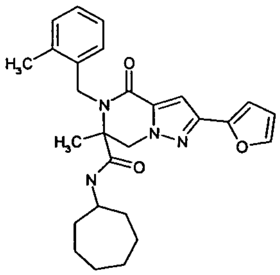 | 460,5811 | 461 |
| 128 | 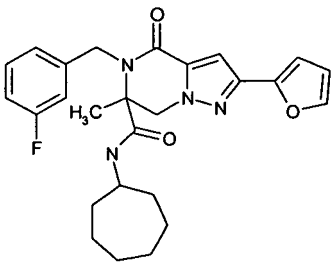 | 464,5444 | 465 |
| 129 | 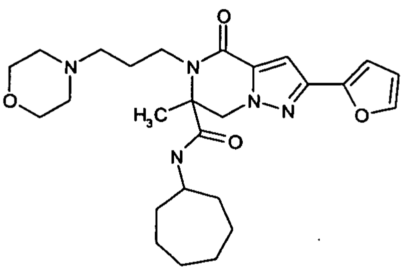 | 483,6159 | LC MS, m/z: 484 (M+1). 1H NMR:1.34-1.81 (m, 17H, Alk), 2.29-2.41 (m, 6H, Alk), 3.15-3.25 (m, 1H, CONHCH), 3.55-3.71 (m, 6H, Alk), 4.16-4.22, 4.80-4.86 (dd,2H, CH2-cycle), J=13.8)), 6.43-6.47 (m, 1H, CH(furan)), 6.64-6.66 (m,1H, CH(furan)), 6.82-6.84 (m, 1H, CH(pyrazol)), 7.49-7.51 (m, 1H, CH(furan)), 7.63-7.67 (m, 1H, NH) |
| 130 | 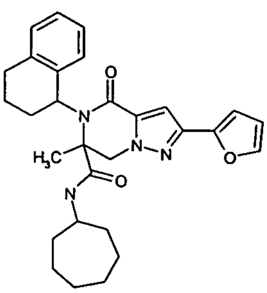 | 486,6193 | 487 |

(continued)

| | Table 1.05(4) | | |
|---|---|---|---|
| № | Formula | Mol.w. | LC MS, m/z (M+1), 1H NMR |
| 131 | 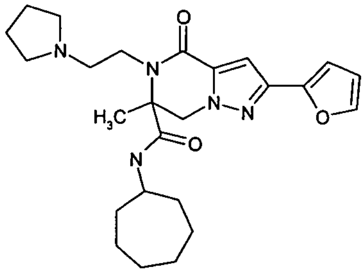 | 453,5894 | LC MS, m/z: 454 (M+1). 1H NMR:1.28-1.81 (m, 19H, Alk), 2.5 (s, 2H, NHCH2), 2.85-2.95 (m, 4H, Alk), 3.45-3.65 (m, 2H, Alk), 3.71-3.83 (m, 1 H, CONHCH), 4.10-4.17, 4.84-4.91 (dd, 2H, CH2-cycle, J=13.4), 6.41-6.45 (m, 1 H, CH(furan)), 6.62-6.66 (m, 1H, CH(furan)), 6.84 (s, 1H, CH (pyrazol)), 7.46 (br, s, 1 H, CH(furan)), 7.90-7.96 (m, 1H, NH) |
| 132 | 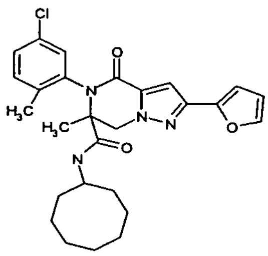 | 495,0261 | 496 |
| 133 | 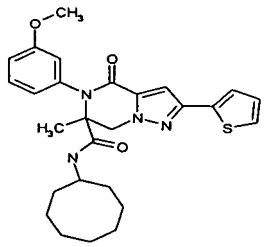 | 492,6451 | 493 |
| 134 | 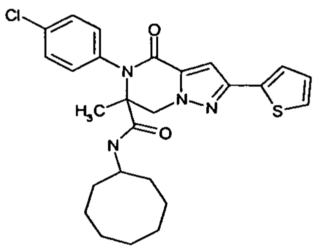 | 497,0636 | 498 |
| 135 | 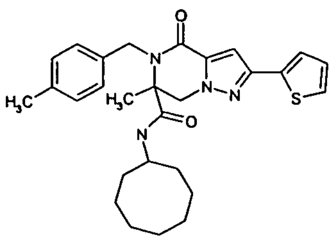 | 490,6728 | 491 |
| 136 | 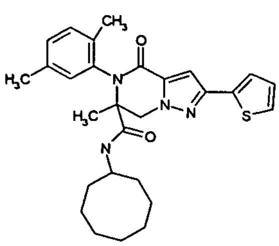 | 490,6728 | 491 |

(continued)

| | Table 1.05(4) | | |
|---|---|---|---|
| № | Formula | Mol.w. | LC MS, m/z (M+1), 1H NMR |
| 137 | 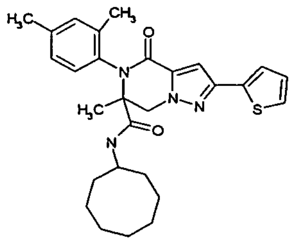 | 490,6728 | 491 |
| 138 | 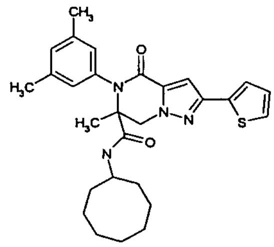 | 490,6728 | 491 |
| 139 | 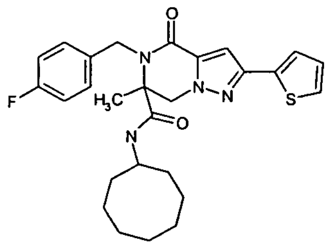 | 494,6361 | 495 |
| 140 | 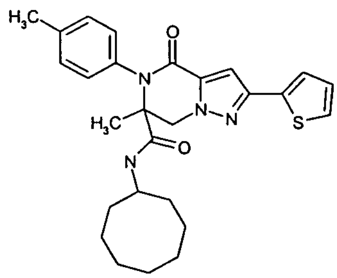 | 476,6457 | 477 |

Table 1.05(5)

| № | Formula | Mol.w. |
|---|---|---|
| 1 | 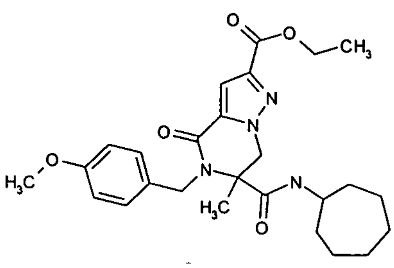 | 482,58468 |

(continued)

| № | Formula | Mol.w. |
|---|---|---|
| 2 | 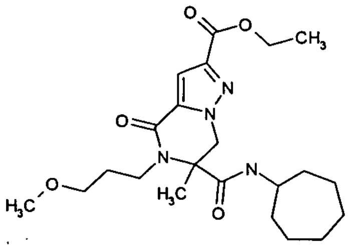 | 434,54008 |
| 3 | 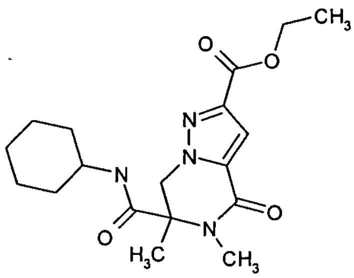 | 362,43232 |
| 4 | 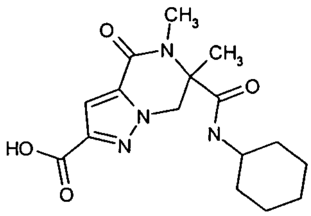 | 334,37814 |
| 5 | 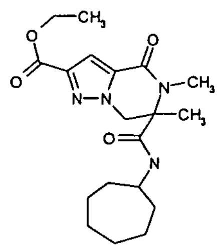 | 376,45941 |
| 6 | 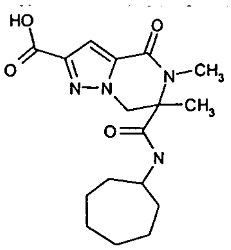 | 348,40523 |
| 7 | 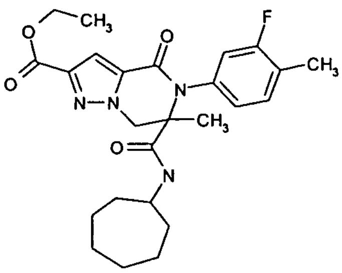 | 470,54862 |

(continued)

| № | Formula | Mol.w. |
|---|---|---|
| 8 | 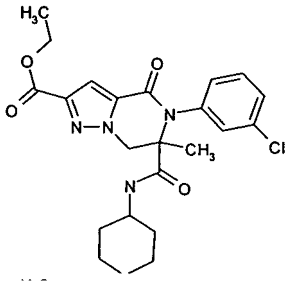 | 458,94904 |
| 9 | 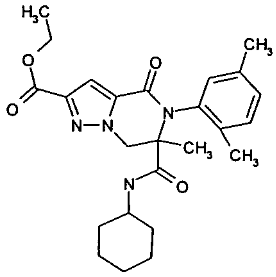 | 452,55819 |
| 10 | 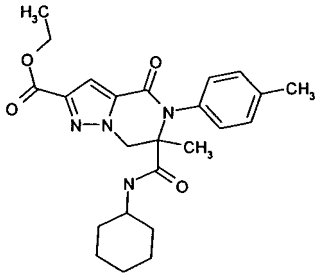 | 438,5311 |
| 11 | 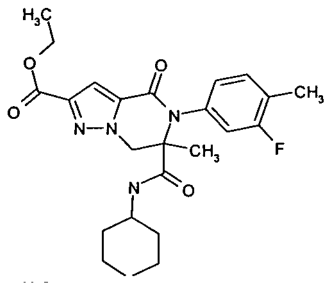 | 456,52153 |
| 12 | 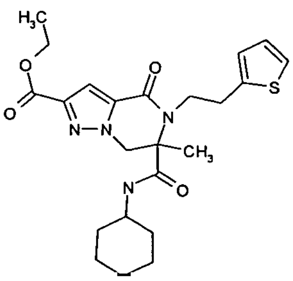 | 458,58395 |

(continued)

| № | Formula | Mol.w. |
|---|---|---|
| 13 | 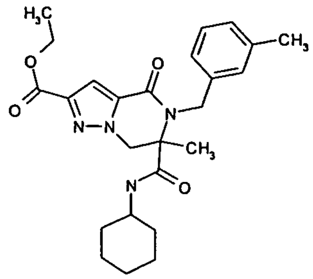 | 452,55819 |
| 14 | 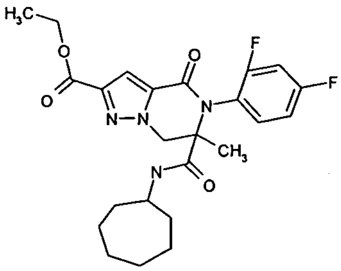 | 474,51196 |
| 15 | 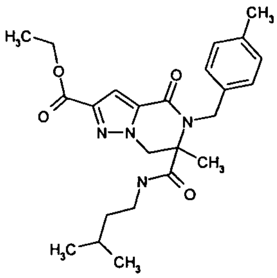 | 440,54704 |
| 16 | 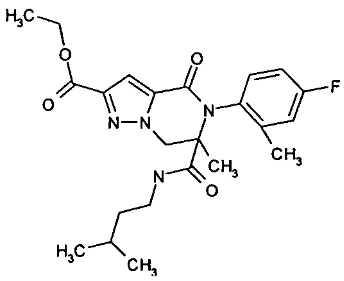 | 444,51038 |
| 17 | 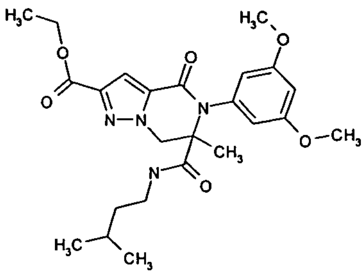 | 472,54584 |

(continued)

| № | Formula | Mol.w. |
|---|---|---|
| 18 | 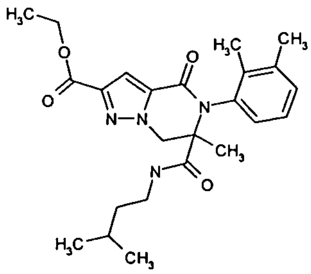 | 440,54704 |
| 19 | 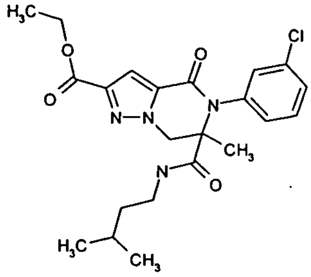 | 446,93789 |
| 20 | 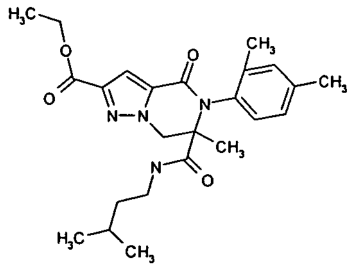 | 440,54704 |
| 21 | 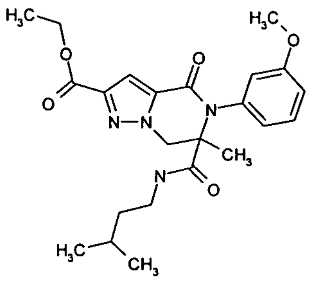 | 442,51935 |
| 22 | 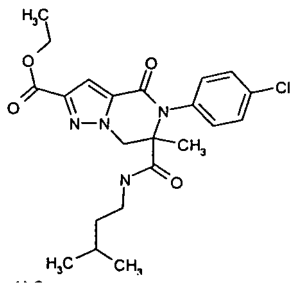 | 446,93789 |

(continued)

| № | Formula | Mol.w. |
|---|---|---|
| 23 | 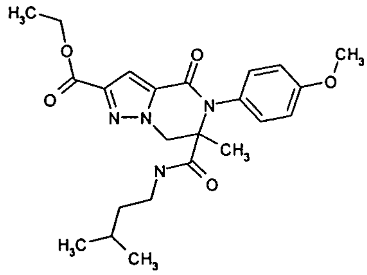 | 442,51935 |
| 24 | 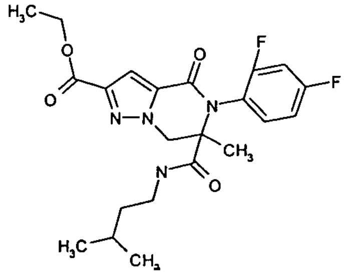 | 448,47372 |
| 25 | 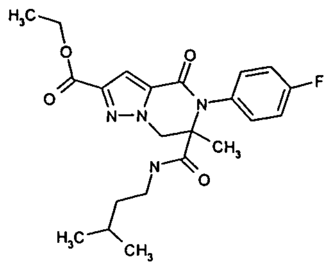 | 430,48329 |
| 26 | 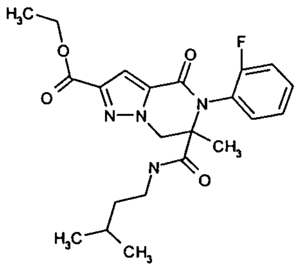 | 430,48329 |
| 27 | 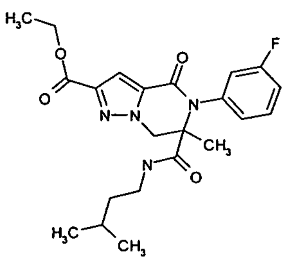 | 430,48329 |
| 28 | 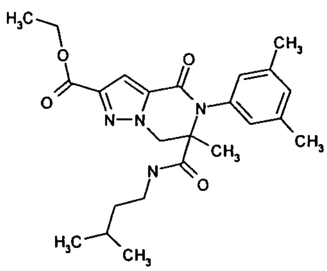 | 440,54704 |

(continued)

| № | Formula | Mol.w. |
|---|---|---|
| 29 | 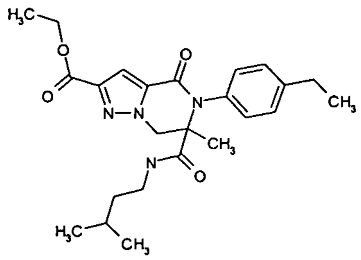 | 440,54704 |
| 30 | 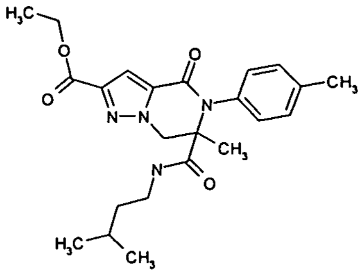 | 426,51995 |
| 31 | 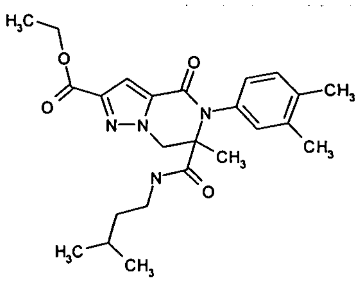 | 440,54704 |
| 32 | 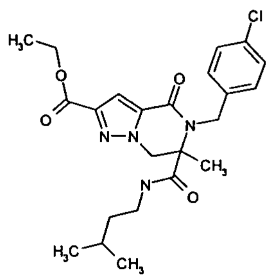 | 460,96498 |
| 33 | 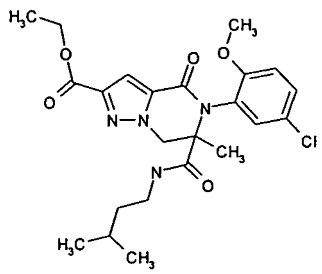 | 476,96438 |

(continued)

| № | Formula | Mol.w. |
|---|---|---|
| 34 | 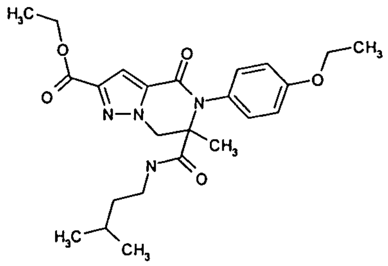 | 456,54644 |
| 35 | 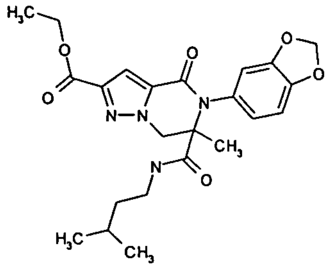 | 456,50281 |
| 36 | 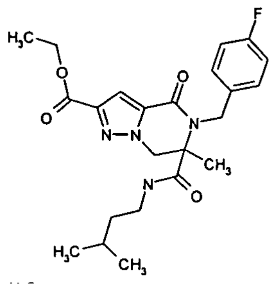 | 444,51038 |
| 37 | 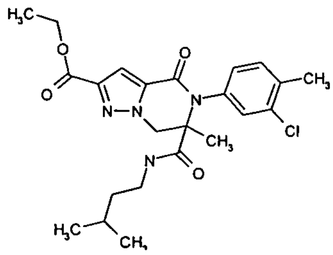 | 460,96498 |
| 38 | 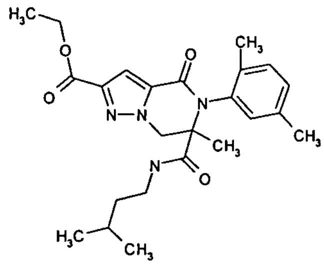 | 440,54704 |
| 39 | 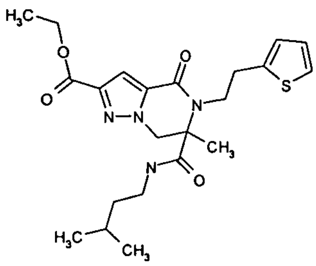 | 446,5728 |

(continued)

| № | Formula | Mol.w. |
|---|---|---|
| 40 | 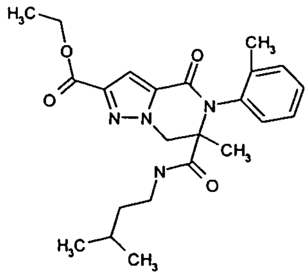 | 426,51995 |
| 41 | 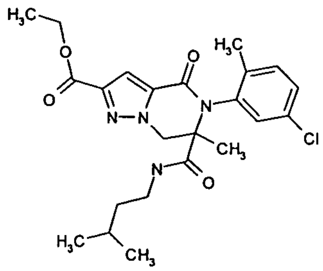 | 460,96498 |
| 42 | 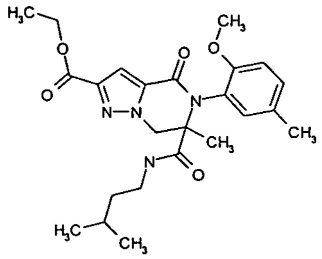 | 456,54644 |
| 43 | 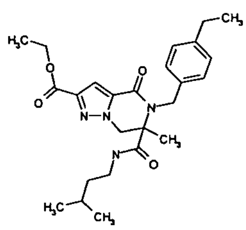 | 454,57413 |
| 44 | 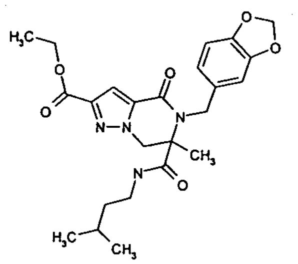 | 470,5299 |
| 45 | 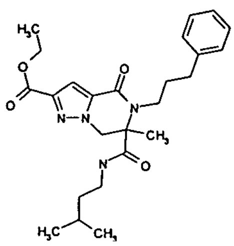 | 454,57413 |

(continued)

| № | Formula | Mol.w. |
|---|---|---|
| 46 | 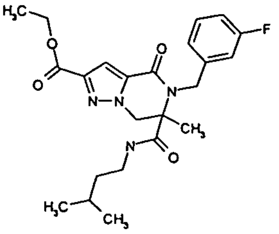 | 444,51038 |
| 47 | 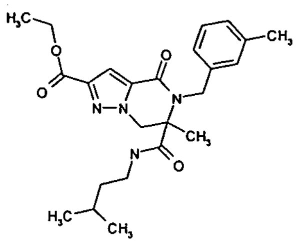 | 440,54704 |
| 48 | 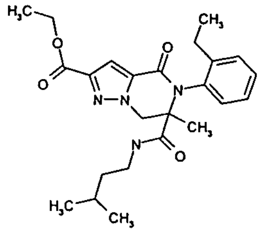 | 440,54704 |
| 49 | 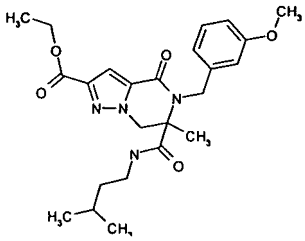 | 456,54644 |
| 50 | 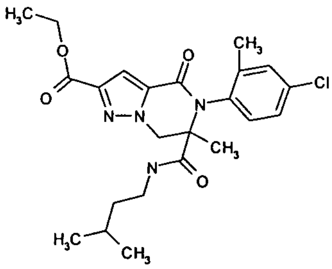 | 460,96498 |
| 51 | 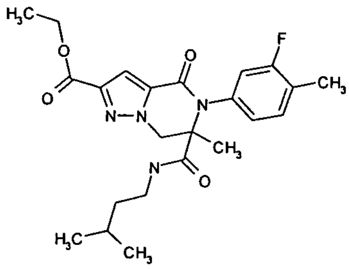 | 444,51038 |

(continued)

| № | Formula | Mol.w. |
|---|---|---|
| 52 | 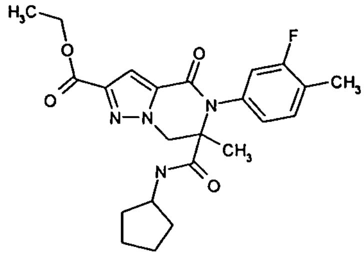 | 442,49444 |
| 53 | 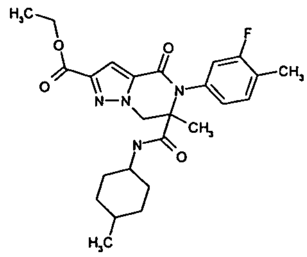 | 470,54862 |
| 54 | 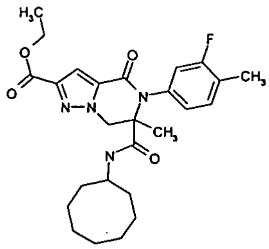 | 484,57571 |
| 55 | 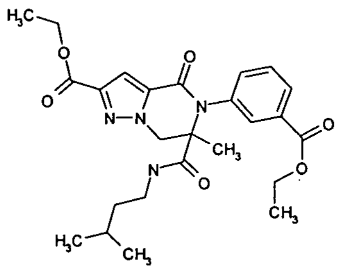 | 484,55699 |
| 56 | 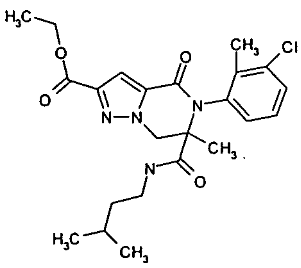 | 460,96498 |
| 57 | 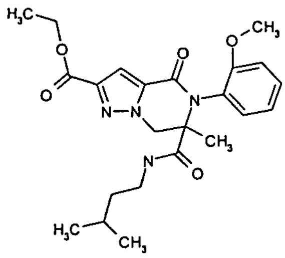 | 442,51935 |

(continued)

| № | Formula | Mol.w. |
|---|---|---|
| 58 | 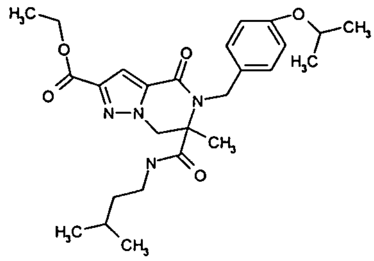 | 484,60062 |
| 59 | 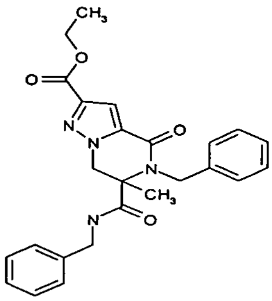 | 446,51037 |
| 60 | 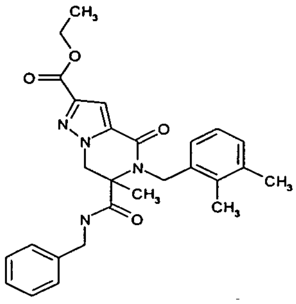 | 474,56455 |
| 61 | 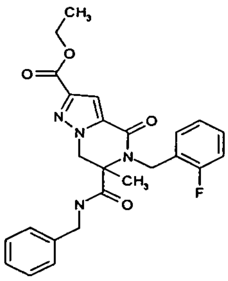 | 464,5008 |
| 62 | 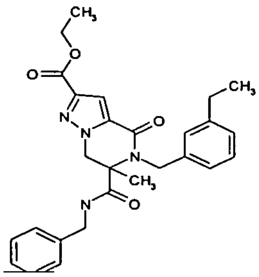 | 474,56455 |

(continued)

| № | Formula | Mol.w. |
|---|---|---|
| 63 | 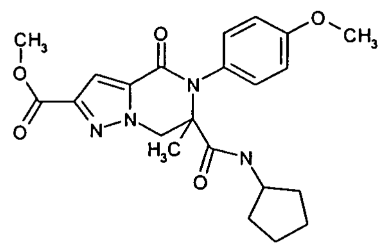 | 426,47632 |
| 64 | 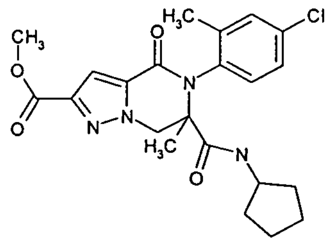 | 444,92195 |
| 65 | 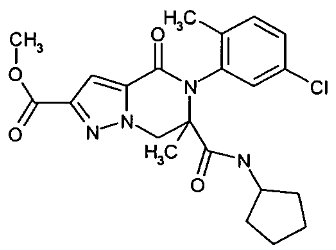 | 444,92195 |
| 66 | 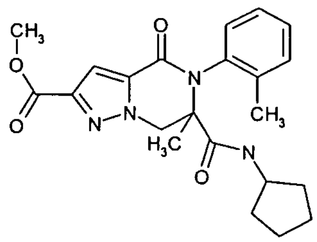 | 410,47692 |
| 67 | 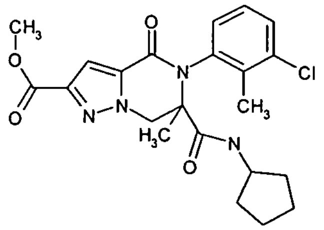 | 444,92195 |
| 68 | 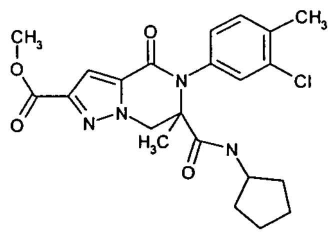 | 444,92195 |

(continued)

| № | Formula | Mol.w. |
|---|---|---|
| 69 | 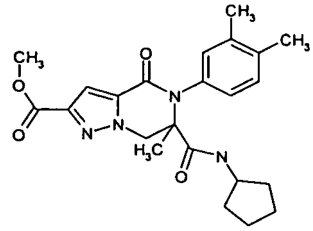 | 424,50401 |
| 70 | 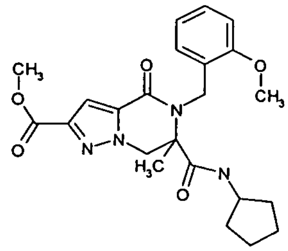 | 440,50341 |
| 71 | 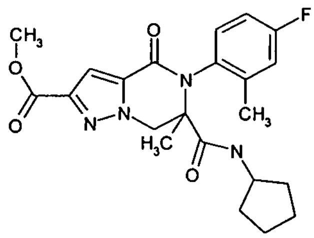 | 428,46735 |
| 72 | 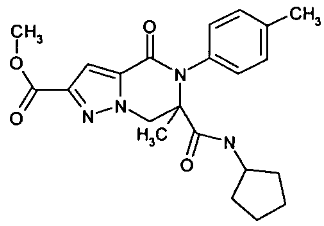 | 410,47692 |
| 73 | 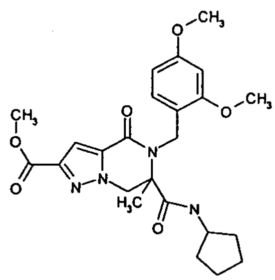 | 470,5299 |
| 74 | 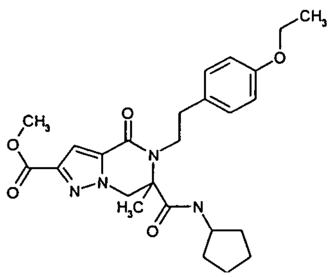 | 468,55759 |

(continued)

| № | Formula | Mol.w. |
|---|---|---|
| 75 | 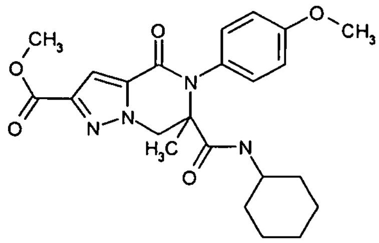 | 440,50341 |
| 76 | 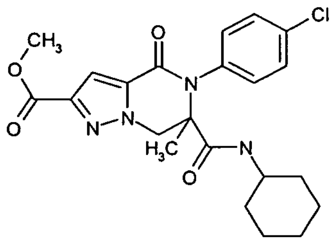 | 444,92195 |
| 77 | 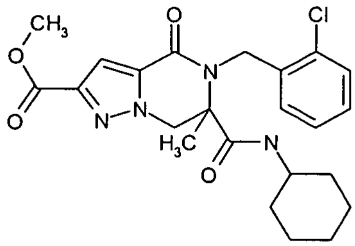 | 458,94904 |
| 78 | 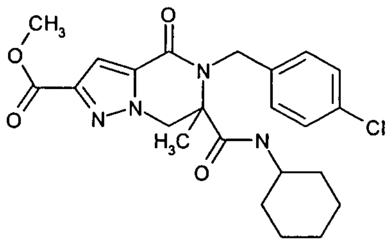 | 458,94904 |
| 79 | 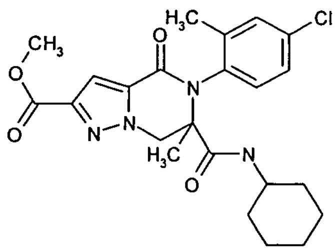 | 458,94904 |
| 80 | 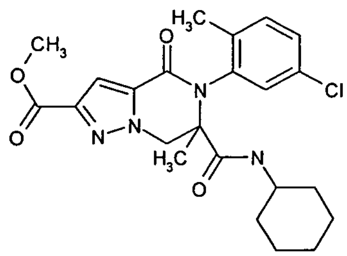 | 458,94904 |

(continued)

| № | Formula | Mol.w. |
|---|---|---|
| 81 | 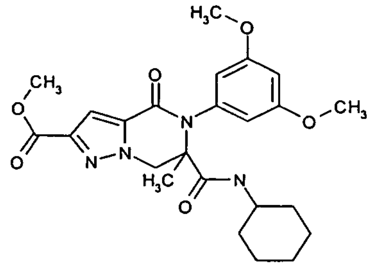 | 470,5299 |
| 82 | 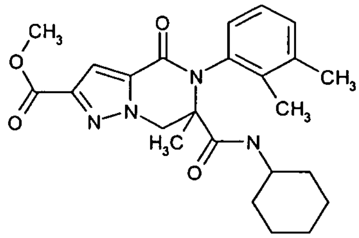 | 438,5311 |
| 83 | 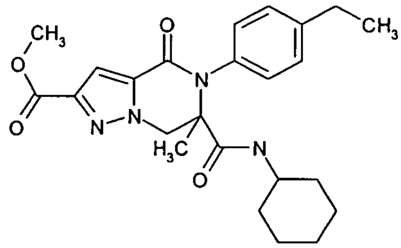 | 438,5311 |
| 84 | 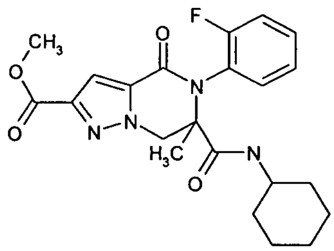 | 428,46735 |
| 85 | 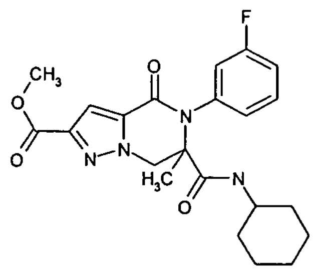 | 428,46735 |
| 86 | 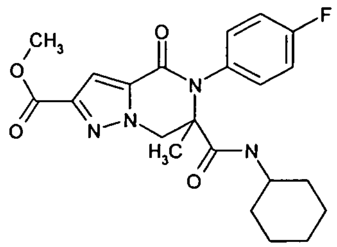 | 428,46735 |

(continued)

| № | Formula | Mol.w. |
|---|---|---|
| 87 | 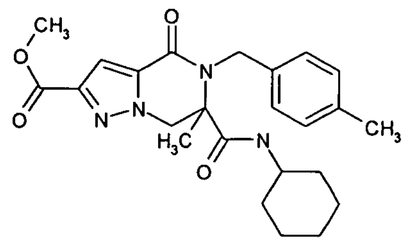 | 438,5311 |
| 88 | 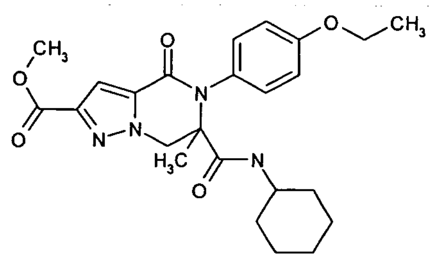 | 454,5305 |
| 89 | 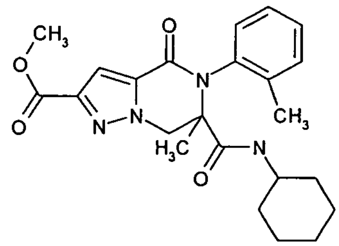 | 424,50401 |
| 90 | 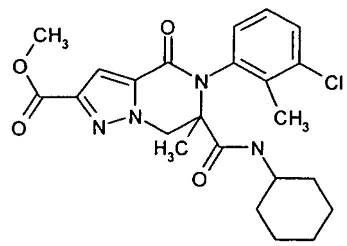 | 458,94904 |
| 91 | 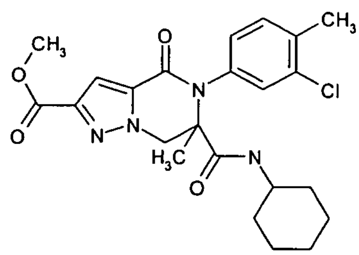 | 458,94904 |
| 92 | 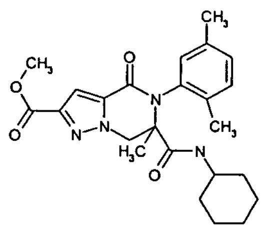 | 438,5311 |

(continued)

| № | Formula | Mol.w. |
|---|---|---|
| 93 | 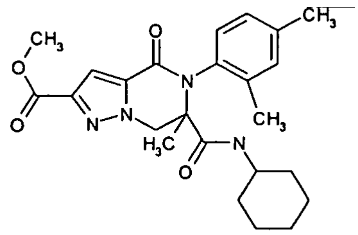 | 438,5311 |
| 94 | 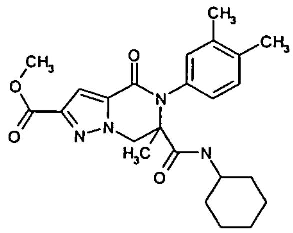 | 438,5311 |
| 95 | 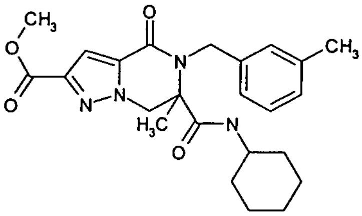 | 438,5311 |
| 96 | 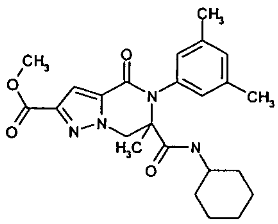 | 438,5311 |
| 97 | 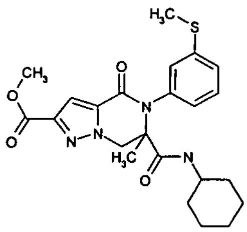 | 456,56801 |
| 98 | 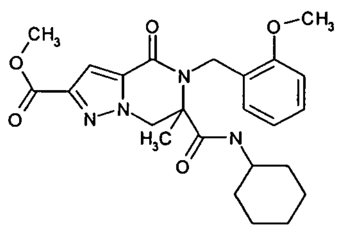 | 454,5305 |

(continued)

| № | Formula | Mol.w. |
|---|---|---|
| 99 | 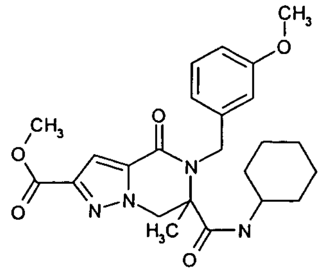 | 454,5305 |
| 100 | 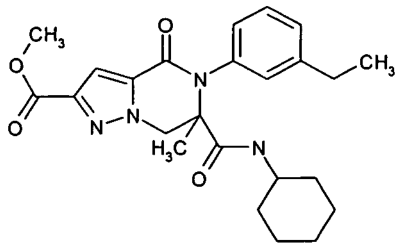 | 438,5311 |
| 101 | 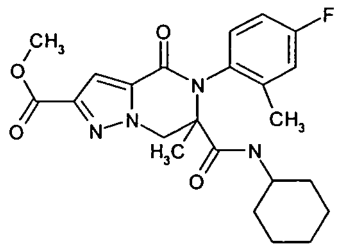 | 442,49444 |
| 102 | 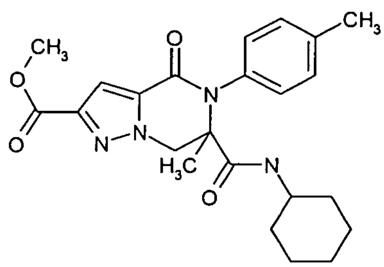 | 424,50401 |
| 103 | 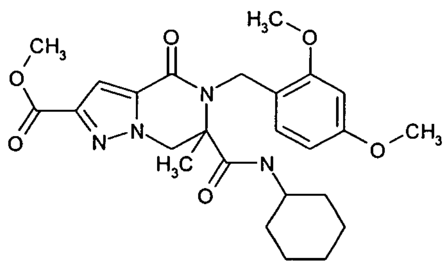 | 484,55699 |
| 104 | 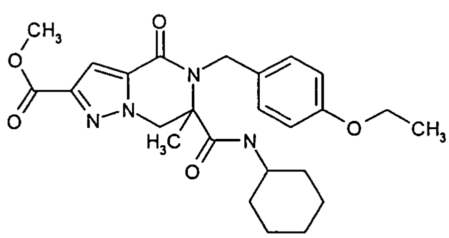 | 468,55759 |

(continued)

| № | Formula | Mol.w. |
|---|---|---|
| 105 | 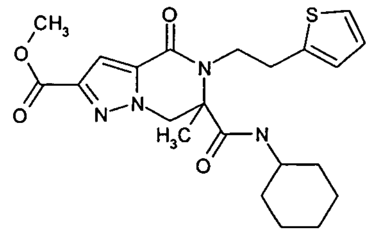 | 444,55686 |
| 106 | 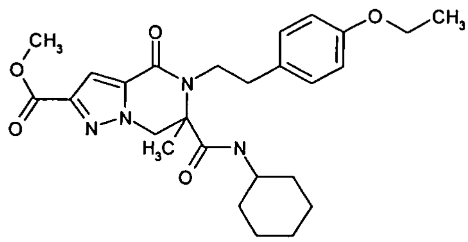 | 482,58468 |
| 107 | 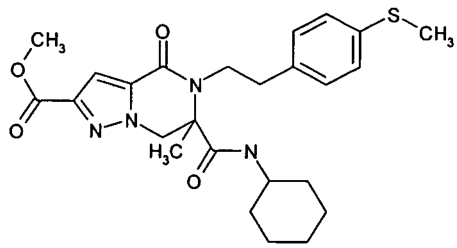 | 484,62219 |
| 108 | 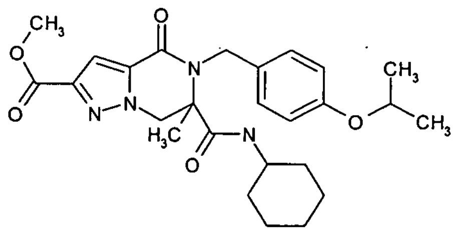 | 482,58468 |
| 109 | 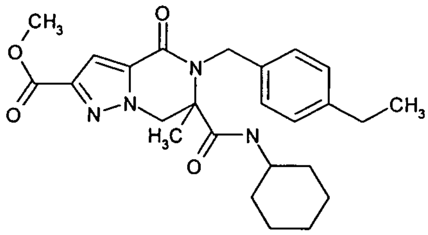 | 452,55819 |
| 110 | 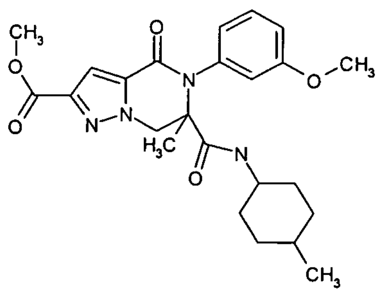 | 454,5305 |

(continued)

| № | Formula | Mol.w. |
|---|---|---|
| 111 | 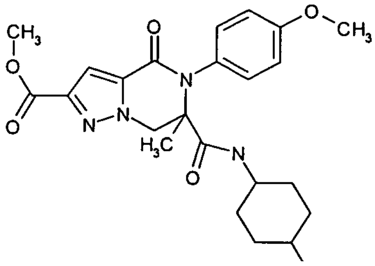 | 454,5305 |
| 112 | 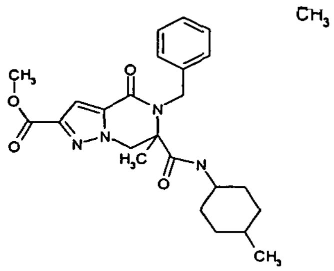 | 438,5311 |
| 113 | 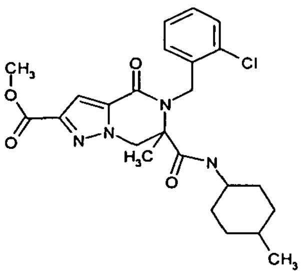 | 472,97613 |
| 114 | 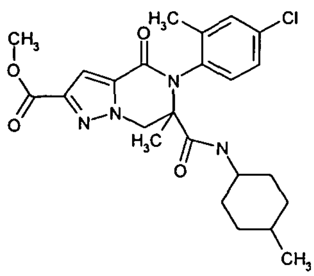 | 472,97613 |
| 115 | 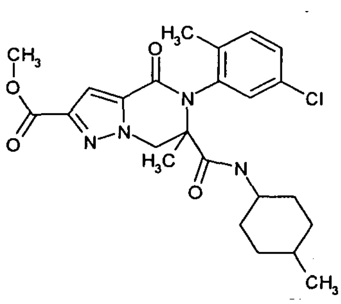 | 472,97613 |

(continued)

| № | Formula | Mol.w. |
|---|---|---|
| 116 | 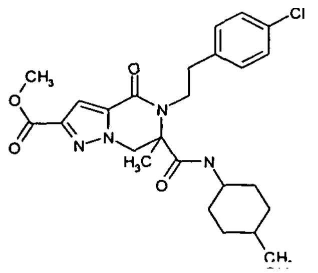 | 487,00322 |
| 117 | 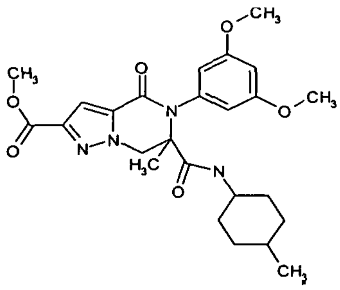 | 484,55699 |
| 118 | 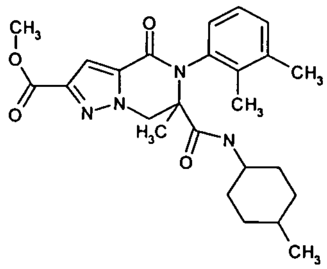 | 452,55819 |
| 119 | 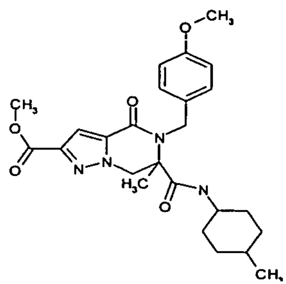 | 468,55759 |
| 120 | 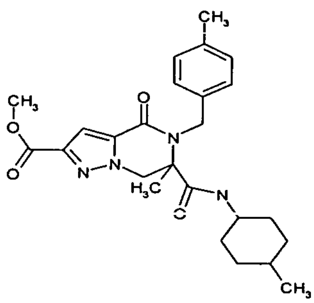 | 452,55819 |

(continued)

| № | Formula | Mol.w. |
|---|---|---|
| 121 | 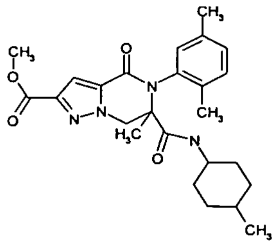 | 452,55819 |
| 122 | 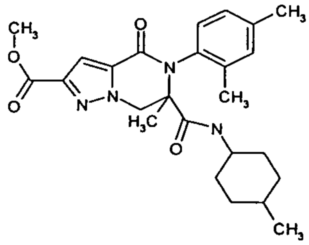 | 452,55819 |
| 123 | 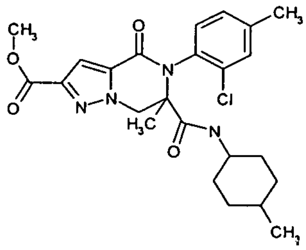 | 472,97613 |
| 124 | 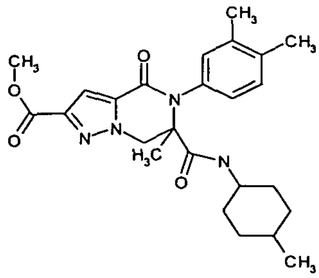 | 452,55819 |
| 125 | 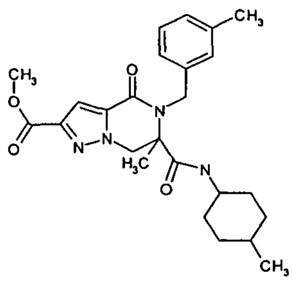 | 452,55819 |
| 126 | 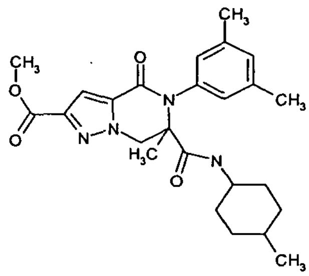 | 452,55819 |

(continued)

| № | Formula | Mol.w. |
|---|---|---|
| 127 | 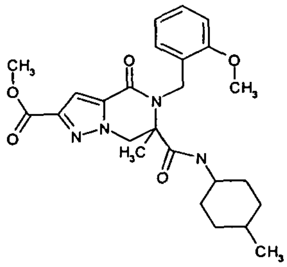 | 468,55759 |
| 128 | 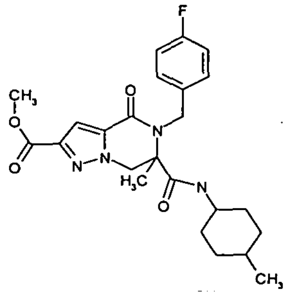 | 456,52153 |
| 129 | 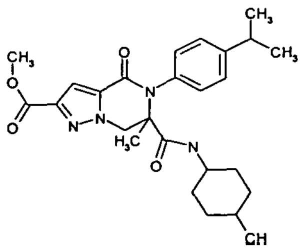 | 466,58528 |
| 130 | 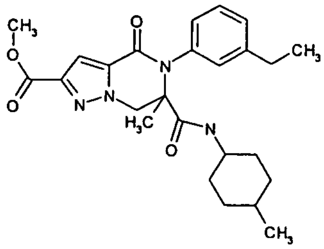 | 452,55819 |
| 131 | 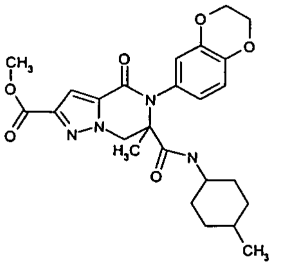 | 482,54105 |
| 132 | 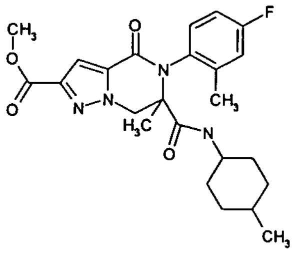 | 456,52153 |

(continued)

| № | Formula | Mol.w. |
|---|---|---|
| 133 | 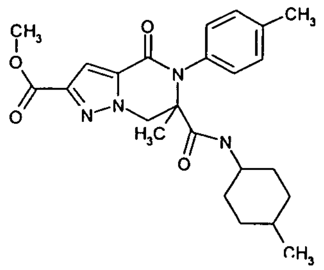 | 438,5311 |
| 134 | 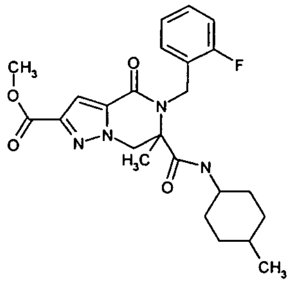 | 456,52153 |
| 135 | 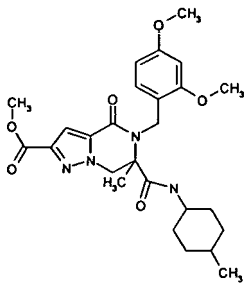 | 498,58408 |
| 136 | 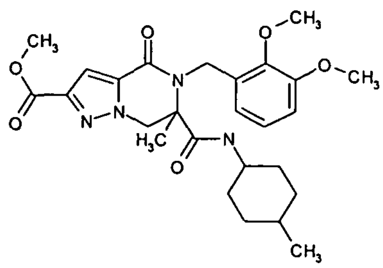 | 498,58408 |
| 137 | 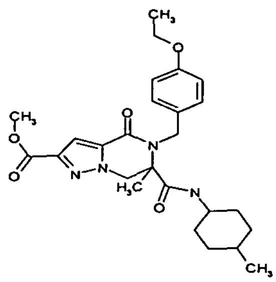 | 482,58468 |

(continued)

| № | Formula | Mol.w. |
|---|---|---|
| 138 | 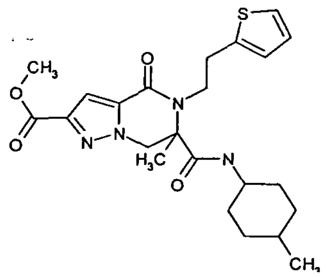 | 458,58395 |
| 139 | 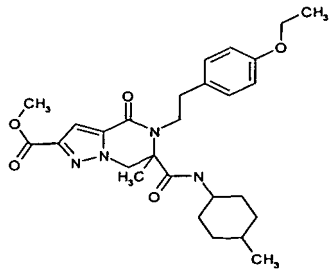 | 496,61177 |
| 140 | 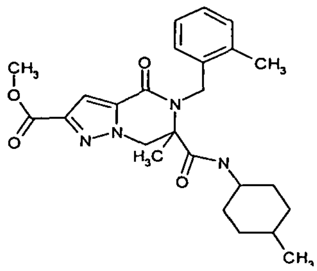 | 452,55819 |
| 141 | 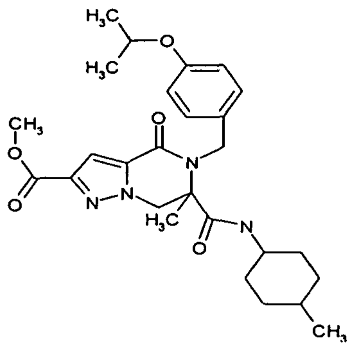 | 496,61177 |
| 142 | 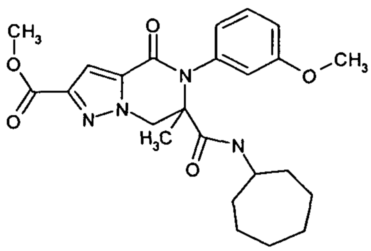 | 454,5305 |

(continued)

| № | Formula | Mol.w. |
|---|---|---|
| 143 | 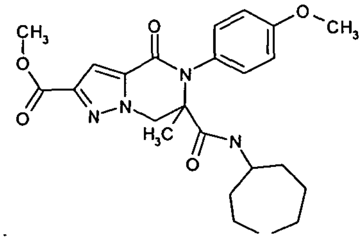 | 454,5305 |
| 144 | 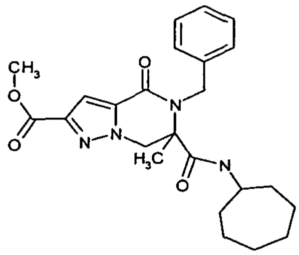 | 438,5311 |
| 145 | 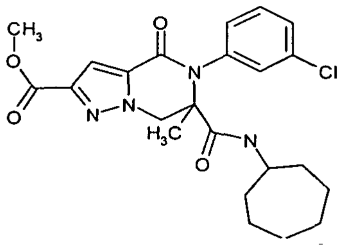 | 458,94904 |
| 146 | 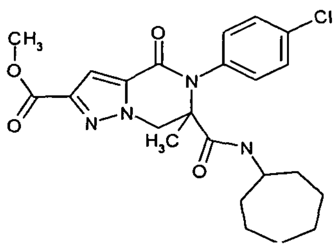 | 458,94904 |
| 147 | 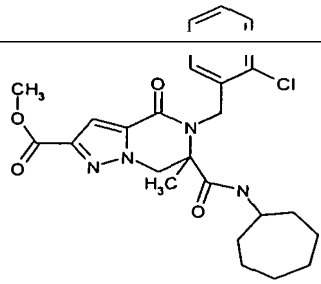 | 472,97613 |
| 148 | 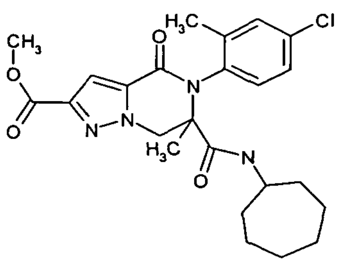 | 472,97613 |

(continued)

| № | Formula | Mol.w. |
|---|---|---|
| 149 | 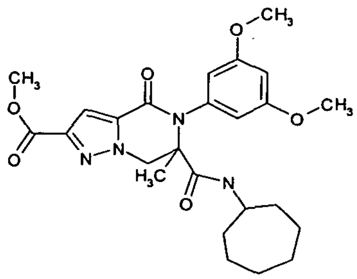 | 484,55699 |
| 150 | 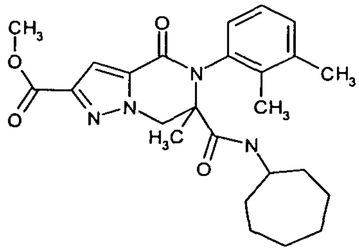 | 452,55819 |
| 151 | 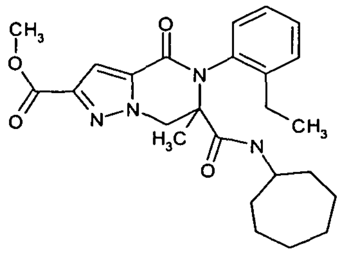 | 452,55819 |
| 152 | 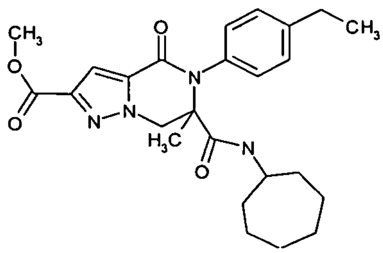 | 452.55819 |
| 153 | 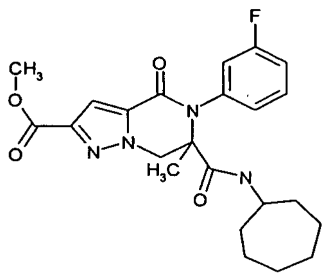 | 442,49444 |

(continued)

| № | Formula | Mol.w. |
|---|---|---|
| 154 | 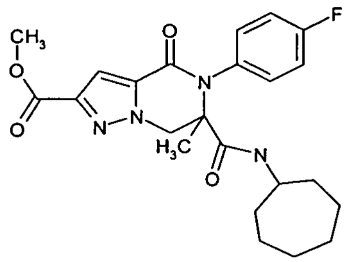 | 442,49444 |
| 155 | 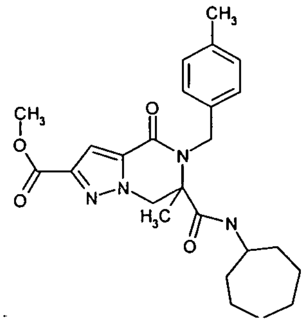 | 452,55819 |
| 156 | 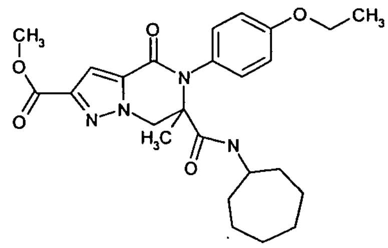 | 468,55759 |
| 157 | 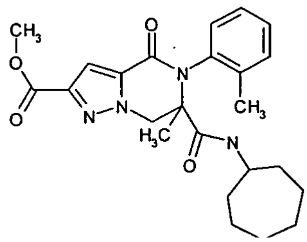 | 438,5311 |
| 158 | 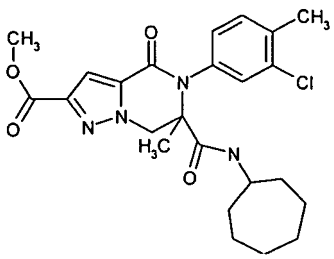 | 472,97613 |
| 159 | 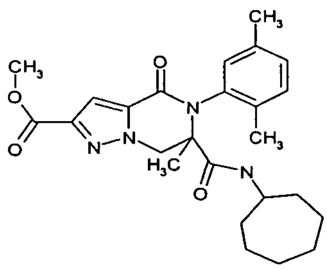 | 452,55819 |

(continued)

| № | Formula | Mol.w. |
|---|---|---|
| 160 | 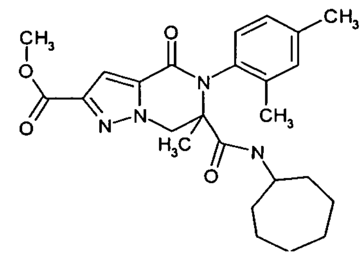 | 452,55819 |
| 161 | 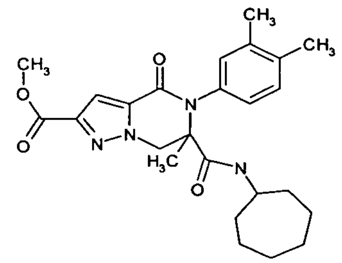 | 452,55819 |
| 162 | 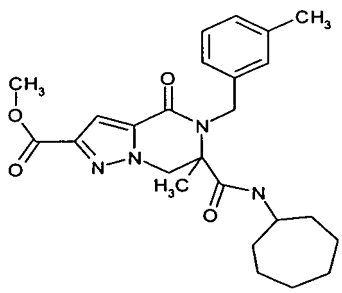 | 452,55819 |
| 163 | 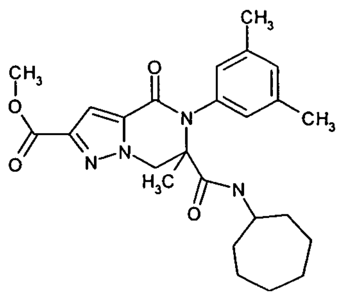 | 452,55819 |
| 164 | 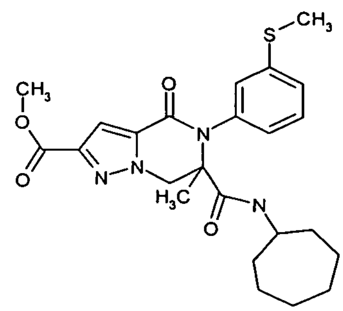 | 470,5951 |

(continued)

| № | Formula | Mol.w. |
|---|---|---|
| 165 | 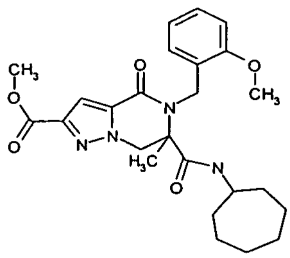 | 468,55759 |
| 166 | 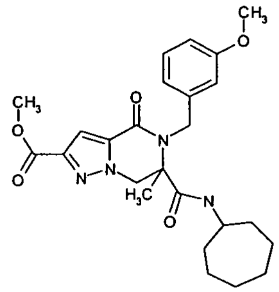 | 468,55759 |
| 167 | 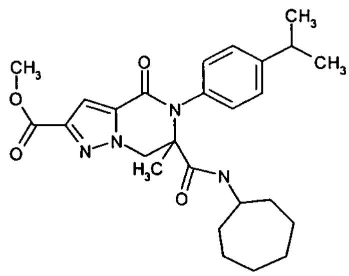 | 466,58528 |
| 168 | 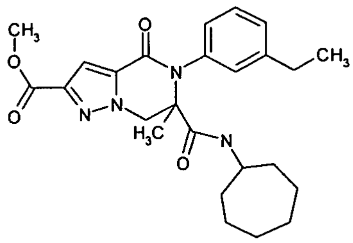 | 452,55819 |
| 169 | 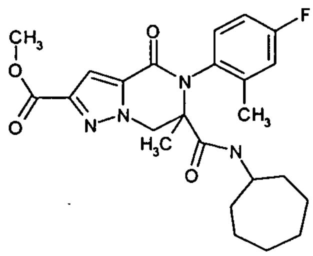 | 456,52153 |
| 170 | 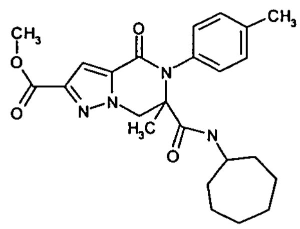 | 438,5311 |

(continued)

| № | Formula | Mol.w. |
|---|---|---|
| 171 | 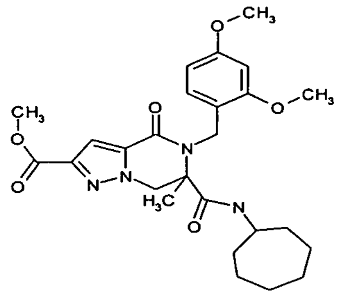 | 498,58408 |
| 172 | 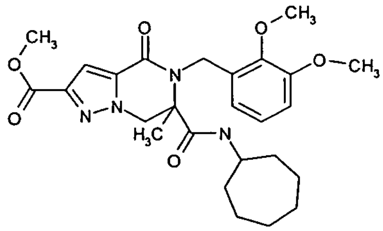 | 498,58408 |
| 173 | 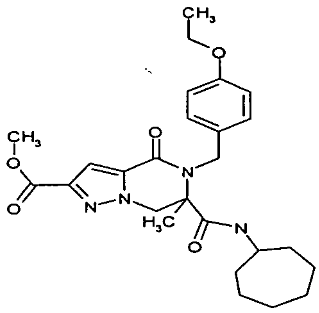 | 482,58468 |
| 174 | 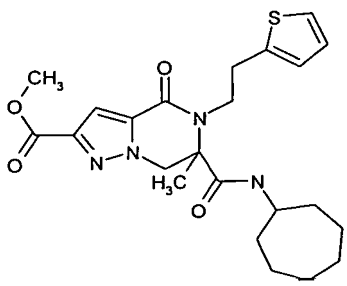 | 458,58395 |
| 175 | 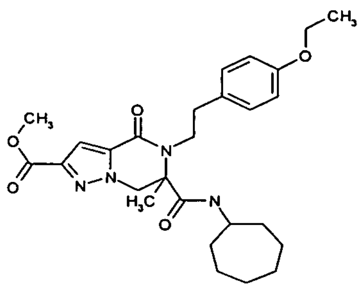 | 496,61177 |

(continued)

| № | Formula | Mol.w. |
|---|---|---|
| 176 | 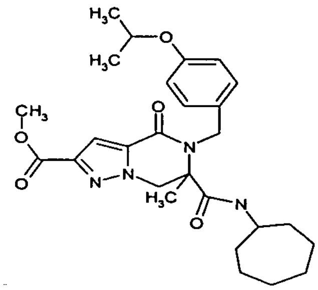 | 496,61177 |
| 177 | 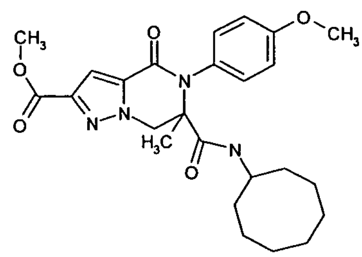 | 468,55759 |
| 178 | 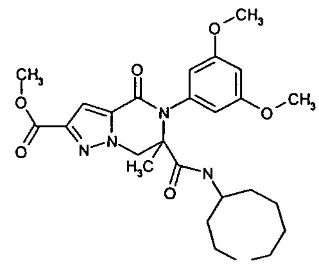 | 498,58408 |
| 179 | 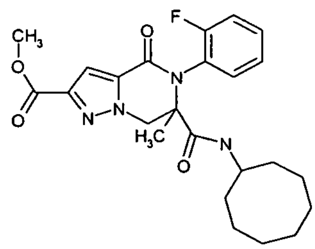 | 456,52153 |
| 180 | 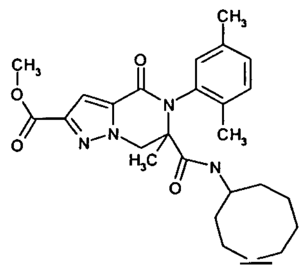 | 466,58528 |
| 181 | 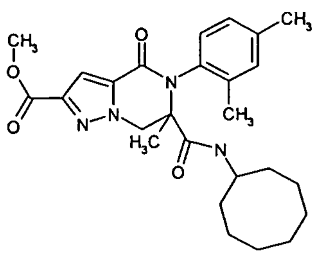 | 466,58528 |

(continued)

| № | Formula | Mol.w. |
|---|---|---|
| 182 | 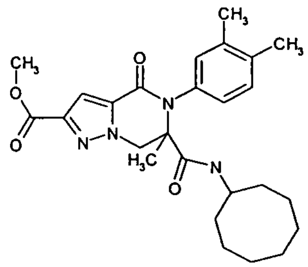 | 466,58528 |
| 183 | 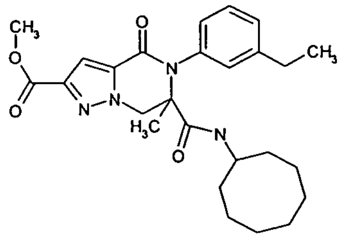 | 466,58528 |
| 184 | 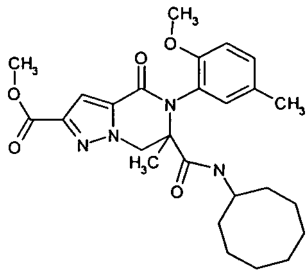 | 482,58468 |
| 185 | 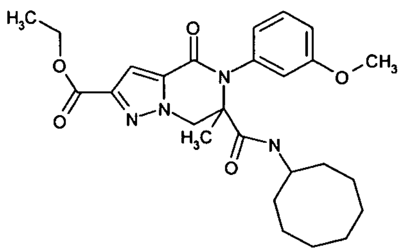 | 482,58468 |
| 186 | 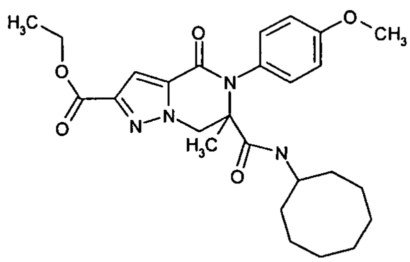 | 482,58468 |
| 187 | 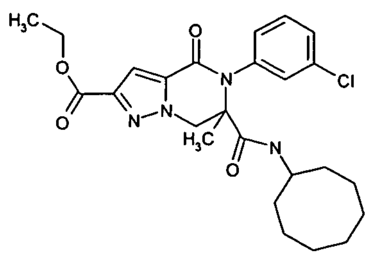 | 487,00322 |

(continued)

| № | Formula | Mol.w. |
|---|---|---|
| 188 | 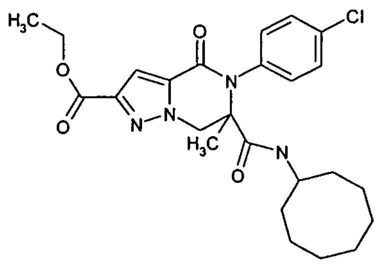 | 487,00322 |
| 189 | 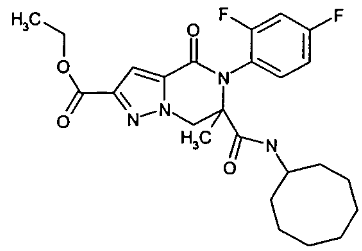 | 488,53905 |
| 190 | 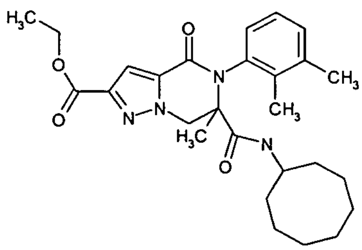 | 480,61237 |
| 191 | 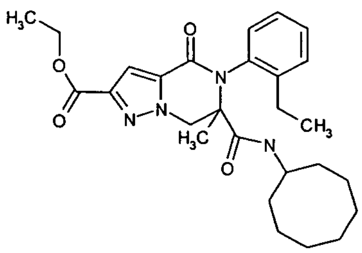 | 480,61237 |
| 192 | 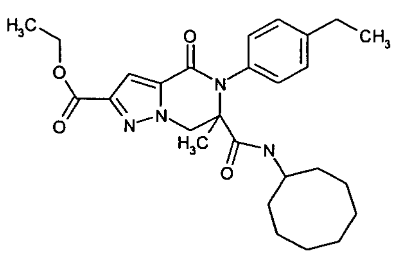 | 480,61237 |
| 193 | 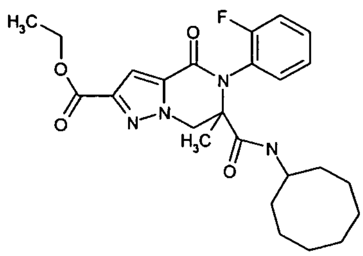 | 470,54862 |

(continued)

| № | Formula | Mol.w. |
|---|---|---|
| 194 | 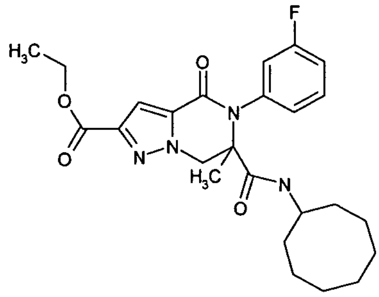 | 470,54862 |
| 195 | 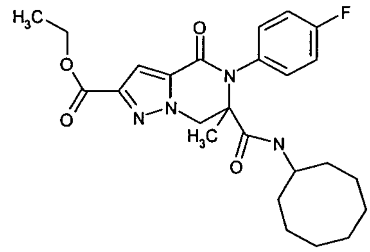 | 470,54862 |
| 196 | 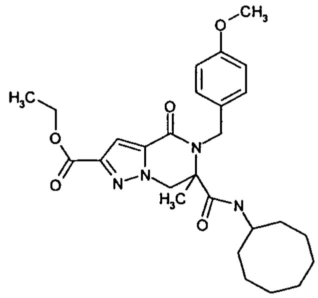 | 496,61177 |
| 197 | 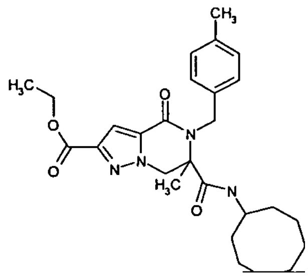 | 480,61237 |
| 198 | 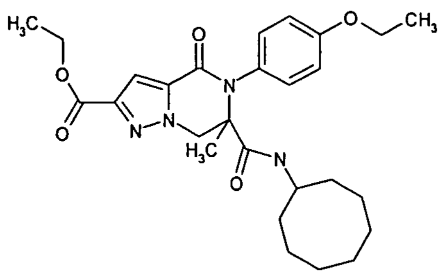 | 496,61177 |

(continued)

| № | Formula | Mol.w. |
|---|---|---|
| 199 | 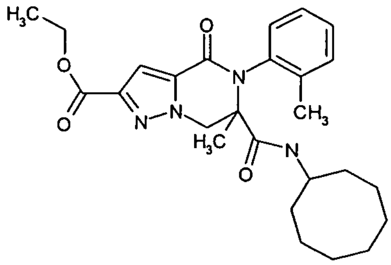 | 466,58528 |
| 200 | 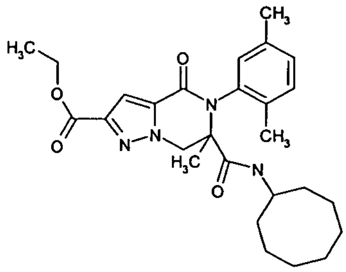 | 480,61237 |
| 201 | 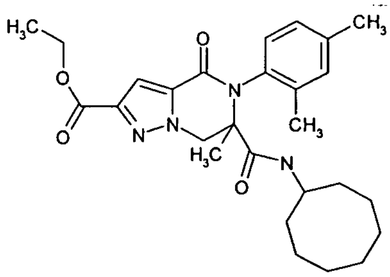 | 480,61237 |
| 202 | 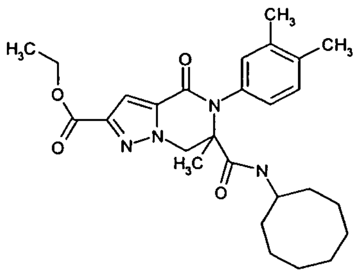 | 480,61237 |
| 203 | 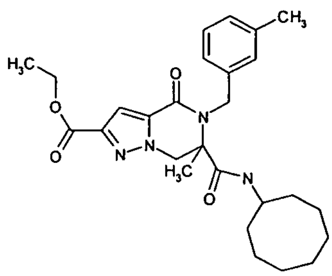 | 480,61237 |

(continued)

| № | Formula | Mol.w. |
|---|---|---|
| 204 | 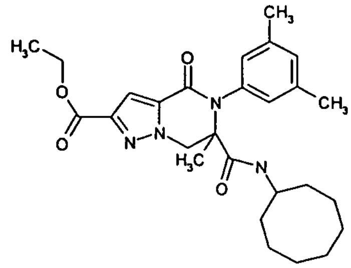 | 480,61237 |
| 205 | 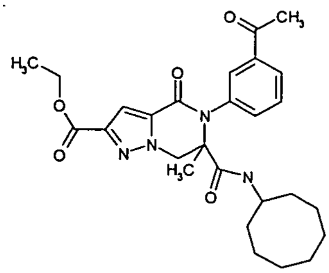 | 494,59583 |
| 206 | 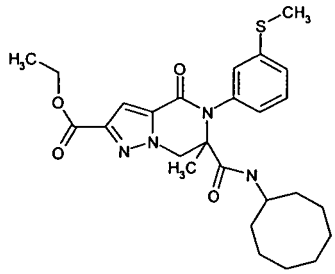 | 498,64928 |
| 207 | 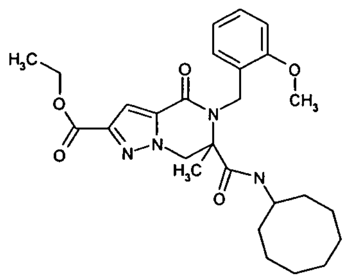 | 496,61177 |
| 208 | 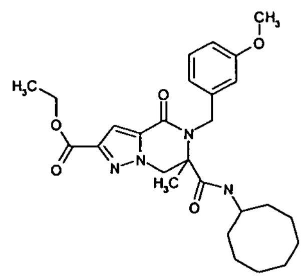 | 496,61177 |

(continued)

| № | Formula | Mol.w. |
|---|---|---|
| 209 | 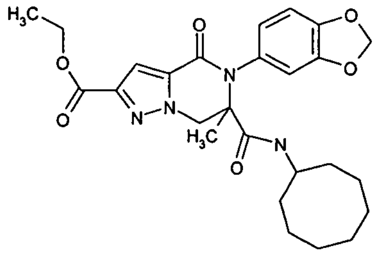 | 496,56814 |
| 210 | 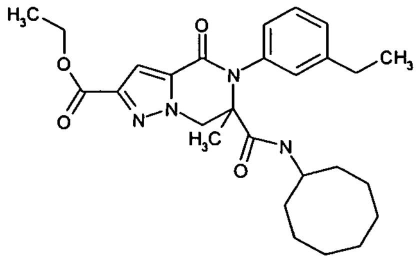 | 480,61237 |
| 211 | 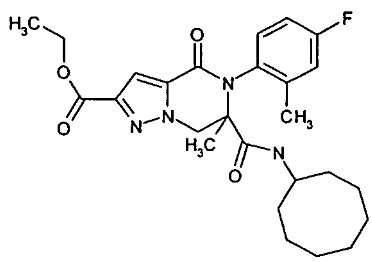 | 484,57571 |
| 212 | 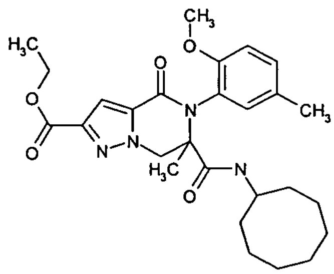 | 496,61177 |
| 213 | 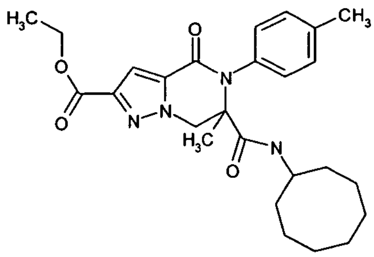 | 466,58528 |
| 214 | 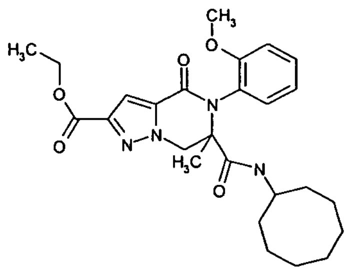 | 482,58468 |

(continued)

| № | Formula | Mol.w. |
|---|---|---|
| 215 | 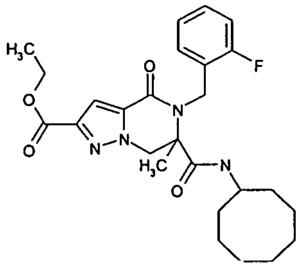 | 484,57571 |
| 216 | 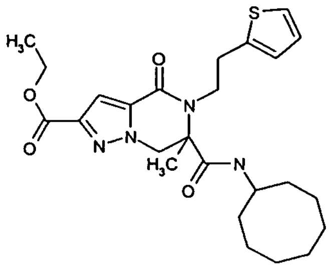 | 486,63813 |
| 217 | 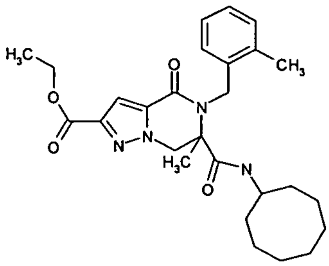 | 480,61237 |
| 218 | 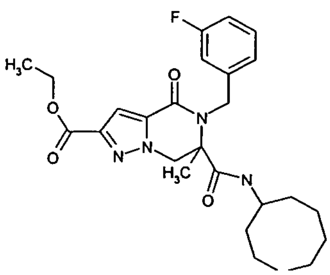 | 484,57571 |
| 219 | 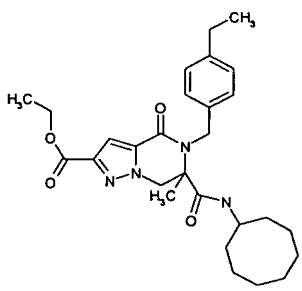 | 494,63946 |

(continued)

| № | Formula | Mol.w. |
|---|---|---|
| 220 | 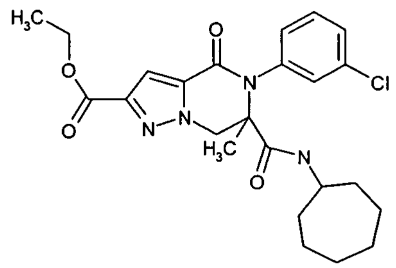 | 472, 97613 |
| 221 | 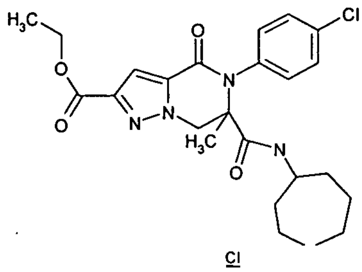 | 472,97613 |
| 222 | 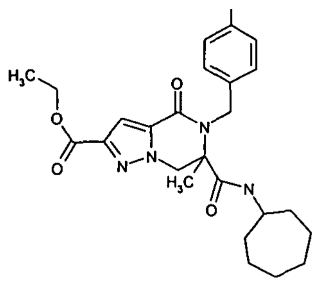 | 487,00322 |
| 223 | 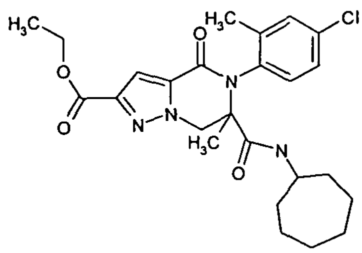 | 487,00322 |
| 224 | 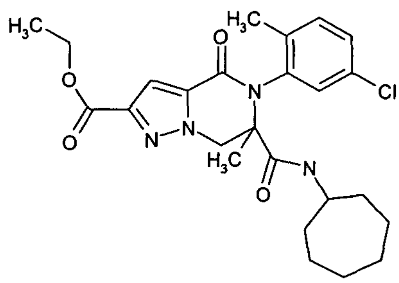 | 487,00322 |

(continued)

| Nº | Formula | Mol.w. |
|---|---|---|
| 225 | 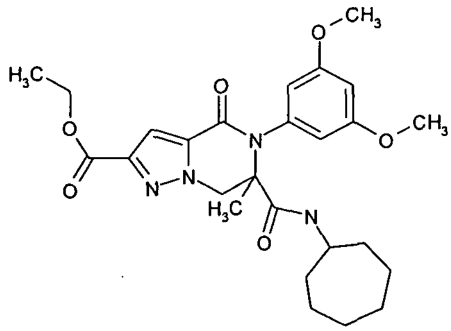 | 498,58408 |
| 226 | 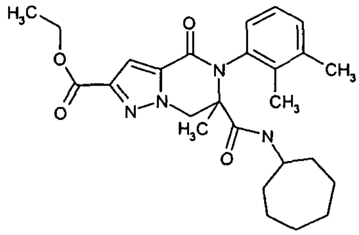 | 466,58528 |
| 227 | 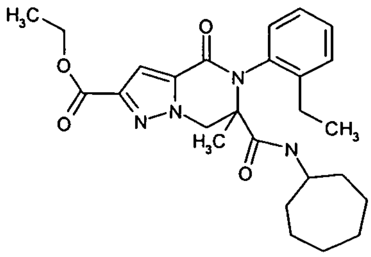 | 466,58528 |
| 228 | 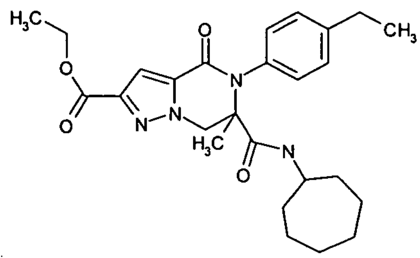 | 466,58528 |
| 229 | 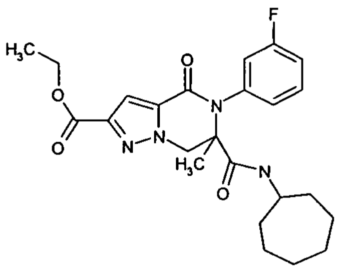 | 456,52153 |

(continued)

| № | Formula | Mol.w. |
|---|---|---|
| 230 | 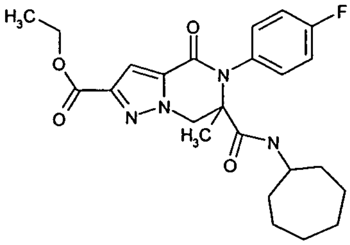 | 456,52153 |
| 231 | 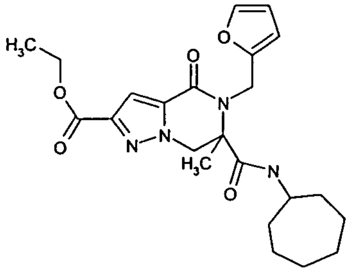 | 442,51935 |
| 232 | 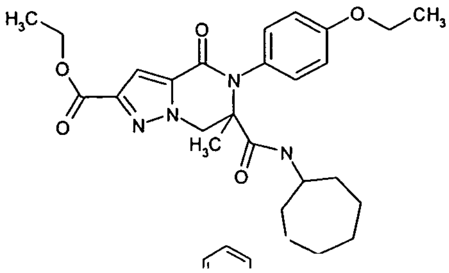 | 482,58468 |
| 233 | 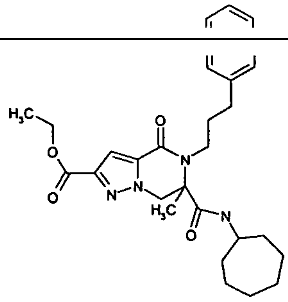 | 480,61237 |
| 234 | 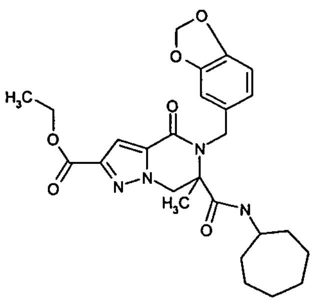 | 496,56814 |

(continued)

| № | Formula | Mol.w. |
|---|---|---|
| 235 | 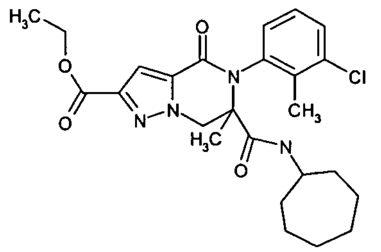 | 487,00322 |
| 236 | 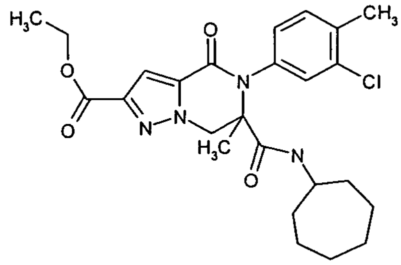 | 487,00322 |
| 237 | 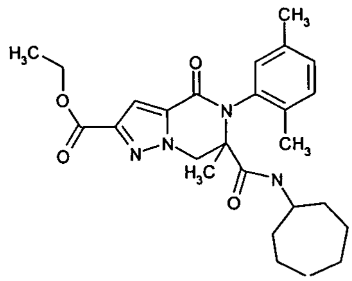 | 466,58528 |
| 238 | 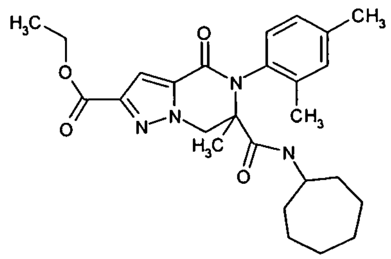 | 466,58528 |
| 239 | 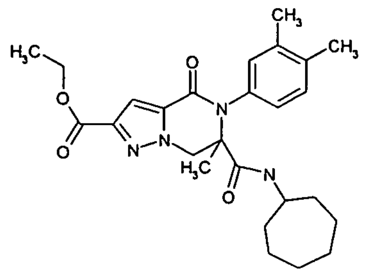 | 466,58528 |

(continued)

| № | Formula | Mol.w. |
|---|---|---|
| 240 | 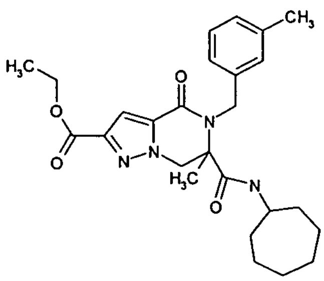 | 466,58528 |
| 241 | 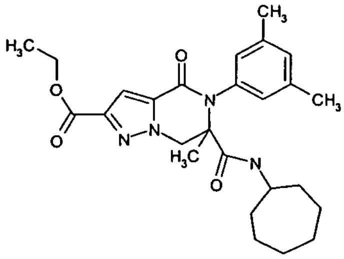 | 466,58528 |
| 242 | 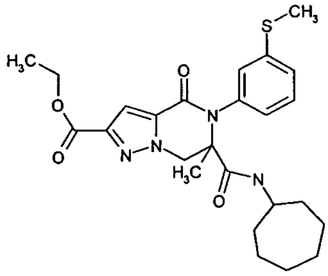 | 484,62219 |
| 243 | 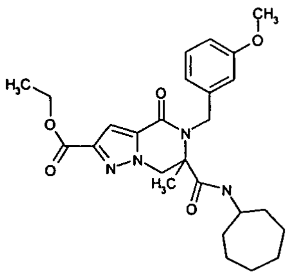 | 482,58468 |
| 244 | 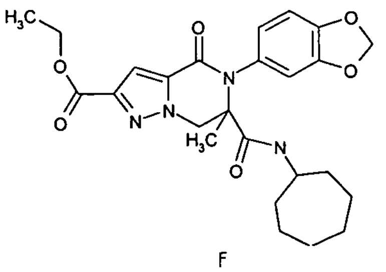 F | 482,54105 |

(continued)

| № | Formula | Mol.w. |
|---|---|---|
| 245 | 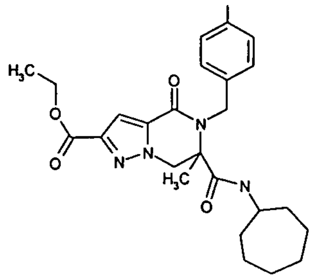 | 470,54862 |
| 246 | 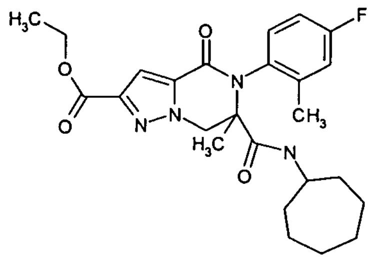 | 470,54862 |
| 247 | 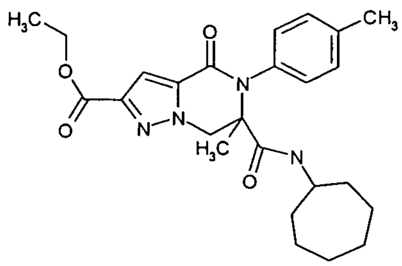 | 452,55819 |
| 248 | 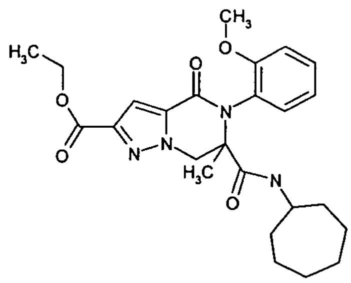 | 468,55759 |
| 249 | 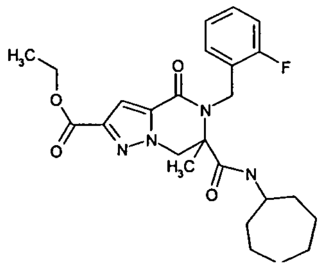 | 470,54862 |

(continued)

| № | Formula | Mol.w. |
|---|---|---|
| 250 | 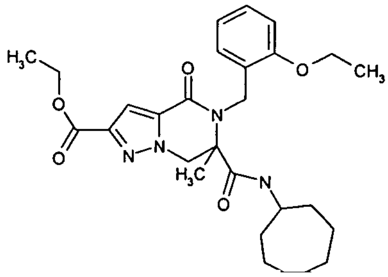 | 496,61177 |
| 251 | 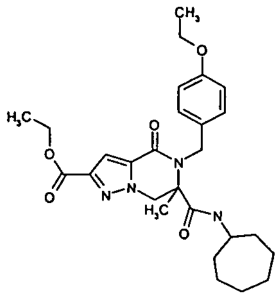 | 496,61177 |
| 252 | 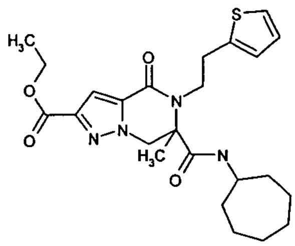 | 472,61104 |
| 253 | 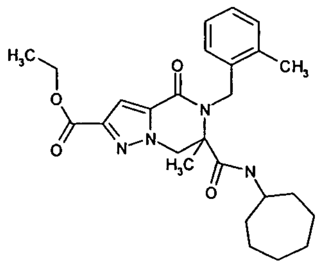 | 466,58528 |
| 254 | 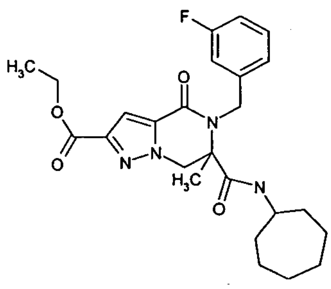 | 470,54862 |

(continued)

| № | Formula | Mol.w. |
|---|---|---|
| 255 | 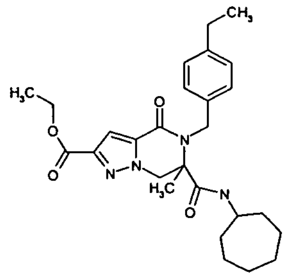 | 480,61237 |
| 256 | 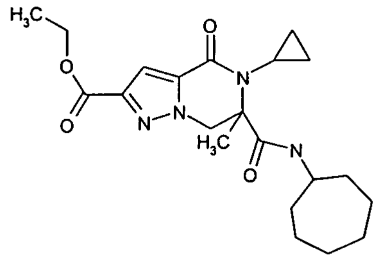 | 402,49765 |
| 257 | 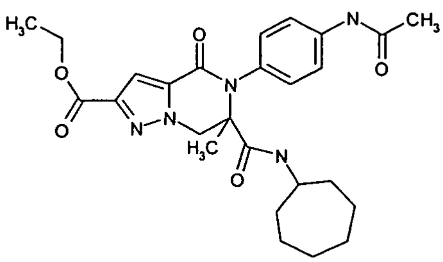 | 495,58341 |
| 258 | 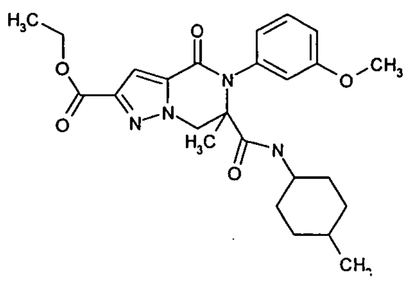 | 468,55759 |
| 259 | 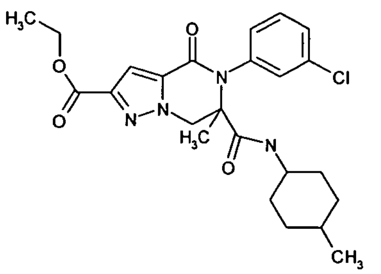 | 472,97613 |

(continued)

| № | Formula | Mol.w. |
|---|---|---|
| 260 | 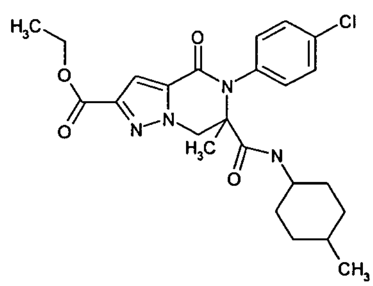 | 472,97613 |
| 261 | 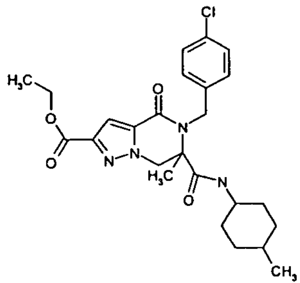 | 487,00322 |
| 262 | 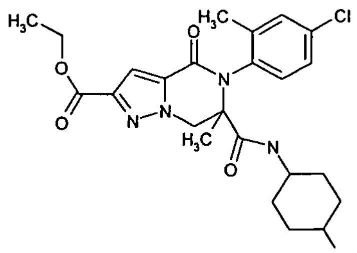 | 487,00322 |
| 263 | 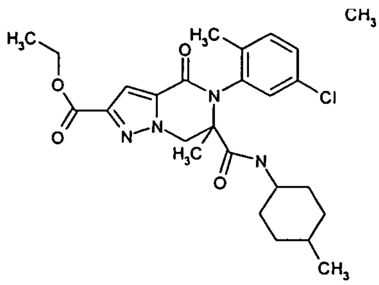 | 487,00322 |
| 264 | 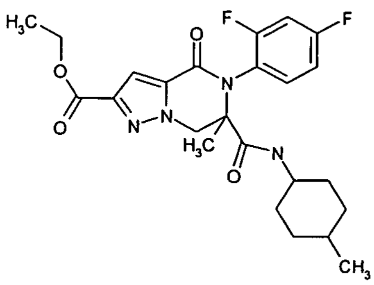 | 474,51196 |

(continued)

| № | Formula | Mol.w. |
|---|---|---|
| 265 | 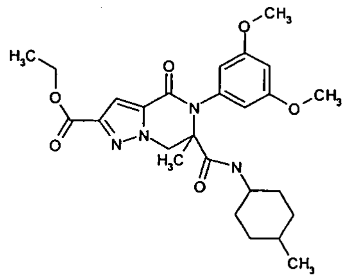 | 498,58408 |
| 266 | 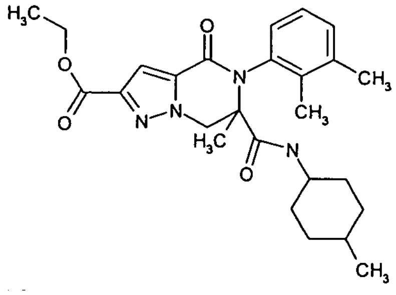 | 466,58528 |
| 267 | 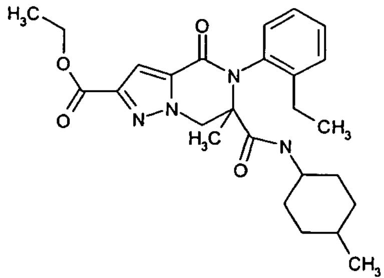 | 466,58528 |
| 268 | 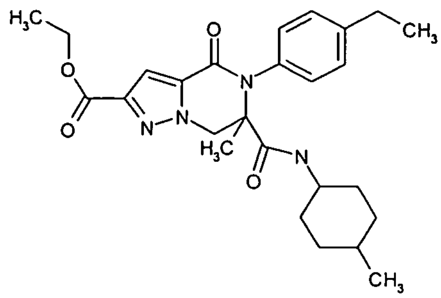 | 466, 58528 |
| 269 | 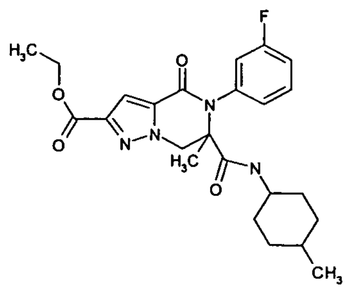 | 456,52153 |

(continued)

| № | Formula | Mol.w. |
|---|---|---|
| 270 | 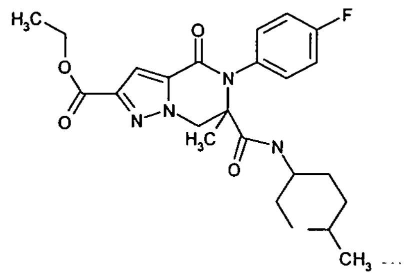 | 456,52153 |
| 271 | 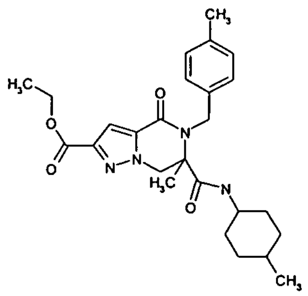 | 466,58528 |
| 272 | 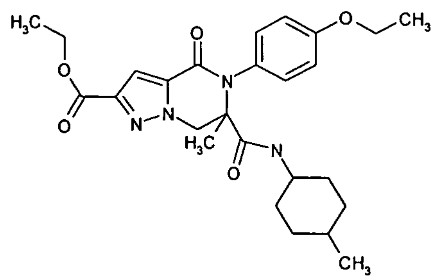 | 482,58468 |
| 273 | 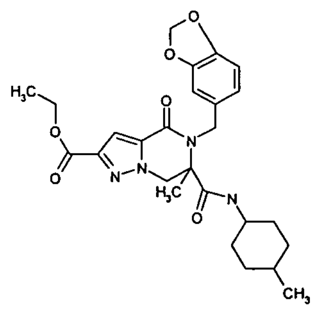 | 496,56814 |
| 274 | 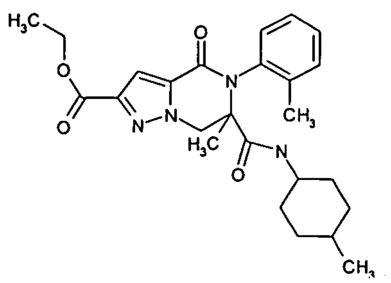 | 452,55819 |

(continued)

| № | Formula | Mol.w. |
|---|---|---|
| 275 | 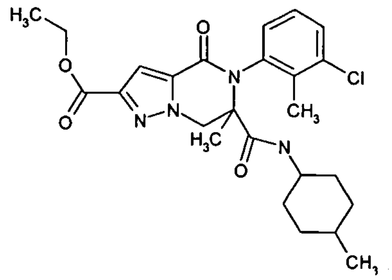 | 487,00322 |
| 276 | 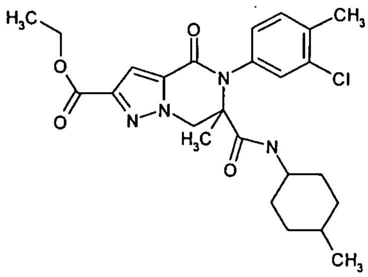 | 487,00322 |
| 277 | 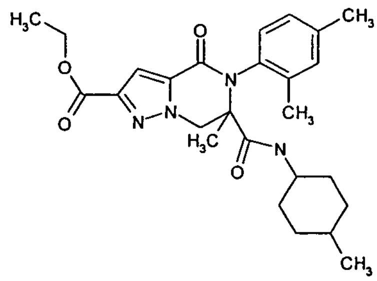 | 466,58528 |
| 278 | 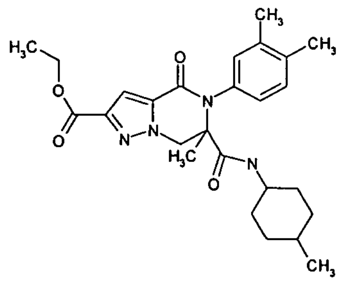 | 466,58528 |
| 279 | 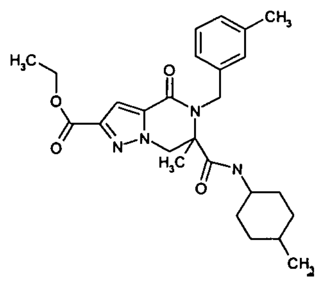 | 466,58528 |

(continued)

| № | Formula | Mol.w. |
|---|---|---|
| 280 | 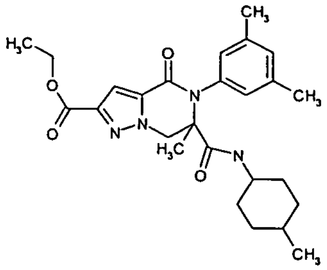 | 466,58528 |
| 281 | 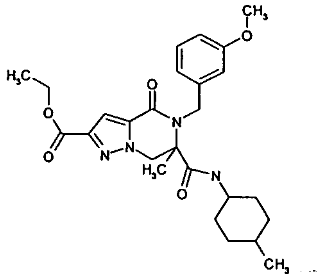 | 482,58468 |
| 282 | 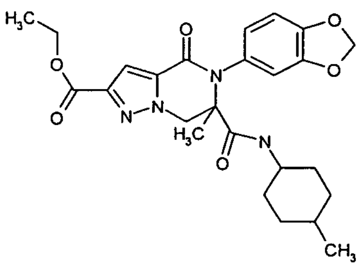 | 482,54105 |
| 283 | 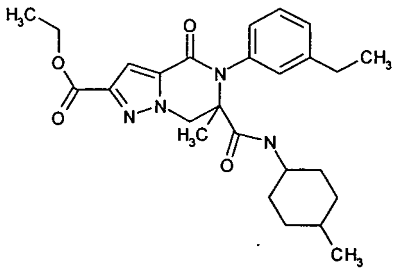 | 466,58528 |
| 284 | 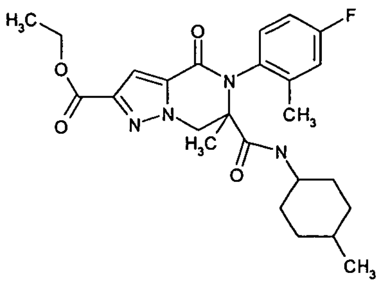 | 470,54862 |

(continued)

| № | Formula | Mol.w. |
|---|---|---|
| 285 | 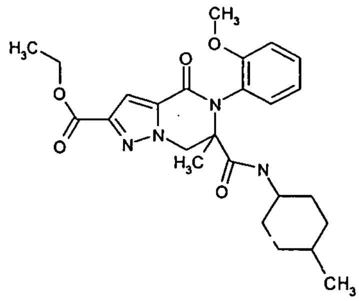 | 468,55759 |
| 286 | 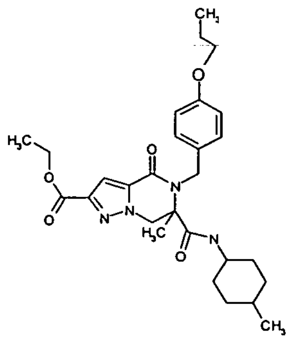 | 496,61177 |
| 287 | 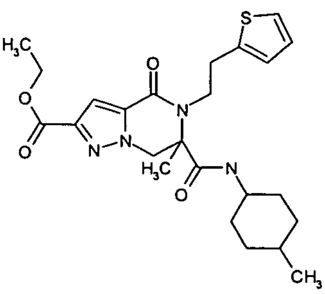 | 472,61104 |
| 288 | 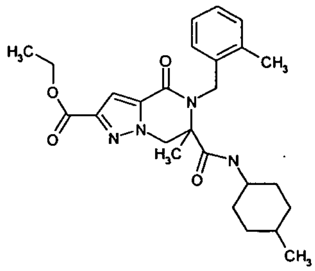 | 466,58528 |
| 289 | 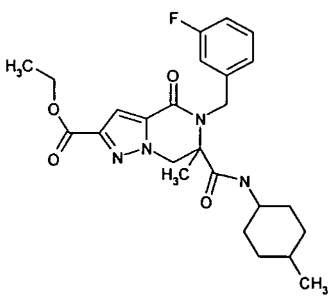 | 470,54862 |

(continued)

| № | Formula | Mol.w. |
|---|---|---|
| 290 | 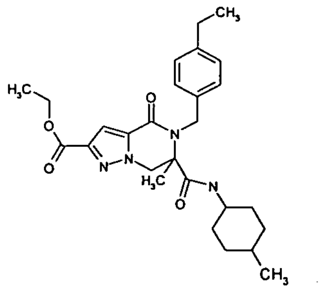 | 480,61237 |
| 291 | 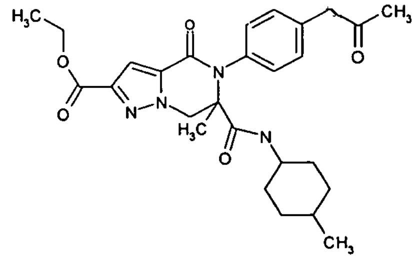 | 495,58341 |
| 292 | 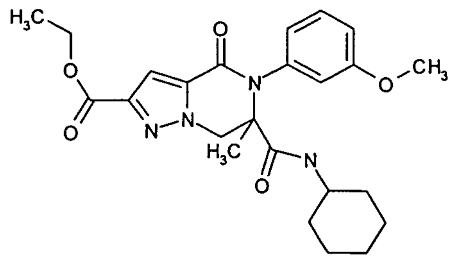 | 454,5305 |
| 293 | 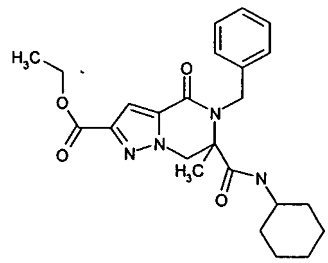 | 438,5311 |
| 294 | 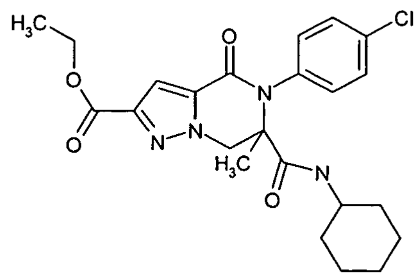 | 458,94904 |

(continued)

| № | Formula | Mol.w. |
|---|---|---|
| 295 | 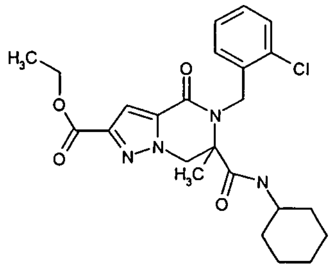 | 472,97613 |
| 296 | 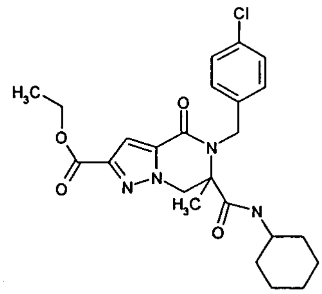 | 472,97613 |
| 297 | 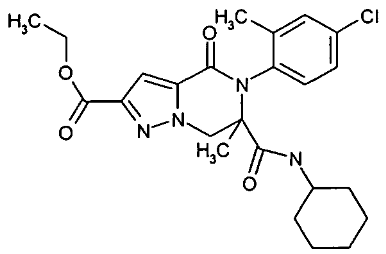 | 472,97613 |
| 298 | 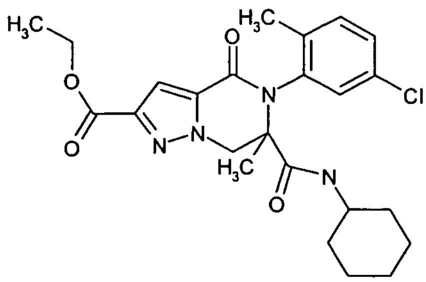 | 472,97613 |
| 299 | 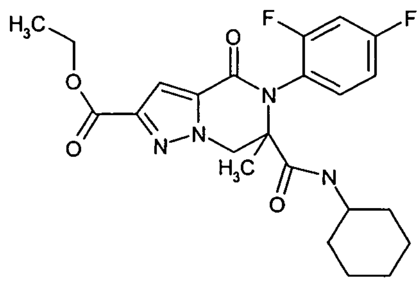 | 460,48487 |

(continued)

| № | Formula | Mol.w. |
|---|---|---|
| 300 | 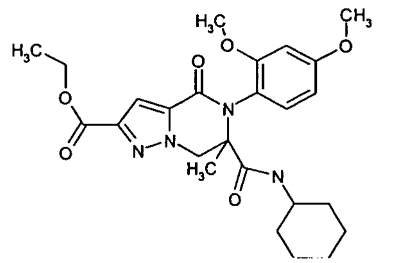 | 484,55699 |
| 301 | 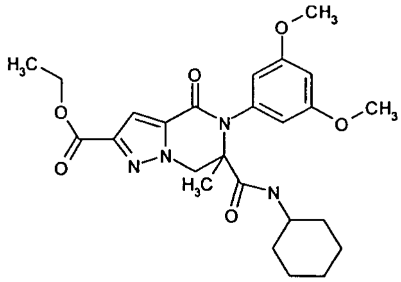 | 484,55699 |
| 302 | 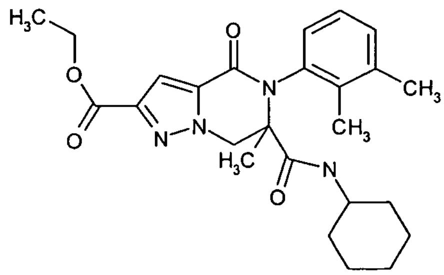 | 452,55819 |
| 303 | 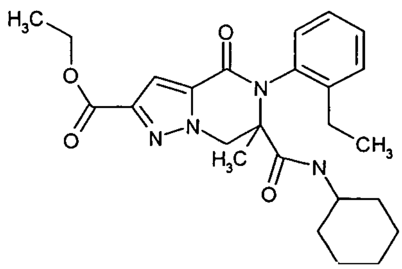 | 452,55819 |
| 304 | 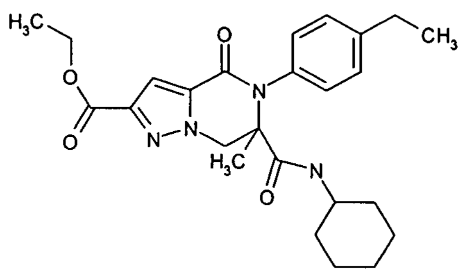 | 452,55819 |

(continued)

| № | Formula | Mol.w. |
|---|---|---|
| 305 | 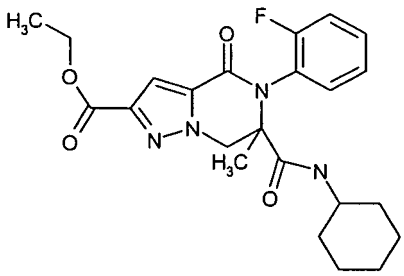 | 442,49444 |
| 306 | 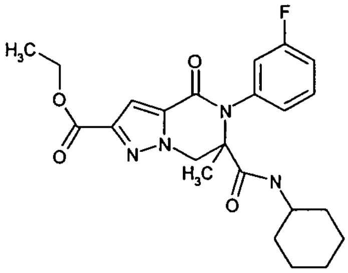 | 442,49444 |
| 307 | 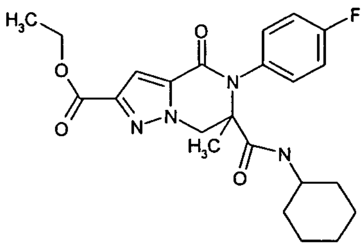 | 442,49444 |
| 308 | 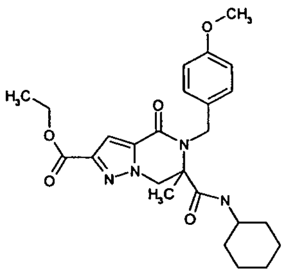 | 468,55759 |
| 309 | 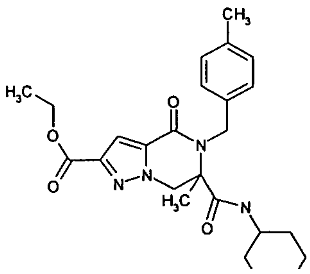 | 452,55819 |

(continued)

| № | Formula | Mol.w. |
|---|---|---|
| 310 | 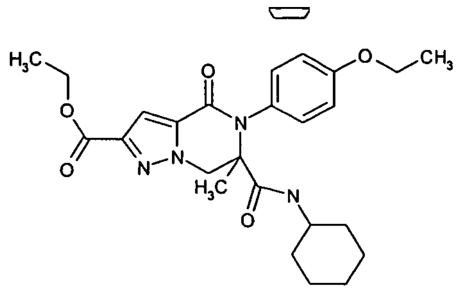 | 468,55759 |
| 311 | 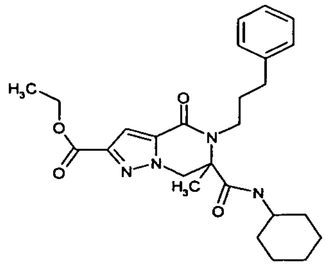 | 466,58528 |
| 312 | 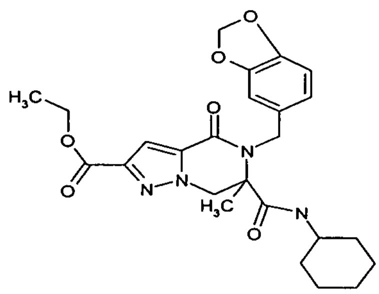 | 482,54105 |
| 313 | 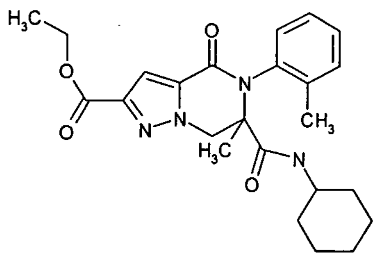 | 438,5311 |
| 314 | 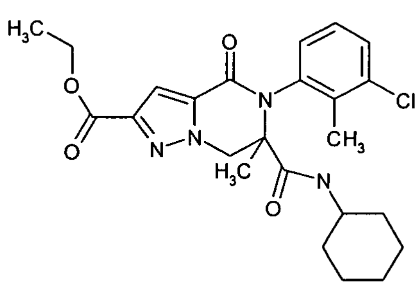 | 472,97613 |

(continued)

| № | Formula | Mol.w. |
|---|---|---|
| 315 | 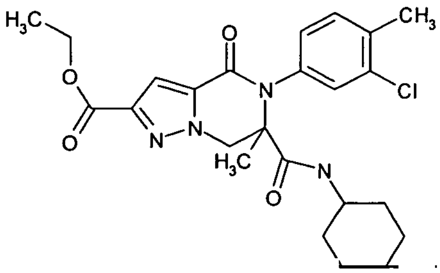 | 472,97613 |
| 316 | 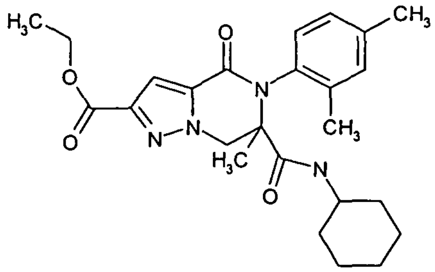 | 452,55819 |
| 317 | 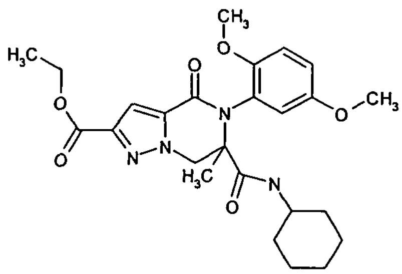 | 484,55699 |
| 318 | 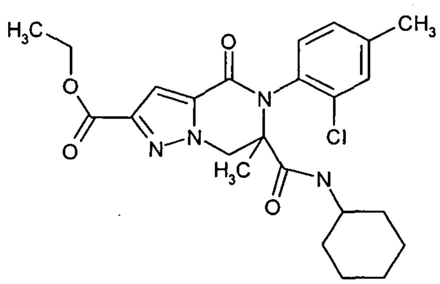 | 472,97613 |
| 319 | 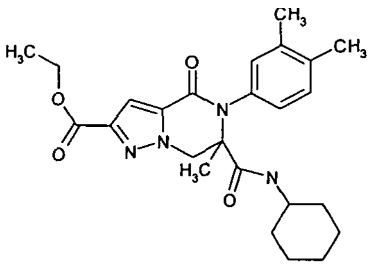 | 452,55819 |

(continued)

| № | Formula | Mol.w. |
|---|---|---|
| 320 | 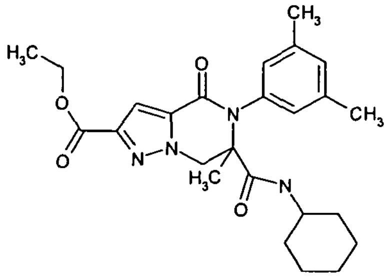 | 452,55819 |
| 321 | 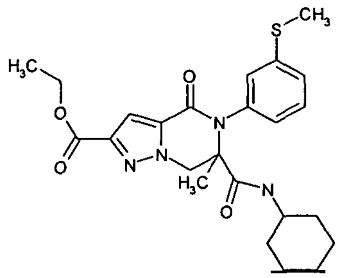 | 470,5951 |
| 322 | 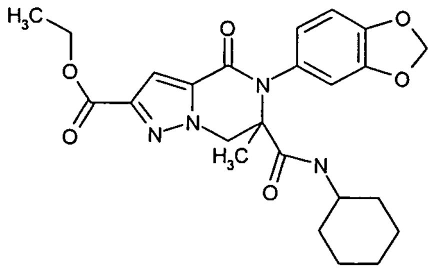 | 468,51396 |
| 323 | 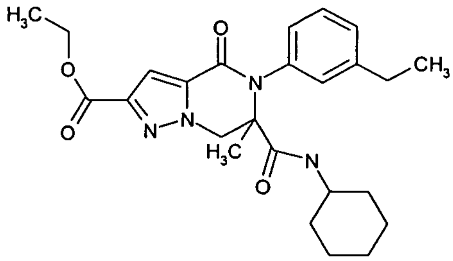 | 452,55819 |
| 324 | 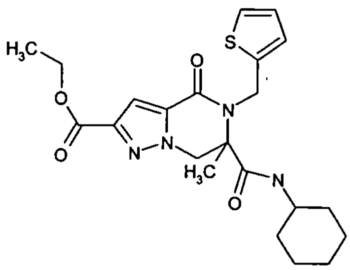 | 444,55686 |

(continued)

| № | Formula | Mol.w. |
|---|---|---|
| 325 | 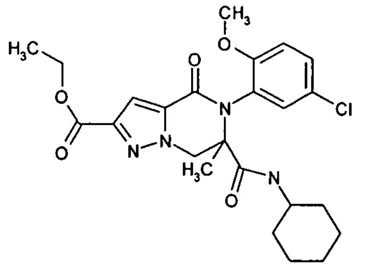 | 488,97553 |
| 326 | 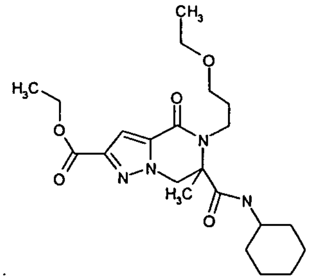 | 434,54008 |
| 327 | 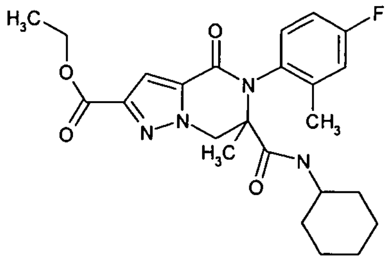 | 456,52153 |
| 328 | 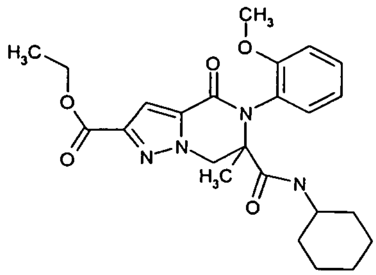 | 454,5305 |
| 329 | 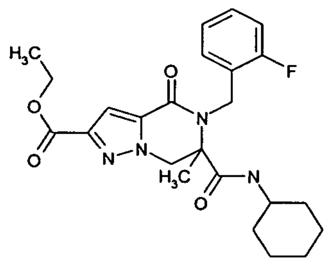 | 456,52153 |

(continued)

| No | Formula | Mol.w. |
|---|---|---|
| 330 | 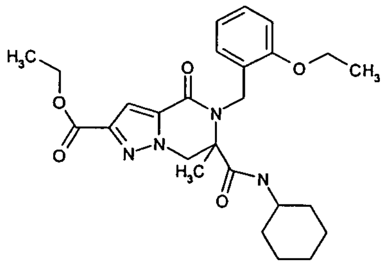 | 482,58468 |
| 331 | 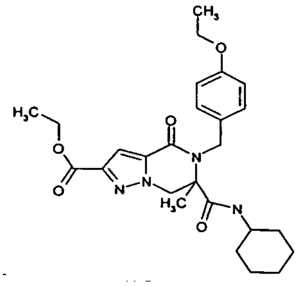 | 482,58468 |
| 332 | 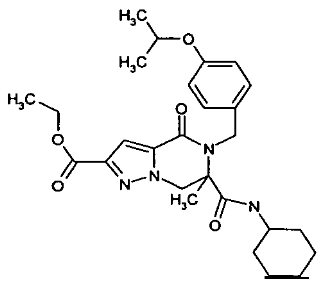 | 496,61177 |
| 333 | 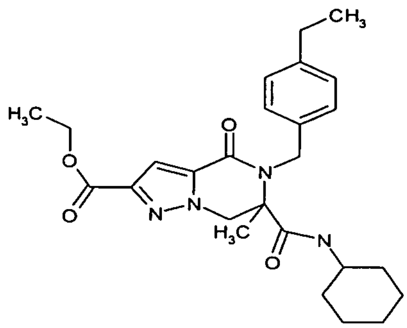 | 466,58528 |
| 334 | 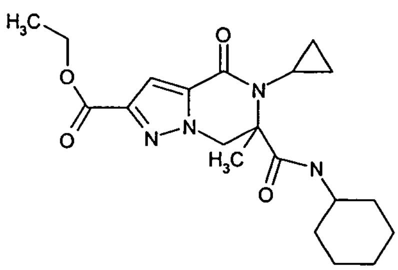 | 388,47056 |

(continued)

| № | Formula | Mol.w. |
|---|---|---|
| 335 | 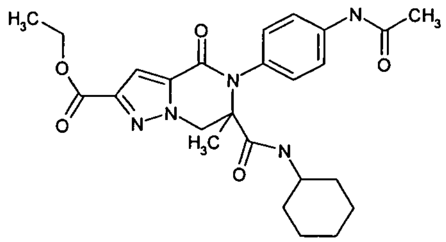 | 481,55632 |
| 336 | 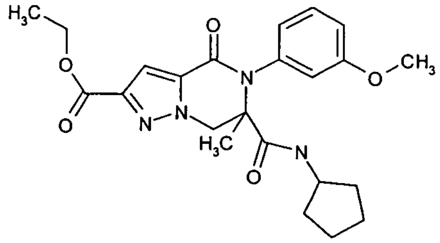 | 440,50341 |
| 337 | 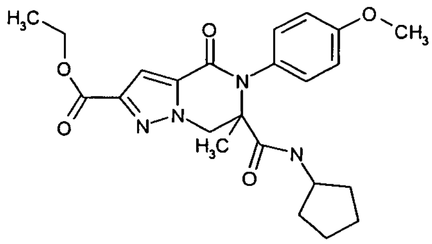 | 440,50341 |
| 338 | 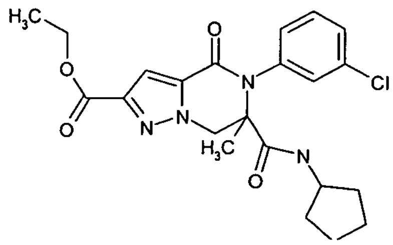 | 444,92195 |
| 339 | 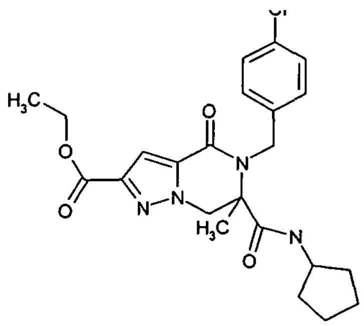 | 458,94904 |
| 340 | 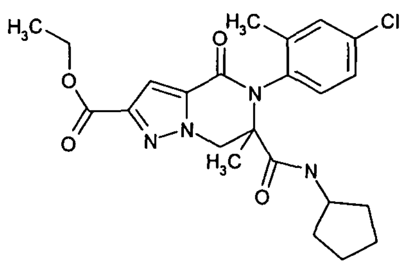 | 458,94904 |

(continued)

| № | Formula | Mol.w. |
|---|---|---|
| 341 | 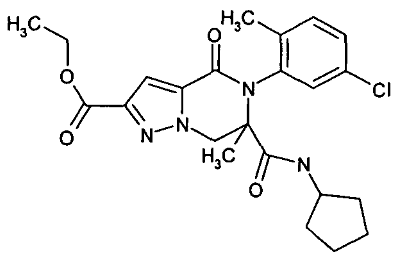 | 458,94904 |
| 342 | 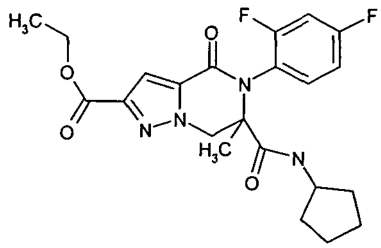 | 446,45778 |
| 343 | 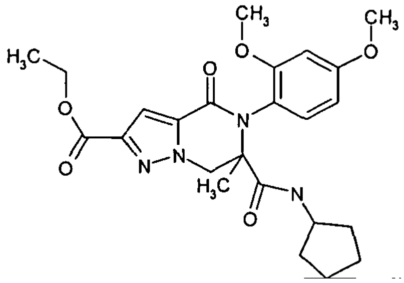 | 470,5299 |
| 344 | 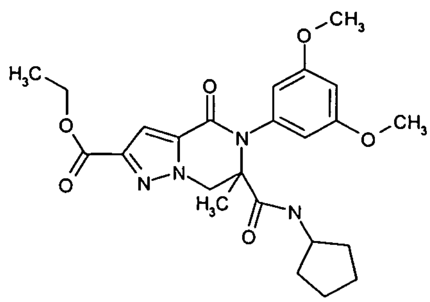 | 470,5299 |
| 345 | 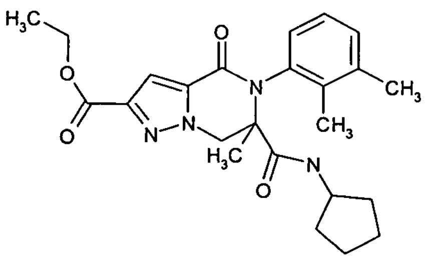 | 438,5311 |
| 346 | 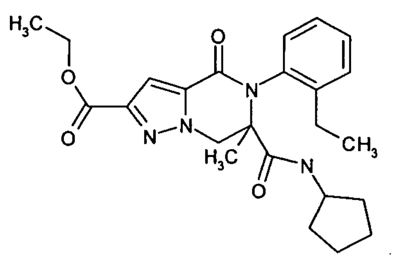 | 438,5311 |

(continued)

| № | Formula | Mol.w. |
|---|---|---|
| 347 | 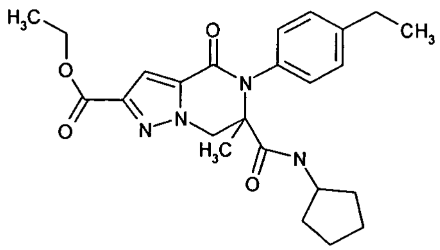 | 438,5311 |
| 348 | 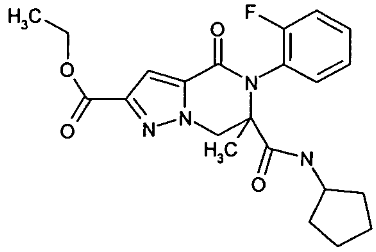 | 428,46735 |
| 349 | 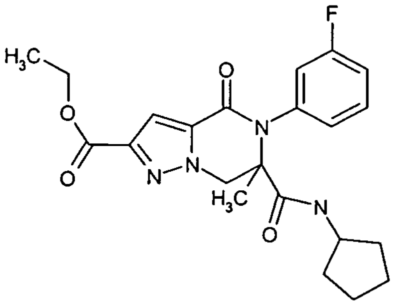 | 428,46735 |
| 350 | 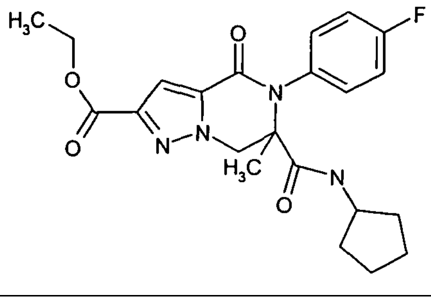 | 428,46735 |
| 351 | 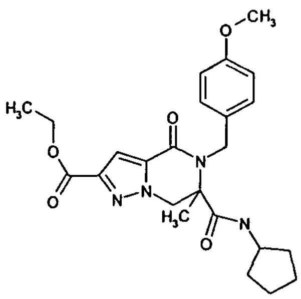 | 454,5305 |

(continued)

| № | Formula | Mol.w. |
|---|---|---|
| 352 | 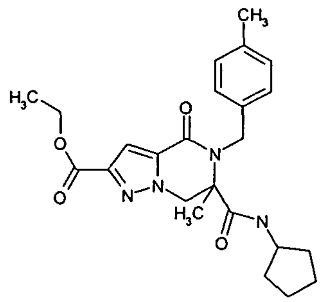 | 438,5311 |
| 353 | 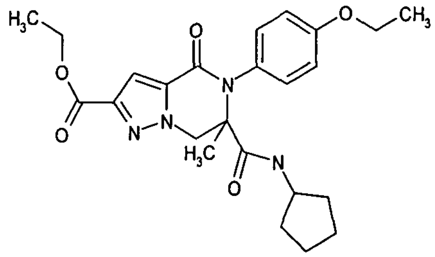 | 454,5305 |
| 354 | 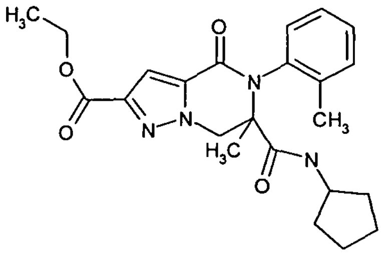 | 424,50401 |
| 355 | 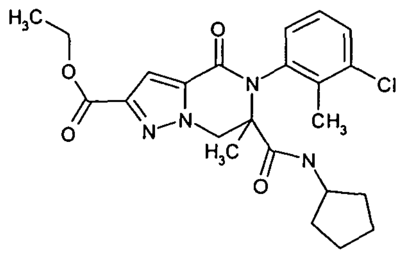 | 458,94904 |
| 356 | 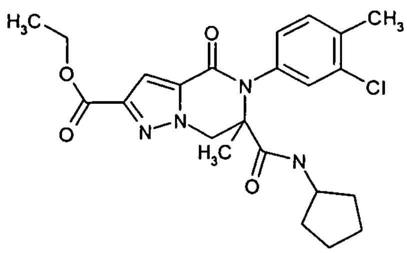 | 458,94904 |

(continued)

| № | Formula | Mol.w. |
|---|---|---|
| 357 | 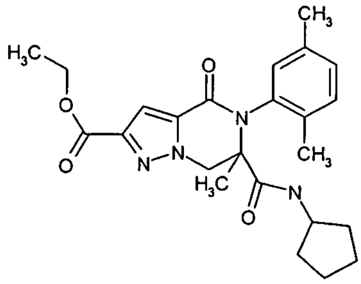 | 438,5311 |
| 358 | 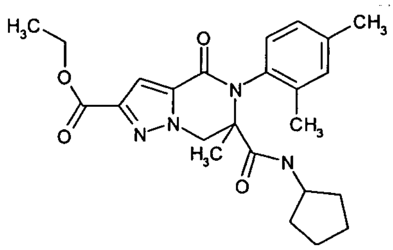 | 438,5311 |
| 359 | 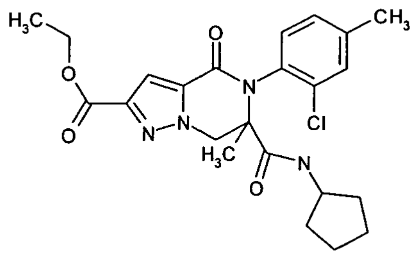 | 458,94904 |
| 360 | 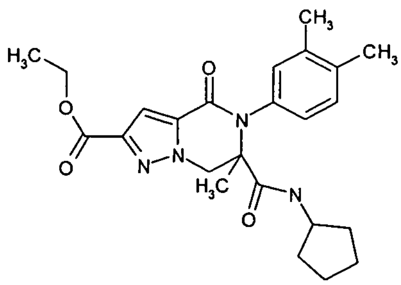 | 438, 5311 |
| 361 | 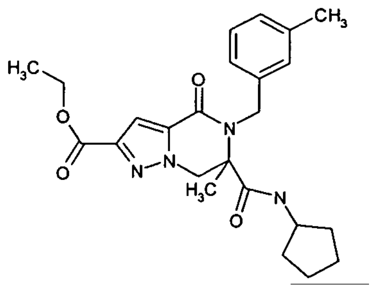 | 438,5311 |

(continued)

| № | Formula | Mol.w. |
|---|---|---|
| 362 | 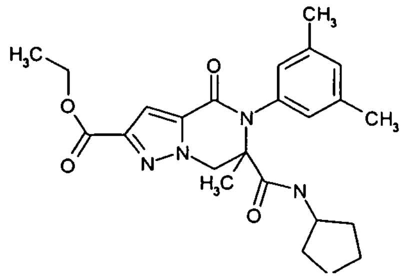 | 438,5311 |
| 363 | 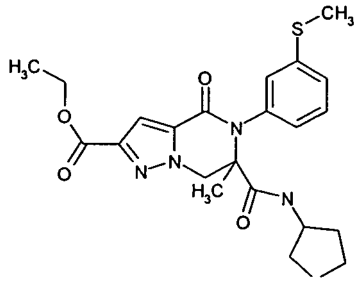 | 456,56801 |
| 364 | 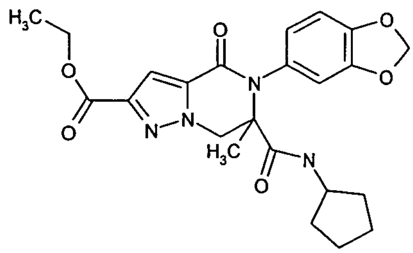 | 454,48687 |
| 365 | 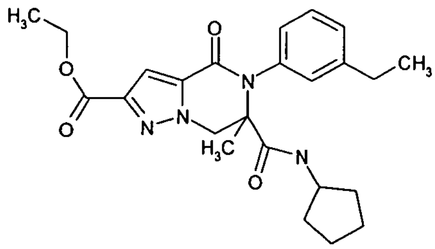 | 438,5311 |
| 366 | 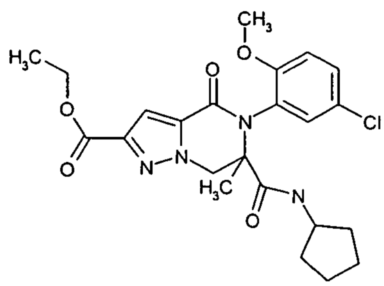 | 474,94844 |
| 367 | 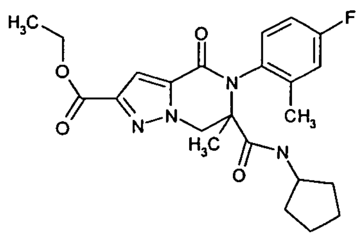 | 442,49444 |

(continued)

| № | Formula | Mol.w. |
|---|---|---|
| 368 | 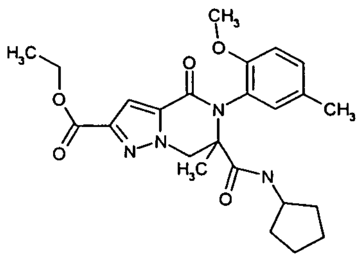 | 454,5305 |
| 369 | 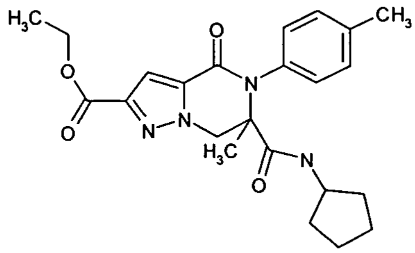 | 424,50401 |
| 370 | 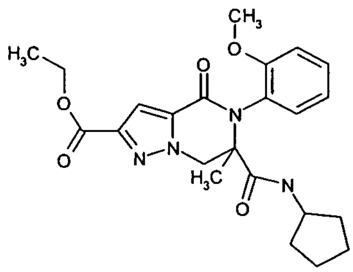 | 440,50341 |
| 371 | 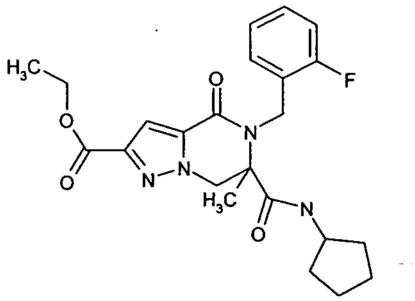 | 442,49444 |
| 372 | 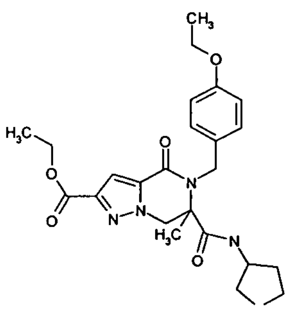 | 468,55759 |

(continued)

| № | Formula | Mol.w. |
|---|---|---|
| 373 | 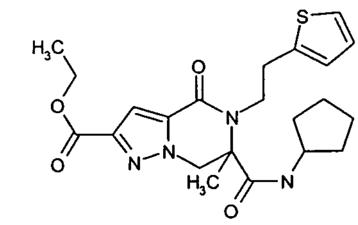 | 444,55686 |
| 374 | 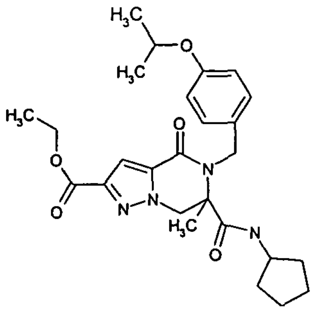 | 482,58468 |
| 375 | 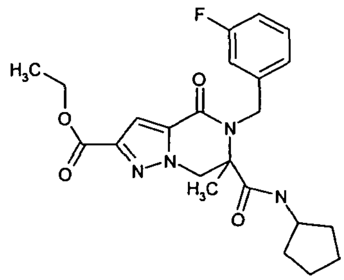 | 442,49444 |
| 376 | 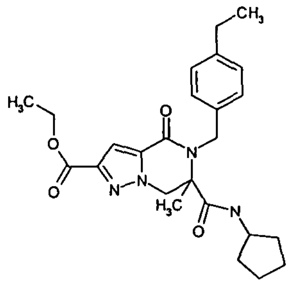 | 452,55819 |
| 377 | 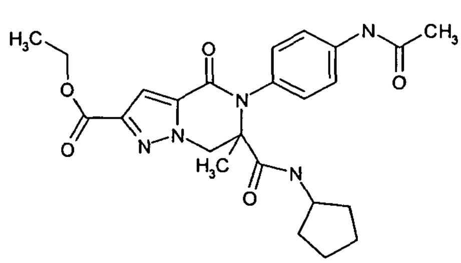 | 467,52923 |

(continued)

| № | Formula | Mol.w. |
|---|---|---|
| 378 | 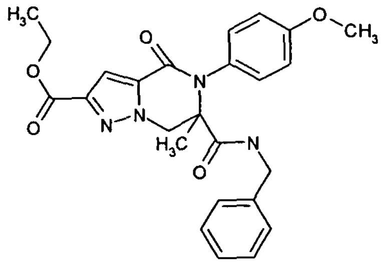 | 462,50977 |
| 379 | 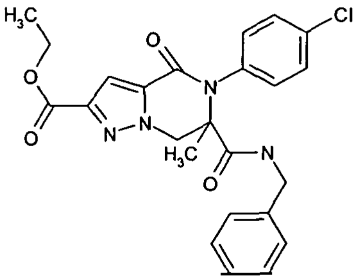 | 466,92831 |
| 380 | 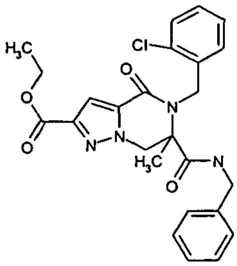 | 480,9554 |
| 381 | 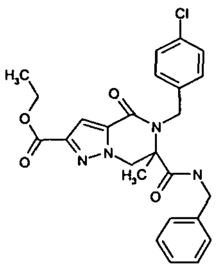 | 480,9554 |
| 382 | 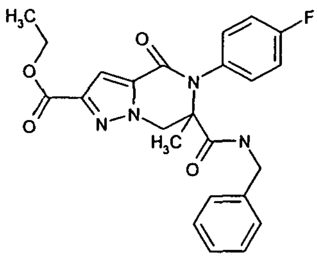 | 450,47371 |

(continued)

| № | Formula | Mol.w. |
|---|---|---|
| 383 | 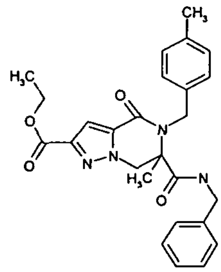 | 460,53746 |
| 384 | 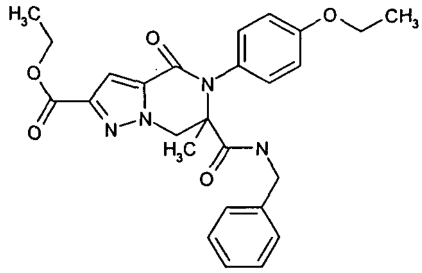 | 476,53686 |
| 385 | 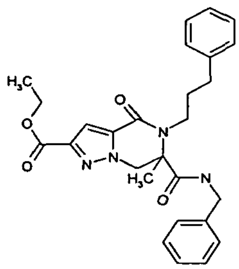 | 474,56455 |
| 386 | 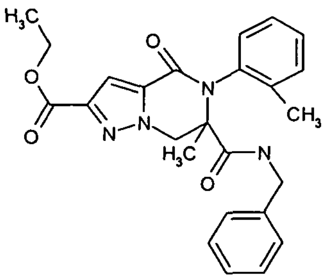 | 446,51037 |
| 387 | 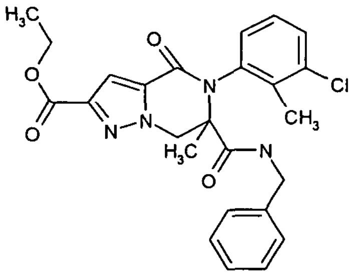 | 480,9554 |

(continued)

| № | Formula | Mol.w. |
|---|---|---|
| 388 | 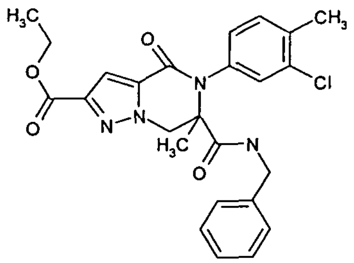 | 480,9554 |
| 389 | 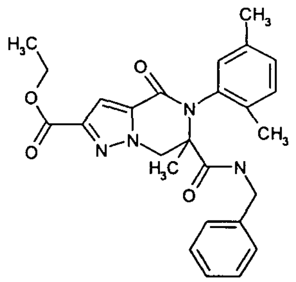 | 460,53746 |
| 390 | 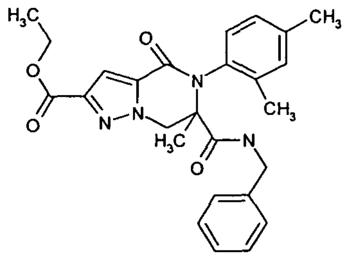 | 460,53746 |
| 391 | 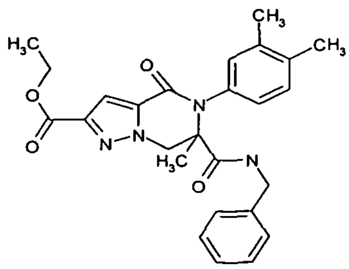 | 460,53746 |
| 392 | 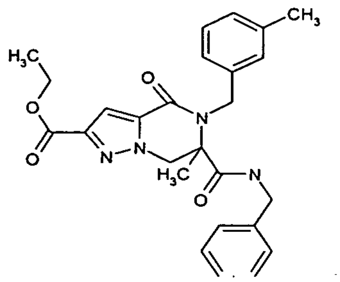 | 460,53746 |

(continued)

| № | Formula | Mol.w. |
|---|---|---|
| 393 | 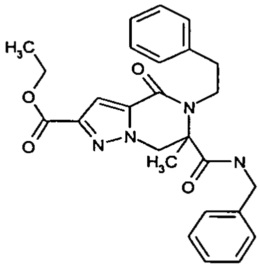 | 460,53746 |
| 394 | 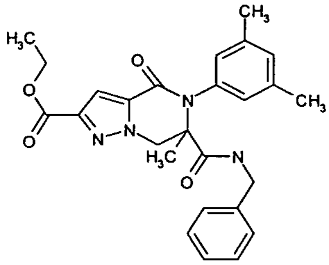 | 460,53746 |
| 395 | 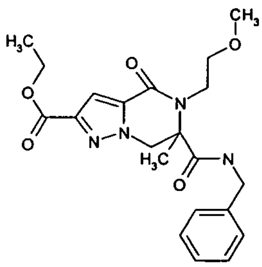 | 414,46517 |
| 396 | 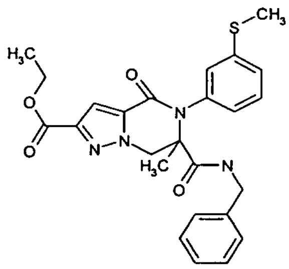 | 478,57437 |
| 397 | 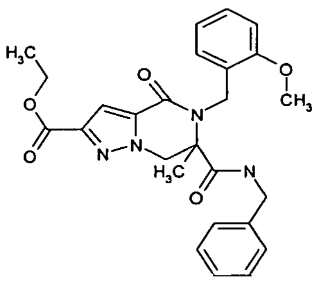 | 476,53686 |

(continued)

| № | Formula | Mol.w. |
|---|---|---|
| 398 | 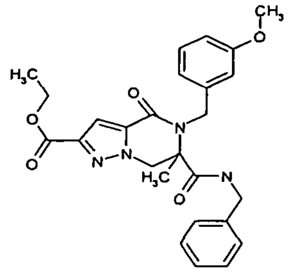 | 476,53686 |
| 399 | 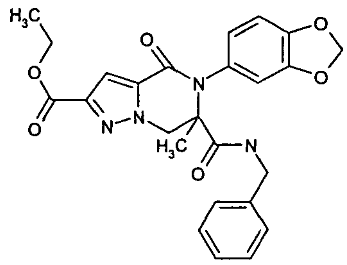 | 476,49323 |
| 400 | 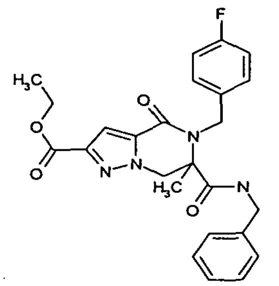 | 464,5008 |
| 401 | 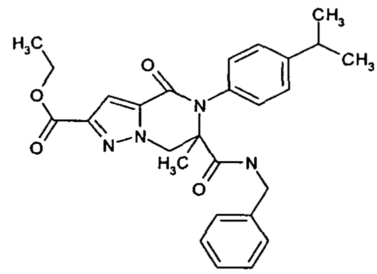 | 474,56455 |
| 402 | 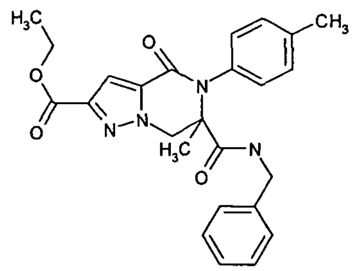 | 446,51037 |

(continued)

| № | Formula | Mol.w. |
|---|---|---|
| 403 | 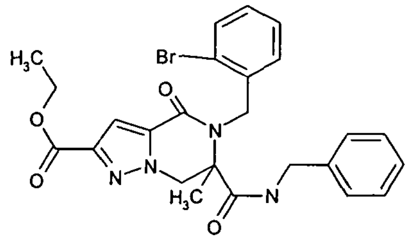 | 525,4064 |
| 404 | 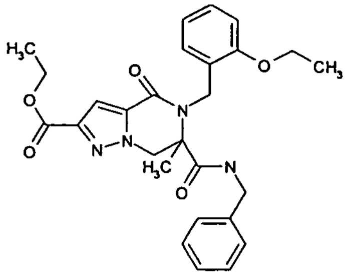 | 490,56395 |
| 405 | 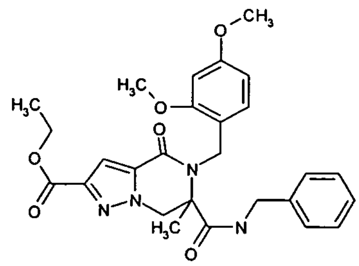 | 506,56335 |
| 406 | 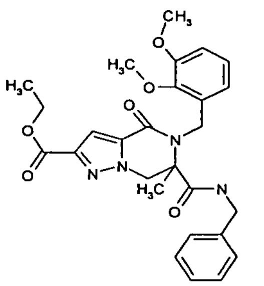 | 506,56335 |
| 407 | 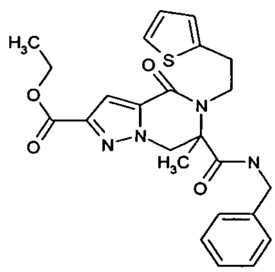 | 466,56322 |

(continued)

| № | Formula | Mol.w. |
|---|---|---|
| 408 | 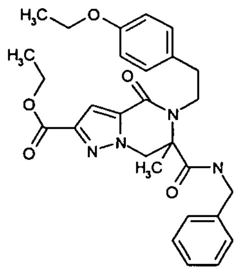 | 504,59104 |
| 409 | 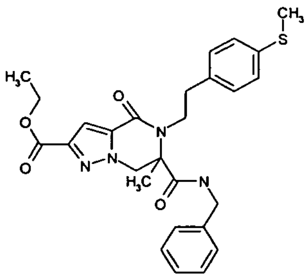 | 506,62855 |
| 410 | 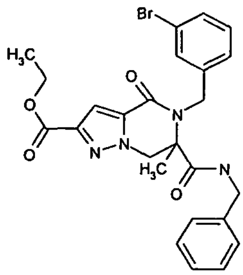 | 525,4064 |
| 411 | 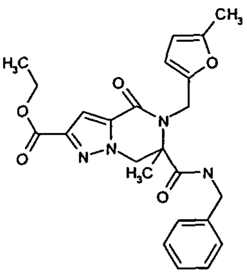 | 450,49862 |
| 412 | 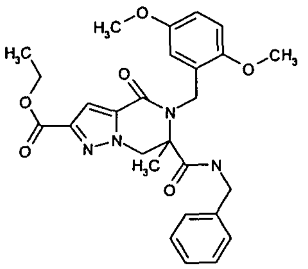 | 506,56335 |

(continued)

| № | Formula | Mol.w. |
|---|---|---|
| 413 | 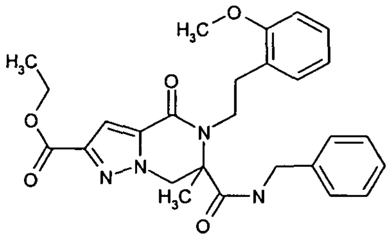 | 490,56395 |
| 414 | 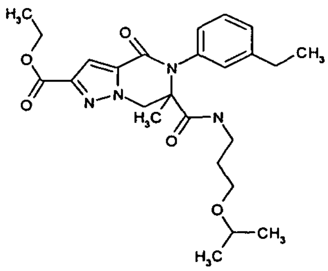 | 470,57353 |
| 415 | 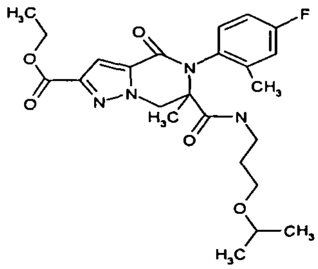 | 474,53687 |
| 416 | 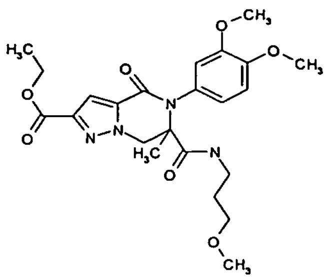 | 474,51815 |
| 417 | 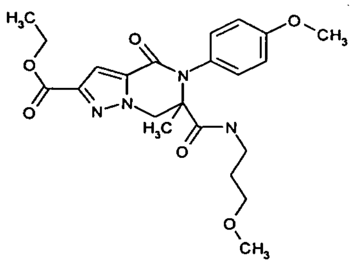 | 444,49166 |

(continued)

| № | Formula | Mol.w. |
|---|---|---|
| 418 | 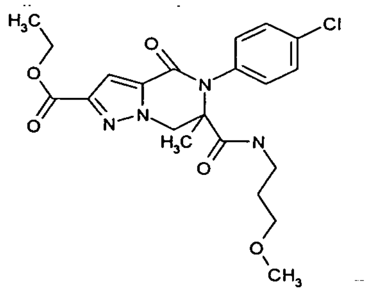 | I 448,9102 |
| 419 | 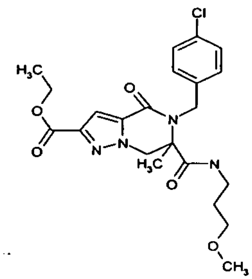 | 462,93729 |
| 420 | 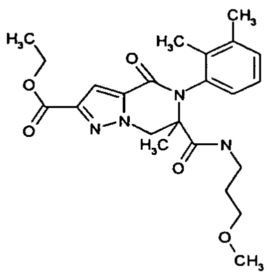 | 442,51935 |
| 421 | 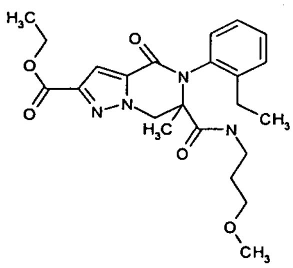 | 442,51935 |
| 422 | 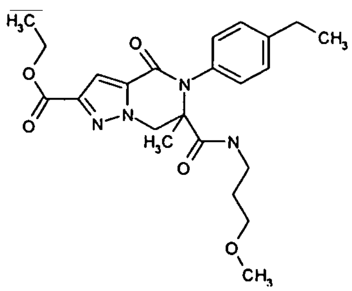 | 442,51935 |

(continued)

| № | Formula | Mol.w. |
|---|---|---|
| 423 | 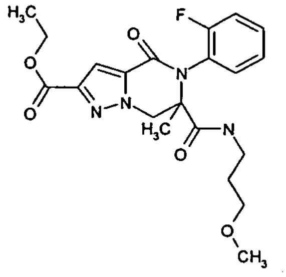 | 432,4556 |
| 424 | 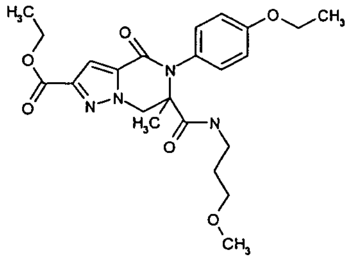 | i 458,51875 |
| 425 | 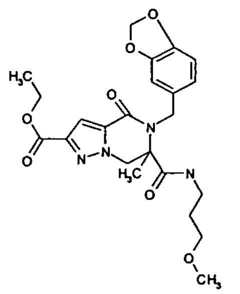 | 472,50221 |
| 426 | 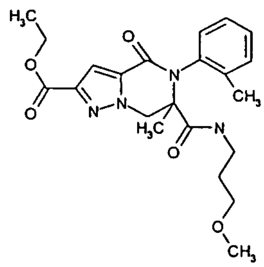 | 428,49226 |
| 427 | 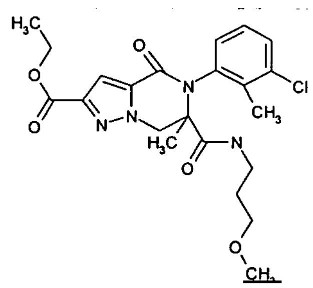 | 462,93729 |

(continued)

| № | Formula | Mol.w. |
|---|---|---|
| 428 | 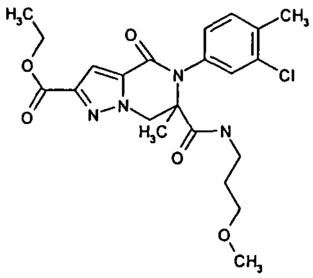 | 462,93729 |
| 429 | 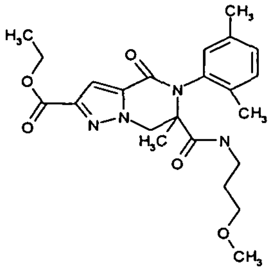 | 442,51935 |
| 430 | 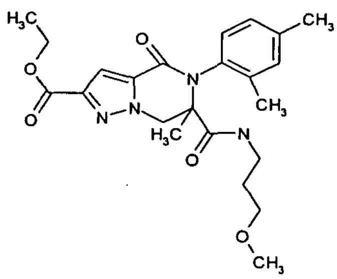 | 442,51935 |
| 431 | 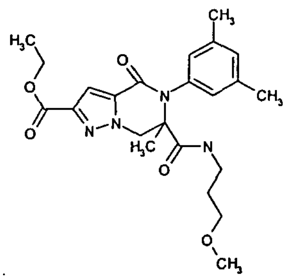 | 442,51935 |
| 432 | 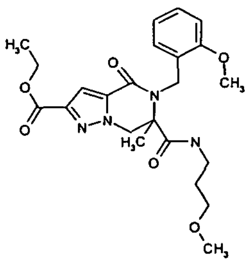 | 458,51875 |

(continued)

| № | Formula | Mol.w. |
|---|---|---|
| 433 | 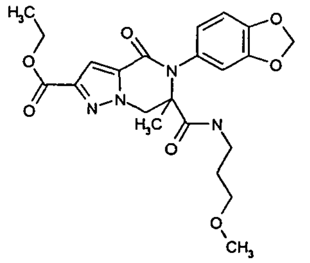 | 458,47512 |
| 434 | 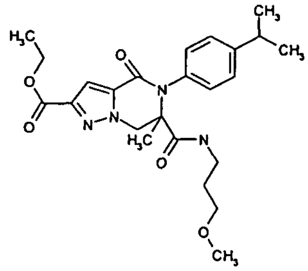 | 456,54644 |
| 435 | 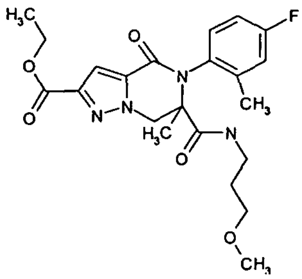 | 446,48269 |
| 436 | 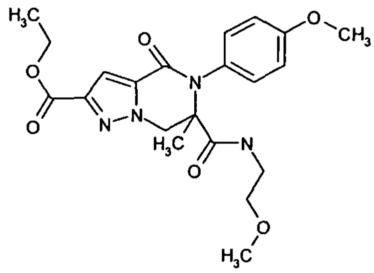 | 430,46457 |
| 437 | 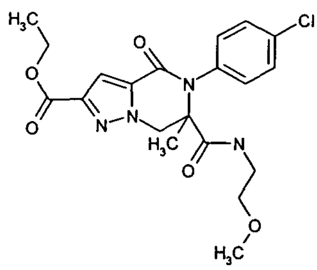 | 434,88311 |

(continued)

| Nº | Formula | Mol.w. |
|---|---|---|
| 438 | 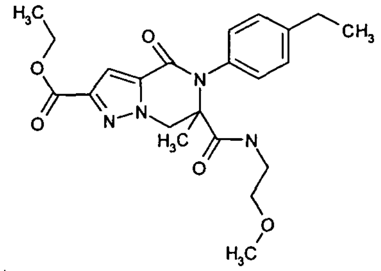 | 428,49226 |
| 439 | 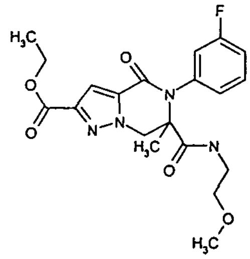 | 418,42851 |
| 440 | 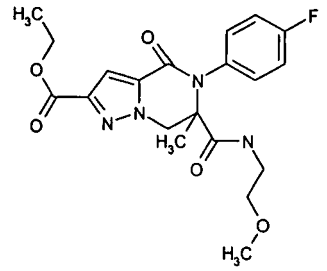 | 418,42851 |
| 441 | 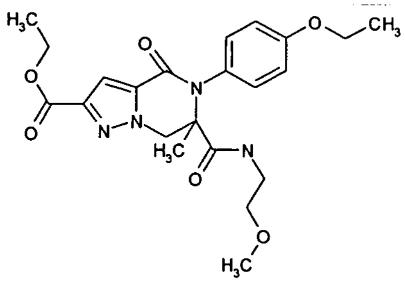 | 444,49166 |
| 442 | 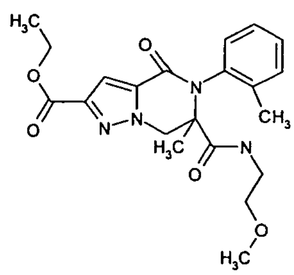 | 414,46517 |

(continued)

| № | Formula | Mol.w. |
|---|---|---|
| 443 | 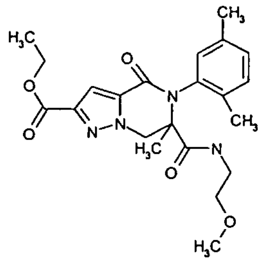 | 428,49226 |
| 444 | 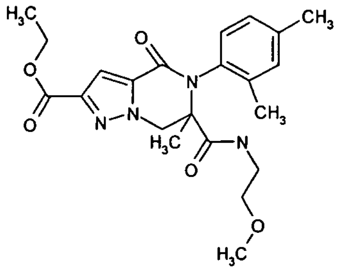 | 428,49226 |
| 445 | 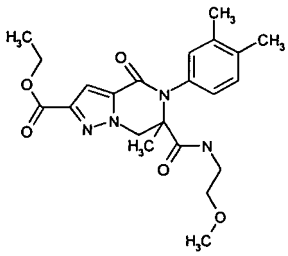 | 428,49226 |

| | Table 1.05.(6) | | |
|---|---|---|---|
| № | Formula | Mol. Weight | *m/z* (M+1) |
| 1 | 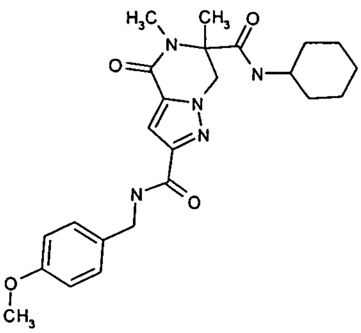 | 453,54577 | 454 |
| 2 | 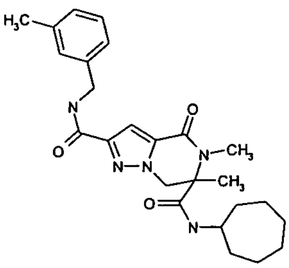 | 451,57346 | 452 |

(continued)

| | Table 1.05.(6) | | |
|---|---|---|---|
| № | Formula | Mol. Weight | *m*/*z* (M+1) |
| 3 | 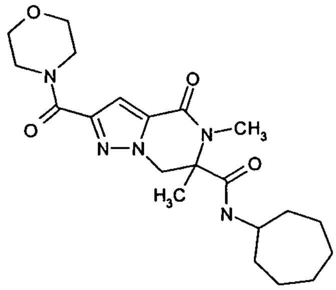 | 417,51232 | 418 |
| 4 | 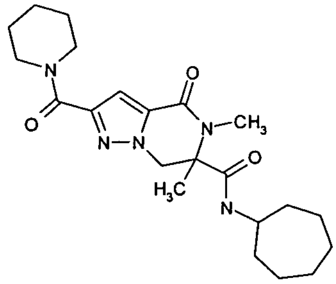 | 415,54001 | 416 |
| 5 | 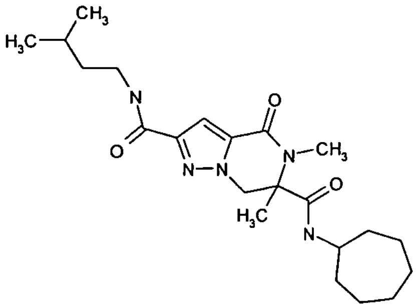 | 417,55595 | 418 |
| 6 | 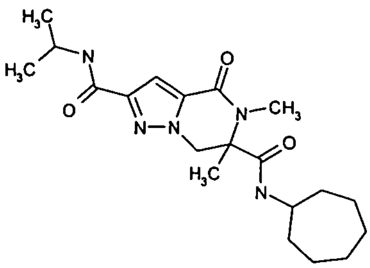 | 389,50177 | 390 |
| 7 | 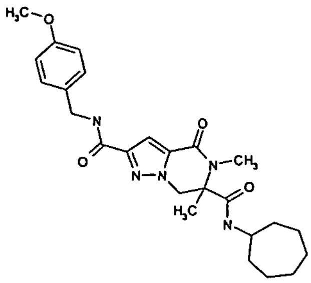 | 467,57286 | 468 |

(continued)

| | Table 1.05.(6) | | |
|---|---|---|---|
| № | Formula | Mol. Weight | m/z (M+1) |
| 8 | 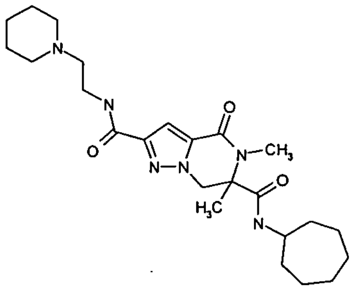 | 458,60886 | 459 |
| 9 | 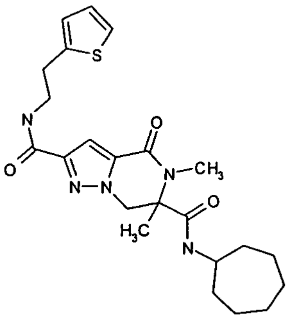 | 457,59922 | 458 |
| 10 | 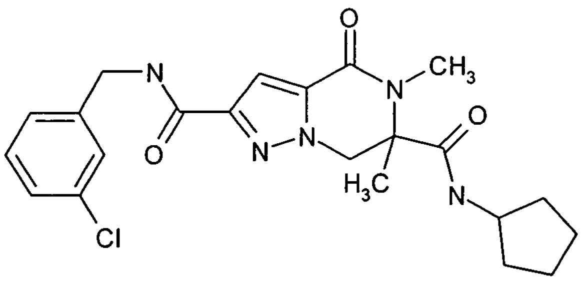 | 443,93722 | 444 |
| 11 | 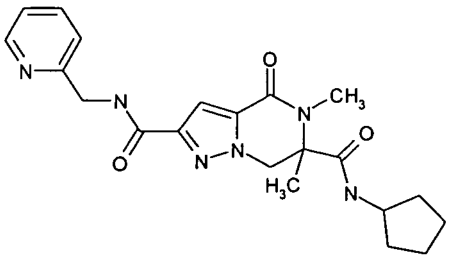 | 410,47977 | 411 |
| 12 | 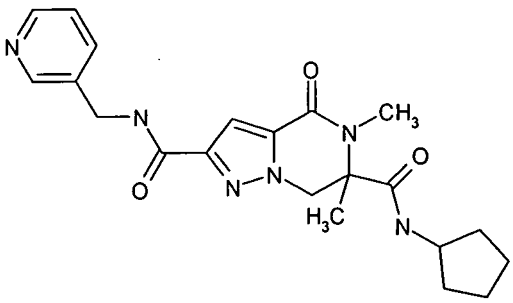 | 410.47977 | 411 |

(continued)

| | Table 1.05.(6) | | |
|---|---|---|---|
| № | Formula | Mol. Weight | *m*/*z* (M+1) |
| 13 | 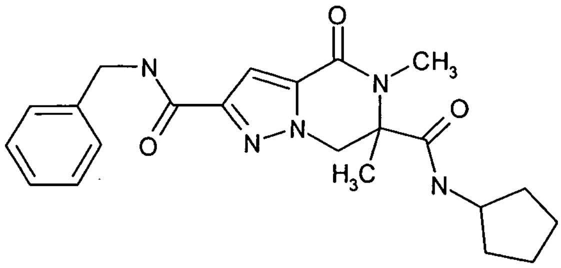 | 409,49219 | 410 |
| 14 | 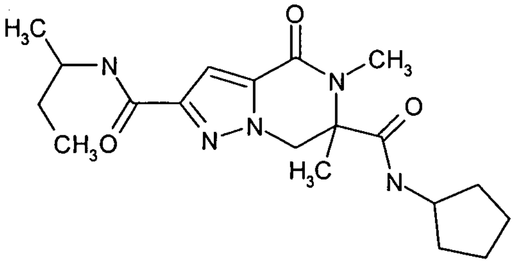 | 375,47468 | 376 |
| 15 | 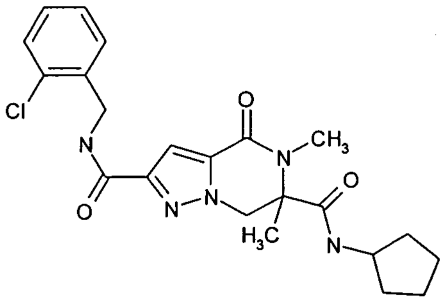 | 443,93722 | 444 |
| 16 | 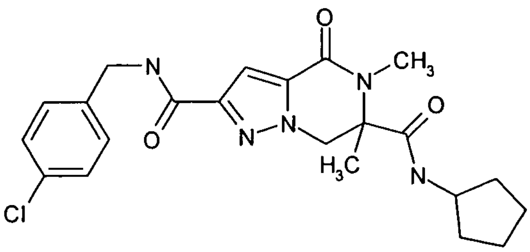 | 443,93722 | 444 |
| 17 | 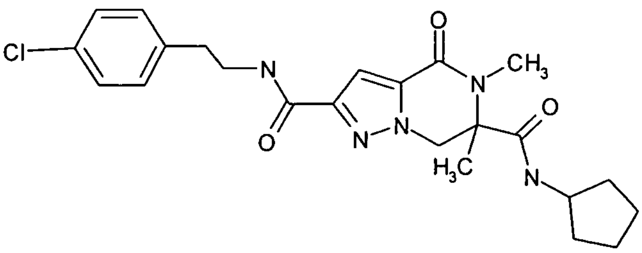 | 457,96431 | 458 |

(continued)

| | Table 1.05.(6) | | |
|---|---|---|---|
| № | Formula | Mol. Weight | *m/z* (M+1) |
| 18 | 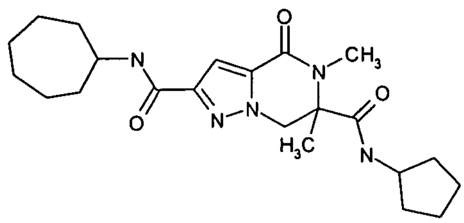 | 415,54001 | 416 |
| 19 | 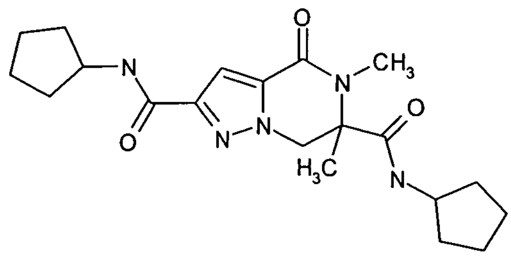 | 387,48583 | 388 |
| 20 | 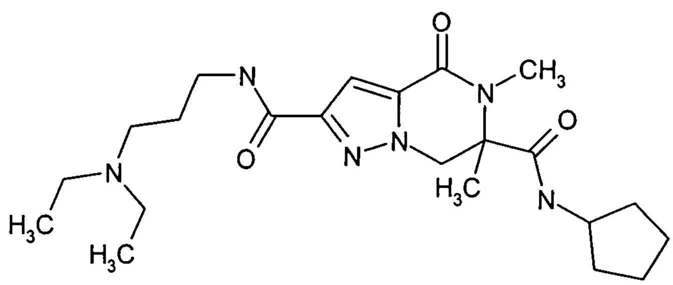 | 432,57062 | 433 |
| 21 | 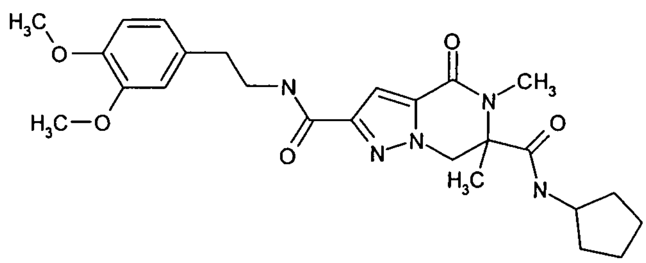 | 483,57226 | 484 |
| 22 | 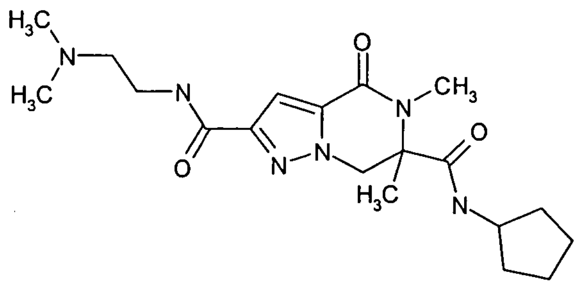 | 390,48935 | 391 |

(continued)

| | Table 1.05.(6) | | |
|---|---|---|---|
| № | Formula | Mol. Weight | *m*/*z* (M+1) |
| 23 | 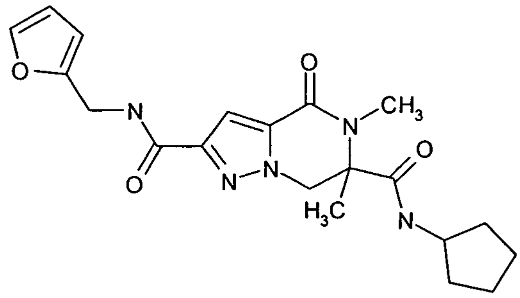 | 399,45335 | 400 |
| 24 | 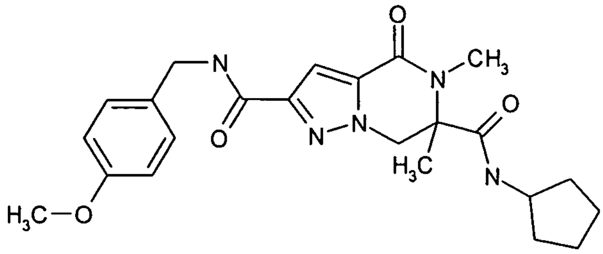 | 439,51868 | 440 |
| 25 | 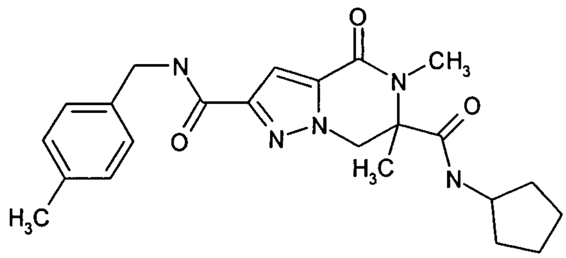 | 423,51928 | 424 |
| 26 | 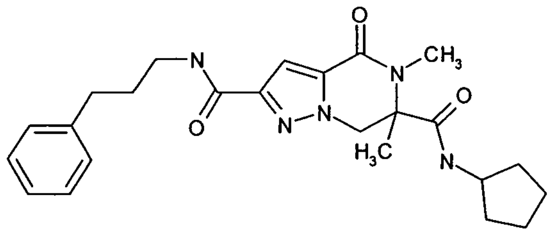 | 437,54637 | 438 |
| 27 | 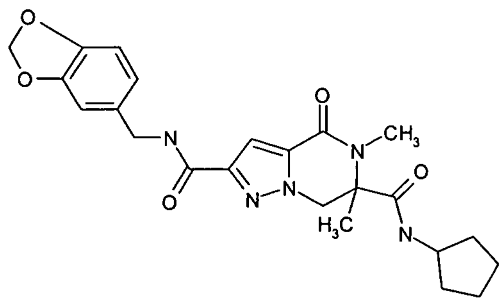 | 453,50214 | 454 |

(continued)

| | Table 1.05.(6) | | |
|---|---|---|---|
| № | Formula | Mol. Weight | *m/z* (M+1) |
| 28 | 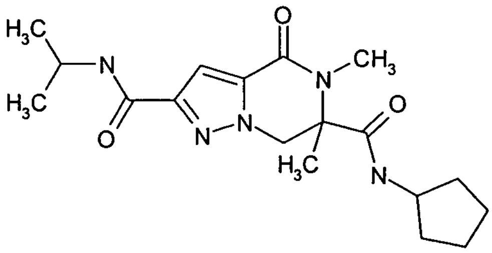 | 361,44759 | 362 |
| 29 | 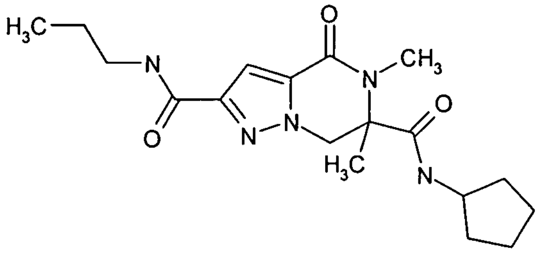 | 361,44759 | 362 |
| 30 | 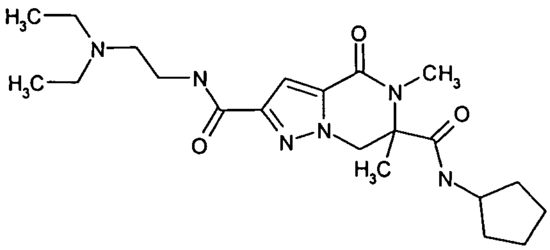 | 418,54353 | 419 |
| 31 | 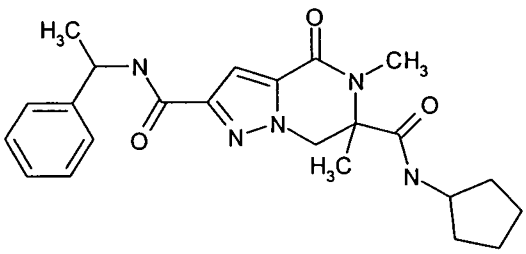 | 423,51928 | 424 |
| 32 | 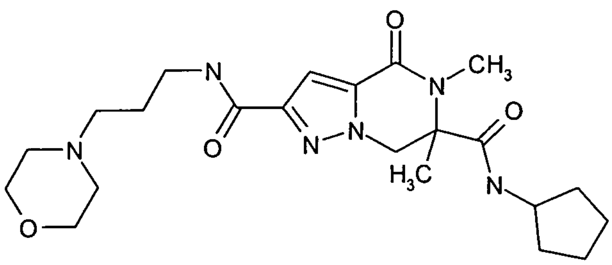 | 446,55408 | 447 |

(continued)

| | Table 1.05.(6) | | |
|---|---|---|---|
| № | Formula | Mol. Weight | *m/z* (M+1) |
| 33 | 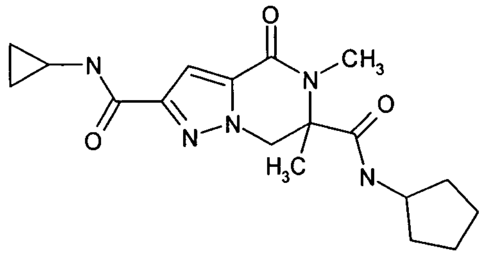 | 359,43165 | 360 |
| 34 I | 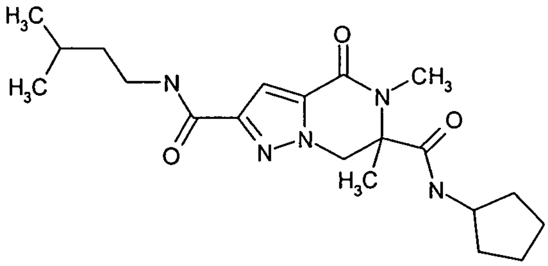 | 389,50177 | 390 |
| 35 | 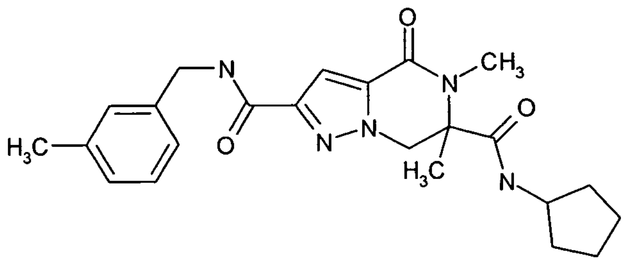 | 423,51928 | 424 |
| 36 | 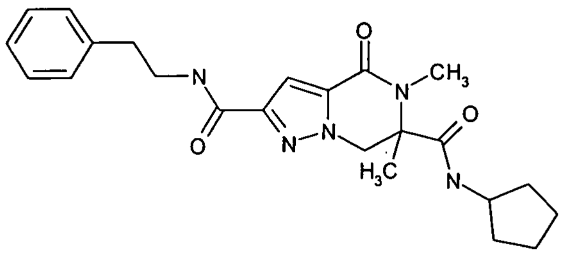 | 423,51928 | 424 |
| 37 | 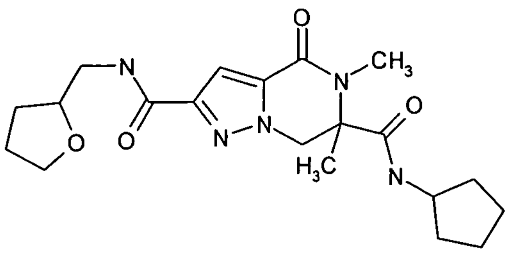 | 403,48523 | 404 |

(continued)

| | Table 1.05.(6) | | |
|---|---|---|---|
| № | Formula | Mol. Weight | m/z (M+1) |
| 38 | 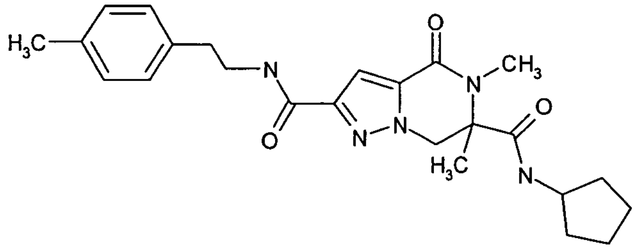 | 437,54637 | 438 |
| 39 | 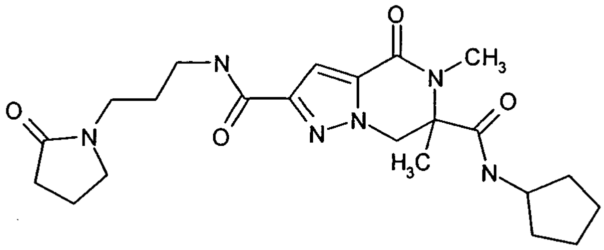 | 444,53814 | 445 |
| 40 | 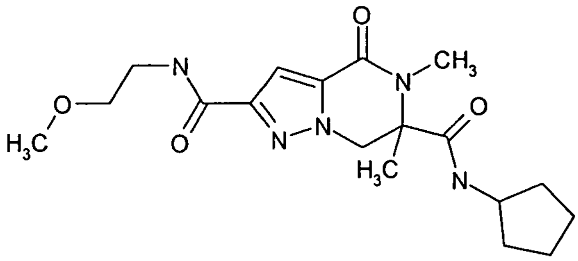 | 377,44699 | 378 |
| 41 | 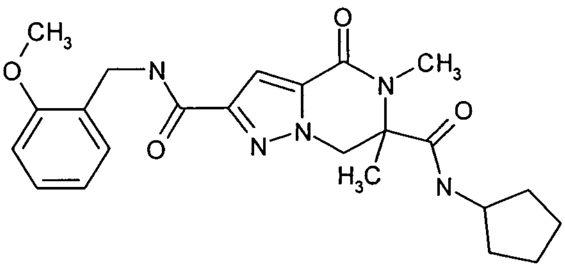 | 439,51868 | 440 |
| 42 | 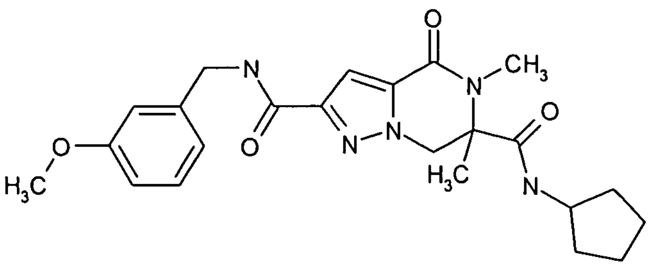 | 439,51868 | 440 |

(continued)

| | Table 1.05.(6) | | |
|---|---|---|---|
| № | Formula | Mol. Weight | *m*/*z* (M+1) |
| 43 | 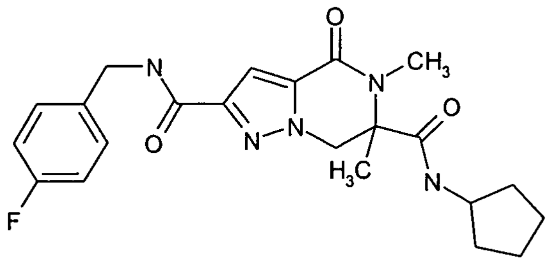 | 427,48262 | 428 |
| 44 | 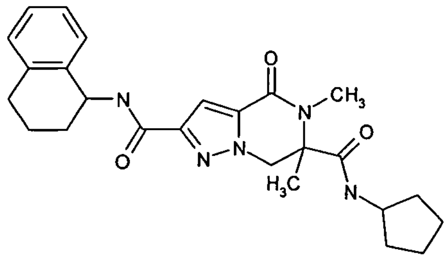 | 449,55752 | 450 |
| 45 | 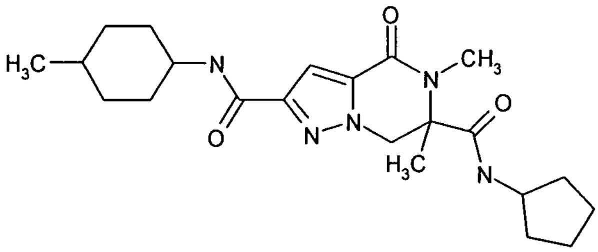 | 415,54001 | 416 |
| 46 | 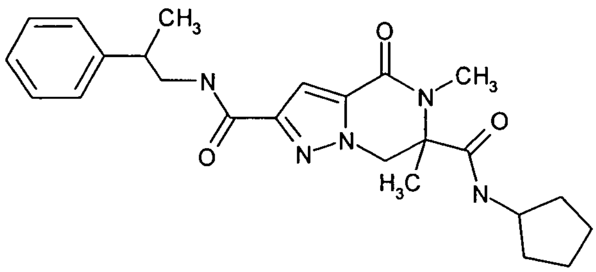 | 437,54637 | 438 |
| 47 | 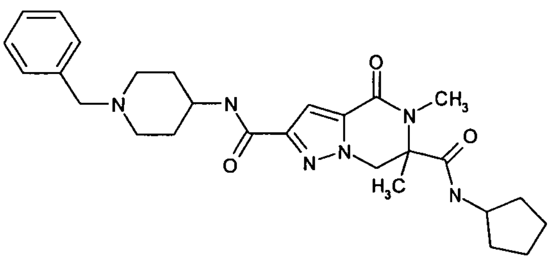 | 492,62637 | 493 |

(continued)

| | Table 1.05.(6) | | |
|---|---|---|---|
| № | Formula | Mol. Weight | *m*/*z* (M+1) |
| 48 | 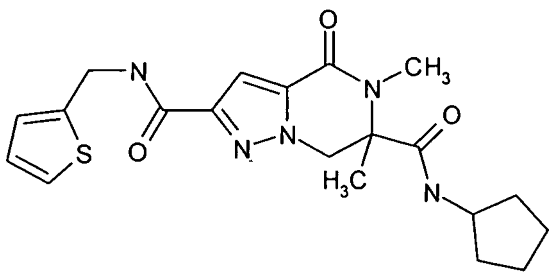 | 415,51795 | 416 |
| 49 | 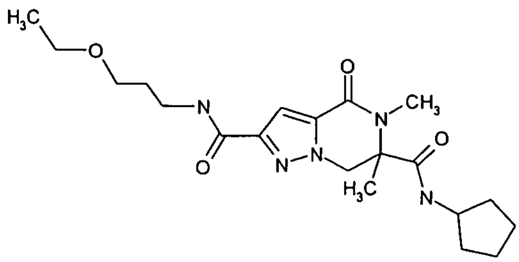 | 405,50117 | 406 |
| 50 | 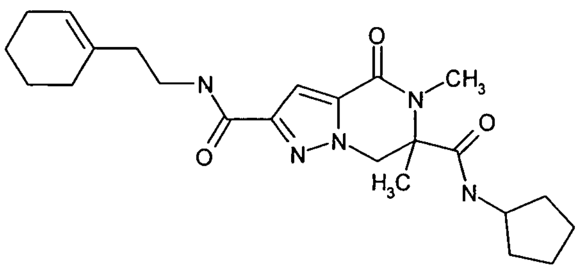 | 427,55116 | 428 |
| 51 | 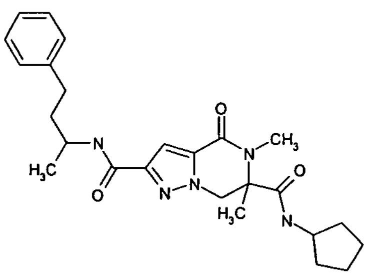 | 451,57346 | 452 |
| 52 | 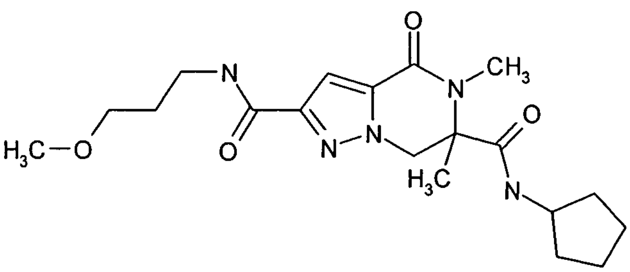 | 391,47408 | 392 |

(continued)

| | Table 1.05.(6) | | |
|---|---|---|---|
| № | Formula | Mol. Weight | *m/z* (M+1) |
| 53 | 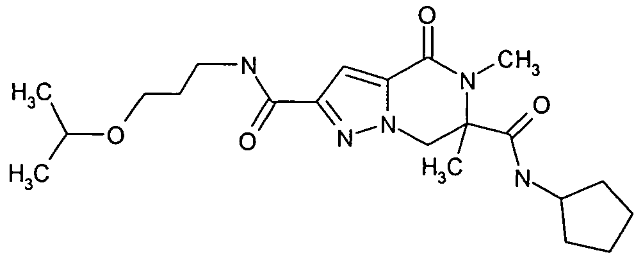 | 419,52826 | 420 |
| 54 | 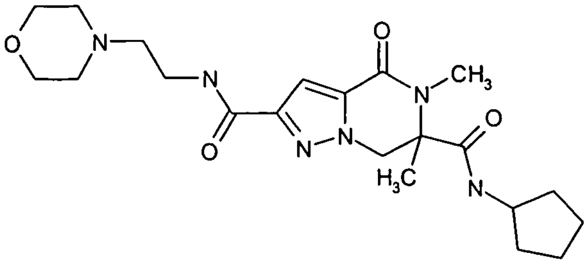 | 432,52699 | 433 |
| 55 | 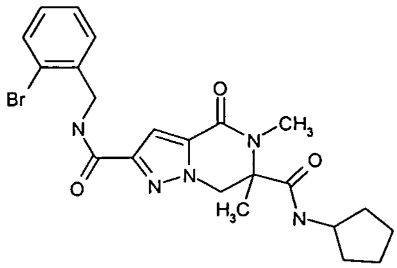 | 488,38822 | 489 |
| 56 | 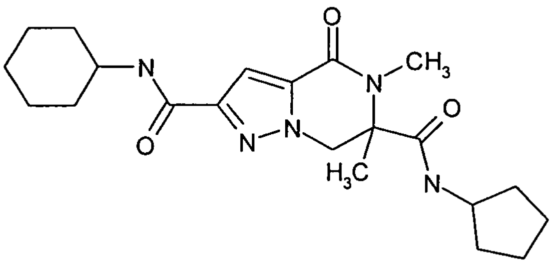 | 401,51292 | 402 |
| 57 | 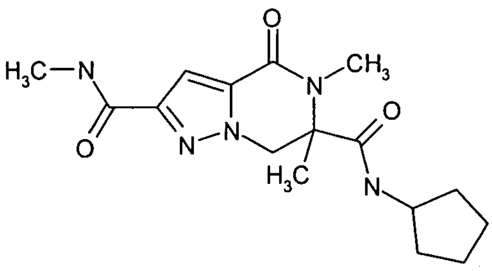 | 333,39341 | 334 |

(continued)

| | Table 1.05.(6) | | |
|---|---|---|---|
| № | Formula | Mol. Weight | *m/z* (M+1) |
| 58 | 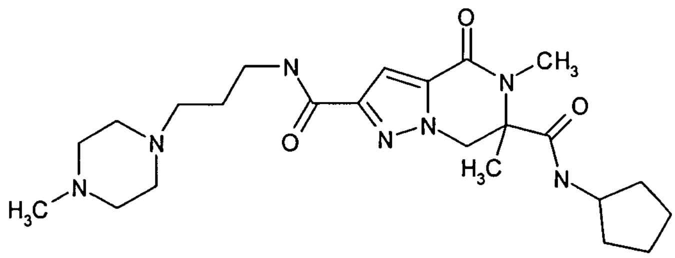 | 459,59644 | 460 |
| 59 | 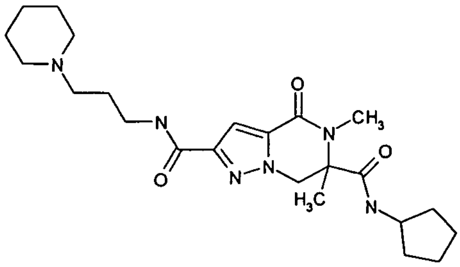 | 444,58177 | 445 |
| 60 | 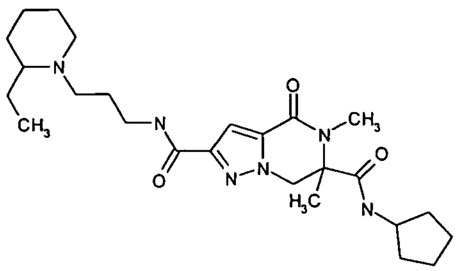 | 472,63595 | 473 |
| 61 | 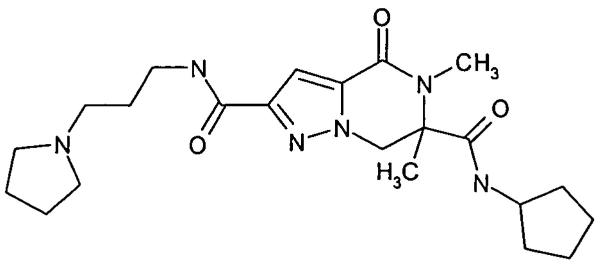 | 430,55468 | 431 |
| 62 | 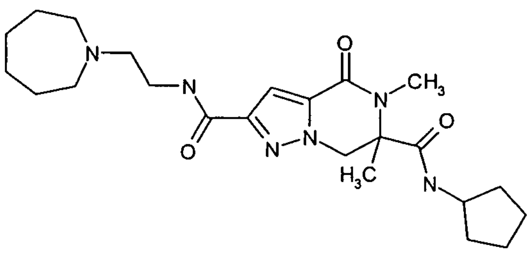 | 444,58177 | 445 |

(continued)

| | Table 1.05.(6) | | |
|---|---|---|---|
| № | Formula | Mol. Weight | *m*/*z* (M+1) |
| 63 | 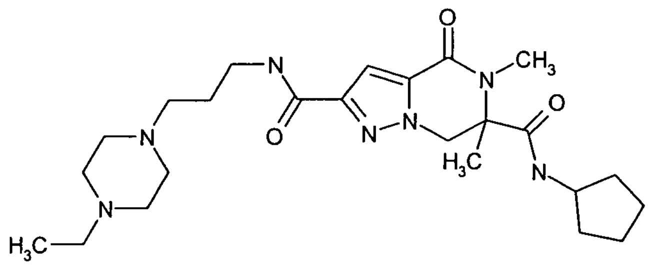 | 473,62353 | 474 |
| 64 | 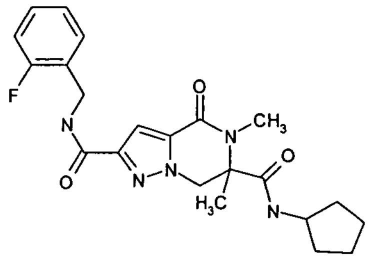 | 427,48262 | 428 |
| 65 | 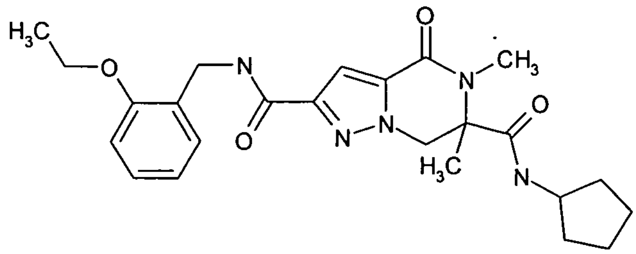 | 453,54577 | 454 |
| 66 | 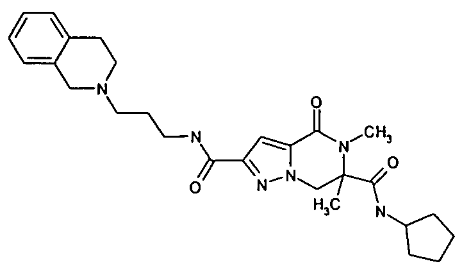 | 492,62637 | 493 |
| 67 | 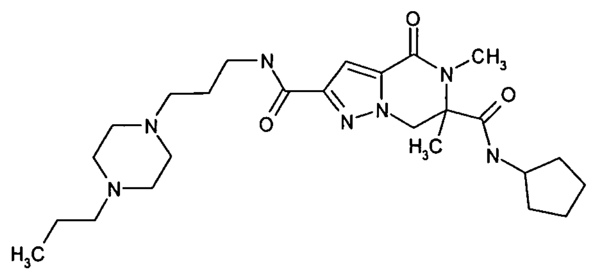 | 487,65062 | 488 |

(continued)

| | Table 1.05.(6) | | |
|---|---|---|---|
| № | Formula | Mol. Weight | *m*/*z* (M+1) |
| 68 | 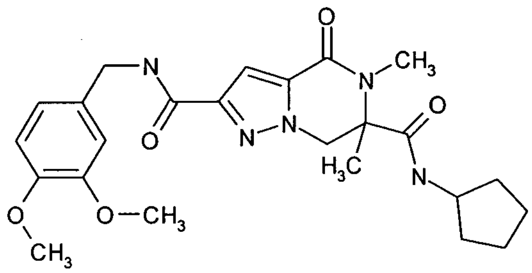 | 469,54517 | 470 |
| 69 | 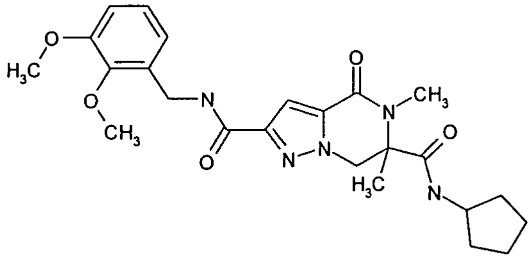 | 469,54517 | 470 |
| 70 | 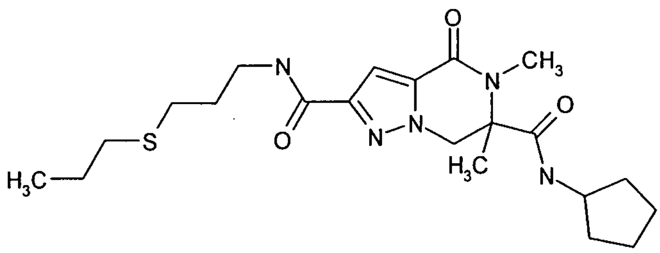 | 435,59286 | 436 |
| 71 | 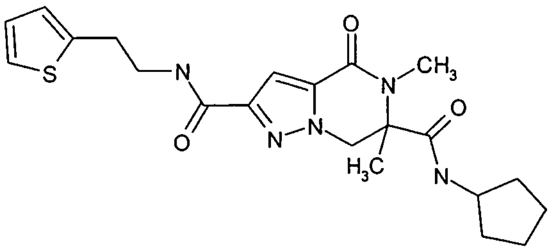 | 429,54504 | 430 |
| 72 | 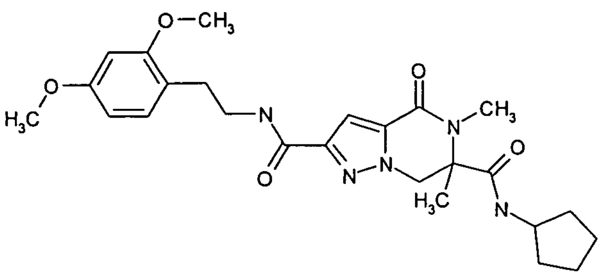 | 483,57226 | 484 |

(continued)

| | Table 1.05.(6) | | |
|---|---|---|---|
| № | Formula | Mol. Weight | m/z (M+1) |
| 73 | 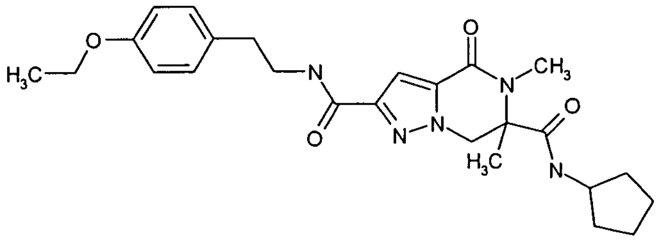 | 467,57286 | 468 |
| 74 | 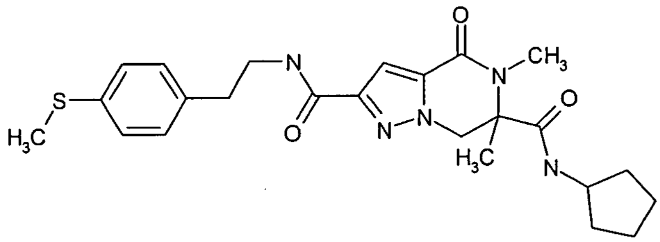 | 469,61037 | 470 |
| 75 | 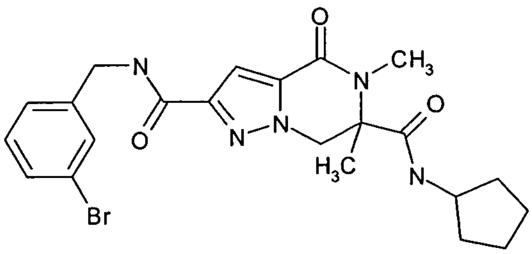 | 488,38822 | 489 |
| 76 | 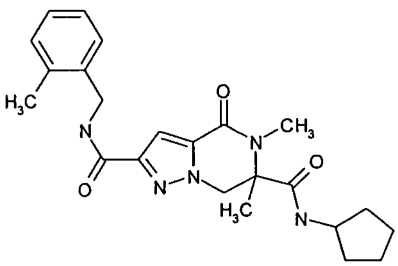 | 423,51928 | 424 |
| 77 | 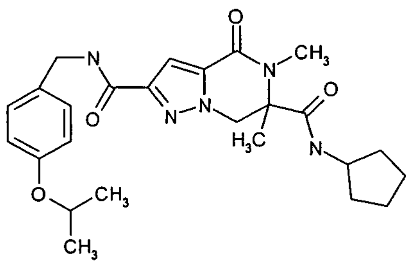 | 467,57286 | 468 |

(continued)

| | Table 1.05.(6) | | |
|---|---|---|---|
| № | Formula | Mol. Weight | *m*/*z* (M+1) |
| 78 | 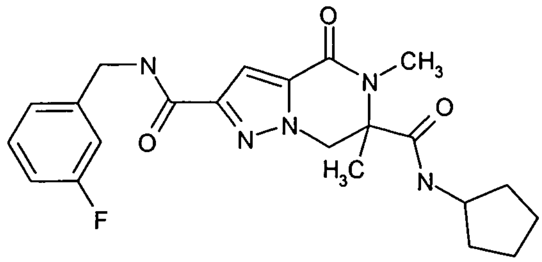 | 427,48262 | 428 |
| 79 | 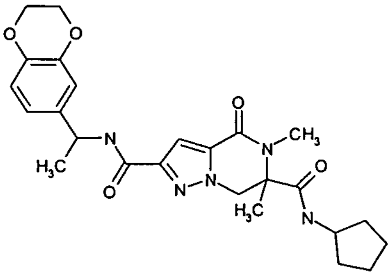 | 481,55632 | 482 |
| 80 | 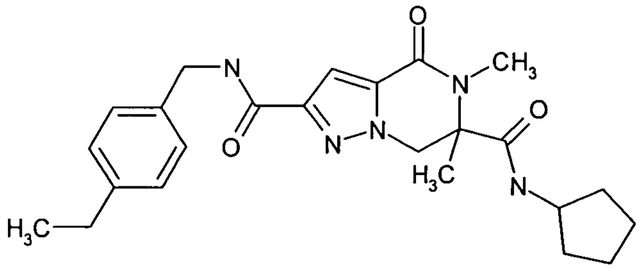 | 437,54637 | 438 |
| 81 | 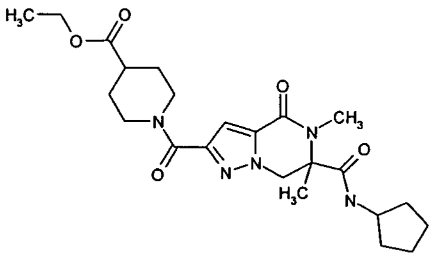 | 459,54996 | 460 |
| 82 | 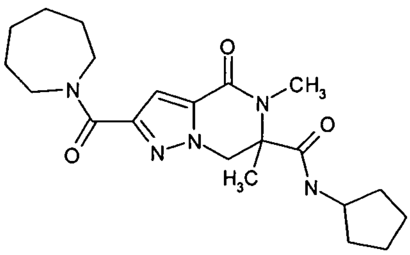 | 401,51292 | 402 |

(continued)

| | Table 1.05.(6) | | |
|---|---|---|---|
| № | Formula | Mol. Weight | *m*/*z* (M+1) |
| 83 | 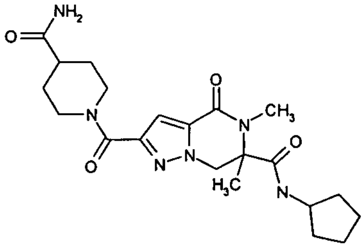 | 430,51105 | 431 |
| 84 | 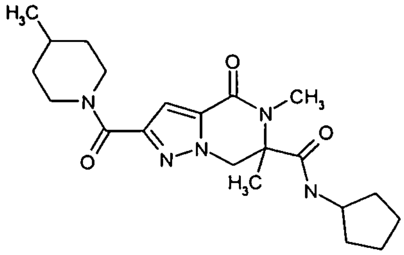 | 401,51292 | 402 |
| 85 | 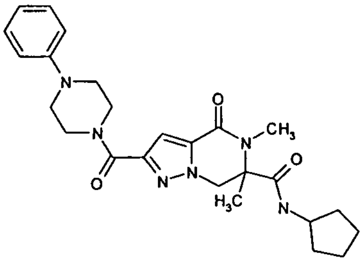 | 464,57219 | 465 |
| 86 | 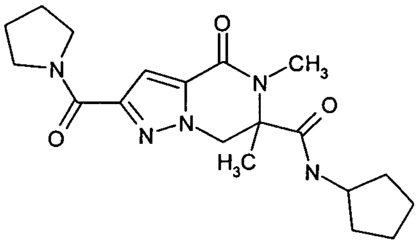 | 373,45874 | 374 |
| 87 | 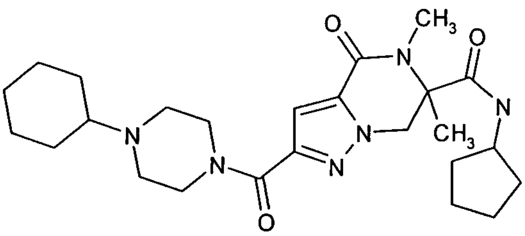 | 470,62001 | 471 |

(continued)

| | Table 1.05.(6) | | |
|---|---|---|---|
| № | Formula | Mol. Weight | *m*/*z* (M+1) |
| 88 | 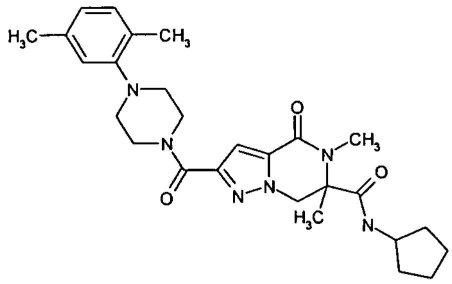 | 492,62637 | 493 |
| 89 | 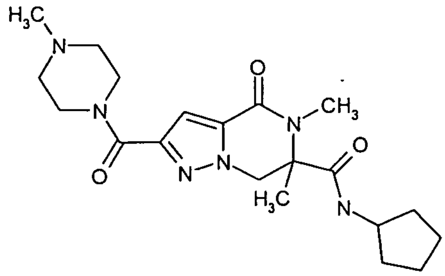 | 402,5005 | 403 |
| 90 | 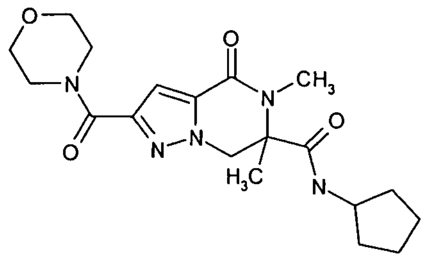 | 389,45814 | 390 |
| 91 | 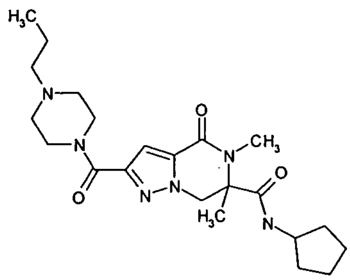 | 430,55468 | 431 |
| 92 | 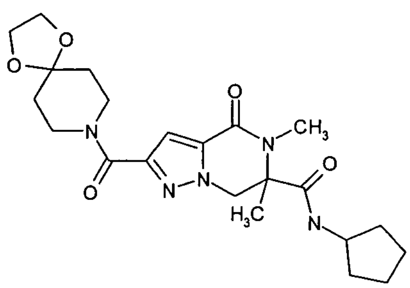 | 445,52287 | 446 |

(continued)

| | Table 1.05.(6) | | |
|---|---|---|---|
| № | Formula | Mol. Weight | *m/z* (M+1) |
| 93 | 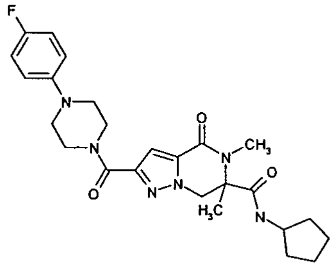 | 482,56262 | 483 |
| 94 | 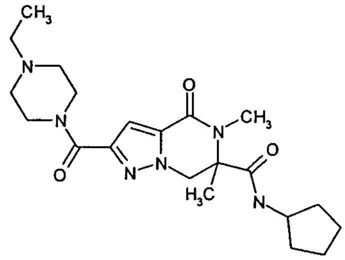 | 416,52759 | 417 |
| 95 | 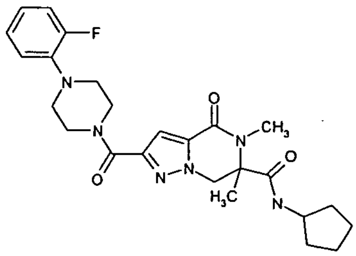 | 482,56262 | 483 |
| 96 | 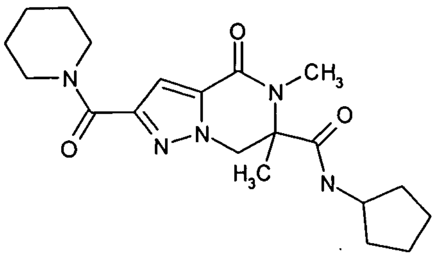 | 387,48583 | 388 |
| 97 | 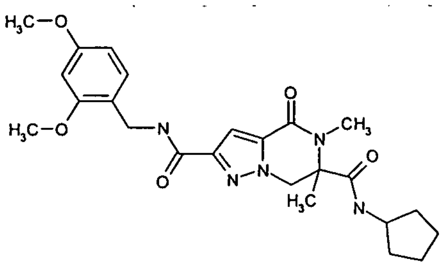 | 469,54517 | 470 |

(continued)

| | Table 1.05.(6) | | |
|---|---|---|---|
| № | Formula | Mol. Weight | m/z (M+1) |
| 98 | 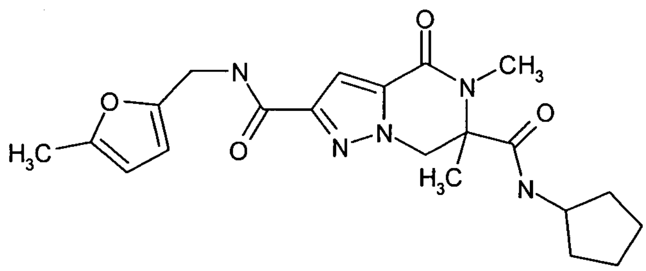 | 413,48044 | 414 |
| 99 | 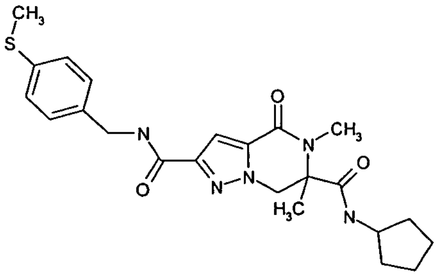 | 455,58328 | 456 |
| 100 | 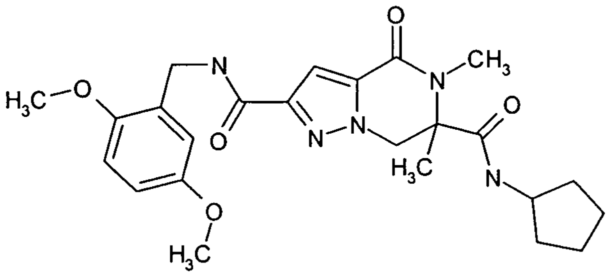 | 469,54517 | 470 |
| 101 | 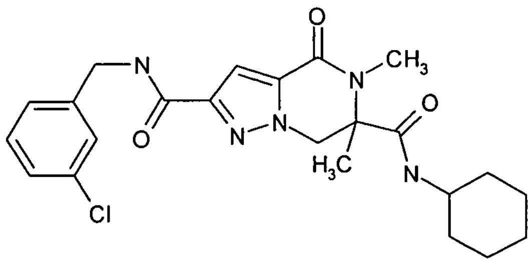 | 457,96431 | 458 |
| 102 | 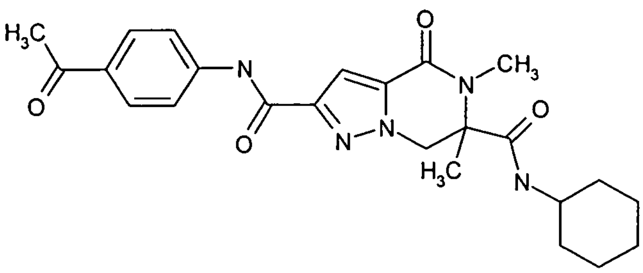 | 451,52983 | 452 |

(continued)

| | Table 1.05.(6) | | |
|---|---|---|---|
| № | Formula | Mol. Weight | *m*/*z* (M+1) |
| 103 | 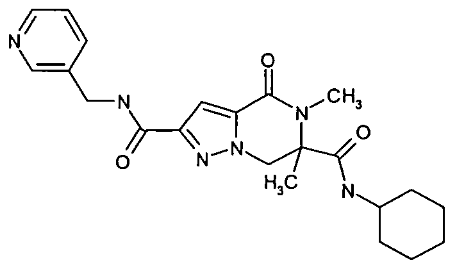 | 424,50686 | 425 |
| 104 | 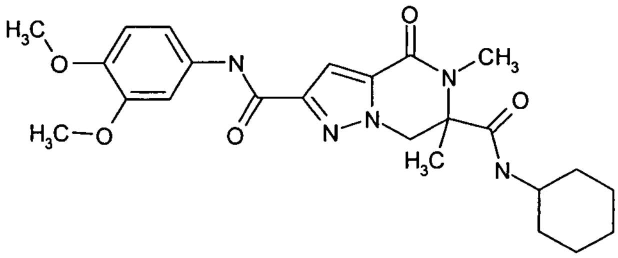 | 469,54517 | 470 |
| 105 | 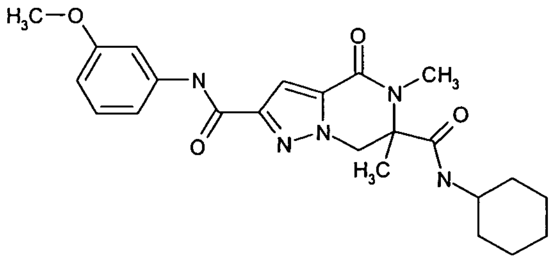 | 439,51868 | 440 |
| 106 | 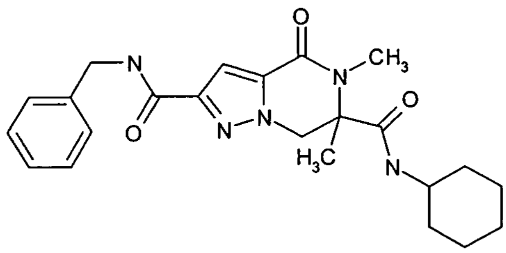 | 423,51928 | 424 |
| 107 | 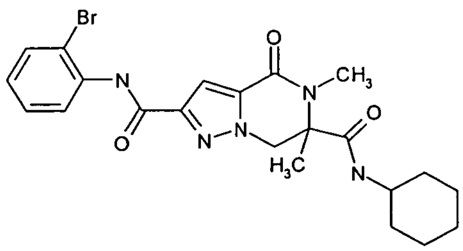 | 488,38822 | 489 |

(continued)

| | Table 1.05.(6) | | |
|---|---|---|---|
| № | Formula | Mol. Weight | m/z (M+1) |
| 108 | 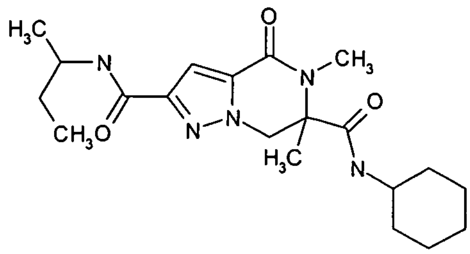 | 389,50177 | 390 |
| 109 | 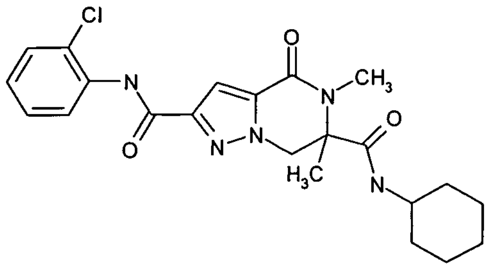 | 443,93722 | 444 |
| 110 | 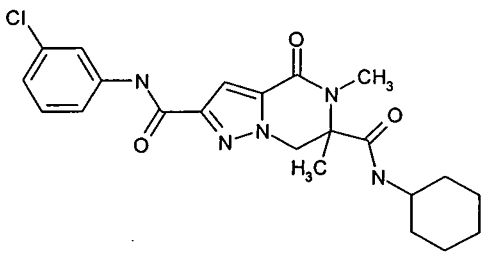 | 443,93722 | 444 |
| 111 | 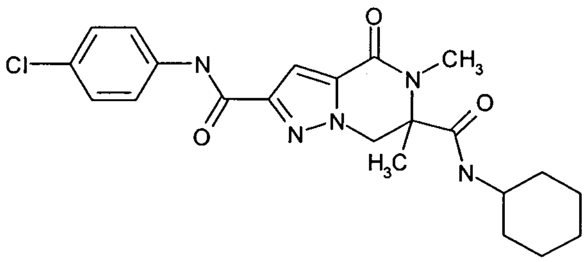 | 443,93722 | 444 |
| 112 | 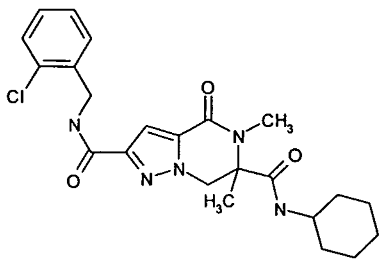 | 457,96431 | 458 |

(continued)

| | Table 1.05.(6) | | |
|---|---|---|---|
| № | Formula | Mol. Weight | *m*/*z* (M+1) |
| 113 | 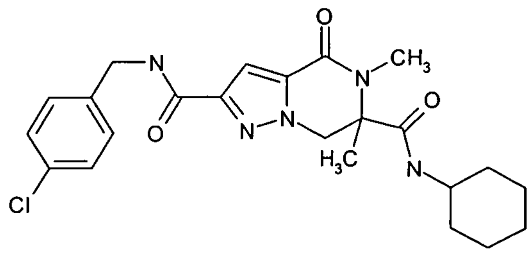 | 457,96431 | 458 |
| 114 | 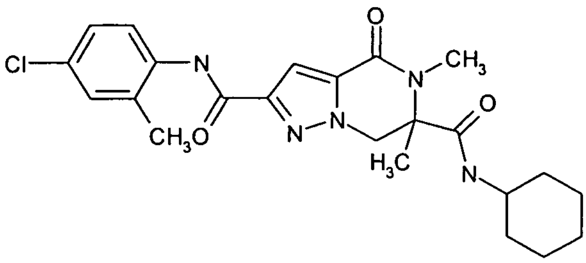 | 457,96431 | 458 |
| 115 | 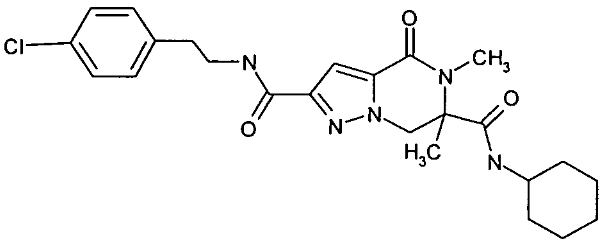 | 471,9914 | 472 |
| 116 | 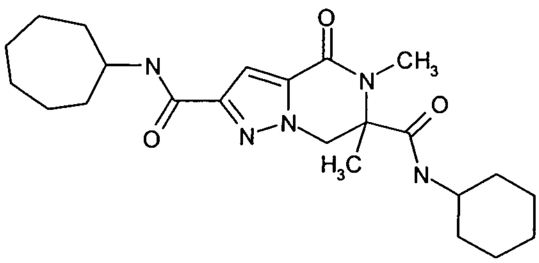 | 429,5671 | 430 |
| 117 | 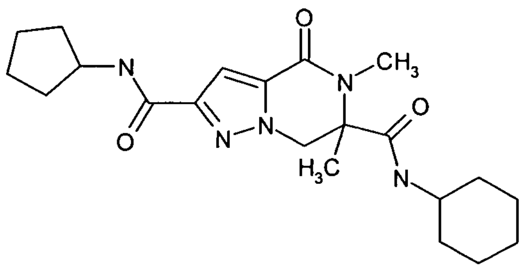 | 401,51292 | 402 |

(continued)

| | Table 1.05.(6) | | |
|---|---|---|---|
| № | Formula | Mol. Weight | *m*/*z* (M+1) |
| 118 | 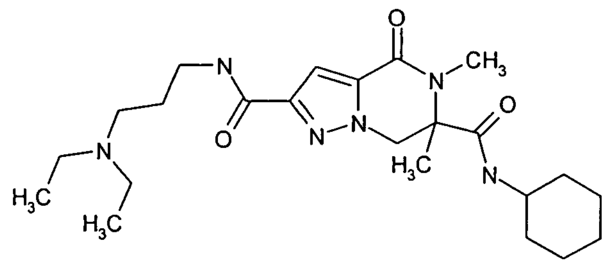 | 446,59771 | 447 |
| 119 | 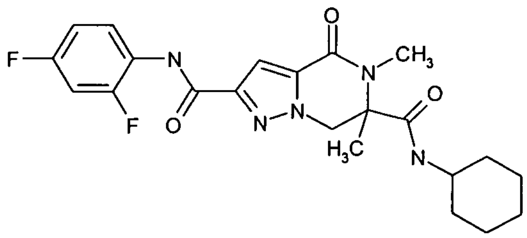 | 445,47305 | 446 |
| 120 | 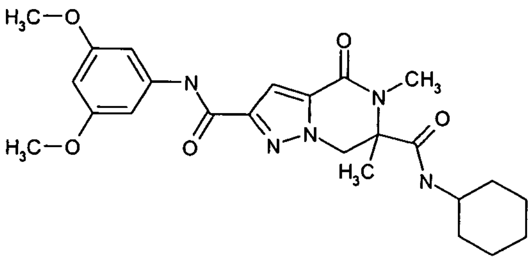 | 469,54517 | 470 |
| 121 | 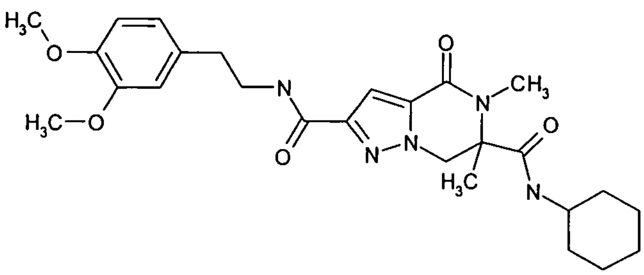 | 497,59935 | 498 |
| 122 | 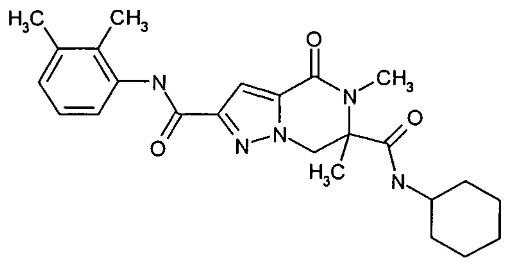 | 437,54637 | 438 |

(continued)

| | Table 1.05.(6) | | |
|---|---|---|---|
| № | Formula | Mol. Weight | m/z (M+1) |
| 123 | 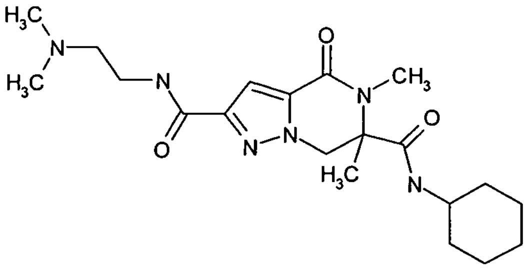 | 404,51644 | 405 |
| 124 | 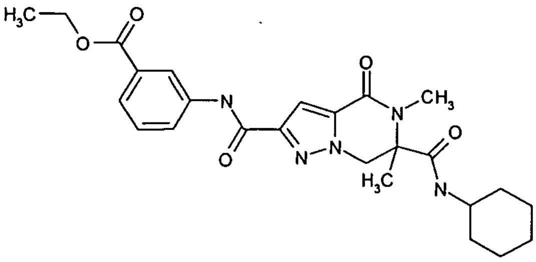 | 481,55632 | 482 |
| 125 | 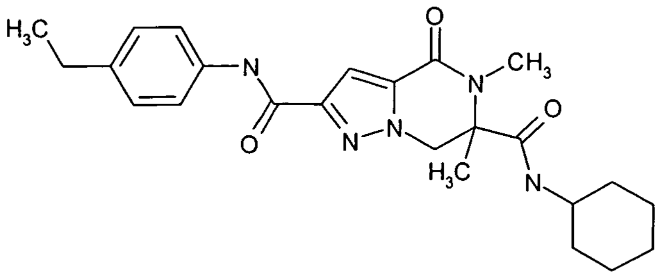 | 437,54637 | 438 |
| 126 | 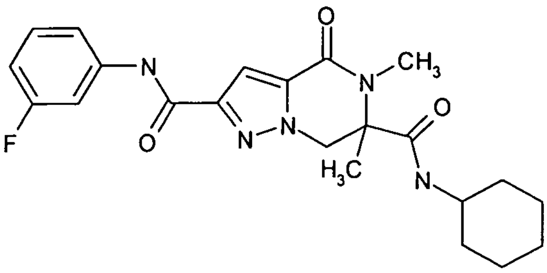 | 427,48262 | 428 |
| 127 | 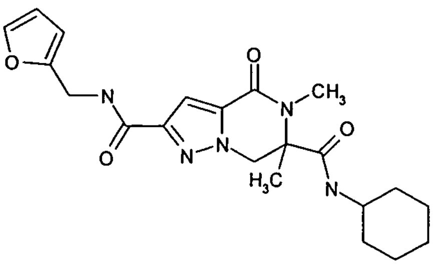 | 413,48044 | 414 |

(continued)

| | Table 1.05.(6) | | |
|---|---|---|---|
| № | Formula | Mol. Weight | *m/z* (M+1) |
| 128 | 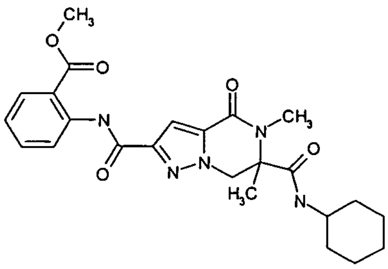 | 467,52923 | 468 |
| 129 | 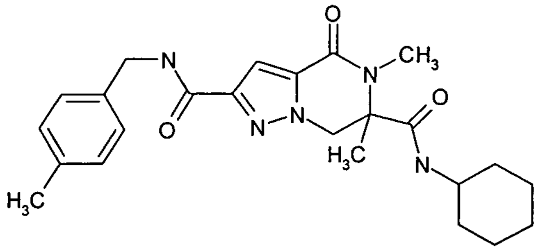 | 437,54637 | 438 |
| 130 | 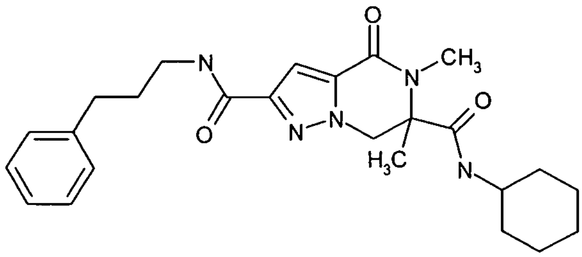 | 451,57346 | 452 |
| 131 | 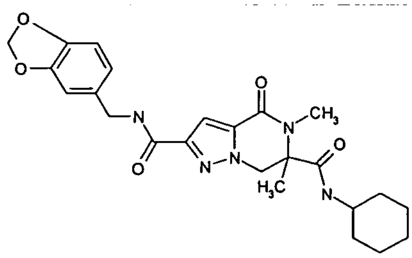 | 467,52923 | 468 |
| 132 | 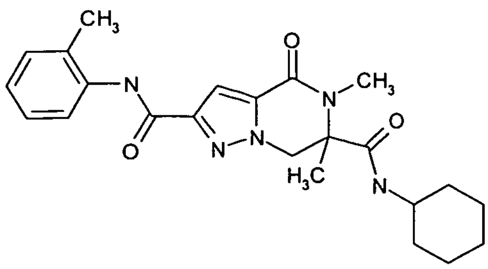 | 423,51928 | 424 |

(continued)

| | Table 1.05.(6) | | |
|---|---|---|---|
| № | Formula | Mol. Weight | *m*/*z* (M+1) |
| 133 | 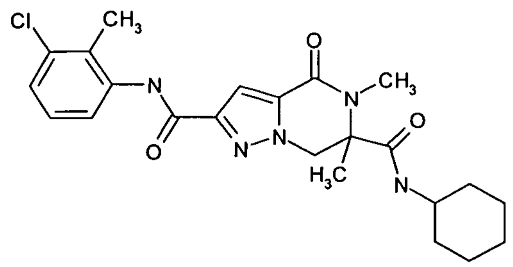 | 457,96431 | 458 |
| 134 | 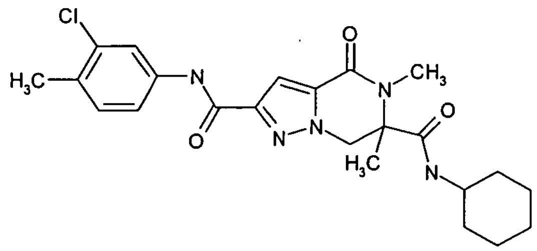 | 457,96431 | 458 |
| 135 | 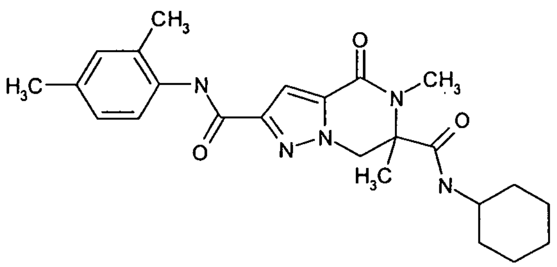 | 437,54637 | 438 |
| 136 | 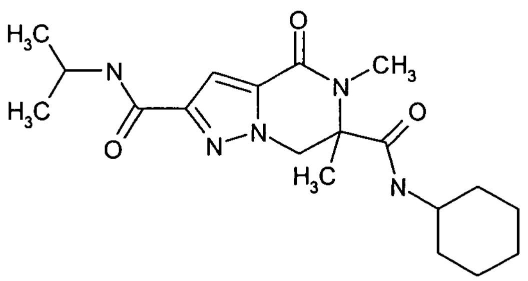 | 375,47468 | 376 |
| 137 | 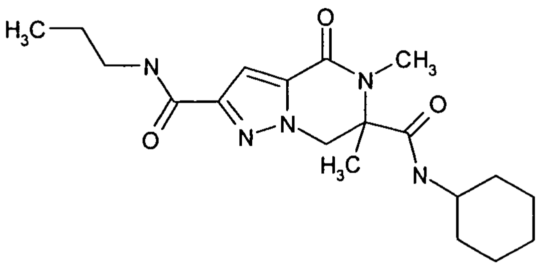 | 375,47468 | 376 |

(continued)

| | Table 1.05.(6) | | |
|---|---|---|---|
| № | Formula | Mol. Weight | *m/z* (M+1) |
| 138 | 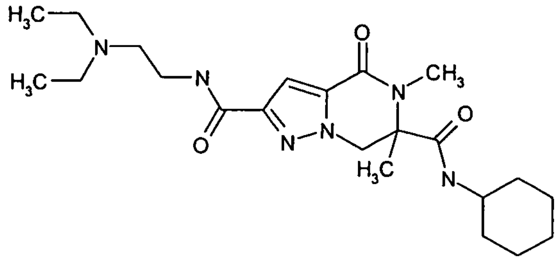 | 432,57062 | 433 |
| 139 | 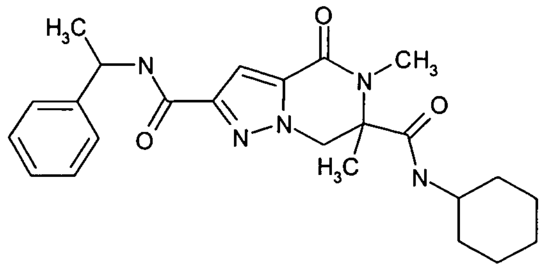 | 437,54637 | 438 |
| 140 | 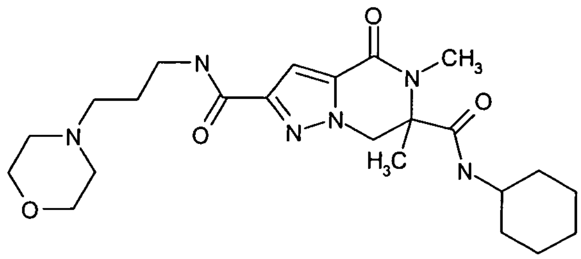 | 460,58117 | 461 |
| 141 | 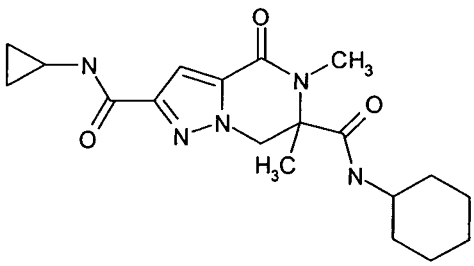 | 373,45874 | 374 |
| 142 | 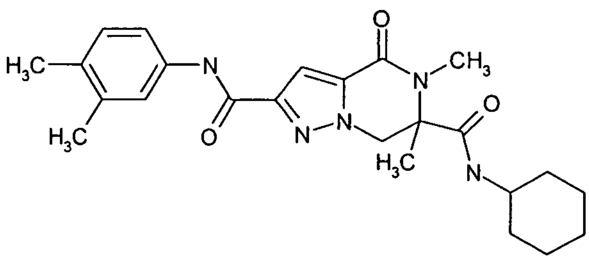 | 437,54637 | 438 |

(continued)

| | Table 1.05.(6) | | |
|---|---|---|---|
| № | Formula | Mol. Weight | *m/z* (M+1) |
| 143 | 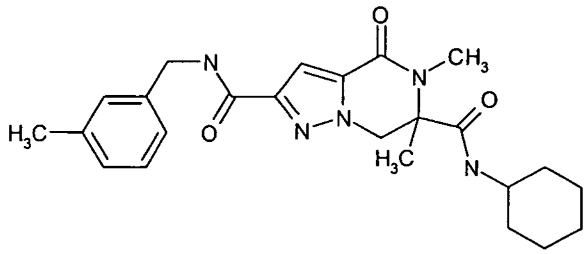 | 437,54637 | 438 |
| 144 | 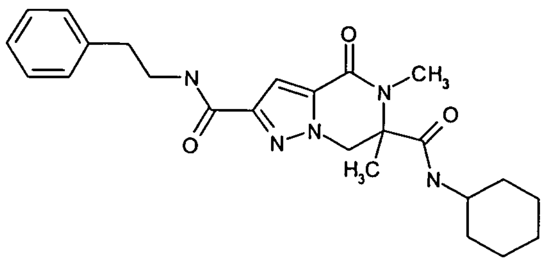 | 437,54637 | 438 |
| 145 | 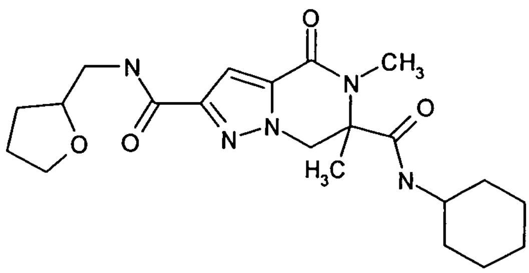 | 417,51232 | 418 |
| 146 | 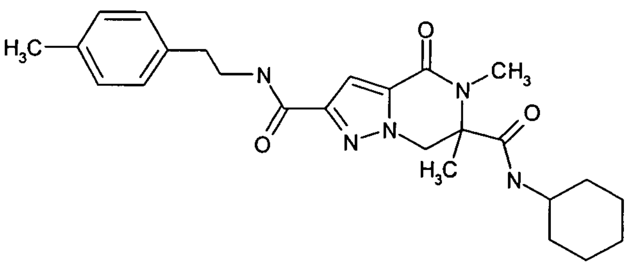 | 451,57346 | 452 |
| 147 | 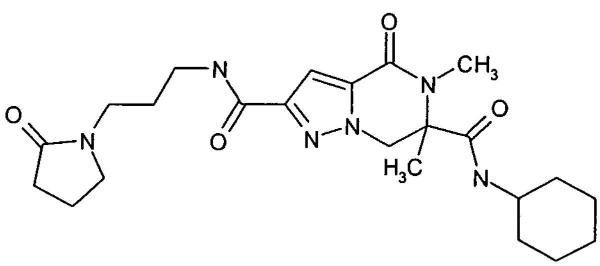 | 458,56523 | 459 |

(continued)

| | Table 1.05.(6) | | |
|---|---|---|---|
| № | Formula | Mol. Weight | *m/z* (M+1) |
| 148 | 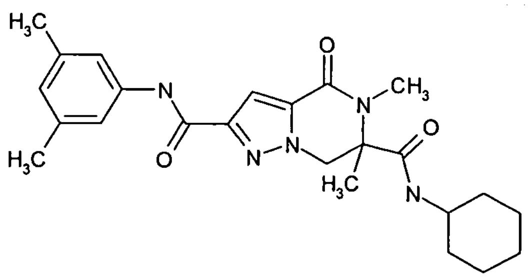 | 437,54637 | 438 |
| 149 | 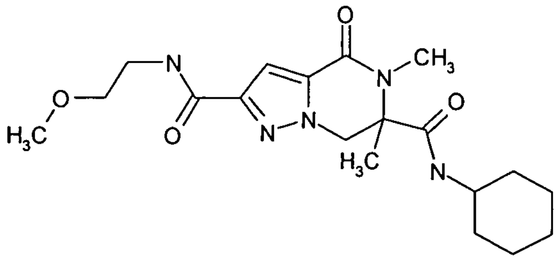 | 391,47408 | 392 |
| 150 | 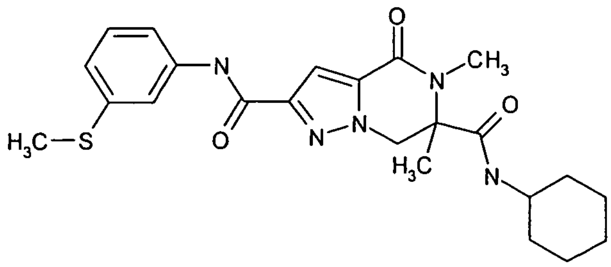 | 455,58328 | 456 |
| 151 | 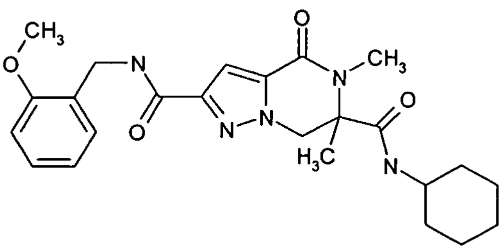 | 453,54577 | 454 |
| 152 | 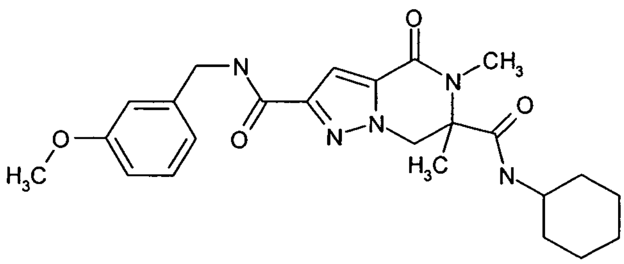 | 453,54577 | 454 |

(continued)

| | Table 1.05.(6) | | |
|---|---|---|---|
| № | Formula | Mol. Weight | *m*/*z* (M+1) |
| 153 | 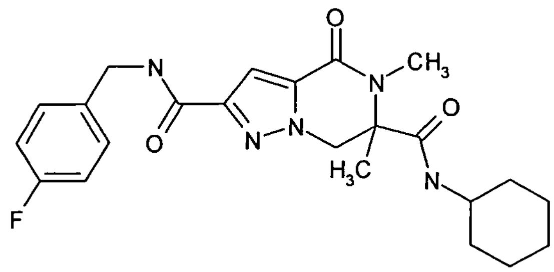 | 441,50971 | 442 |
| 154 | 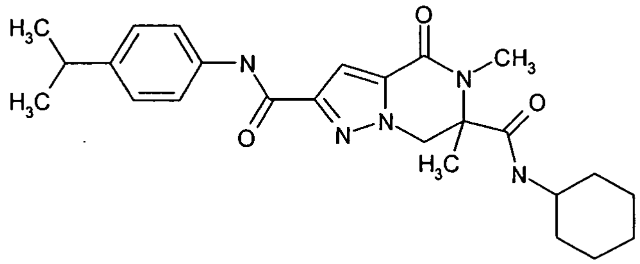 | 451,57346 | 452 |
| 155 | 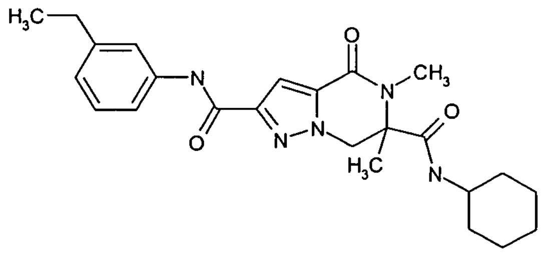 | 437,54637 | 438 |
| 156 | 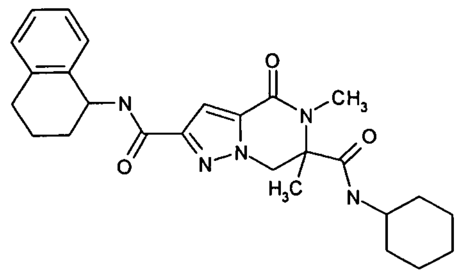 | 463,58461 | 464 |
| 157 | 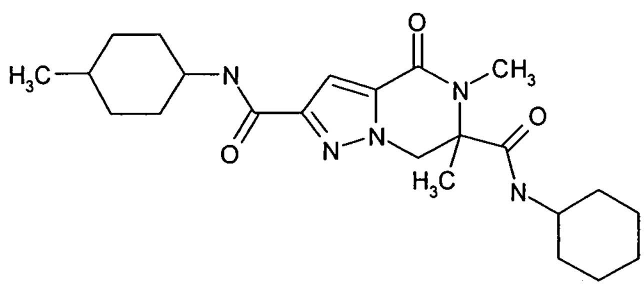 | 429,5671 | 430 |

(continued)

| | Table 1.05.(6) | | |
|---|---|---|---|
| № | Formula | Mol. Weight | *m*/*z* (M+1) |
| 158 | 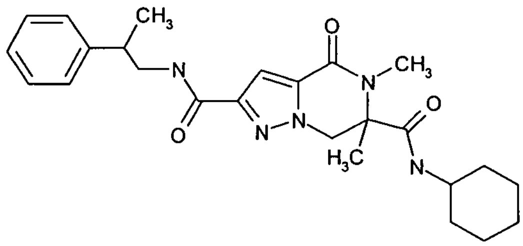 | 451,57346 | 452 |
| 159 | 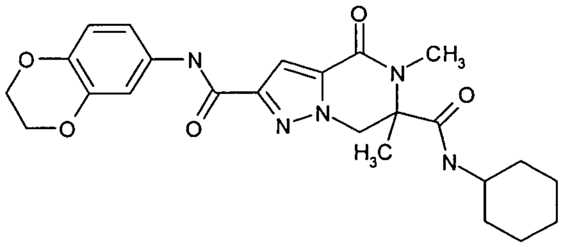 | 467,52923 | 468 |
| 160 | 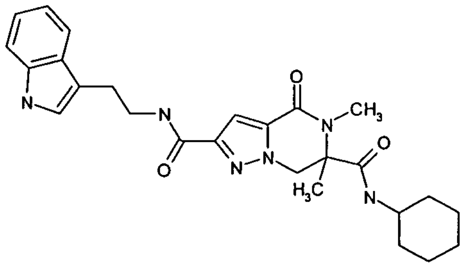 | 476,58334 | 477 |
| 161 | 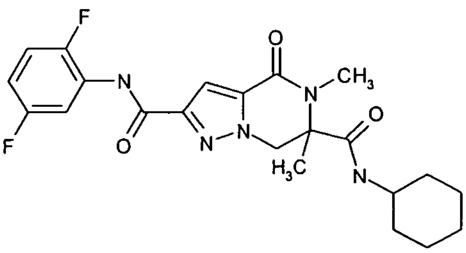 | 445,47305 | 446 |
| 162 | 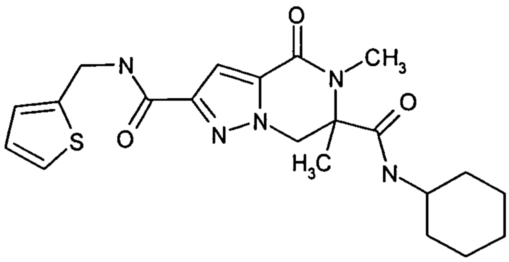 | 429,54504 | 430 |

(continued)

| | Table 1.05.(6) | | |
|---|---|---|---|
| Nº | Formula | Mol. Weight | m/z (M+1) |
| 163 | 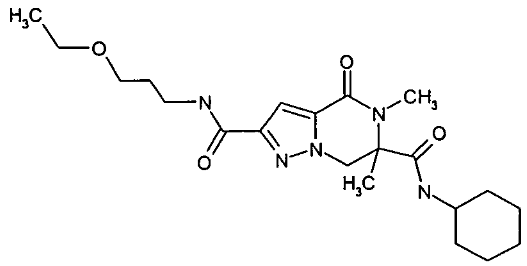 | 419,52826 | 420 |
| 164 | 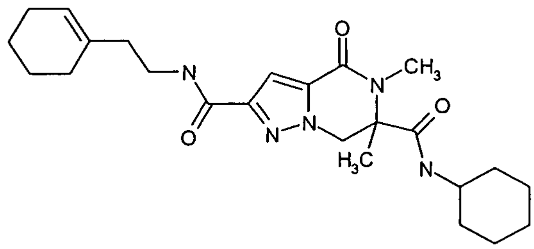 | 441,57825 | 442 |
| 165 | 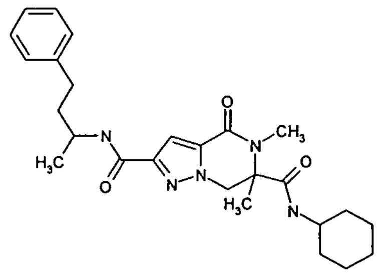 | 465,60055 | 466 |
| 166 | 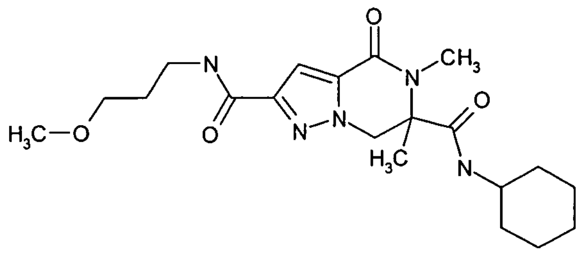 | 405,50117 | 406 |
| 167 | 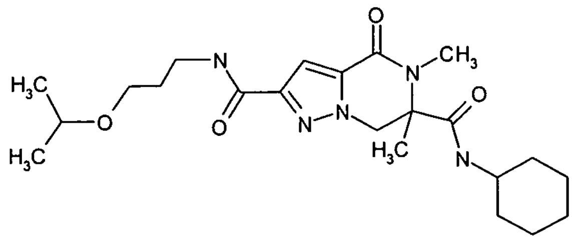 | 433,55535 | 434 |

(continued)

| | Table 1.05.(6) | | |
|---|---|---|---|
| № | Formula | Mol. Weight | *m*/*z* (M+1) |
| 168 | 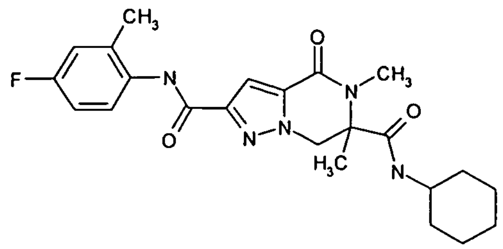 | 441,50971 | 442 |
| 169 | 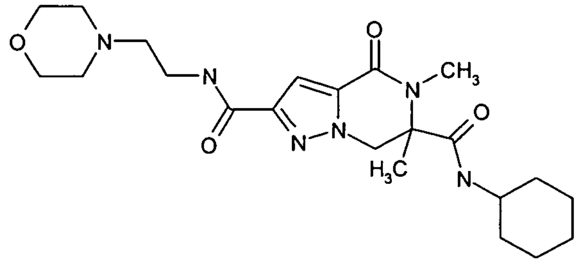 | 446,55408 | 447 |
| 170 | 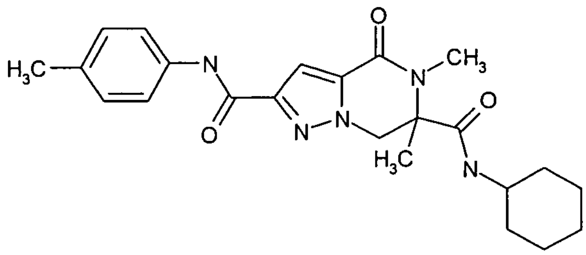 | 423,51928 | 424 |
| 171 | 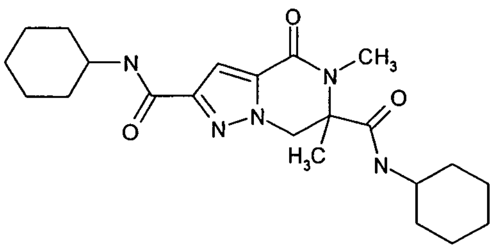 | 415,54001 | 416 |
| 172 | 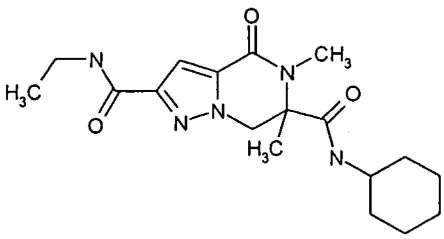 | 361,44759 | 362 |
| 173 | 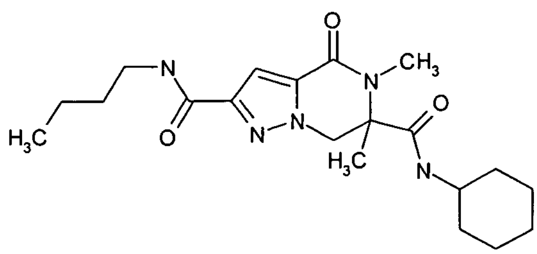 | 389,50177 | 390 |

(continued)

| | Table 1.05.(6) | | |
|---|---|---|---|
| № | Formula | Mol. Weight | *m*/*z* (M+1) |
| 174 | 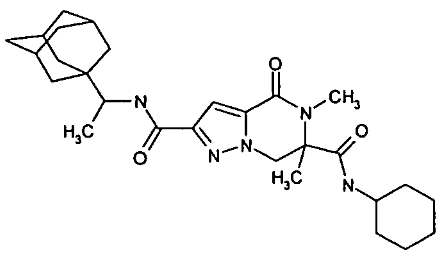 | 495,67067 | 496 |
| 175 | 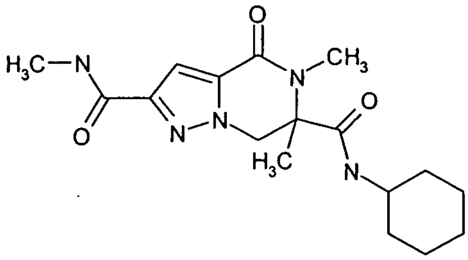 | 347,4205 | 348 |
| 176 | 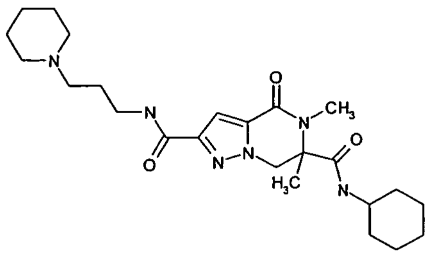 | 458,60886 | 459 |
| 177 | 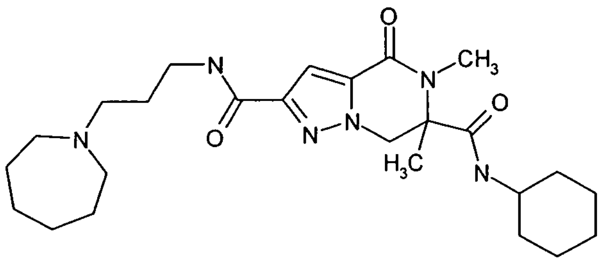 | 472,63595 | 473 |
| 178 | 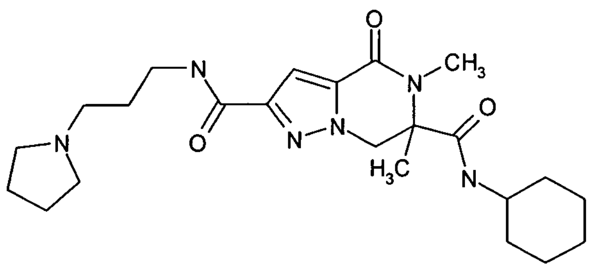 | 444,58177 | 445 |

(continued)

| | Table 1.05.(6) | | |
|---|---|---|---|
| № | Formula | Mol. Weight | *m/z* (M+1) |
| 179 | 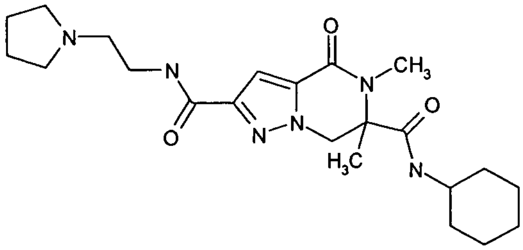 | 430,55468 | 431 |
| 180 | 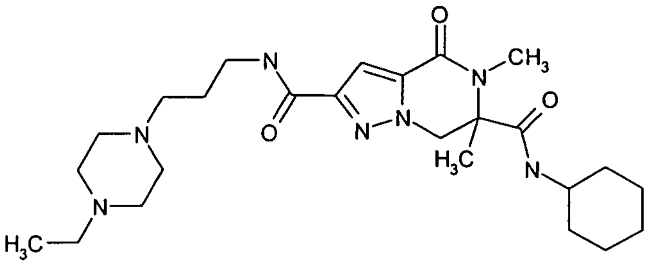 | 487,65062 | 488 |
| 181 | 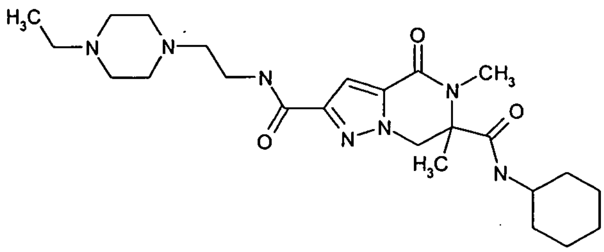 | 473,62353 | 474 |
| 182 | 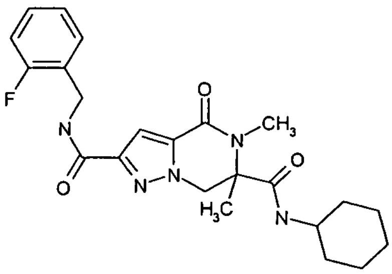 | 441,50971 | 442 |
| 183 | 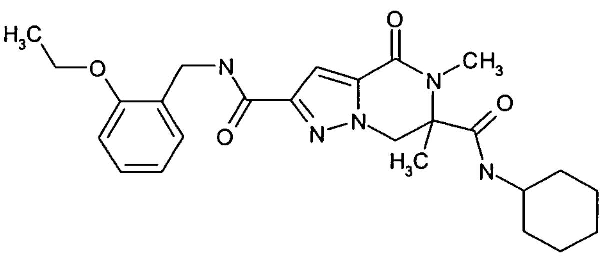 | 467,57286 | 468 |

(continued)

| | Table 1.05.(6) | | |
|---|---|---|---|
| № | Formula | Mol. Weight | *m/z* (M+1) |
| 184 | 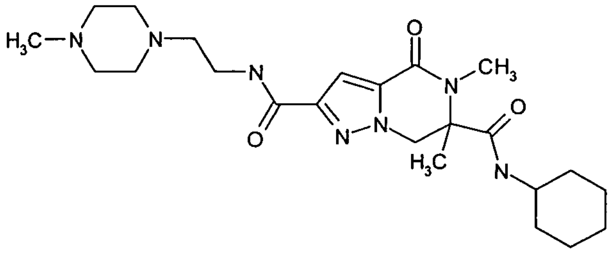 | 459,59644 | 460 |
| 185 | 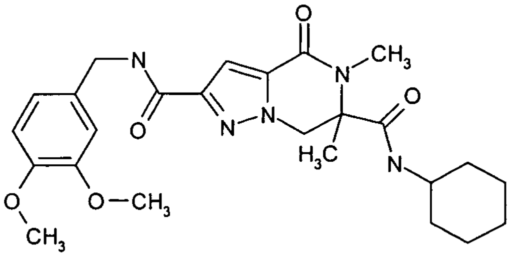 | 483,57226 | 484 |
| 186 | 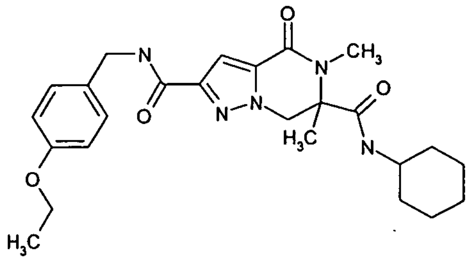 | 467,57286 | 468 |
| 187 | 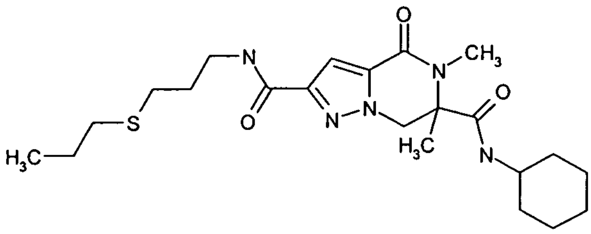 | 449,61995 | 450 |
| 188 | 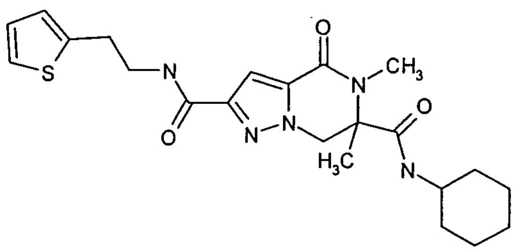 | 443,57213 | 444 |

(continued)

| | Table 1.05.(6) | | |
|---|---|---|---|
| № | Formula | Mol. Weight | m/z (M+1) |
| 189 | 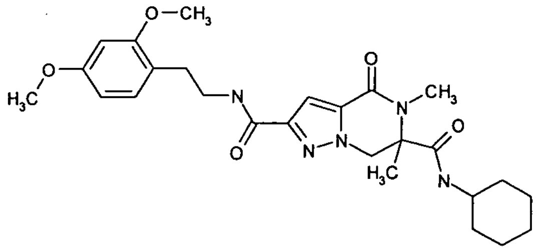 | 497,59935 | 498 |
| 190 | 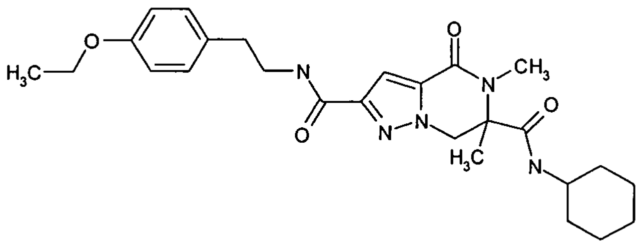 | 481,59995 | 482 |
| 191 | 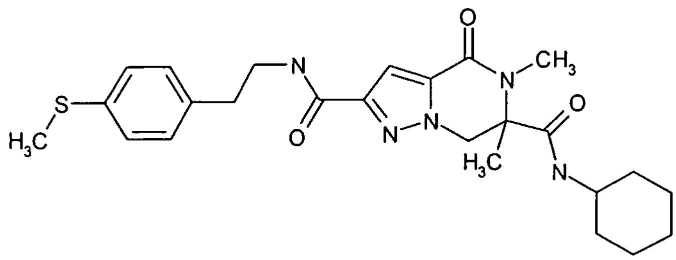 | 483,63746 | 484 |
| 192 | 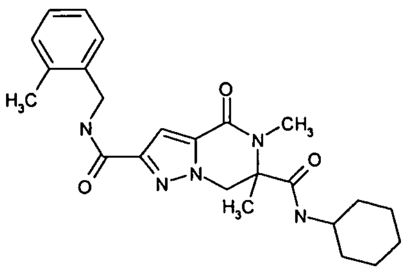 | 437,54637 | 438 |
| 193 | 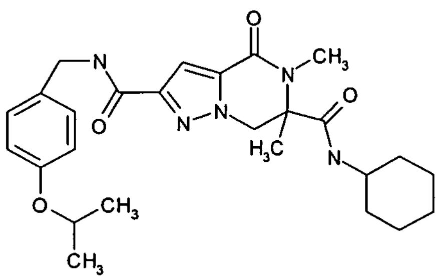 | 481,59995 | 482 |

(continued)

| | Table 1.05.(6) | | |
|---|---|---|---|
| № | Formula | Mol. Weight | *m/z* (M+1) |
| 194 | 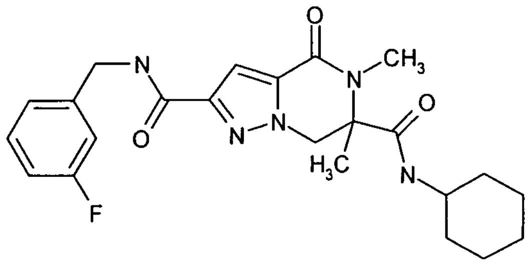 | 441,50971 | 442 |
| 195 | 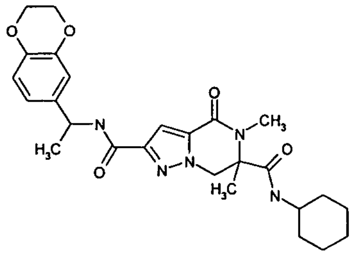 | 495,58341 | 496 |
| 196 | 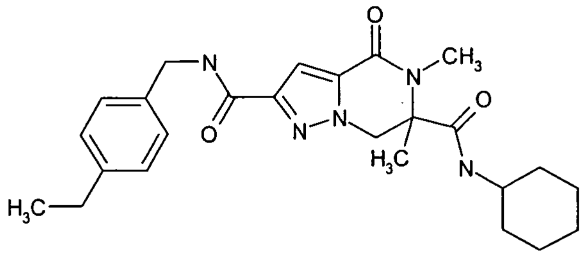 | 451,57346 | 452 |
| 197 | 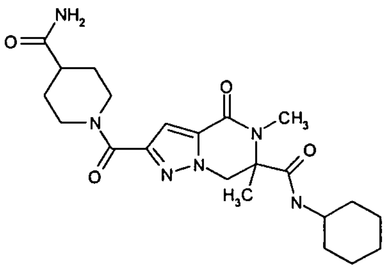 | 444.53814 | 445 |
| 198 | 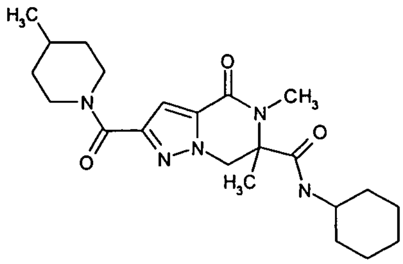 | 415,54001 | 416 |

(continued)

| | Table 1.05.(6) | | |
|---|---|---|---|
| № | Formula | Mol. Weight | *m*/*z* (M+1) |
| 199 | 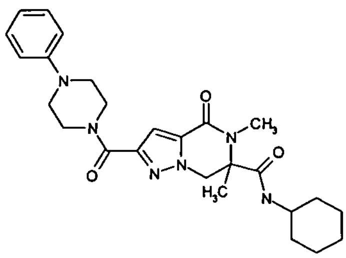 | 478,59928 | 479 |
| 200 | 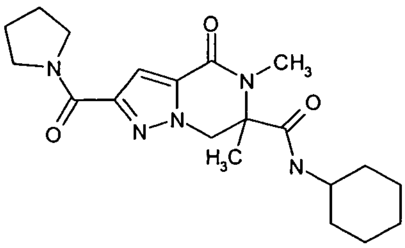 | 387,48583 | 388 |
| 201 | 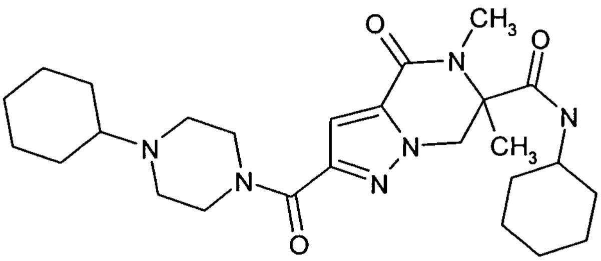 | 484,6471 | 485 |
| 202 | 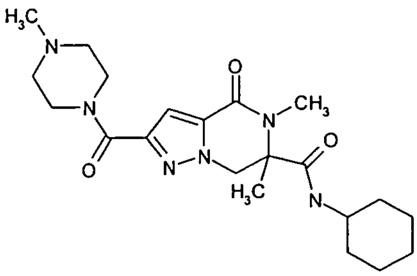 | 416,52759 | 417 |
| 203 | 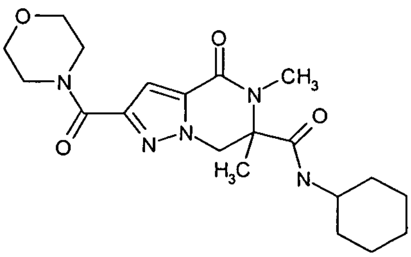 | 403,48523 | 404 |

(continued)

| | Table 1.05.(6) | | |
|---|---|---|---|
| № | Formula | Mol. Weight | *m*/*z* (M+1) |
| 204 | 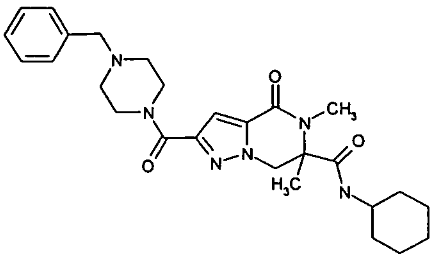 | 492,62637 | 493 |
| 205 | 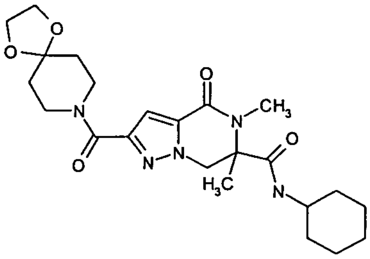 | 459,54996 | 460 |
| 206 | 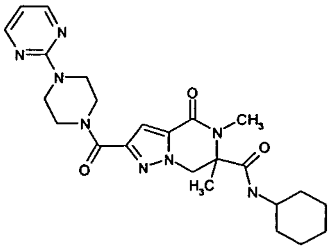 | 480,57444 | 481 |
| 207 | 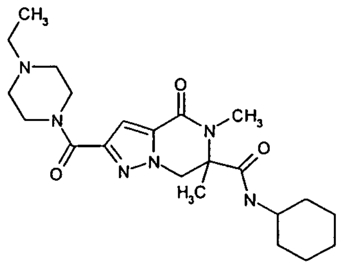 | 430,55468 | 431 |
| 208 | 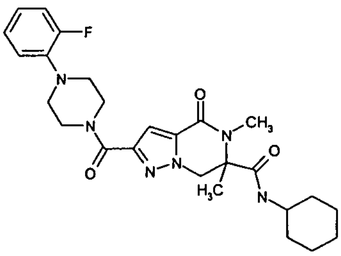 | 496,58971 | 497 |

(continued)

| | Table 1.05.(6) | | |
|---|---|---|---|
| № | Formula | Mol. Weight | *m*/*z* (M+1) |
| 209 | 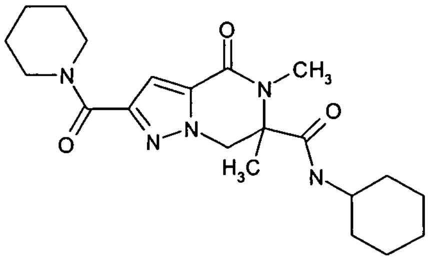 | 401,51292 | 402 |
| 210 | 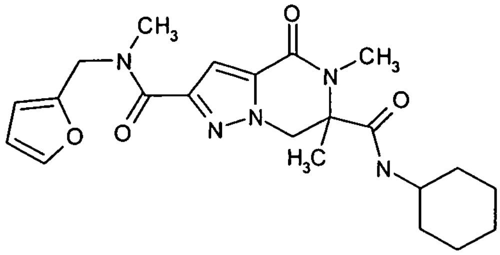 | 427,50753 | 428 |
| 211 | 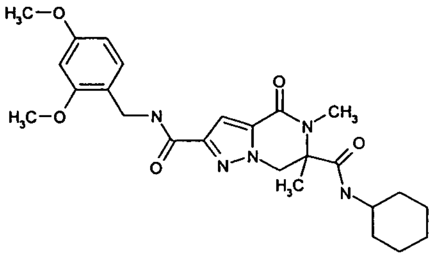 | 483,57226 | 484 |
| 212 | 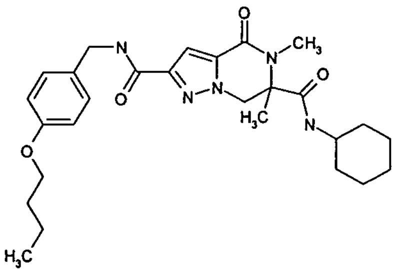 | 495,62704 | 496 |
| 213 | 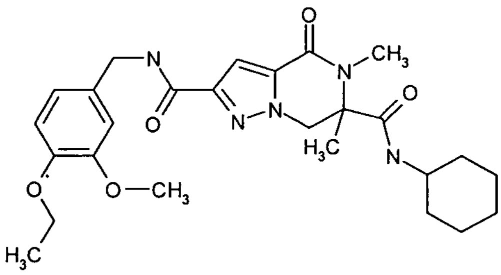 | 497,59935 | 498 |

(continued)

| | Table 1.05.(6) | | |
|---|---|---|---|
| № | Formula | Mol. Weight | m/z (M+1) |
| 214 | 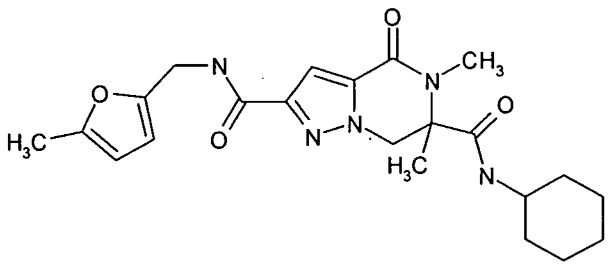 | 427,50753 | 428 |
| 215 | 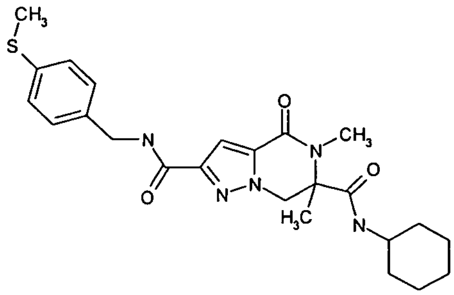 | 469,61037 | 470 |
| 216 | 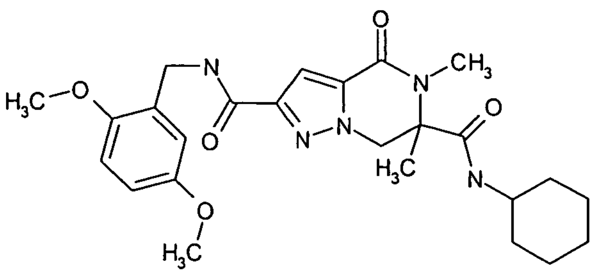 | 483,57226 | 484 |
| 217 | 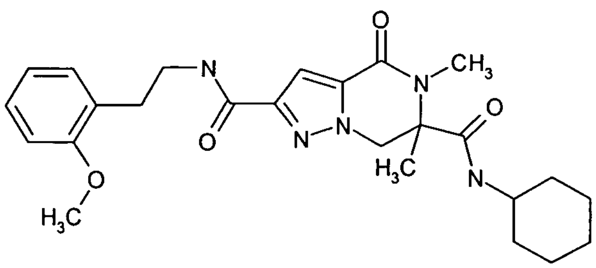 | 467,57286 | 468 |
| 218 | 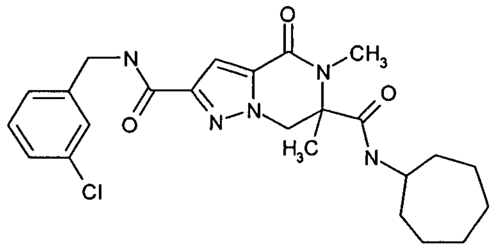 | 471,9914 | 472 |

(continued)

| | Table 1.05.(6) | | |
|---|---|---|---|
| Nº | Formula | Mol. Weight | m/z (M+1) |
| 219 | 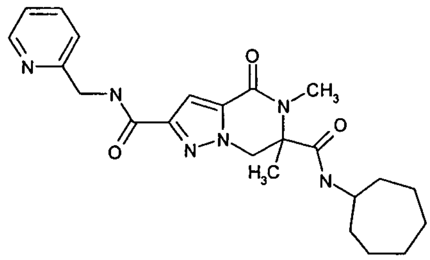 | 438,53395 | 439 |
| 220 | 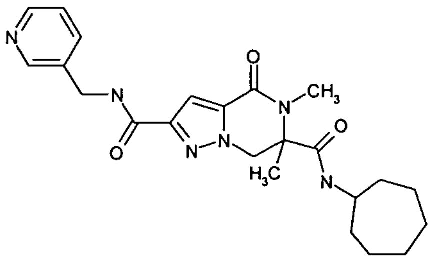 | 438,53395 | 439 |
| 221 | 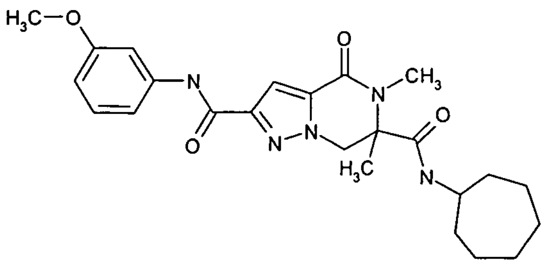 | 453,54577 | 454 |
| 222 | 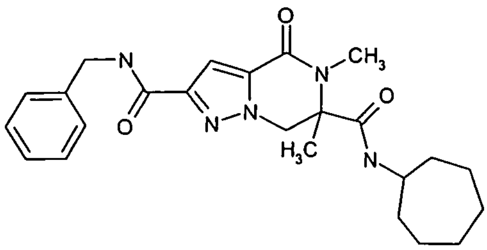 | 437,54637 | 438 |
| 223 | 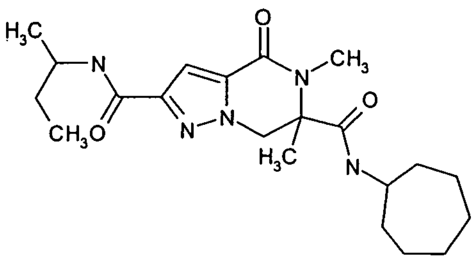 | 403,52886 | 404 |

(continued)

| | Table 1.05.(6) | | |
|---|---|---|---|
| № | Formula | Mol. Weight | *m/z* (M+1) |
| 224 | 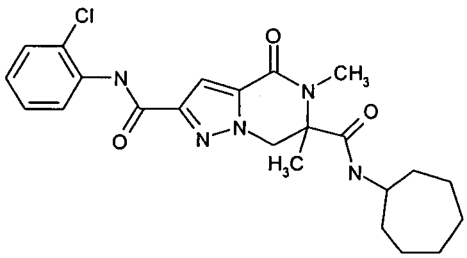 | 457,96431 | 458 |
| 225 | 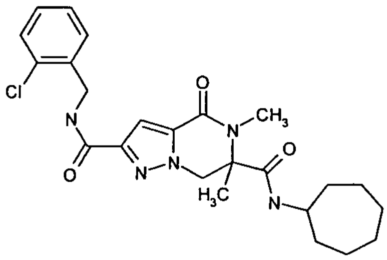 | 471,9914 | 472 |
| 226 | 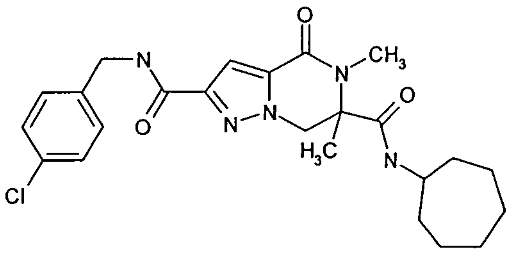 | 471,9914 | 472 |
| 227 | 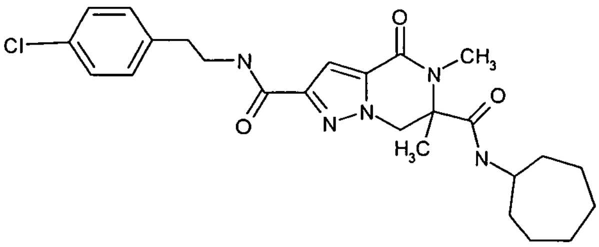 | 486,01849 | 487 |
| 228 | 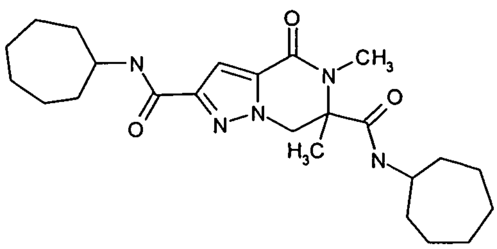 | 443,59419 | 444 |

(continued)

| | Table 1.05.(6) | | |
|---|---|---|---|
| № | Formula | Mol. Weight | *m/z* (M+1) |
| 229 | 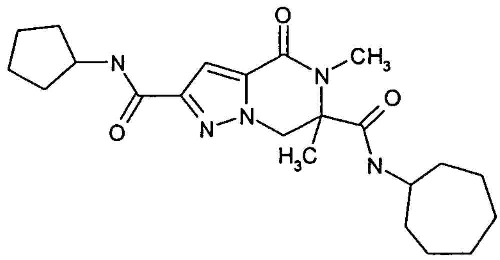 | 415,54001 | 416 |
| 230 | 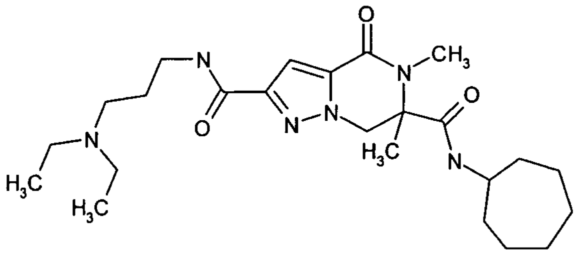 | 460,6248 | 461 |
| 231 | 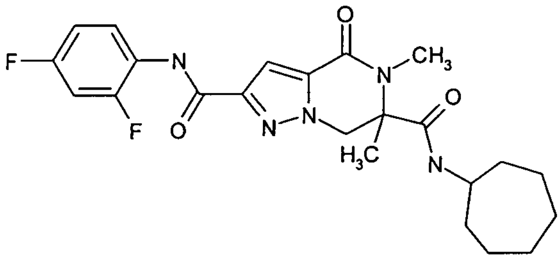 | 459,50014 | 460 |
| 232 | 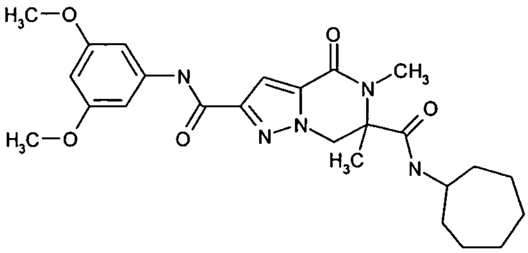 | 483,57226 | 484 |
| 233 | 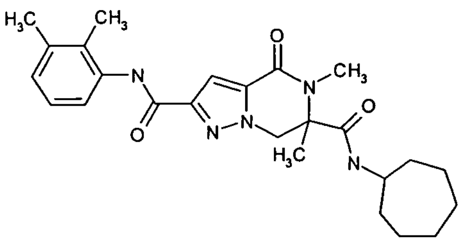 | 451,57346 | 452 |

(continued)

| | Table 1.05.(6) | | |
|---|---|---|---|
| № | Formula | Mol. Weight | *m*/*z* (M+1) |
| 234 | 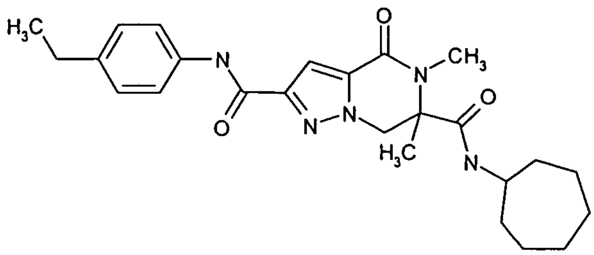 | 451,57346 | 452 |
| 235 | 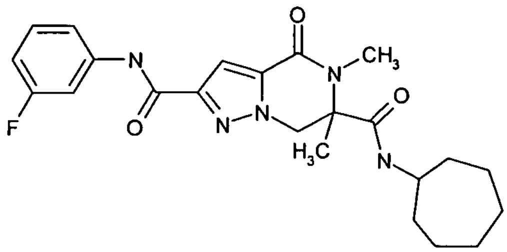 | 441,50971 | 442 |
| 236 | 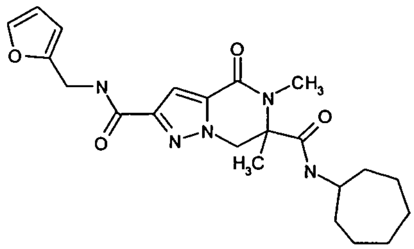 | 427,50753 | 428 |
| 237 | 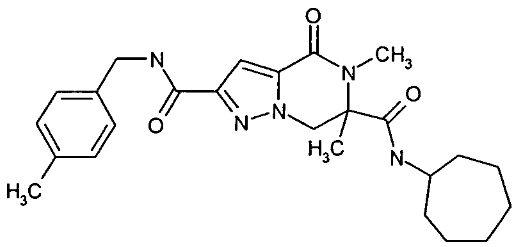 | 451,57346 | 452 |
| 238 | 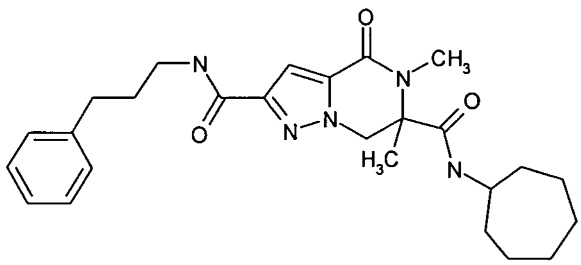 | 465,60055 | 466 |
| 239 | 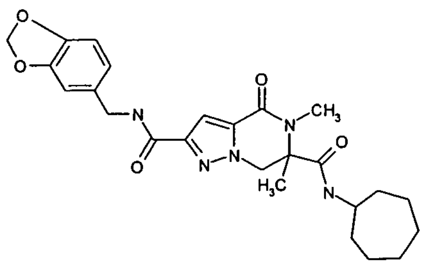 | 481,55632 | 482 |

(continued)

| | Table 1.05.(6) | | |
|---|---|---|---|
| № | Formula | Mol. Weight | *m/z* (M+1) |
| 240 | 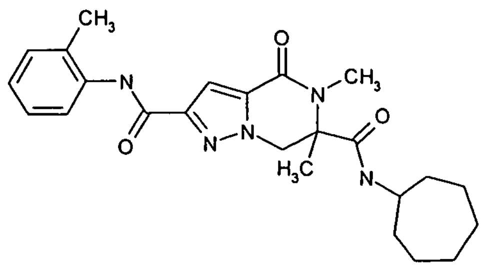 | 437,54637 | 438 |
| 241 | 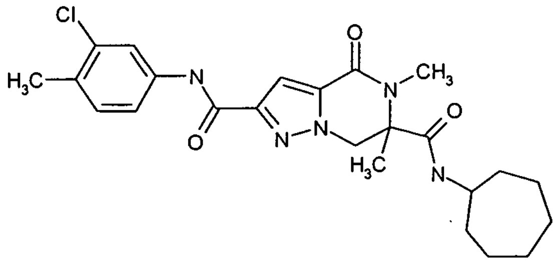 | 471,9914 | 472 |
| 242 | 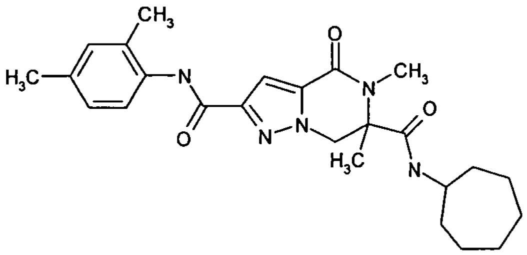 | 451,57346 | 452 |
| 243 | 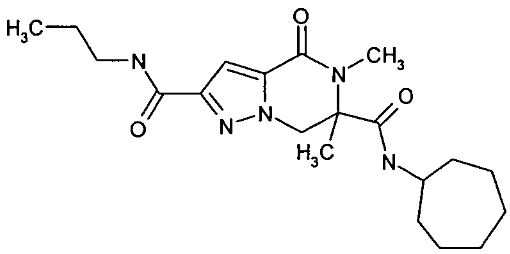 | 389,50177 | 390 |
| 244 | 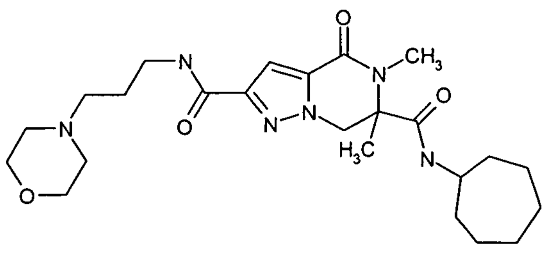 | 474,60826 | 475 |

(continued)

| | Table 1.05.(6) | | |
|---|---|---|---|
| № | Formula | Mol. Weight | *m*/*z* (M+1) |
| 245 | 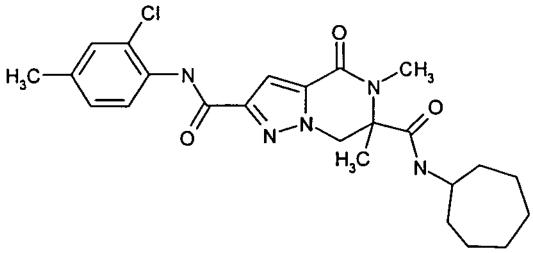 | 471,9914 | 472 |
| 246 | 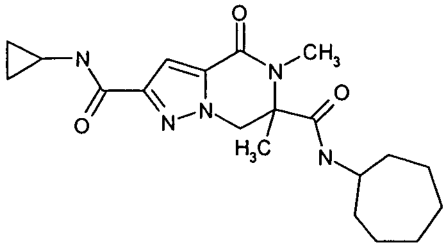 | 387,48583 | 388 |
| 247 | 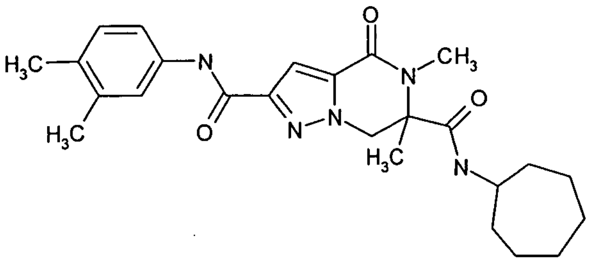 | 451,57346 | 452 |
| 248 | 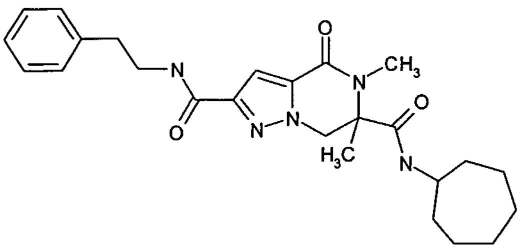 | 451,57346 | 452 |
| 249 | 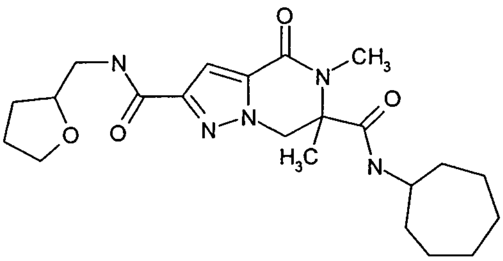 | 431,53941 | 432 |

(continued)

| | Table 1.05.(6) | | |
|---|---|---|---|
| Nº | Formula | Mol. Weight | m/z (M+1) |
| 250 | 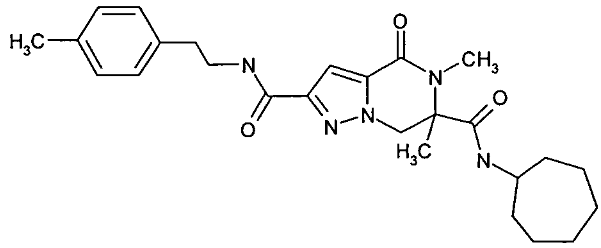 | 465,60055 | 466 |
| 251 | 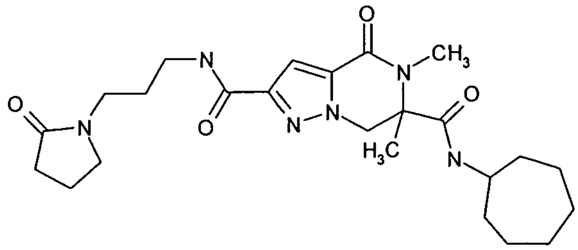 | 472,59232 | 473 |
| 252 | 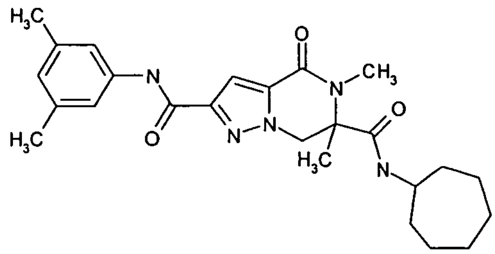 | 451,57346 | 452 |
| 253 | 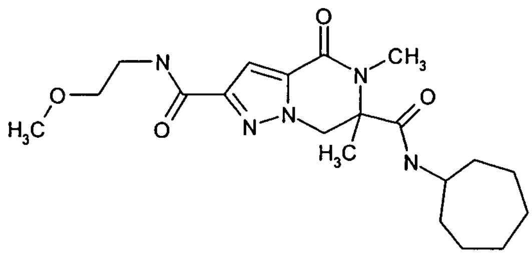 | 405,50117 | 406 |
| 254 | 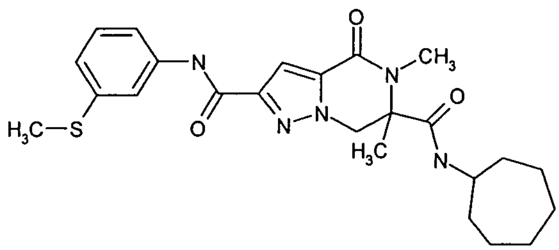 | 469,61037 | 470 |

(continued)

| | Table 1.05.(6) | | |
|---|---|---|---|
| № | Formula | Mol. Weight | *m*/*z* (M+1) |
| 255 | 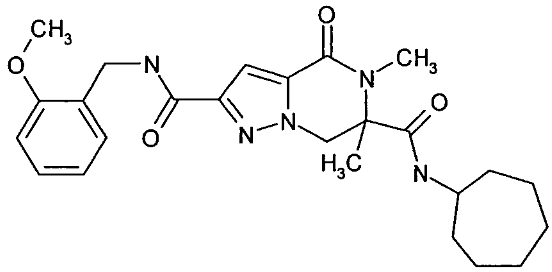 | 467,57286 | 468 |
| 256 | 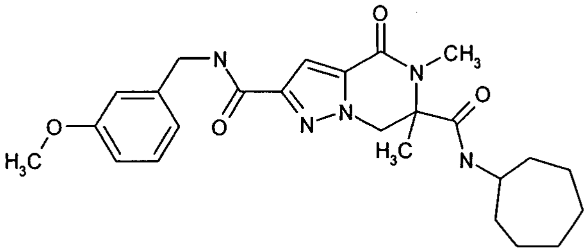 | | |
| 257 | 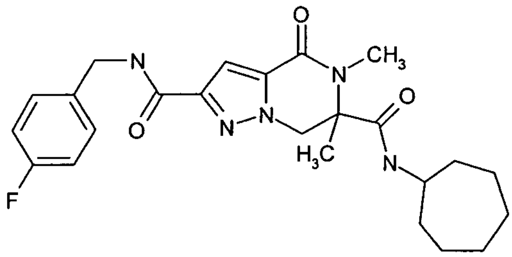 | 455,5368 | 456 |
| 258 | 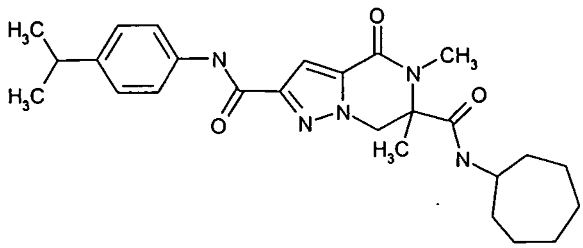 | 465,60055 | 466 |
| 259 | 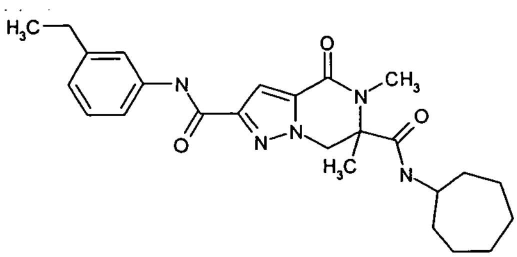 | 451,57346 | 452 |

(continued)

| | Table 1.05.(6) | | |
|---|---|---|---|
| № | Formula | Mol. Weight | m/z (M+1) |
| 260 | 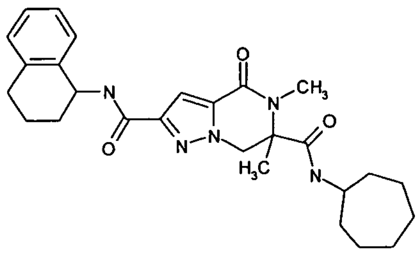 | 477,6117 | 478 |
| 261 | 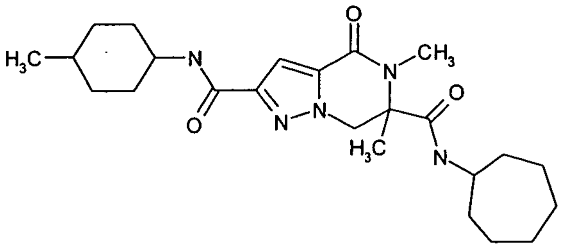 | 443,59419 | 444 |
| 262 | 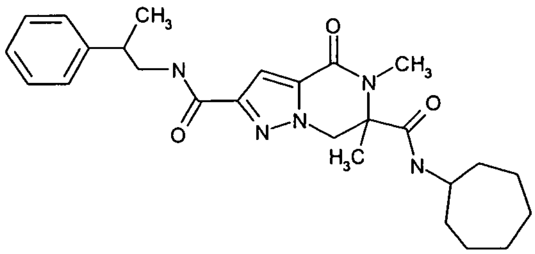 | 465,60055 | 466 |
| 263 | 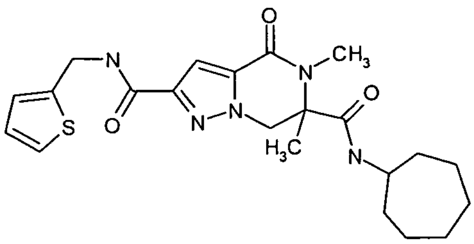 | 443,57213 | 444 |
| 264 | 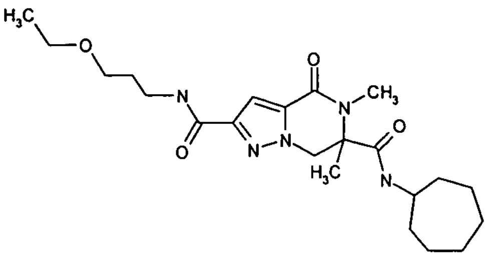 | 433,55535 | 434 |

(continued)

| | Table 1.05.(6) | | |
|---|---|---|---|
| № | Formula | Mol. Weight | m/z (M+1) |
| 265 | 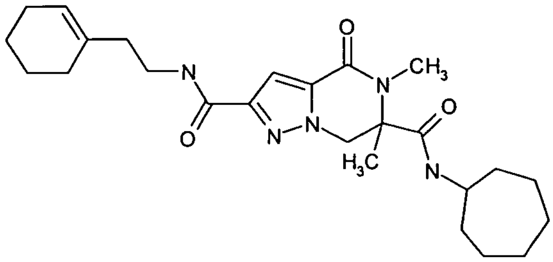 | 455,60534 | 456 |
| 266 | 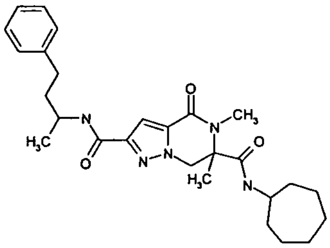 | 479,62764 | 480 |
| 267 | 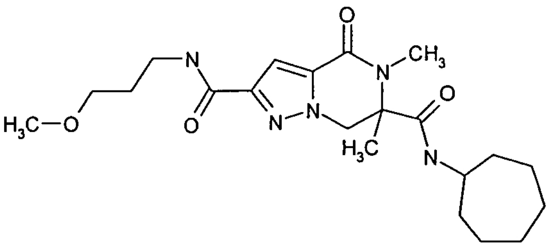 | 419,52826 | 420 |
| 268 | 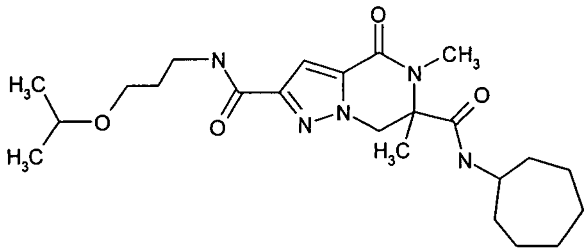 | 447,58244 | 448 |
| 269 | 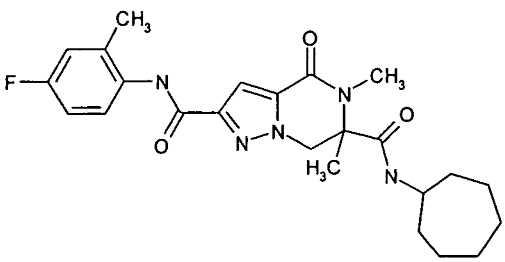 | 455,5368 | 456 |

(continued)

| | Table 1.05.(6) | | |
|---|---|---|---|
| № | Formula | Mol. Weight | *m/z* (M+1) |
| 270 | 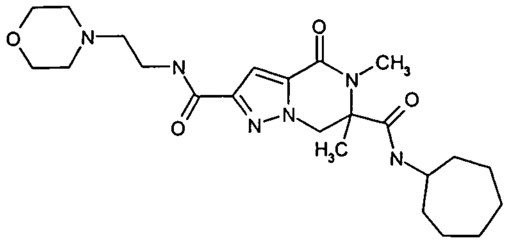 | 460,58117 | 461 |
| 271 | 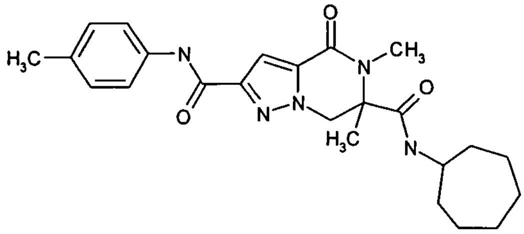 | 437,54637 | 438 |
| 272 | 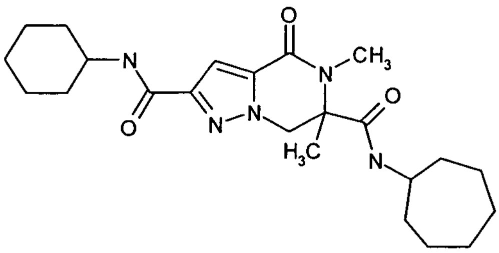 | 429,5671 | 430 |
| 273 | 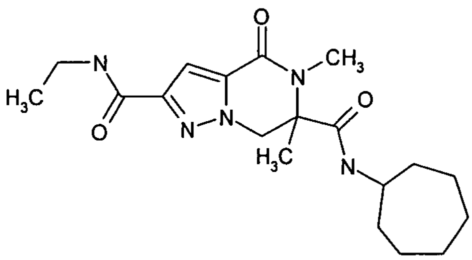 | 375,47468 | 376 |
| 274 | 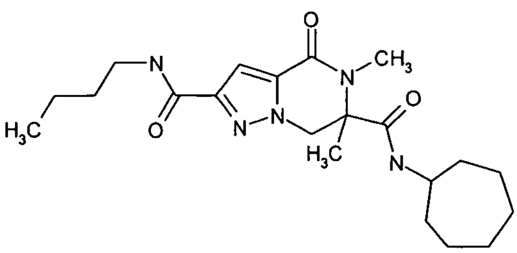 | 403,52886 | 404 |

(continued)

| | Table 1.05.(6) | | |
|---|---|---|---|
| № | Formula | Mol. Weight | m/z (M+1) |
| 275 | 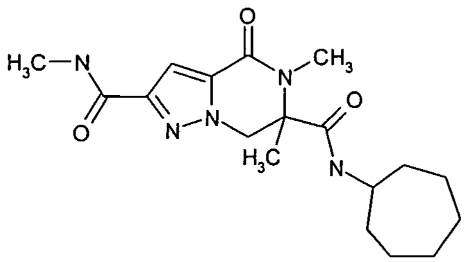 | 361,44759 | 362 |
| 276 | 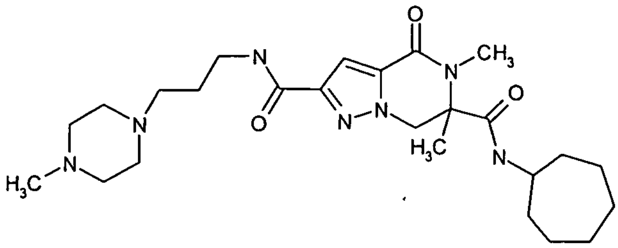 | 487,65062 | 488 |
| 277 | 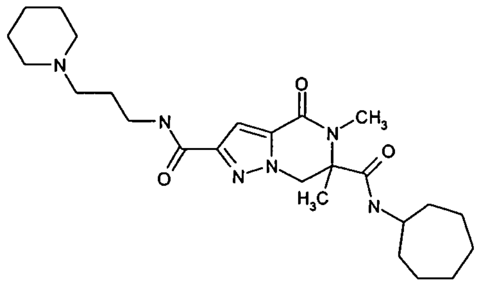 | 472,63595 | 473 |
| 278 | 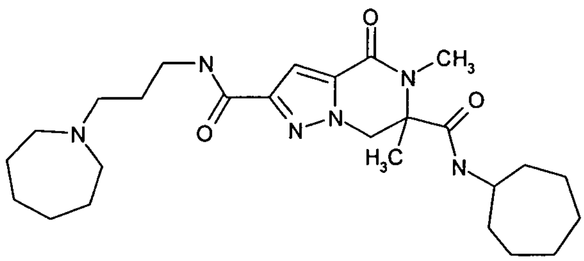 | 486,66304 | 487 |
| 279 | 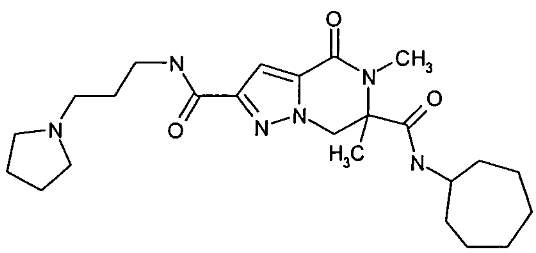 | 458,60886 | 459 |

(continued)

| | Table 1.05.(6) | | |
|---|---|---|---|
| № | Formula | Mol. Weight | *m*/*z* (M+1) |
| 280 | 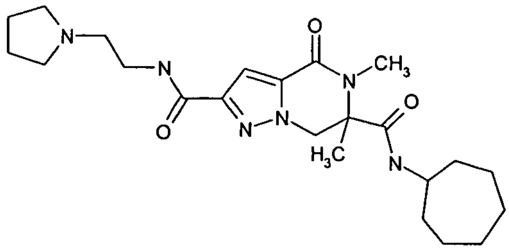 | 444,58177 | 445 |
| 281 | 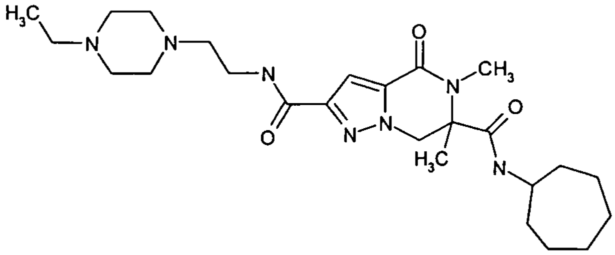 | 487,65062 | 488 |
| 282 | 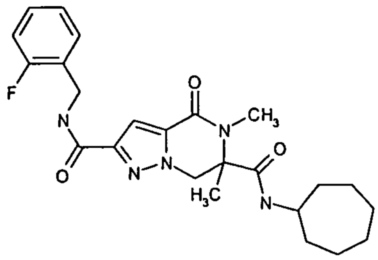 | 455,5368 | 456 |
| 283 | 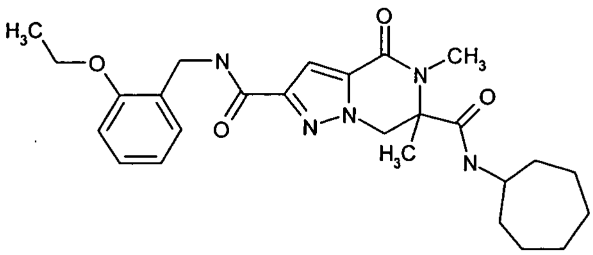 | 481,59995 | 482 |
| 284 | 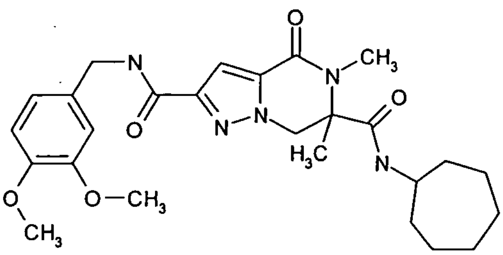 | 497,59935 | 498 |

(continued)

| | Table 1.05.(6) | | |
|---|---|---|---|
| № | Formula | Mol. Weight | m/z (M+1) |
| 285 | 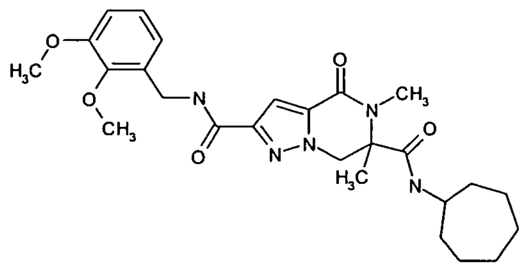 | 497,59935 | 498 |
| 286 | 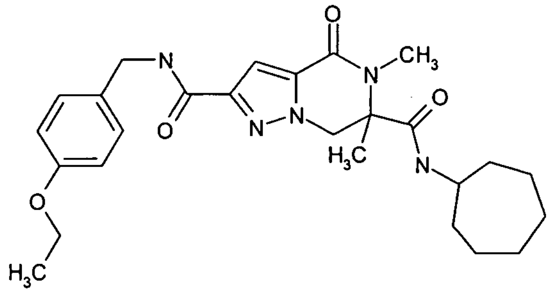 | 481,59995 | 482 |
| 287 | 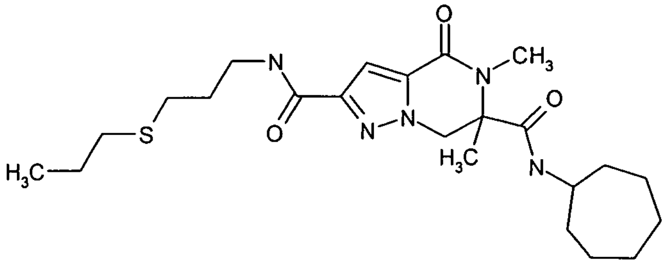 | 463,64704 | 464 |
| 288 | 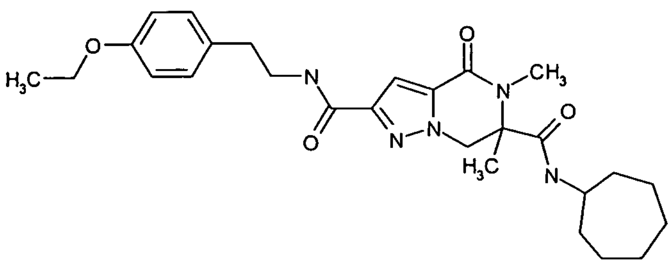 | 495,62704 | 496 |
| 289 | 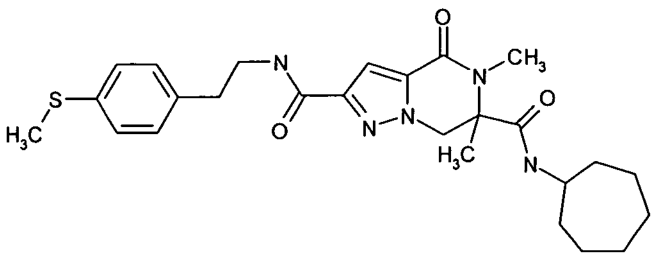 | 497,66455 | 498 |

(continued)

| | Table 1.05.(6) | | |
|---|---|---|---|
| № | Formula | Mol. Weight | *m/z* (M+1) |
| 290 | 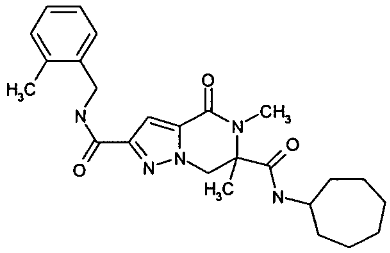 | 451,57346 | 452 |
| 291 | 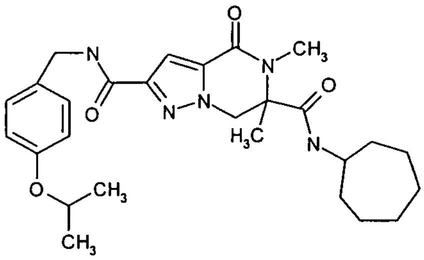 | 495,62704 | 496 |
| 292 | 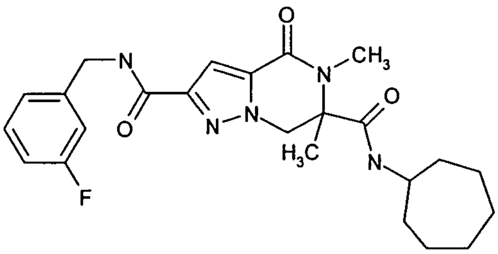 | 455,5368 | 456 |
| 293 | 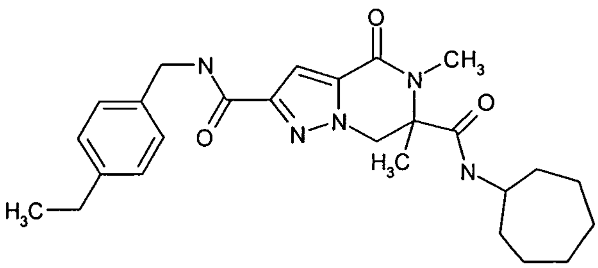 | 465,60055 | 466 |
| 294 | 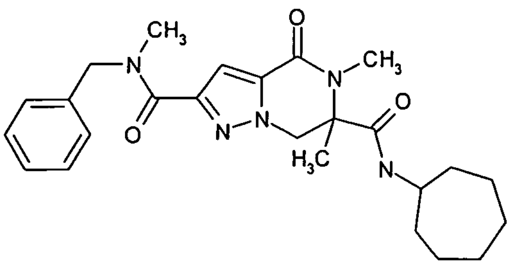 | 451,57346 | 452 |

(continued)

| | Table 1.05.(6) | | |
|---|---|---|---|
| № | Formula | Mol. Weight | m/z (M+1) |
| 295 | 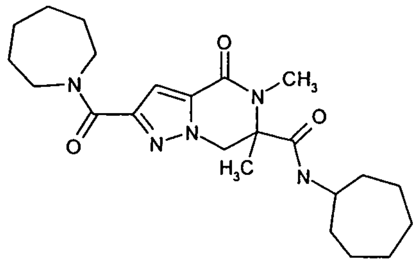 | 429,5671 | 430 |
| 296 | 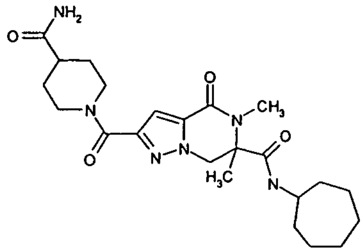 | 458,56523 | 459 |
| 297 | 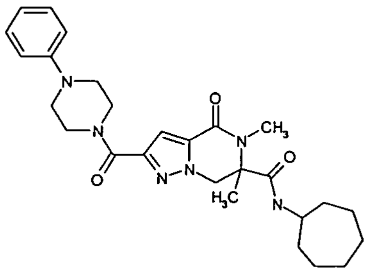 | 492,62637 | 493 |
| 298 | 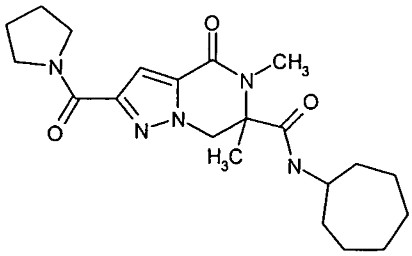 | 401,51292 | 402 |
| 299 | 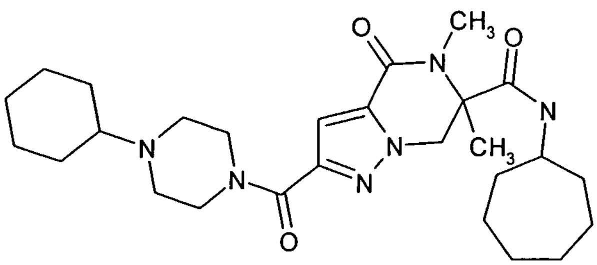 | 498,67419 | 499 |

(continued)

| | Table 1.05.(6) | | |
|---|---|---|---|
| № | Formula | Mol. Weight | m/z (M+1) |
| 300 | 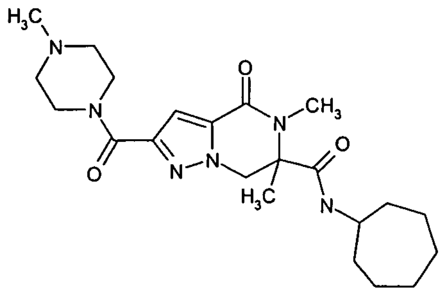 | 430,55468 | 431 |
| 301 | 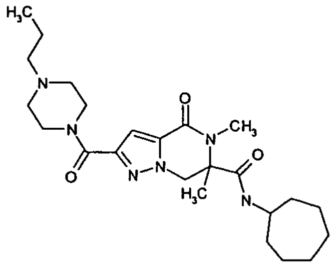 | 458,60886 | 459 |
| 302 | 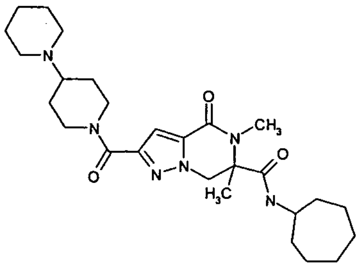 | 498,67419 | 499 |
| 303 | 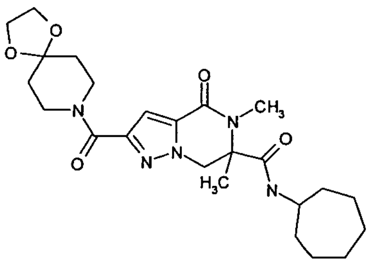 | 473,57705 | 474 |
| 304 | 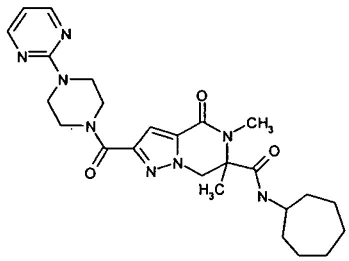 | 494,60153 | 495 |

(continued)

| | Table 1.05.(6) | | |
|---|---|---|---|
| № | Formula | Mol. Weight | *m*/*z* (M+1) |
| 305 | 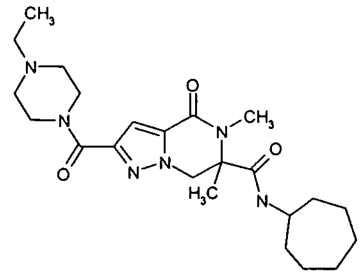 | 444,58177 | 445 |
| 306 | 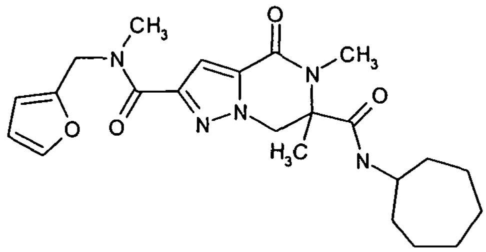 | 441,53462 | 442 |
| 307 | 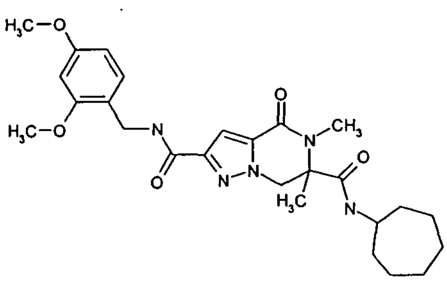 | 497,59935 | 498 |
| 308 | 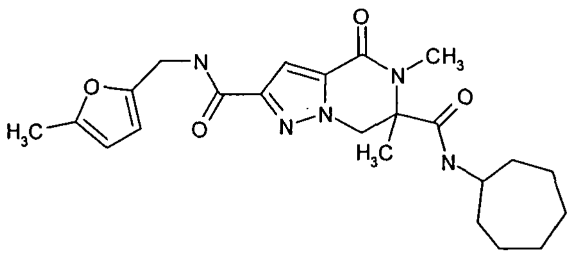 | 441,53462 | 442 |
| 309 | 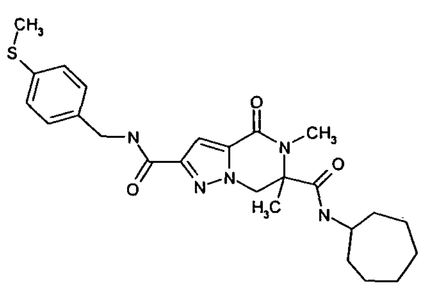 | 483,63746 | 484 |

(continued)

| | Table 1.05.(6) | | |
|---|---|---|---|
| № | Formula | Mol. Weight | *m*/*z* (M+1) |
| 310 | 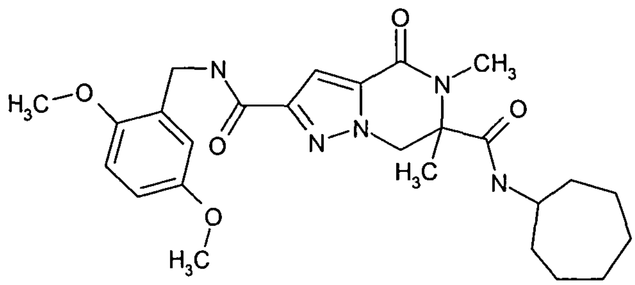 | 497,59935 | 498 |
| 311 | 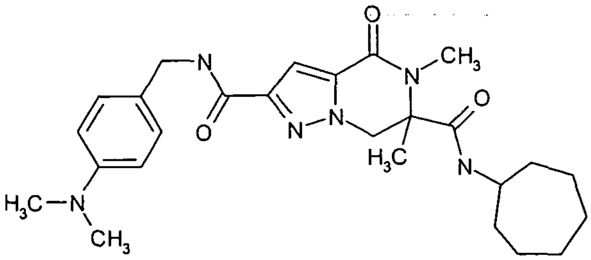 | 480,61522 | 481 |
| 312 | 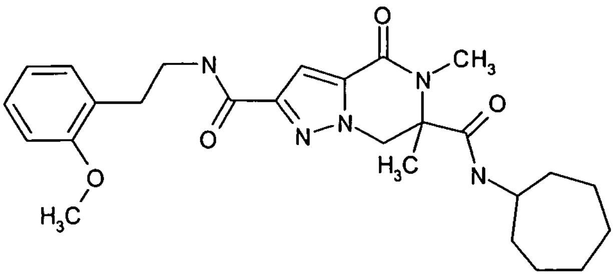 | 481,59995 | 482 |
| 313 | 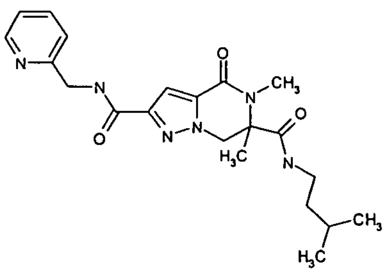 | 412,49571 | 413 |
| 314 | 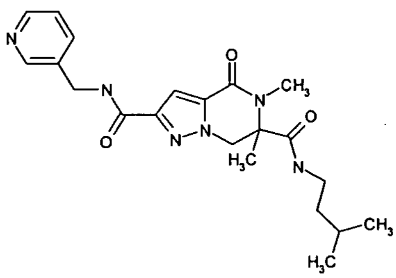 | 412,49571 | 413 |

(continued)

| | Table 1.05.(6) | | |
|---|---|---|---|
| № | Formula | Mol. Weight | *m/z* (M+1) |
| 315 | 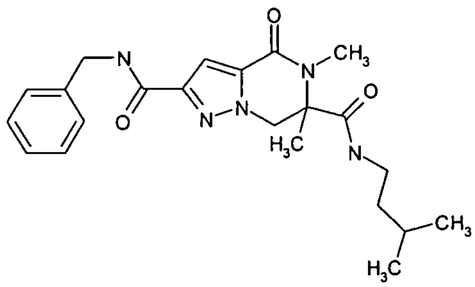 | 411,50813 | 412 |
| 316 | 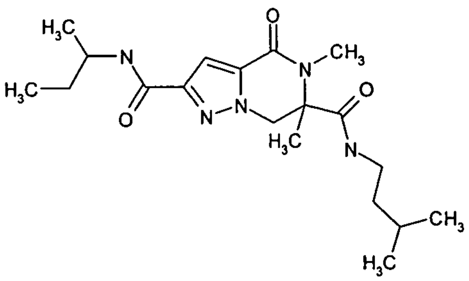 | 377,49062 | 378 |
| 317 | 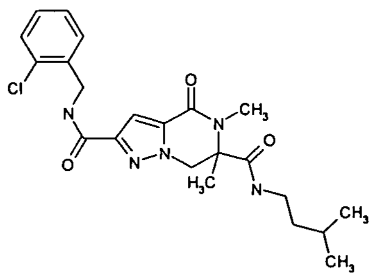 | 445,95316 | 446 |
| 318 | 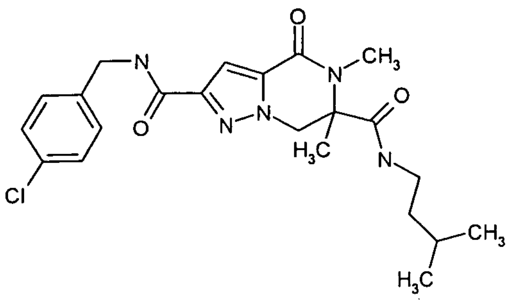 | 445,95316 | 446 |
| 319 | 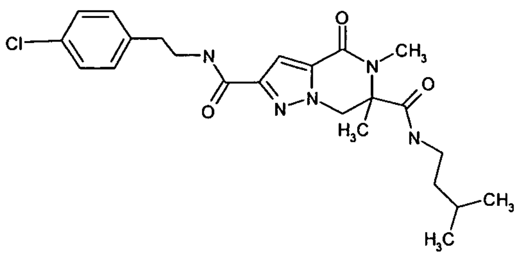 | 459,98025 | 460 |

(continued)

| | Table 1.05.(6) | | |
|---|---|---|---|
| № | Formula | Mol. Weight | m/z (M+1) |
| 320 | 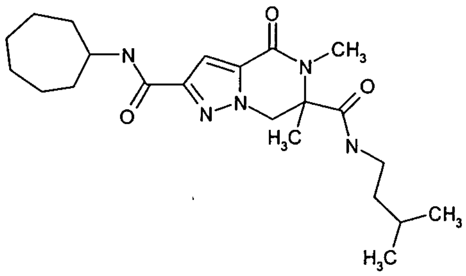 | 417,55595 | 418 |
| 321 | 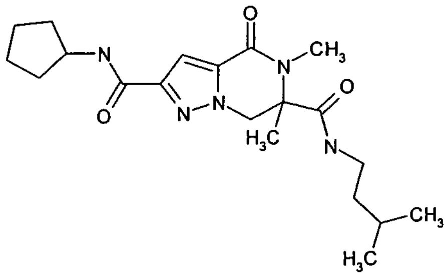 | 389,50177 | 390 |
| 322 | 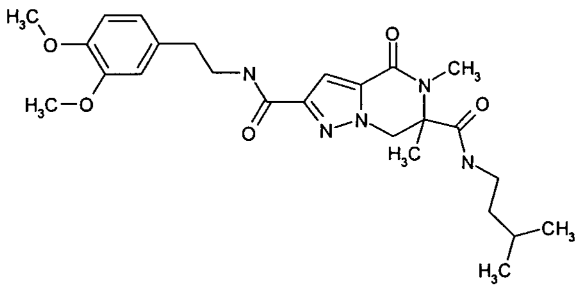 | 485,5882 | 486 |
| 323 | 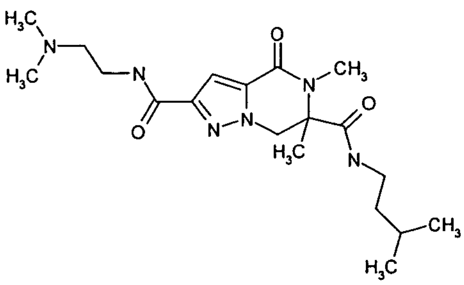 | 392,50529 | 393 |
| 324 | 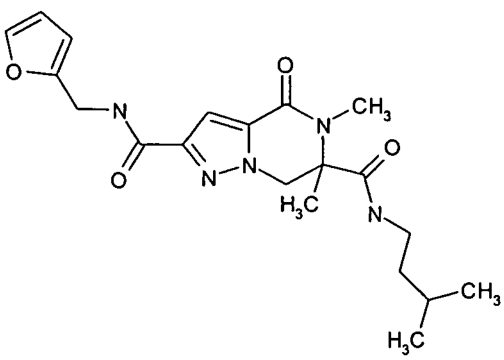 | 401,46929 | 402 |

(continued)

| | Table 1.05.(6) | | |
|---|---|---|---|
| № | Formula | Mol. Weight | m/z (M+1) |
| 325 | 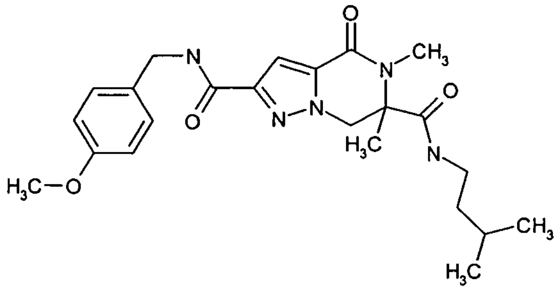 | 441,53462 | 442 |
| 326 | 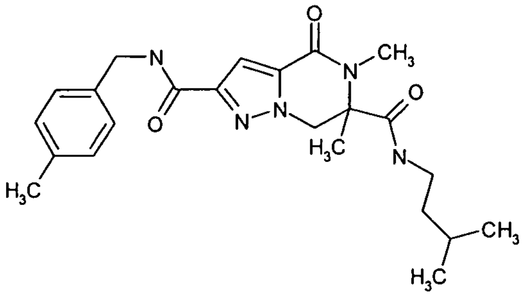 | 425,53522 | 426 |
| 327 | 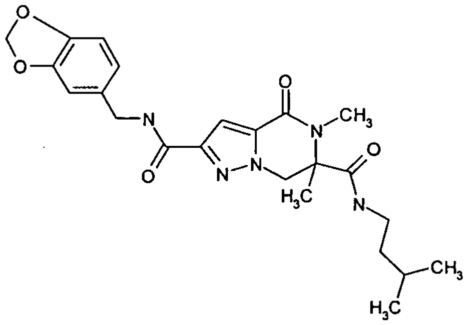 | 455,51808 | 456 |
| 328 | 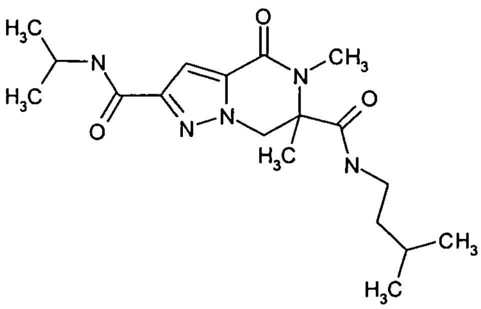 | 363,46353 | 364 |
| 329 | 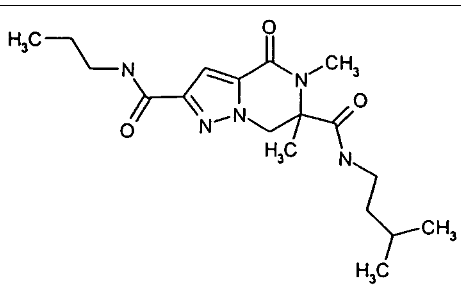 | 363,46353 | 364 |

(continued)

| | Table 1.05.(6) | | |
|---|---|---|---|
| № | Formula | Mol. Weight | *m*/*z* (M+1) |
| 330 | 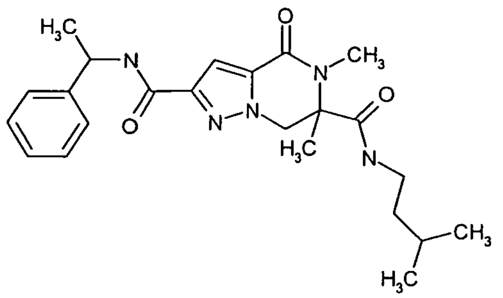 | 425,53522 | 426 |
| 331 | 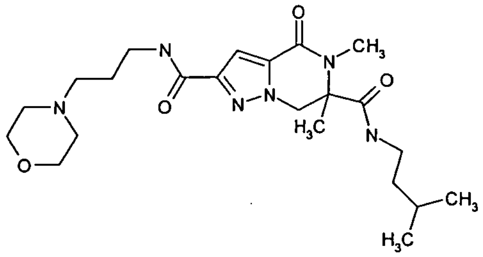 | 448,57002 | 449 |
| 332 | 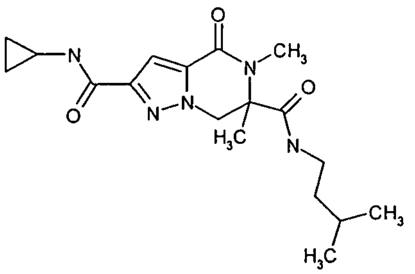 | 361,44759 | 362 |
| 333 | 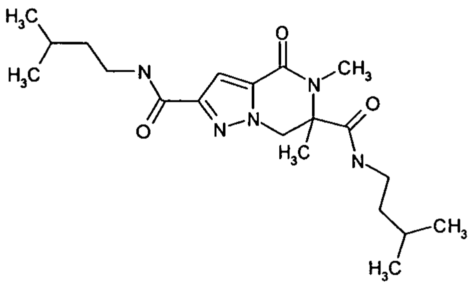 | 391,51771 | 392 |
| 334 | 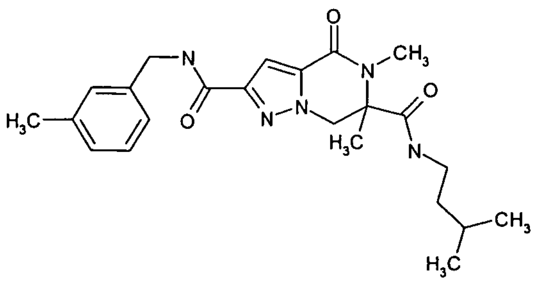 | 425,53522 | 426 |

(continued)

| | Table 1.05.(6) | | |
|---|---|---|---|
| № | Formula | Mol. Weight | *m*/*z* (M+1) |
| 335 | 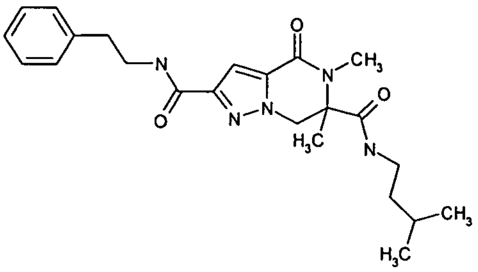 | 425,53522 | 426 |
| 336 | 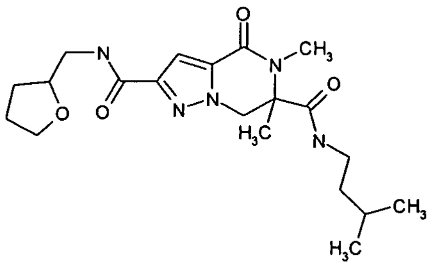 | 405,50117 | 406 |
| 337 | 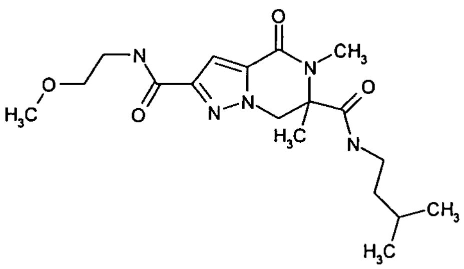 | 379,46293 | 380 |
| 338 | 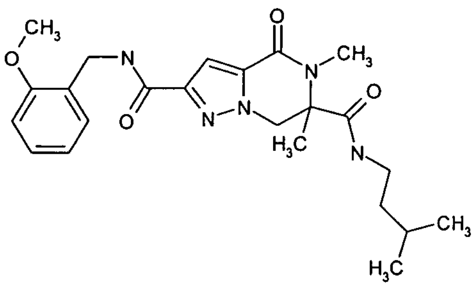 | 441,53462 | 442 |
| 339 | 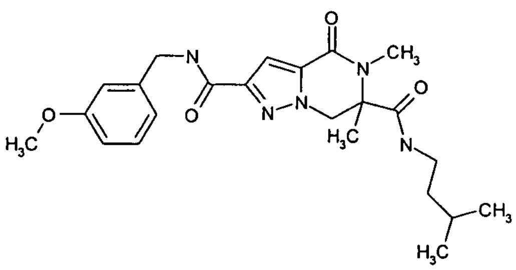 | 441,53462 | 442 |

(continued)

| | Table 1.05.(6) | | |
|---|---|---|---|
| № | Formula | Mol. Weight | m/z (M+1) |
| 340 | 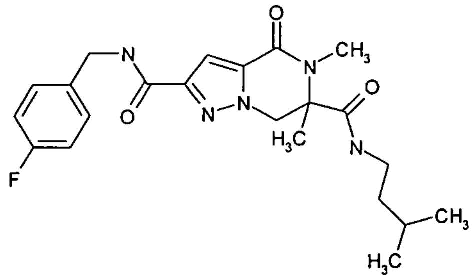 | 429,49856 | 430 |
| 341 | 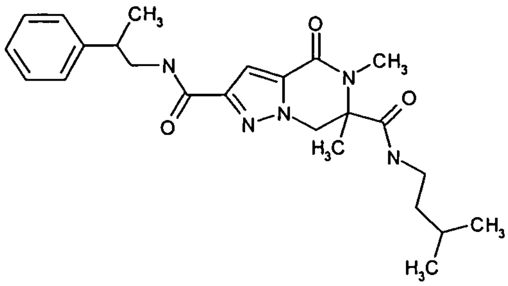 | 439,56231 | 440 |
| 342 | 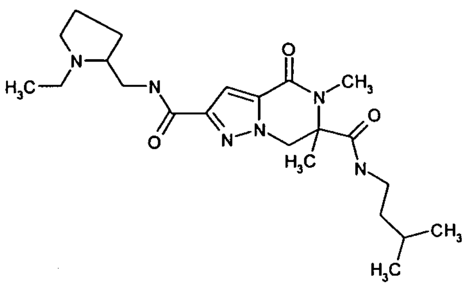 | 432,57062 | 433 |
| 343 | 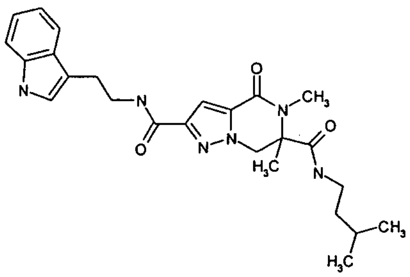 | 464,57219 | 465 |
| 344 | 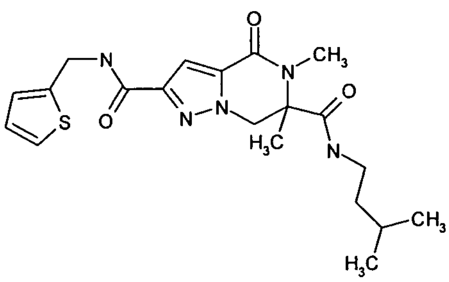 | 417,53389 | 418 |

(continued)

| | Table 1.05.(6) | | |
|---|---|---|---|
| № | Formula | Mol. Weight | m/z (M+1) |
| 345 | 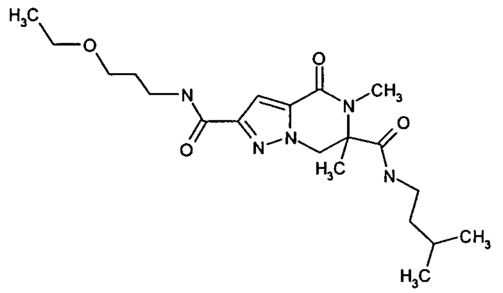 | 407,51711 | 408 |
| 346 | 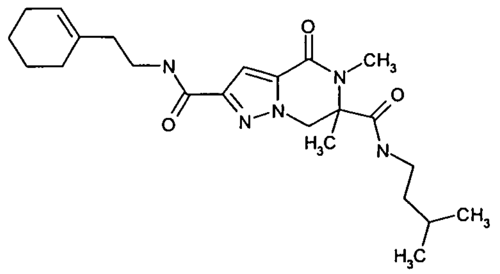 | 429,5671 | 430 |
| 347 | 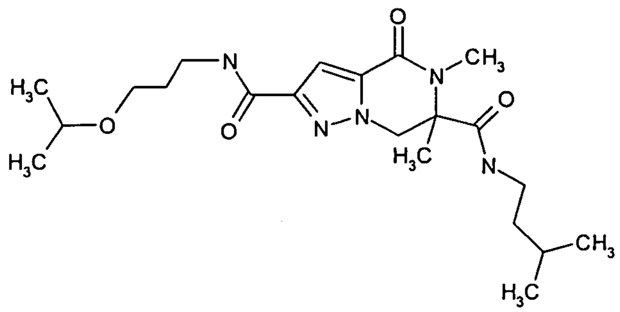 | 421,5442 | 422 |
| 348 | 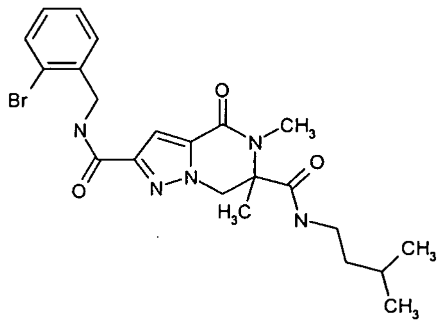 | 490,40416 | 491 |
| 349 | 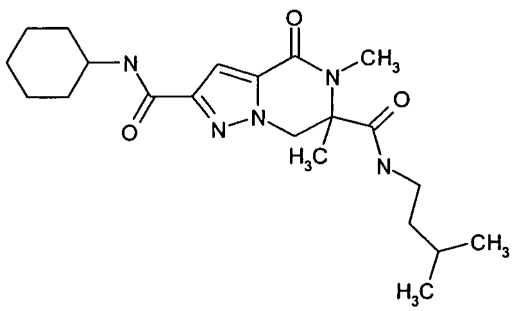 | 403,52886 | 404 |

(continued)

| | Table 1.05.(6) | | |
|---|---|---|---|
| № | Formula | Mol. Weight | *m*/*z* (M+1) |
| 350 | 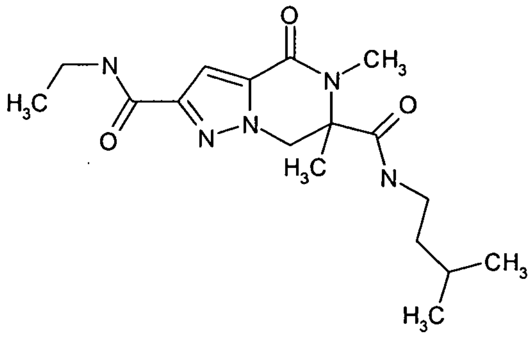 | 349,43644 | 350 |
| 351 | 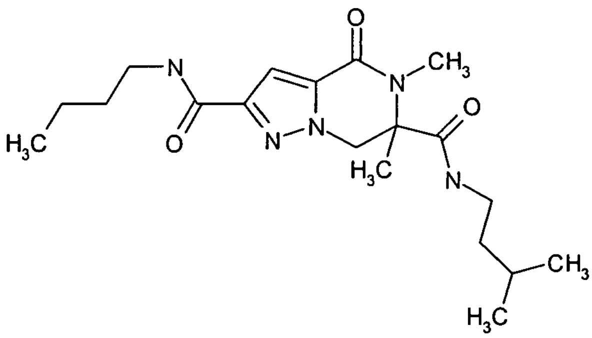 | 377,49062 | 378 |
| 352 | 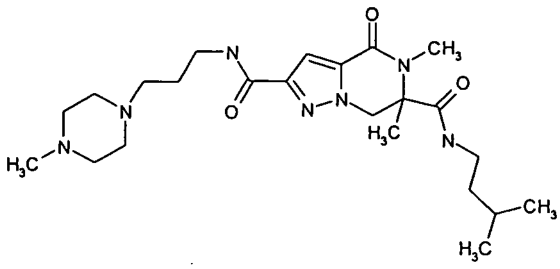 | 461,61238 | 462 |
| 353 | 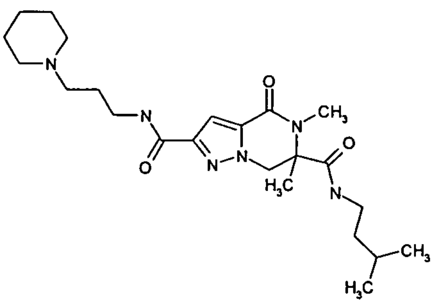 | 446,59771 | 447 |

(continued)

| | Table 1.05.(6) | | |
|---|---|---|---|
| № | Formula | Mol. Weight | *m*/*z* (M+1) |
| 354 | 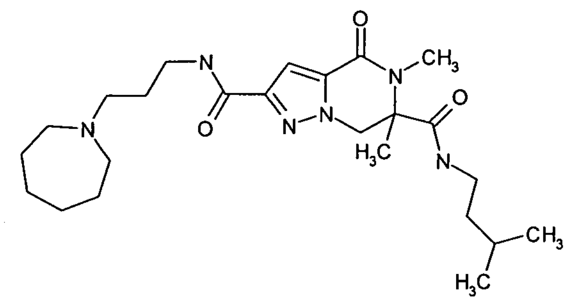 | 460,6248 | 461 |
| 355 | 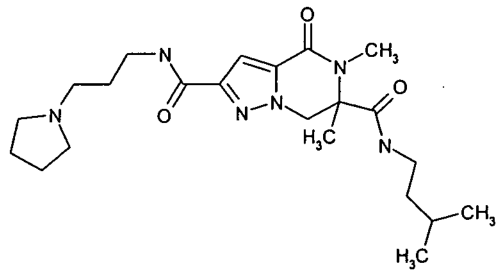 | 432,57062 | 433 |
| 356 | 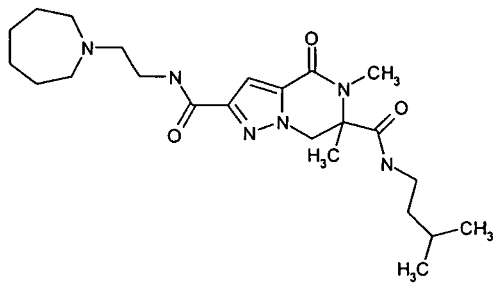 | 446,59771 | 447 |
| 357 | 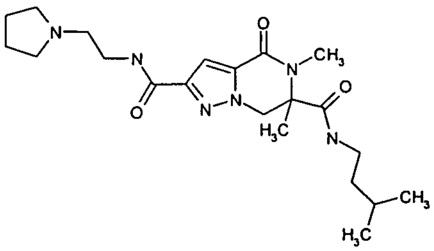 | 418,54353 | 419 |
| 358 | 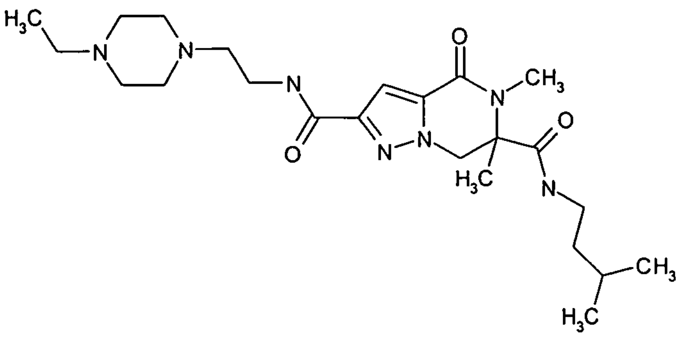 | 461,61238 | 462 |

(continued)

| | Table 1.05.(6) | | |
|---|---|---|---|
| № | Formula | Mol. Weight | *m*/*z* (M+1) |
| 359 | 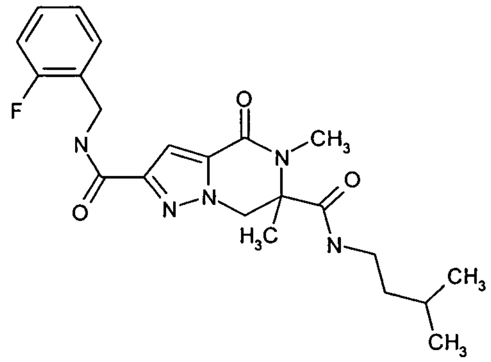 | 429,49856 | 430 |
| 360 | 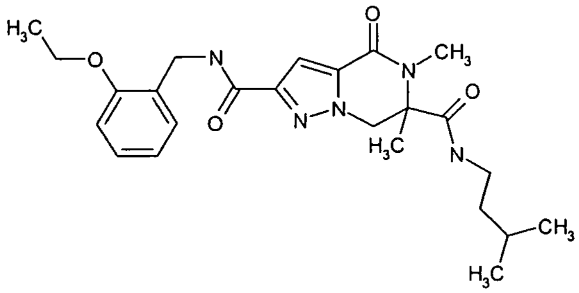 | 455,56171 | 456 |
| 361 | 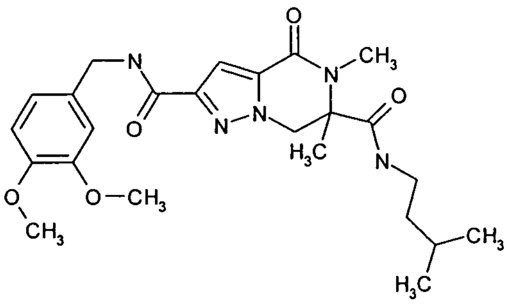 | 471,56111 | 472 |
| 362 | 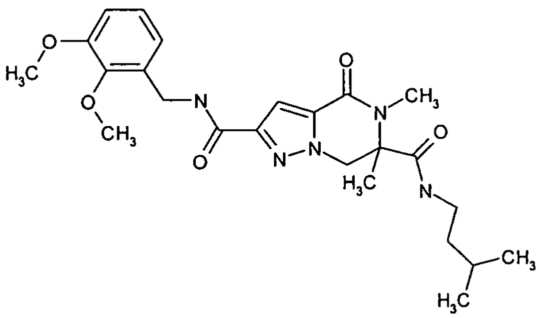 | 471,56111 | 472 |

(continued)

| | Table 1.05.(6) | | |
|---|---|---|---|
| № | Formula | Mol. Weight | *m*/*z* (M+1) |
| 363 | 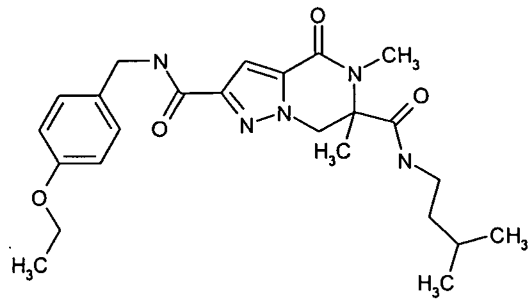 | 455,56171 | 456 |
| 364 | 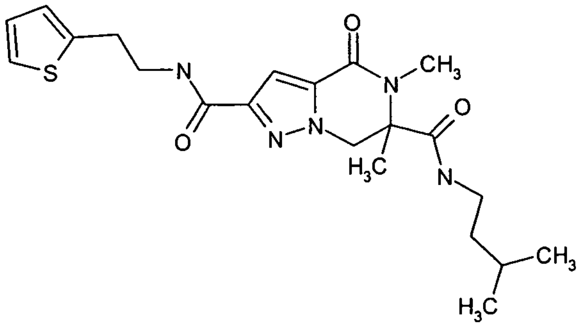 | 431,56098 | 432 |
| 365 | 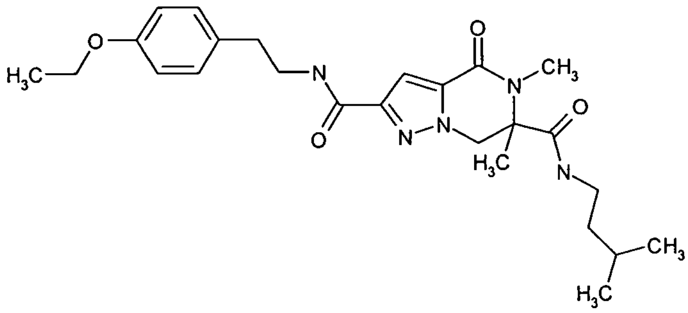 | 469,5888 | 470 |
| 366 | 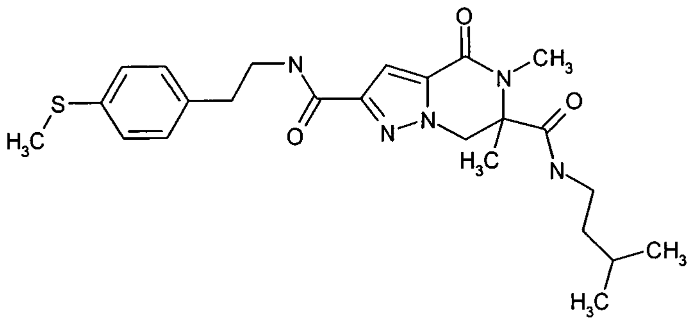 | 471,62631 | 472 |

(continued)

| | Table 1.05.(6) | | |
|---|---|---|---|
| № | Formula | Mol. Weight | m/z (M+1) |
| 367 | 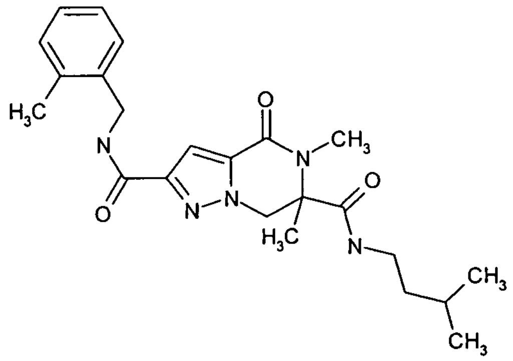 | 425,53522 | 426 |
| 368 | 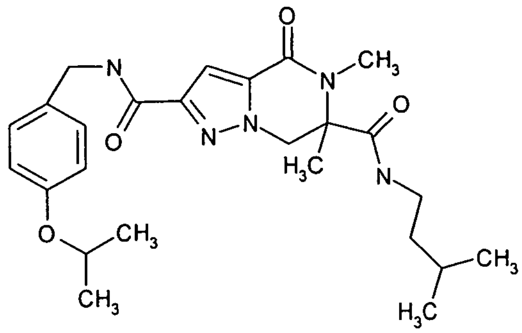 | 469,5888 | 470 |
| 369 | 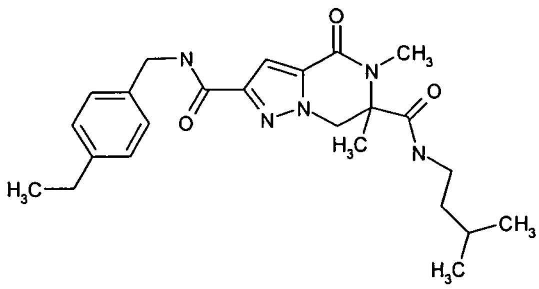 | 439,56231 | 440 |
| 370 | 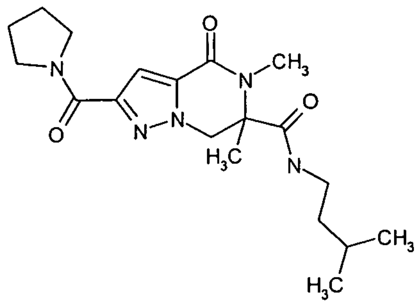 | 375,47468 | 376 |

(continued)

| | Table 1.05.(6) | | |
|---|---|---|---|
| № | Formula | Mol. Weight | m/z (M+1) |
| 371 | 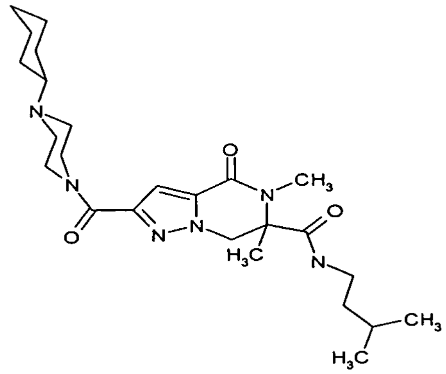 | 472,63595 | 473 |
| 372 | 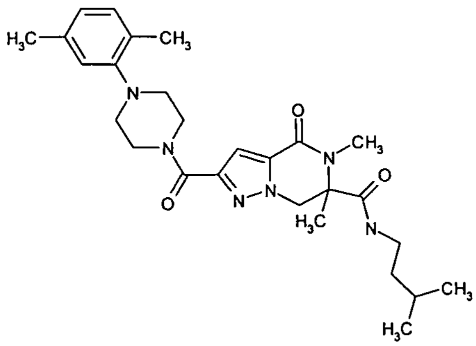 | 494,64231 | 495 |
| 373 | 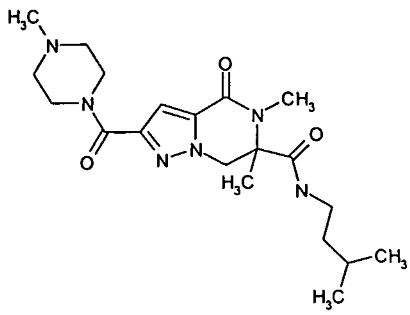 | 404,51644 | 405 |
| 374 | 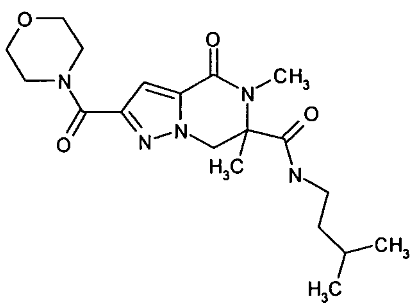 | 391,47408 | 392 |

(continued)

| | Table 1.05.(6) | | |
|---|---|---|---|
| № | Formula | Mol. Weight | *m*/*z* (M+1) |
| 375 | 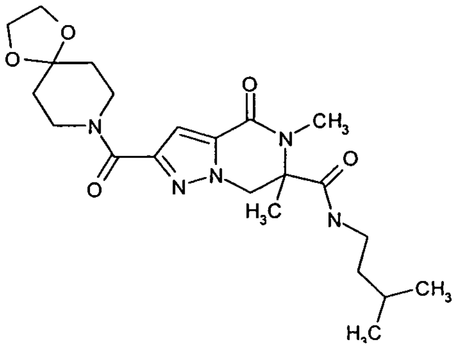 | 447,53881 | 448 |
| 376 | 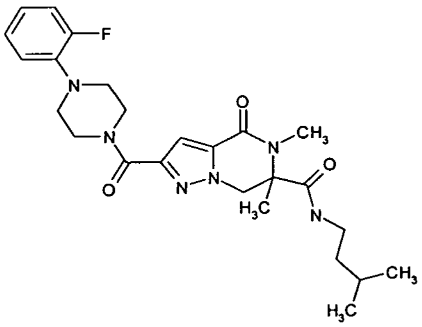 | 484,57856 | 485 |
| 377 | 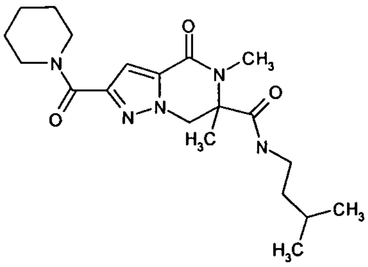 | 389,50177 | 390 |
| 378 | 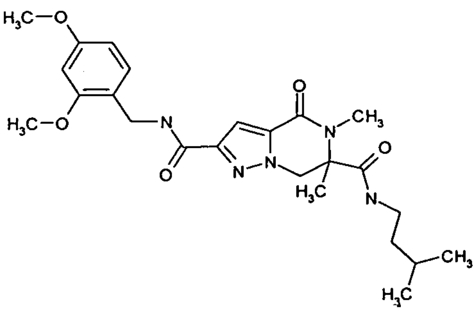 | 471,56111 | 472 |

(continued)

| | Table 1.05.(6) | | |
|---|---|---|---|
| № | Formula | Mol. Weight | *m/z* (M+1) |
| 379 | 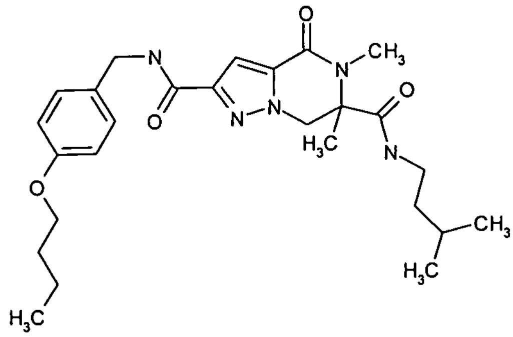 | 483,61589 | 484 |
| 380 | 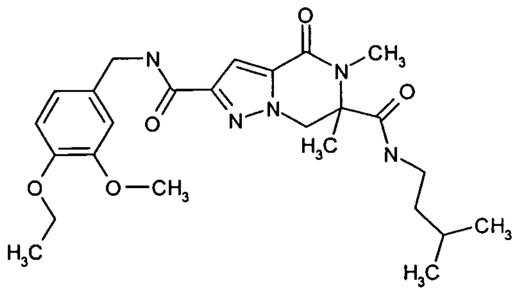 | 485,5882 | 486 |
| 381 | 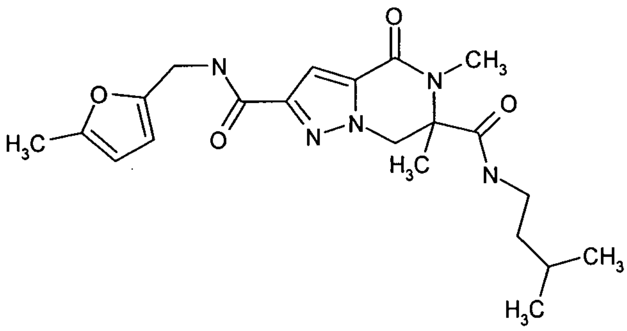 | 415,49638 | 416 |
| 382 | 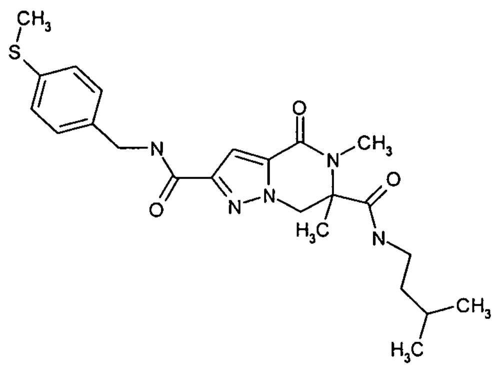 | 457,59922 | 458 |

(continued)

| | Table 1.05.(6) | | |
|---|---|---|---|
| № | Formula | Mol. Weight | *m/z* (M+1) |
| 383 | 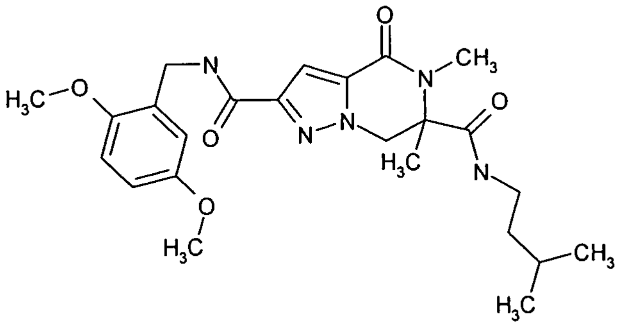 | 471,56111 | 472 |
| 384 | 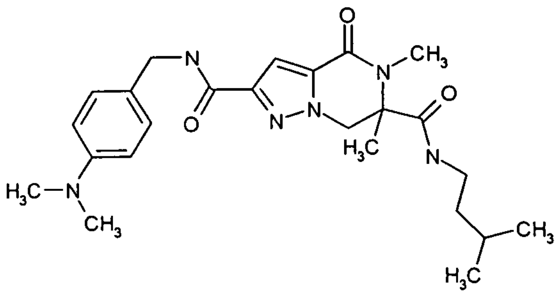 | 454,57698 | 455 |
| 385 | 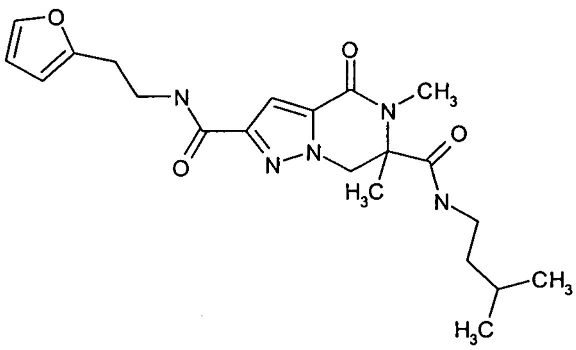 | 415,49638 | 416 |
| 386 | 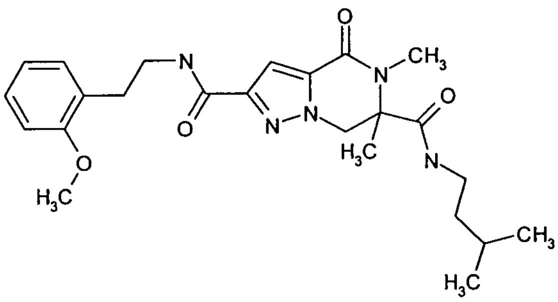 | 455,56171 | 456 |

(continued)

| | Table 1.05.(6) | | |
|---|---|---|---|
| № | Formula | Mol. Weight | m/z (M+1) |
| 387 | 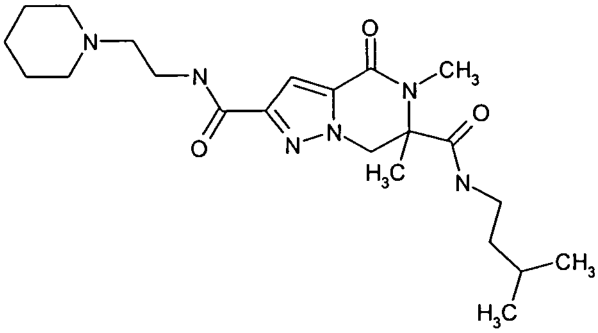 | 432,57062 | 433 |
| 388 | 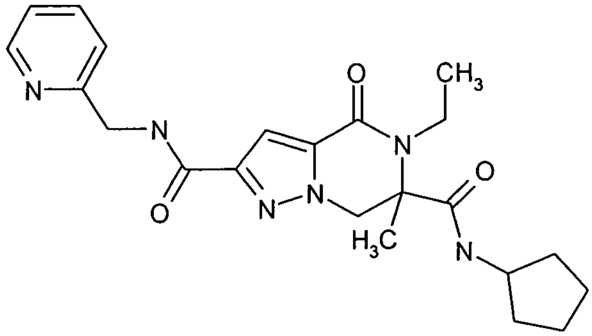 | 424,50686 | 425 |
| 389 | 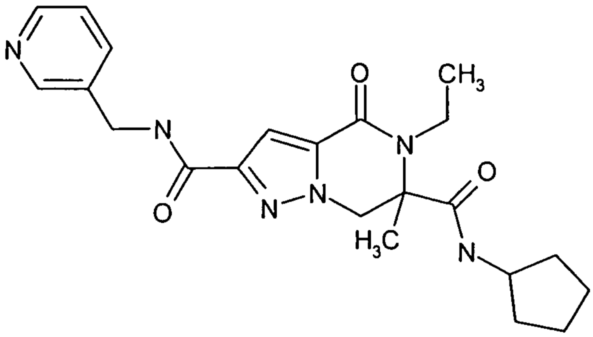 | 424,50686 | 425 |
| 390 | 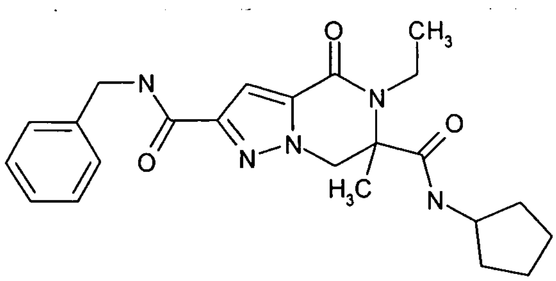 | 423,51928 | 424 |

(continued)

| | Table 1.05.(6) | | |
|---|---|---|---|
| № | Formula | Mol. Weight | m/z (M+1) |
| 391 | 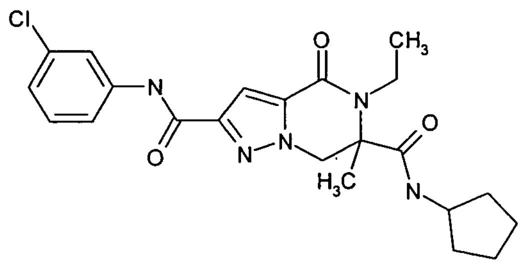 | 443,93722 | 444 |
| 392 | 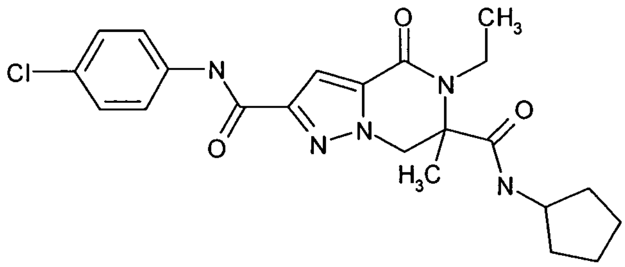 | 443,93722 | 444 |
| 393 | 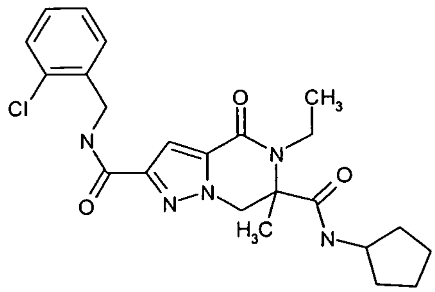 | 457,96431 | 458 |
| 394 | 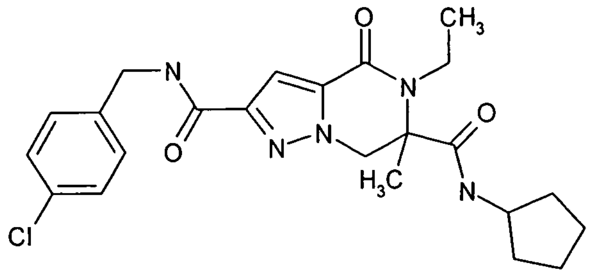 | 457,96431 | 458 |
| 395 | 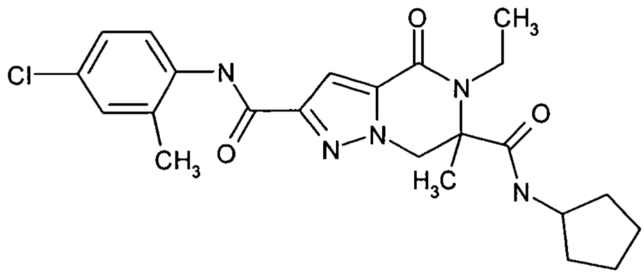 | 457,96431 | 458 |

(continued)

| | Table 1.05.(6) | | |
|---|---|---|---|
| № | Formula | Mol. Weight | *m/z* (M+1) |
| 396 | 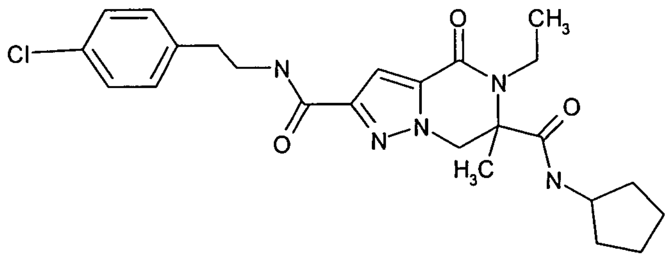 | 471,9914 | 472 |
| 397 | 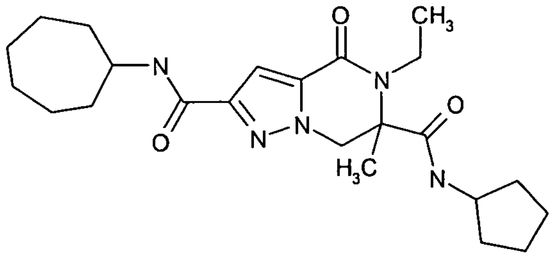 | 429,5671 | 430 |
| 398 | 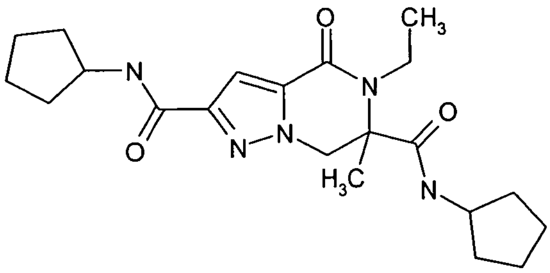 | 401,51292 | 402 |
| 399 | 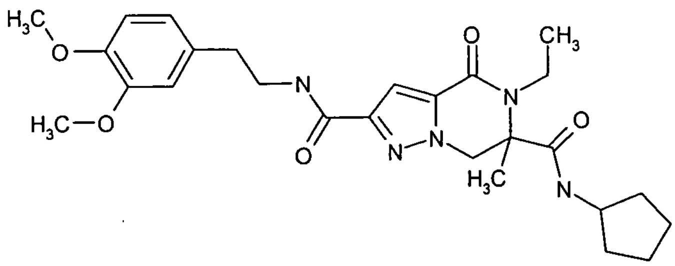 | 497,59935 | 498 |
| 400 | 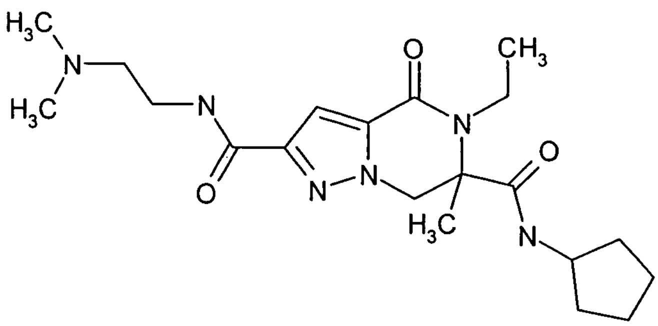 | 404,51644 | 405 |

(continued)

| | Table 1.05.(6) | | |
|---|---|---|---|
| № | Formula | Mol. Weight | m/z (M+1) |
| 401 | 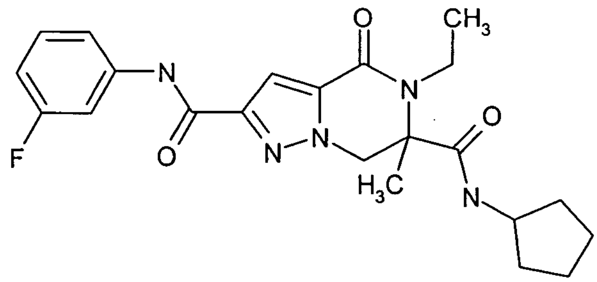 | 427,48262 | 428 |
| 402 | 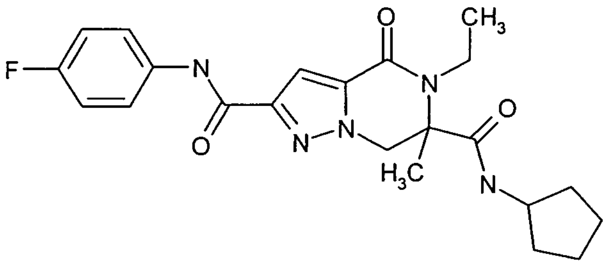 | 427,48262 | 428 |
| 403 | 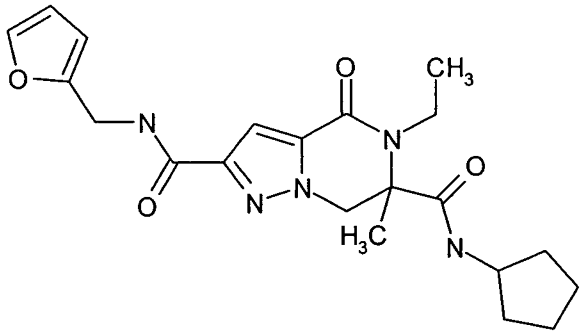 | 413,48044 | 414 |
| 404 | 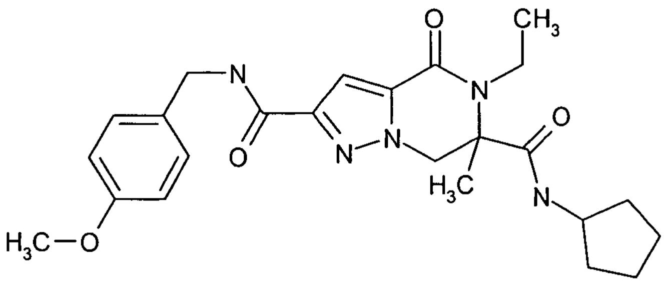 | 453,54577 | 454 |
| 405 | 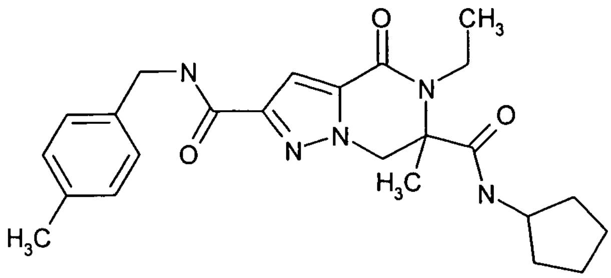 | 437,54637 | 438 |

(continued)

| | Table 1.05.(6) | | |
|---|---|---|---|
| № | Formula | Mol. Weight | *m/z* (M+1) |
| 406 | 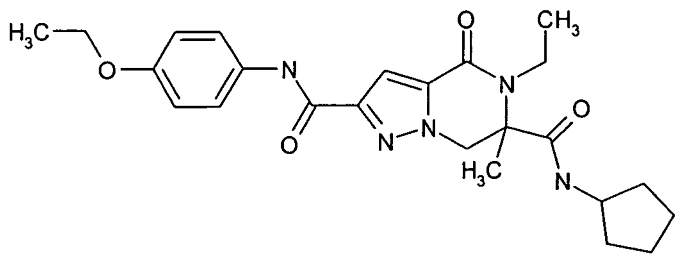 | 453,54577 | 454 |
| 407 | 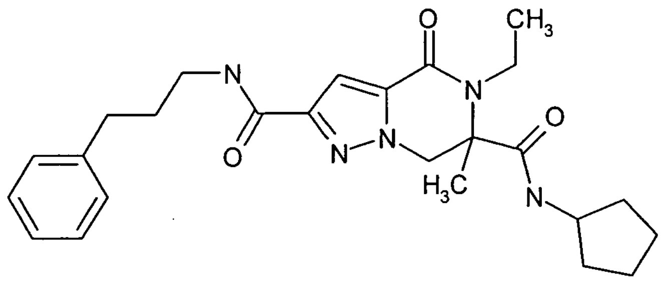 | 451,57346 | 452 |
| 408 | 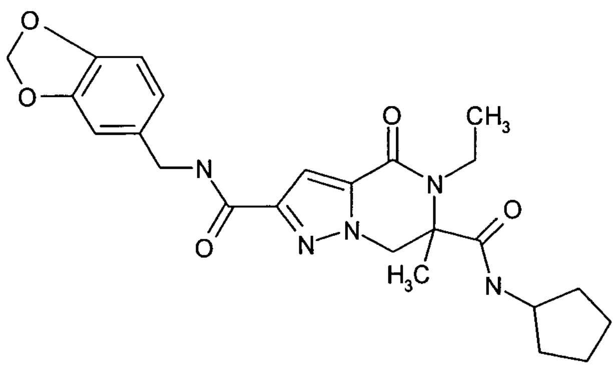 | 467,52923 | 468 |
| 409 | 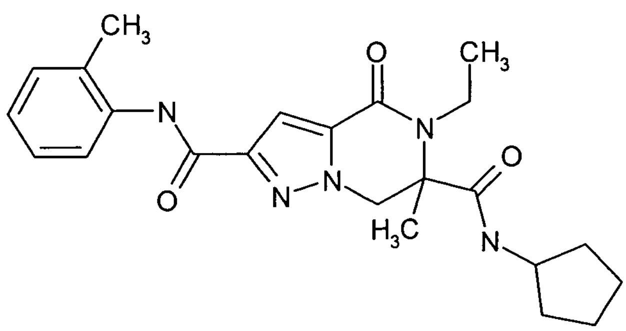 | 423,51928 | 424 |
| 410 | 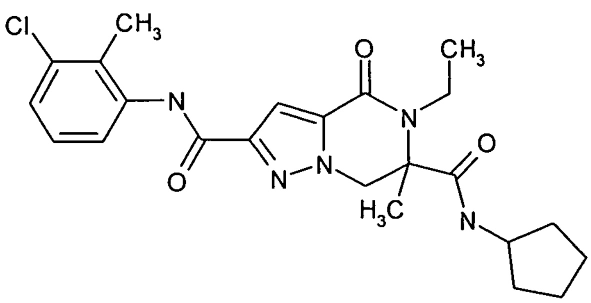 | 457,96431 | 458 |

(continued)

| | Table 1.05.(6) | | |
|---|---|---|---|
| № | Formula | Mol. Weight | m/z (M+1) |
| 411 | 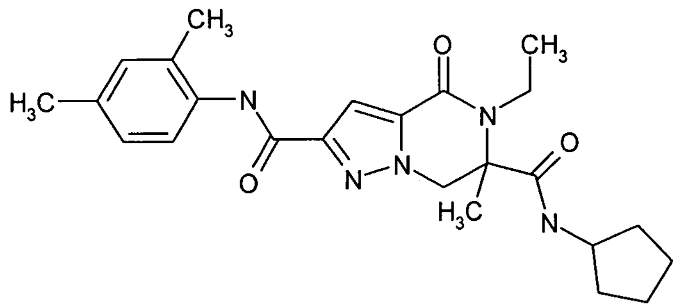 | 437,54637 | 438 |
| 412 | 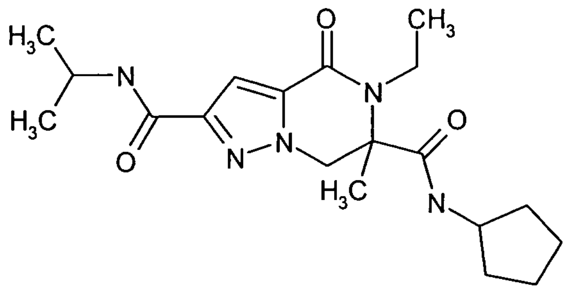 | 375,47468 | 376 |
| 413 | 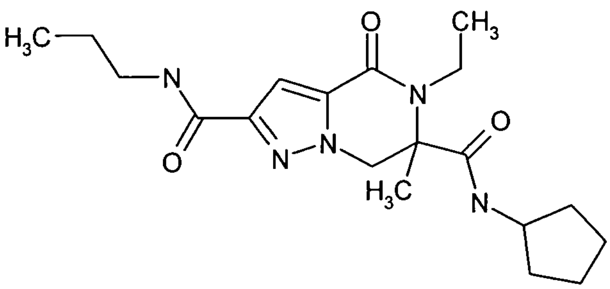 | 375,47468 | 376 |
| 414 | 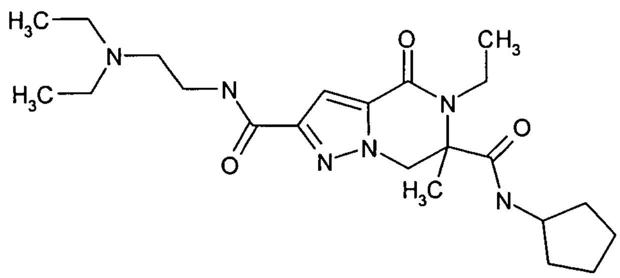 | 432,57062 | 433 |
| 415 | 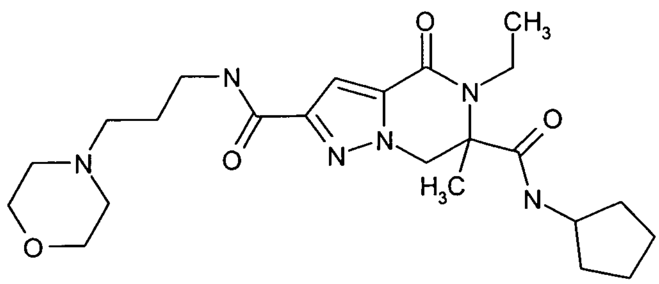 | 460,58117 | 461 |

(continued)

| | Table 1.05.(6) | | |
|---|---|---|---|
| № | Formula | Mol. Weight | m/z (M+1) |
| 416 | 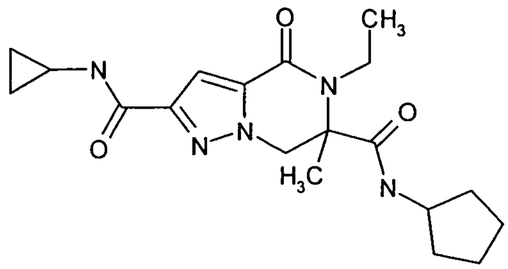 | 373,45874 | 374 |
| 417 | 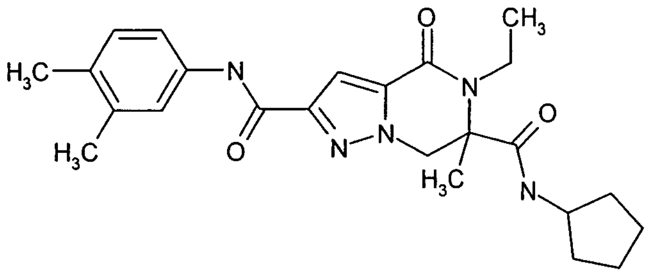 | 437,54637 | 438 |
| 418 | 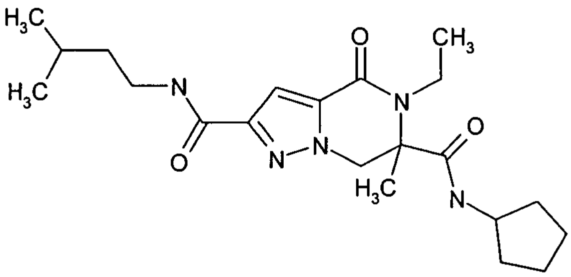 | 403,52886 | 404 |
| 419 | 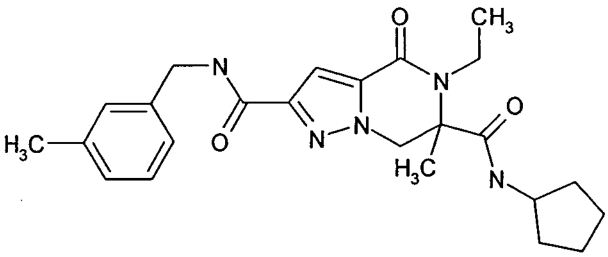 | 437,54637 | 438 |
| 420 | 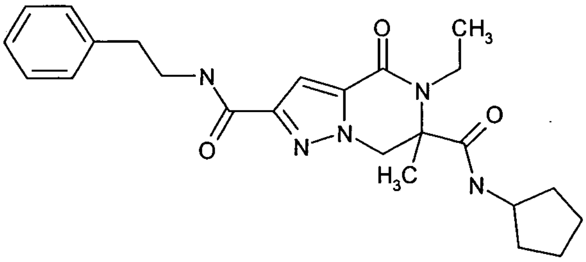 | 437,54637 | 438 |

(continued)

| | Table 1.05.(6) | | |
|---|---|---|---|
| № | Formula | Mol. Weight | *m/z* (M+1) |
| 421 | 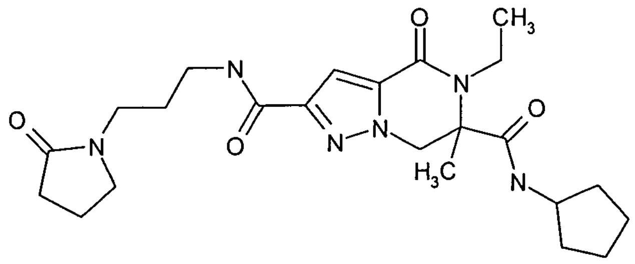 | 458,56523 | 459 |
| 422 | 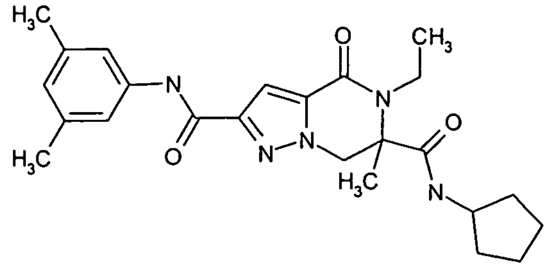 | 437,54637 | 438 |
| 423 | 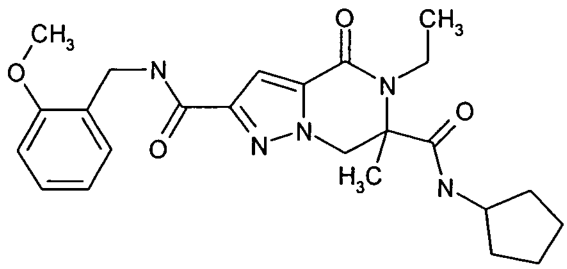 | 453,54577 | 454 |
| 424 | 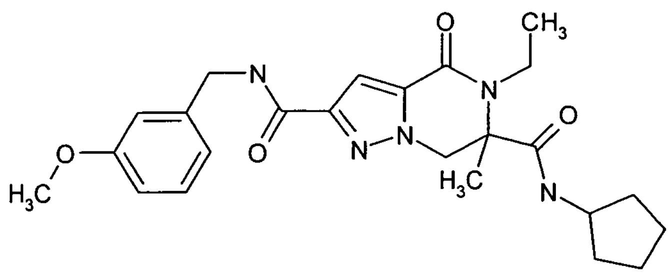 | 453,54577 | 454 |
| 425 | 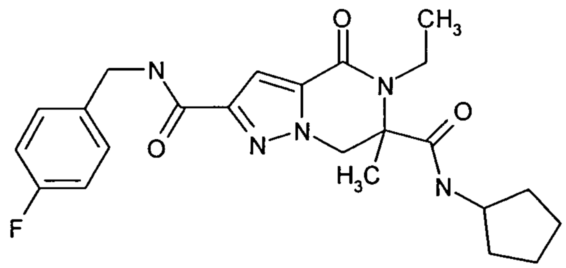 | 441,50971 | 442 |

(continued)

| | Table 1.05.(6) | | |
|---|---|---|---|
| № | Formula | Mol. Weight | *m/z* (M+1) |
| 426 | 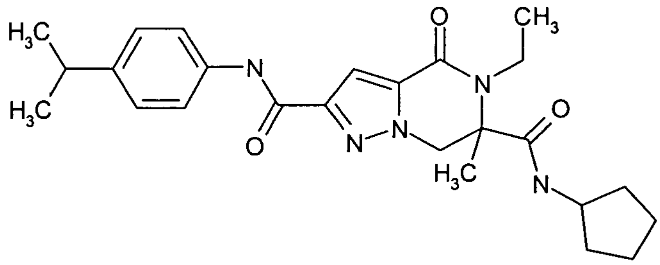 | 451,57346 | 452 |
| 427 | 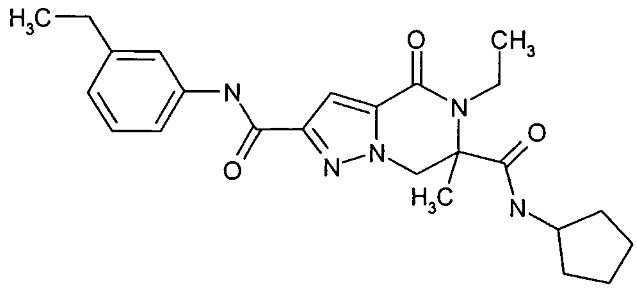 | 437,54637 | 438 |
| 428 | 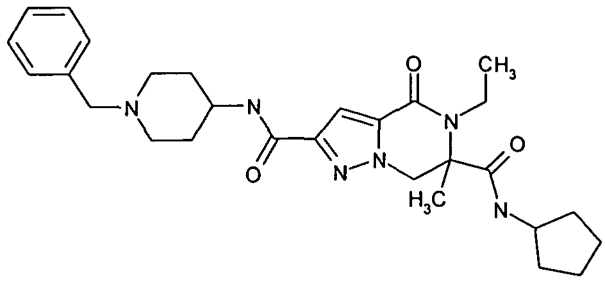 | 506,65346 | 507 |
| 429 | 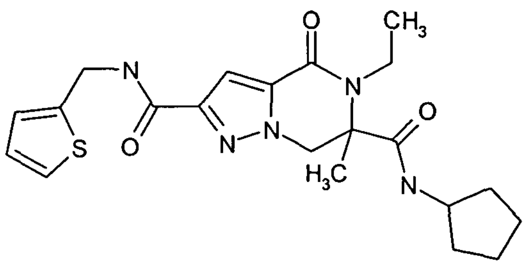 | 429,54504 | 430 |
| 430 | 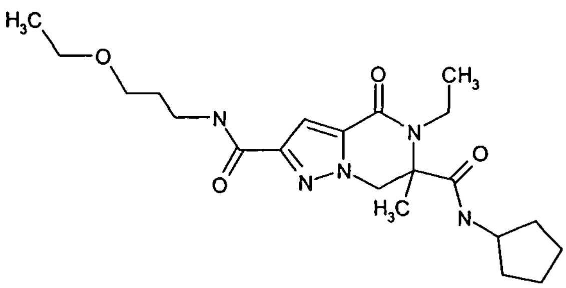 | 419,52826 | 420 |

(continued)

| | Table 1.05.(6) | | |
|---|---|---|---|
| № | Formula | Mol. Weight | *m*/*z* (M+1) |
| 431 | 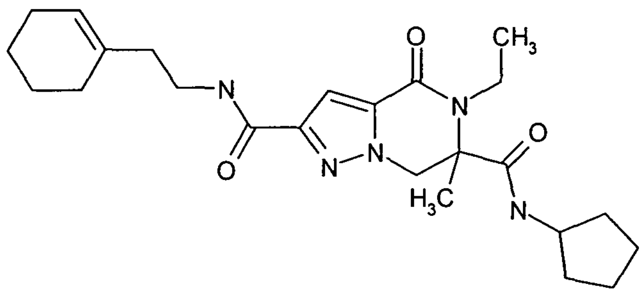 | 441,57825 | 442 |
| 432 | 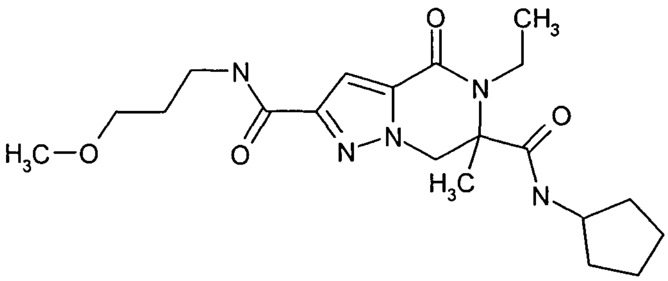 | 405,50117 | 406 |
| 433 | 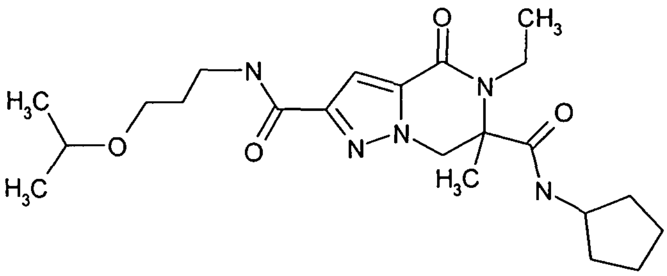 | 433,55535 | 434 |
| 434 | 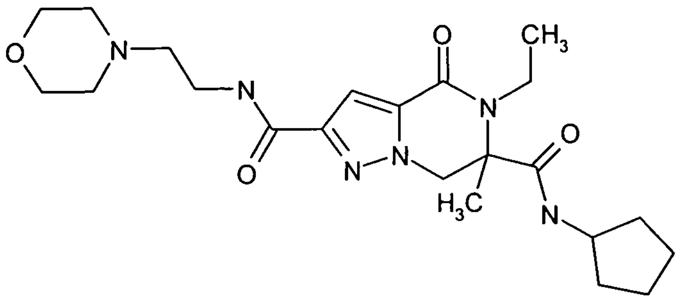 | 446,55408 | 447 |
| 435 | 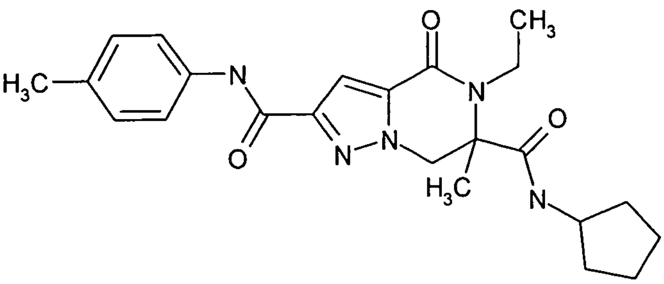 | 423,51928 | 424 |

(continued)

| | Table 1.05.(6) | | |
|---|---|---|---|
| № | Formula | Mol. Weight | *m*/*z* (M+1) |
| 436 | 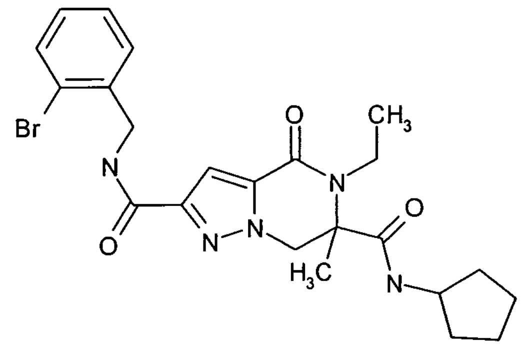 | 502,41531 | 503 |
| 437 | 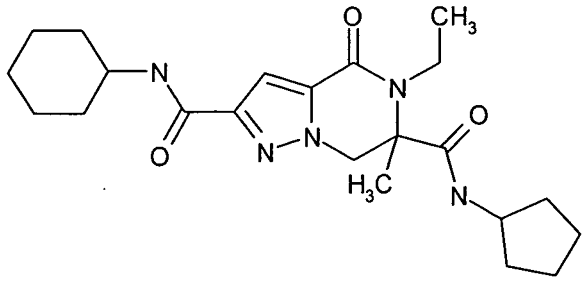 | 415,54001 | 416 |
| 438 | 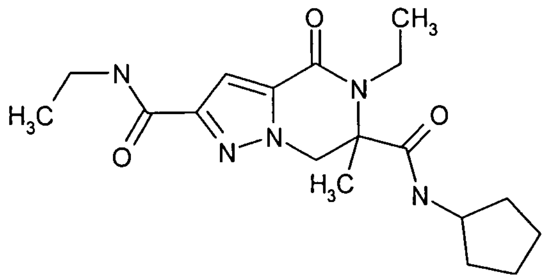 | 361,44759 | 362 |
| 439 | 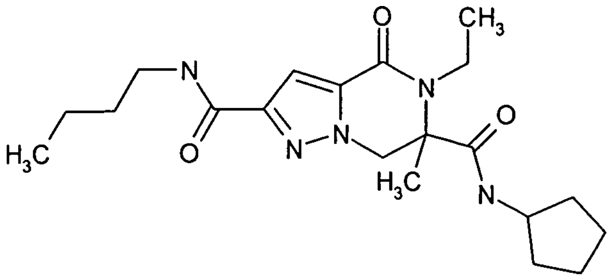 | 389,50177 | 390 |
| 440 | 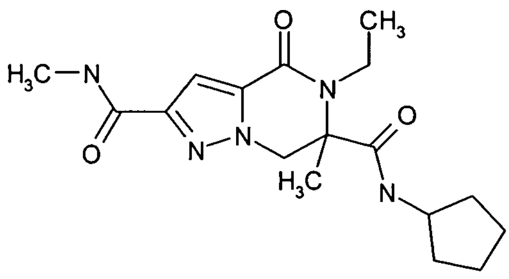 | 347,4205 | 348 |

(continued)

| | Table 1.05.(6) | | |
|---|---|---|---|
| № | Formula | Mol. Weight | *m/z* (M+1) |
| 441 | 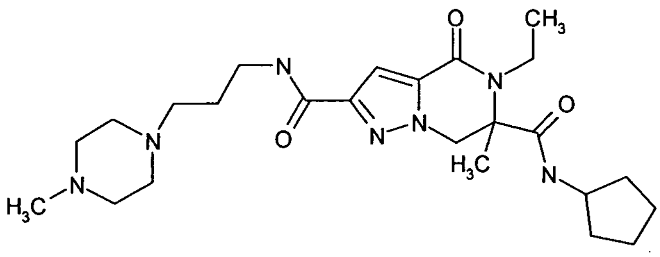 | 473,62353 | 474 |
| 442 | 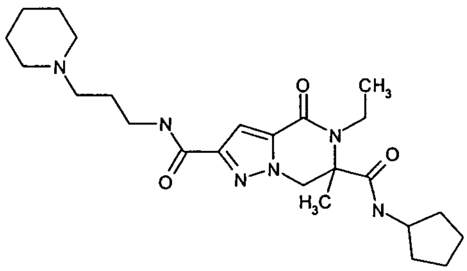 | 458,60886 | 459 |
| 443 | 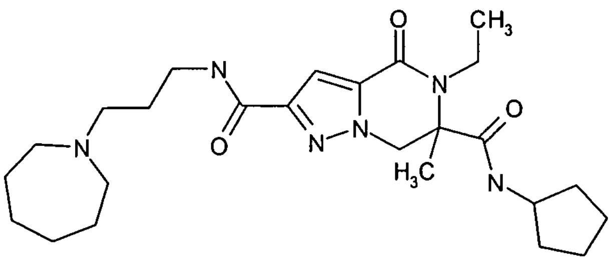 | 472,63595 | 473 |
| 444 | 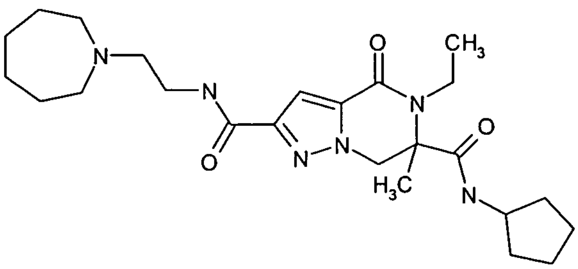 | 458,60886 | 459 |
| 445 | 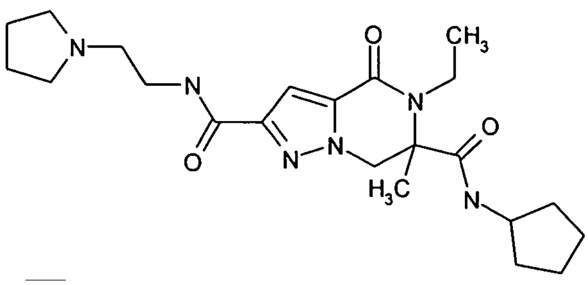 | 430,55468 | 431 |

(continued)

| | Table 1.05.(6) | | |
|---|---|---|---|
| № | Formula | Mol. Weight | *m*/*z* (M+1) |
| 446 | 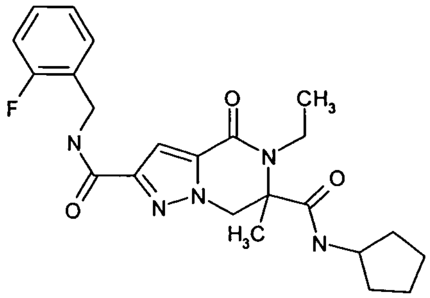 | 441,50971 | 442 |
| 447 | 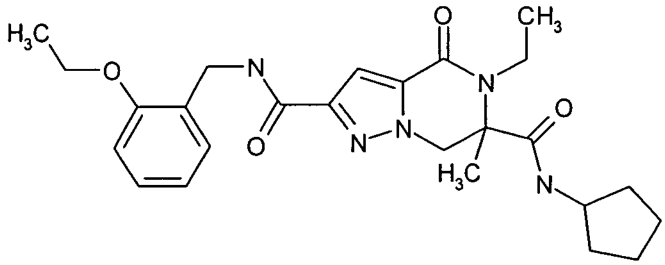 | 467,57286 | 468 |
| 448 | 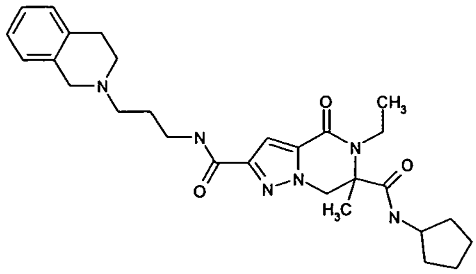 | 506,65346 | 507 |
| 449 | 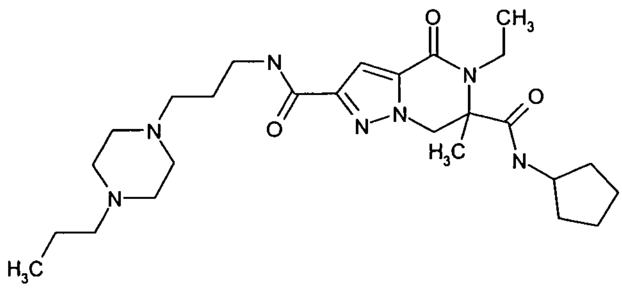 | 501,67771 | 502 |
| 450 | 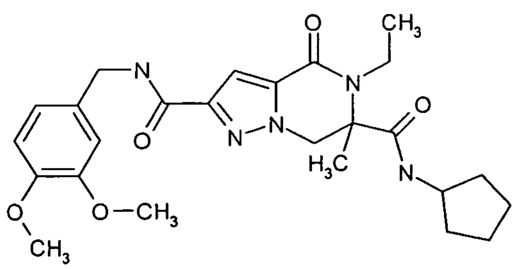 | 483,57226 | 484 |

(continued)

| | Table 1.05.(6) | | |
|---|---|---|---|
| № | Formula | Mol. Weight | m/z (M+1) |
| 451 | 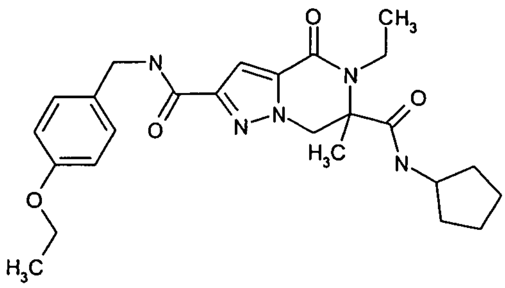 | 467,57286 | 468 |
| 452 | 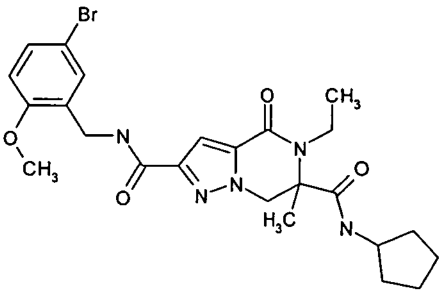 | 532,4418 | 533 |
| 453 | 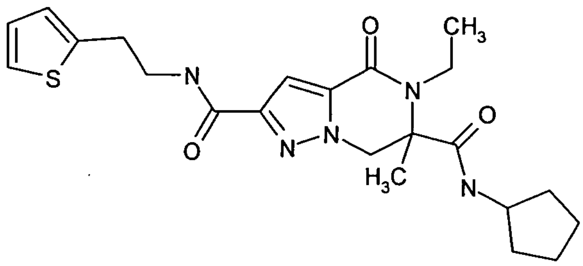 | 443,57213 | 444 |
| 454 | 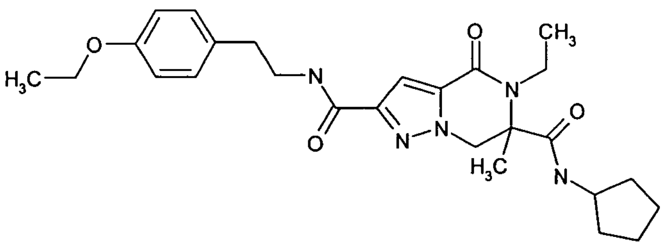 | 481,59995 | 482 |
| 455 | 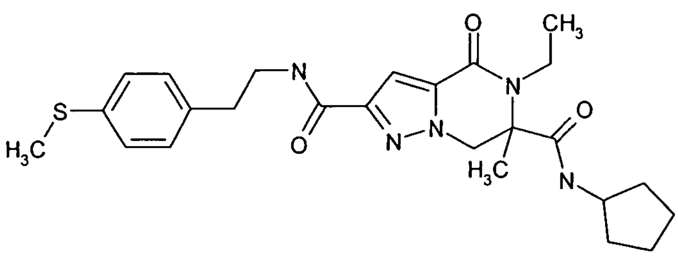 | 483,63746 | 484 |

(continued)

| | Table 1.05.(6) | | |
|---|---|---|---|
| № | Formula | Mol. Weight | *m*/*z* (M+1) |
| 456 | 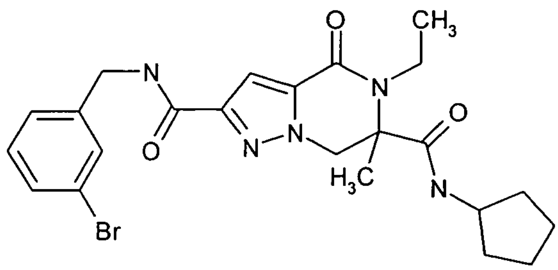 | 502,41531 | 503 |
| 457 | 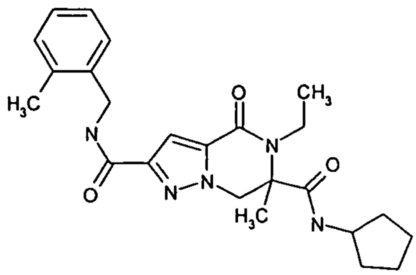 | 437,54637 | 438 |
| 458 | 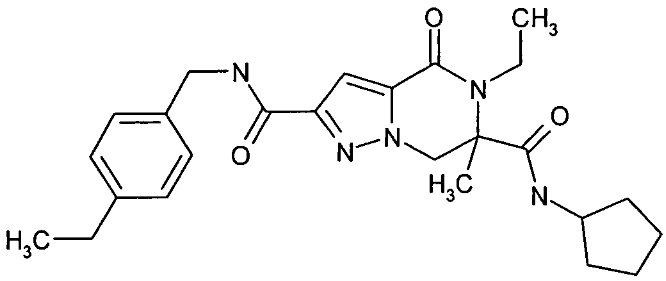 | 451,57346 | 452 |
| 459 | 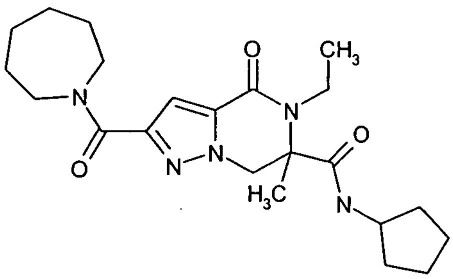 | 415,54001 | 416 |
| 460 | 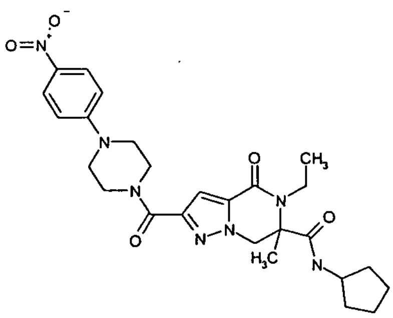 | 523,59681 | 524 |

(continued)

| | Table 1.05.(6) | | |
|---|---|---|---|
| № | Formula | Mol. Weight | *m/z* (M+1) |
| 461 | 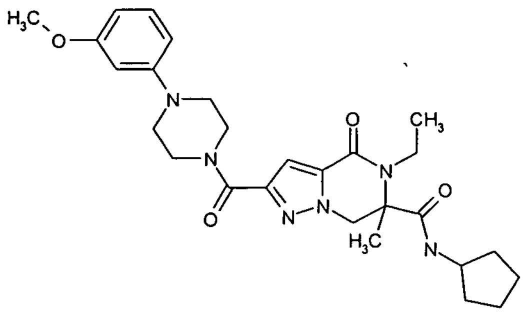 | 508,62577 | 509 |
| 462 | 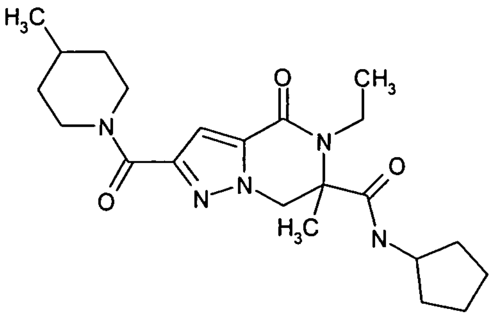 | 415,54001 | 416 |
| 463 | 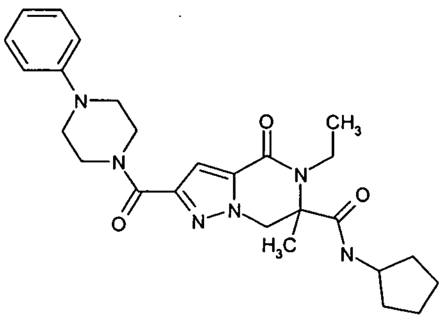 | 478,59928 | 479 |
| 464 | 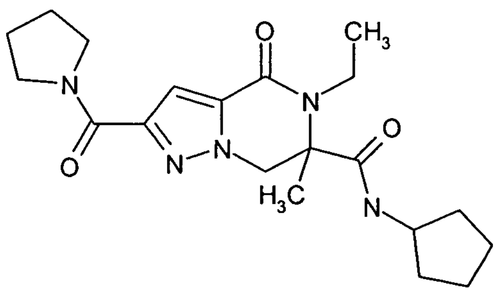 | 387,48583 | 388 |

(continued)

| | Table 1.05.(6) | | |
|---|---|---|---|
| № | Formula | Mol. Weight | *m/z* (M+1) |
| 465 | 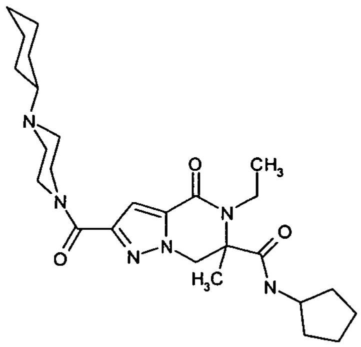 | 484,6471 | 485 |
| 466 | 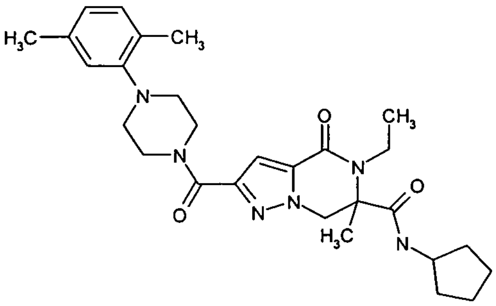 | 506,65346 | 507 |
| 467 | 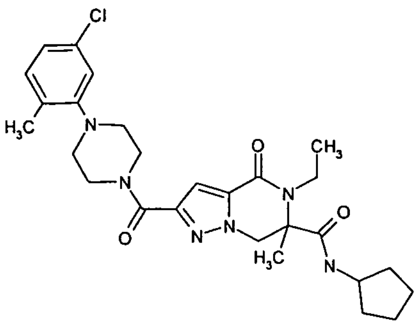 | 527,0714 | 528 |
| 468 | 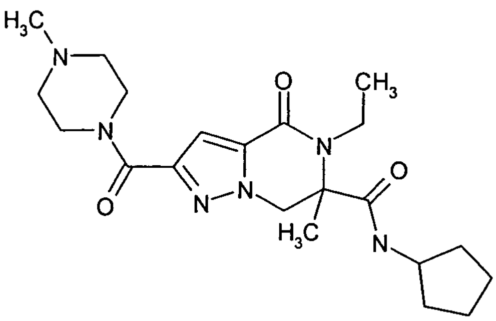 | 416,52759 | 417 |

(continued)

| | Table 1.05.(6) | | |
|---|---|---|---|
| № | Formula | Mol. Weight | *m*/*z* (M+1) |
| 469 | 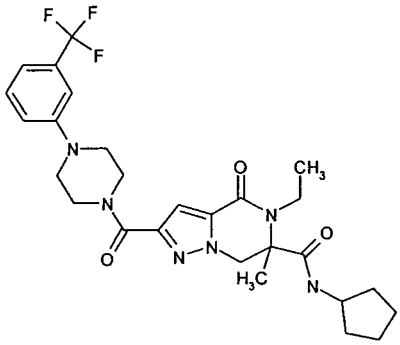 | 546,59766 | 547 |
| 470 | 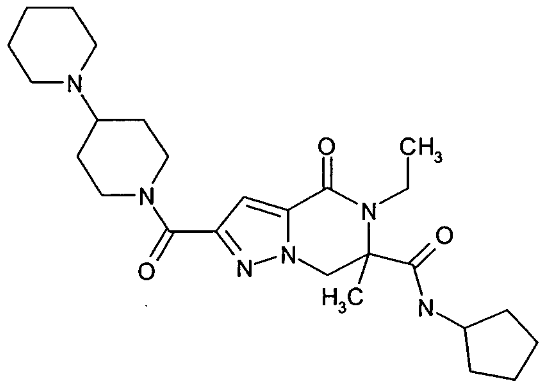 | 484,6471 | 485 |
| 471 | 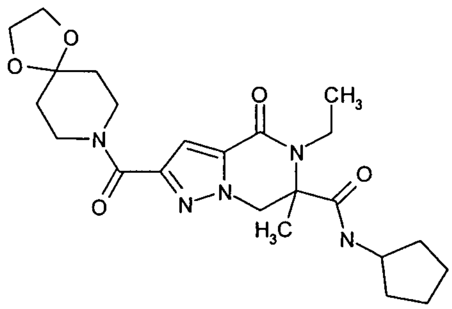 | 459,54996 | 460 |
| 472 | 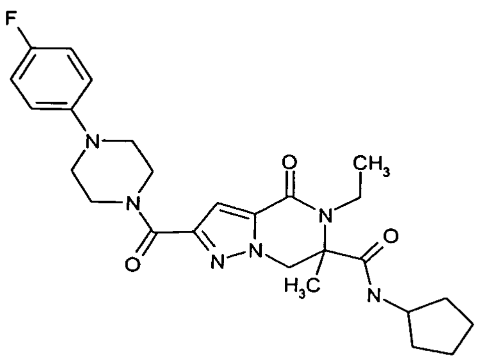 | 496,58971 | 497 |

(continued)

| | Table 1.05.(6) | | |
|---|---|---|---|
| № | Formula | Mol. Weight | *m*/*z* (M+1) |
| 473 | 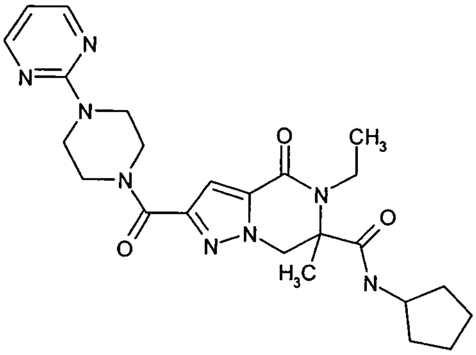 | 480,57444 | 481 |
| 474 | 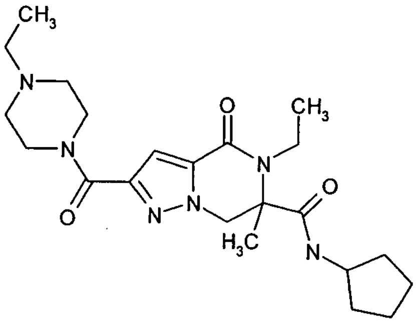 | 430,55468 | 431 |
| 475 | 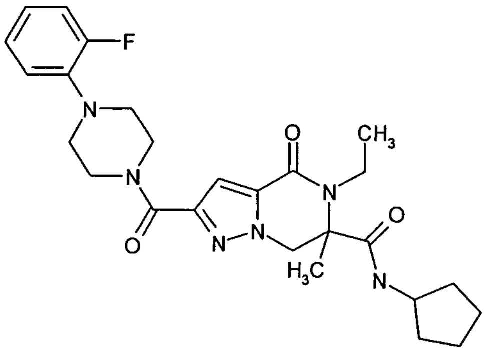 | 496,58971 | 497 |
| 476 | 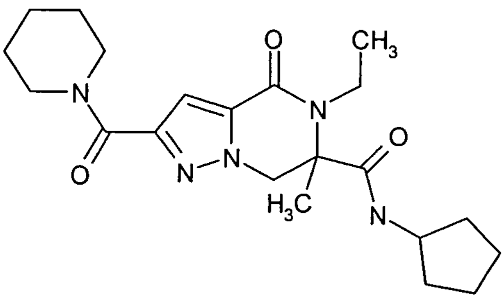 | 401,51292 | 402 |

(continued)

| | Table 1.05.(6) | | |
|---|---|---|---|
| № | Formula | Mol. Weight | *m*/*z* (M+1) |
| 477 | 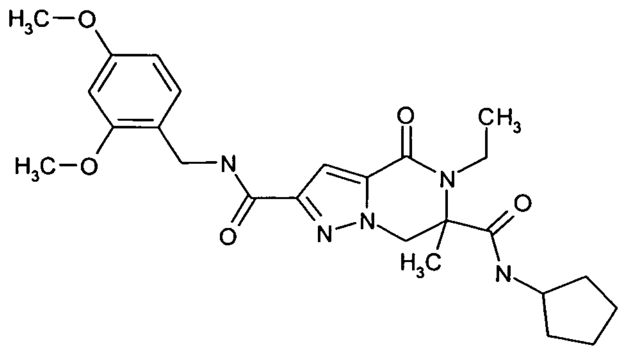 | 483,57226 | 484 |
| 478 | 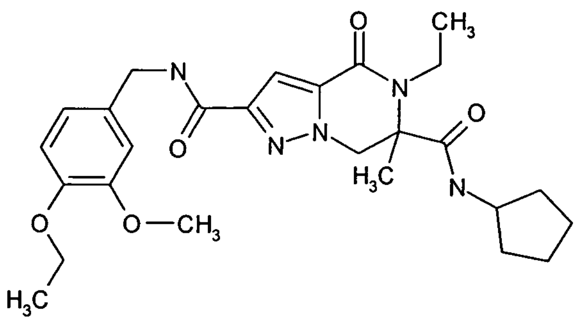 | 497,59935 | 498 |
| 479 | 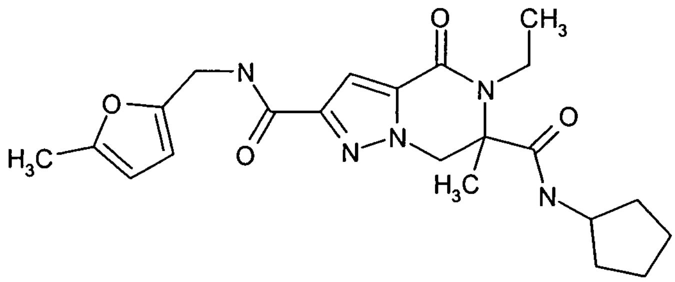 | 427,50753 | 428 |
| 480 | 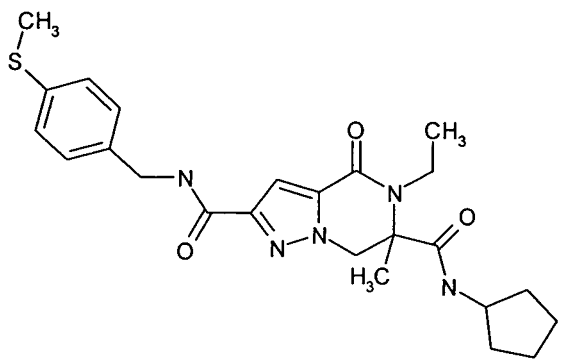 | 469,61037 | 470 |

(continued)

| | Table 1.05.(6) | | |
|---|---|---|---|
| № | Formula | Mol. Weight | m/z (M+1) |
| 481 | 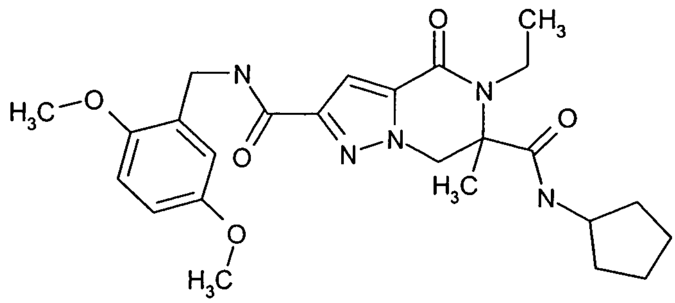 | 483,57226 | 484 |
| 482 | 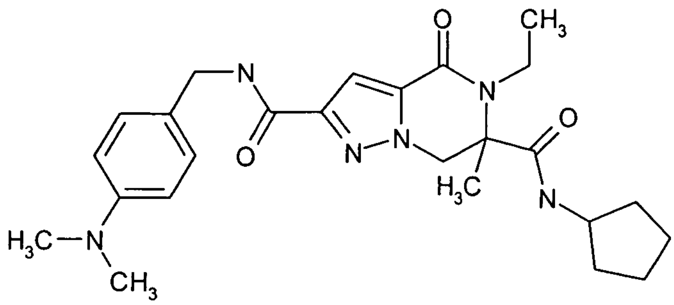 | 466,58813 | 467 |
| 483 | 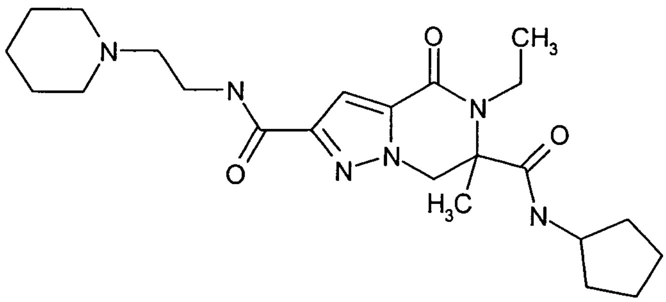 | 444,58177 | 445 |
| 484 | 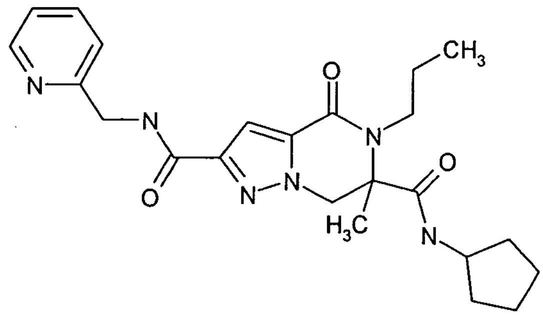 | 438,53395 | 439 |
| 485 | 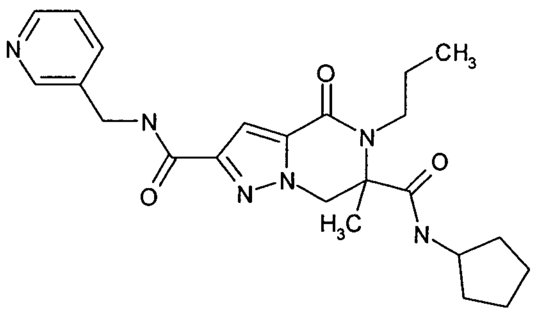 | 438,53395 | 439 |

(continued)

| | Table 1.05.(6) | | |
|---|---|---|---|
| Nº | Formula | Mol. Weight | *m/z* (M+1) |
| 486 | 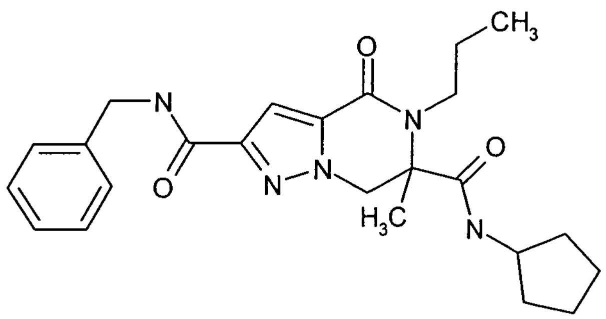 | 437,54637 | 438 |
| 487 | 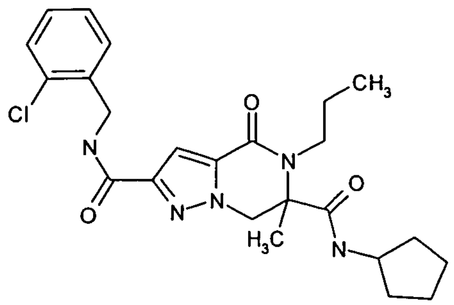 | 471,9914 | 472 |
| 488 | 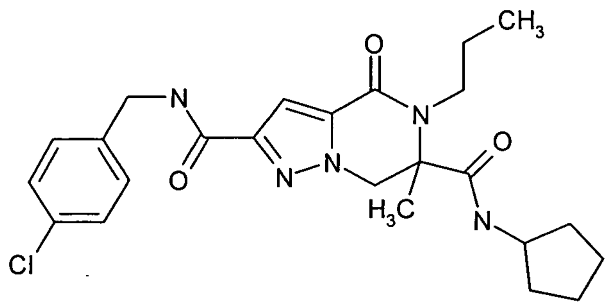 | 471,9914 | 472 |
| 489 | 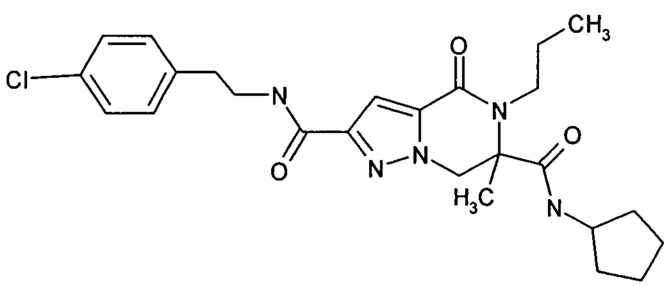 | 486,01849 | 487 |
| 490 | 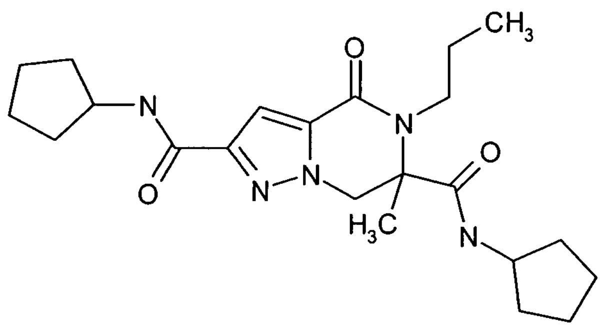 | 415,54001 | 416 |

(continued)

| | Table 1.05.(6) | | |
|---|---|---|---|
| № | Formula | Mol. Weight | *m*/*z* (M+1) |
| 491 | 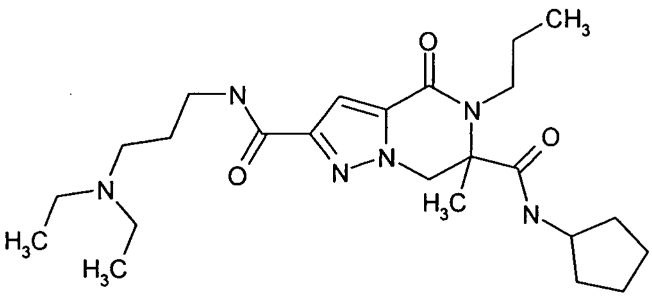 | 460,6248 | 461 |
| 492 | 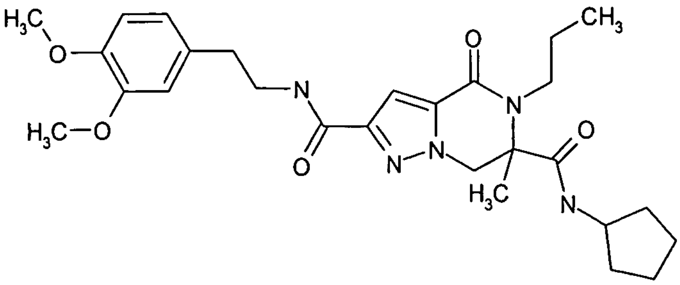 | 511,62644 | 512 |
| 493 | 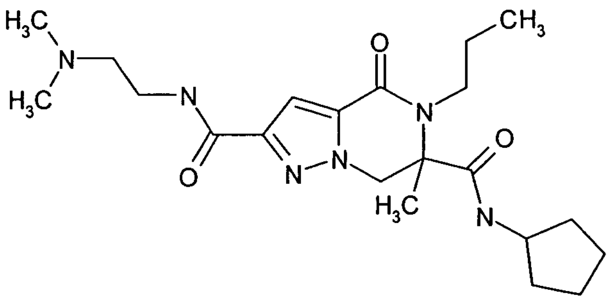 | 418,54353 | 419 |
| 494 | 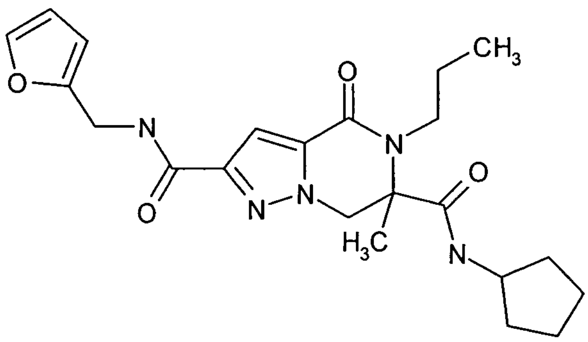 | 427,50753 | 428 |

(continued)

| | Table 1.05.(6) | | |
|---|---|---|---|
| № | Formula | Mol. Weight | m/z (M+1) |
| 495 | 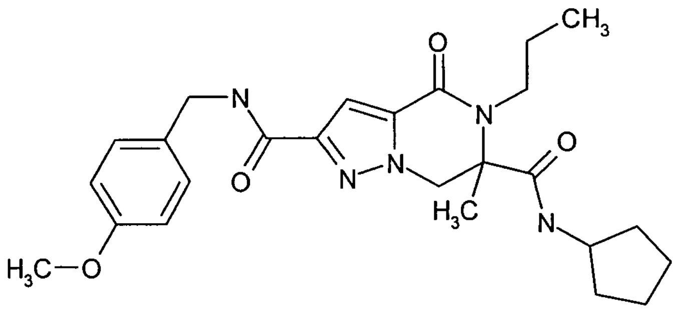 | 467,57286 | 468 |
| 496 | 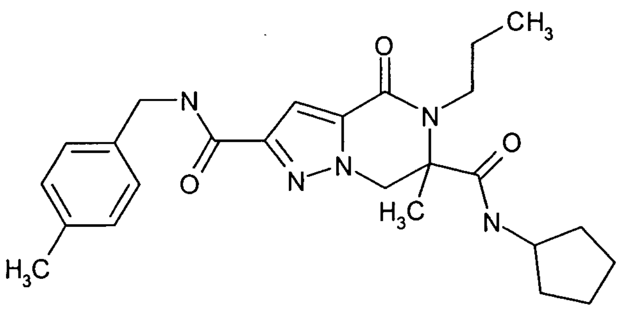 | 451,57346 | 452 |
| 497 | 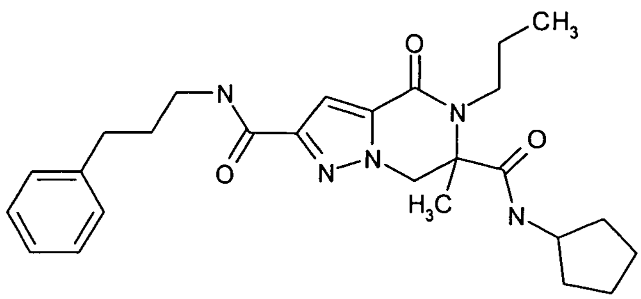 | 465,60055 | 466 |
| 498 | 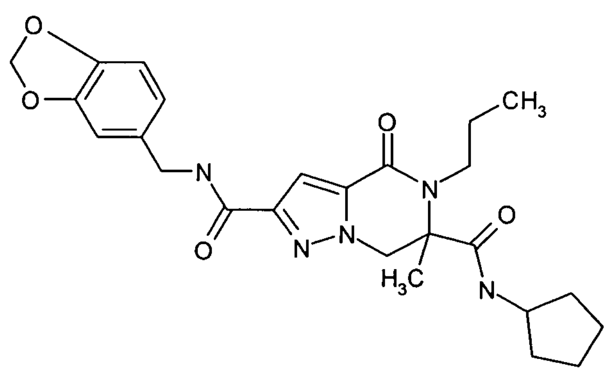 | 481,55632 | 482 |

(continued)

| | Table 1.05.(6) | | |
|---|---|---|---|
| № | Formula | Mol. Weight | m/z (M+1) |
| 499 | 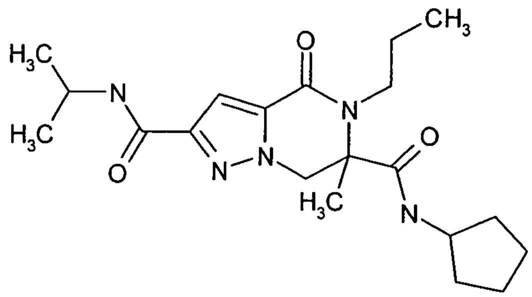 | 389,50177 | 390 |
| 500 | 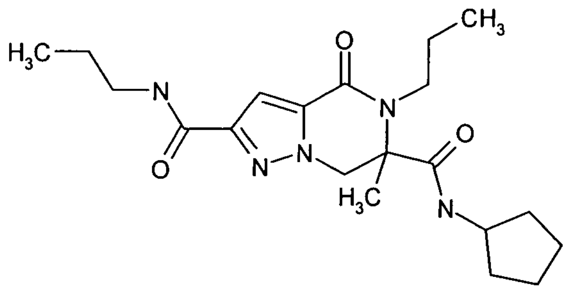 | 389,50177 | 390 |
| 501 | 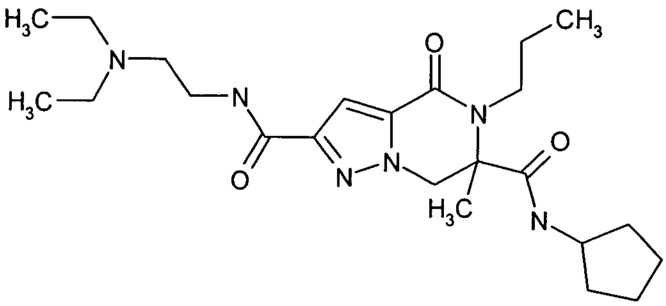 | 446,59771 | 447 |
| 502 | 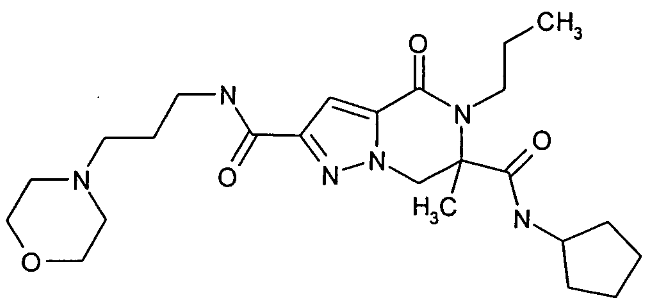 | 474,60826 | 475 |
| 503 | 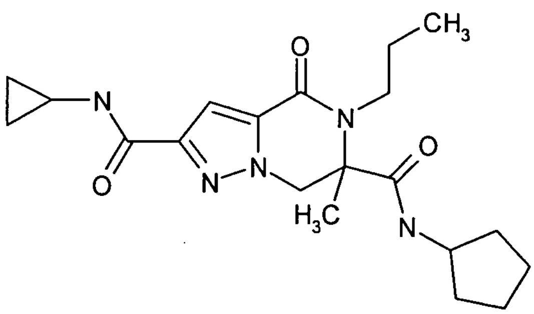 | 387,48583 | 388 |

(continued)

| | Table 1.05.(6) | | |
|---|---|---|---|
| № | Formula | Mol. Weight | *m*/*z* (M+1) |
| 504 | 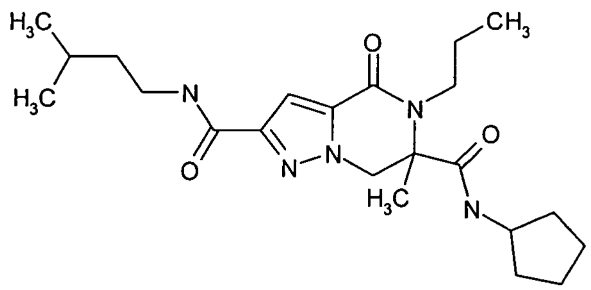 | 417,55595 | 418 |
| 505 | 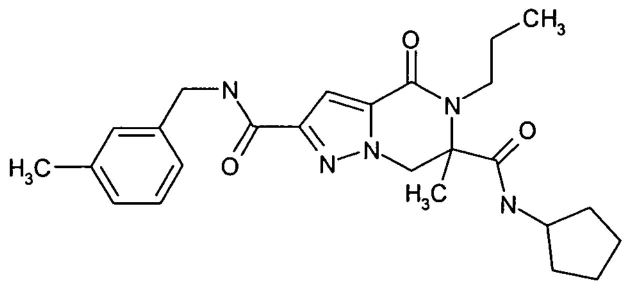 | 451,57346 | 452 |
| 506 | 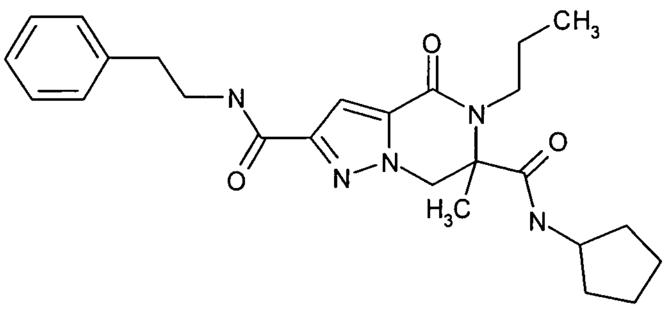 | 451,57346 | 452 |
| 507 | 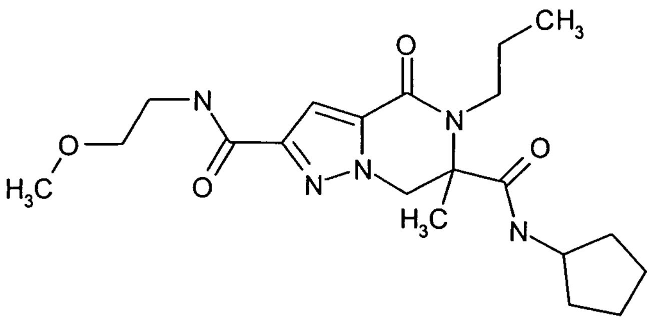 | 405,50117 | 406 |
| 508 | 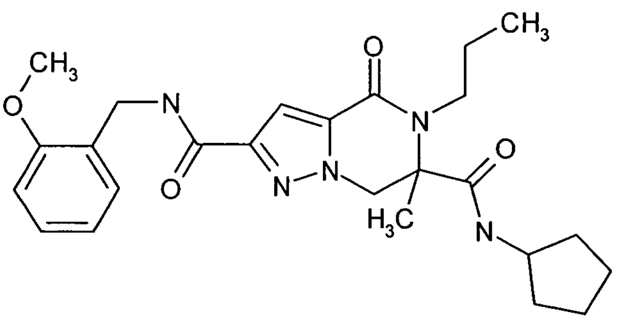 | 467,57286 | 468 |

(continued)

| | Table 1.05.(6) | | |
|---|---|---|---|
| № | Formula | Mol. Weight | *m/z* (M+1) |
| 509 | 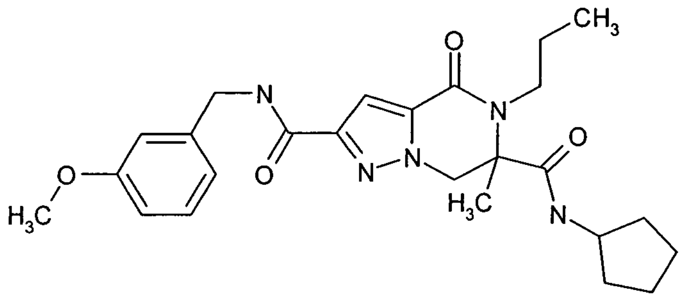 | 467,57286 | 468 |
| 510 | 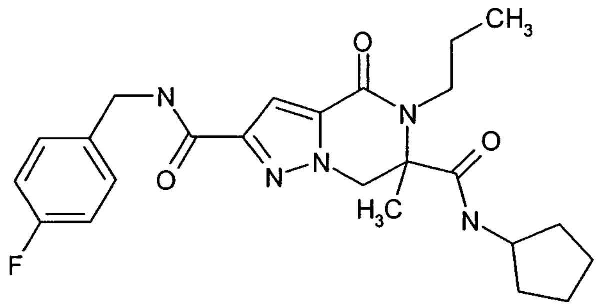 | 455,5368 | 456 |
| 511 | 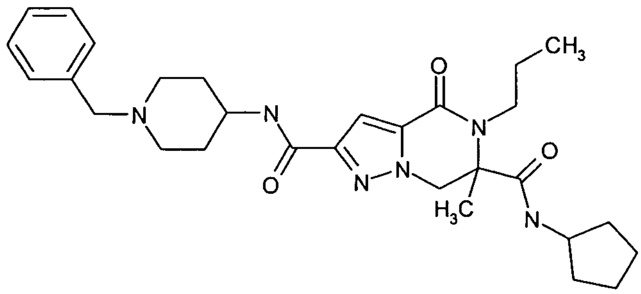 | 520,68055 | 521 |
| 512 | 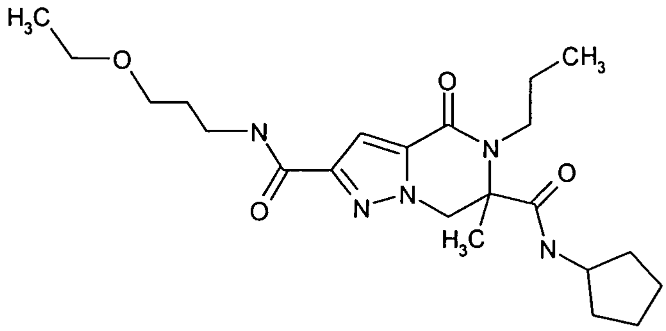 | 433,55535 | 434 |

(continued)

| | Table 1.05.(6) | | |
|---|---|---|---|
| № | Formula | Mol. Weight | m/z (M+1) |
| 513 | 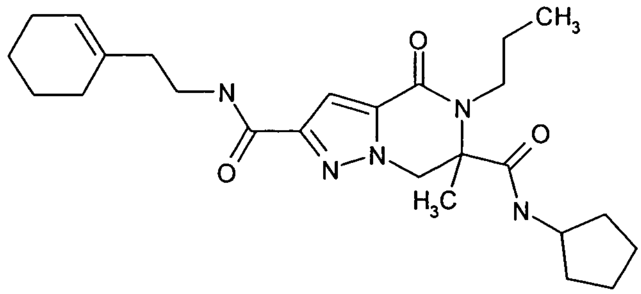 | 455,60534 | 456 |
| 514 | 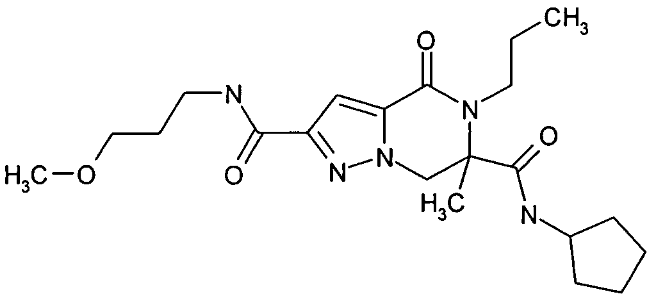 | 419,52826 | 420 |
| 515 | 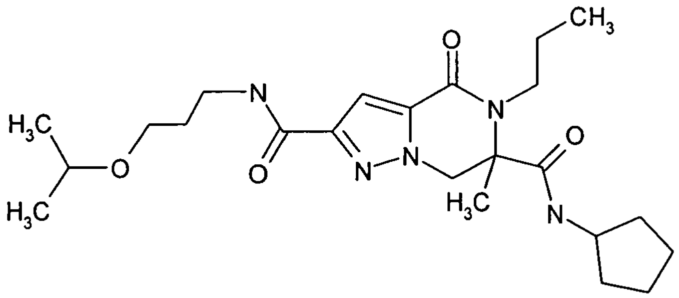 | 447,58244 | 448 |
| 516 | 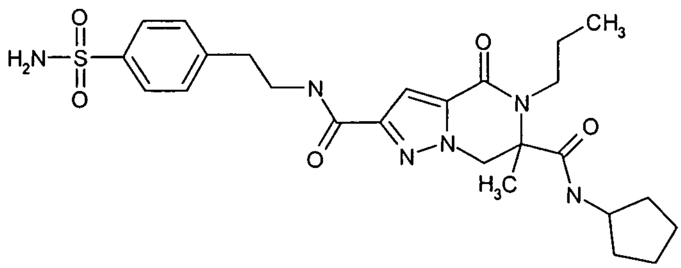 | 530,65093 | 531 |
| 517 | 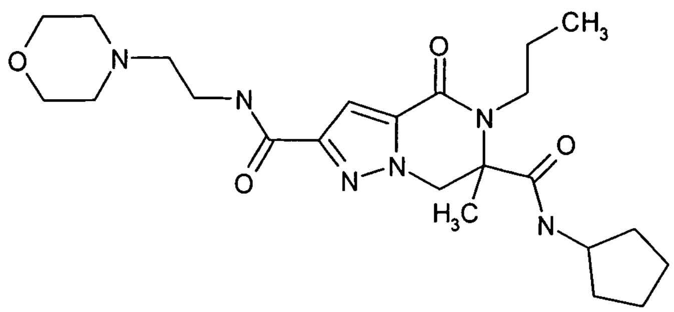 | 460,58117 | 461 |

(continued)

| | Table 1.05.(6) | | |
|---|---|---|---|
| № | Formula | Mol. Weight | *m/z* (M+1) |
| 518 | 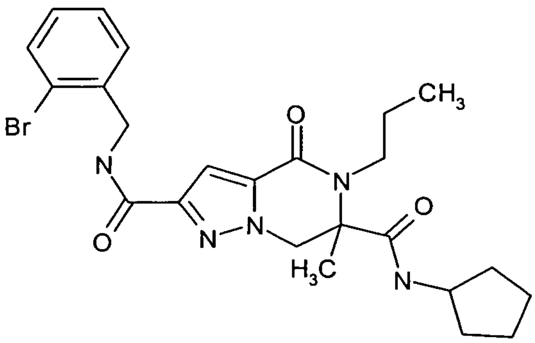 | 516,4424 | 517 |
| 519 | 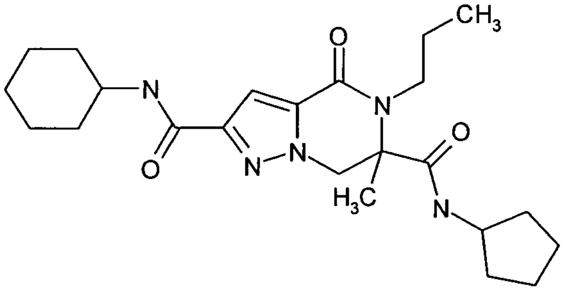 | 429,5671 | 430 |
| 520 | 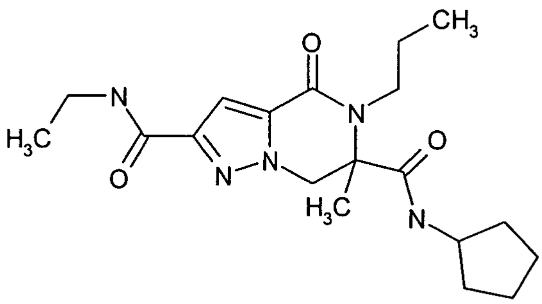 | 375,47468 | 376 |
| 521 | 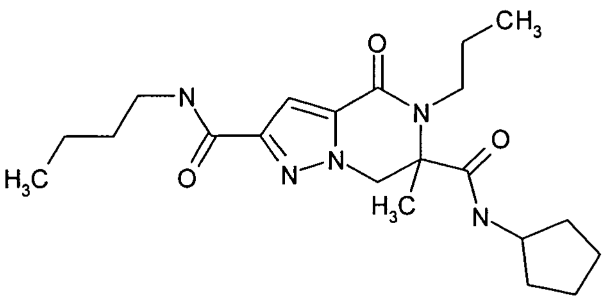 | 403,52886 | 404 |
| 522 | 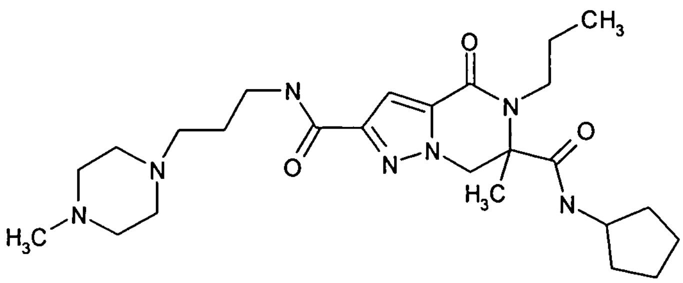 | 487,65062 | 488 |

(continued)

| | Table 1.05.(6) | | |
|---|---|---|---|
| № | Formula | Mol. Weight | m/z (M+1) |
| 523 | 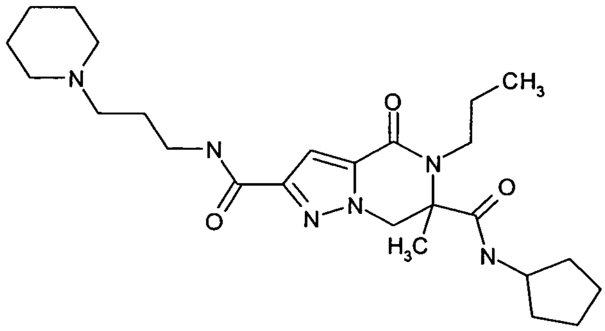 | 472,63595 | 473 |
| 524 | 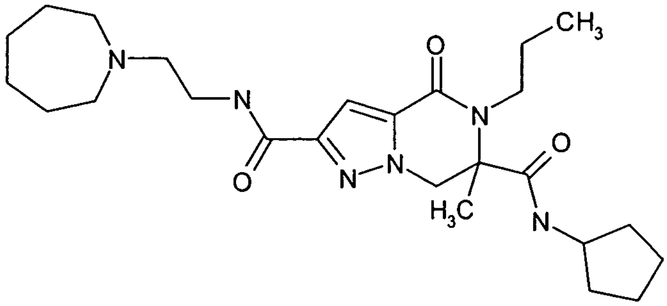 | 472,63595 | 473 |
| 525 | 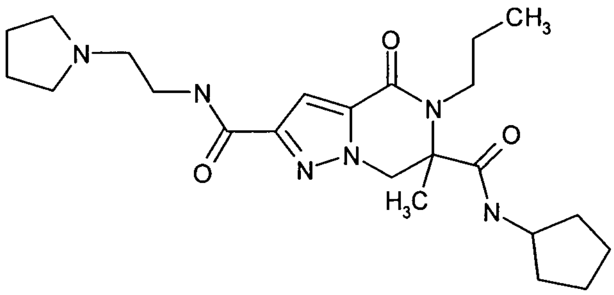 | 444,58177 | 445 |
| 526 | 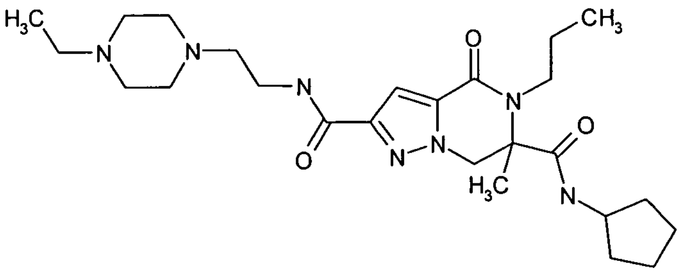 | 487,65062 | 488 |

(continued)

| | Table 1.05.(6) | | |
|---|---|---|---|
| № | Formula | Mol. Weight | m/z (M+1) |
| 527 | 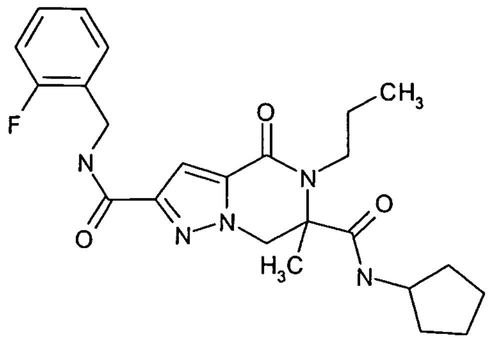 | 455,5368 | 456 |
| 528 | 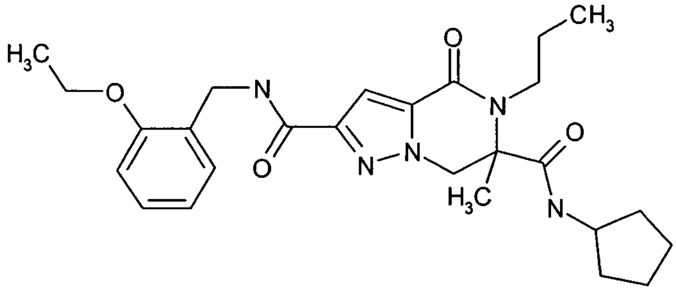 | 481,59995 | 482 |
| 529 | 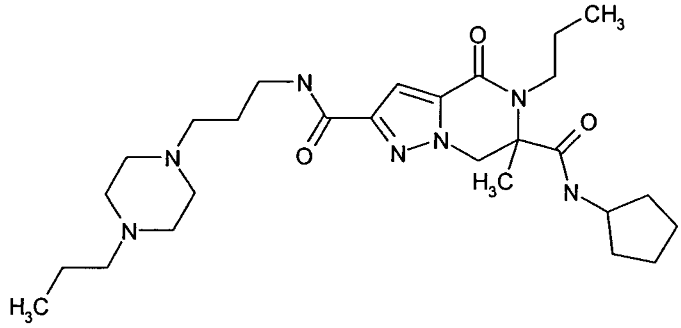 | 515,7048 | 516 |
| 530 | 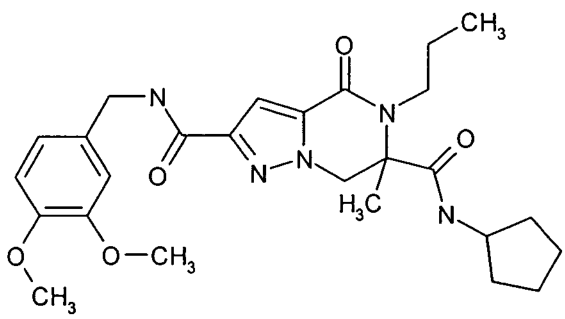 | 497,59935 | 498 |

(continued)

| | Table 1.05.(6) | | |
|---|---|---|---|
| № | Formula | Mol. Weight | *m/z* (M+1) |
| 531 | 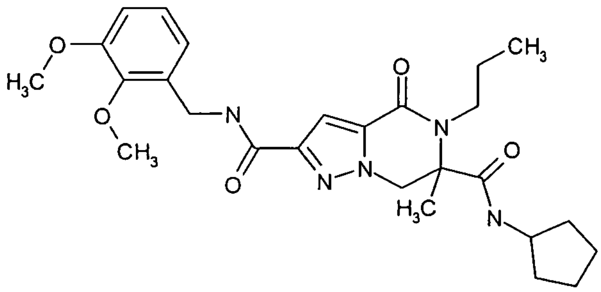 | 497,59935 | 498 |
| 532 | 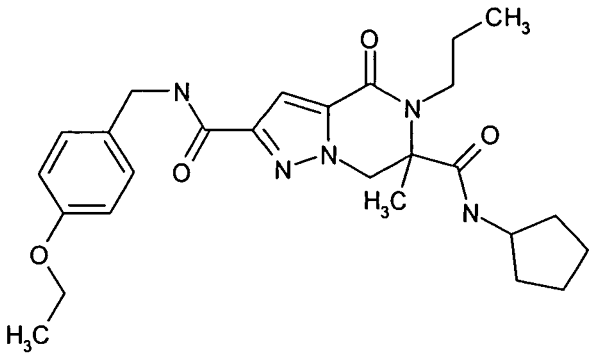 | 481,59995 | 482 |
| 533 | 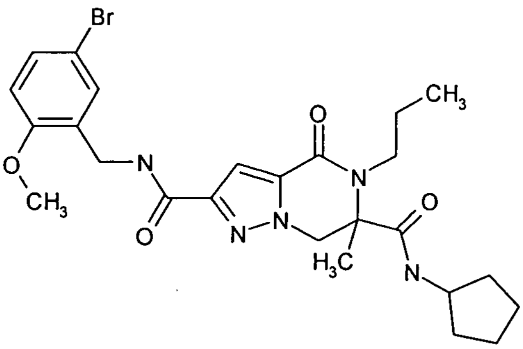 | 546,46889 | 547 |
| 534 | 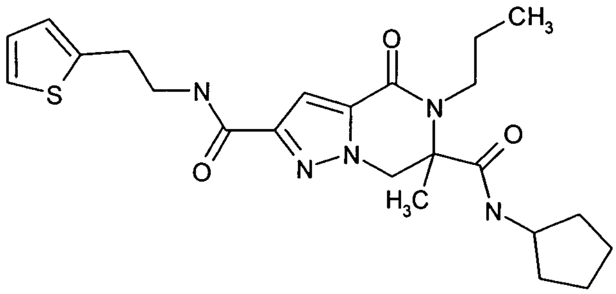 | 457,59922 | 458 |

(continued)

| | Table 1.05.(6) | | |
|---|---|---|---|
| № | Formula | Mol. Weight | m/z (M+1) |
| 535 | 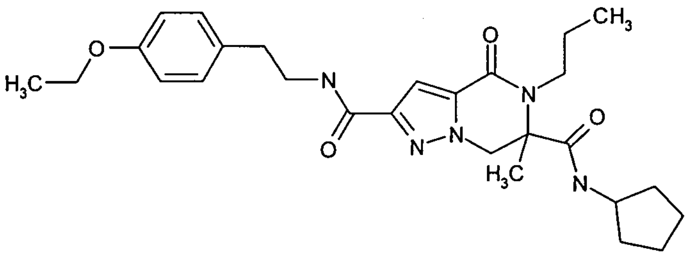 | 495,62704 | 496 |
| 536 | 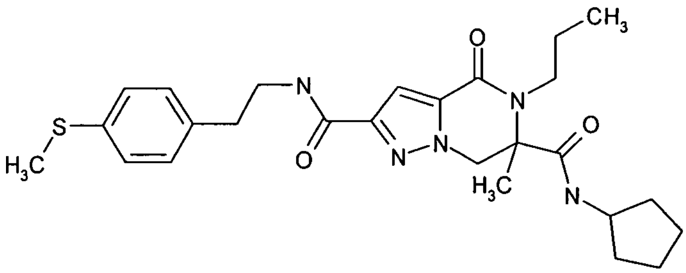 | 497,66455 | 498 |
| 537 | 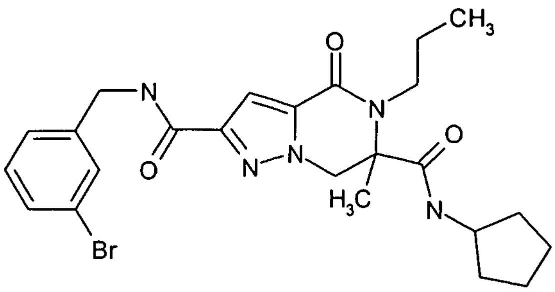 | 516,4424 | 517 |
| 538 | 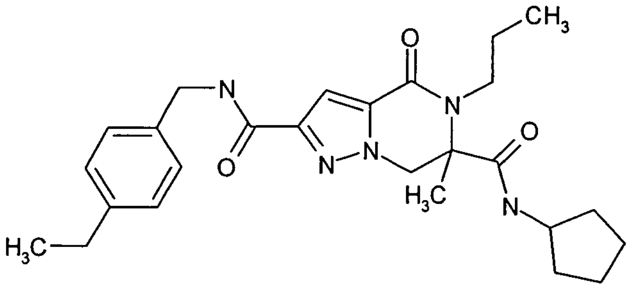 | 465,60055 | 466 |
| 539 | 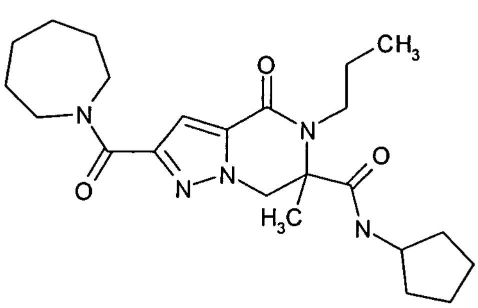 | 429,5671 | 430 |

(continued)

| | Table 1.05.(6) | | |
|---|---|---|---|
| № | Formula | Mol. Weight | m/z (M+1) |
| 540 | 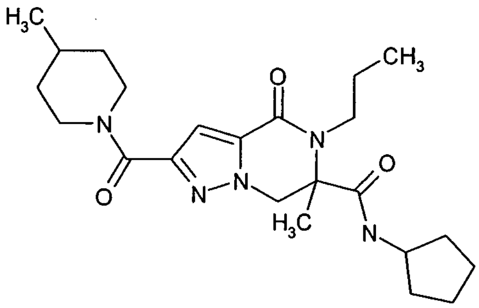 | 429,5671 | 430 |
| 541 | 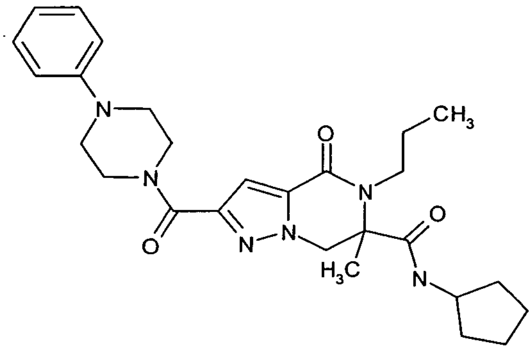 | 492,62637 | 493 |
| 542 | 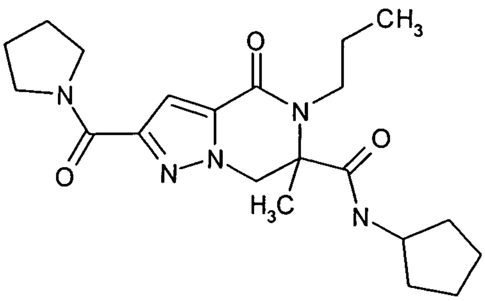 | 401,51292 | 402 |
| 543 | 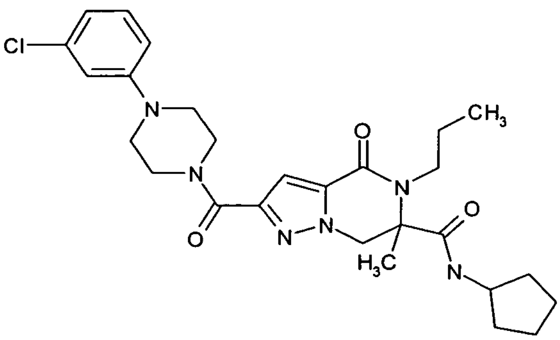 | 527,0714 | 528 |

(continued)

| | Table 1.05.(6) | | |
|---|---|---|---|
| № | Formula | Mol. Weight | *m*/*z* (M+1) |
| 544 | 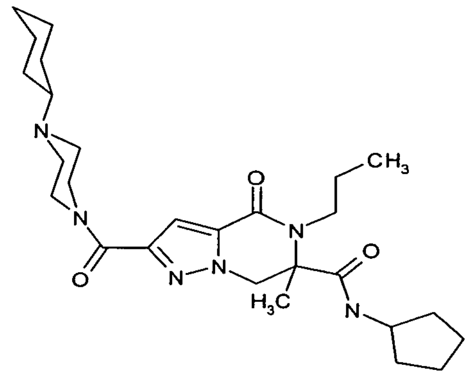 | 498,67419 | 499 |
| 545 | 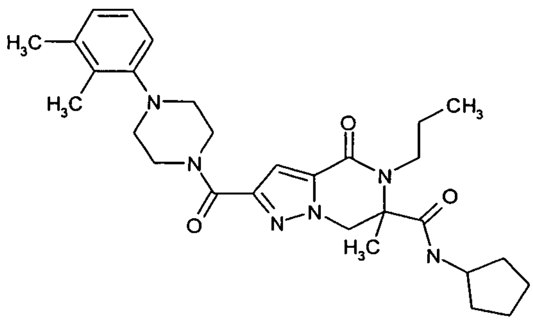 | 520,68055 | 521 |
| 546 | 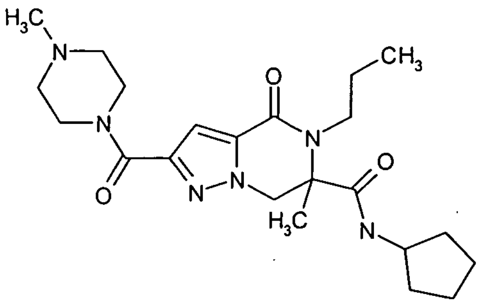 | 430,55468 | 431 |
| 547 | 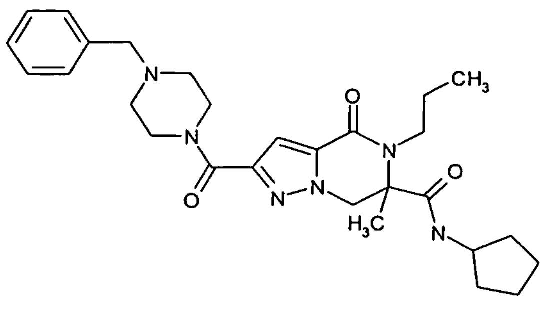 | 506,65346 | 507 |

(continued)

| | Table 1.05.(6) | | |
|---|---|---|---|
| № | Formula | Mol. Weight | *m*/*z* (M+1) |
| 548 | 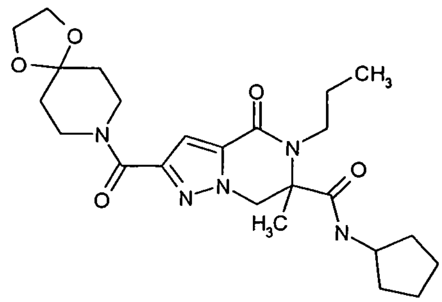 | 473,57705 | 474 |
| 549 | 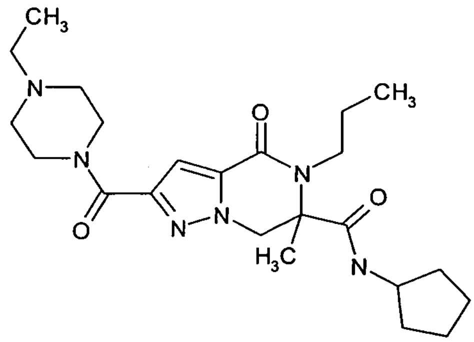 | 444,58177 | 445 |
| 550 | 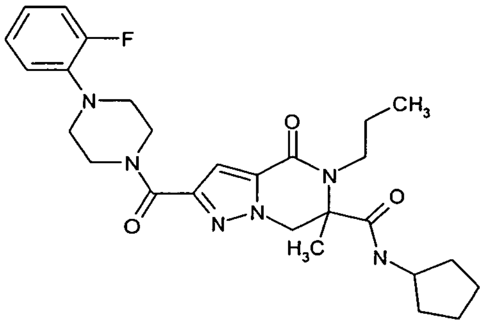 | 510,6168 | 511 |
| 551 | 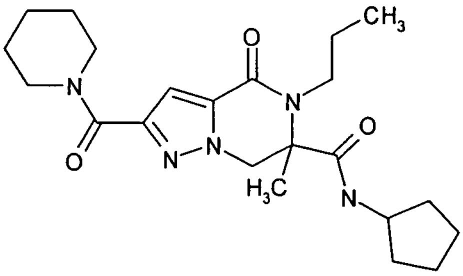 | 415,54001 | 416 |

(continued)

| | Table 1.05.(6) | | |
|---|---|---|---|
| № | Formula | Mol. Weight | *m*/*z* (M+1) |
| 552 | 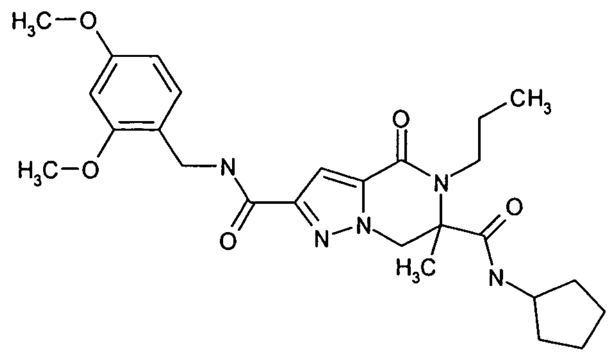 | 497,59935 | 498 |
| 553 | 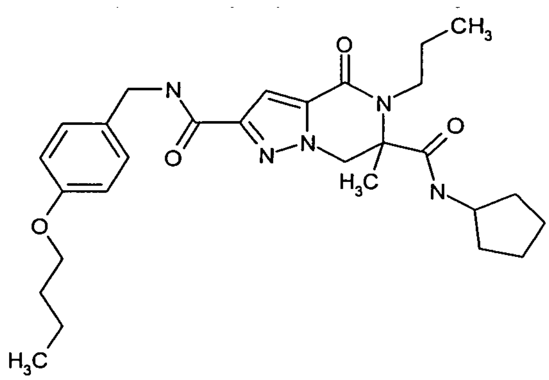 | 509,65413 | 510 |
| 554 | 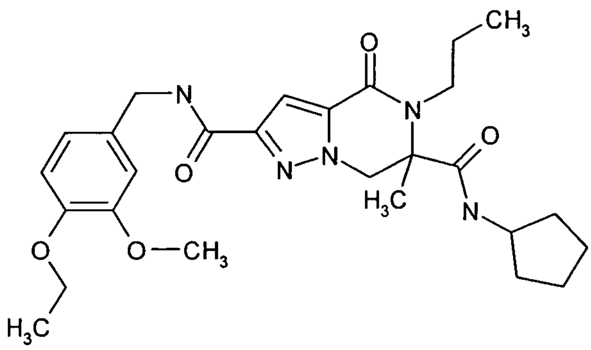 | 511,62644 | 512 |
| 555 | 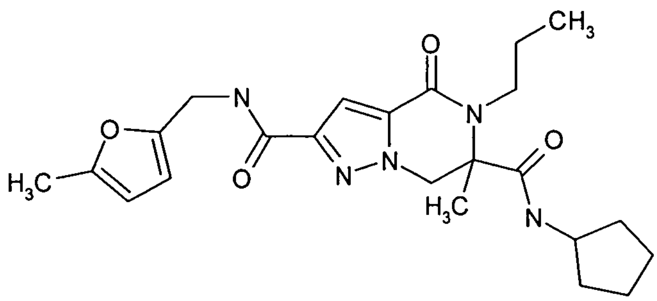 | 441,53462 | 442 |

(continued)

| | Table 1.05.(6) | | |
|---|---|---|---|
| № | Formula | Mol. Weight | *m/z* (M+1) |
| 556 | 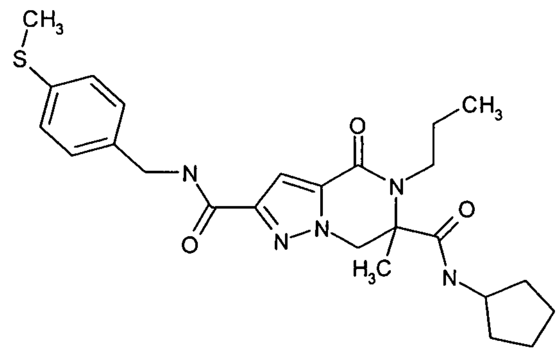 | 483,63746 | 484 |
| 557 | 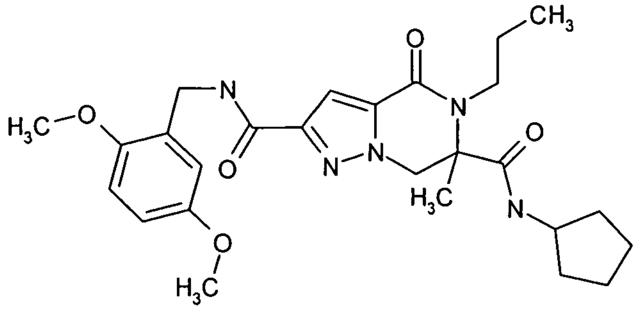 | 497,59935 | 498 |
| 558 | 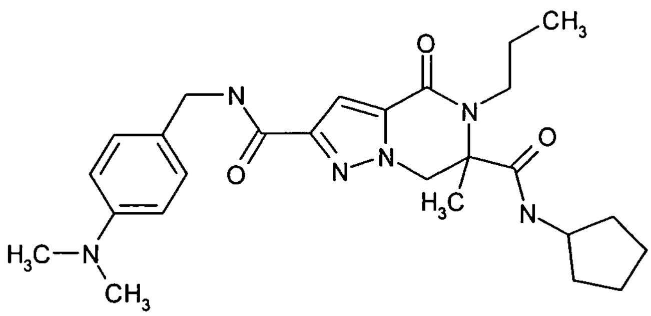 | 480,61522 | 481 |
| 559 | 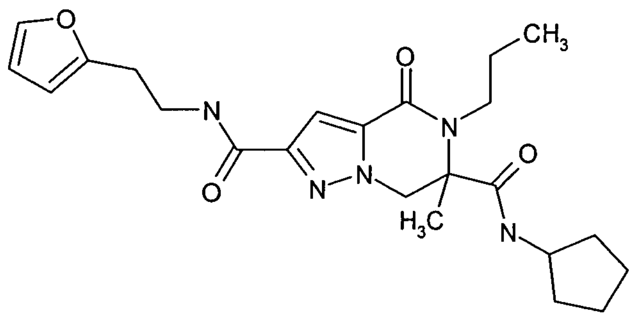 | 441,53462 | 442 |

(continued)

| | Table 1.05.(6) | | |
|---|---|---|---|
| № | Formula | Mol. Weight | *m/z* (M+1) |
| 560 | 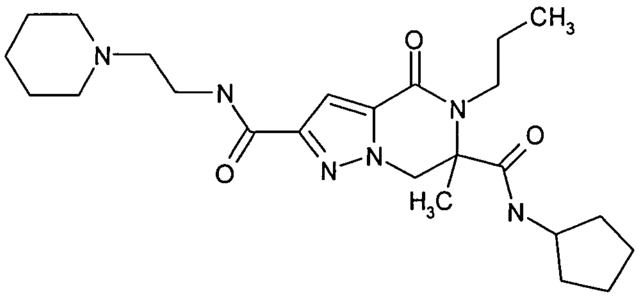 | 458,60886 | 459 |

**Table 1.06.**

| № | Formula | Mol.w. | HRMS m/z | Spectral data |
|---|---|---|---|---|
| 1 | 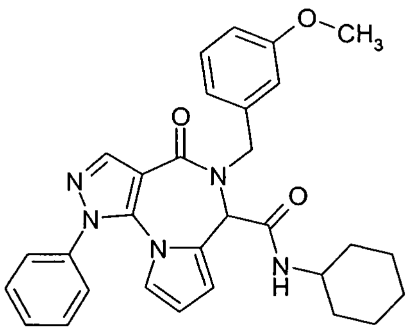 | 510.25 | 510.2493 | $^1$HNMR (400MHz, DMSO+$CCl_4$): δ 0.75-1.7 (m, 10 H, cyclohexane), 3.26-3.3 (m, 1 H, CH), 3.73 (s, 3 H, $CH_3$), 4.67 (d, *J* = 2.3 Hz, 1 H, $CH_2$), 4.8 (d, *J*= 2.3 Hz, 1 H, $CH_2$), 5.35 (s, 1 H, CH), 6.0-6.1 (d, *J* = 4.9 Hz, 2 H, ArH), 6.15-6.22 (s, 1 H, ArH), 6.8 (d, *J* = 4.0 Hz, 1 H, NH), 7.0-7.1 (d+d, *J* = 7.3 Hz, 2 H, ArH), 7.2-7.29 (t, *J* = 7.3 Hz, 1 H, ArH), 7.38-7.51 (m, 5 H, ArH), 7.82 (s, 1 H, ArH); 510.2493 ($M^+$). |
| 2 | 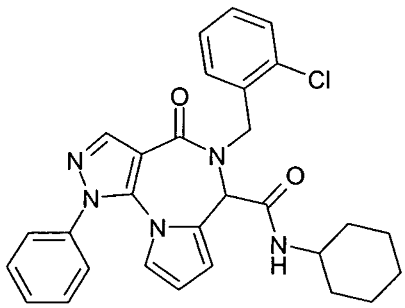 | 514.2004 | 514.2005 | $^1$HNMR (400MHz, DMSO+$CCl_4$): δ 1.0-1.75 (m, 10 H, cyclohexane), 3,3-3.48 (m, 1 H, CH), 4.5-4.6 (d, *J* = 2.0 Hz, 1 H, $CH_2$), 5.16 (s, 1 H, CH), 5.3-5.44 (d, *J* = 2.0 Hz, 1 H, $CH_2$), 5.75-5.8 (t, *J*= 8.4 Hz, 1 H, ArH), 6.0 (d, *J*= 7.9, 1 H, ArH), 6.22 (d, *J*= 7.9 Hz, 1 H, ArH), 7.0-7.5 (m, 10 H, ArH, NH), 7.86 (s, 1 H, ArH) |
| 3 | 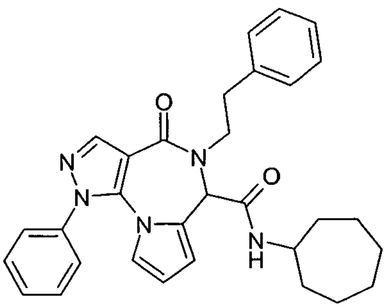 | 508.2707 | 508.2703 | $^1$HNMR (400MHz, DMSO+$CCl_4$): δ 1.1-1.63 (m, 12 H, cyclooctane), 2.7-2.84 (m, 1 H, $CH_2$), 2.9-3.08 (m, 1 H, $CH_2$), 3.5-3.69 (m, 2 H, $CH_2$), 4.0-4.2 (m, 1 H, CH), 5.5 (s, 1 H, CH), 6.07-6.1 (t, *J* = 8.2 Hz, 1 H, ArH), 6.21 (d, *J* = 7.7 Hz, 1 H, ArH), 6.27 (d, *J* = 7.7 Hz, 1 H, ArH), 7.0-7.08 (d, *J* = 4.2 Hz, 1 H, NH), 7.1-7.22 (m, 5 H, ArH), 7.37-7.5 (m, 5 H, ArH), 7.8 (s, 1 H, ArH) |

(continued)

| № | Formula | Mol.w. | HRMS m/z | Spectral data |
|---|---|---|---|---|
| 4 | 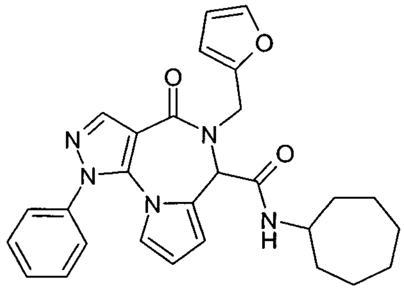 | 484.2343 | 484.2341 | $^1$HNMR (400MHz, DMSO+$CCl_4$): δ 1.09-1.64 (m, 12 H, cyclooctane), 3.46-3.6 (m, 1 H, CH), 4.7 (d, *J* = 2.6 Hz, 1 H, $CH_2$), 4.84 (d, *J*= 2.6 Hz, 1 H, $CH_2$), 5.25 (s, 1 H, CH), 6.0-6.1 (m, 2 H, ArH), 6.11-6.17 (t, *J*= 8.5 Hz, 1 H, ArH), 6.35-6.4 (d, *J* = 7.8 Hz, 1 H, ArH), 6.46 (d, *J* = 7.8 Hz, 1 H, ArH), 6.6 (d, *J* = 5.2 Hz, 1 H, NH), 7.31-7.57 (m, 6 H, ArH), 7.81 (s, 1 H, ArH) |
| 5 | 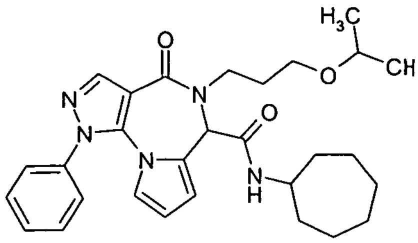 | 504.2969 | 504.2963 | $^1$HNMR (400MHz, DMSO+$CCl_4$): δ 1.1-1.2 (s, 6H, 2$CH_3$), 1.23-1.7 (m, 12 H, 6$CH_2$, cyclooctane), 1.66-1.7 (m, 1 H, $CH_2$), 1.7-1.87 (m, 1 H, $CH_2$), 3.3-3.55 (m, 4 H, 2$CH_2$), 3.59-3.7 (m, 1 H, CH), 3.85-4.1 (m, 1 H, CH), 5.3 (s, 1H, CH), 6.05-6.1 (t, *J* = 8.2 Hz, 1 H, ArH), 6.2 (d, *J* = 7.7 Hz, 1 H, ArH), 6.28 (d, *J*= 7.7 Hz, 1 H, ArH), 7.05-7.12 (br s, 1 H, NH), 7.35-7.57 (m, 5 H, ArH), 7.8 (s, 1 H, ArH) |
| 6 | 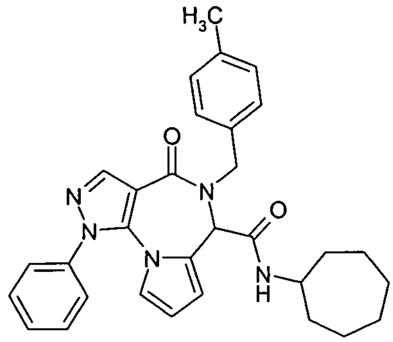 | 508.2707 | 508.2702 | $^1$HNMR (400MHz, DMSO+$CCl_4$): δ 0.8-1.55 (m, 12 H, cyclooctane), 2.21 (s, 3 H, $CH_3$), 3.35-3.46 (m, 1 H, CH), 4.54 (d, *J*= 2.7 Hz, 1 H, $CH_2$), 4.9 (d, *J* = 2.7 Hz, 1 H, $CH_2$), 5.3 (s, 1 H, CH), 6.05 (t, *J*= 8.2 Hz, 1 H, ArH), 6.1 (d, *J* = 7.7 Hz, 1H, ArH), 6.18 (d, *J* = 7.7 Hz, 1 H, ArH), 7.15 (d, *J* = 7.3 Hz, 2 H, ArH), 7.3-7.5 (m, 7 H, ArH), 7.82 (s, 1 H, ArH) |
| 7 | 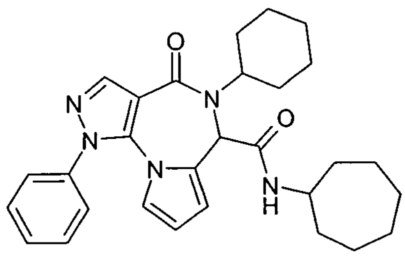 | 486.2863 | 486.2865 | $^1$HNMR (400MHz, DMSO+$CCl_4$): δ 1.09-2.1 (m, 22 H, cyclooctane, cyclohexane), 3.5-3.67 (m, 1 H, CH), 4.41-4.6 (m, 1 H, CH), 5.4 (s, 1 H, CH), 6.07 (t, *J* = 8.6 Hz, 1 H, ArH), 6.15 (d, *J*= 7.9 Hz, 1 H, ArH), 6.24 (d, *J* = 7.9 Hz, 1 H, ArH), 6.6-6.8 (d, *J* = 4.4 Hz, 1 H, NH), 7.3-7.5 (m, 5 H, ArH), 7.8 (s, 1 Hz H, ArH) |

(continued)

| № | Formula | Mol.w. | HRMS m/z | Spectral data |
|---|---|---|---|---|
| 8 | 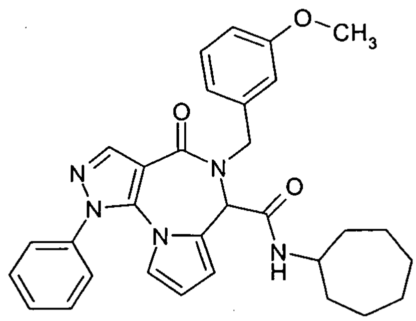 | 524.2656 | 524.2651 | $^{1}$HNMR (400MHz, DMSO+$CCl_4$): δ 0.8-1.53 (m, 12 H, cyclooctane), 3.38-3.5 (m, 1 H, CH), 3.75 (s, 3 H, $CH_3$), 4.51 (d, *J*= 2.8 Hz, 1 H, $CH_2$), 4.88 (d, *J* = 2.8 Hz, 1 H, $CH_2$), 5.32 (s, 1 H, CH), 6.0 (s, 1 H, ArH), 6.01 (t, *J* = 8.4 Hz, 1 H, ArH), 6.07 (d, *J* = 7.8 Hz, 1 H, ArH), 6.14 (d, *J* = 7.8 Hz, 1 H, ArH), 6.8 (d, *J* = 5.2 Hz, 1 H, NH), 7.0-7.1 (m, 2 H, ArH), 7.19-7.3 (m, 1 H, ArH), 7.36-7.5 (m, 5 H, ArH), 7.84 (s, 1 H, ArH) |
| 9 | 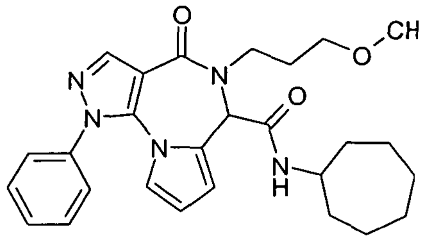 | 476.2656 | 476.2652 | $^{1}$HNMR (400MHz, DMSO+$CCl_4$): δ 1.1-1.64 (m, 12 H, cyclooctane), 1.66-2.0 (2m, 2 H, $CH_2$), 3.25 (s, 3 H, $CH_3$), 3.3-3.38 (m, 2 H, $CH_2$), 3.5-3.65 (m, 2 H, $CH_2$), 3.72-3.9 (m, 1 H, CH), 5.27 (s, 1 H, CH), 6.05 (t, *J* = 8.3 Hz, 1 H, ArH), 6.16 (d, *J* = 7.7 Hz, 1 H, ArH), 6.2 (d, *J*= 7.7 Hz, 1 H, ArH), 7.2 (d, *J*= 3.8 Hz, 1 H, NH), 7.35-7.5 (m, 5 H, ArH), 7.79 (s, 1 H, ArH) |
| 10 | 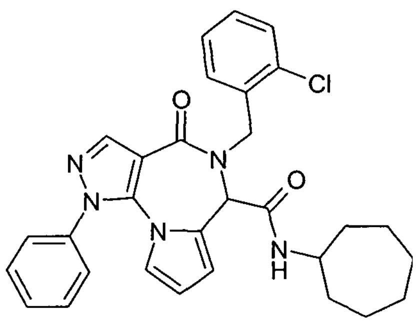 | 528.2161 | 528.2154 | $^{1}$HNMR (400MHz, DMSO+$CCl_4$): δ 1.2-1.6 (m, 12 H, cyclooctane), 3.55-3.67 (m, 1 H, CH), 4.5-4.59 (d, *J*= 2.0 Hz, 1 H, $CH_2$), 5.1 (s, 1 H, CH), 5.26-5.4 (d, *J*= 2.0 Hz, 1 H, $CH_2$), 5.75-5.84 (t, *J*= 8.4 Hz, 1 H, ArH), 5.94-6.07 (d, *J* = 7.8 Hz, 1 H, ArH), 6.13-6.24 (d, *J*= 7.8 Hz, 1 H, ArH), 6.95-7.0 (m, 1 H, NH), 7.15-7.8 (m, 4 H, ArH), 7.82-7.51 (m, 5 H, ArH), 7.8 (s, 1 H, ArH) |
| 11 | 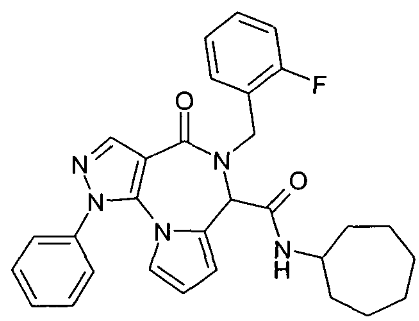 | 512.2456 | 512.2452 | $^{1}$HNMR (400MHz, DMSO+$CCl_4$): δ 1.0-1.63 (m, 12 H, cyclooctane), 3.50-3.62 (m, 1 H, CH), 4.68-4.76 (*d, J* = 2.2 Hz, 1 H, $CH_2$), 4.82-4.9 (d, *J* = 2.2 Hz, 1H, $CH_2$), 5.4 (s, 1 H, CH), 6.0-6.15 (m, 2 H, ArH), 6.17 (d, *J*= 8.2 Hz, 1 H, ArH), 6.27-6.45 (m, 1 H, NH), 6.97-7.05 (t(F), *J* = 9.0 Hz, 1 H, ArH), 7.2-7.4 (dt(F), *J* = 9.1 Hz, 2 H, ArH), 7.41-7.5 (m, 6 H, ArH), 7.8 (s, 1 H, ArH) |

(continued)

| № | Formula | Mol.w. | HRMS m/z | Spectral data |
|---|---|---|---|---|
| 12 | 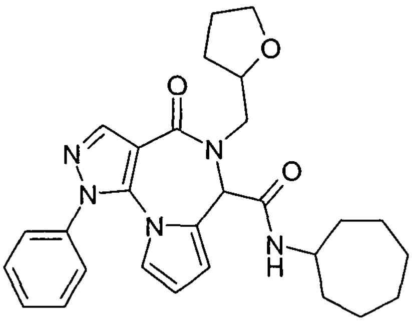 | 488.2656 | 488.265 | $^1$HNMR (400MHz, DMSO+$CCl_4$): δ 1.0-2.17 (m, 16 H, cyclooctane, tetrahydrofurane), 3.0-3.12 (m, 1 H, CH), 3.55-4.17 (m, 4 H, $CH_2$), 4.3-4.4 (m, 1 H, CH), 4.5-4.6 (t, *J* = 2.6 Hz, 1 H, CH), 5.3, 5.5 (s, s, 1 H, $CH_3$), 6.05 (t, *J* = 8.5 Hz, 1 H, ArH), 6.15 (d, *J* = 7.9 Hz, 1 H, ArH), 6.16 (d, *J* = 7.9 Hz, 1 H, ArH), 7.3-7.5 (m, 5 H, ArH), 7.78 (s, 1 H, ArH), 7.9 (d, *J* = 6.3 Hz, 1 H, NH) |

| | **Table 1.07** | | |
|---|---|---|---|
| **№** | **Formula** | **Mol. W.** | **LC MS, *m/z* (M+1)** |
| 1 | 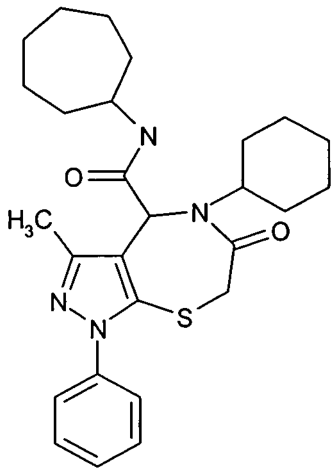 | 480,67757 | 481 |
| 2 | 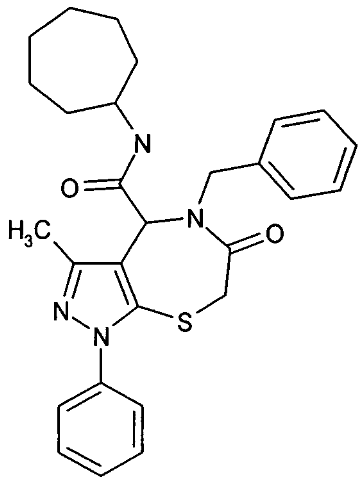 | 488,65684 | 489 |

**Table 1.08.**

| № | Formula | Mol. w. | Spectral data |
|---|---|---|---|
| 1 | 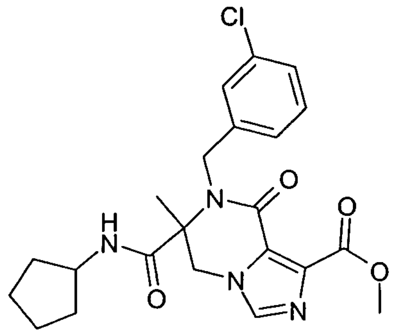 | 444,92 | $^1$HNMR (400MHz, DMSO+$CCl_4$, 25 °C): δ=1.13-1.81 (m, 8H; cyclopentane), 1.49 (s, 3 H; $CH_3$), 3.81 (s, 3 H; $CH_3$), 3.81-3.83 (m, 1 H; CH), 4.11 (d, *J* = 5.9 Hz, 1 H; $CH_2$), 4.22 (d, *J* = 5.8 Hz, 1 H; $CH_2$), 4.9 (d, *J* = 5.9 Hz, 1 H; $CH_2$), 5.27 (d, *J*= 5.8 Hz, 1 H; $CH_2$), 7.15 (m, 4 H; ArH), 7.41 (d, *J*=4.0 Hz, 1 H; NH), 7.68 (s, 1 H; ArH); $^{13}$C NMR (75 MHz, DMSO, 25 °C): δ=20.33, 23.46, 23.52, 31.66, 31.82, 45.58, 50.73, 51.13, 51.57, 64.80, 123.51, 125.30, 2×126.44, 129.98, 133.01, 134.18, 136.34, 142.27, 157.94, 162.80, 169.22; HRMS *m*/*z* 445.1641 [M$^+$]. |
| 2 | 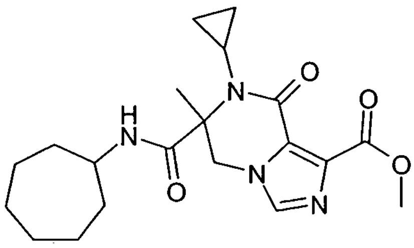 | 388,47 | $^1$HNMR (400MHz, DMSO+$CCl_4$, 25 °C): δ=0.7-0.88 (m, 4 H; cyclopropane), 1.2-1.71 (m, 12 H; cycloheptane), 1.67 (s, 3 H; $CH_3$), 2.46-2.55 (br s, 1 H; CH), 3.58-3.7 (m, 1 H; CH), 3.8 (s, 3 H; $CH_3$), 4.08 (d, *J*= 5.7 Hz, 1 H; $CH_2$), 4.73 (d, *J*= 5.7 Hz, 1 H; $CH_2$), 7.31 (br s, 1 H; NH), 7.63 (s, 1 H; ArH); $^{13}$C NMR (75 MHz, DMSO, 25 °C) δ=7.8, 8.89, 20.32, 23.62, 23.67, 25.54, 27.45, 27.55, 2x33.80, 50.62, 51.12, 51.50, 65.18, 124.65, 133.63, 135.84, 158.00, 162.81, 169.16; HRMS *m*/*z* 388.2184 [M$^+$]. |
| 3 | 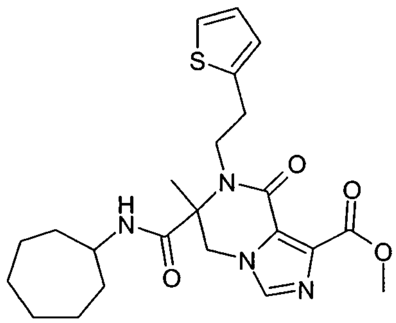 | 458,58 | $^1$HNMR (400MHz, DMSO+$CCl_4$, 25 °C): δ=1.23-1.62 (m, 12 H; cycloheptane), 1.52 (s, 3 H; $CH_3$), 3.11 (t, *J* = 4.4 Hz, $CH_2$), 3.3-3.45 (m, 1 H; CH), 3.6-3.72 (m, 1 H; CH), 3.81 (s, 3 H; $CH_3$), 4.06 (d, *J*= 6.0 Hz, 1 H; $CH_2$), 4.76 (d, *J*= 6.0 Hz, 1 H; $CH_2$), 6.85 (d, *J*= 7.2 Hz, 1 H; ArH), 6.9 (d+d, *J*= 6.8 Hz, 1 H; ArH), 7.15 (d, *J*= 7.2 Hz, 1 H; ArH), 7.51 (d, *J*= 4.0 Hz, 1 H; NH), 7.62 (s, 1 H; ArH); $^{13}$C NMR (75 MHz, DMSO, 25 °C) δ=19.72, 2x23.46, 27.13, 27.17, 28.82, 33.48, 33.51, 44.43, 50.40, 50.53, 51.09, 64.10, 123.01, 123.55, 124.83, 126.62, 134.04, 135.89, 140.80, 156.77, 162.51, 168.26; HRMS m/z 458.2061 [M$^+$]. |
| 4 | 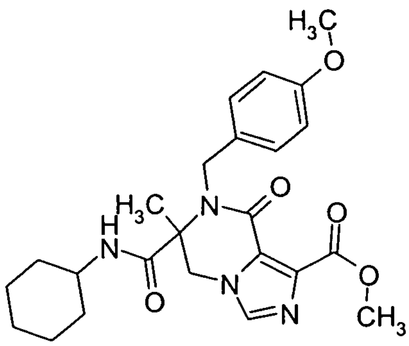 | 454,53 | $^1$HNMR (400MHz, DMSO+$CCl_4$, 25 °C): δ=1.0-1.68 (m, 10 H; cyclohexane), 1.5 (s, 3 H; $CH_3$), 3.39-3.42 (m, 1 H; CH), 3.7 (s, 3 H; $CH_3$), 3.8 (s, 3 H; $CH_3$), 4.05 (d, *J*= 6.2 Hz, 1 H; $CH_2$), 4.28 (d, *J*= 5.8 Hz, 1 H; $CH_2$), 4.87 (d, *J*= 6.2 Hz, 1 H; $CH_2$), 5.21 (d, *J* = 5.8 Hz, 1 H; $CH_2$), 6.8 (d, *J* = 7.8 Hz, 2 H; ArH), 712 (t, *J* = 5.2 Hz, 2 H; ArH), 7.22 (br s, 1 H; NH), 7.62 (s, 1 H; ArH); HRMS *m*/*z* 454.5260 [M$^+$]. |

(continued)

| № | Formula | Mol. w. | Spectral data |
|---|---|---|---|
| 5 | 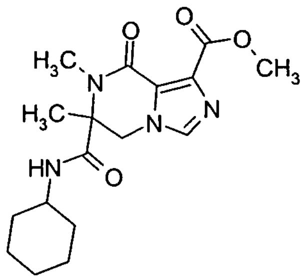 | 348,41 | $^1$HNMR (400MHz, DMSO+$CCl_4$, 25 °C): δ=1.1-1.83 (m, 10 H; cyclohexane), 1.53 (s, 3 H; $CH_3$), 3.05 (s, 1 H; $CH_3$), 3.46-3.51 (m, 1 H; CH), 3.80 (s, 3 H; $CH_3$), 4.11 (d, *J* = 5.8 Hz, 1 H; $CH_2$), 4.71 (d, *J* = 5.8 Hz, 1 H; $CH_2$), 7.52 (d, *J*= 4.2 Hz, 1 H; NH), 7.61 (s, 1 H; ArH); HRMS *m*/*z* 348.4015 [$M^+$]. |
| 6 | 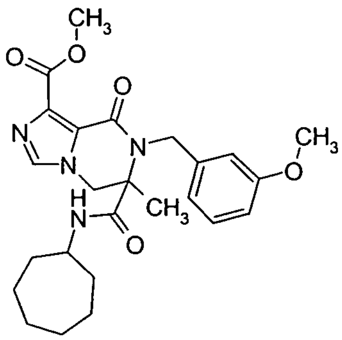 | 468,56 | $^1$HNMR (400MHz, DMSO+$CCl_4$, 25 °C): δ=1.12-1.7 (m, 12 H; cycloheptane), 1.56 (s, 3 H; $CH_3$), 3.5-3.61 (m, 1 H; CH), 3.7 (s, 3 H; $CH_3$), 3.8 (s, 3 H; $CH_3$), 4.07 (d, *J* = 6.0 Hz, 1 H; $CH_2$), 4.27 (d, *J* = 5.8 Hz, 1 H; $CH_2$), 4.83 (d, *J* = 6.0 Hz, 1 H; $CH_2$), 5.22 (d, *J* = 5.8 Hz, 1 H; $CH_2$), 6.7 (d, *J* = 4.4 Hz, 1 H; NH), 6.84 (s+d, 2 H; ArH), 7.24 (d+d, *J*= 7,6 Hz, 2 H; ArH), 7.62 (s, 1 H; ArH); HRMS *m*/*z* 468.5527 [$M^+$]. |
| 7 | 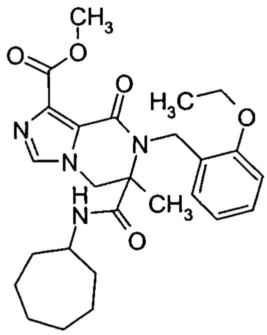 | 482,58 | $^1$HNMR (400MHz, DMSO+$CCl_4$, 25 °C): δ=1.2-1.8 (m, 12 H; cycloheptane), 1.54 (s, 6 H; 2$CH_3$), 3.5-3.61 (m, 1 H; CH), 3.71 (s, 3 H; $CH_3$), 4.1 (m, 3 H; CH, $CH_2$), 4.4 (d, *J* = 6.2 Hz, 1 H; $CH_2$), 4.90 (d, *J*= 6.0 Hz, 1 H; $CH_2$), 5.12 (d, *J* = 5.9 Hz, 1 H; $CH_2$), 6.8 (d+d, *J* = 7.8 Hz, 2 H; ArH), 7.13 (t, 2 H; ArH), 6.8 (d, *J* = 4.4 Hz, 1 H; NH), 7.24 7.62 (s, 1 H; ArH); HRMS *m*/*z* 482.5791 [$M^+$]. |
| 8 | 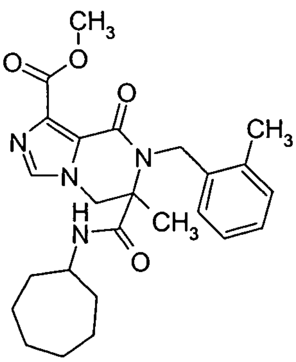 | 452,56 | $^1$HNMR (400MHz, DMSO+$CCl_4$, 25 °C): δ=1.27-1.66 (m, 12 H; cycloheptane), 1.50 (s, 3 H; $CH_3$), 2.32 (s, 3 H; $CH_3$), 3.58-3.67 (m, 1 H; CH), 3.8 (s, 3 H; $CH_3$), 4.1 (d, *J* = 6.2 Hz, 1 H; $CH_2$), 4.2 (d, *J* = 5.8 Hz, 1 H; $CH_2$), 4.88 (d, *J* = 6.2 Hz, 1 H; $CH_2$), 5.27 (d, *J* = 5.8 Hz, 1 H; $CH_2$), 7.0-7.21 (m, 4 H; ArH), 7.5 (d, *J* = 4.6 Hz, 1 H; NH), 7.7 (s, 1 H; ArH); HRMS *m*/*z* 452.5539 [$M^+$]. |

(continued)

| № | Formula | Mol. w. | Spectral data |
|---|---|---|---|
| 9 | 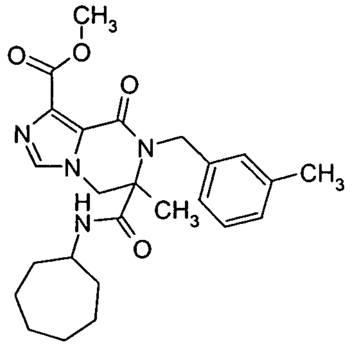 | 452.56 | $^1$HNMR (400MHz, DMSO+$CCl_4$, 25 °C): δ=1.2-1.61 (m, 12 H; cycloheptane), 1.51 (s, 3 H; $CH_3$), 2.31 (s, 3 H; $CH_3$), 3.51-3.65 (m, 1 H; CH), 3.8 (s, 3 H; $CH_3$), 4.06 (d, *J* = 6.2 Hz, 1 H; $CH_2$), 4.25 (d, *J*= 5.8 Hz, 1 H; $CH_2$), 4.84 (d, *J* = 6.2 Hz, 1 H; $CH_2$), 5.27 (d, *J*= 5.8 Hz, 1 H; $CH_2$), 7.0 (d, *J*= 4.0 Hz, 1 H; NH), 7.08-7.2 (m, 4 H; ArH), 7.67 (s, 1 H; ArH); HRMS *m*/*z* 452.5538 [$M^+$]. |
| 10 | 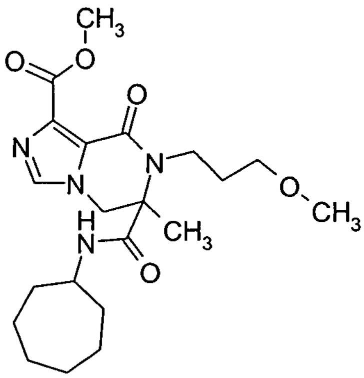 | 420,51 | $^1$HNMR (400MHz, DMSO+$CCl_4$, 25 °C): δ=1.2-1.71 (m, 12 H; cycloheptane), 1.52 (s, 3 H; $CH_3$), 1.8-2.0 (m, 2 H; $CH_2$), 3.22 (s, 3 H; $CH_3$), 3.3-3.5 (m, 3 H; $CH_2$), 3.5-3.67 (m, 2 H; CH, $CH_2$), 3.8 (s, 3 H; $CH_3$), 4.08 (d, *J*= 5.8 Hz, 1 H; $CH_2$), 4.78 (d, *J*= 5.8 Hz, 1 H; $CH_2$), 7.44 (d, *J*= 4.8 Hz, 1 H; NH), 7.6 (s, 1 H; ArH); HRMS *m*/*z* 420.5095 [$M^+$]. |
| 11 | 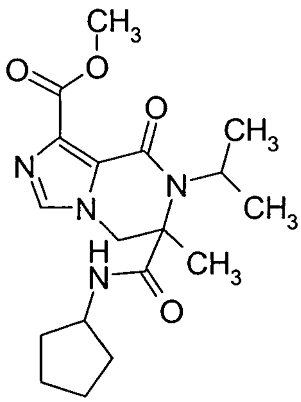 | 362,43 | $^1$HNMR (400MHz, DMSO+$CCl_4$, 25 °C): δ=1.12-2.0 (m, 8 H; cyclopentane), 1.4 (s, 3 H; $CH_3$), 1.5 (s, 3 H; $CH_3$), 1.6 (s, 3 H; $CH_3$), 3.12-3.3 (m, 1 H; CH), 3.8 (s, 3 H; $CH_3$), 3.9-4.02 (m, 1 H; CH), 4.2 (d, *J*= 5.8 Hz, 1 H; $CH_2$), 4.5 (d, *J* = 5.8 Hz, 1 H; $CH_2$), 7.58 (s, 4 H; ArH), 7.9 (d, *J*= 4.2 Hz, 1 H; NH); HRMS *m*/*z* 362.4287 [$M^+$]. |
| 12 | 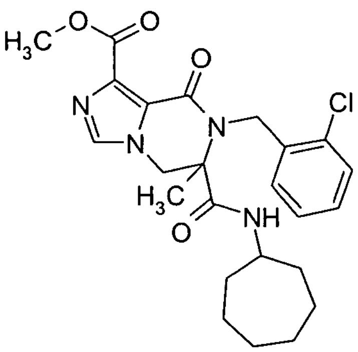 | 472,98 | $^1$HNMR (400MHz, DMSO+$CCl_4$, 25 °C): δ=1.24-1.63 (m, 12 H; cycloheptane), 1.5 (s, 3 H; $CH_3$), 3.61-3.7 (m, 1 H; CH), 3.81 (s, 3 H; $CH_3$), 4.2 (d, *J* = 6.2 Hz, 1 H; $CH_2$), 4.29 (d, *J* = 5.8 Hz, 1 H; $CH_2$), 4.94 (d, *J* = 6.2 Hz, 1 H; $CH_2$), 5.33 (d, *J*= 5.8 Hz, 1 H; $CH_2$), 7.12-7.3 (m, 4 H; ArH), 7.44-7.53 (br s, 1 H; NH), 7.7 (s, 1 H; ArH); HRMS *m*/*z* 472.9711 [$M^+$]. |

(continued)

| № | Formula | Mol. w. | Spectral data |
|---|---|---|---|
| 13 | 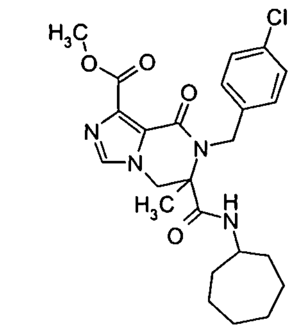 | 472,98 | $^1$HNMR (400MHz, DMSO+$CCl_4$, 25 °C): δ=1.21-1.75 (m, 12 H; cycloheptane), 1.51 (s, 3 H; $CH_3$), 3.53-3.7 (m, 1 H; CH), 3.85 (s, 3 H; $CH_3$), 4.1 (d, *J* = 6.0 Hz, 1 H; $CH_2$), 4.21 (d, *J*= 5.8 Hz, 1 H; $CH_2$), 4.88 (d, *J*= 6.0 Hz, 1 H; $CH_2$), 5.31 (d, *J*= 5.8 Hz, 1 H; $CH_2$), 7.21 (m, 4 H; ArH), 7.42 (d, *J*= 4.4 Hz, 1 H; NH), 7.69 (s, 1 H; ArH); HRMS *m*/*z* 472.9719 [$M^+$]. |
| 14 | 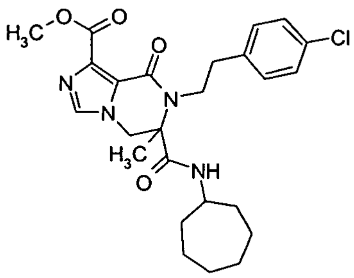 | 487,00 | $^1$HNMR (400MHz, DMSO+$CCl_4$, 25 °C): δ=1.27-1.77 (m, 12 H; cycloheptane), 1.51 (s, 3 H; $CH_3$), 2.8-3.0 (t, *J* = 3.8 Hz, 1 H; $CH_2$), 3.21-3.3 (q, *J*= 3.8 Hz, 1 H; $CH_2$), 3.57-3.72 (m, 1 H; CH), 3.8-3.85 (m, 1 H; CH), 3.85 (s, 3 H; $CH_3$), 4.06 (d, *J* = 5.7 Hz, 1 H; $CH_2$), 4.76 (d, *J* = 5.7 Hz, 1 H; $CH_2$), 7.22 (m, 4 H; ArH), 7.42 (br s, 1 H; NH), 7.67 (s, 1 H; ArH); HRMS m/z 486.9980 [$M^+$]. |
| 15 | 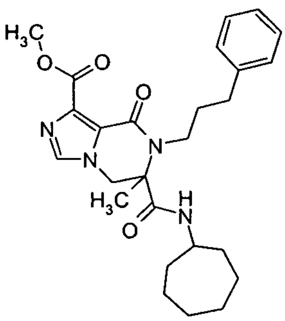 | 466,59 | $^1$HNMR (400MHz, DMSO+$CCl_4$, 25 °C): δ=1.25-1.76 (m, 12 H; cycloheptane), 1.53 (s, 3 H; $CH_3$), 1.8-2.0 (m+m, 2H, $CH_2$), 2.62 (t, *J*=4.0 Hz, 2 H, $CH_2$), 3.18-3.28 (m, 1 H; $CH_2$), 3.52-3.74 (m, 2 H; CH, $CH_2$), 3.81 (s, 3 H; $CH_3$), 4.02 (d, *J*= 5.6 Hz, 1 H; $CH_2$), 4.72 (d, *J*= 5.6 Hz, 1 H; $CH_2$), 7.1-7.27 (m, 5 H; ArH), 7.4 (d, *J* = 4.4 Hz, 1 H; NH), 7.63 (s, 1 H; ArH); HRMS m/z 466.5803 [$M^+$]. |
| 16 | 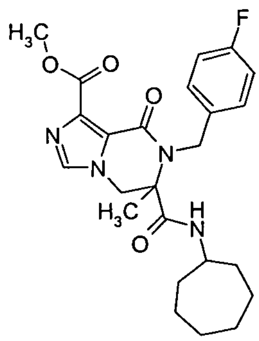 | 456,52 | $^1$HNMR (400MHz, DMSO+$CCl_4$, 25 °C): δ=1.22-1.7 (m, 12 H; cycloheptane), 1.5 (s, 3 H; $CH_3$), 3.51-3.67 (m, 1 H; CH), 3.82 (s, 3 H; $CH_3$), 4.08 (d, *J* = 6.2 Hz, 1 H; $CH_2$), 4.20 (d, *J* = 5.8 Hz, 1 H; $CH_2$), 4.89 (d, *J* = 6.2 Hz, 1 H; $CH_2$), 5.3 (d, *J* = 5.8 Hz, 1 H; $CH_2$), 7.0 (dd, *J* = 7.6 Hz, 2 H; ArH), 7.28-7.39 (m(F), 2 H, ArH), 7.41 (d, *J* = 4.4 Hz, 1 H; NH), 7.68 (s, 1 H; ArH); HRMS *m*/*z* 456.5170 [$M^+$]. |

(continued)

| № | Formula | Mol. w. | Spectral data |
|---|---|---|---|
| 17 | 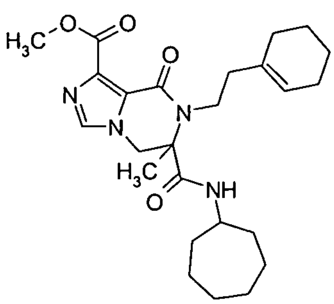 | 456,59 | $^1$HNMR (400MHz, DMSO+$CCl_4$, 25 °C): δ=1.21-1.73 (m, 12 H; cycloheptane), 1.21-1.73 (m, 4 H; cyclohexene), 1.53 (s, 3 H; $CH_3$), 2.04 (m, 2 H; $CH_2$), 2.1-2.26 (m, 4 H; cyclohexene), 3.13-3.26 (m, 1 H; $CH_2$), 3.6-3.8 (m, 2 H; CH, $CH_2$), 3.8 (s, 3 H; $CH_3$), 4.05 (d, *J*= 5.7 Hz, 1 H; $CH_2$), 4.72 (d, *J* = 5.7 Hz, 1 H; $CH_2$), 5.4 (s, 1 H, CH), 7.37 (d, *J* = 4.4 Hz, 1 H; NH), 7.64 (s, 1 H; ArH); HRMS *m*/*z* 456.5858 [$M^+$]. |
| 18 | 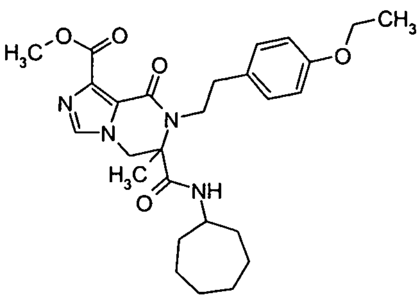 | 496,61 | $^1$HNMR (400MHz, DMSO+$CCl_4$, 25 °C): δ=1.3-1.65 (m, 12 H; cycloheptane), 1.45 (t, *J*= 5.4 Hz, 3 H; $CH_3$), 1.52 (s, 3 H; $CH_3$), 2.8-3.0 (m, 2 H; $CH_2$), 3.21-3.29 (m, 1 H; $CH_2$), 3.59-3.71 (m, 1 H; CH), 3.86 (s, 3 H; $CH_3$), 3.9-4.03 (m, 4 H; CH, $CH_2$), 4.77 (d, *J* = 5.8 Hz, 1 H; $CH_2$), 6.82 (d, *J* = 7.8 Hz, 2 H; ArH), 7.11 (d, *J* = 7.8 Hz, 2 H; ArH), 7.41 (br s, 1 H; NH), 7.64 (s, 1 H; ArH); HRMS m/z 496.6071 [$M^+$]. |
| 19 | 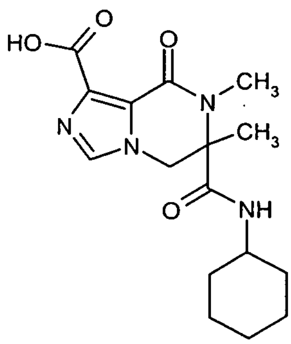 | 334,38 | $^1$HNMR (400MHz, DMSO+$CCl_4$, 25 °C): δ=1.0-1.5 (m, 10 H; cyclohexane), 1.62 (s, 3 H; $CH_3$), 3.03 (s, 3 H; $CH_3$), 3.41-3.58 (m, 1 H; CH), 4.3 (d, *J* = 5.8 Hz, 1 H; $CH_2$), 4.8 (d, *J* = 5.8 Hz, 1 H; $CH_2$), 7.88 (s, 1 H; ArH), 7.9 (d, *J* = 4.2 Hz, 1 H; NH), 14.63 (s, 1 H; OH); $^{13}$C NMR (75 MHz, DMSO, 25 °C) δ=19.46, 24.50, 24.61, 24.99, 29.10, 31.81, 31.89, 48.81, 50.03, 64.89, 123.22, 135.14, 137.98, 159.95, 161.25, 167.63; HRMS *m*/*z* 334.1712 [$M^+$]. |
| 20 | 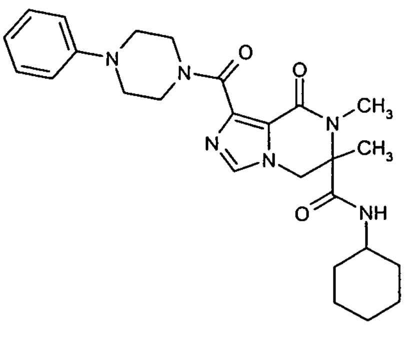 | 478,60 | $^1$HNMR (400MHz, DMSO+$CCl_4$, 25 °C): δ=1.1-1.8 (m, 10 H; cyclohexane), 1.61 (s, 3 H; $CH_3$), 2.91 (s, 3 H; $CH_3$), 3.0-3.33 (m, 6 H; 3$CH_2$), 3.4-3.54 (m, I H; CH), 3.8-3.91 (m, 2 H; $CH_2$), 4.17 (d, *J* = 5.9 Hz, 1 H; $CH_2$), 4.72 (d, *J* = 5.9 Hz, 1 H; $CH_2$), 6.75 (t, *J* = 5.2 Hz, 1 H; ArH), 6.88 (d, *J* = 7.8 Hz, 2 H; ArH), 7.22 (t, *J* = 5.2 Hz, 2 H; ArH), 7.68 (d, *J* = 4.2 Hz, I H; NH), 7.71 (s, 1 H; ArH); $^{13}$C NMR (75 MHz, DMSO, 25 °C) δ=19.92, 24.54, 24.64, 25.06, 28.09, 31.91, 45.73, 48.06, 48.51, 48.62, 50.19, 64.13, 2×115.86, 119.25, 119.61, 2×128.81, 128.91, 136.38, 137.52, 150.75, 158.17, 163.09, 168.64; HRMS *m*/*z* 478.2772 [$M^+$]. |

(continued)

| № | Formula | Mol. w. | Spectral data |
|---|---|---|---|
| 21 | 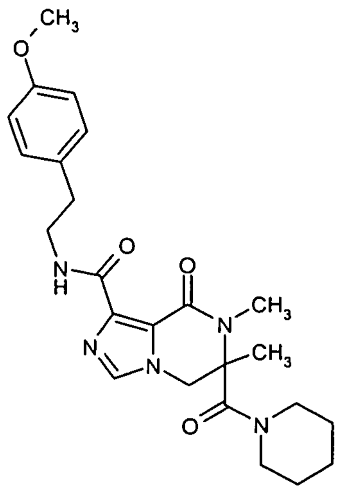 | 453,55 | $^1$HNMR (400MHz, DMSO+$CCl_4$, 25 °C): δ=1.1-1.35 (m, 5 H; cyclohexane), 1.58 (s, 3 H; $CH_3$), 1.59-1.76 (m, 5 H; cyclohexane), 3.01 (s, 3 H; $CH_3$), 3.45-3.57 (m, 1 H; CH), 4.25 (d, *J* = 5.8 Hz, 1 H; $CH_2$), 4.4-4.52 (m, 2 H; $CH_2$), 4.84-4.95 (m, 1 H; $CH_2$), 6.8 (d, *J* = 7.8 Hz, 1 H; ArH), 7.24 (d, *J* = 7.8 Hz, 1 H; ArH), 7.86 (s, 1 H; ArH), 7.92 (d, *J* = 4.0 Hz, 1 H; NH), 10.86 (br s, 1 H; NH); $^{13}$C NMR (75 MHz, DMSO, 25 °C) δ=19.79, 24.52, 24.63, 25.01, 29.03, 31.83, 31.92, 48.58, 50.07, 54.98, 64.04, 2×113.73, 121.17, 2×128.54, 131.14, 136.68, 138.05, 158.18, 159.96, 160.22, 168.19; HRMS *m*/*z* 453.2445 [$M^+$]. |

| | Table 1.09 | | |
|---|---|---|---|
| **№** | **Formula** | **Mol. w.** | **LC MS, *m*/*z* (M+1)** |
| 1 | 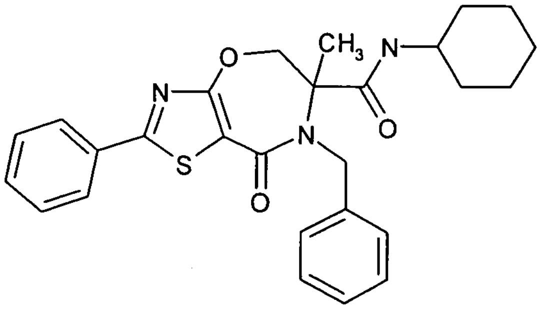 | 475,61448 | 476 |
| 2 | 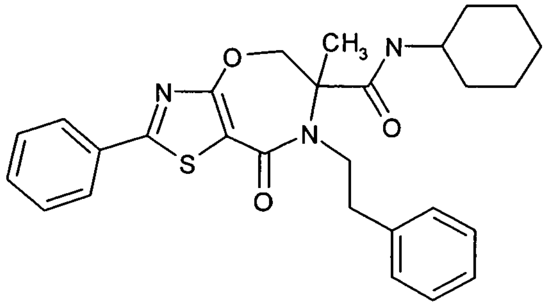 | 489,64157 | 490 |

(continued)

| | Table 1.09 | | |
|---|---|---|---|
| № | Formula | Mol. w. | LC MS, *m/z* (M+1) |
| 3 | 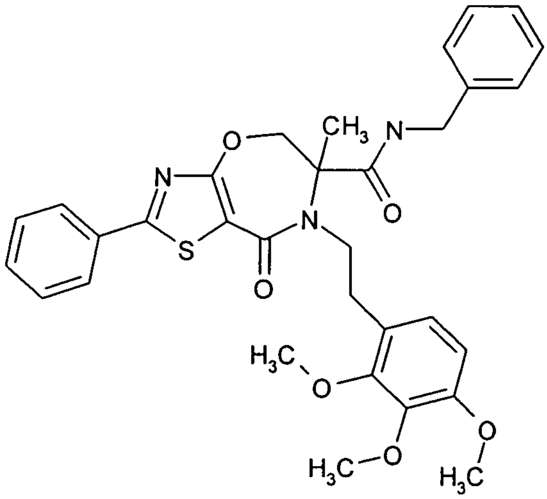 | 587,70031 | 588 |

| | Table 1.10(1) | | |
|---|---|---|---|
| № | Formula | Mol. w. | LC MS, *m/z*(M+1), 1H NMR |
| 1 | 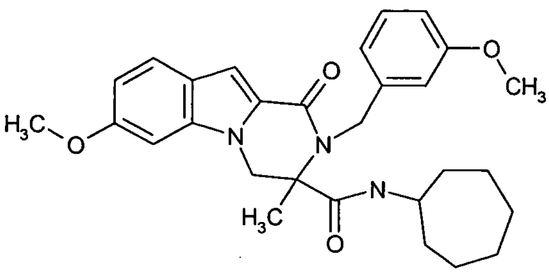 | 489,6200 | LC MS, m/z: 490. (M+1). 1H NMR: m. 1.09 - 1.57 (15H, CH of cycloheptyl and CH3 of cycle), m. 3.50-3.62 (1 H, CH of cycloheptyl), s. 3.77 (3H, OCH3 at m-MeOPh), s. 3.85 (3H, OCH3 at 6CH of indolic ring), dd. 4.00, 4.93 (J=12.65Hz, 2H, CH2 of cycle), dd. 4.28, 5.26 (J=16.80 Hz, 2H, N-CH2-Ph) m. 6.67-6.75 (2H, CH at C5 of indolic ring and CH of m-MeOPh), s. 6.85 (1H, CH at C7 of indolic ring), br.s. 6.89 (2H, 2CH of m-MeOPh), s. 7.00 (1 H, CH at C3 of indolic ring), m. 7.09-7.22 (2H, CH of m-MeOPh and NH of CO-NH-cycloheptyl), d. 7.47 (J=9.2Hz, 1 H, CH at C4 of indolic ring) |

(continued)

| | Table 1.10(1) | | |
|---|---|---|---|
| № | Formula | Mol. w. | LC MS, *m/z*(M+1), 1H NMR |
| 2 | 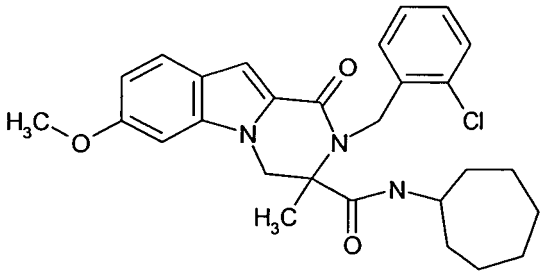 | 494,0385 | LC MS, m/z: 495. (M+1). 1H NMR: m. 1.15 - 1.647 (15H, CH of cycloheptyl and CH3 of cycle), m. 3.59-3.70 (1H, CH of cycloheptyl), s. 3.86 (3H, OCH3 at C6 of indolic ring), dd. 4.10, 5.00 (J=12.95Hz, 2H, CH2 of cycle), dd. 4.30, 5.27 (J=17.40Hz, 2H, N-CH2-Ph) d. 6.71(JH4H5 = 8.80Hz, 1H, CH at C5 of indolic ring), s. 6.856 (1H, CH at C7 of indolic ring), s. 6.99 (1H, CH at C3 of indolic ring), m. 7.16-7.26 (2H, 2CH of o ClPh), m. 7.32-7.38 (2H, 2CH of o-ClPh), d. 7.43 (J=8.10Hz,1H, NH of CO-NH-cycloheptyl) d. 7.48 (JH4H5 = 8.80Hz, 1H, CH at C5 of indolic ring) |
| 3 | 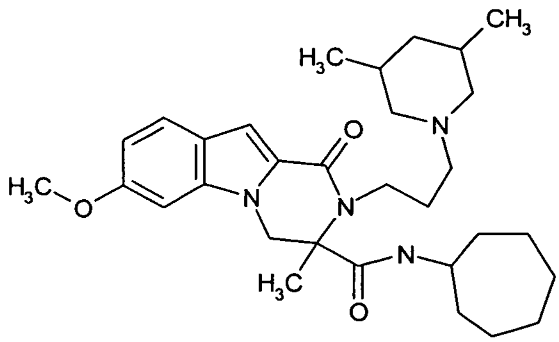 | 522,7373 | LC MS, m/z: 523. (M+1). 1H NMR: m. 0.40 - 2.35 (32H, CH and CH3 of alkyl), m. 2.75-2.90 (1 H, CH of alkyl), m. 3.14-3.26 (1 H, CH of alkyl), m. 3.48-3.62 (1 H, CH of alkyl), m. 3.65-3.75 (1 H, CH of cycloheptyl), s. 3.84 (3H, OCH3 at C6 of indolic ring), dd. 4.04, 4.70 (J=12.65Hz, 2H, CH2 of cycle), d. 6.69. (JH4H5 = 9.15Hz, 1 H, CH at C5 of indolic ring), s. 6.85 (1 H, CH at C7 of indolic ring), s. 6.92 (1 H, CH at C3 of indolic ring), br.d. 7.30 (1 H, NH of CO-NH-cycloheptyl), d. 7.44 (JH4H5 = 9.15Hz, 1 H, CH at C4 of indolic ring) |
| 4 | 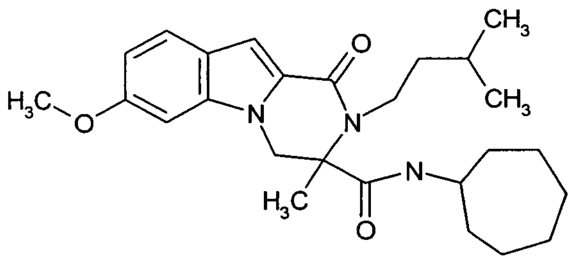 | 439,6031 | LC MS, m/z: 440. (M+1). 1H NMR: d. 0.97 (J=6.05Hz, 6H, 2CH3 of alkyl), m. 1.29 - 1.72 (18H, CH of alkyl and CH3 of cycle), m. 3.12-3.20 (1 H, CH of alkyl), m. 3.48-3.55 (1 H, CH of alkyl), m. 3.65-3.75 (1 H, CH of cycloheptyl) s. 3.84 (3H, OCH3 at C6 of indolic ring), dd. 4.04, 4.69 (J=12.35Hz, 2H, CH2 of cycle), d. 6.67. (JH4H5 = 8.70Hz, 1 H, CH at C5 of indolic ring), s. 6.85 (1 H, CH at C7 of indolic ring), s. 6.92 (1 H, CH at C3 of indolic ring), d. 7.37 (J=8.30Hz, 1 H, NH of CO-NH-cycloheptyl), d. 7.43 (JH4H5 = 8.70Hz, 1H, CH at C4 of indolic ring) |

(continued)

| | Table 1.10(1) | | |
|---|---|---|---|
| № | Formula | Mol. w. | LC MS, *m/z*(M+1), 1H NMR |
| 5 | 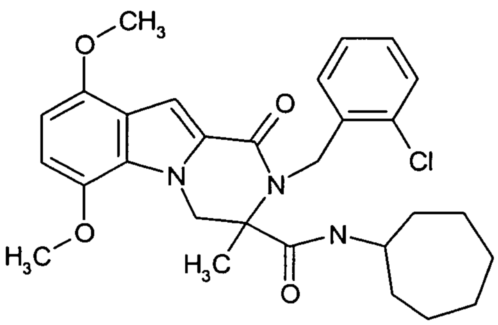 | 524,0650 | LC MS, m/z: 525. (M+1). 1H NMR: m.1.20 - 1.64 (15H, CH of cycloheptyl and CH3 of cycle), m. 3.58-3.70 (1H, CH of cycloheptyl), s. 3.87 (3H, OCH3 at C4 of indolic ring), s. 3.91 (3H, OCH3 at C7 of indolic ring), dd. 4.25, 5.27 (J=18.70Hz, 2H, N-CH2-Ph) dd. 4.30, 5.40 (J=13.20Hz, 2H, CH2 of cycle), d. 6.30 (JH5H6 = 8.45 Hz 1H, H at C5 of indolic ring), d. 6.56 (JH5H6 = 8.45 Hz 1 H, H at C6 of indolic ring), s. 7.07 (1 H, H at C3 of indolic ring), m. 7.16-7.26 (4H, 4CH of o-ClPh) d. 7.50 (J=7.8Hz, 1 H NH of CO-NH-cycloheptyl) |
| 6 | 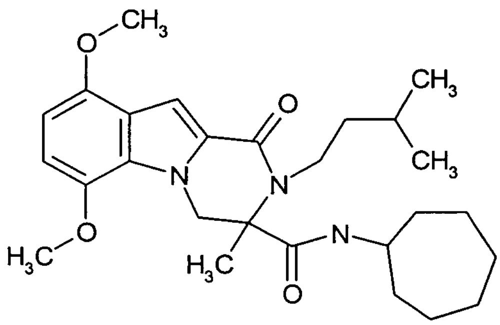 | 469,6296 | LC MS, m/z: 470. (M+1). 1H NMR: d. 0.96 (J=6.10Hz, 6H, 2CH3 of alkyl), m.1.30 - 1.72 (18H, CH of alkyl and CH3 of cycle), m. 3.04-3.15 (1 H, CH of alkyl), m. 3.51-3.61 (1H, CH of alkyl), m. 3.66-3.76 (1 H, CH of cycloheptyl) s. 3.86 (3H, OCH3 at C4 of indolic ring), s. 3.89 (3H, OCH3 at C7 of indolic ring), dd. 4.28, 5.04 (J=13.00Hz, 2H, CH2 of cycle), d. 6.27 (JH5H6 = 8.20 Hz 1 H, H at C5 of indolic ring), d. 6.52 (JH5H6 = 8.20 Hz 1 H, H at C6 of indolic ring), s. 6.98 (1 H, H at C3 of indolic ring), d. 7.37 (J=8.2Hz, 1 H, NH of CO-NH-cycloheptyl) |
| 7 | 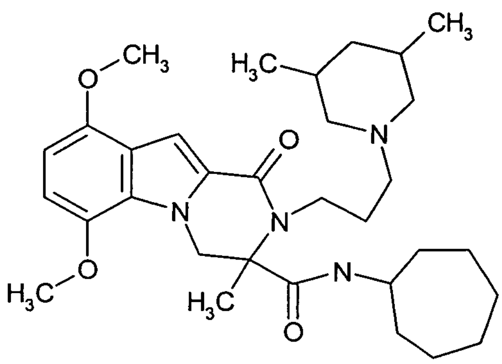 | 552,7638 | LC MS, m/z: 553. (M+1). 1H NMR: mm. 0.41 - 2.31 (32H, CH and CH3 of alkyl), m. 2.72-2.84(1 H, CH of alkyl), m. 3.10-3.22 (1 H, CH of alkyl), m. 3.45-3.59(1H, CH of alkyl), m. 3.64-3.74 (1H, CH of cycloheptyl), s. 3.86 (3H, OCH3 at C4 of indolic ring), s. 3.90 (3H, OCH3 at C7 of indolic ring), dd. 4.29, 5.03 (J=12.80Hz, 2H, CH2 of cycle), d. 6.27 (JH5H6 = 8.25 Hz 1H, H at C5 of indolic ring), d. 6.54 (JH5H6 = 8.25 Hz 1H, H at C6 of indolic ring), s. 6.99 (1 H, H at C3 of indolic ring), br.d. 7.36 (1H, NH of CO-NH-cycloheptyl) |

(continued)

| | Table 1.10(1) | | |
|---|---|---|---|
| № | Formula | Mol. w. | LC MS, *m/z*(M+1), 1H NMR |
| 8 | 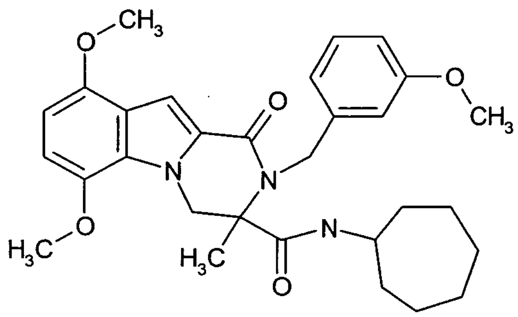 | 519,6465 | LC MS, m/z: 520. (M+1). 1H NMR: m. 1.16-1.67 (15H, CH of cycloheptyl and CH3 of cycle), m. 3.55-3.65 (1 H, CH of cycloheptyl), s. 3.77 (3H, OCH3 at m-MeOPh), s. 3.87 (3H, OCH3 at C4 of indolic ring), s. 3.90 (3H, OCH3 at C7 of indolic ring),dd. 4.19, 5.13 (J=13.20Hz, 2H, CH2 of cycle), dd. 4.24, 5.26 (J=16.10 Hz, 2H, N-CH2-Ph) d. 6.29 (JH5H6 = 8.05 Hz 1H, H at C5 of indolic ring), d. 6.54(JH5H6 = 8.05 Hz 1H, H at C6 of indolic ring), d. 6.72 (J=8.7Hz, 1 H, CH of m-MeOPh), m. 6.82-6.85 (2H, 2CH of m-MeOPh), s. 7.07(1H, CH at C3 of indolic ring), m. 7.12-7.21 (2H, CH of m-MeOPh and NH of CO-NH-cycloheptyl) |
| 9 | 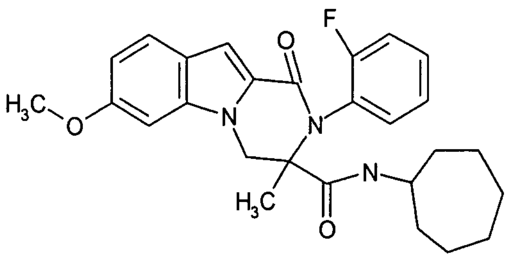 | 463,5569 | 464 |
| 10 | 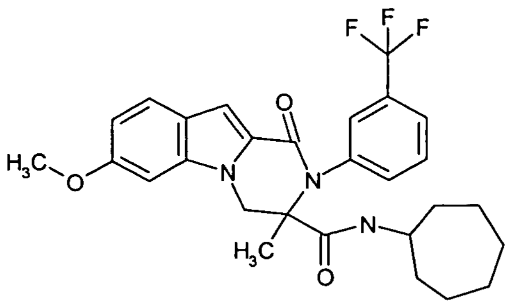 | 513,5648 | 514 |

(continued)

| | Table 1.10(1) | | |
|---|---|---|---|
| № | Formula | Mol. w. | LC MS, *m/z*(M+1), 1H NMR |
| 11 | 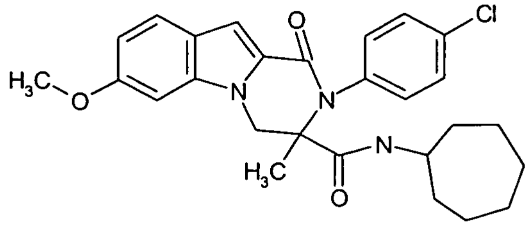 | 480,0115 | LC MS, m/z: 481. (M+1). 1H NMR: m. 1.06-1.70 (15H, CH of cycloheptyl and CH3 of cycle), m. 3.58-3.61 (1 H, CH of cycloheptyl), s. 3.86 (3H, OCH3 at C6 of indolic ring), dd. 4.22, 5.09 (J=12.85Hz, 2H, CH2 of cycle), d. 6.69. (JH4H5 = 8.75Hz, 1 H, CH at C5 of indolic ring), s. 6.85 (1H, CH at C7 of indolic ring), br.s. 6.92 (JH3H4-2.2Hz, 1 H, CH at C3 of indolic ring), m. 7.35-7.44 (4H, 4CH of p-ClPh), br.d. 7.49 (JH4H5 = 8.75Hz, JH3H4-2.2Hz, 1H,CH at C4 of indolic ring) br.d. 7.30 (J=7.60Hz, 1 H, NH of CO-NH- cycloheptyl) |
| 12 | 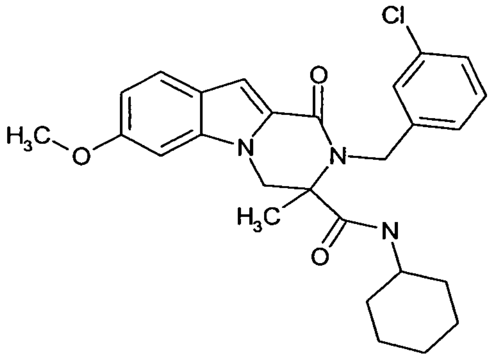 | 480,0115 | 481 |
| 13 | 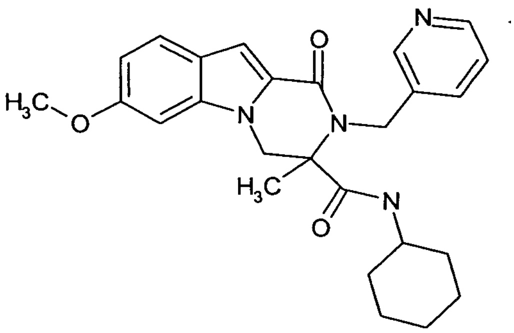 | 446,5540 | 447 |
| 14 | 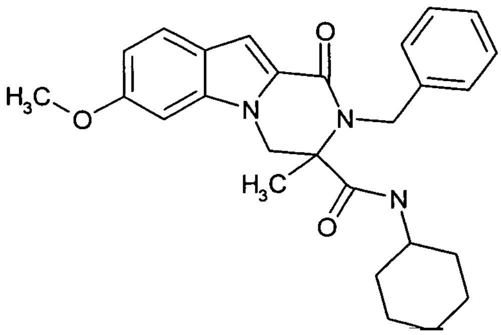 | 445,5664 | 446 |

(continued)

| | Table 1.10(1) | | |
|---|---|---|---|
| № | Formula | Mol. w. | LC MS, *m*/*z*(M+1), 1H NMR |
| 15 | 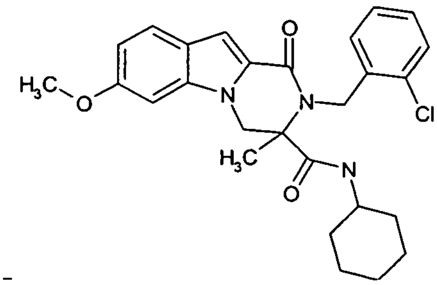 | 480,0115 | 481 |
| 16 | 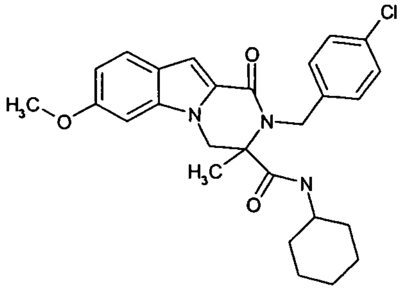 | 480,0115 | 481 |
| 17 | 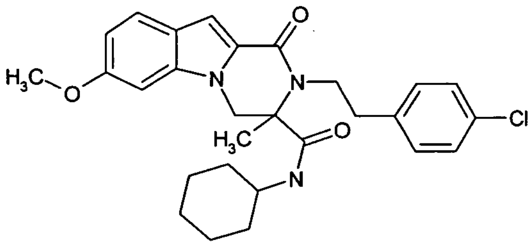 | 494,0385 | 495 |
| 18 | 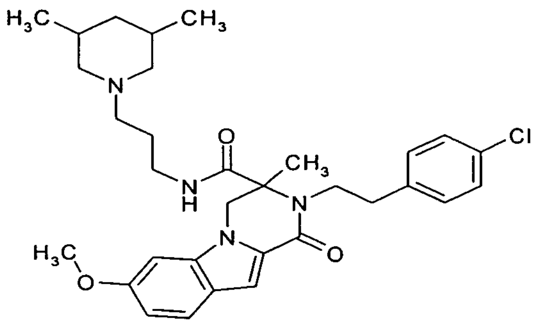 | 565,16 | 566 |
| 19 | 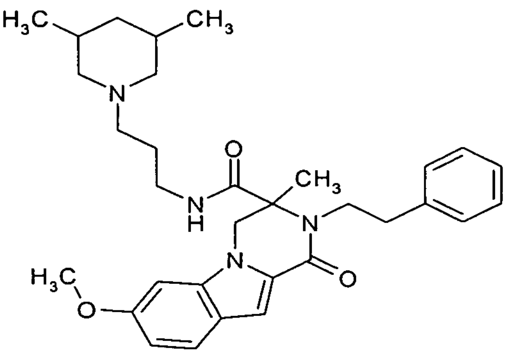 | 530,72 | 531 |

(continued)

| | Table 1.10(1) | | |
|---|---|---|---|
| № | Formula | Mol. w. | LC MS, *m*/*z*(M+1), 1H NMR |
| 20 | 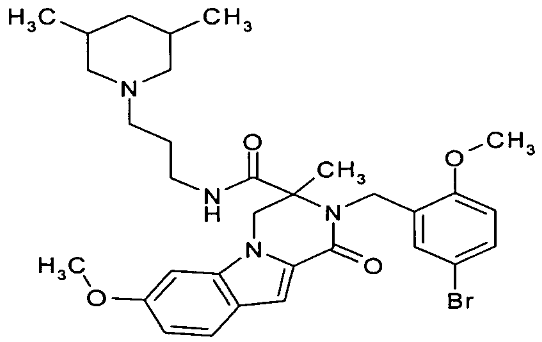 | 625,61 | 626 |
| 21 | 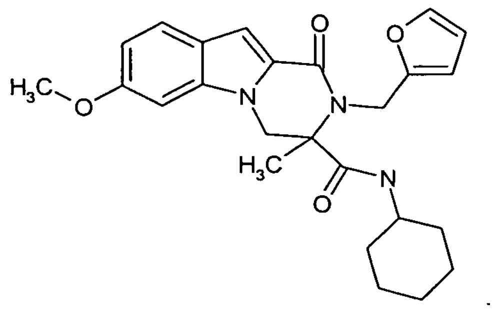 | 435,5276 | 436 |
| 22 | 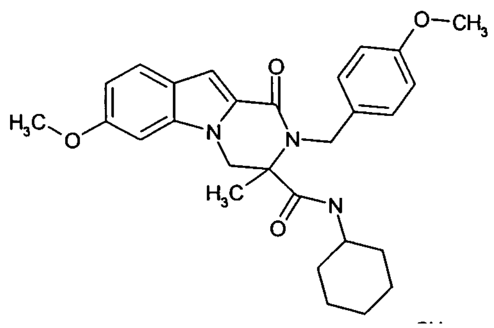 | 475,5929 | 476 |
| 23 | 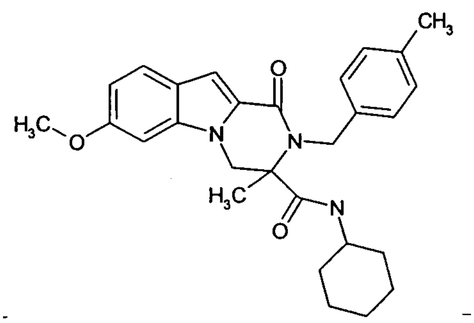 | 459,5935 | 460 |

(continued)

| | Table 1.10(1) | | |
|---|---|---|---|
| № | Formula | Mol. w. | LC MS, *m/z*(M+1), 1H NMR |
| 24 | 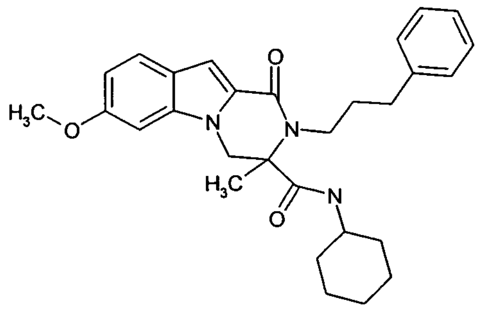 | 473,6206 | 474 |
| 25 | 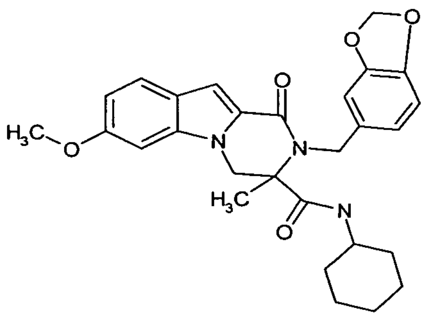 | 489,5764 | 490 |
| 26 | 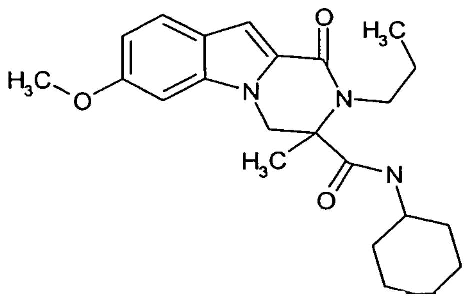 | 397,5218 | 398 |
| 27 | 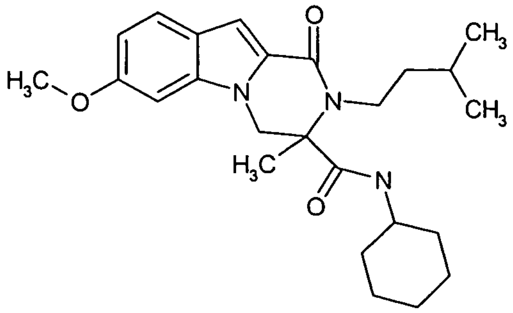 | 425,5760 | 426 |
| 28 | 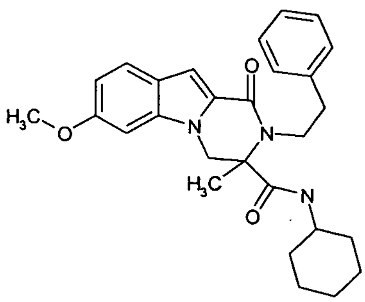 | 459,5935 | 460 |

(continued)

| | Table 1.10(1) | | |
|---|---|---|---|
| № | Formula | Mol. w. | LC MS, *m/z*(M+1), 1H NMR |
| 29 | 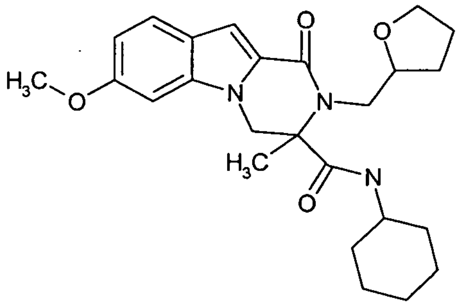 | 439,5595 | 440 |
| 30 | 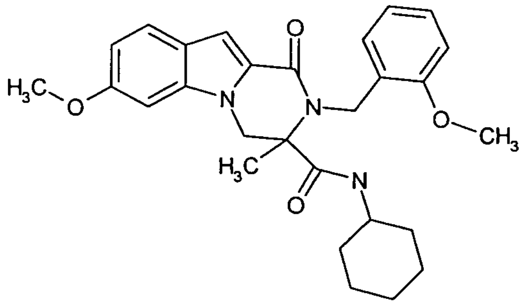 | 475,5929 | 476 |
| 31 | 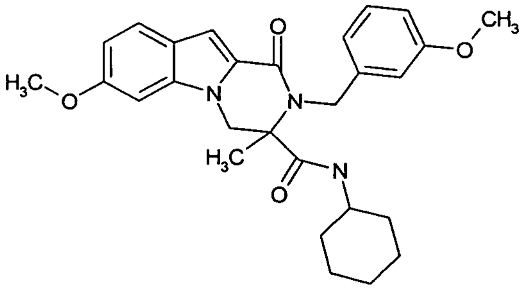 | 475,5929 | 476 |
| 32 | 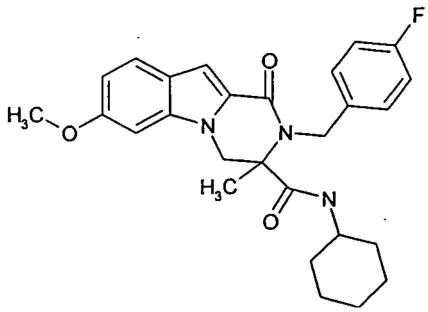 | 463,5569 | 464 |
| 33 | 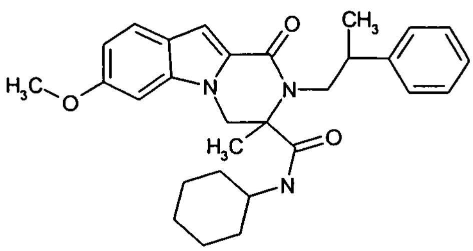 | 473,6206 | 474 |

(continued)

| | **Table 1.10(1)** | | |
|---|---|---|---|
| **№** | **Formula** | **Mol. w.** | **LC MS, *m/z*(M+1), 1H NMR** |
| 34 | 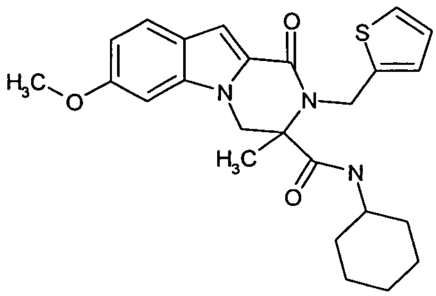 | 451,5922 | 452 |
| 35 | 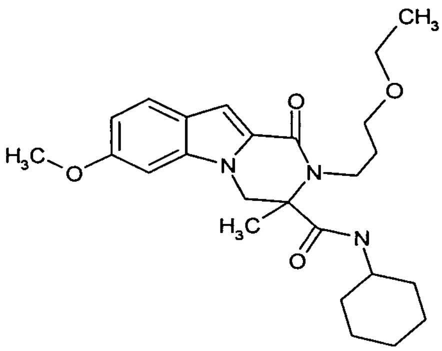 | 441,5754 | 442 |
| 36 | 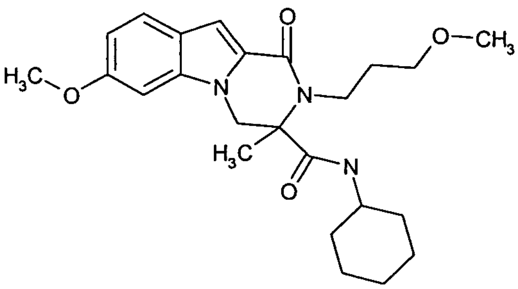 | 427,5483 | 428 |
| 37 | 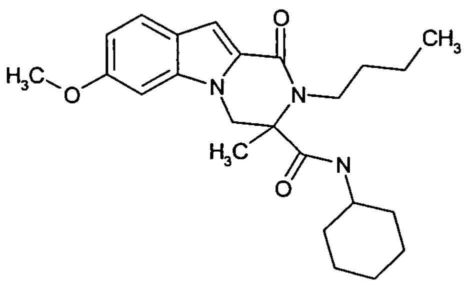 | 411,5489 | 412 |
| 38 | 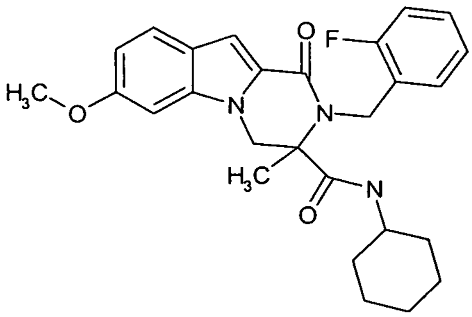 | 463,5569 | 464 |

(continued)

| | Table 1.10(1) | | |
|---|---|---|---|
| № | Formula | Mol. w. | LC MS, *m/z*(M+1), 1H NMR |
| 39 | 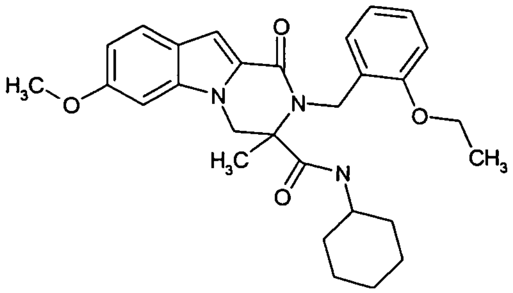 | 489,6200 | 490 |
| 40 | 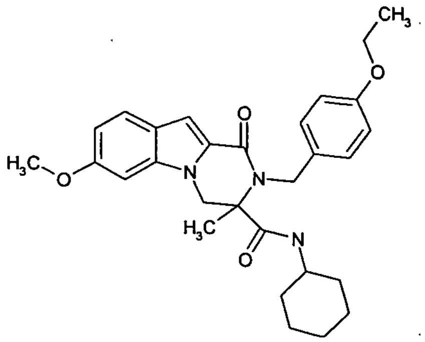 | 489,6200 | 490 |
| 41 | 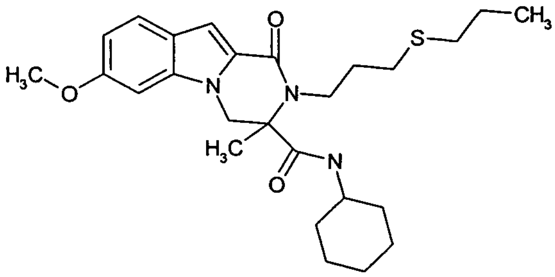 | 471,6671 | 472 |
| 42 | 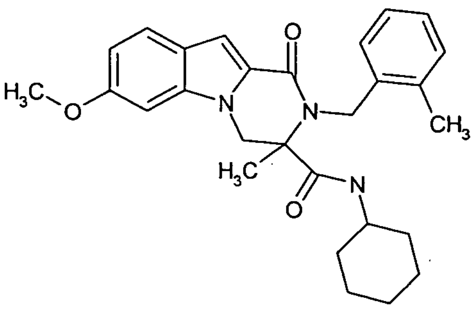 | 459,5935 | 460 |
| 43 | 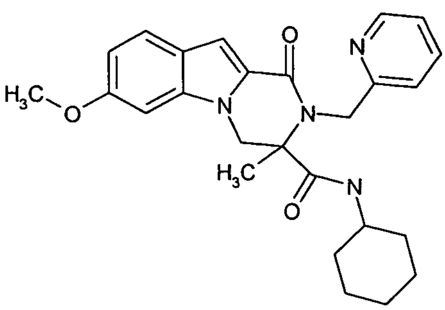 | 446,5540 | 447 |

(continued)

| | Table 1.10(1) | | |
|---|---|---|---|
| № | Formula | Mol. w. | LC MS, *m/z*(M+1), 1H NMR |
| 44 | 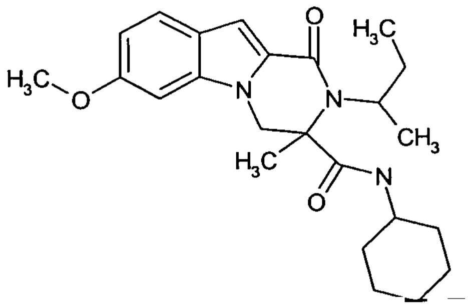 | 411,5489 | 412 |
| 45 | 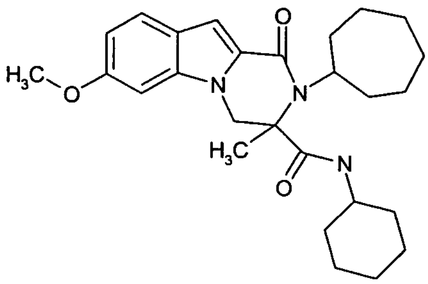 | 451,6142 | 452 |
| 46 | 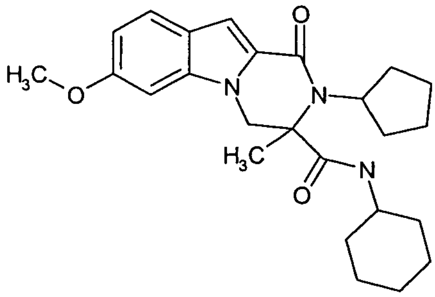 | 423,5601 | 424 |
| 47 | 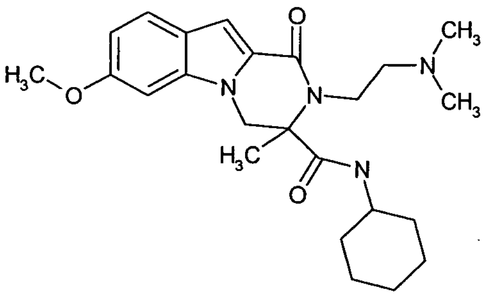 | 426,5636 | 427 |
| 48 | 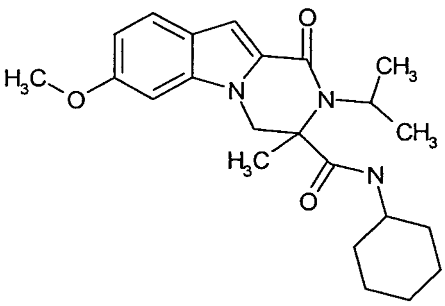 | 397,5218 | 398 |

(continued)

| | Table 1.10(1) | | |
|---|---|---|---|
| № | Formula | Mol. w. | LC MS, *m/z*(M+1), 1H NMR |
| 49 | 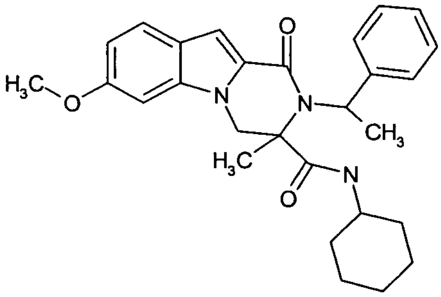 | 459,5935 | 460 |
| 50 | 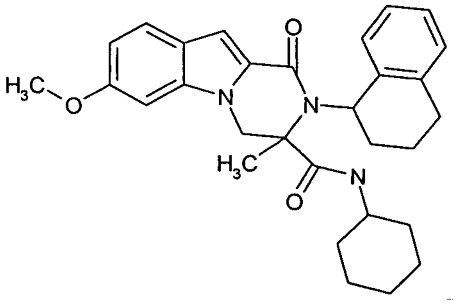 | 485,6318 | 486 |
| 51 | 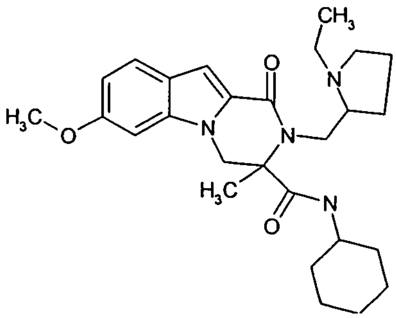 | 466,6289 | 467 |
| 52 | 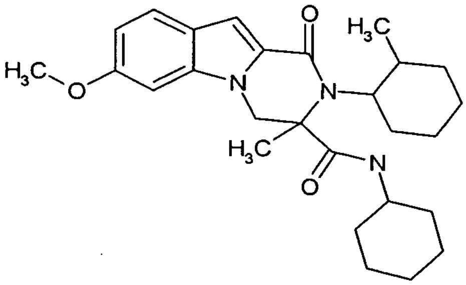 | 451,6142 | 452 |
| 53 | 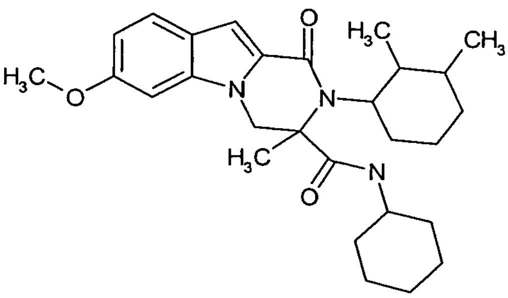 | 465,6413 | 466 |

(continued)

| | Table 1.10(1) | | |
|---|---|---|---|
| № | Formula | Mol. w. | LC MS, *m*/*z*(M+1), 1H NMR |
| 54 | 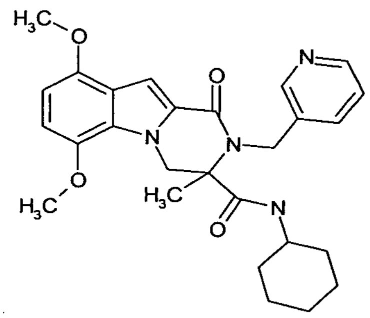 | 476,5805 | 477 |
| 55 | 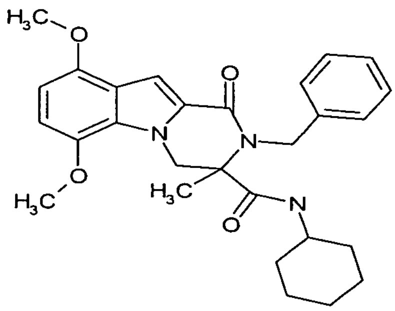 | 475,5929 | 476 |
| 56 | 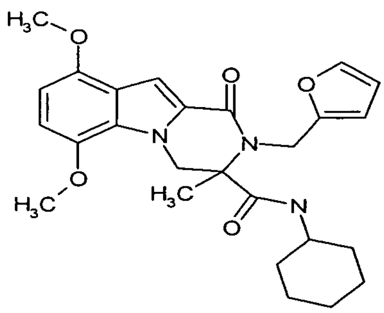 | 465,5541 | 466 |
| 57 | 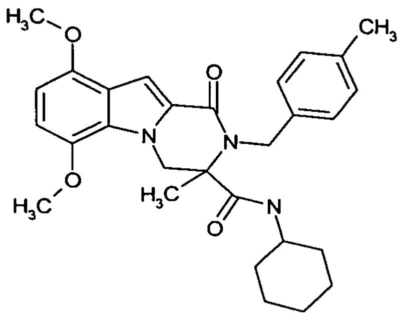 | 489,6200 | 490 |
| 58 | 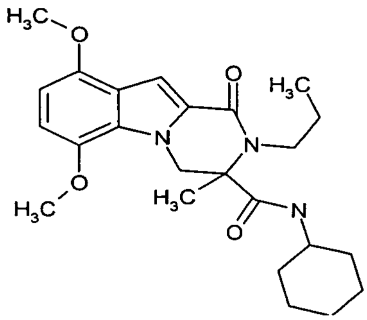 | 427,5483 | 428 |

(continued)

| | Table 1.10(1) | | |
|---|---|---|---|
| № | Formula | Mol. w. | LC MS, *m*/*z*(M+1), 1H NMR |
| 59 | 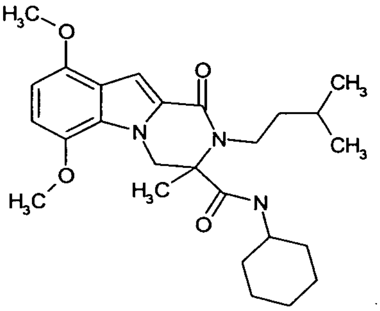 | 455,6025 | 456 |
| 60 | 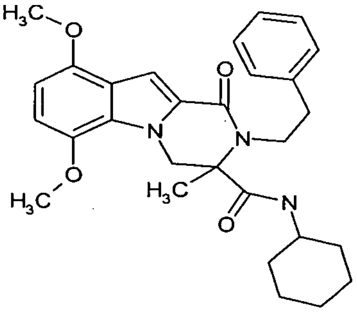 | 489,6200 | 491 |
| 61 | 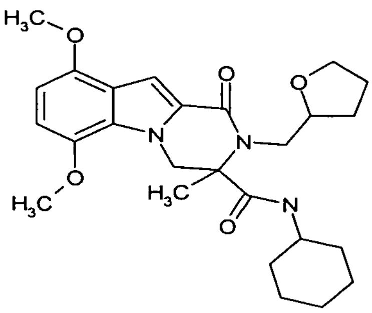 | 469,5860 | 470 |
| 62 | 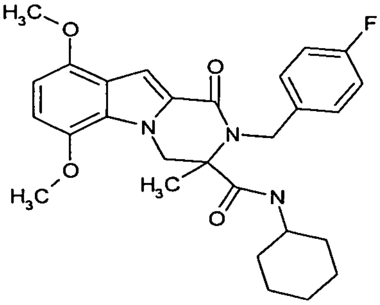 | 493,5833 | 494 |
| 63 | 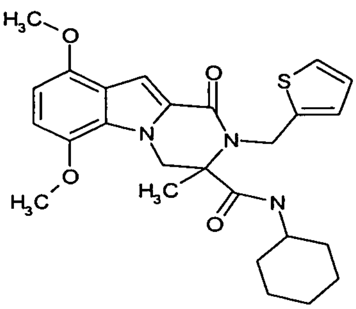 | 481,6187 | 482 |

(continued)

| | Table 1.10(1) | | |
|---|---|---|---|
| № | Formula | Mol. w. | LC MS, *m*/*z*(M+1), 1H NMR |
| 64 | 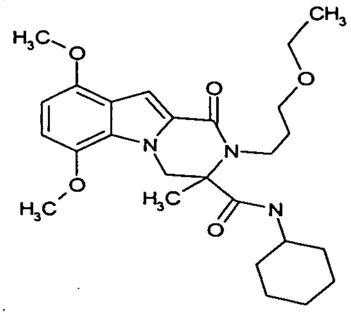 | 471,6019 | 472 |
| 65 | 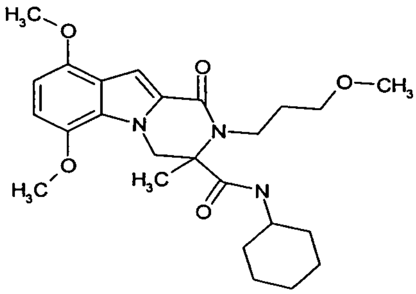 | 457,5748 | 458 |
| 66 | 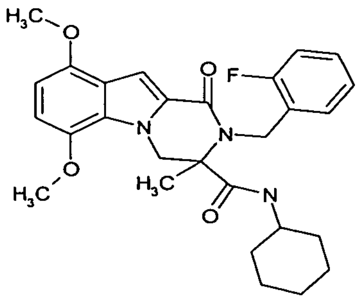 | 493,5833 | 494 |
| 67 | 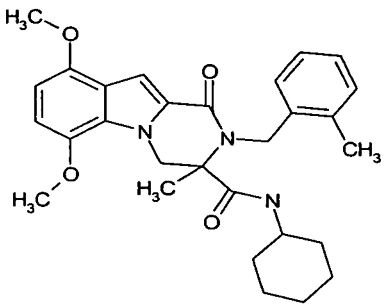 | 489,6200 | 490 |
| 68 | 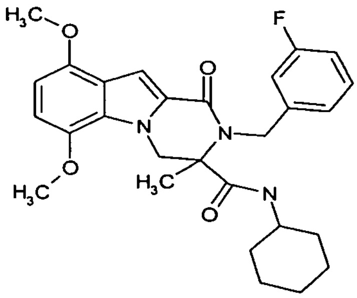 | 493,5833 | 494 |

(continued)

| | Table 1.10(1) | | |
|---|---|---|---|
| № | Formula | Mol. w. | LC MS, *m*/*z*(M+1), 1H NMR |
| 69 | 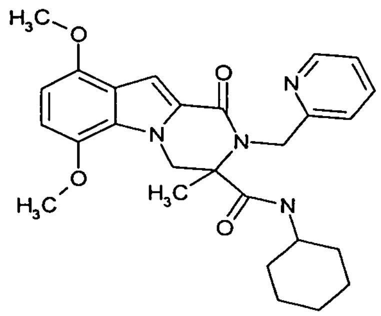 | 476,5805 | 477 |
| 70 | 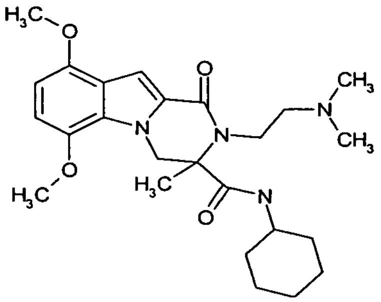 | 456,5901 | 457 |
| 71 | 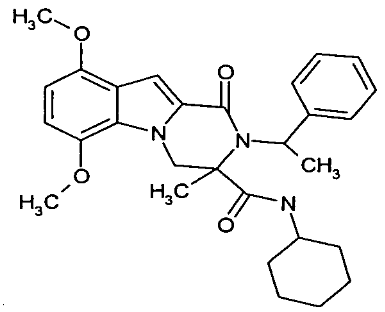 | 489,6200 | 490 |
| 72 | 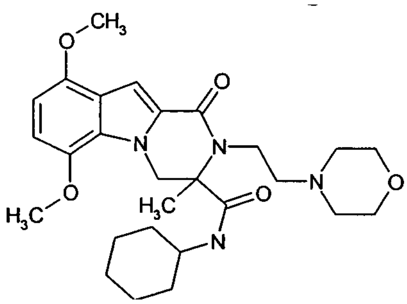 | 498,6277 | 499 |
| 73 | 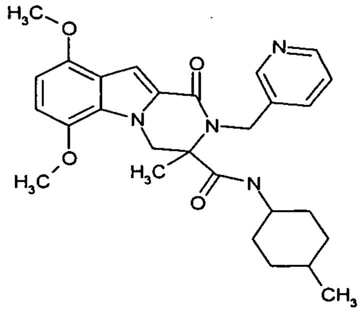 | 490,6076 | 491 |

(continued)

| | Table 1.10(1) | | |
|---|---|---|---|
| № | Formula | Mol. w. | LC MS, *m/z*(M+1), 1H NMR |
| 74 | 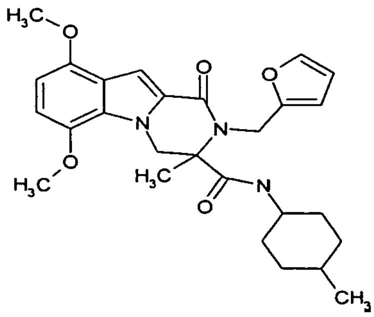 | 479,5812 | 480 |
| 75 | 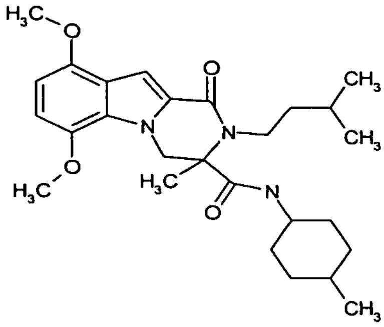 | 469,6296 | 470 |
| 76 | 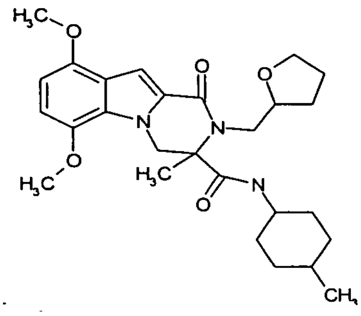 | 483,6130 | 484 |
| 77 | 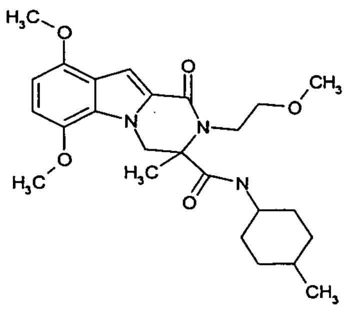 | 457,5748 | 458 |
| 78 | 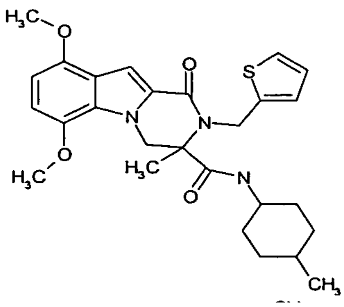 | 495,6458 | 496 |

(continued)

| | Table 1.10(1) | | |
|---|---|---|---|
| № | Formula | Mol. w. | LC MS, *m/z*(M+1), 1H NMR |
| 79 | 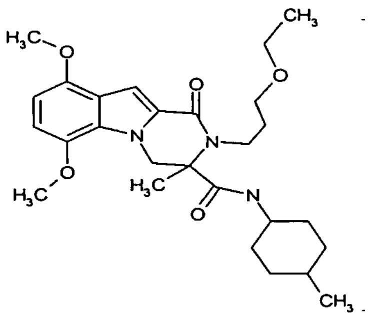 | 485,6290 | 486 |
| 80 | 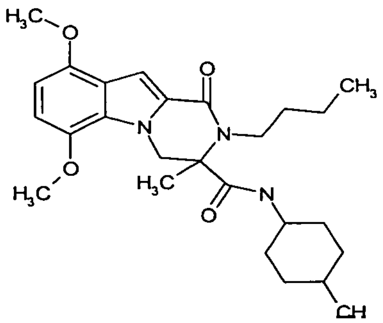 | 455,6025 | 456 |
| 81 | 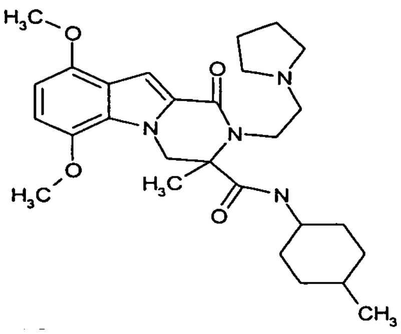 | 496,6554 | 497 |
| 82 | 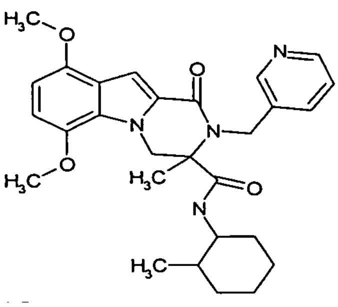 | 490,6076 | 491 |
| 83 | 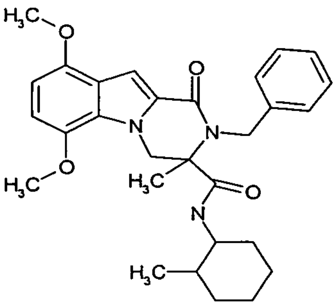 | 489,6200 | 490 |

(continued)

| | Table 1.10(1) | | |
|---|---|---|---|
| № | Formula | Mol. w. | LC MS, *m*/*z*(M+1), 1H NMR |
| 84 | 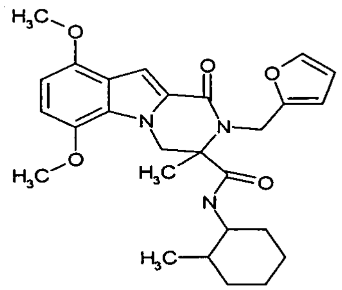 | 479,5812 | 480 |
| 85 | 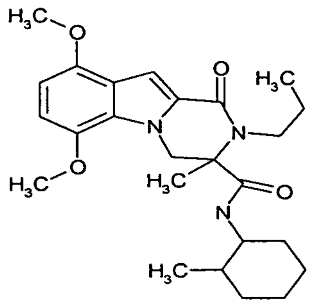 | 441,5754 | 442 |
| 86 | 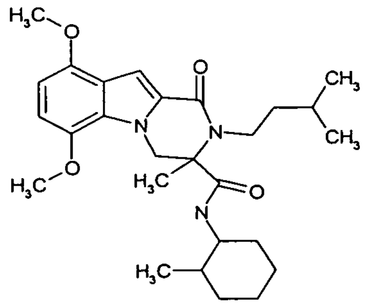 | 469,6296 | 470 |
| 87 | 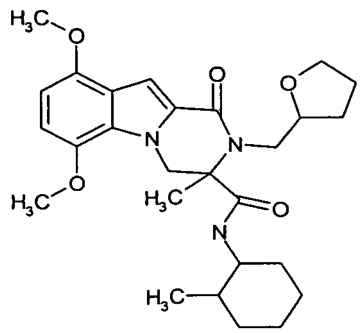 | 483,6130 | 484 |
| 88 | 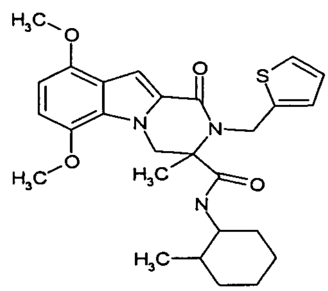 | 495,6458 | 496 |

(continued)

| | Table 1.10(1) | | |
|---|---|---|---|
| № | Formula | Mol. w. | LC MS, *m*/*z*(M+1), 1H NMR |
| 89 | 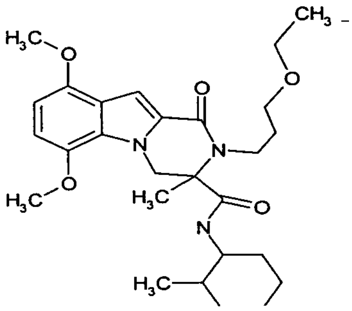 | 485,6290 | 486 |
| 90 | 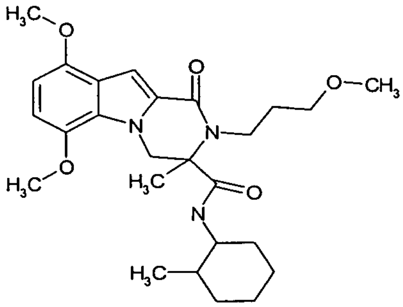 | 471,6019 | 472 |
| 91 | 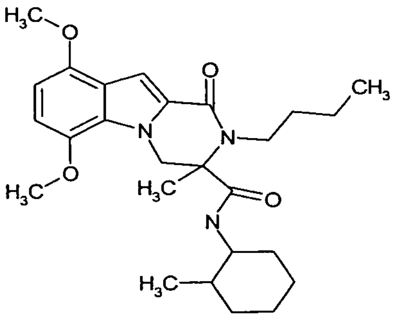 | 455,6025 | 456 |
| 92 | 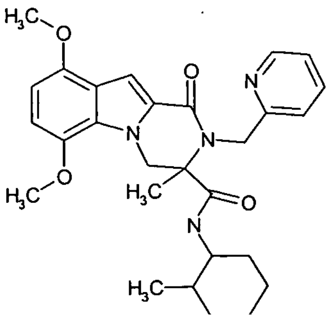 | 490,6076 | 491 |
| 93 | 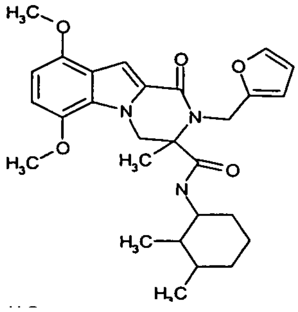 | 493,6083 | 494 |

(continued)

| | Table 1.10(1) | | |
|---|---|---|---|
| № | Formula | Mol. w. | LC MS, *m/z*(M+1), 1H NMR |
| 94 | 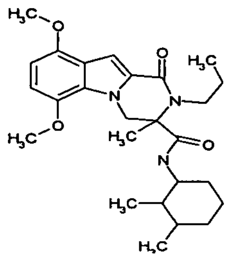 | 455,6025 | 456 |
| 95 | 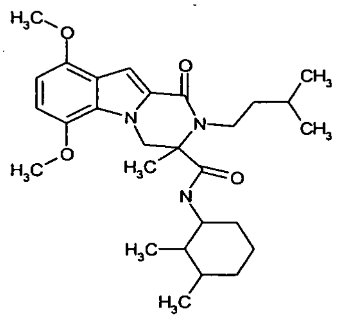 | 483,6567 | 484 |
| 96 | 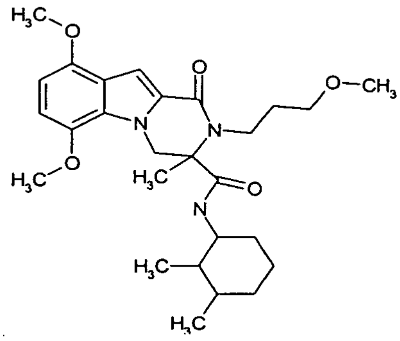 | 485,6290 | 486 |
| 97 | 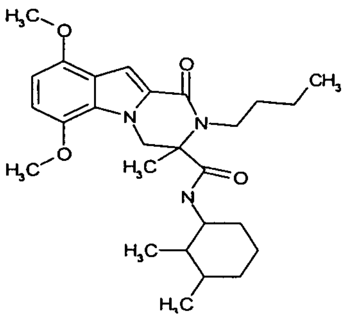 | 469,6296 | 470 |
| 98 | 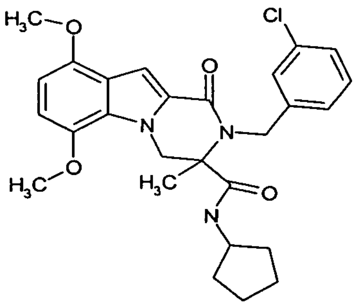 | 496,0109 | 497 |

(continued)

| | Table 1.10(1) | | |
|---|---|---|---|
| № | Formula | Mol. w. | LC MS, *m/z*(M+1), 1H NMR |
| 99 | 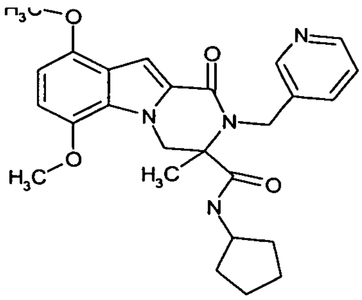 | 462,5534 | 463 |
| 100 | 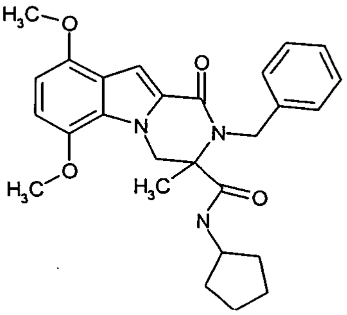 | 461,5658 | 462 |
| 101 | 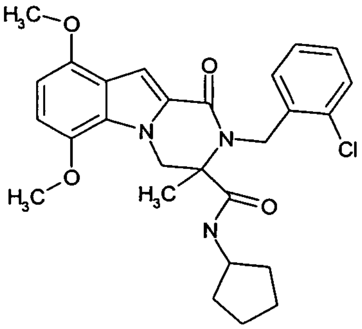 | 496,0109 | 497 |
| 102 | 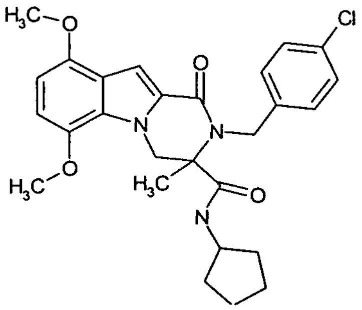 | 496,0109 | 497 |
| 103 | 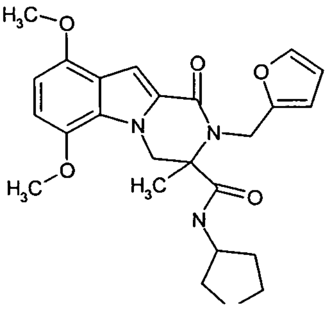 | 451,5270 | 452 |

(continued)

| | Table 1.10(1) | | |
|---|---|---|---|
| № | Formula | Mol. w. | LC MS, *m*/*z*(M+1), 1H NMR |
| 104 | 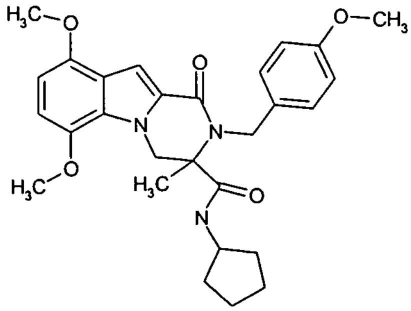 | 491,5923 | 492 |
| 105 | 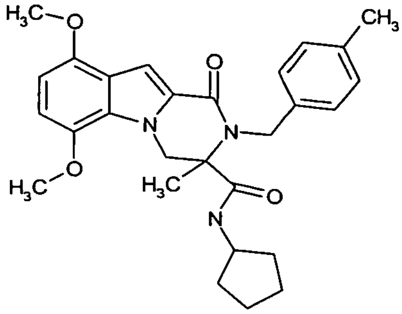 | 475,5929 | 476 |
| 106 | 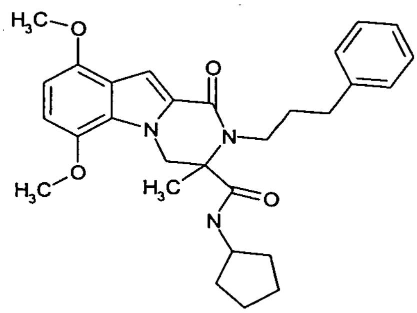 | 489,6200 | 490 |
| 107 | 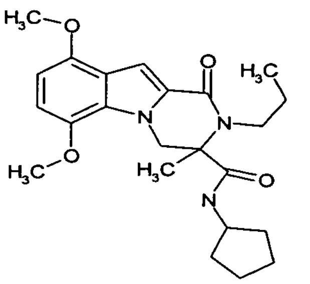 | 413,5212 | 414 |
| 108 | 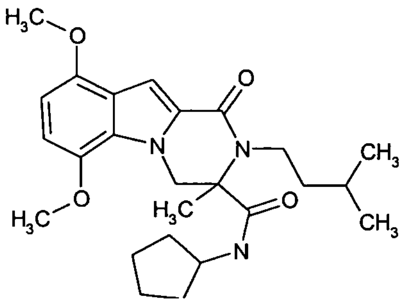 | 441,5754 | 442 |

(continued)

| | Table 1.10(1) | | |
|---|---|---|---|
| № | Formula | Mol. w. | LC MS, *m/z*(M+1), 1H NMR |
| 109 | 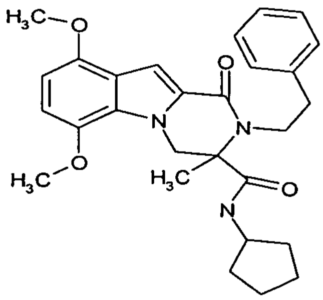 | 475,5929 | 476 |
| 110 | 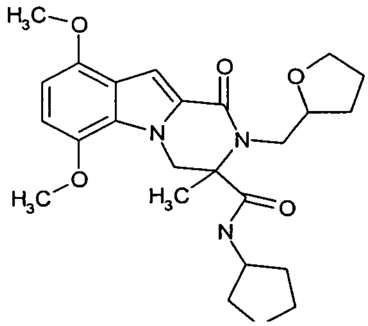 | 455,5589 | 456 |
| 111 | 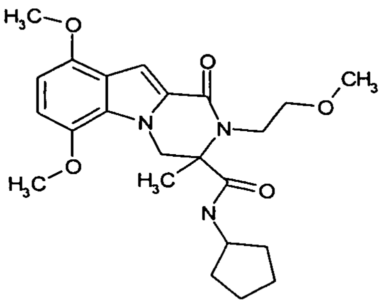 | 429,5206 | 430 |
| 112 | 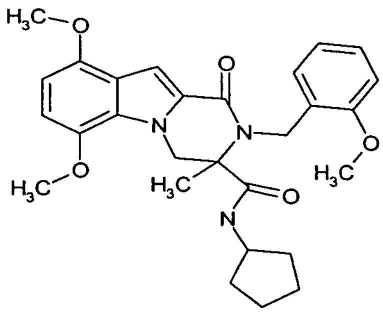 | 491,5923 | 492 |
| 113 | 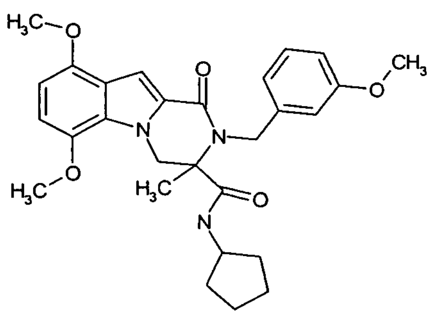 | 491,5923 | 492 |

(continued)

| | Table 1.10(1) | | |
|---|---|---|---|
| № | Formula | Mol. w. | LC MS, *m*/*z*(M+1), 1H NMR |
| 114 | 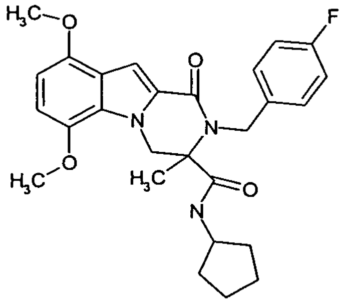 | 479,5563 | 480 |
| 115 | 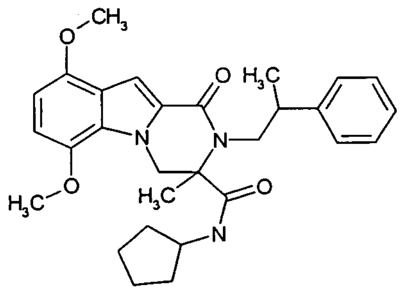 | 489,6200 | 490 |
| 116 | 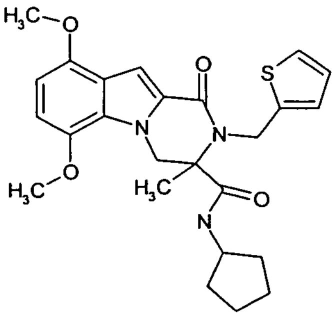 | 467,5916 | 468 |
| 117 | 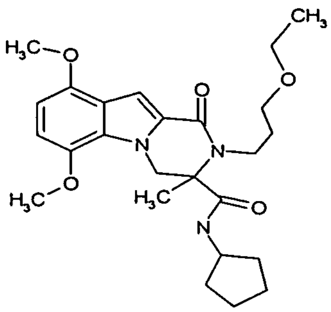 | 457,5748 | 458 |
| 118 | 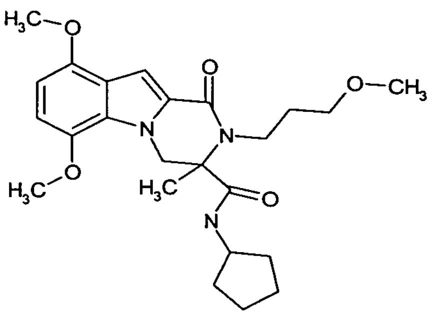 | 443,5477 | 444 |

(continued)

| | Table 1.10(1) | | |
|---|---|---|---|
| № | Formula | Mol. w. | LC MS, *m*/*z*(M+1), 1H NMR |
| 119 | 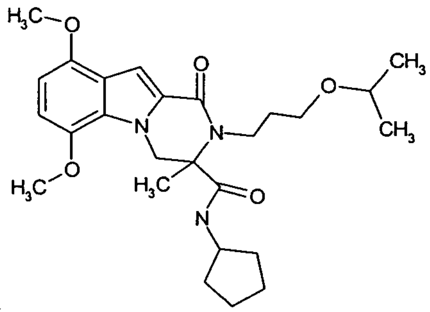 | 471,6019 | 472 |
| 120 | 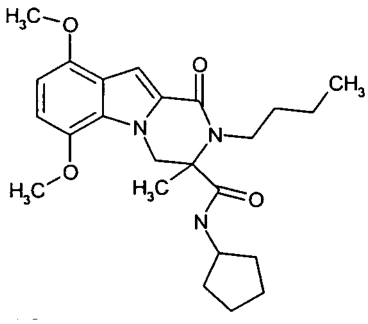 | 427,5483 | 428 |
| 121 | 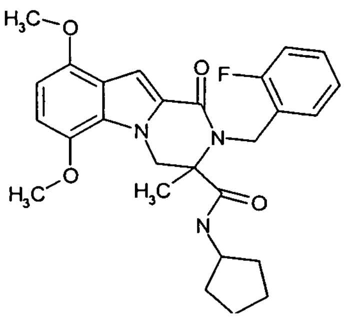 | 479,5563 | 480 |
| 122 | 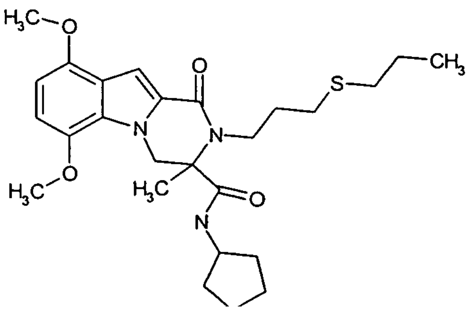 | 487,6665 | 488 |

(continued)

| | Table 1.10(1) | | |
|---|---|---|---|
| № | Formula | Mol. w. | LC MS, *m*/*z*(M+1), 1H NMR |
| 123 | 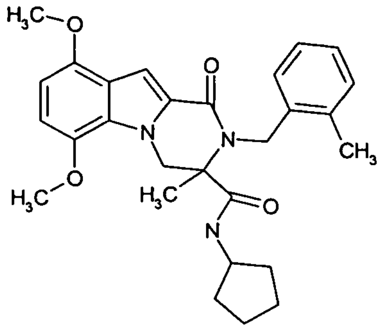 | 475,5929 | 476 |
| 124 | 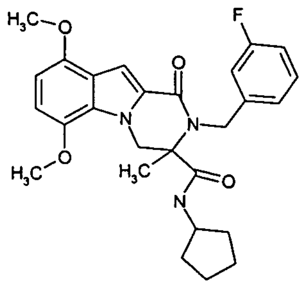 | 479,5563 | 480 |
| 125 | 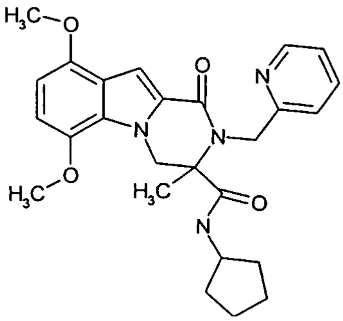 | 462,5534 | 463 |
| 126 | 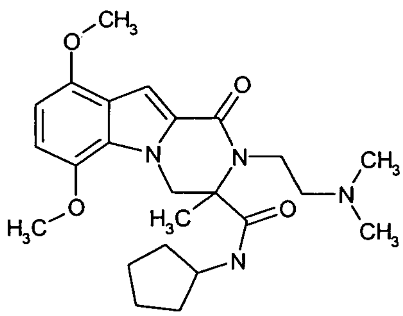 | 442,5630 | 443 |
| 127 | 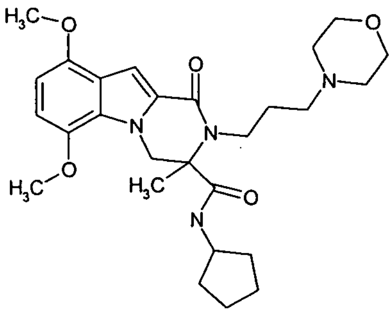 | 498,6277 | 499 |

(continued)

| | Table 1.10(1) | | |
|---|---|---|---|
| № | Formula | Mol. w. | LC MS, *m*/*z*(M+1), 1H NMR |
| 128 | 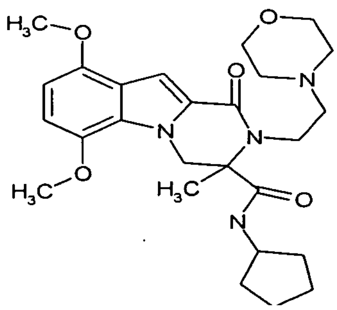 | 484,6006 | 485 |
| 129 | 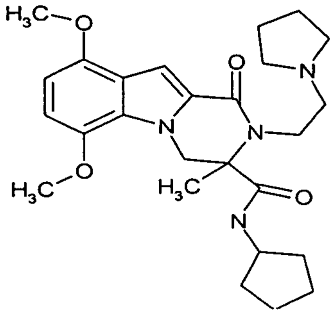 | 468,6012 | 469 |
| 130 | 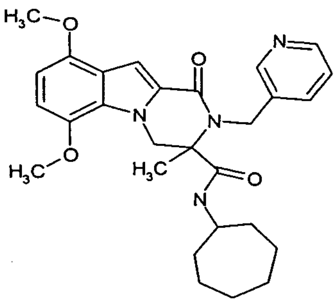 | 490,6076 | 491 |
| 131 | 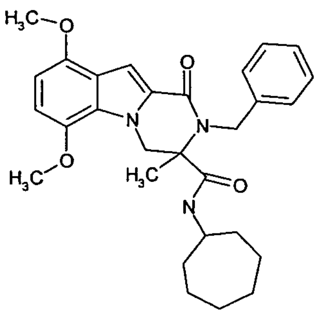 | 489,6200 | 490 |
| 132 | 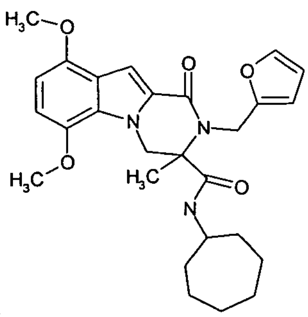 | 479,5812 | 480 |

(continued)

| | Table 1.10(1) | | |
|---|---|---|---|
| № | Formula | Mol. w. | LC MS, *m*/*z*(M+1), 1H NMR |
| 133 | 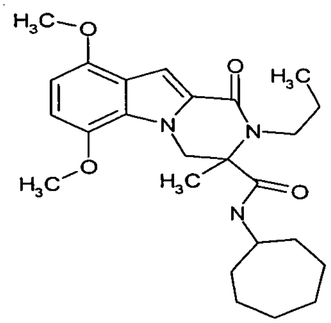 | 441,5754 | 442 |
| 134 | 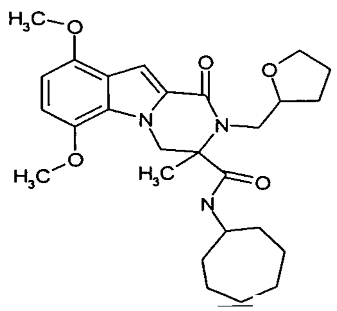 | 483,6130 | 484 |
| 135 | 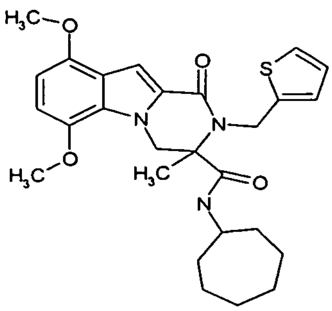 | 495,6458 | 496 |
| 136 | 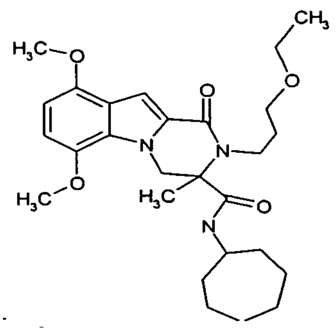 | 485,6290 | 486 |
| 137 | 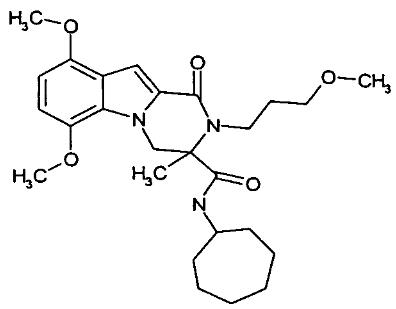 | 471,6019 | 472 |

(continued)

| | Table 1.10(1) | | |
|---|---|---|---|
| № | Formula | Mol. w. | LC MS, *m*/*z*(M+1), 1H NMR |
| 138 | 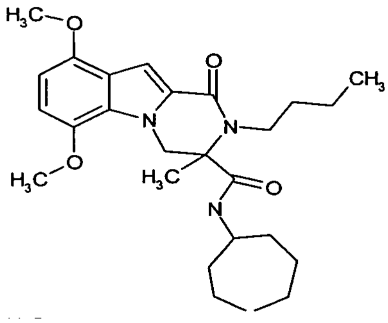 | 455,6025 | 456 |
| 139 | 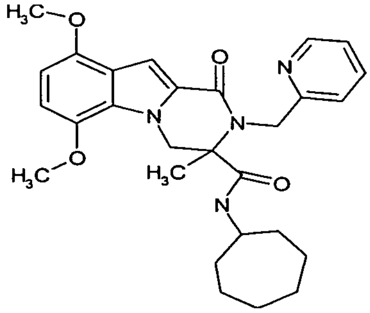 | 490,6076 | 491 |
| 140 | 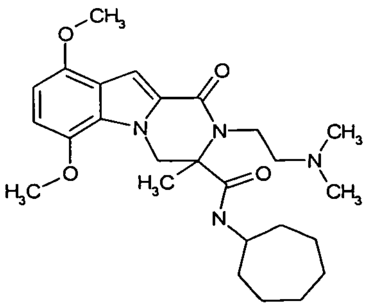 | 470,6172 | 471 |
| 141 | 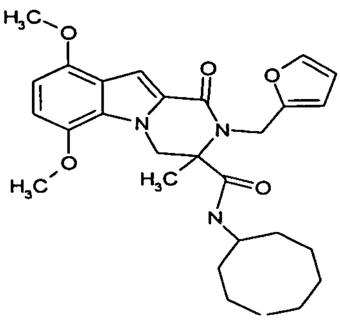 | 493,6083 | 494 |
| 142 | 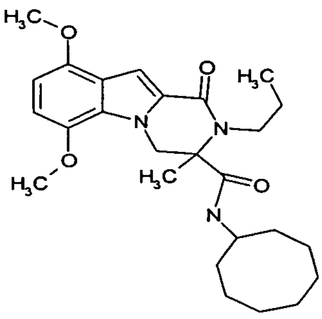 | 455,6025 | 456 |

(continued)

| | Table 1.10(1) | | |
|---|---|---|---|
| № | Formula | Mol. w. | LC MS, *m/z*(M+1), 1H NMR |
| 143 | 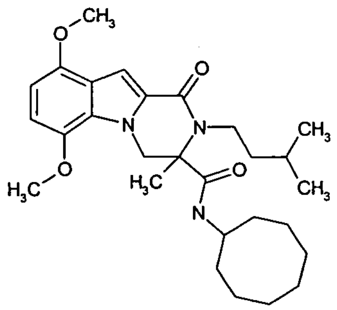 | 483,6567 | 484 |
| 144 | 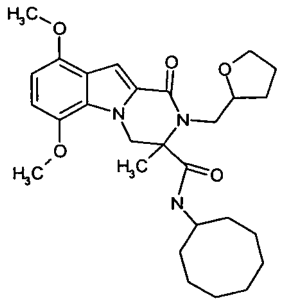 | 497,6401 | 498 |
| 145 | 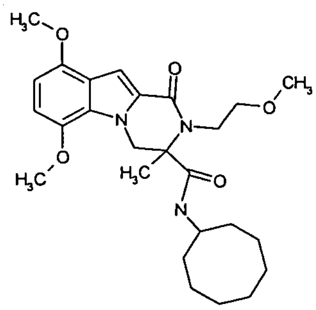 | 471,6019 | 472 |
| 146 | 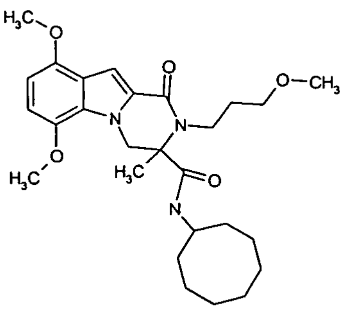 | 485,6290 | 486 |

(continued)

| | Table 1.10(1) | | |
|---|---|---|---|
| № | Formula | Mol. w. | LC MS, *m/z*(M+1), 1H NMR |
| 147 | 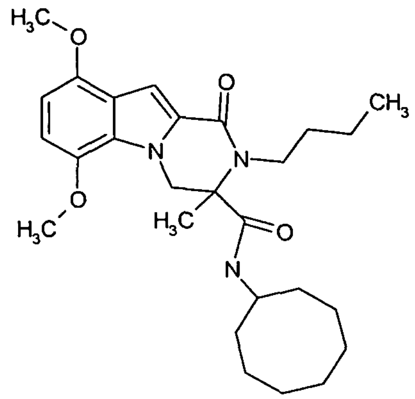 | 469,6296 | 470 |
| 148 | 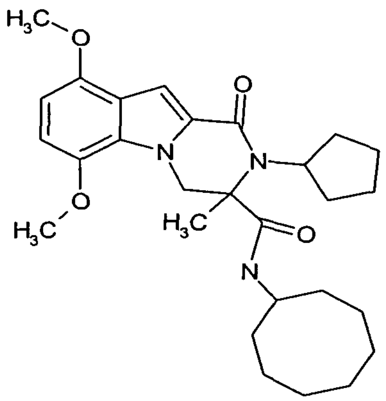 | 481,6407 | 482 |
| 149 | 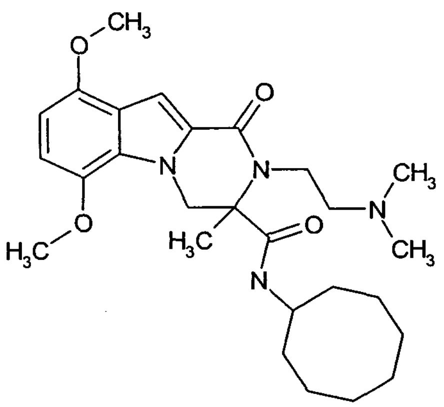 | 484,6443 | 485 |

| | Table 1.10(2) | | |
|---|---|---|---|
| № | Formula | Mol. w. | LC MS, *m/z* (M+1), 1H NMR |
| 1 | 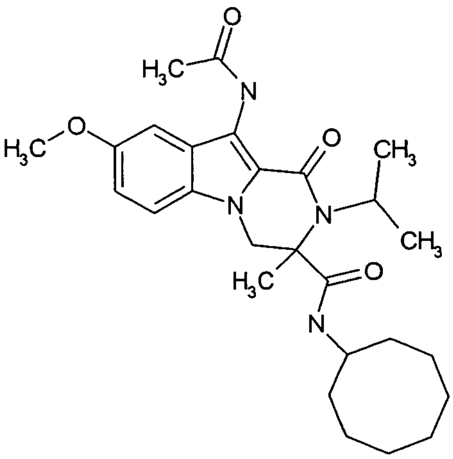 | 482,6283 | LC MS, m/z: 483 (M+1). 1H NMR: s. 1.51 (3H, CH3 at C3 of 1,2,3,4-tetrahydropyrazino[1,2-a]indolic ring), d. 1.57 (J = 6.60 Hz, 6H, CH3 of isopropylic group), m. 1.40 - 1.80 (14H, CH of cyclooctylic ring), s. 2.22 (3H, CH3 NAc), m. 3.26 (J = 6.60 Hz, 1H, CH of isopropylic group), s. 3.82 (3H, OCH3 at C8 of 1,2,3,4-tetrahydropyrazino[1,2 a]indolic ring), m. 3.85 - 3.94 (1H, CH at C1 of cyclooctylic ring), d. 4.15 (J = 12.00 Hz, 1H, CH at C4 of 1,2,3,4-tetrahydropyrazino[1,2-a]indolic ring), d. 4.29 (J = 12.00 Hz, 1H, CH at C4 of 1,2,3,4-tetrahydropyrazino[1,2-a]indolic ring), dd. 6.88 (JH6H7 = 8.95 Hz, JH7H9 = 3.00 Hz, 1H, CH at C7 of 1,2,3,4-tetrahydropyrazino [1,2-a]indolic ring), d. 7.21 (JH6H7 = 8.95 Hz, 1H, CH at C6 of 1,2,3,4-tetrahydropyrazino [1,2-a]indolic ring), d. 7.64 (JH7H9 = 3.00 Hz, 1H, CH at C9 of 1,2,3,4-tetrahydropyrazino [1,2-a]indolic ring), d. 7.95 (J = 8.00 Hz, 1H, NH at cyclooctylic ring), s. 9.93 (1H, NH ofNHAc) |
| 2 | 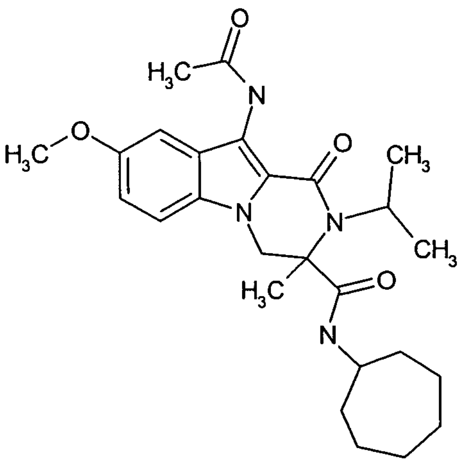 | 468,6012 | LC MS, m/z: 469 (M+1). 1H NMR: s. 1.51 (3H, CH3 at C3 of 1,2,3,4-tetrahydropyrazino[1,2-a]indolic ring), d. 1.56 (J = 6.2 Hz, 6H, CH3 of isopropylic group), m. 1.4 - 1.8 (12H, CH of cycloheptylic ring), s. 2.21 (3H, CH3 NAc), m. 3.25 (J = 6.2 Hz, 1H, CH of isopropylic group), s. 3.81 (3H, OCH3 at C8 of 1,2,3,4-tetrahydropyrazino[1,2 a]indolic ring), m. 3.75 - 3.89 (1H, CH at C1 of cycloheptylic ring), d. 4.17 (J = 11.75 Hz, 1H, CH at C4 of 1,2,3,4-tetrahydropyrazino[1,2-a]indolic ring), d. 4.28 (J = 11.75 Hz, 1H, CH at C4 of 1,2,3,4-tetrahydropyrazino[1,2-a]indolic ring), dd. 6.88 (JH6H7 = 9.35 Hz, JH7H9 = 2.00 Hz, 1H, CH at C7 of 1,2,3,4-tetrahydropyrazino[1,2-a]indolic ring), d. 7.23 (JH6H7 = 9.35 Hz, 1H, CHat C6 of 1,2,3,4-tetrahydropyrazino[1,2-a]indolic ring), d. 7.61 (JH7H9 = 2.00 Hz, 1H, CH at C9 of 1,2,3,4-tetrahydropyrazino[1,2-a]indolic ring), d. 8.01 (J = 8.1 Hz, 1H, NH at cycloheptylic ring), s. 9.92 (1H, NH ofNHAc) |

(continued)

| | Table 1.10(2) | | |
|---|---|---|---|
| № | Formula | Mol. w. | LC MS, *m/z* (M+1), 1H NMR |
| 3 | 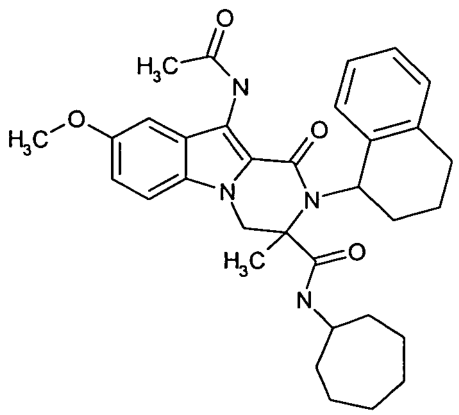 | 556,7112 | LC MS, m/z: 557 (M+1). 1H NMR: m. 1.12 -1.90 (12H, CH of cyclohexylic or cycloheptylic ring), s. 1.81 (3H, CH3 at C3 of 1,2,3,4-tetrahydropyrazino[1,2-a]indolic ring), s. 2.11 (3H, CH3 NAc), m. 2.00 - 2.26 (2H, CH of cyclohexylic or cycloheptylic ring), m. 2.66 - 2.83 (2H CH of cyclohexylic or cycloheptylic ring), m. 2.83 - 3.04 (2H CH of cyclohexylic or cycloheptylic ring), m. 3.61 -3.72 (1H, CH at C1 of cycloheptylic ring), s. 3.81 (3H, OCH3 at C8 of 1,2,3,4-tetrahydropyrazino[1,2-a]indolic ring), d. 4.23 (J = 12.10 Hz, 1H, CH at C4 of 1,2,3,4-tetrahydropyrazino[1,2-a]indolic ring), m. 4.50 - 4.60 (1H, CH at C1 of cyclohexylic ring), d. 4.87 (J = 12.10 Hz, 1H, CH at C4 of 1,2,3,4-tetrahydropyrazino[1,2-a]indolic ring), br. d. 6.90 (JH6H7 = 8.80 Hz, JH7H9 ~ 2.00 Hz, 1H, CH at C7 of 1,2,3,4-tetrahydropyrazino [1,2-a]indolic ring), m. 6.96 - 7.06 (3H, CH at phenylic ring), m. 7.10 7.15 (1H, CH at phenylic ring), d. 7.22 (JH6H7 = 8.80 Hz, 1H, CH at C6 of 1,2,3,4-tetrahydropyrazino[1,2-a]indolic ring), d. 7.44(JH7H9 ~ 2.00 Hz, 1H, CH at C9 of 1,2,3,4-tetrahydropyrazino[1,2-a]indolic ring), |
| 4 | 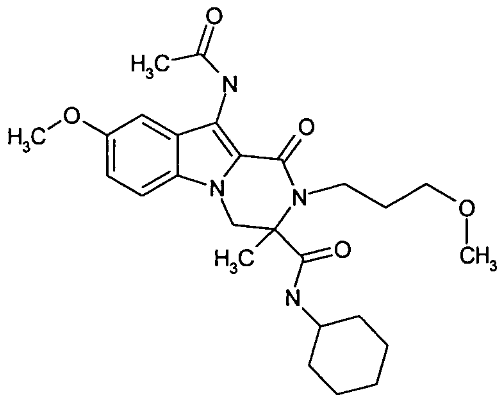 | 484,6006 | 485 |
| 5 | 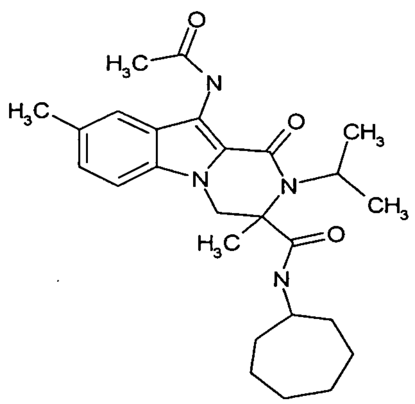 | 452,6018 | 453 |

(continued)

| | Table 1.10(2) | | |
|---|---|---|---|
| № | Formula | Mol. w. | LC MS, *m/z* (M+1), 1H NMR |
| 6 | 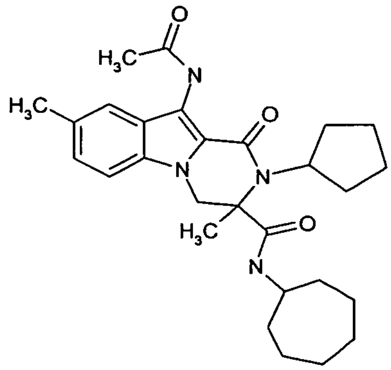 | 478,6401 | 479 |
| 7 | 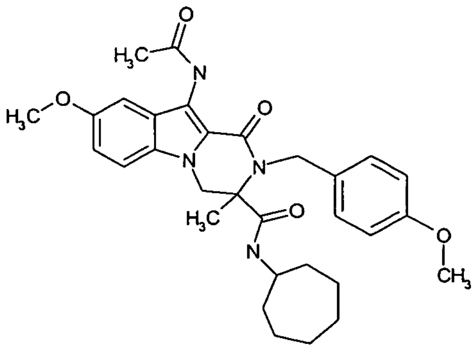 | 546,6723 | 547 |
| 8 | 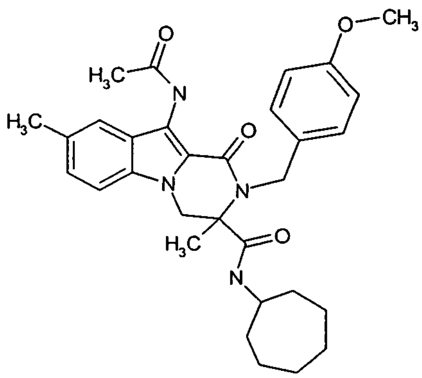 | 530,6729 | 531 |
| 9 | 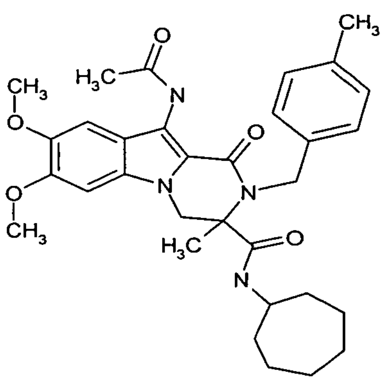 | 560,6994 | LC MS, m/z: 561 (M+1). 1H NMR: m. 1.10 - 1.71 (12H, CH of cycloheptylic ring), s. 1.49 (3H, CH3 at C3 of 1,2,3,4-tetrahydropyrazino [1,2-a]indol-1-onic ring), s. 2.19 (3H, CH3 of NHCOCH3), s. 2.32 (3H, CH3 at C4 of CH2C6H4 -group), m. 3.55 - 3.67 (1H, CH at C1 of cycloheptylic rin... |

(continued)

| | Table 1.10(2) | | |
|---|---|---|---|
| № | Formula | Mol. w. | LC MS, *m/z* (M+1), 1H NMR |
| 10 | 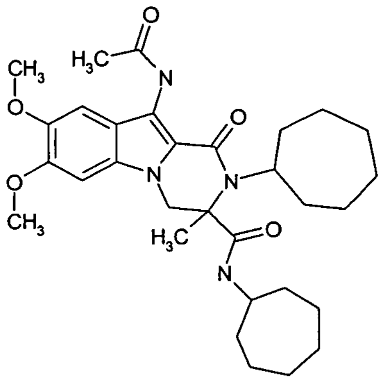 | 552,7201 | LC MS, m/z: 553 (M+1). 1H NMR: m. 1.25 - 2.06 (22H, CH of cycloheptylic rings), s. 1.47 (3H, CH3 at C3 of 1,2,3,4-tetrahydropyrazino [1,2-a]indol-1-onic ring), s. 2.20 (3H, CH3 of NHCOCH3), m. 2.38 - 2.55 (2H - 1H CH at C1 and 1H CH of cycloheptylic rings), m. 2.76 - 2.94 (1H, CH a... |
| 11 | 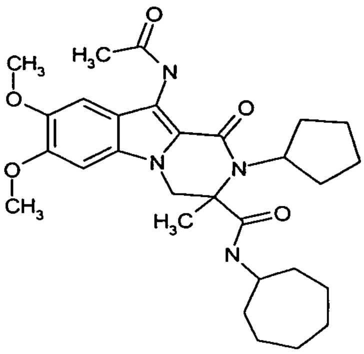 | 524,666 | LC MS, m/z: 525 (M+1). 1H NMR: m. 1.16 - 2.03 (20H, CH of cycloheptylic and cyclopentylic ring), s. 1.48 (3H, CH3 at C3 of 1,2,3,4-tetrahydropyrazino[1,2-a]indol-1 onic ring), s. 2.04 (3H, CH3 of NHCOCH3), s. 3.82 (3H, CH3 at OCH3 at C5 orC6 of indolic ring), s. 3.85 (3H, CH3 at O... |
| 12 | 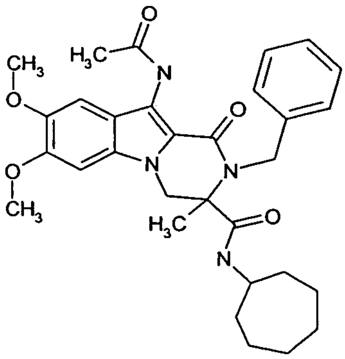 | 546,6723 | LC MS, m/z: 547 (M+1). 1H NMR: m. 1.12 - 1.70 (12H, CH of cycloheptylic ring), s. 1.51 (3H, CH3 at C3 of 1,2,3,4-tetrahydropyrazino [1,2-a]indol-1-onic ring), s. 2.18 (3H, CH3 of NHCOCH3), s. 3.82 (3H, CH3 at OCH3 at C5 orC6 of indolic ring), m. 3.56 - 3.69 (1H, CH at C1 of cyclohe... |
| 13 | 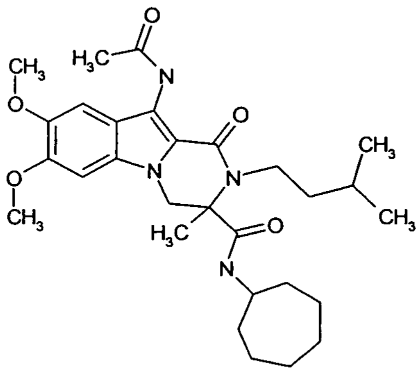 | 526,6819 | LC MS, m/z: 527 (M+1). 1 H NMR: d. 0.98 (J = 6.00 Hz, 6H, 2 CH3 groups of i-butylic group), m. 1.3 - 1.75 (15H - 12H CH of cycloheptylic ring and 3H CH and CH2 of i-butylic group), s. 1.57 (3H, CH3 at C3 of 1,2,3,4-tetrahydropyrazino[1,2-a]indol-1-onic ring), s. 2.19 (3H, CH3 of NH... |

(continued)

| | Table 1.10(2) | | |
|---|---|---|---|
| № | Formula | Mol. w. | LC MS, *m/z* (M+1), 1H NMR |
| 14 | 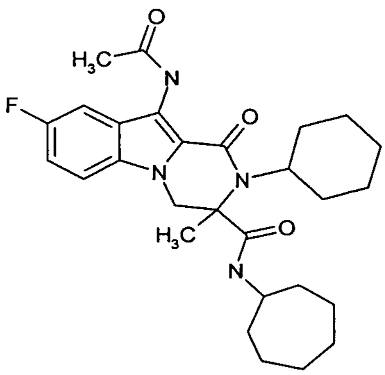 | 496,6305 | LC MS, m/z: 4497 (M+1). 1H NMR: m. 1.12 - 2.00 (22H, CH2 of cyclohexylic and cycloheptylic rings), s. 1.49 (3H, CH3 at C3 of 1,2,3,4-tetrahydropyrazino[1,2-a]indol-1-onic ring), s. 2.21 (3H, CH3 of NHCOCH3), m. 2.69 - 2.80 (1 H, CH at C1 of cyclohexylic ring), 3.80 - 3.91 (1H, CH at... |
| 15 | 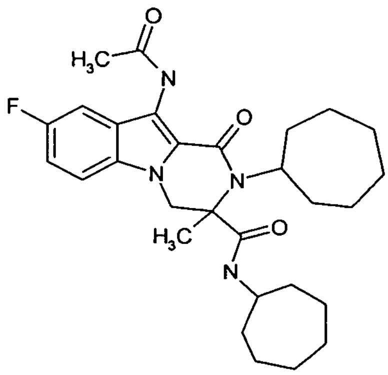 | 510,6576 | LC MS, m/z: 511 (M+1). 1H NMR: m. 1.23 - 1.90 (22H, CH2 of cycloheptylic rings), s. 1.49 (3H, CH3 at C3 of 1,2,3,4-tetrahydropyrazino [1,2-a]indol-1-onic ring), m. 1.93 - 2.05 (1H CH of cycloheptylic ring), s. 2.21 (3H, CH3 of NHCOCH3), m. 2.40 - 2.54 (1H CH of cycloheptylic ring),... |
| 16 | 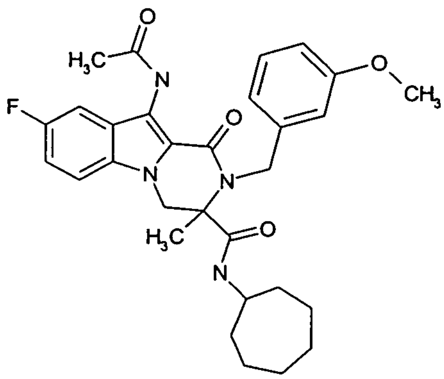 | 534,6363 | LC MS, m/z: 535 (M+1). 1H NMR: m. 1.10 - 1.67 (12H, CH2 of cycloheptylic rings), s. 1.58 (3H, CH3 at C3 of 1,2,3,4-tetrahydropyrazino [1,2-a]indol-1-onic ring), s. 2.20 (3H, CH3 of NHCOCH3), m. 3.54 - 3.56 (1H, CH at C1 of cycloheptylic ring of CONHC7H13), s. 3.779 and s. 3.783 (3... |

| | Table 1.10(3) | | |
|---|---|---|---|
| № | Formula | Mol. w. | LC MS, *m/z* (M+1), 1H NMR |
| 1 | 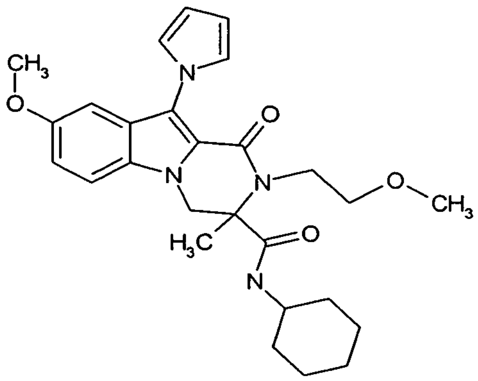 | 478,59643 | 479 |
| 2 | 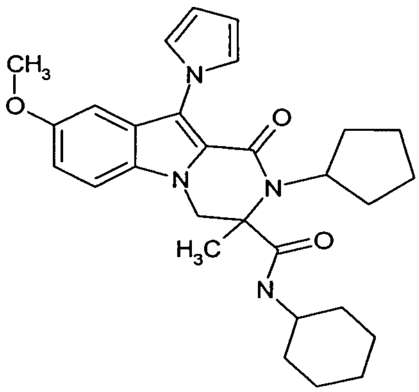 | 488,63527 | 489 |
| 3 | 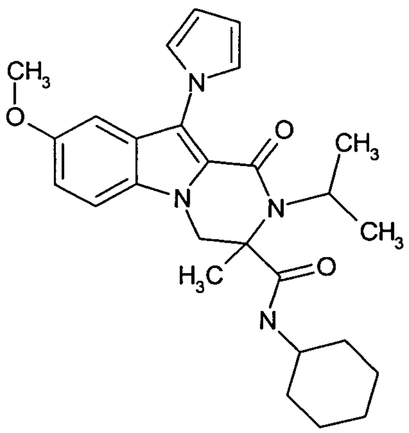 | 462,59703 | 463 |

(continued)

| | Table 1.10(3) | | |
|---|---|---|---|
| № | Formula | Mol. w. | LC MS, *m/z* (M+1), 1H NMR |
| 4 | 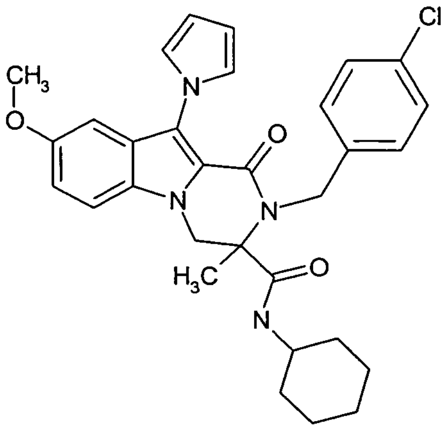 | 545,08666 | 546 |
| 5 | 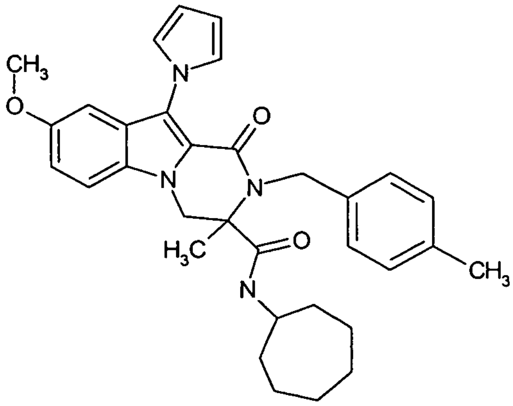 | 538,69581 | 539 |
| 6 | 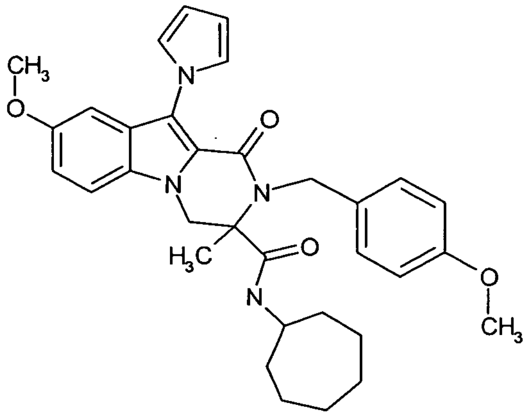 | 554,69521 | 555 |
| 7 | | 506,65061 | 507 |

(continued)

| | Table 1.10(3) | | |
|---|---|---|---|
| № | Formula | Mol. w. | LC MS, *m/z* (M+1), 1H NMR |
| 8 | 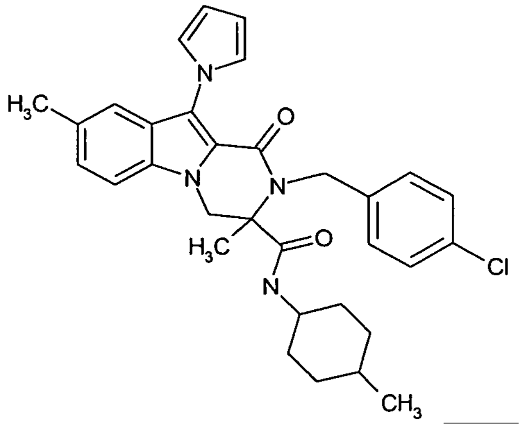 | 543,11435 | LC MS, m/z: 544 (M+1). 1H NMR: m. 0.79 - 1.66 (9H, CH of cyclohexylic ring), d. 0.83 (J = 6.60 Hz, 3H, CH3 at C4 of cyclohexylic ring), s. 1.52 and 1.56 (3H, CH3 at C3 of 1,2,3,4-tetrahydropyrazino[1,2-a]indol-1-onic ring - isomeri c set), s. 2.43 (CH3 at C8 of 1,2,3,4-tetrahydropy... |
| 9 | 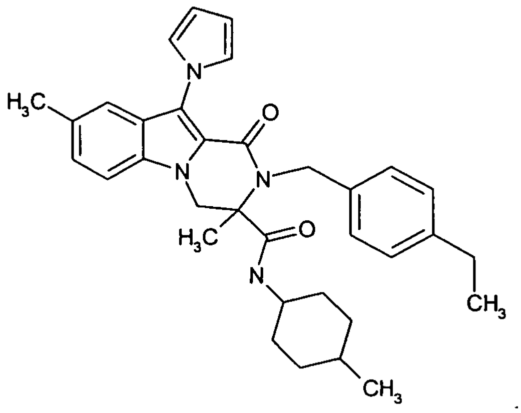 | 536,7235 | LC MS, m/z: 537 (M+1). 1H NMR: d. 0.81 and 0.83 (J = 6.90 Hz, 3H, CH3 at C4 of cyclohexylic ring - isomeric set), m. 0.86 - 1.53 (9H, CH of cyclohexylic ring), t. 1.23 (J = 7.44 Hz, 3H, CH3 of CH2CH3 -group), s. 1.54 and 1.59 (3H, CH3 at C3 of 1,2,3,4-tetrahydropyrazino[1,2-a]ind... |
| 10 | 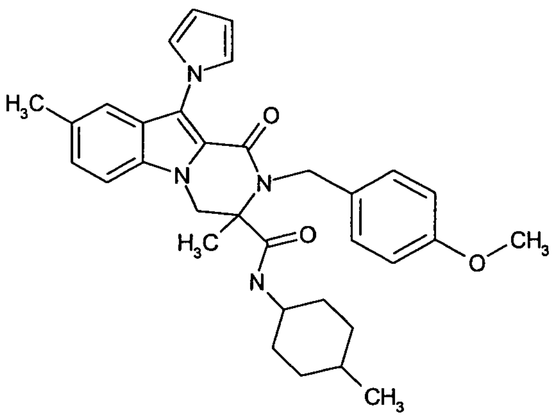 | 538,69581 | LC MS, m/z: 539 (M+1). 1H NMR: d. 0.81 and 0.83 (J = 7.30 Hz, 3H, CH3 at C4 of cyclohexylic ring - isomeric set), m. 0.86 - 1.63 (9H, CH of cyclohexylic ring), s. 1.54 and 1.60 (3H, CH3 at C3 of 1,2,3,4-tetrahydropyrazino[1,2-a]indol-1-onic ring - isomeric set), s. 2.43 (CH3 at C8... |

(continued)

| | Table 1.10(3) | | |
|---|---|---|---|
| № | Formula | Mol. w. | LC MS, *m/z* (M+1), 1H NMR |
| 11 | 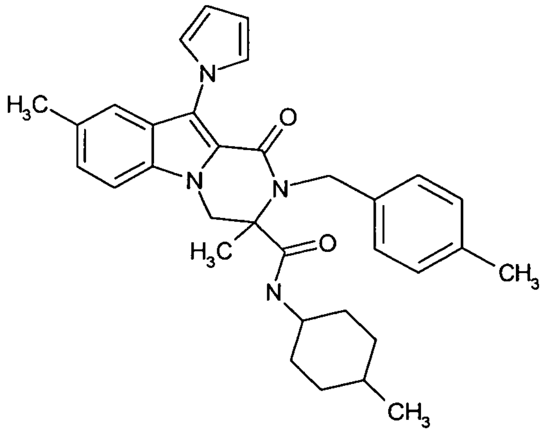 | 522,69641 | LC MS, m/z: 544 (M+1). 1 H NMR: 423d. 0.81 and 0.83 (J = 7.30 Hz, 3H, CH3 at C4 of cyclohexylic ring - isomeric set), m. 0.76 - 1.65 (9H, CH of cyclohexylic ring), s. 1.52 and 1.57 (3H, CH3 at C3 of 1,2,3,4-tetrahydropyrazino[1,2-a]indol-1-onic ring - isomeric set), s. 2.43 (CH3 at C8... |
| 12 | 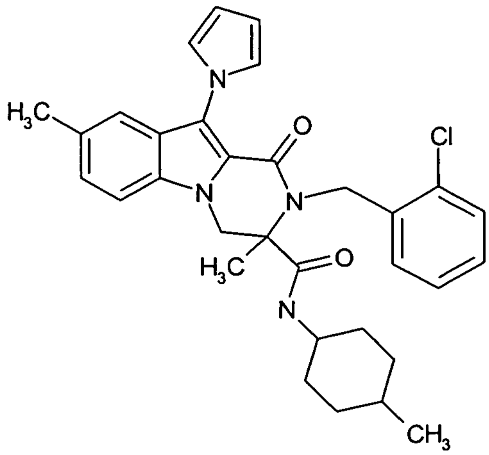 | 543,11435 | LC MS, m/z: 544 (M+1). 1H NMR: d. 0.82 and 0.84 (J = 5.80 Hz, 3H, CH3 at C4 of cyclohexylic ring - isomeric set), m. 0.80 - 1.64 (9H, CH of cyclohexylic ring), s. 1.52 and 1.55 (3H, CH3 at C3 of 1,2,3,4-tetrahydropyrazino[1,2-a]indol-1-onic ring - isomeric set), s. 2.43 (CH3 at C8... |

| | Table 1.11 | | |
|---|---|---|---|
| № | Formula | Mol. w. | LC MS, m/z (M+1) |
| 1 | 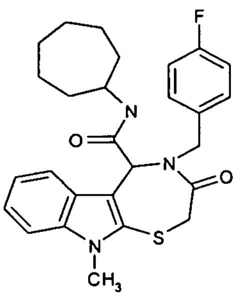 | 479,62145 | 480 |

(continued)

| | Table 1.11 | | |
|---|---|---|---|
| № | Formula | Mol. w. | LC MS, m/z (M+1) |
| 2 | 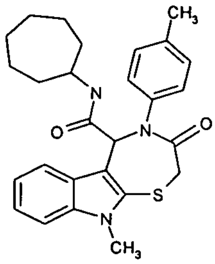 | 461,63102 | 462 |
| 3 | 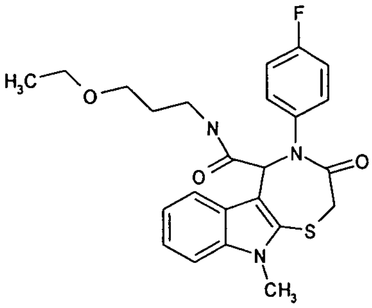 | 455,55552 | 456 |
| 4 | 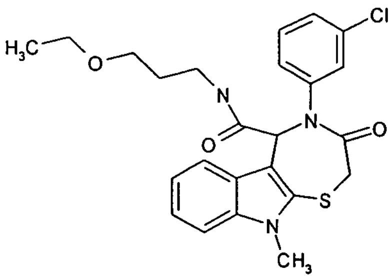 | 472,01012 | 473 |
| 5 | 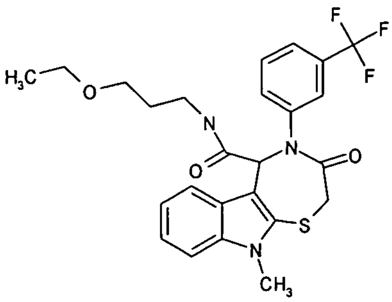 | 505,56347 | 506 |
| 6 | 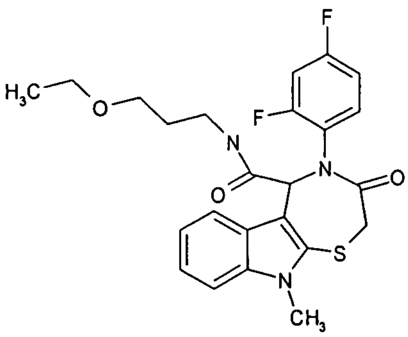 | 473,54595 | 474 |

(continued)

| | Table 1.11 | | |
|---|---|---|---|
| № | Formula | Mol. w. | LC MS, m/z (M+1) |
| 7 | 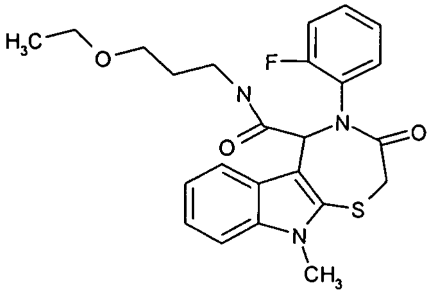 | 455,55552 | 456 |
| 8 | 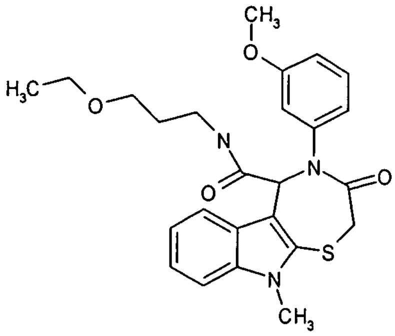 | 467,59158 | 468 |
| 9 | 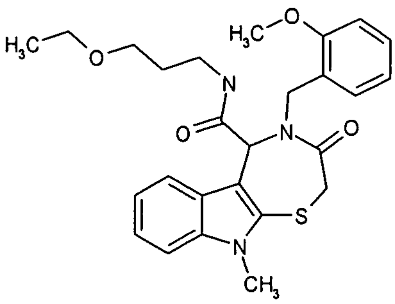 | 481,61867 | 482 |
| 10 | 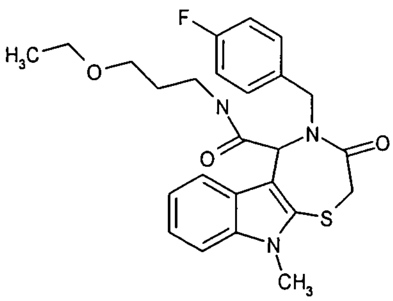 | 469,58261 | 470 |
| 11 | 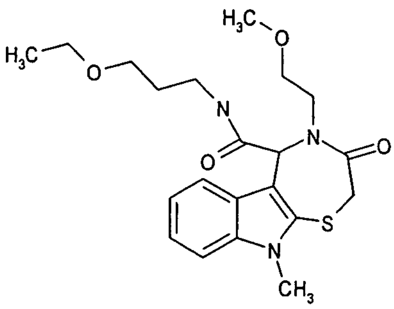 | 419,54698 | 420 |

(continued)

| | Table 1.11 | | |
|---|---|---|---|
| № | Formula | Mol. w. | LC MS, m/z (M+1) |
| 12 | 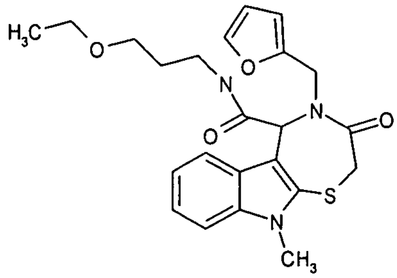 | 441,55334 | 442 |
| 13 | 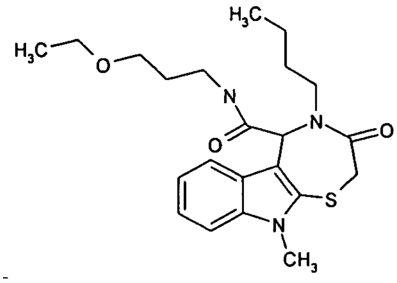 | 417,57467 | 418 |
| 14 | 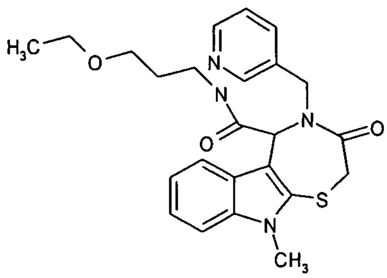 | 452,57976 | 453 |
| 15 | 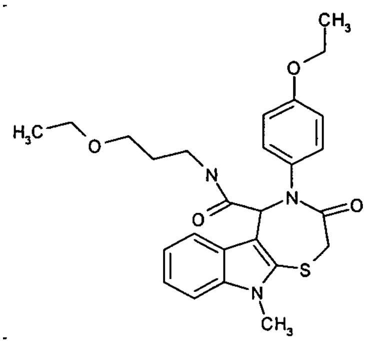 | 481,61867 | 482 |
| 16 | 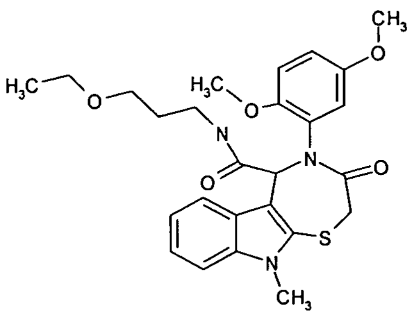 | 497,61807 | 498 |

(continued)

| | Table 1.11 | | |
|---|---|---|---|
| № | Formula | Mol. w. | LC MS, m/z (M+1) |
| 17 | 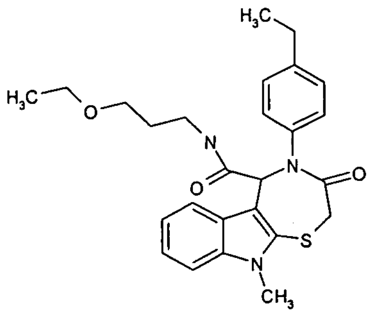 | 465,61927 | 466 |
| 18 | 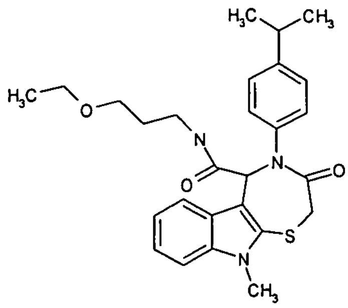 | 479,64636 | 480 |
| 19 | 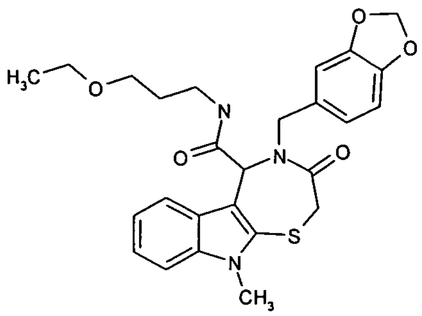 | 495,60213 | 496 |
| 20 | 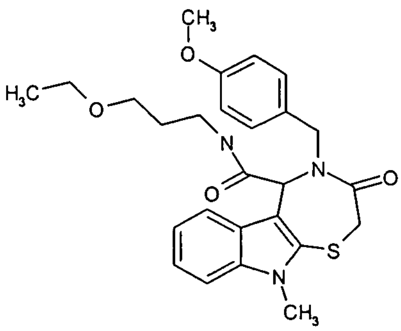 | 481,61867 | 482 |

(continued)

| | Table 1.11 | | |
|---|---|---|---|
| № | Formula | Mol. w. | LC MS, m/z (M+1) |
| 21 | 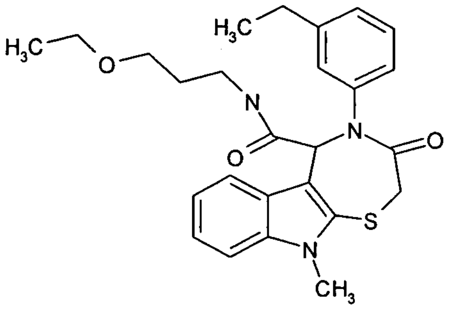 | 465,61927 | 466 |
| 22 | 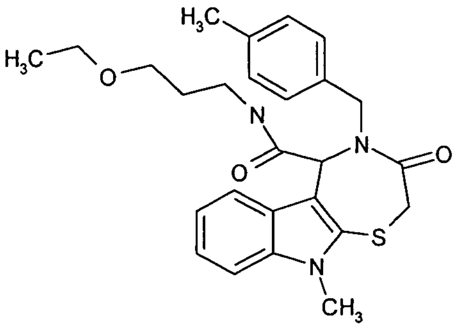 | 465,61927 | 466 |
| 23 | 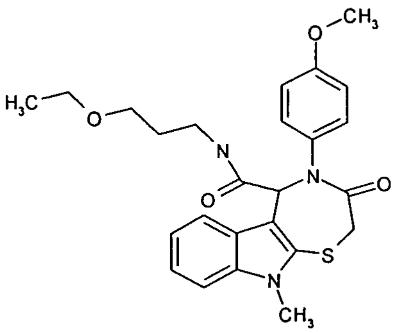 | 467,59158 | 468 |
| 24 | 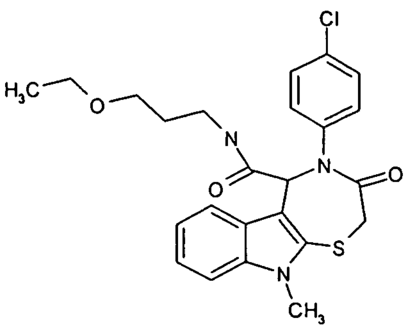 | 472,01012 | 473 |

(continued)

| | Table 1.11 | | |
|---|---|---|---|
| № | Formula | Mol. w. | LC MS, m/z (M+1) |
| 25 | 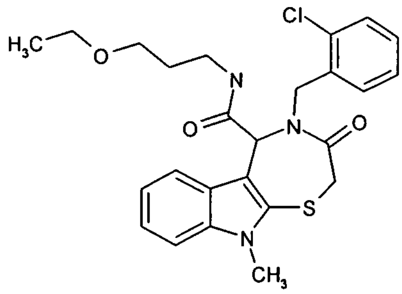 | 486,03721 | 487 |
| 26 | 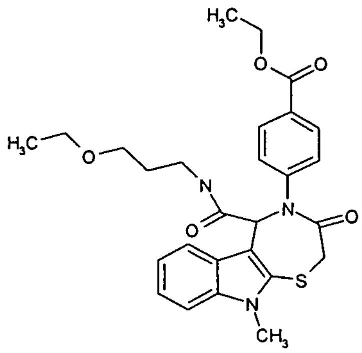 | 509,62922 | 510 |
| 27 | 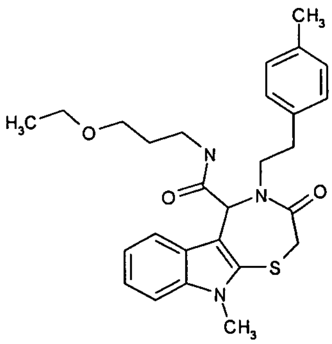 | 479,64636 | 480 |
| 28 | 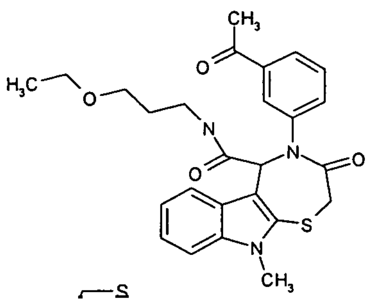 | 479,60273 | 480 |

(continued)

| | Table 1.11 | | |
|---|---|---|---|
| № | Formula | Mol. w. | LC MS, m/z (M+1) |
| 29 | 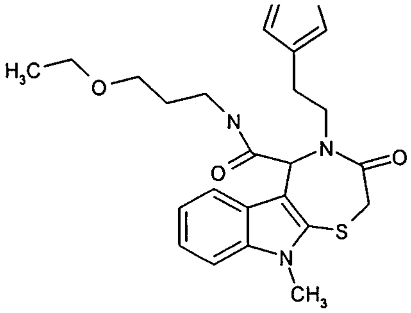 | 471,64503 | 472 |
| 30 | 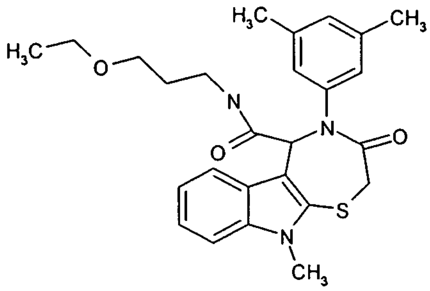 | 465,61927 | 466 |
| 31 | 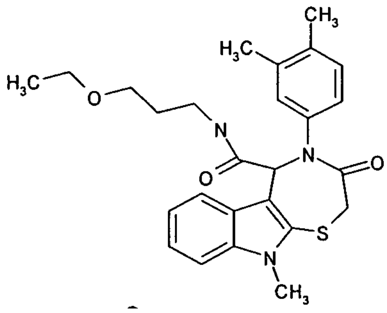 | 465,61927 | 466 |
| 32 | 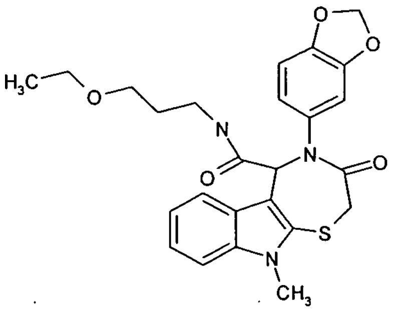 | 481,57504 | 482 |
| 33 | 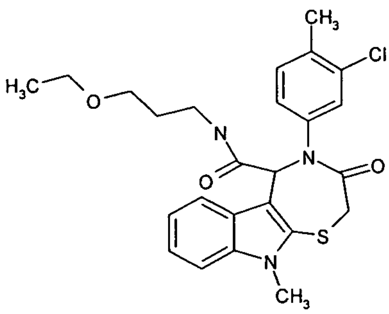 | 486,03721 | 487 |

(continued)

| | Table 1.11 | | |
|---|---|---|---|
| № | Formula | Mol. w. | LC MS, m/z (M+1) |
| 34 | 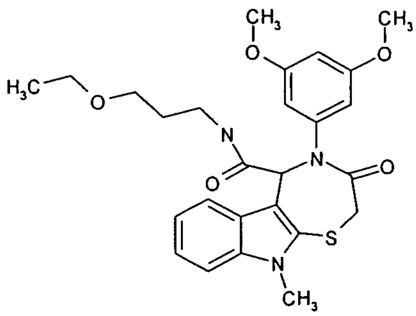 | 497,61807 | 498 |
| 35 | 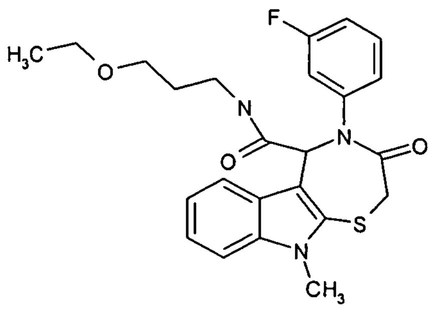 | 455,55552 | 456 |
| 36 | 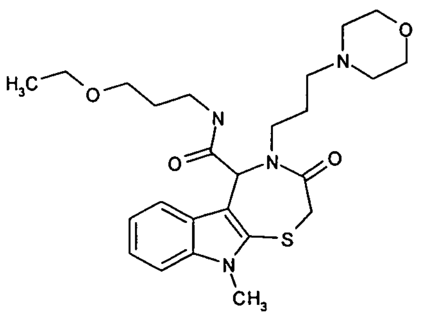 | 488,65407 | 489 |
| 37 | 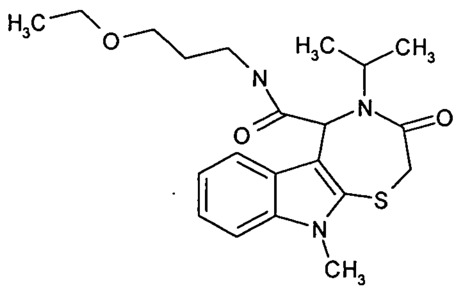 | 403,54758 | 404 |
| 38 | 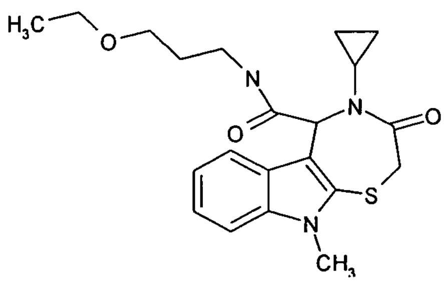 | 401,53164 | 402 |

(continued)

| | Table 1.11 | | |
|---|---|---|---|
| № | Formula | Mol. w. | LC MS, m/z (M+1) |
| 39 | 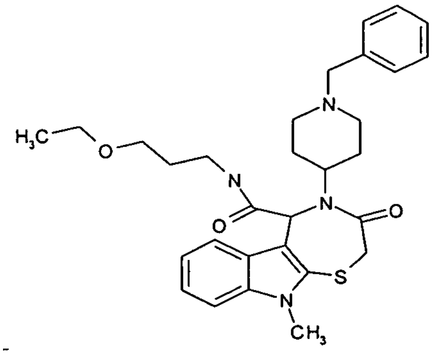 | 534,72636 | 535 |
| 40 | 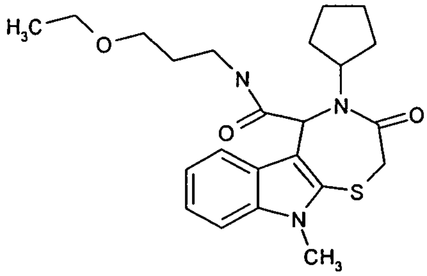 | 429,58582 | 430 |
| 41 | 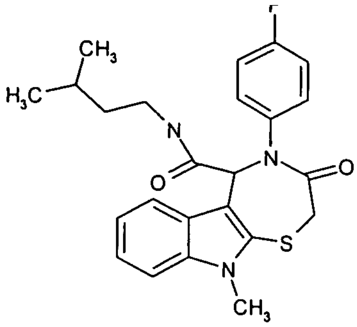 | 439,55612 | 440 |
| 42 | 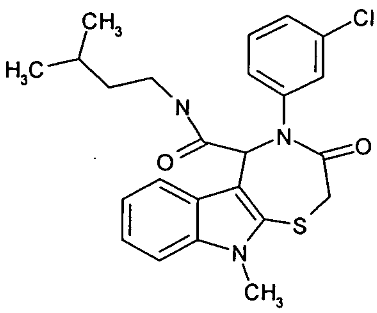 | 456,01072 | 457 |

(continued)

| | Table 1.11 | | |
|---|---|---|---|
| № | Formula | Mol. w. | LC MS, m/z (M+1) |
| 43 | 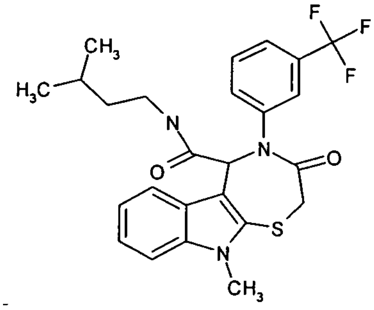 | 489,56407 | 490 |
| 44 | 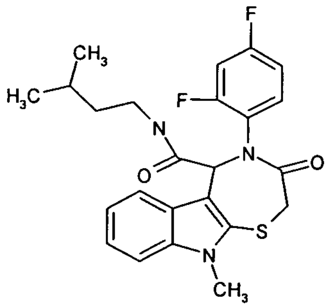 | 457,54655 | 458 |
| 45 | 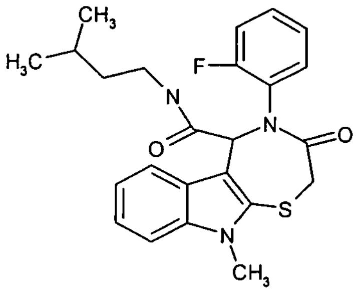 | 439,55612 | 440 |
| 46 | 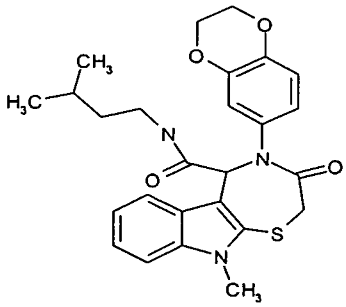 | 479,60273 | 480 |
| 47 | 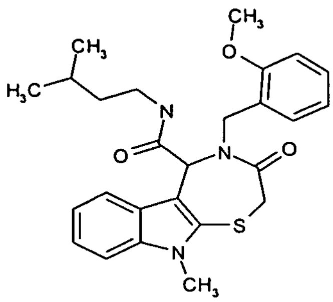 | 465,61927 | 466 |

(continued)

| | Table 1.11 | | |
|---|---|---|---|
| № | Formula | Mol. w. | LC MS, m/z (M+1) |
| 48 | 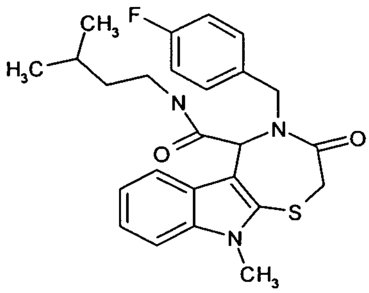 | 453,58321 | 454 |
| 49 | 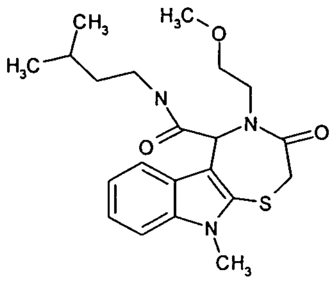 | 403,54758 | 404 |
| 50 | 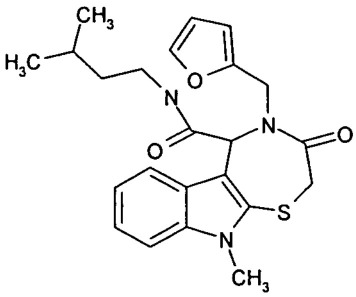 | 425,55394 | 426 |
| 51 | 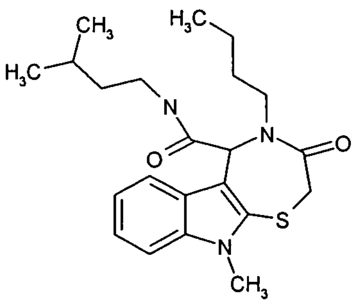 | 401,57527 | 402 |
| 52 | 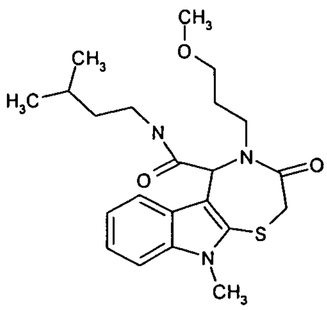 | 417,57467 | 418 |

(continued)

| | Table 1.11 | | |
|---|---|---|---|
| № | Formula | Mol. w. | LC MS, m/z (M+1) |
| 53 | 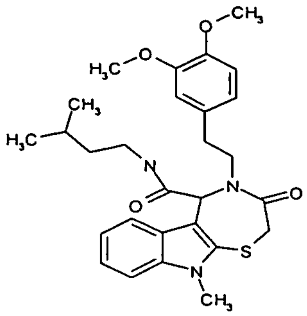 | 509,67285 | 510 |
| 54 | 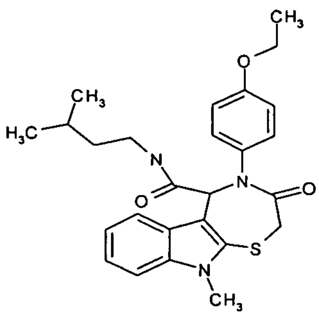 | 465,61927 | 466 |
| 55 | 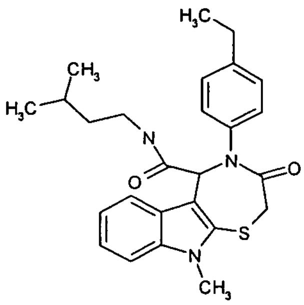 | 449,61987 | 450 |
| 56 | 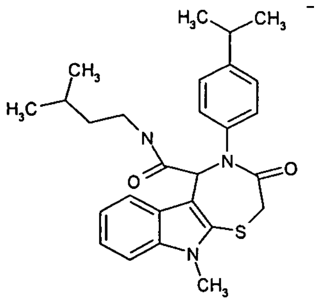 | 463,64696 | 464 |
| 57 | 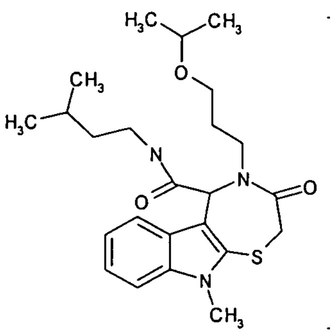 | 445,62885 | 446 |

(continued)

| | Table 1.11 | | |
|---|---|---|---|
| № | Formula | Mol. w. | LC MS, m/z (M+1) |
| 58 | 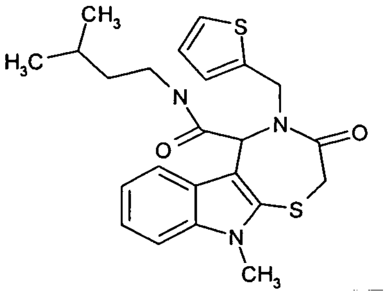 | 441,61854 | 442 |
| 59 | 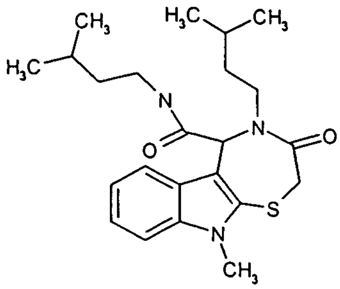 | 415,60236 | 416 |
| 60 | 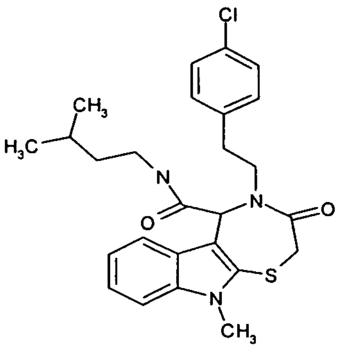 | 484,0649 | 485 |
| 61 | 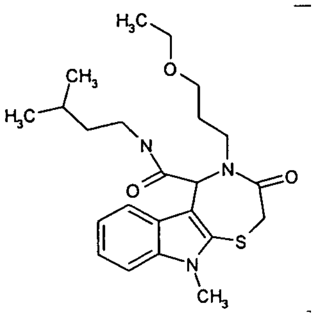 | 431,60176 | 432 |
| 62 | 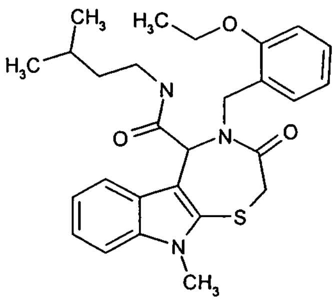 | 479,64636 | 480 |

(continued)

| | Table 1.11 | | |
|---|---|---|---|
| № | Formula | Mol. w. | LC MS, m/z (M+1) |
| 63 | 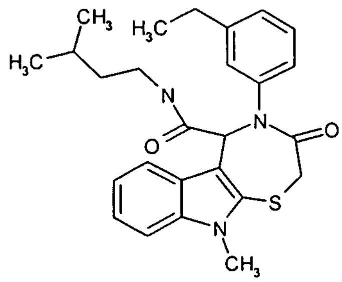 | 449,61987 | 450 |
| 64 | 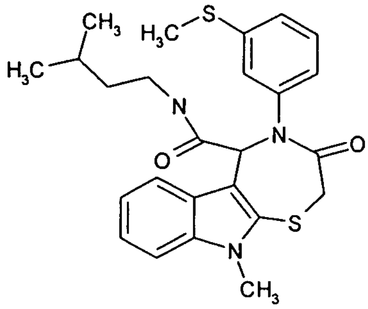 | 467,65678 | 468 |
| 65 | 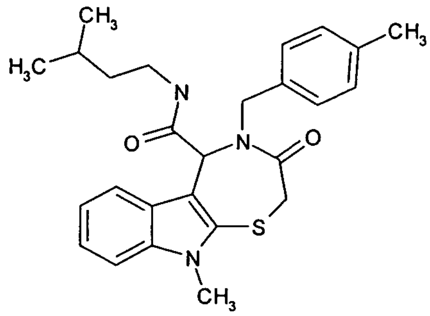 | 449,61987 | 450 |
| 66 | 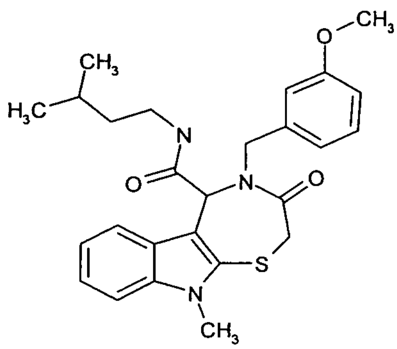 | 465,61927 | 466 |

(continued)

| | Table 1.11 | | |
|---|---|---|---|
| № | Formula | Mol. w. | LC MS, m/z (M+1) |
| 67 | 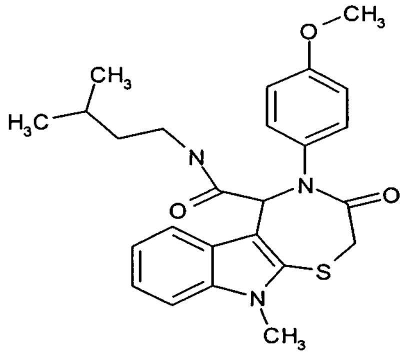 | 451,59218 | 452 |
| 68 | 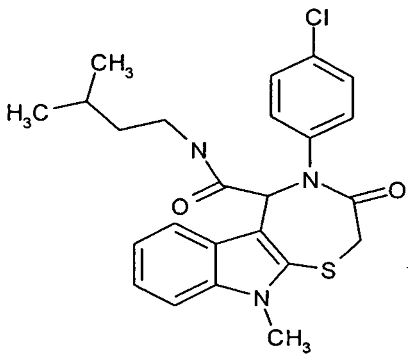 | 456,01072 | 457 |
| 69 | 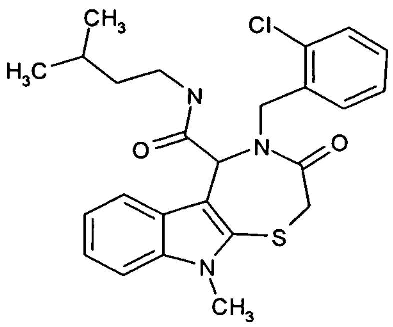 | 470,03781 | 471 |
| 70 | 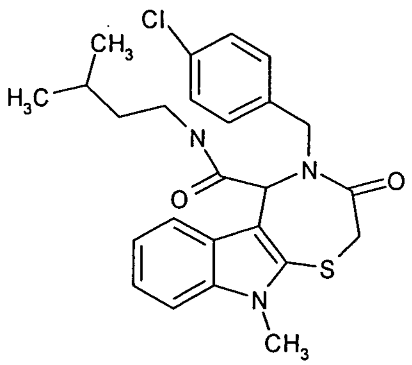 | 470,03781 | 471 |

(continued)

| | Table 1.11 | | |
|---|---|---|---|
| № | Formula | Mol. w. | LC MS, m/z (M+1) |
| 71 | 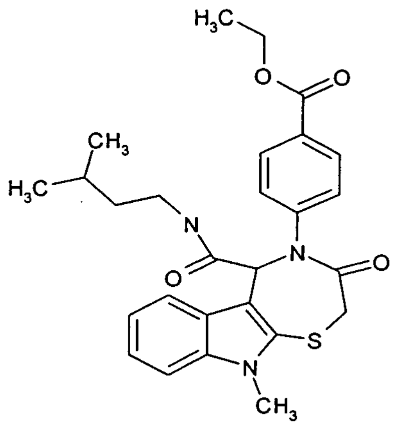 | 493,62982 | 494 |
| 72 | 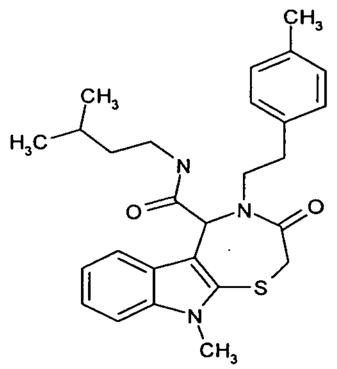 | 463,64696 | 464 |
| 73 | 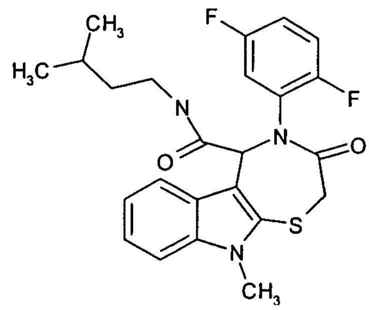 | 457,54655 | 458 |
| 74 | 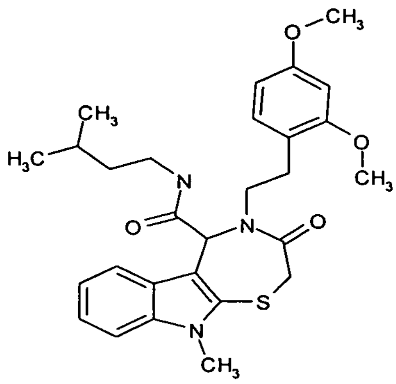 | 509,67285 | 510 |

(continued)

| | Table 1.11 | | |
|---|---|---|---|
| № | Formula | Mol. w. | LC MS, m/z (M+1) |
| 75 | 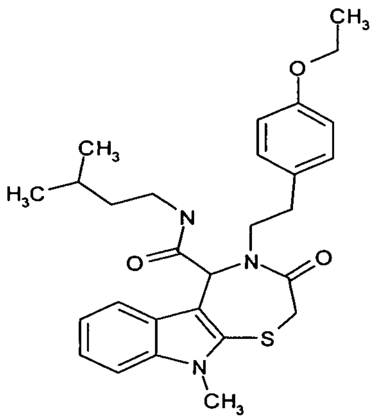 | 493,67345 | 494 |
| 76 | 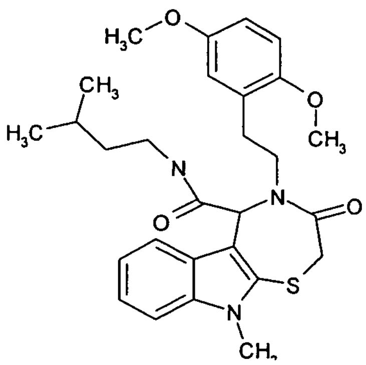 | 509,67285 | 510 |
| 77 | 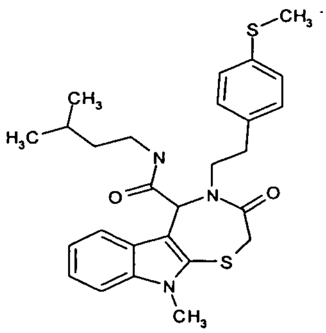 | 495,71096 | 496 |
| 78 | 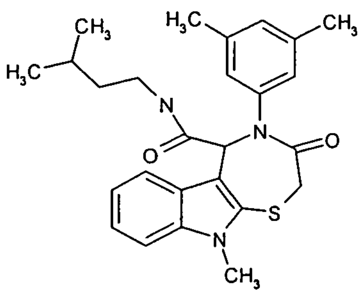 | 449,61987 | 450 |

(continued)

| | Table 1.11 | | |
|---|---|---|---|
| № | Formula | Mol. w. | LC MS, m/z (M+1) |
| 79 | 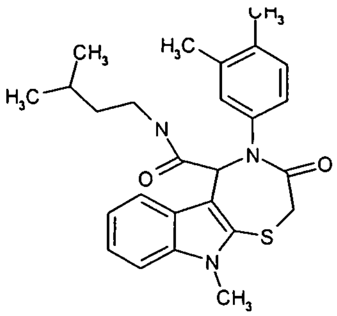 | 449,61987 | 450 |
| 80 | 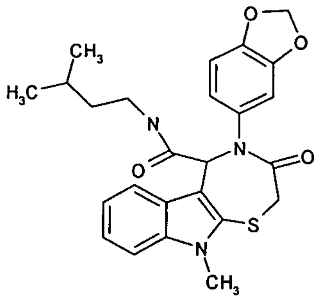 | 465,57564 | 466 |
| 81 | 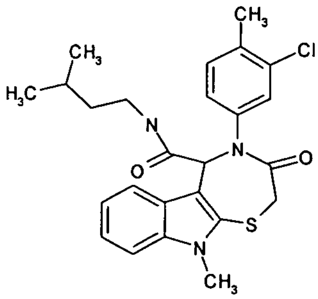 | 470,03781 | 471 |
| 82 | 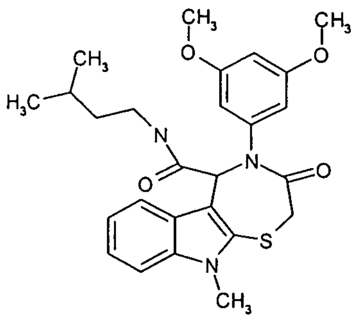 | 481,61867 | 482 |
| 83 | 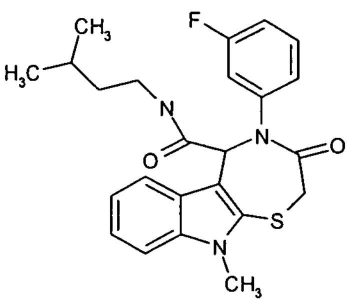 | 439,55612 | 440 |

(continued)

| | Table 1.11 | | |
|---|---|---|---|
| № | Formula | Mol. w. | LC MS, m/z (M+1) |
| 84 | 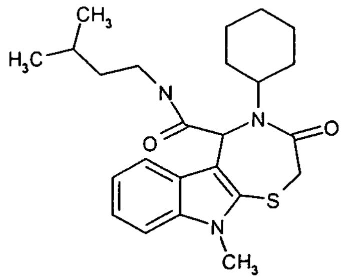 | 427,61351 | 428 |
| 85 | 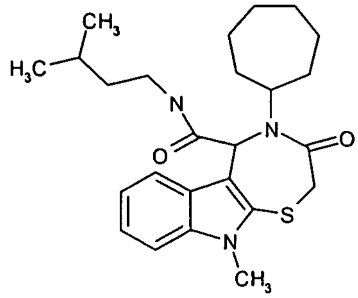 | 441,6406 | 442 |
| 86 | 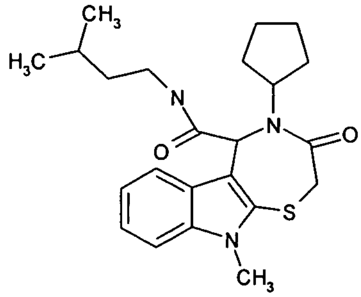 | 413,58642 | 414 |
| 87 | 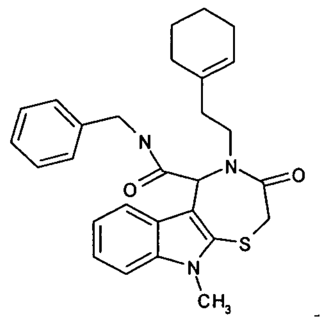 | 473,64217 | 474 |
| 88 | 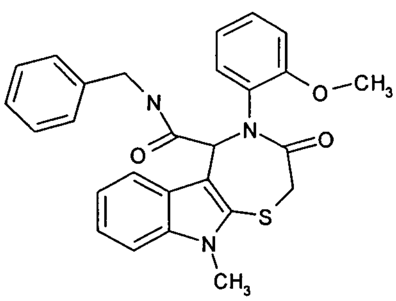 | 471,5826 | 472 |

(continued)

| | Table 1.11 | | |
|---|---|---|---|
| № | Formula | Mol. w. | LC MS, m/z (M+1) |
| 89 | 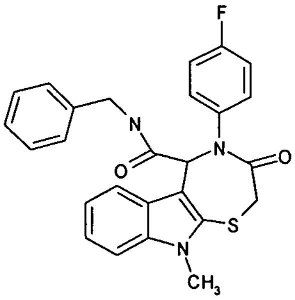 | 459,54654 | 460 |
| 90 | 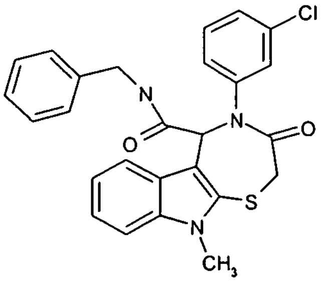 | 476,00114 | 477 |
| 91 | 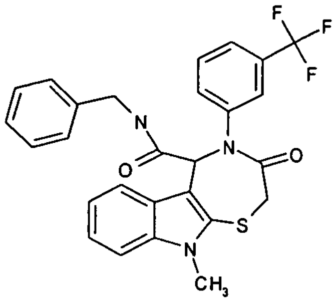 | 509,55449 | 510 |
| 92 | 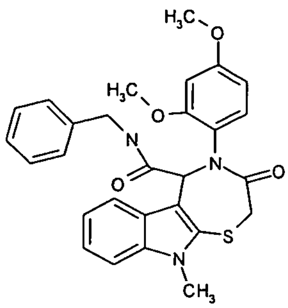 | 501,60909 | 502 |
| 93 | 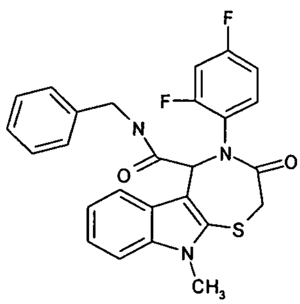 | 477,53697 | 478 |

(continued)

| | Table 1.11 | | |
|---|---|---|---|
| № | Formula | Mol. w. | LC MS, m/z (M+1) |
| 94 | 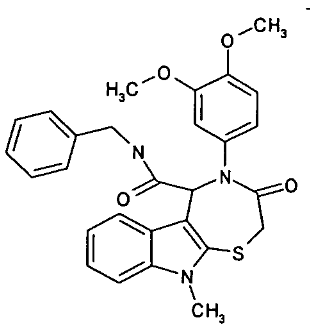 | 501,60909 | 502 |
| 95 | 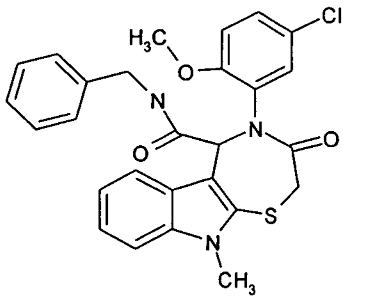 | 506,02763 | 507 |
| 96 | 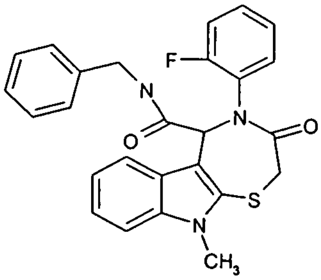 | 459,54654 | 460 |
| 97 | 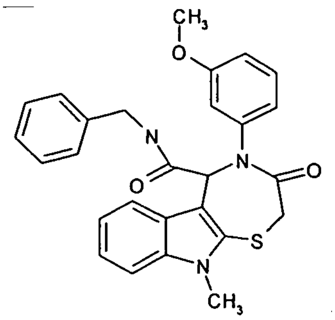 | 471,5826 | 472 |
| 98 | 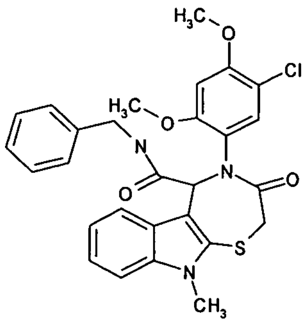 | 536,05412 | 537 |

(continued)

| | Table 1.11 | | |
|---|---|---|---|
| № | Formula | Mol. w. | LC MS, m/z (M+1) |
| 99 | 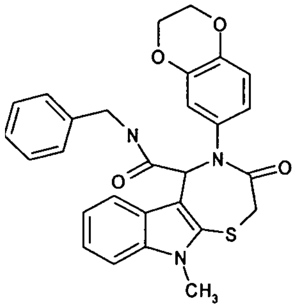 | 499,59315 | 500 |
| 100 | 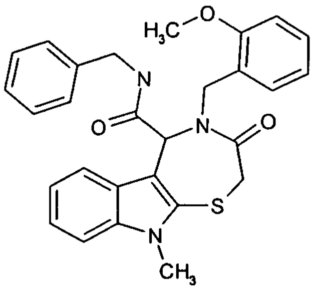 | 485,60969 | 486 |
| 101 | 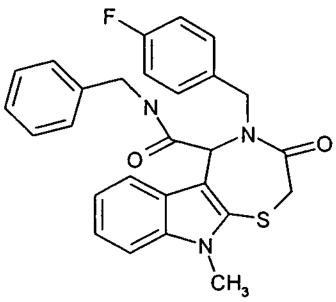 | 473,57363 | 474 |
| 102 | 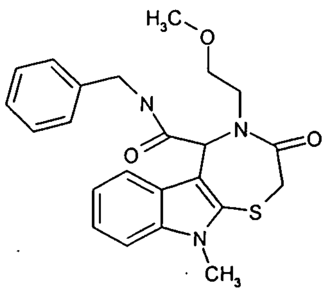 | 423,538 | 424 |
| 103 | 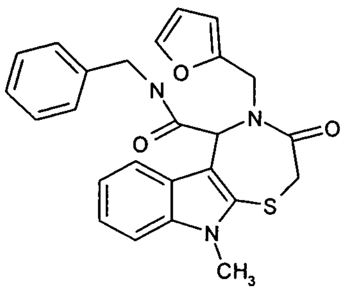 | 445,54436 | 446 |

(continued)

| | Table 1.11 | | |
|---|---|---|---|
| № | Formula | Mol. w. | LC MS, m/z (M+1) |
| 104 | 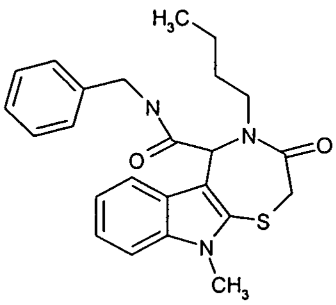 | 421,56569 | 422 |
| 105 | 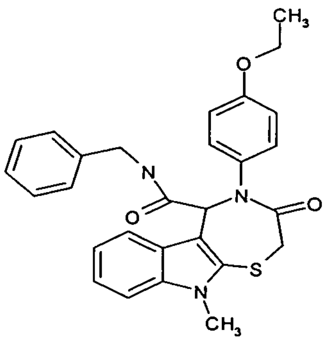 | 485,60969 | 486 |
| 106 | 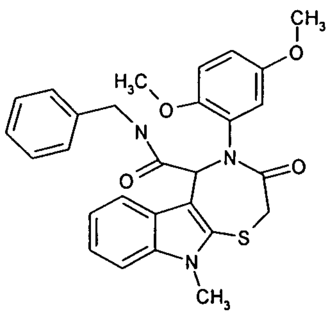 | 501,60909 | 502 |
| 107 | 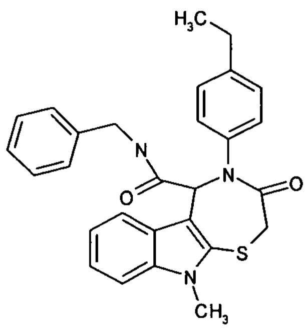 | 469,61029 | 470 |

(continued)

| | Table 1.11 | | |
|---|---|---|---|
| Nº | Formula | Mol. w. | LC MS, m/z (M+1) |
| 108 | 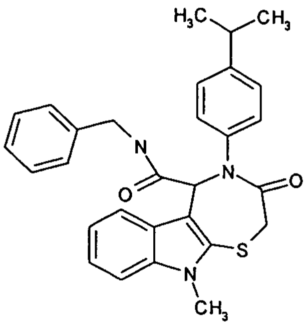 | 483,63738 | 484 |
| 109 | 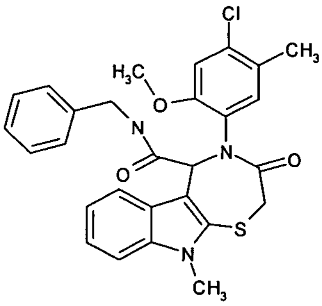 | 520,05472 | 521 |
| 110 | 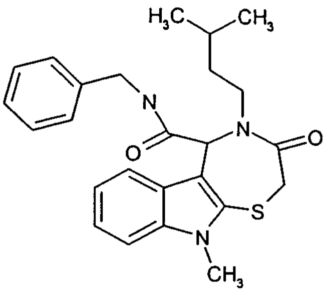 | 435,59278 | 436 |
| 111 | 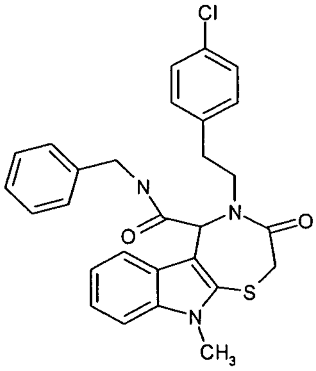 | 504,05532 | 505 |

(continued)

| | Table 1.11 | | |
|---|---|---|---|
| № | Formula | Mol. w. | LC MS, m/z (M+1) |
| 112 | 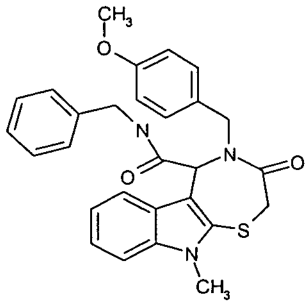 | 485,60969 | 486 |
| 113 | 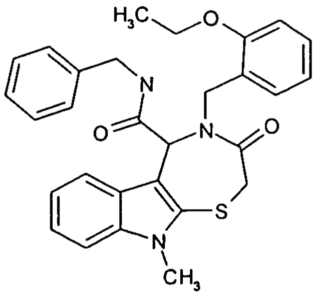 | 499,63678 | 500 |
| 114 | 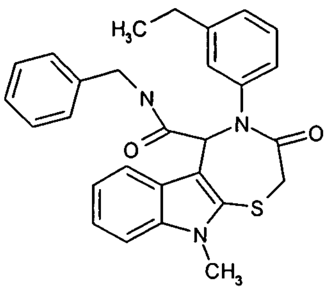 | 469,61029 | 470 |
| 115 | 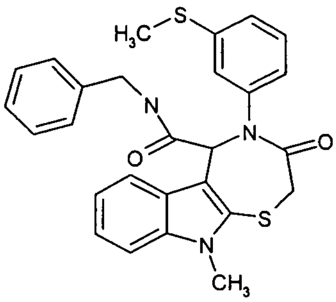 | 487,6472 | 488 |
| 116 | 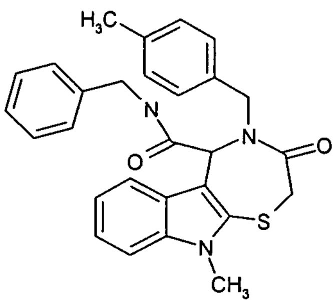 | 469,61029 | 470 |

(continued)

| | Table 1.11 | | |
|---|---|---|---|
| № | Formula | Mol. w. | LC MS, m/z (M+1) |
| 117 | 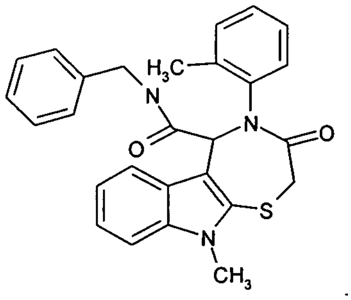 | 455,5832 | 456 |
| 118 | 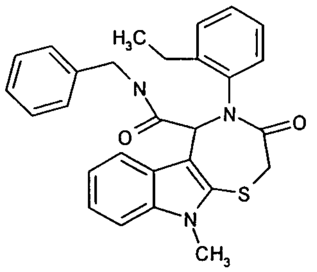 | 469,61029 | 470 |
| 119 | 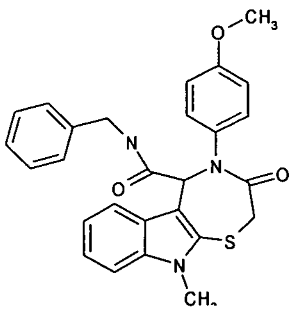 | 471,5826 | 472 |
| 120 | 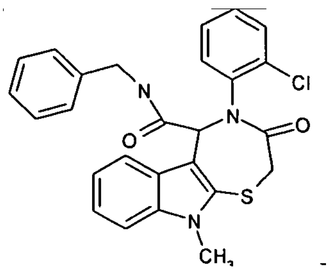 | 476,00114 | 477 |
| 121 | 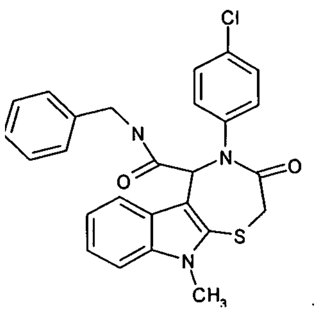 | 476,00114 | 477 |

(continued)

| | Table 1.11 | | |
|---|---|---|---|
| № | Formula | Mol. w. | LC MS, m/z (M+1) |
| 122 | 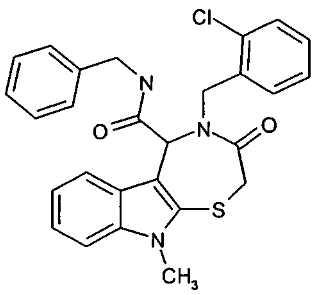 | 490,02823 | 491 |
| 123 | 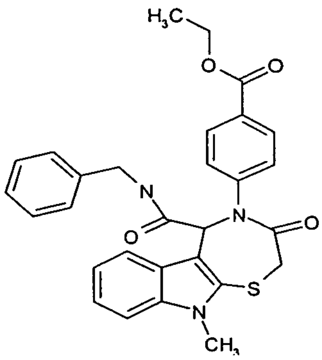 | 513,62024 | 514 |
| 124 | 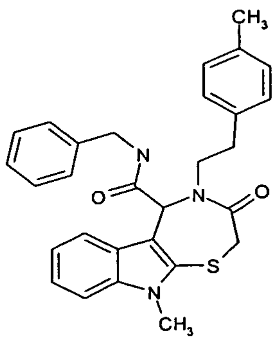 | 483,63738 | 484 |
| 125 | 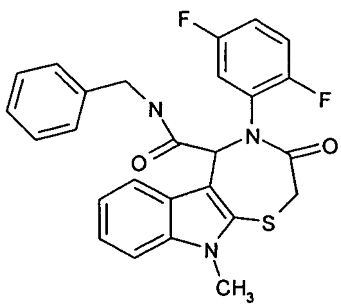 | 477,53697 | 478 |

(continued)

| | Table 1.11 | | |
|---|---|---|---|
| № | Formula | Mol. w. | LC MS, m/z (M+1) |
| 126 | 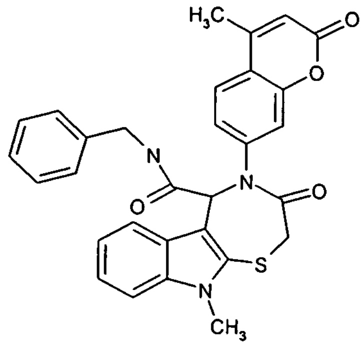 | 523,61545 | 524 |
| 127 | 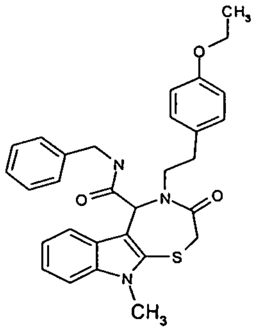 | 513,66387 | 514 |
| 128 | 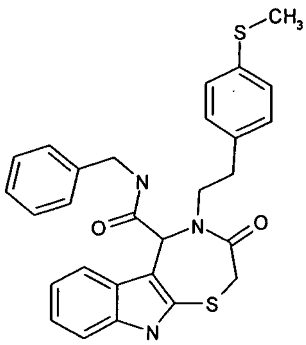 | 515,70138 | 516 |
| 129 | 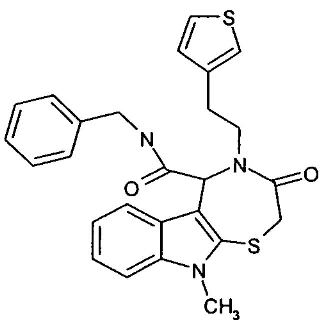 | 475,63605 | 476 |

(continued)

| | Table 1.11 | | |
|---|---|---|---|
| № | Formula | Mol. w. | LC MS, m/z (M+1) |
| 130 | 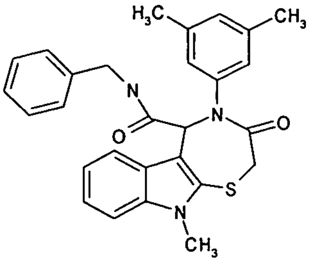 | 469,61029 | 470 |
| 131 | 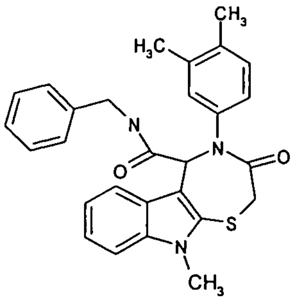 | 469,61029 | 470 |
| 132 | 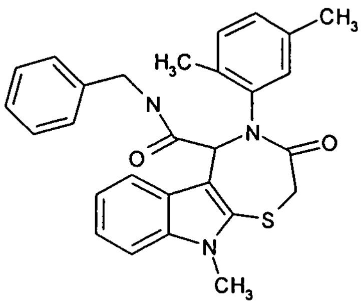 | 469,61029 | 70 |
| 133 | 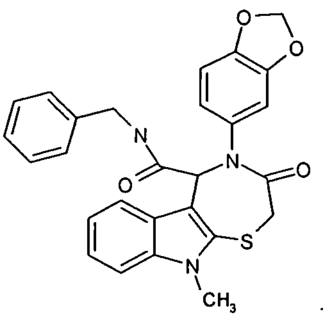 | 485,56606 | 486 |
| 134 | 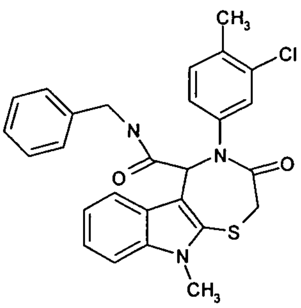 | 490,02823 | 491 |

(continued)

| | Table 1.11 | | |
|---|---|---|---|
| № | Formula | Mol. w. | LC MS, m/z (M+1) |
| 135 | 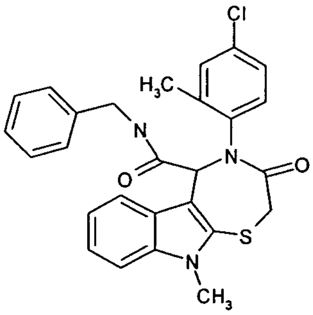 | 490,02823 | 491 |
| 136 | 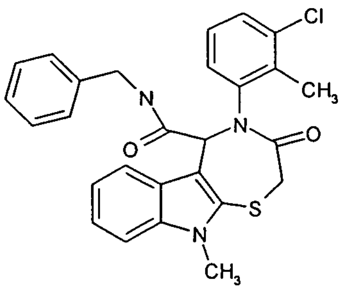 | 490,02823 | 491 |
| 137 | 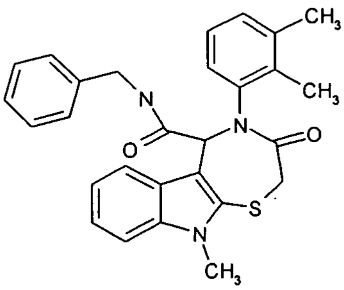 | 469,61029 | 470 |
| 138 | 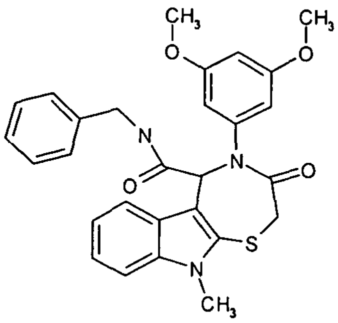 | 501,60909 | 502 |
| 139 | 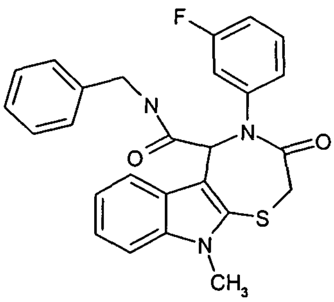 | 459,54654 | 460 |

(continued)

| | Table 1.11 | | |
|---|---|---|---|
| № | Formula | Mol. w. | LC MS, m/z (M+1) |
| 140 | 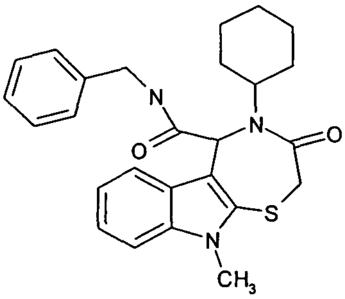 | 447,60393 | 448 |
| 141 | 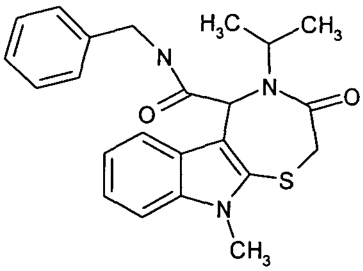 | 407,5386 | 408 |
| 142 | 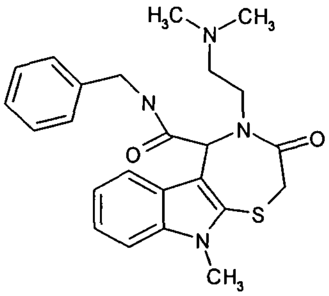 | 436,58036 | 437 |
| 143 | 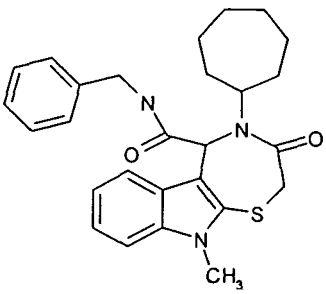 | 461,63102 | 462 |
| 144 | 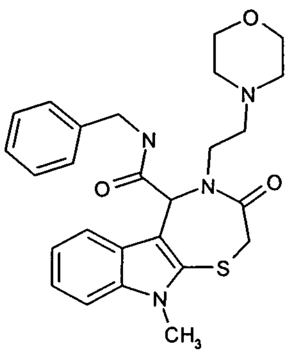 | 478,618 | 479 |

(continued)

| | Table 1.11 | | |
|---|---|---|---|
| № | Formula | Mol. w. | LC MS, m/z (M+1) |
| 145 | 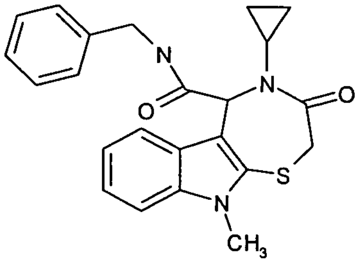 | 405,52266 | 406 |
| 146 | 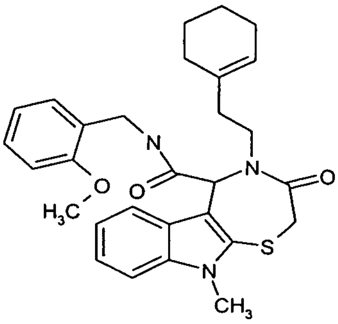 | 503,66866 | 504 |
| 147 | 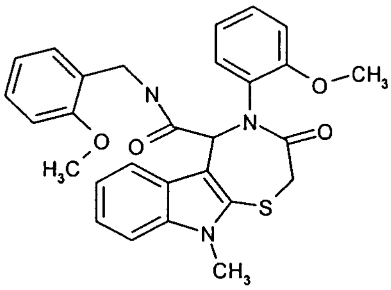 | 501,60909 | 502 |
| 148 | 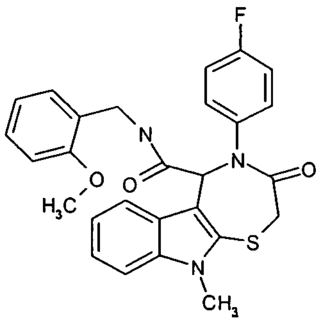 | 489,57303 | 490 |
| 149 | 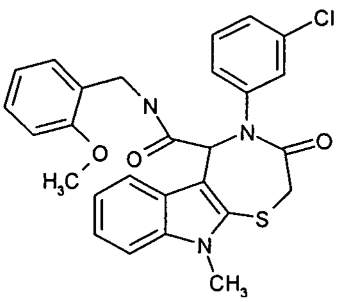 | 506,02763 | 507 |

(continued)

| | Table 1.11 | | |
|---|---|---|---|
| № | Formula | Mol. w. | LC MS, m/z (M+1) |
| 150 | 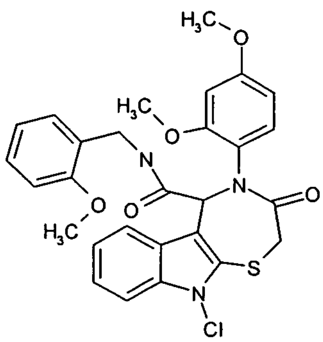 | 531,63558 | 532 |
| 151 | 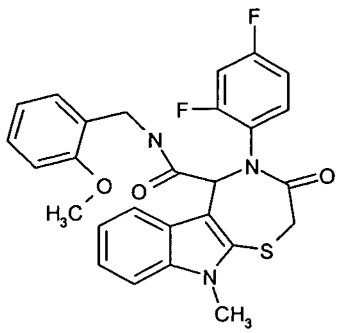 | 507,56346 | 508 |
| 152 | 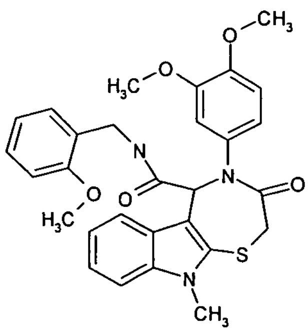 | 531,63558 | 532 |
| 153 | 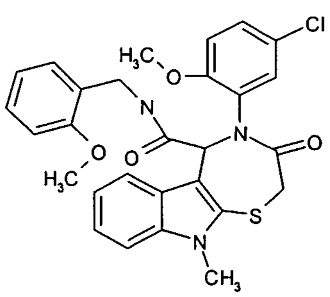 | 536,05412 | 537 |

(continued)

| | Table 1.11 | | |
|---|---|---|---|
| № | Formula | Mol. w. | LC MS, m/z (M+1) |
| 154 | 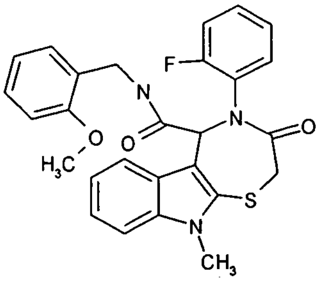 | 489,57303 | 490 |
| 155 | 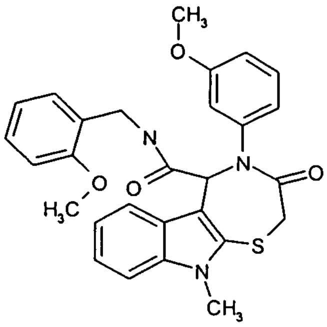 | 501,60909 | 502 |
| 156 | 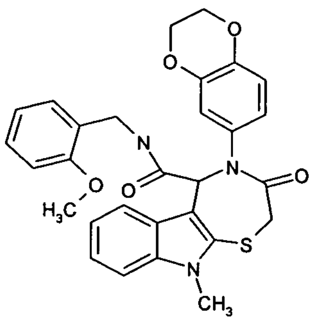 | 529,61964 | 530 |
| 157 | 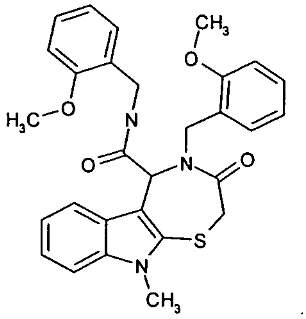 | 515,63618 | 516 |
| 158 | 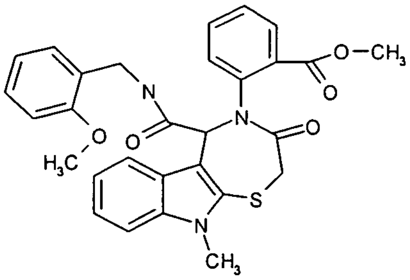 | 529,61964 | 530 |

(continued)

| | Table 1.11 | | |
|---|---|---|---|
| № | Formula | Mol. w. | LC MS, m/z (M+1) |
| 159 | 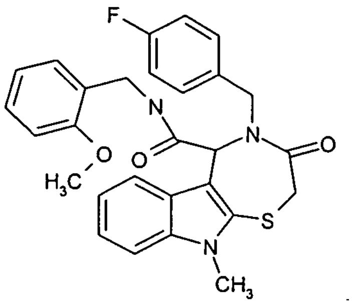 | 503,60012 | 504 |
| 160 | 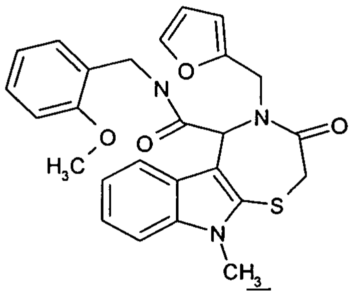 | 475,57085 | 476 |
| 161 | 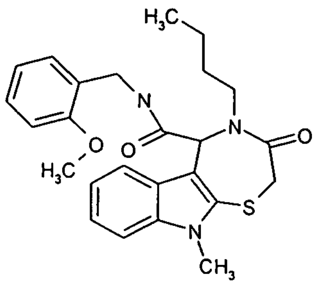 | 451,59218 | 452 |
| 162 | 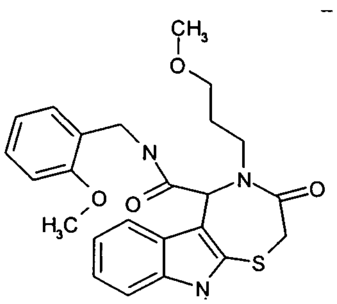 | 467,59158 | 468 |
| 163 | 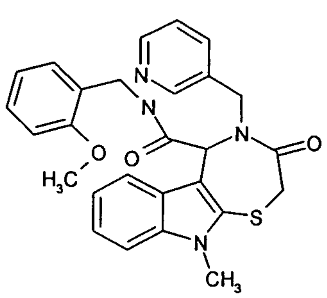 | 486,59727 | 487 |

(continued)

| | Table 1.11 | | |
|---|---|---|---|
| № | Formula | Mol. w. | LC MS, m/z (M+1) |
| 164 | 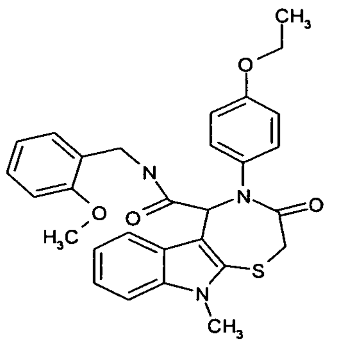 | 515,63618 | 516 |
| 165 | 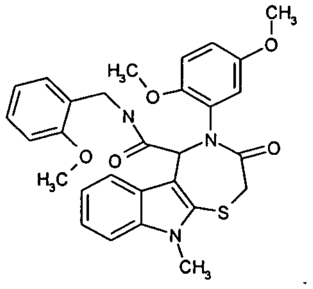 | 531,63558 | 532 |
| 166 | 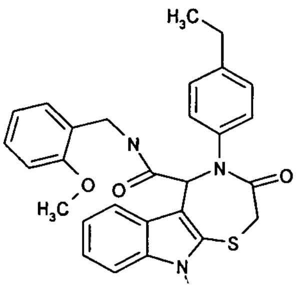 | 499,63678 | 500 |
| 167 | 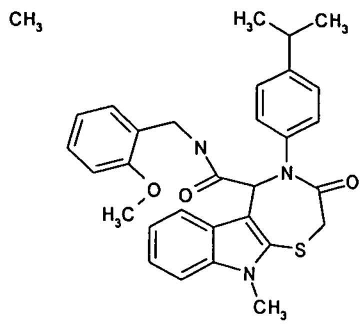 | 513,66387 | 514 |
| 168 | 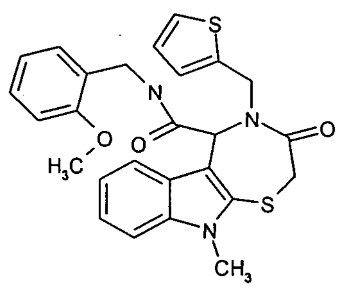 | 491,63545 | 492 |

(continued)

| | Table 1.11 | | |
|---|---|---|---|
| № | Formula | Mol. w. | LC MS, m/z (M+1) |
| 169 | 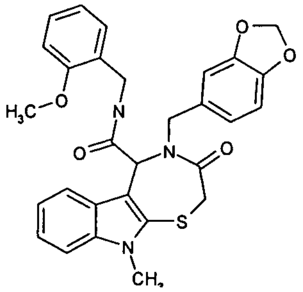 | 529,61964 | 530 |
| 170 | 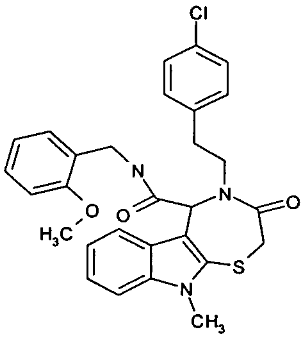 | 534,08181 | 535 |
| 171 | 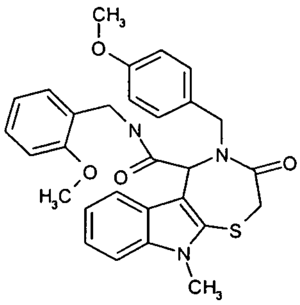 | 515,63618 | 516 |
| 172 | 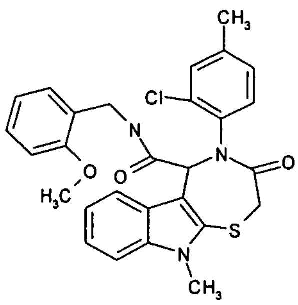 | 520,05472 | 521 |
| 173 | 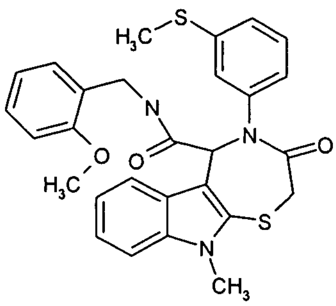 | 517,67369 | 518 |

(continued)

| | Table 1.11 | | |
|---|---|---|---|
| № | Formula | Mol. w. | LC MS, m/z (M+1) |
| 174 | 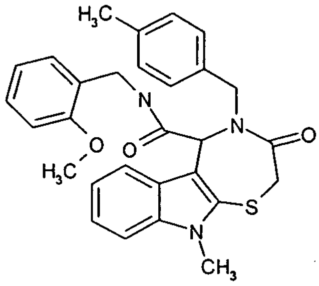 | 499,63678 | 500 |
| 175 | 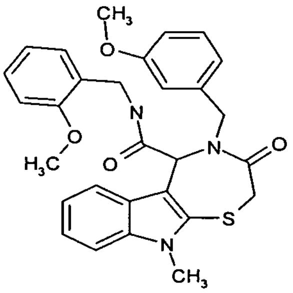 | 515,63618 | 516 |
| 176 | 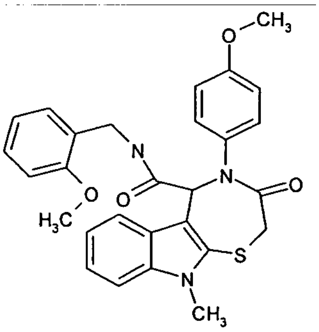 | 501,60909 | 502 |
| 177 | 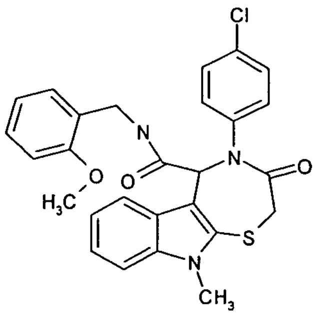 | 506,02763 | 507 |
| 178 | 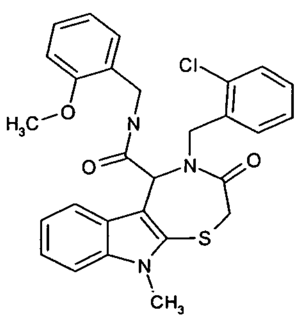 | 520,05472 | 521 |

(continued)

| | Table 1.11 | | |
|---|---|---|---|
| № | Formula | Mol. w. | LC MS, m/z (M+1) |
| 179 | 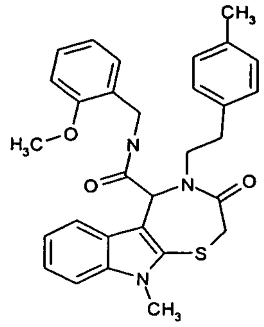 | 513,66387 | 514 |
| 180 | 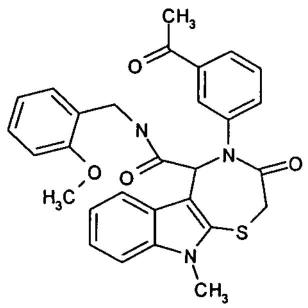 | 513,62024 | 514 |
| 181 | 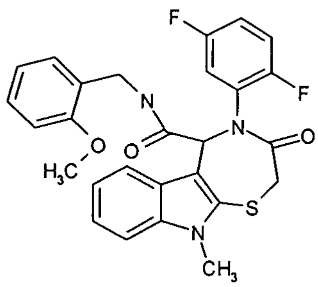 | 507,56346 | 508 |
| 182 | 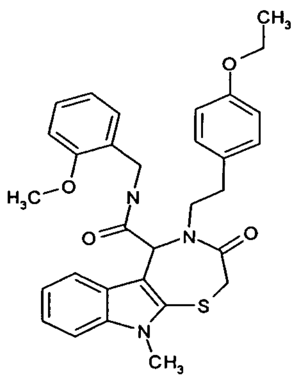 | 543,69036 | 544 |

(continued)

| | Table 1.11 | | |
|---|---|---|---|
| № | Formula | Mol. w. | LC MS, m/z (M+1) |
| 183 | 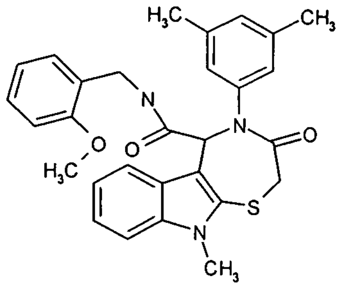 | 499,63678 | 500 |
| 184 | 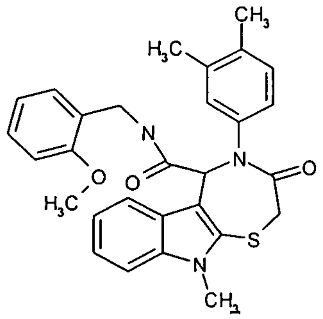 | 499,63678 | 500 |
| 185 | 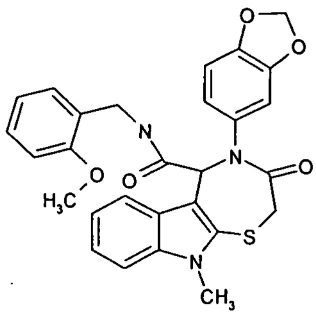 | 515,59255 | 516 |
| 186 | 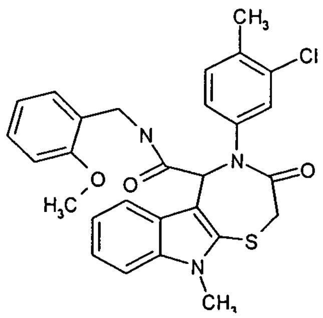 | 520,05472 | 521 |

(continued)

| | Table 1.11 | | |
|---|---|---|---|
| № | Formula | Mol. w. | LC MS, m/z (M+1) |
| 187 | 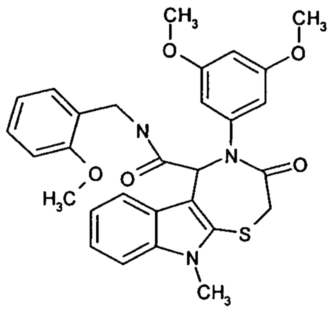 | 531,63558 | 532 |
| 188 | 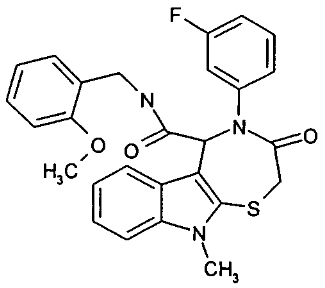 | 489,57303 | 490 |
| 189 | 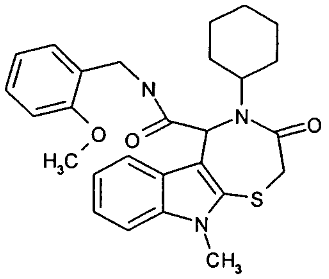 | 477,63042 | 478 |
| 190 | 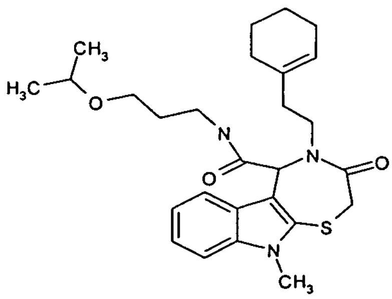 | 483,67824 | 484 |
| 191 | 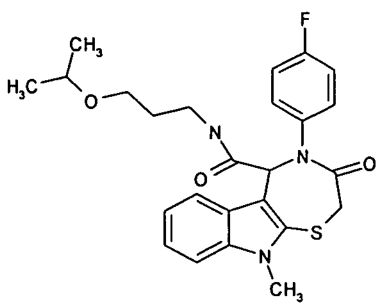 | 469,58261 | 470 |

(continued)

| | Table 1.11 | | |
|---|---|---|---|
| № | Formula | Mol. w. | LC MS, m/z (M+1) |
| 192 | 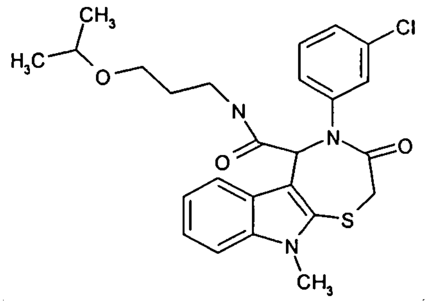 | 486,03721 | 487 |
| 193 | 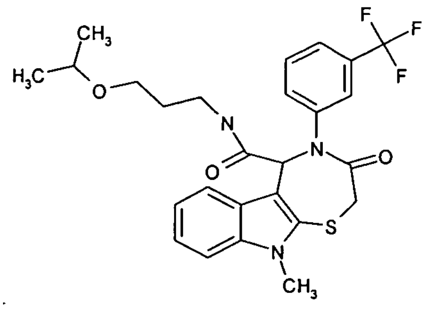 | 519,59056 | 520 |
| 194 | 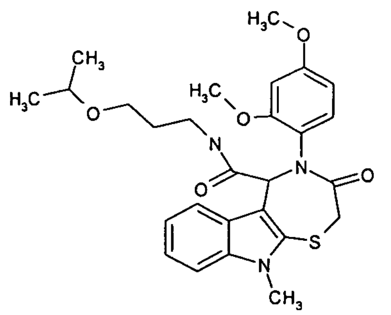 | 511,64516 | 512 |
| 195 | 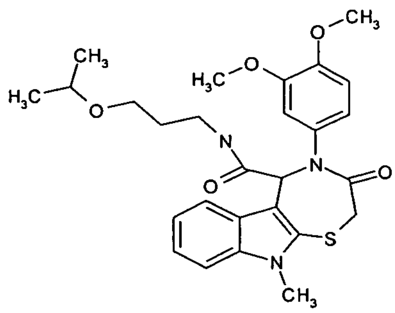 | 511,64516 | 512 |
| 196 | 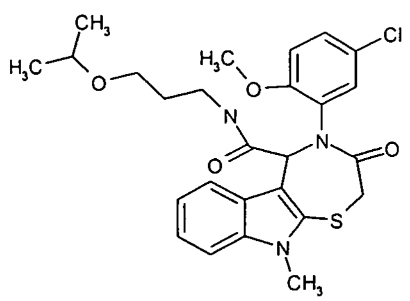 | 516,0637 | 517 |

(continued)

| | Table 1.11 | | |
|---|---|---|---|
| № | Formula | Mol. w. | LC MS, m/z (M+1) |
| 197 | 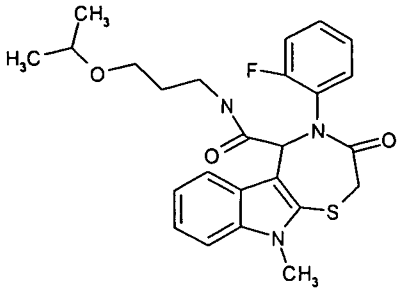 | 469,58261 | 470 |
| 198 | 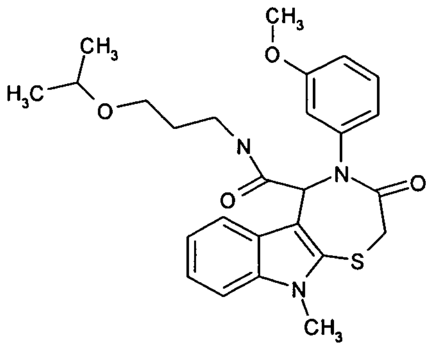 | 481,61867 | 482 |
| 199 | 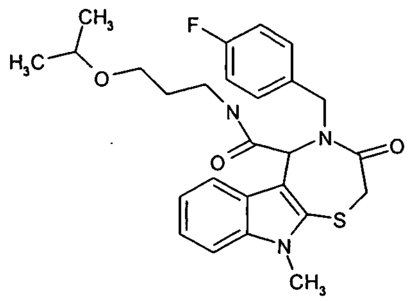 | 483,6097 | 484 |
| 200 | 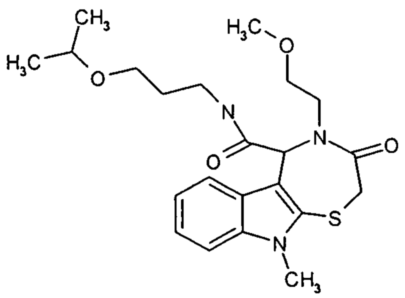 | 433,57407 | 434 |
| 201 | 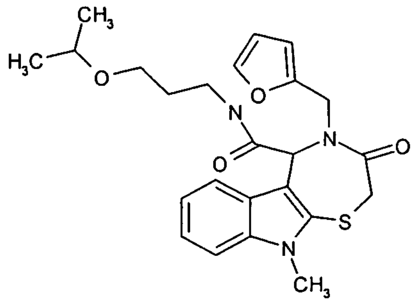 | 455,58043 | 456 |

(continued)

| | Table 1.11 | | |
|---|---|---|---|
| № | Formula | Mol. w. | LC MS, m/z (M+1) |
| 202 | 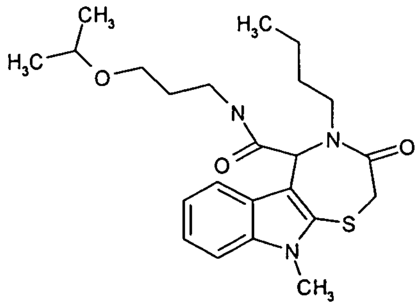 | 431,60176 | 432 |
| 203 | 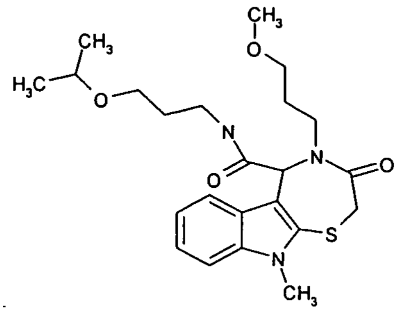 | 447,60116 | 448 |
| 204 | 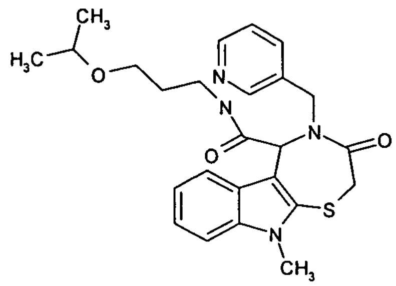 | 466,60685 | 467 |
| 205 | 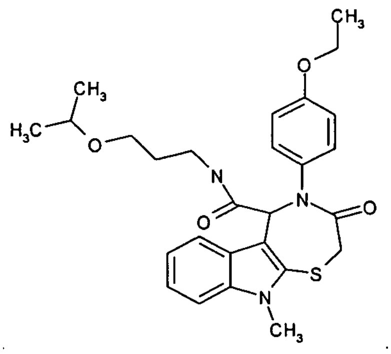 | 495,64576 | 496 |
| 206 | 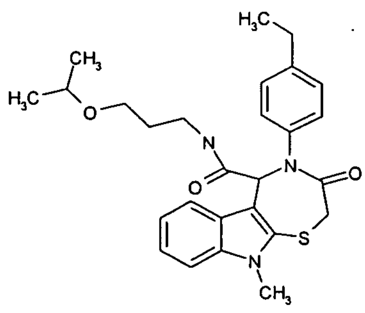 | 479,64636 | 480 |

(continued)

| | Table 1.11 | | |
|---|---|---|---|
| № | Formula | Mol. w. | LC MS, m/z (M+1) |
| 207 | 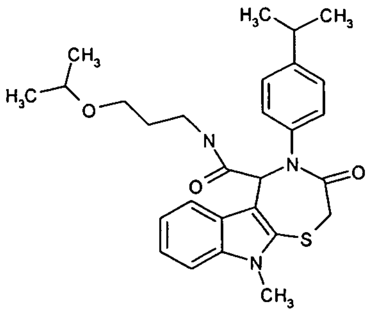 | 493,67345 | 494 |
| 208 | 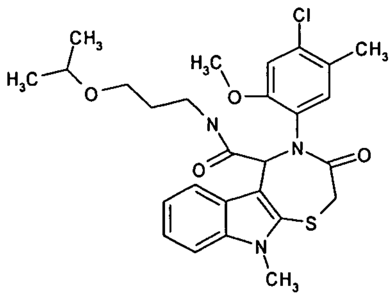 | 530,09079 | 531 |
| 209 | 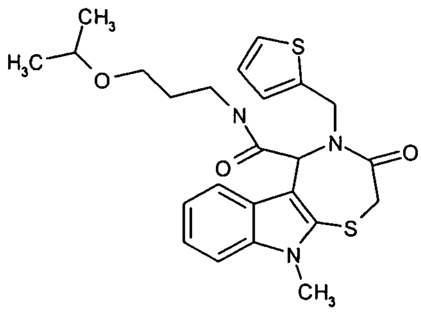 | 471,64503 | 472 |
| 210 | 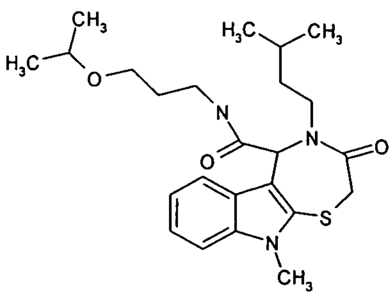 | 445,62885 | 446 |
| 211 | 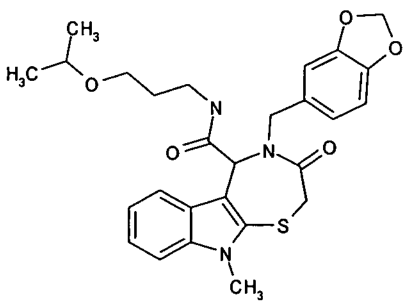 | 509,62922 | 510 |

(continued)

| | Table 1.11 | | |
|---|---|---|---|
| № | Formula | Mol. w. | LC MS, m/z (M+1) |
| 212 | 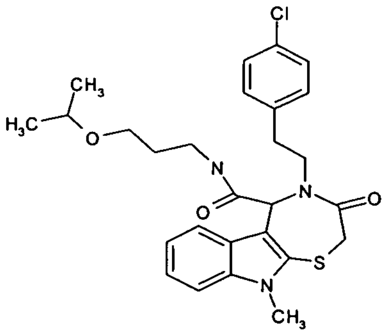 | 514,09139 | 515 |
| 213 | 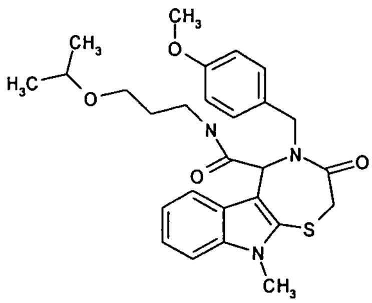 | 495,64576 | 496 |
| 214 | 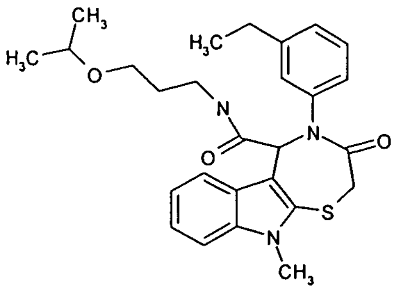 | 479,64636 | 480 |
| 215 | 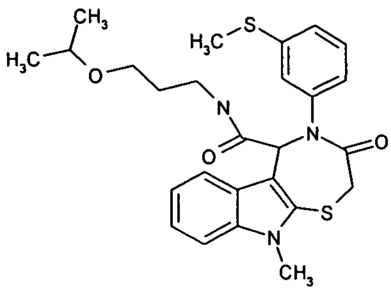 | 497,68327 | 498 |
| 216 | 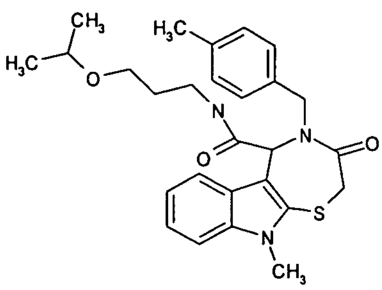 | 479,64636 | 480 |

(continued)

| | Table 1.11 | | |
|---|---|---|---|
| № | Formula | Mol. w. | LC MS, m/z (M+1) |
| 217 | 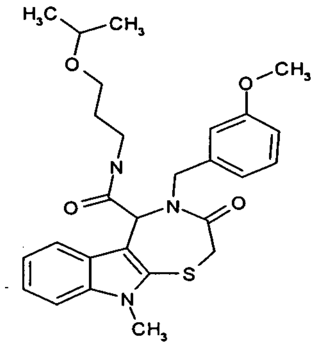 | 495,64576 | 496 |
| 218 | 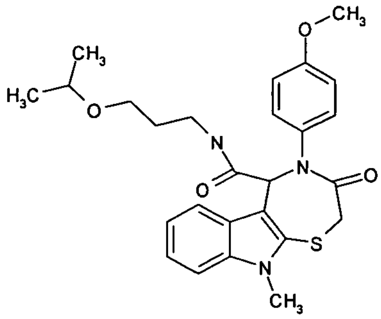 | 481,61867 | 482 |
| 219 | 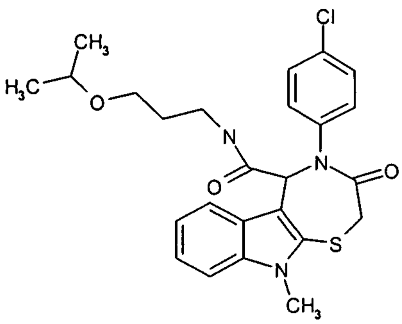 | 486,03721 | 487 |
| 220 | 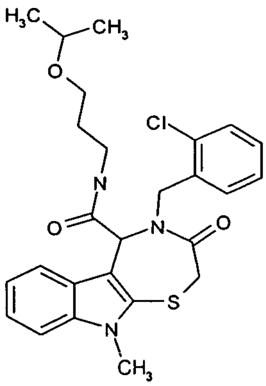 | 500,0643 | 501 |

(continued)

| | Table 1.11 | | |
|---|---|---|---|
| № | Formula | Mol. w. | LC MS, m/z (M+1) |
| 221 | 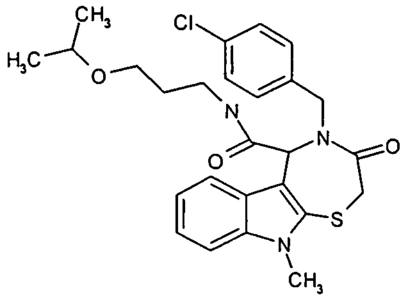 | 500,0643 | 501 |
| 222 | 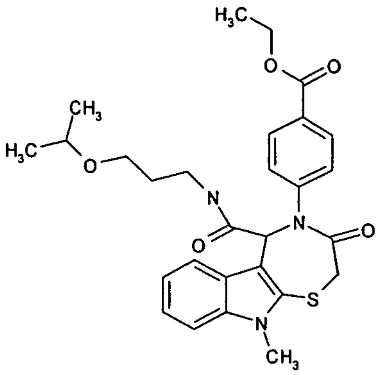 | 523,65631 | 524 |
| 223 | 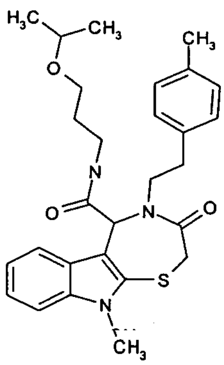 | 493,67345 | 494 |
| 224 | 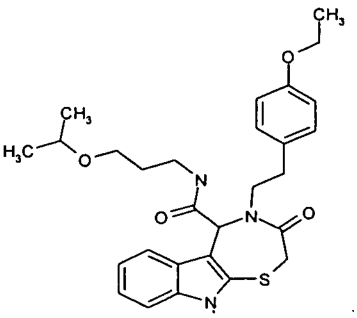 | 523,69994 | 524 |

(continued)

| | Table 1.11 | | |
|---|---|---|---|
| № | Formula | Mol. w. | LC MS, m/z (M+1) |
| 225 | 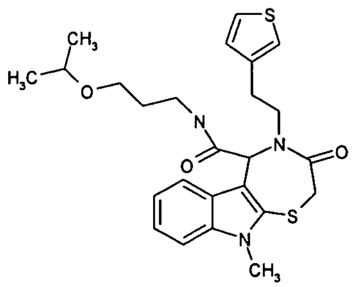 | 485,67212 | 486 |
| 226 | 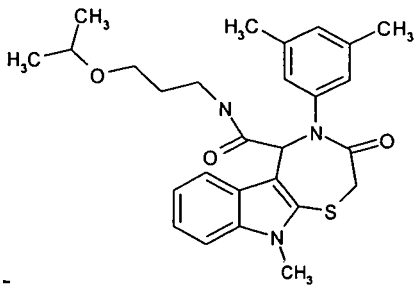 | 479,64636 | 480 |
| 227 | 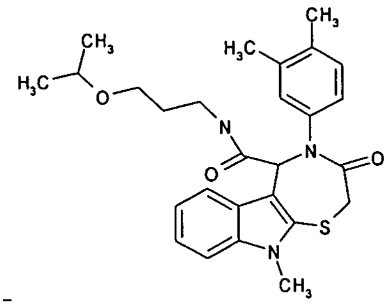 | 479,64636 | 480 |
| 228 | 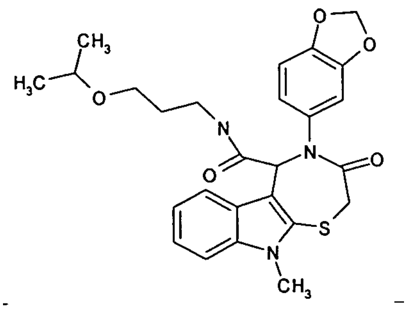 | 495,60213 | 496 |
| 229 | 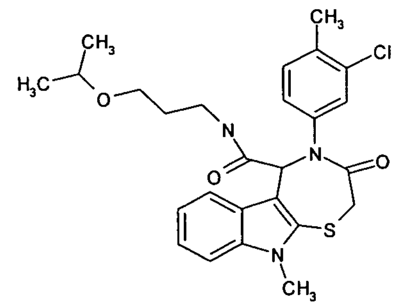 | 500,0643 | 501 |

(continued)

| | Table 1.11 | | |
|---|---|---|---|
| № | Formula | Mol. w. | LC MS, m/z (M+1) |
| 230 | 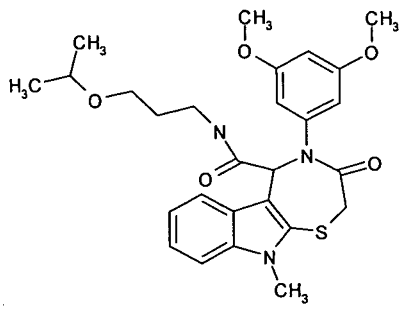 | 511,64516 | 512 |
| 231 | 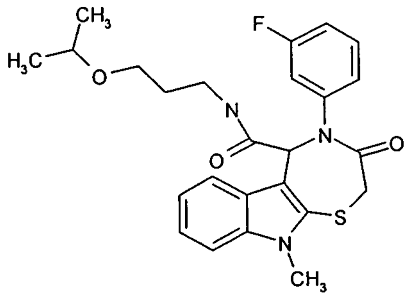 | 469,58261 | 470 |
| 232 | 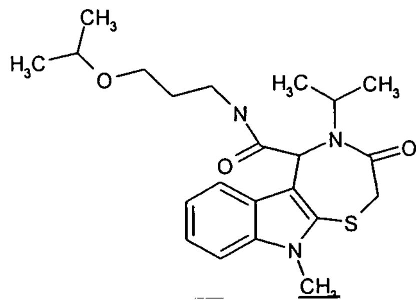 | 417,57467 | 418 |
| 233 | 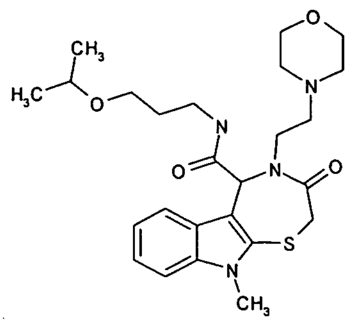 | 488,65407 | 489 |
| 234 | 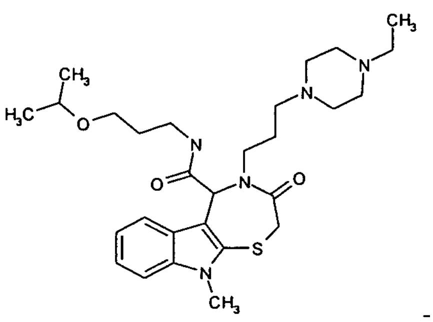 | 529,75061 | 530 |

(continued)

| | Table 1.11 | | |
|---|---|---|---|
| № | Formula | Mol. w. | LC MS, m/z (M+1) |
| 235 | 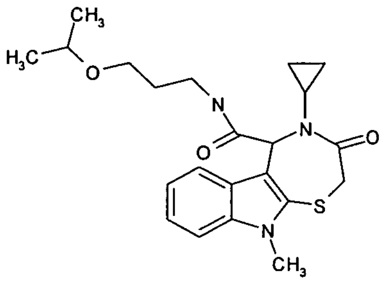 | 415,55873 | 416 |
| 236 | 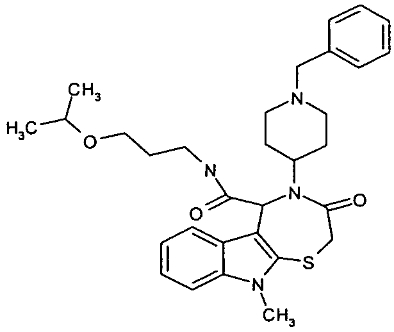 | 548,75345 | 549 |
| 237 | 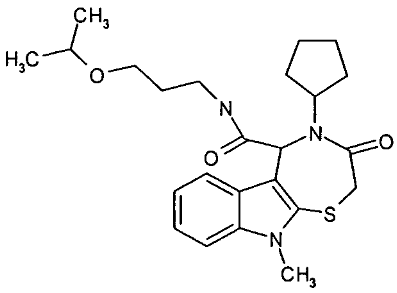 | 443,61291 | 444 |
| 238 | 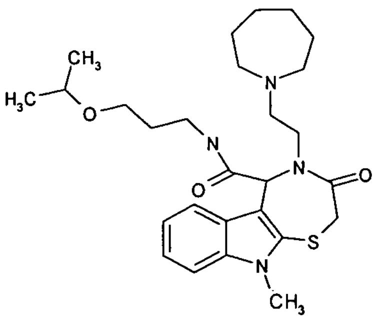 | 500,70885 | 501 |

(continued)

| | Table 1.11 | | |
|---|---|---|---|
| № | Formula | Mol. w. | LC MS, m/z (M+1) |
| 239 | 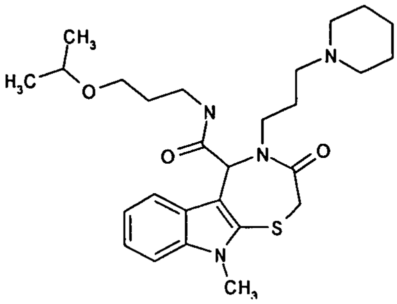 | 500,70885 | 501 |
| 240 | 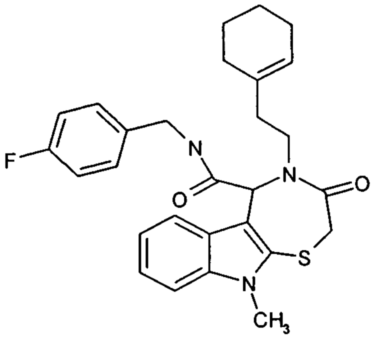 | 491,6326 | 492 |
| 241 | 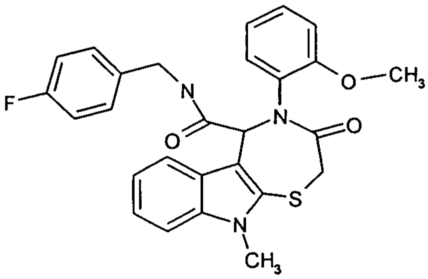 | 489,57303 | 490 |
| 242 | 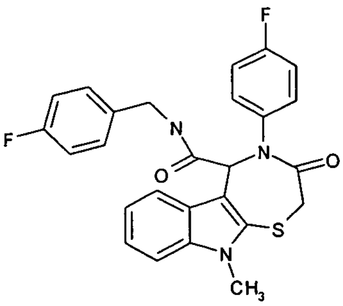 | 477,53697 | 478 |
| 243 | 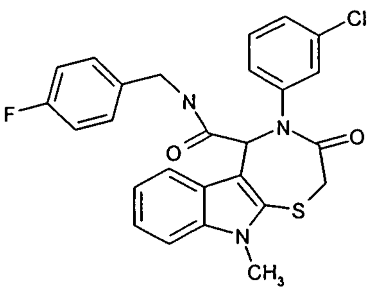 | 493,99157 | 494 |

(continued)

| | Table 1.11 | | |
|---|---|---|---|
| № | Formula | Mol. w. | LC MS, m/z (M+1) |
| 244 | 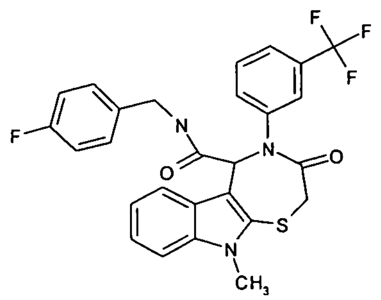 | 527,54492 | 528 |
| 245 | 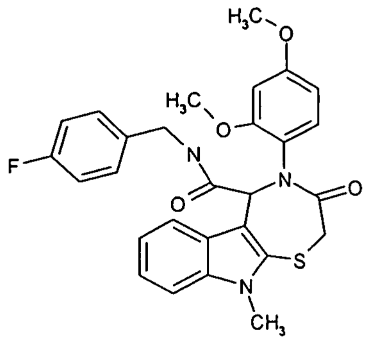 | 519,59952 | 520 |
| 246 | 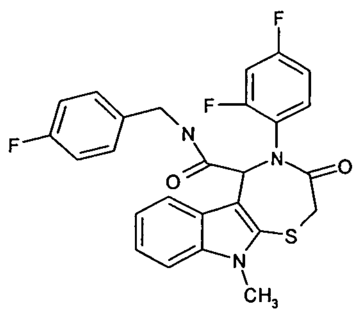 | 495,5274 | 496 |
| 247 | 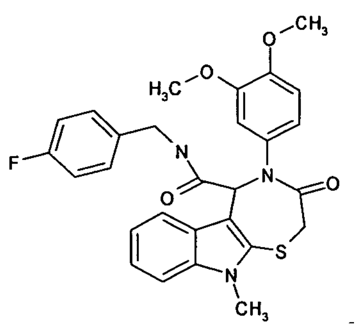 | 519,59952 | 520 |
| 248 | 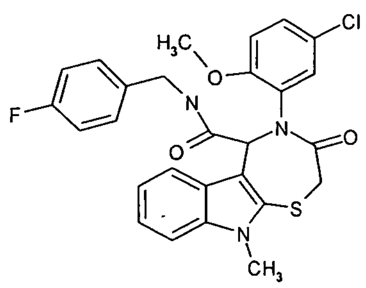 | 524,01806 | 525 |

(continued)

| | Table 1.11 | | |
|---|---|---|---|
| № | Formula | Mol. w. | LC MS, m/z (M+1) |
| 249 | 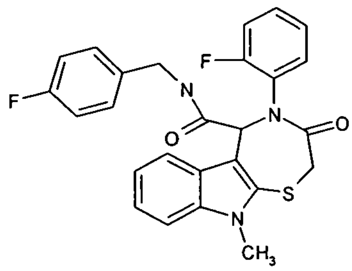 | 477,53697 | 478 |
| 250 | 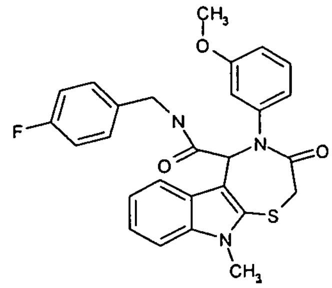 | 489,57303 | 490 |
| 251 | 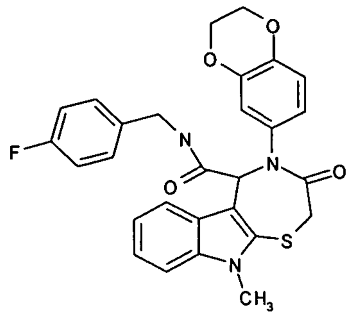 | 517,58358 | 518 |
| 252 | 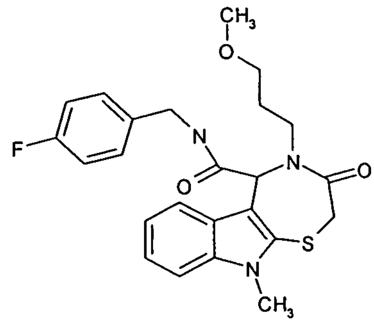 | 455,55552 | 456 |
| 253 | 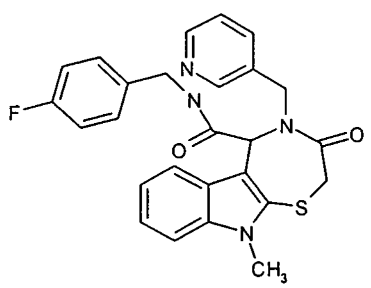 | 474,56121 | 475 |

(continued)

| | Table 1.11 | | |
|---|---|---|---|
| № | Formula | Mol. w. | LC MS, m/z (M+1) |
| 254 | 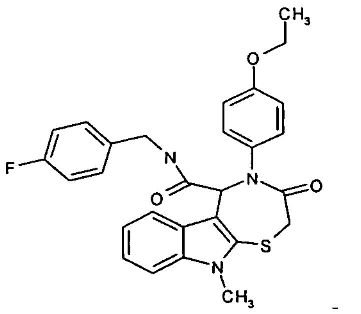 | 503,60012 | 504 |
| 255 | 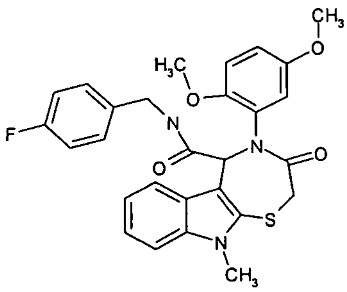 | 519,59952 | 520 |
| 256 | 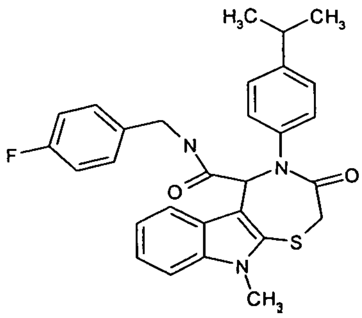 | 501,62781 | 502 |
| 257 | 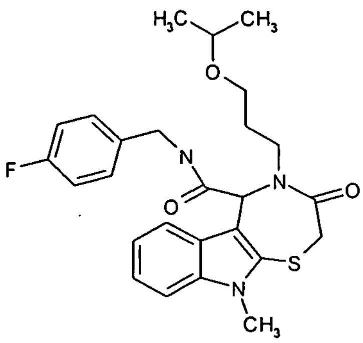 | 483,6097 | 484 |
| 258 | 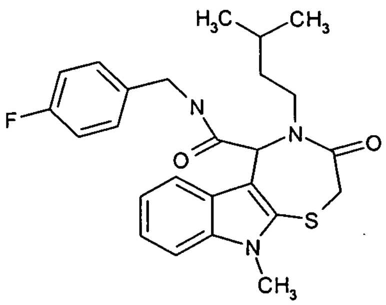 | 453,58321 | 454 |

(continued)

| | Table 1.11 | | |
|---|---|---|---|
| № | Formula | Mol. w. | LC MS, m/z (M+1) |
| 259 | 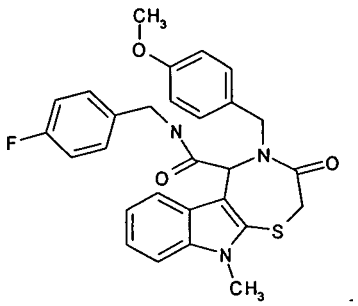 | 503,60012 | 504 |
| 260 | 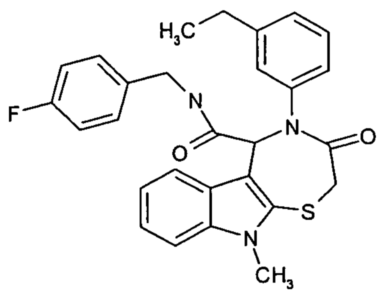 | 487,60072 | 488 |
| 261 | 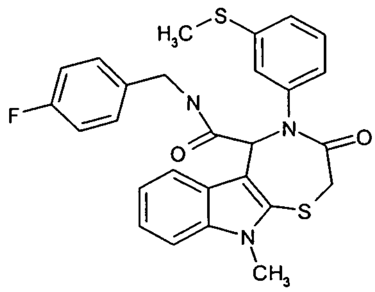 | 505,63763 | 506 |
| 262 | 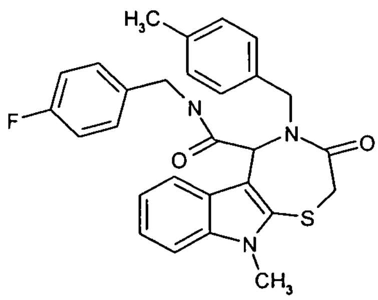 | 487,60072 | 488 |
| 263 | 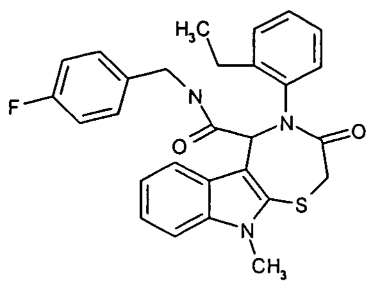 | 487,60072 | 488 |

(continued)

| | Table 1.11 | | |
|---|---|---|---|
| № | Formula | Mol. w. | LC MS, m/z (M+1) |
| 264 | 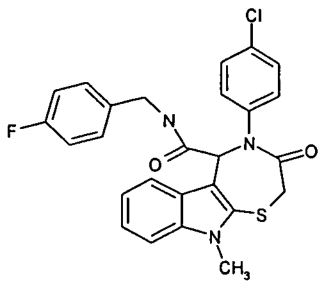 | 493,99157 | 494 |
| 265 | 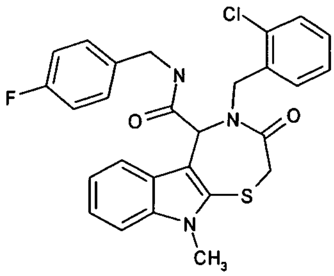 | 508,01866 | 509 |
| 266 | 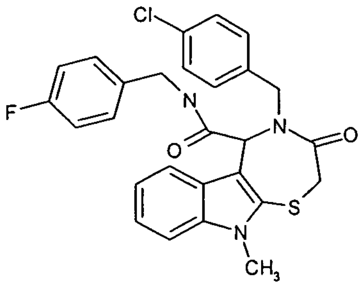 | 508,01866 | 509 |
| 267 | 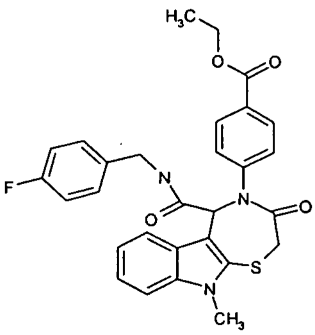 | 531,61067 | 532 |
| 268 | 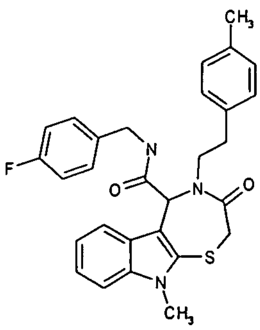 | 501,62781 | 502 |

(continued)

| | Table 1.11 | | |
|---|---|---|---|
| № | Formula | Mol. w. | LC MS, m/z (M+1) |
| 269 | 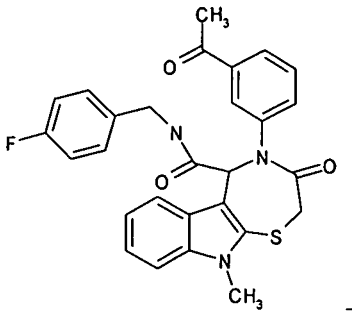 | 501,58418 | 502 |
| 270 | 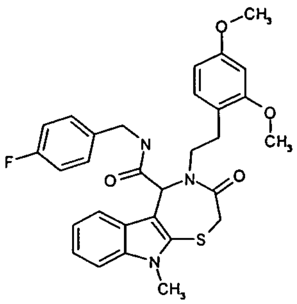 | 547,6537 | 548 |
| 271 | 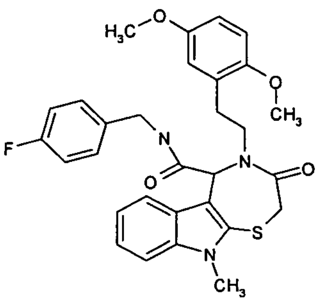 | 547,6537 | 548 |
| 272 | 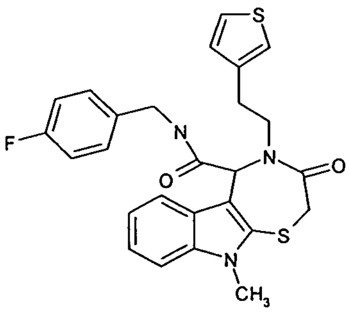 | 493,62648 | 494 |
| 273 | 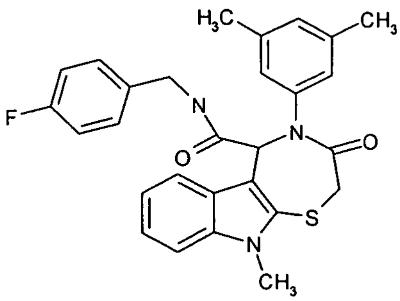 | 487,60072 | 488 |

(continued)

| | Table 1.11 | | |
|---|---|---|---|
| № | Formula | Mol. w. | LC MS, m/z (M+1) |
| 274 | 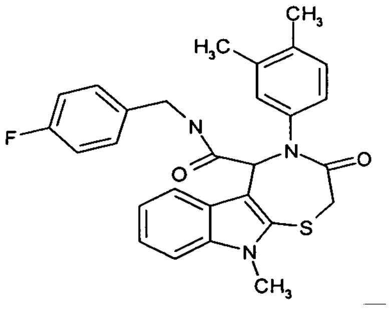 | 487,60072 | 488 |
| 275 | 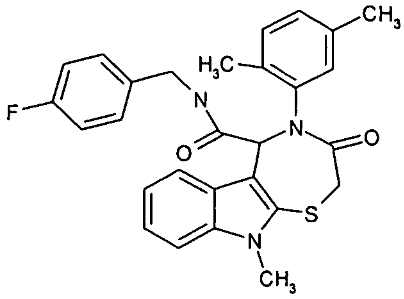 | 487,60072 | 488 |
| 276 | 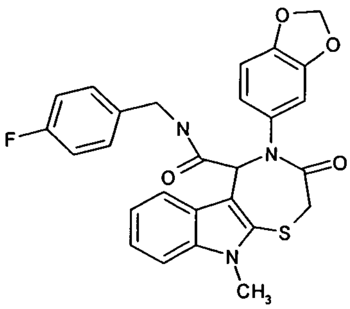 | 503,55649 | 504 |
| 277 | 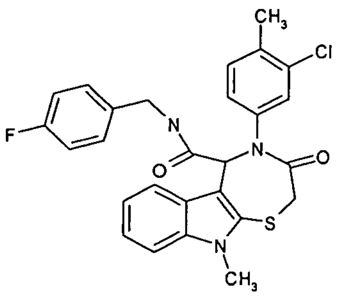 | 508,01866 | 509 |
| 278 | 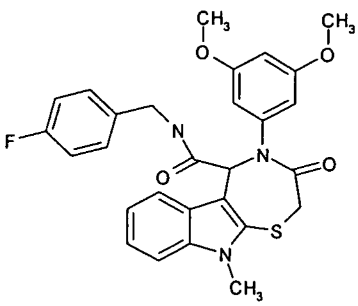 | 519,59952 | 520 |

(continued)

| | Table 1.11 | | |
|---|---|---|---|
| № | Formula | Mol. w. | LC MS, m/z (M+1) |
| 279 | 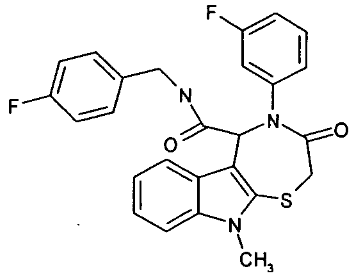 | 477,53697 | 478 |
| 280 | 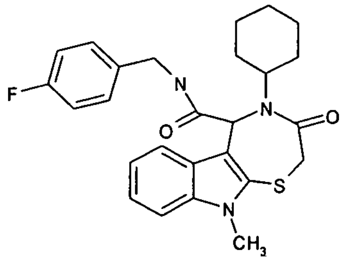 | 465,59436 | 466 |
| 281 | 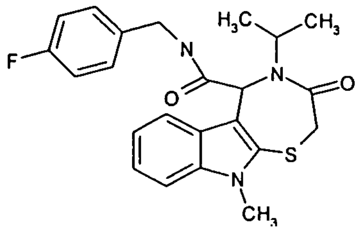 | 425,52903 | 426 |
| 282 | 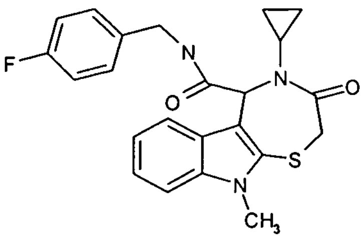 | 423,51309 | 424 |
| 283 | 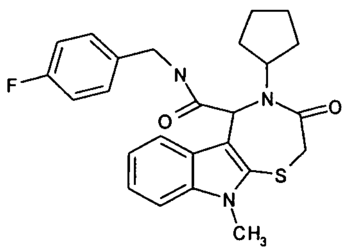 | 451,56727 | 452 |

Table 1.12.

| № | Formula | Mol. w. | LC MS, m/z (M+1) |
|---|---|---|---|
| 1 | 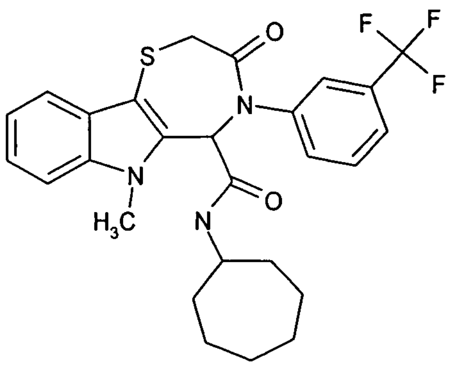 | 515,60231 | 516 |
| 2 | 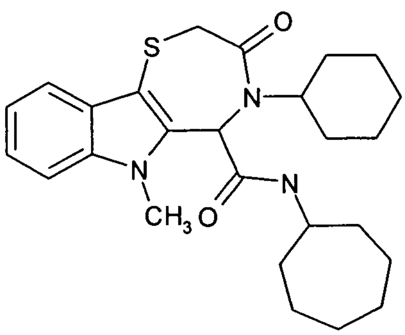 | 453,65175 | 454 |
| 3 | 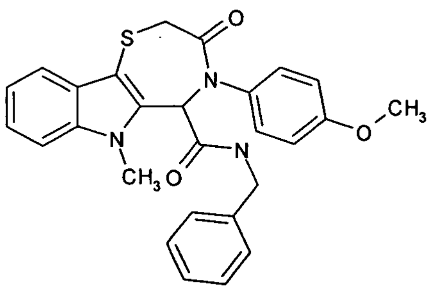 | 471,5826 | 472 |
| 4 | 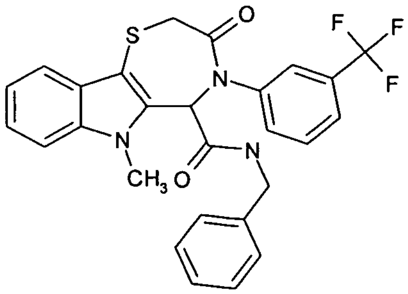 | 509,55449 | 510 |

Table 1.13.

| № | Formula | Mol. w. | LC MS, m/z (M+1) |
|---|---|---|---|
| 1 | 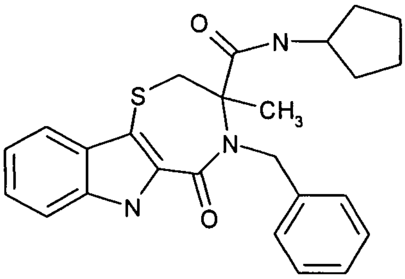 | 433,57684 | 434 |
| 2 | 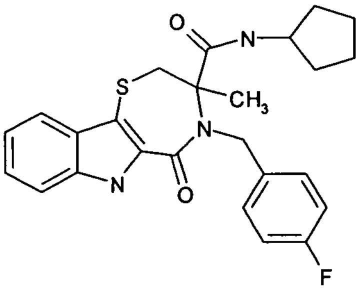 | 451,56727 | 452 |
| 3 | 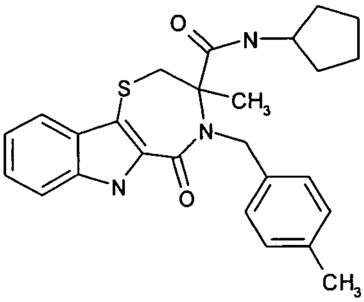 | 447,60393 | 448 |
| 5 | 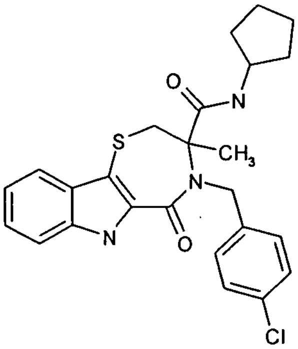 | 468,02187 | 469 |
| 5 | 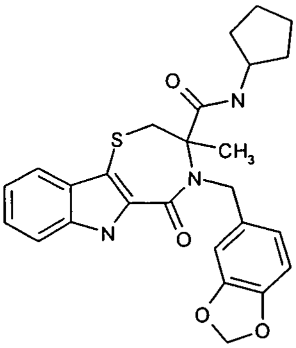 | 477,58679 | 478 |

Table 1.14.

| № n.n. | Формула | Mon. Bec | Масс-спектр, m/z (M+1) |
|---|---|---|---|
| 1 | 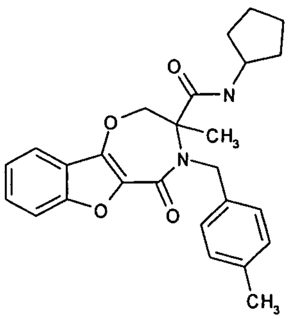 | 432,5241 | 433 |
| 2 | 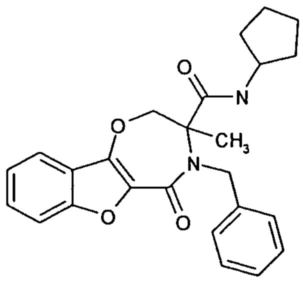 | 418,497 | 419 |
| 3 | 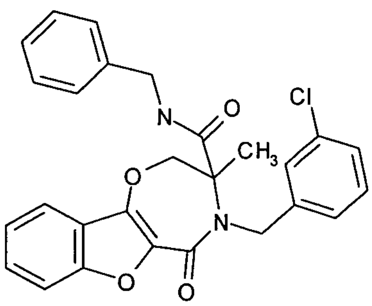 | 474,9484 | 475 |
| 4 | 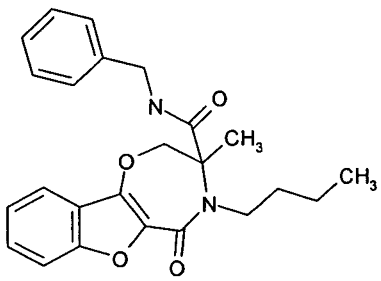 | 406,4858 | 407 |
| 5 | 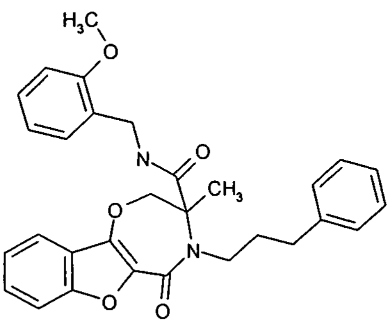 | 498,584 | 499 |

(continued)

| № n.n. | Формула | Mon. Bec | Масс-спектр, m/z (M+1) |
|---|---|---|---|
| 6 | 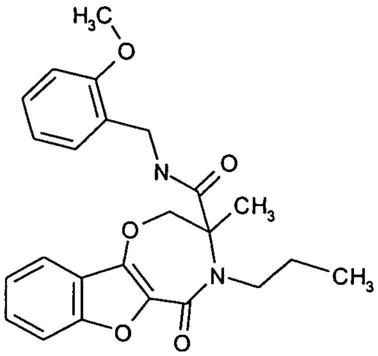 | 422,4852 | 423 |
| 7 | 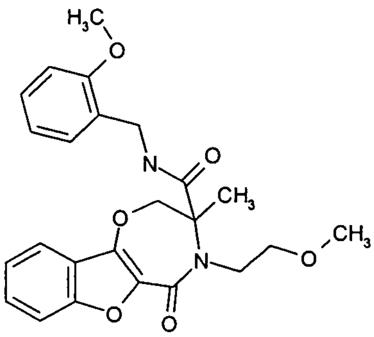 | 438,4846 | 439 |
| 8 | 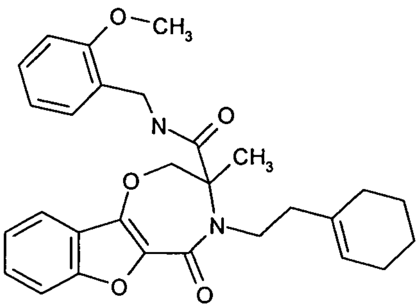 | 488,5888 | 489 |
| 9 | 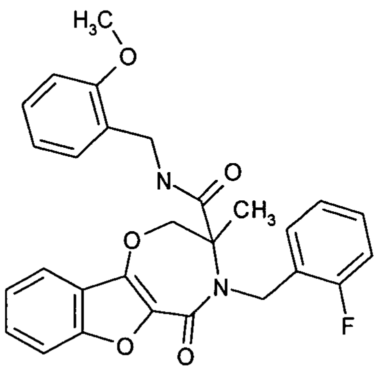 | 488,5203 | 489 |

(continued)

| № n.n. | Формула | Mon. Bec | Масс-спектр, m/z (M+1) |
|---|---|---|---|
| 10 | 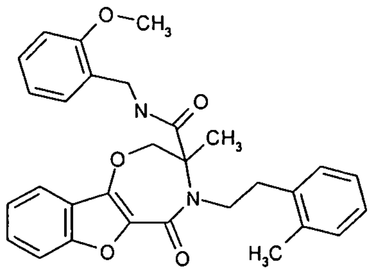 | 498,584 | 499 |
| 11 | 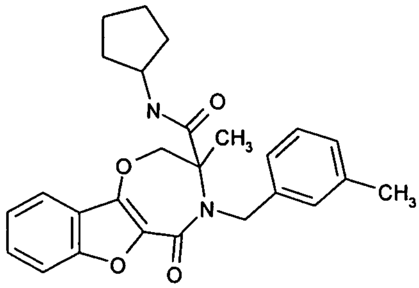 | 432,5241 | 433 |
| 12 | 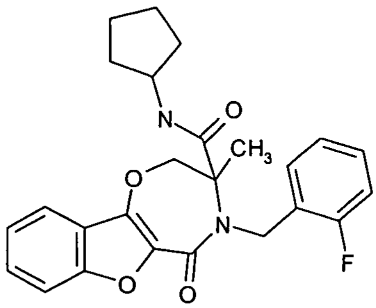 | 436,4874 | 437 |
| 13 | 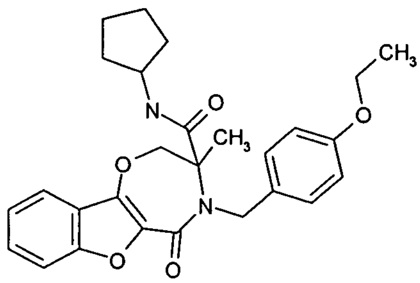 | 462,5506 | 463 |
| 14 | 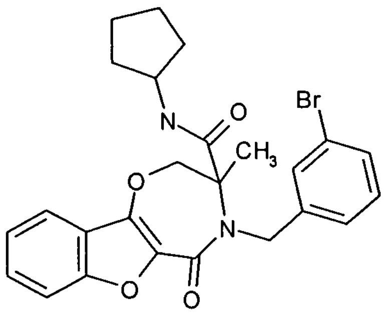 | 497,393 | 498 |

(continued)

| № n.n. | Формула | Mon. Bec | Масс-спектр, m/z (M+1) |
|---|---|---|---|
| 15 | 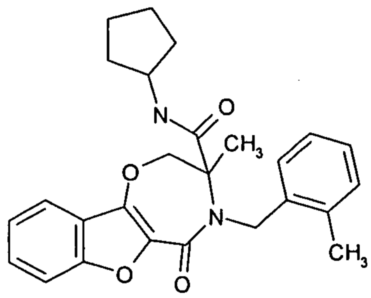 | 432,5241 | 433 |
| 16 | 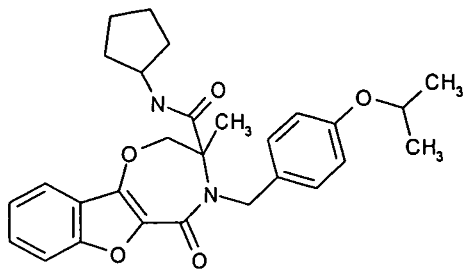 | 476,5776 | 477 |
| 17 | 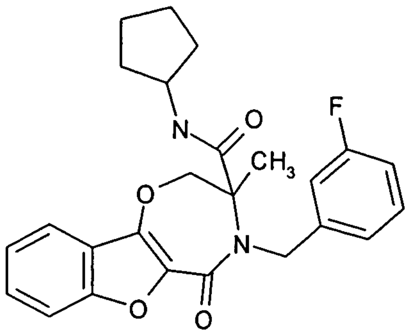 | 436,4874 | 437 |
| 18 | 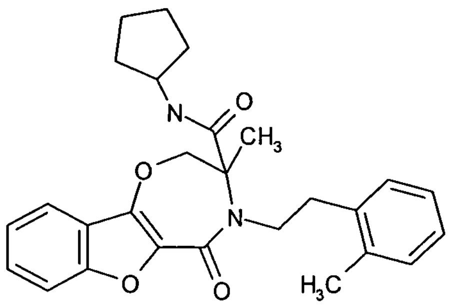 | 446,5512 | 447 |

Table 1.15.

| № | Formula | Mol. w. | LC MS, m/z (M+1) | 1H NMR |
|---|---|---|---|---|
| 1 | 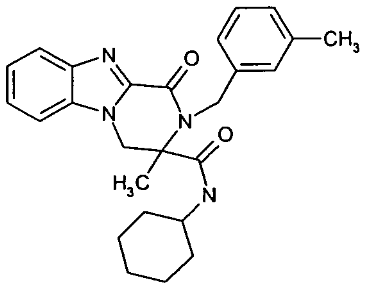 | 430,5546 | 431 | 0.88-1.22 m (5H, cyclohexyl); 1.28-1.38 m (1H, cyclohexyl); 1.42-4.66 m (4H, cyclohexyl); 1.58 s (3H, CH3); 2.34 s (3H, CH3, m-CH3-Pn); 3.38 m (1H, CH, cyclohexyl); 4.20, 5.01 dd (2H, CH2, N-CH2-Phm, J=16.4Hz); 4.27,5.01 dd (2H, CH2, cycle, J=13.... |
| 2 | 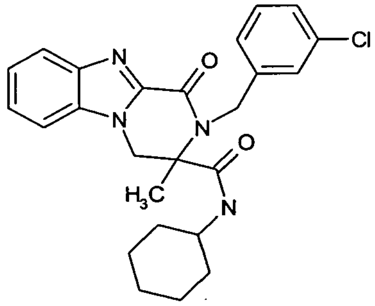 | 450,9725 | 451 | 0.87-1.30 m (6H, cyclohexyl); 1.46-1.71 m (4H, cyclohexyl); 1.60 s (3H, CH3); 3.37 m (1H, CH, cyclohexyl); 4.26, 5.36 dd (2H, CH2, N-CH2-Phm, J=16.8Hz); 4.39, 5.02 dd (2H, CH2, cycle, J=13.6Hz); 7.22 m (1H, CH, Aryl); 7.25-7.33 m (3H, 3CH, Aryl);... |

Table 1.16(1)

| № | Formula | Mol.w. | LC MS, m/z (M+1) | 1H NMR |
|---|---|---|---|---|
| 1 | 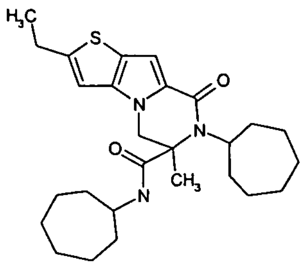 | 469,6948 | 470 | |
| 2 | 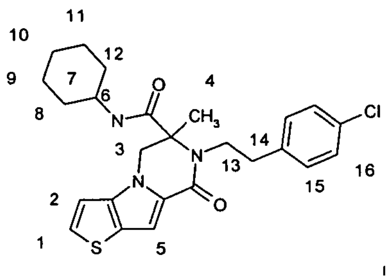 | 470,0378 | 471 | 1.01 - 1.74 (8, 9, 10, 11, 12; 10H; m.), 1.50 (4; 3H, s.), 2.94 (14; 2H; m.), 3.32 + 3.76 (13; 2H; m., m.), 3.51 (7; 1 H; b.m.), 4.08 + 4.73 (3; 2H; AB; 12.8 Hz), 6.95 (5; 1 H; s.), 7.01 (2; 1H; d.; 5.3 Hz), 7.27 (6, 15, 16; 5H; m.), 7.39 (1; 1 H ; d.;... |
| 3 | 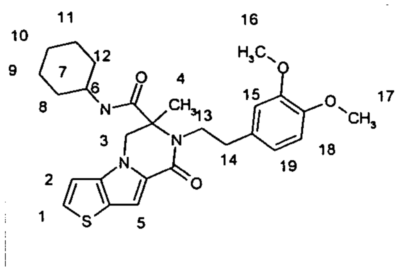 | 495.6458 | 496 | '0.96 - 1.72 (8, 9, 10, 11, 12; 10H; m.), 1.49 (4; 3H, s.), 2.85 (14; 2H; m.), 3.31 + 3.75 (13; 2H; m., m.), 3.51 (7; 1 H; b.m.), 3.77 (16, 17; 6H; s., s.), 4.03 + 4.73 (3; 2H; AB; 12.7 Hz), 6.70 - 6.79 (15, 18, 19; 3H; m.), 6.95 (5; 1 H; s.), 6.99 (2;... |

(continued)

| № | Formula | Mol.w. | LC MS, m/z (M+1) | 1H NMR |
|---|---|---|---|---|
| 4 | 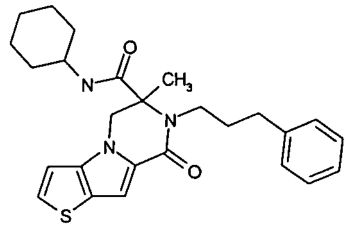 | 449,6199 | 450 | |
| 5 | 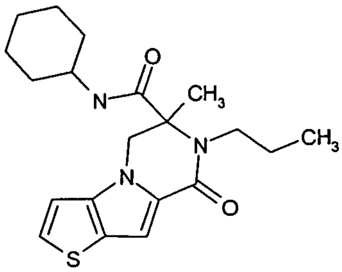 | 373,5211 | 374 | |
| 6 | 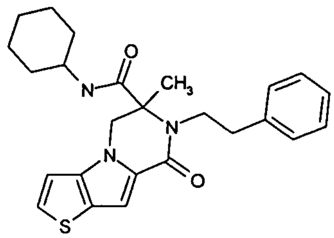 | 435,5928 | 436 | |
| 7 | 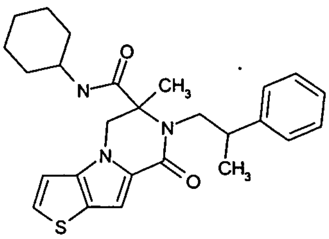 | 449,6199 | 450 | |
| 8 | 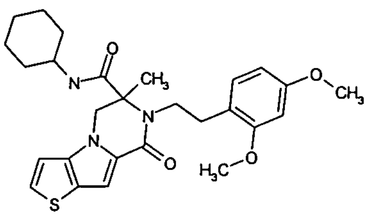 | 495,6458 | 496 | |
| 9 | 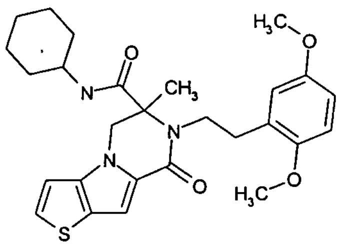 | 495,6458 | 496 | |

(continued)

| № | Formula | Mol.w. | LC MS, m/z (M+1) | 1H NMR |
|---|---|---|---|---|
| 10 | 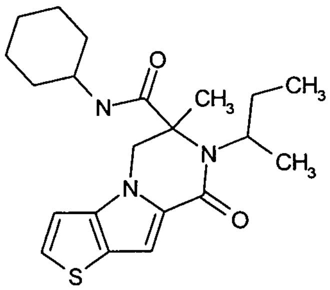 | 387,5482 | 388 | |
| 11 | 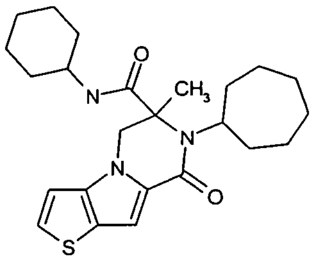 | 427,6135 | 428 | |
| 12 | 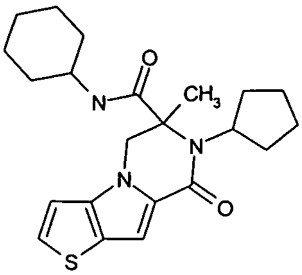 | 399,5593 | 400 | |
| 13 | 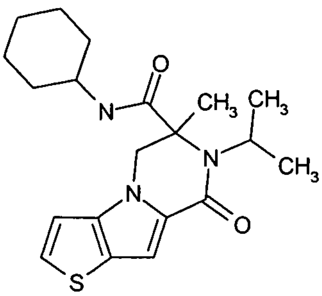 | 373,5211 | 374 | |
| 14 | 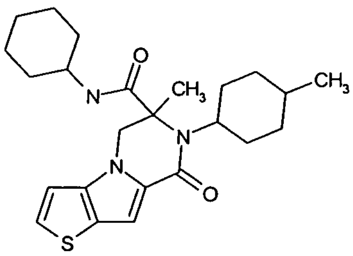 | 427,6135 | 428 | |

(continued)

| № | Formula | Mol.w. | LC MS, m/z (M+1) | 1H NMR |
|---|---|---|---|---|
| 15 | 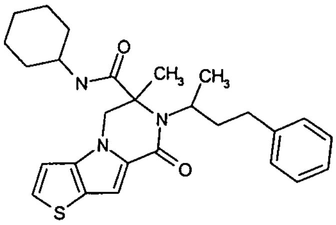 | 463,647 | 464 | |
| 16 | 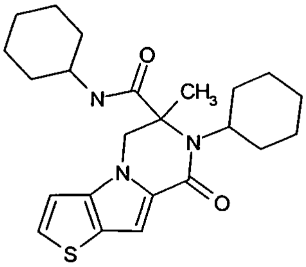 | 413,5864 | 414 | |
| 17 | 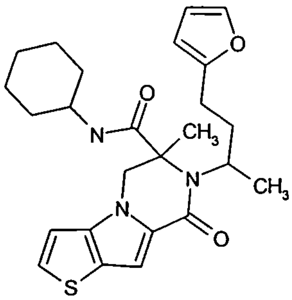 | 453,6081 | 454 | |
| 18 | 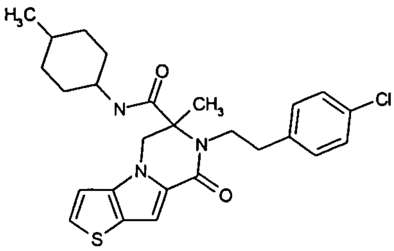 | 84,0649 | 485 | |
| 19 | 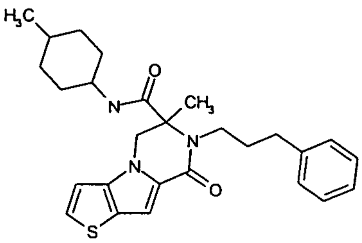 | 463,647 | 464 | |

(continued)

| № | Formula | Mol.w. | LC MS, m/z (M+1) | 1H NMR |
|---|---|---|---|---|
| 20 | 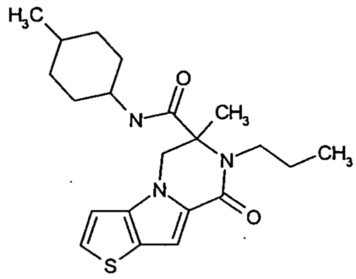 | 387,5482 | 388 | |
| 21 | 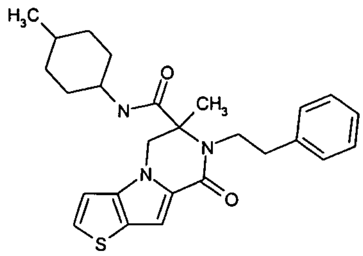 | 449,6199 | 450 | |
| 22 | 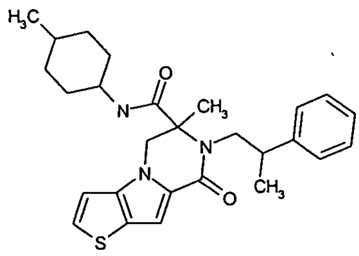 | 463,647 | 464 | |
| 23 | 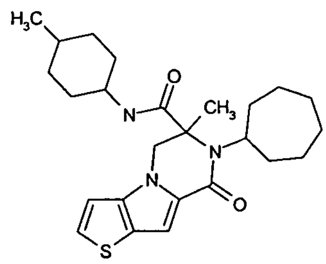 | 441,6406 | 442 | |
| 24 | 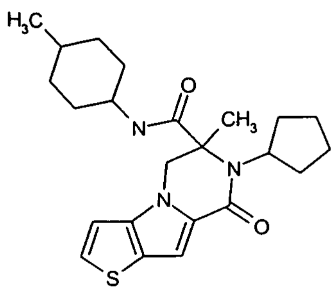 | 413,5864 | 414 | |

(continued)

| № | Formula | Mol.w. | LC MS, m/z (M+1) | 1H NMR |
|---|---|---|---|---|
| 25 | 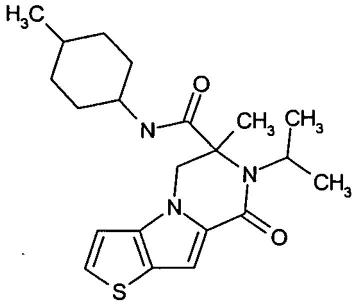 | 387,5482 | 388 | |
| 26 | 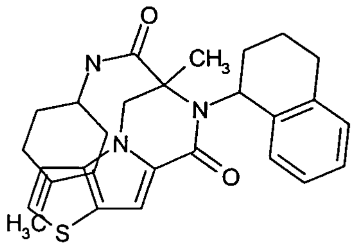 | 475,6581 | 476 | |
| 27 | 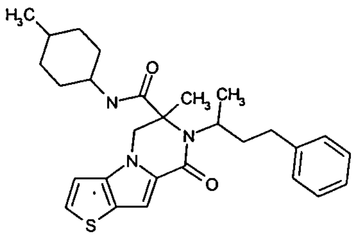 | 477,6741 | 478 | |
| 28 | 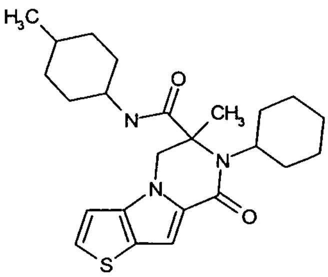 | 427,6135 | 428 | |
| 29 | 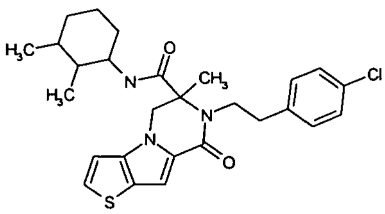 | 498,092 | 499 | |
| 30 | 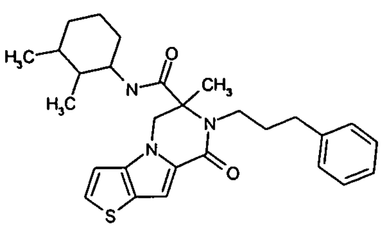 | 77,6741 | 478 | |

(continued)

| № | Formula | Mol.w. | LC MS, m/z (M+1) | 1H NMR |
|---|---|---|---|---|
| 31 | 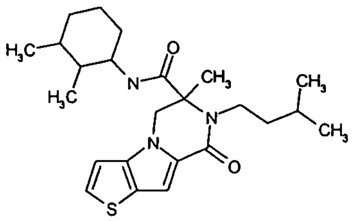 | 429,6295 | 430 | |
| 32 | 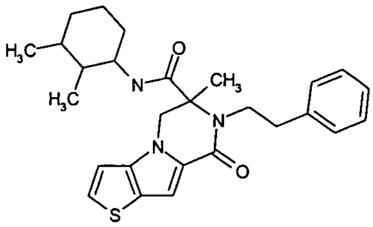 | 463,647 | 464 | |
| 33 | 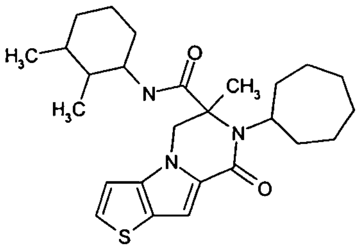 | 455,6677 | 456 | |
| 34 | 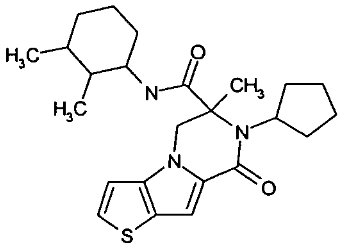 | 427,6135 | 428 | |
| 35 | 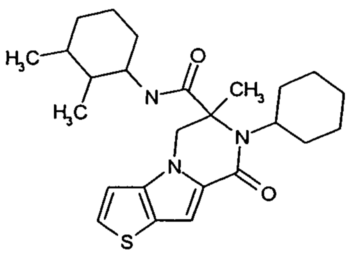 | 441,6406 | 442 | |
| 36 | 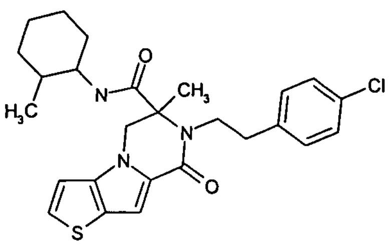 | 84,0649 | 485 | |

(continued)

| № | Formula | Mol.w. | LC MS, m/z (M+1) | 1H NMR |
|---|---|---|---|---|
| 37 | 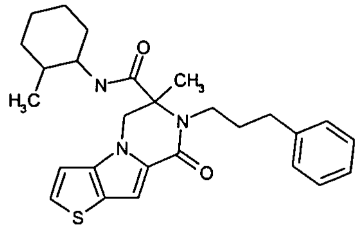 | 463,647 | 464 | |
| 38 | 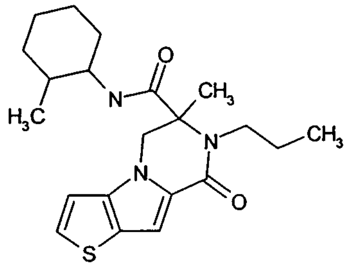 | 387,5482 | 388 | |
| 39 | 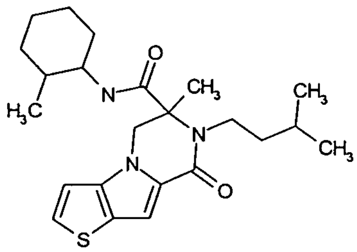 | 415,6024 | 416 | |
| 40 | 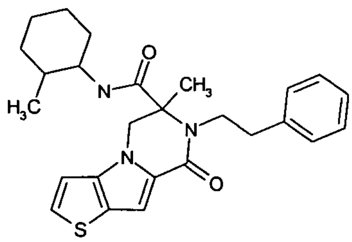 | 449,6199 | 450 | |
| 41 | 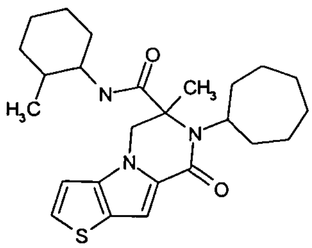 | 441,6406 | 442 | |
| 42 | 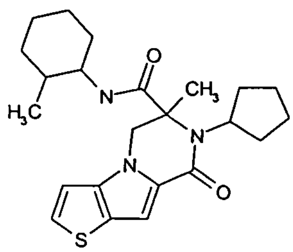 | 413,5864 | 414 | |

(continued)

| № | Formula | Mol.w. | LC MS, m/z (M+1) | 1H NMR |
|---|---|---|---|---|
| 43 | 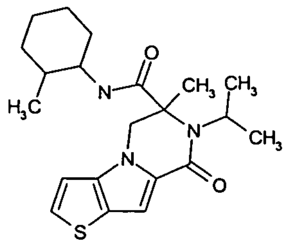 | 387,5482 | 388 | |
| 44 | 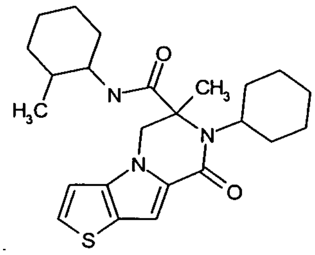 | 427,6135 | 428 | |
| 45 | 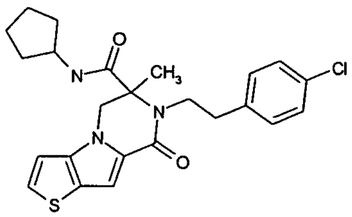 | 456,0107 | 457 | |
| 46 | 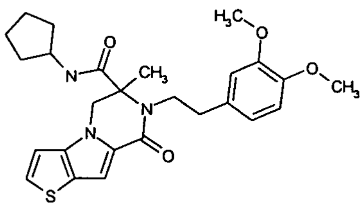 | 481,6187 | 482 | |
| 47 | 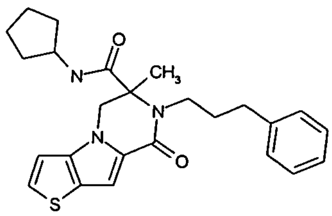 | 435,5928 | 436 | |
| 48 | 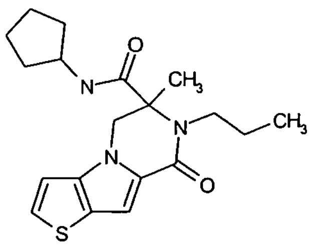 | 359,494 | 360 | |

(continued)

| № | Formula | Mol.w. | LC MS, m/z (M+1) | 1H NMR |
|---|---|---|---|---|
| 49 | 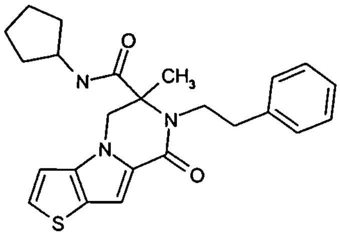 | 421,5657 | 422 | |
| 50 | 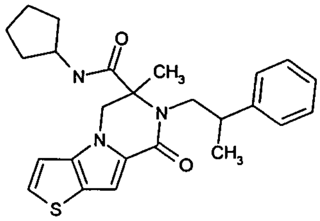 | 435,5928 | 436 | |
| 51 | 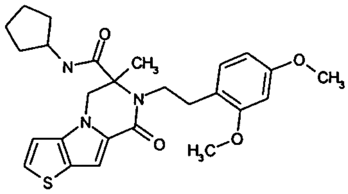 | 481,6187 | 482 | |
| 52 | 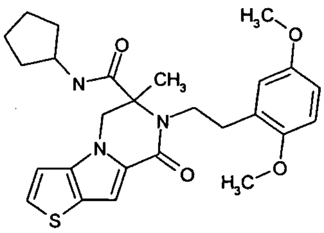 | 481,6187 | 482 | |
| 53 | 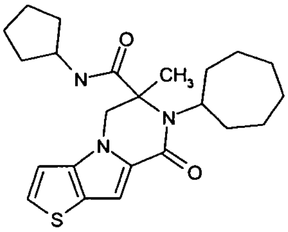 | 413,5864 | 414 | |
| 54 | 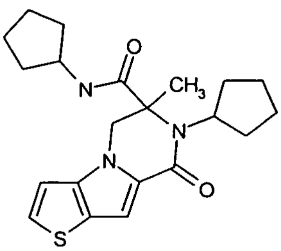 | 385,5322 | 386 | |

(continued)

| № | Formula | Mol.w. | LC MS, m/z (M+1) | 1H NMR |
|---|---|---|---|---|
| 55 | 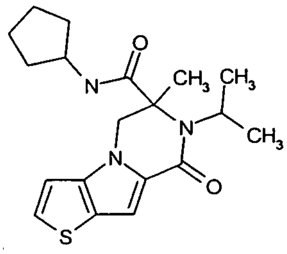 | 359,494 | 360 | |
| 56 | 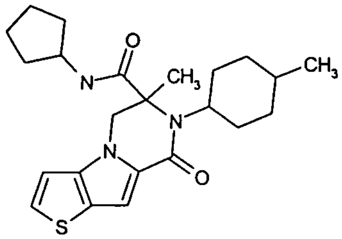 | 413,5864 | 414 | |
| 57 | 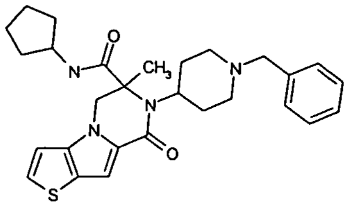 | 490,6728 | 491 | |
| 58 | 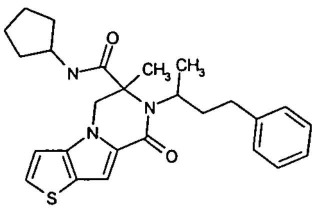 | 449,6199 | 450 | |
| 59 | 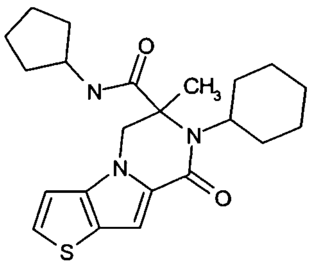 | 399,5593 | 400 | |
| 60 | 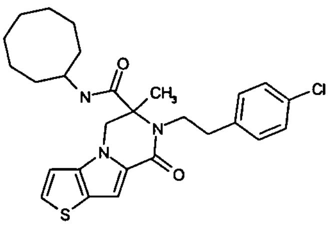 | 498,092 | 499 | |

(continued)

| № | Formula | Mol.w. | LC MS, m/z (M+1) | 1H NMR |
|---|---|---|---|---|
| 61 | 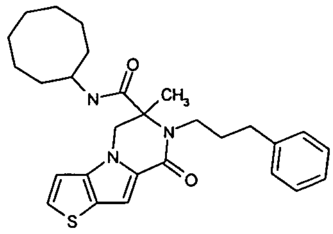 | 477,6741 | 478 | |
| 62 | 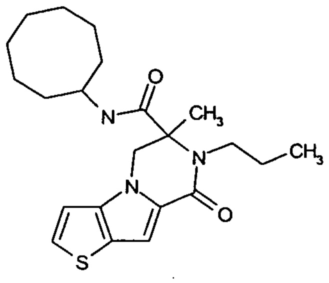 | 401,5753 | 402 | |
| 63 | 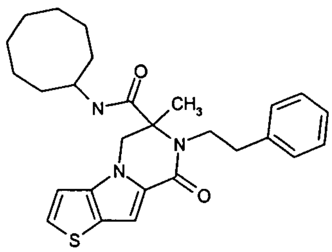 | 463,647 | 464 | |
| 64 | 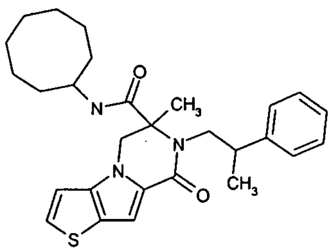 | 477,6741 | 478 | |
| 65 | 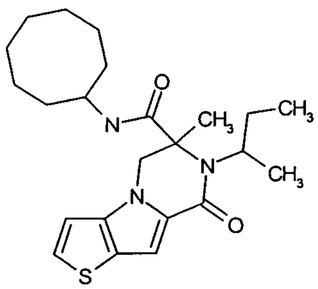 | 415,6024 | 416 | |

(continued)

| № | Formula | Mol.w. | LC MS, m/z (M+1) | 1H NMR |
|---|---|---|---|---|
| 66 | 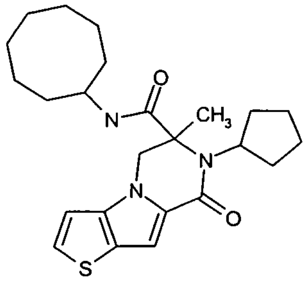 | 427,6135 | 428 | |
| 67 | 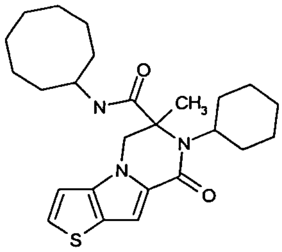 | 441,6406 | 442 | |
| 68 | 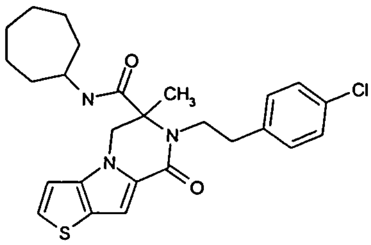 | 484,0649 | 485 | |
| 69 | 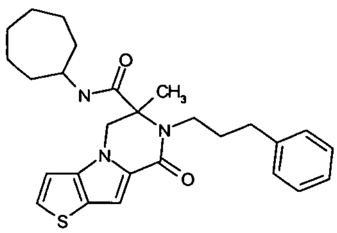 | 463,647 | 464 | |
| 70 | 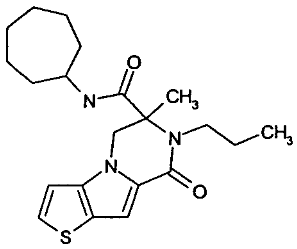 | 387,5482 | 388 | |
| 71 | 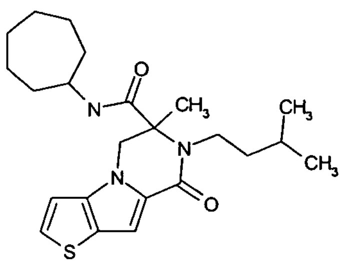 | 415,6024 | 416 | |

(continued)

| № | Formula | Mol.w. | LC MS, m/z (M+1) | 1H NMR |
|---|---|---|---|---|
| 72 | 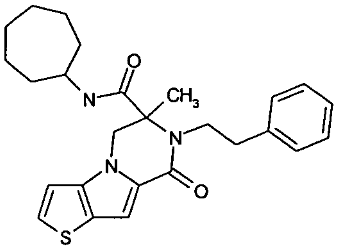 | 449,6199 | 450 | |
| 73 | 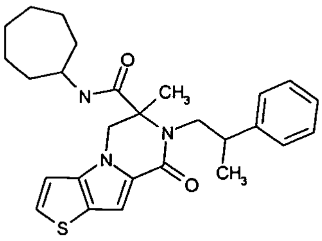 | 463,647 | 464 | |
| 74 | 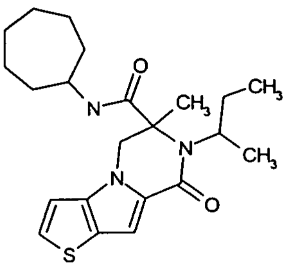 | 401,5753 | 402 | |
| 75 | 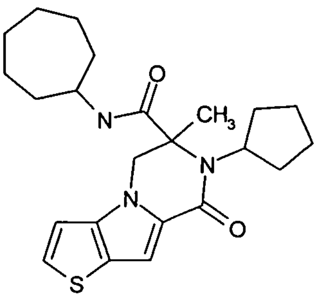 | 413,5864 | 414 | |
| 76 | 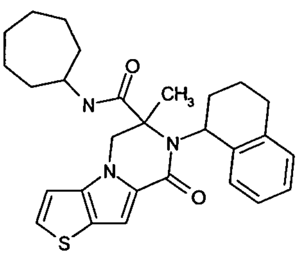 | 475,6581 | 476 | |

(continued)

| № | Formula | Mol.w. | LC MS, m/z (M+1) | 1H NMR |
|---|---|---|---|---|
| 77 | 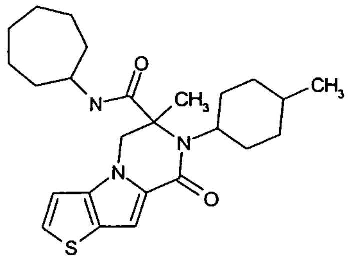 | 441,6406 | 442 | |
| 78 | 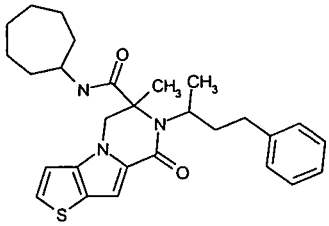 | 477,6741 | 478 | |
| 79 | 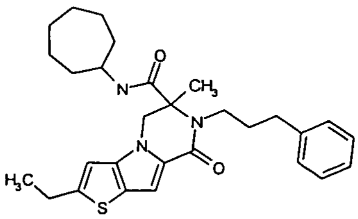 | 491,7011 | 492 | |
| 80 | 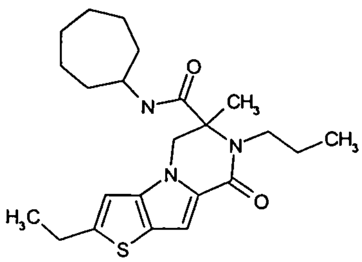 | 415,6024 | 416 | |
| 81 | 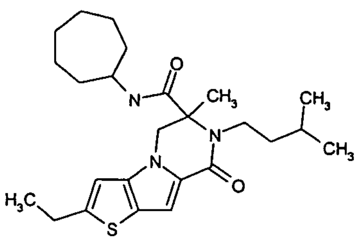 | 443,6565 | 444 | |
| 82 | 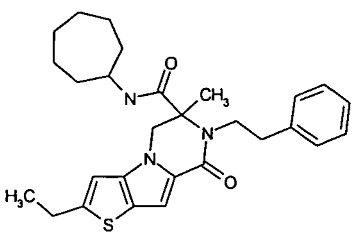 | 477,6741 | 478 | |

(continued)

| № | Formula | Mol.w. | LC MS, m/z (M+1) | 1H NMR |
|---|---|---|---|---|
| 83 | 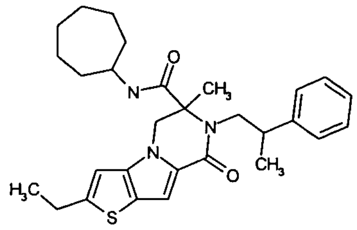 | 491,7011 | 492 | |
| 84 | 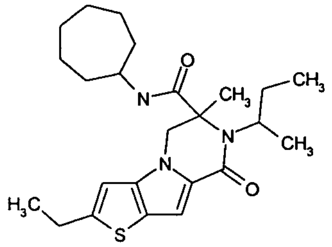 | 429,6295 | 430 | |
| 85 | 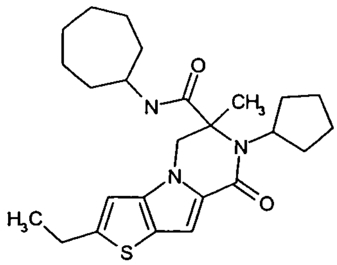 | 441,6406 | 442 | |
| 86 | 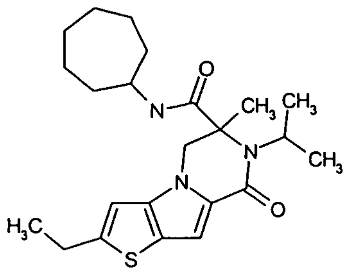 | 415,6024 | 416 | |
| 87 | 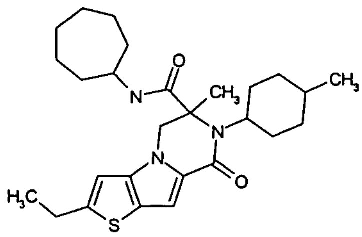 | 469,6948 | 470 | |
| 88 | 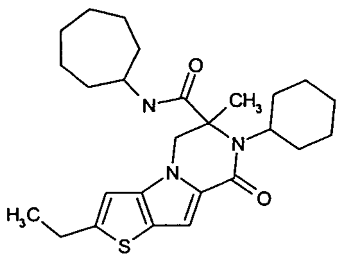 | 455,6677 | 456 | |

(continued)

| № | Formula | Mol.w. | LC MS, m/z (M+1) | 1H NMR |
|---|---|---|---|---|
| 89 | 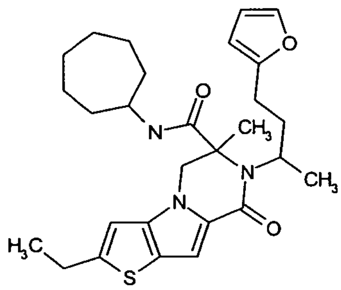 | 495,6894 | 496 | |
| 90 | 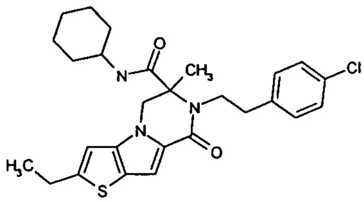 | 498,092 | 499 | |
| 91 | 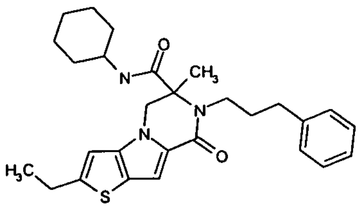 | 477,6741 | 478 | |
| 92 | 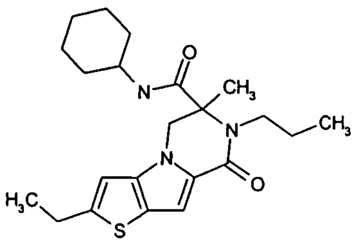 | 401,5753 | 402 | |
| 93 | 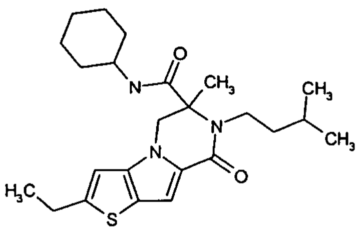 | 429,6295 | 430 | |
| 94 | 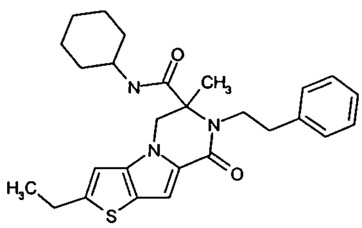 | 463,647 | 464 | |

(continued)

| № | Formula | Mol.w. | LC MS, m/z (M+1) | 1H NMR |
|---|---|---|---|---|
| 95 | 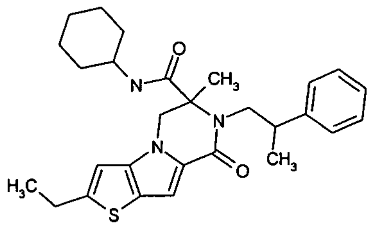 | 477,6741 | 478 | |
| 96 | 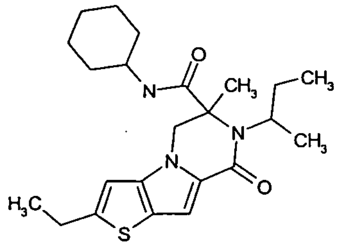 | 415,6024 | 416 | |
| 97 | 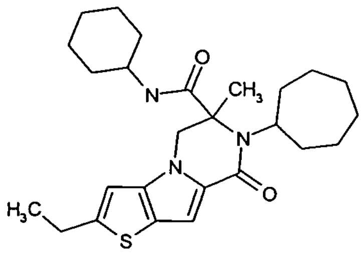 | 455,6677 | 456 | |
| 98 | 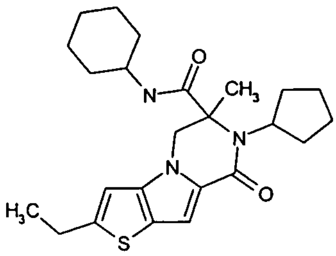 | 427,6135 | 428 | |
| 99 | 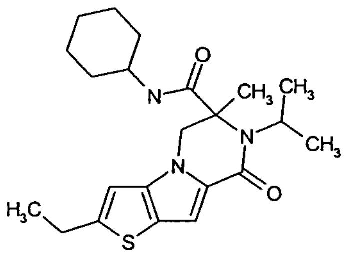 | 401,5753 | 402 | |
| 100 | 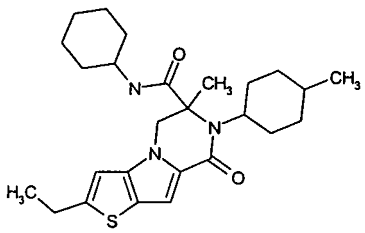 | 455,6677 | 456 | |

(continued)

| № | Formula | Mol.w. | LC MS, m/z (M+1) | 1H NMR |
|---|---|---|---|---|
| 101 | 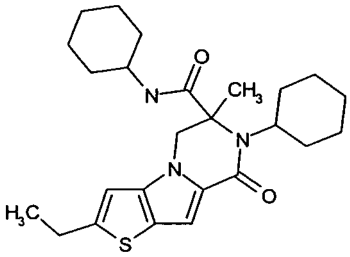 | 441,6406 | 442 | |
| 102 | 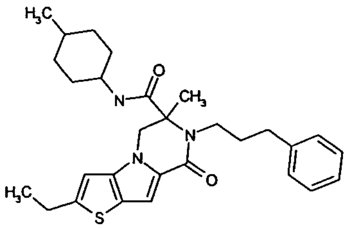 | 491,7011 | 492 | |
| 103 | 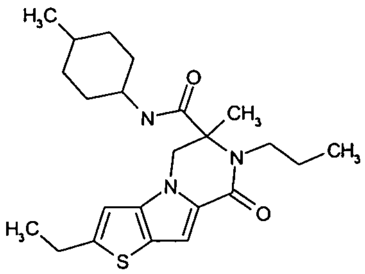 | 415,6024 | 416 | |
| 104 | 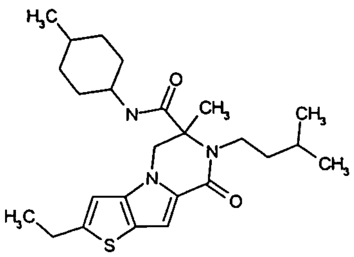 | 443,6565 | 444 | |
| 105 | 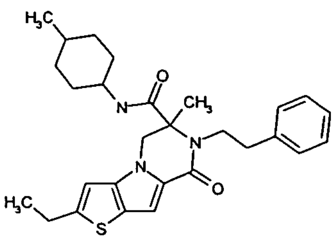 | 477,6741 | 478 | |
| 106 | 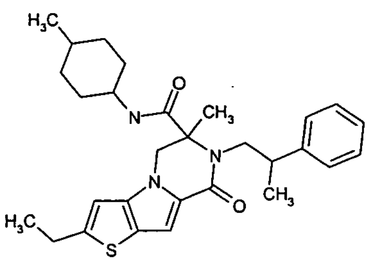 | 491,7011 | 492 | |

(continued)

| № | Formula | Mol.w. | LC MS, m/z (M+1) | 1H NMR |
|---|---|---|---|---|
| 107 | 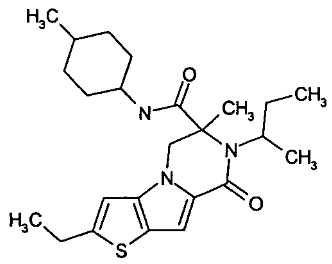 | 429,6295 | 430 | |
| 108 | 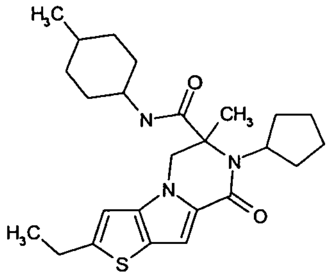 | 441,6406 | 442 | |
| 109 | 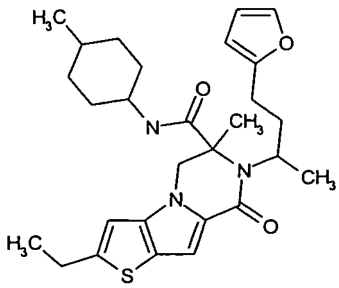 | 495,6894 | 496 | |

Table 1.16(2)

| № | Formula | Mol.w. | LC MS, m/z (M+1) | 1H NMR |
|---|---|---|---|---|
| 1 | 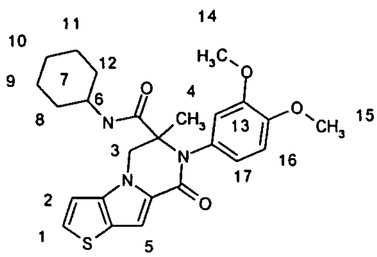 | 467,5916 | 468 | 0.96 - 1.71 (8, 9, 10, 11, 12; 10H; m.), 1.33 (4; 3H, s.), 3.47 (7; 1 H; b.m.), 3.80, 3.84 (14, 15; 6H; s., s.), 4.27 + 4.95 (3; 2H; AB; 12.5 Hz), 6.85 (2, 13; 2H; m.), 6.92 (5; 1H; s.), 6.99 (16, 17; 2H; m.), 7.29 (1; 1 H ; d.; 5.7 Hz), 7.39 (6; 1H; ... |
| 2 | 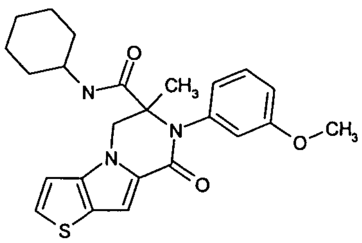 | 437,5651 | 438 | |

(continued)

| № | Formula | Mol.w. | LC MS, m/z (M+1) | 1H NMR |
|---|---|---|---|---|
| 3 | 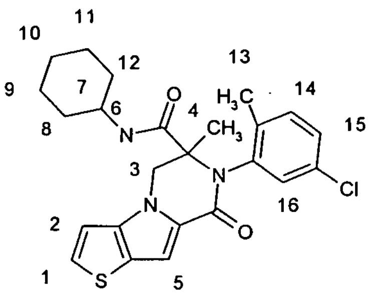 | 456,0107 | 457 | 0.81 - 1.77 (8, 9, 10, 11, 12; 10H; m.), 1.26 (4; 3H, s.), 2.18 (13; 3H; s.), 3.47 (7; 1 H; b.m.), 4.30 + 5.07 (3; 2H; AB; 13.3 Hz), 6.93 (5; 1 H; s.), 6.96 (2; 1 H; d.; 5.3 Hz), 7.24 (14, 15; 2H; m.), 7.34 (1; 1H ; d.; 5.3 Hz), 7.59 (16; 1 H; s.), 7.66... |
| 4 | 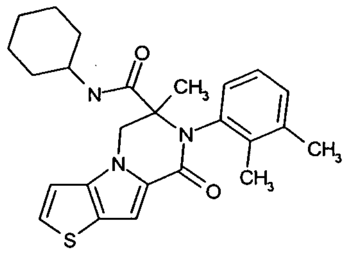 | 435,5928 | 436 | |
| 5 | 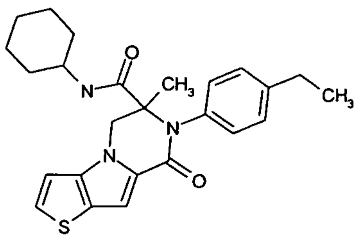 | 435,5928 | 436 | |
| 6 | 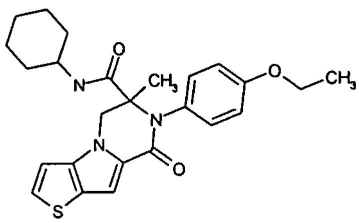 | 451,5922 | 452 | |
| 7 | 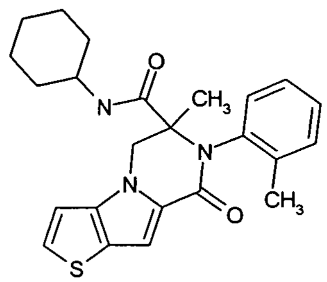 | 421,5657 | 422 | |

(continued)

| № | Formula | Mol.w. | LC MS, m/z (M+1) | 1H NMR |
|---|---|---|---|---|
| 8 | 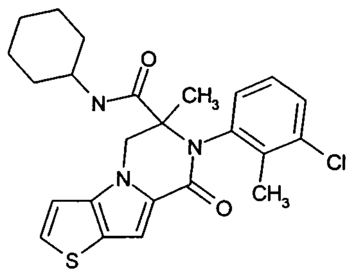 | 456,0107 | 457 | |
| 9 | 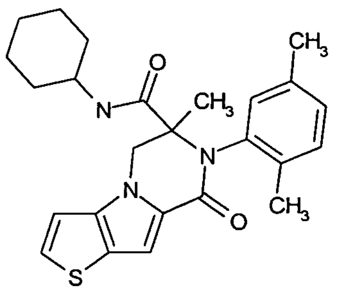 | 435,5928 | 436 | |
| 10 | 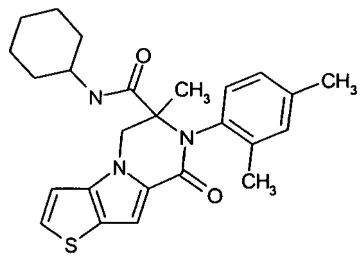 | 435,5928 | 436 | |
| 11 | 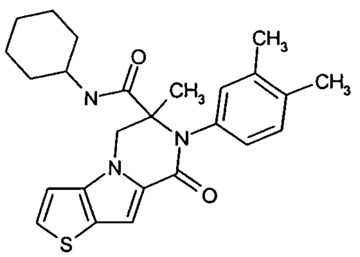 | 435,5928 | 436 | |
| 12 | 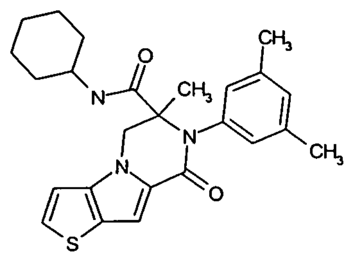 | 435,5928 | 436 | |

(continued)

| № | Formula | Mol.w. | LC MS, m/z (M+1) | 1H NMR |
|---|---|---|---|---|
| 13 | 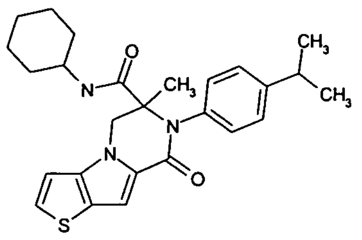 | 449,6199 | 450 | |
| 14 | 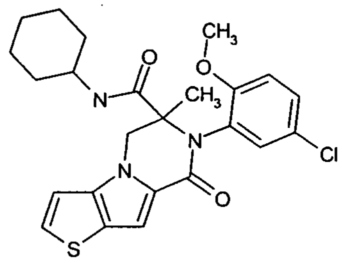 | 472,0101 | 473 | |
| 15 | 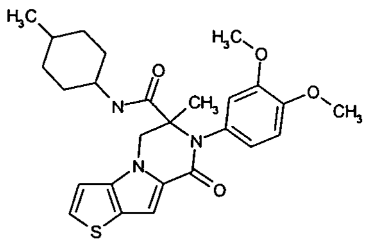 | 481,6187 | 482 | |
| 16 | 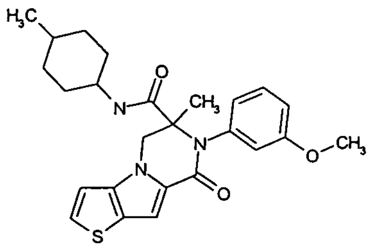 | 451,5922 | 452 | |
| 17 | 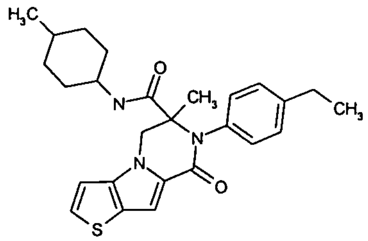 | 449,6199 | 450 | |
| 18 | 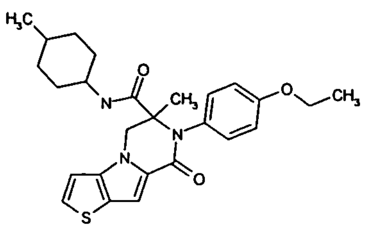 | 465,6193 | 466 | |

(continued)

| № | Formula | Mol.w. | LC MS, m/z (M+1) | 1H NMR |
|---|---|---|---|---|
| 19 | 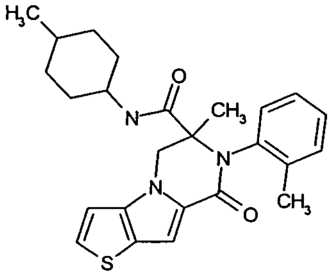 | 435,5928 | 436 | |
| 20 | 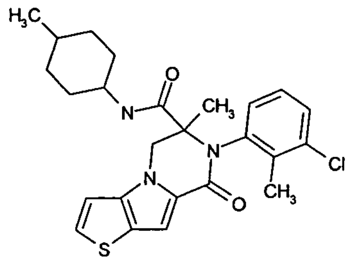 | 470,0378 | 470 | |
| 21 | 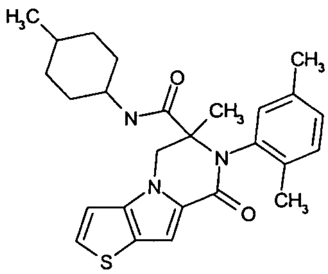 | 449,6199 | 450 | |
| 22 | 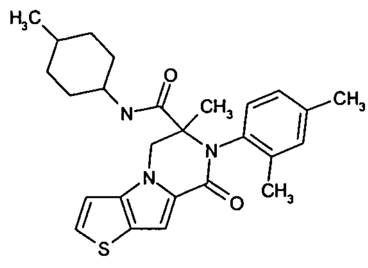 | 449,6199 | 450 | |
| 23 | 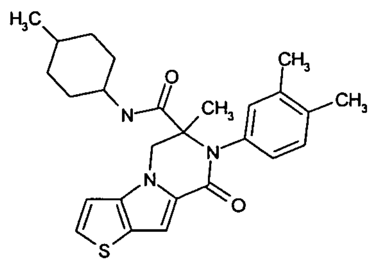 | 449,6199 | 450 | |

(continued)

| № | Formula | Mol.w. | LC MS, m/z (M+1) | 1H NMR |
|---|---|---|---|---|
| 24 | 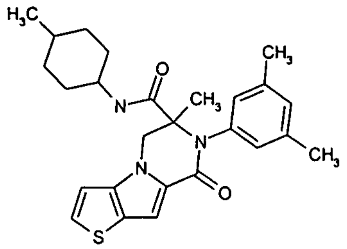 | 449,6199 | 450 | |
| 25 | 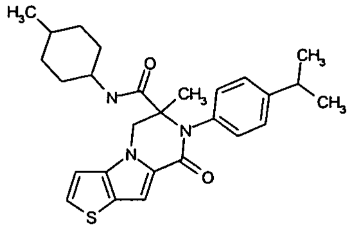 | 463,647 | 464 | |
| 26 | 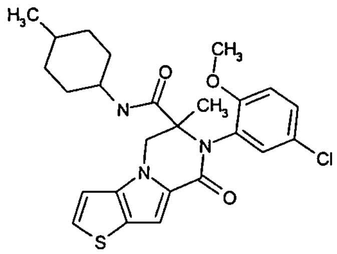 | 486,0372 | 486 | |
| 27 | 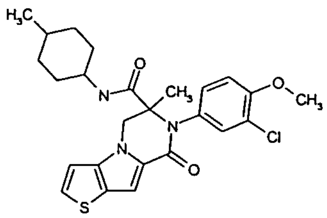 | 486,0372 | 487 | |
| 28 | 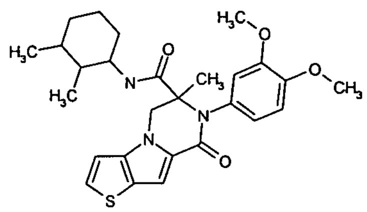 | 495,6458 | 496 | |
| 29 | 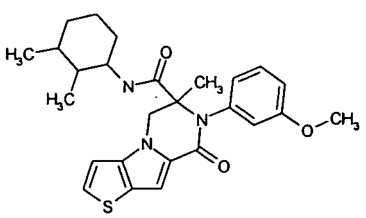 | 465,6193 | 466 | |

(continued)

| № | Formula | Mol.w. | LC MS, m/z (M+1) | 1H NMR |
|---|---|---|---|---|
| 30 | 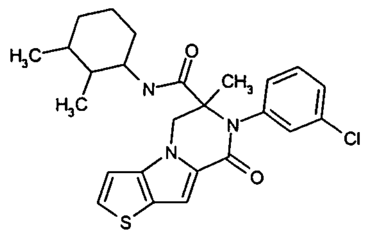 | 470,0378 | 471 | |
| 31 | 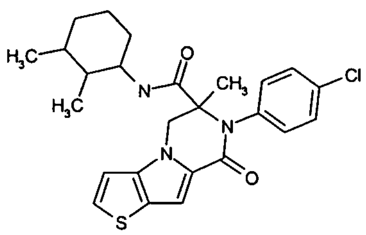 | 470,0378 | 471 | |
| 32 | 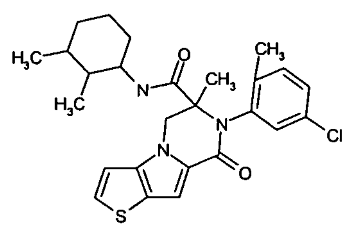 | 484,0649 | 485 | |
| 33 | 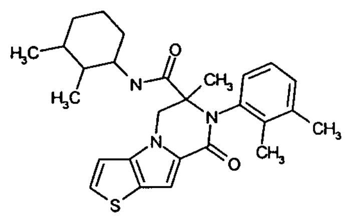 | 463,647 | 464 | |
| 34 | 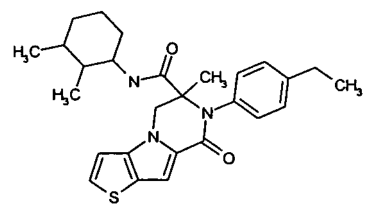 | 463,647 | 464 | |
| 35 | 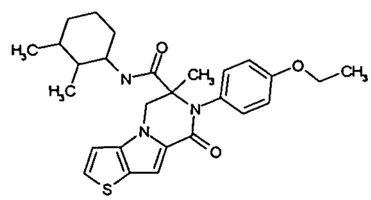 | 479,6464 | 480 | |

(continued)

| № | Formula | Mol.w. | LC MS, m/z (M+1) | 1H NMR |
|---|---|---|---|---|
| 36 | 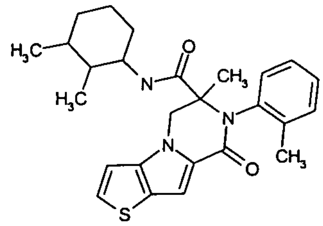 | 449,6199 | 450 | |
| 37 | 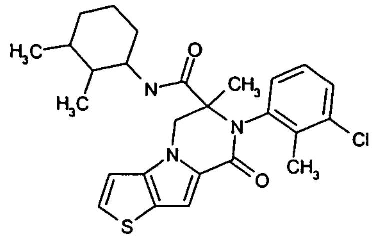 | 484,0649 | 485 | |
| 38 | 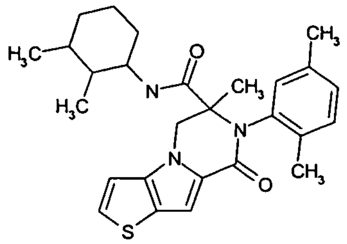 | 463,647 | 464 | |
| 39 | 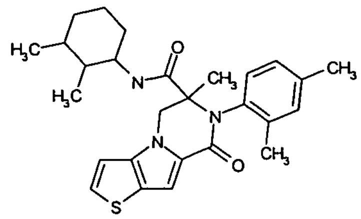 | 463,647 | 464 | |
| 40 | 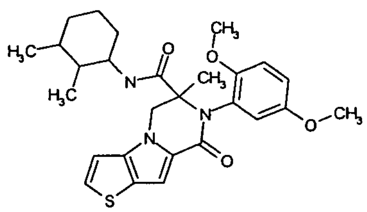 | 495,6458 | 496 | |
| 41 | 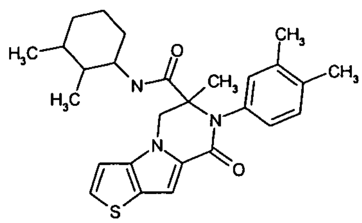 | 463,647 | 464 | |

(continued)

| № | Formula | Mol.w. | LC MS, m/z (M+1) | 1H NMR |
|---|---|---|---|---|
| 42 | 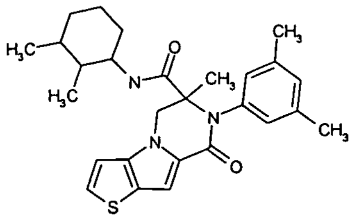 | 463,647 | 464 | |
| 43 | 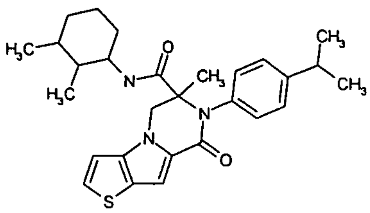 | 477,6741 | 478 | |
| 44 | 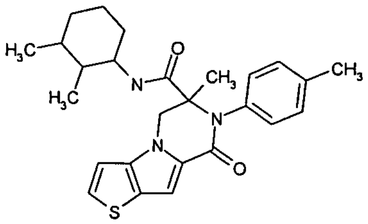 | 449,6199 | 450 | |
| 45 | 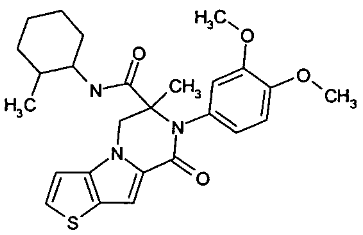 | 481,6187 | 482 | |
| 46 | 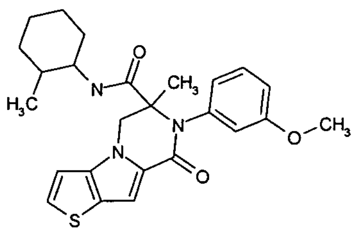 | 451,5922 | 452 | |
| 47 | 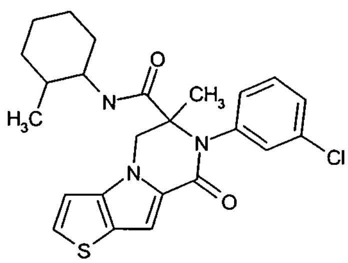 | 456,0107 | 457 | |

(continued)

| № | Formula | Mol.w. | LC MS, m/z (M+1) | 1H NMR |
|---|---|---|---|---|
| 48 | 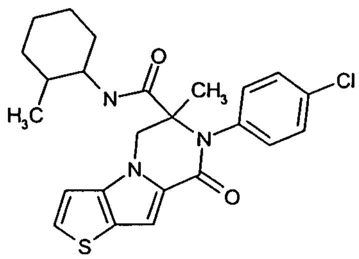 | 456,0107 | 457 | |
| 49 | 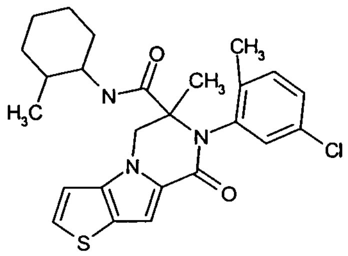 | 470,0378 | 471 | |
| 50 | 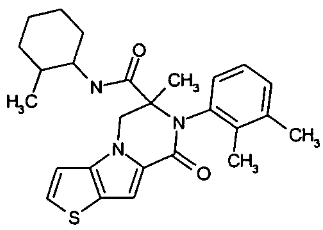 | 449,6199 | 450 | |
| 51 | 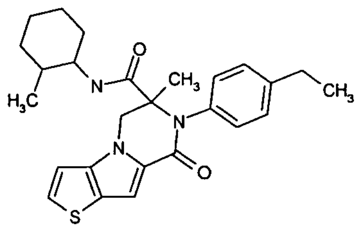 | 449,6199 | 450 | |
| 52 | 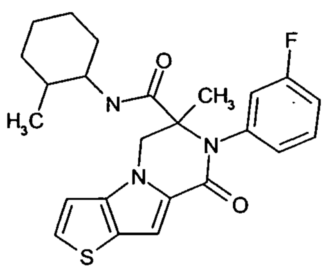 | 439,5561 | 440 | |
| 53 | 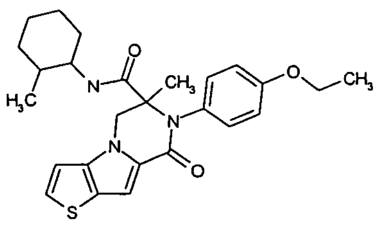 | 465,6193 | 466 | |

(continued)

| № | Formula | Mol.w. | LC MS, m/z (M+1) | 1H NMR |
|---|---|---|---|---|
| 54 | 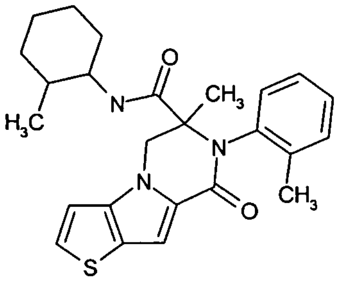 | 435,5928 | 436 | |
| 55 | 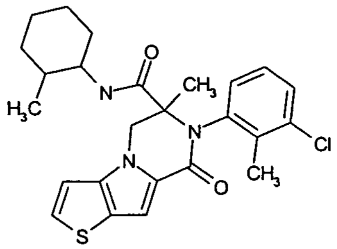 | 470,0378 | 471 | |
| 56 | 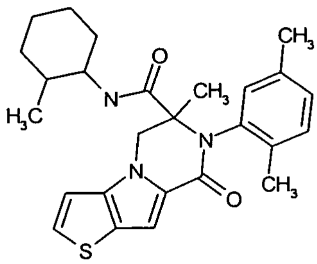 | 449,6199 | 450 | |
| 57 | 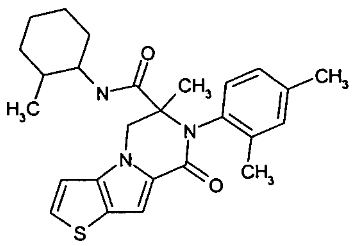 | 449,6199 | 450 | |
| 58 | 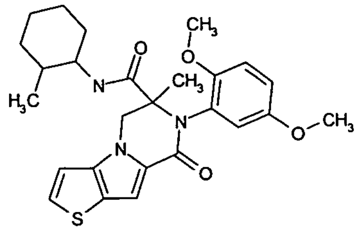 | 481,6187 | 482 | |
| 59 | 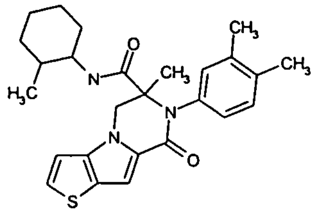 | 449,6199 | 450 | |

(continued)

| № | Formula | Mol.w. | LC MS, m/z (M+1) | 1H NMR |
|---|---|---|---|---|
| 60 | 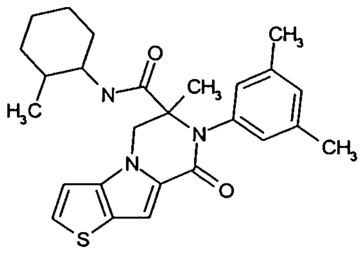 | 449,6199 | 450 | |
| 61 | 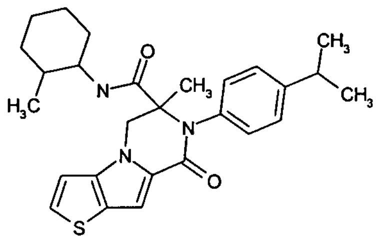 | 463,647 | 464 | |
| 62 | 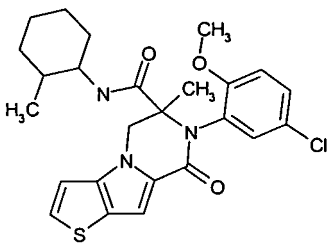 | 486,0372 | 487 | |
| 63 | 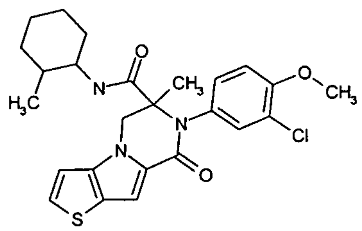 | 486,0372 | 487 | |
| 64 | 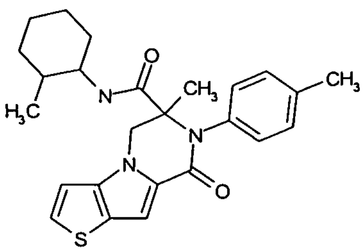 | 435,5928 | 436 | |
| 65 | 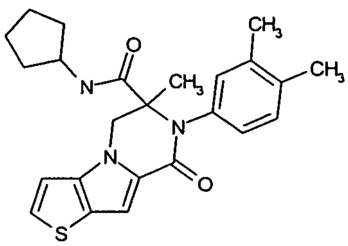 | 421,5657 | 422 | |

(continued)

| Nº | Formula | Mol.w. | LC MS, m/z (M+1) | 1H NMR |
|---|---|---|---|---|
| 66 | 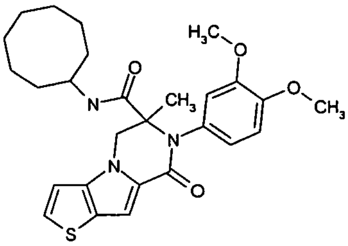 | 495,6458 | 496 | |
| 67 | 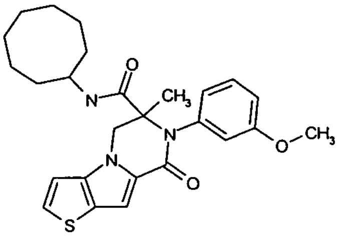 | 465,6193 | 466 | |
| 68 | 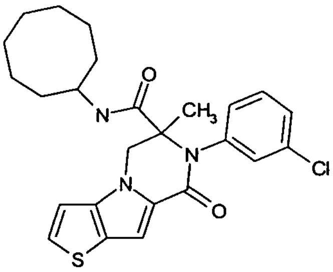 | 470,0378 | 471 | |
| 69 | 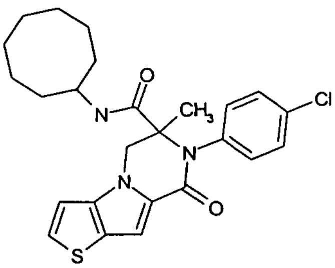 | 470,0378 | 471 | |
| 70 | 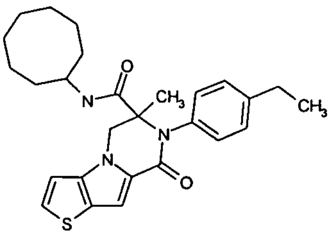 | 463,647 | 464 | |
| 71 | 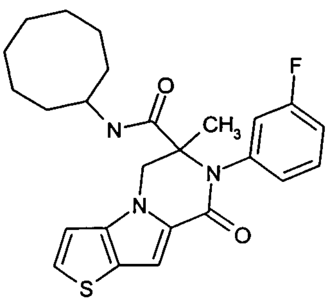 | 453,5832 | 454 | |

(continued)

| № | Formula | Mol.w. | LC MS, m/z (M+1) | 1H NMR |
|---|---|---|---|---|
| 72 | 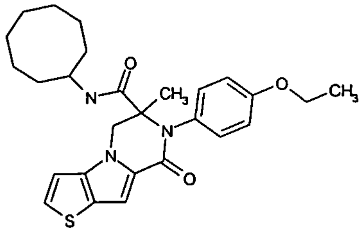 | 479,6464 | 480 | |
| 73 | 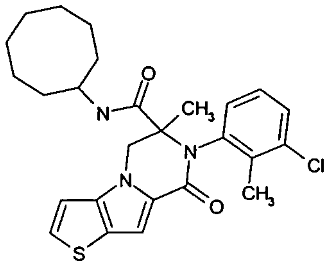 | 484,0649 | 485 | |
| 74 | 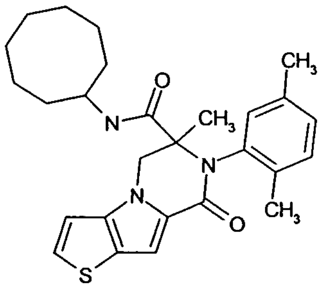 | 463,647 | 464 | |
| 75 | 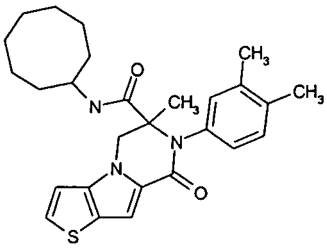 | 463,647 | 464 | |
| 76 | 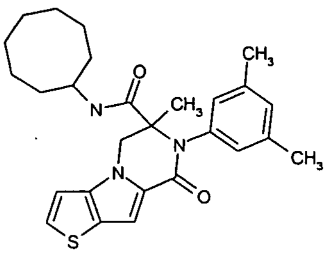 | 463,647 | 464 | |
| 77 | 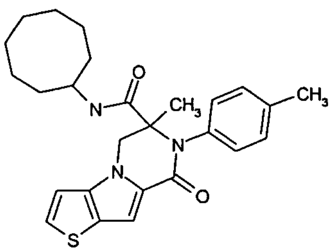 | 449,6199 | 450 | |

(continued)

| № | Formula | Mol.w. | LC MS, m/z (M+1) | 1H NMR |
|---|---|---|---|---|
| 78 | 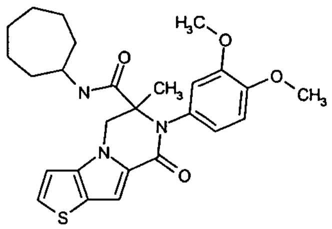 | 481,6187 | 482 | |
| 79 | 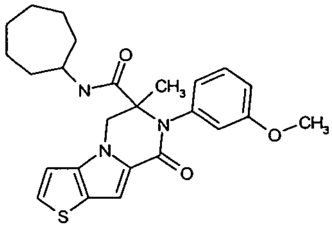 | 451,5922 | 452 | |
| 80 | 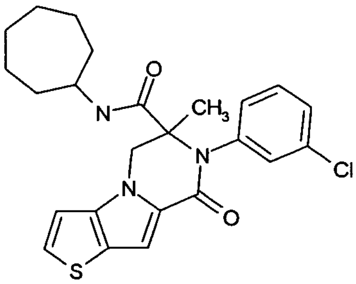 | 456,0107 | 457 | |
| 81 | 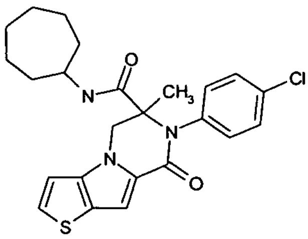 | 456,0107 | 457 | |
| 82 | 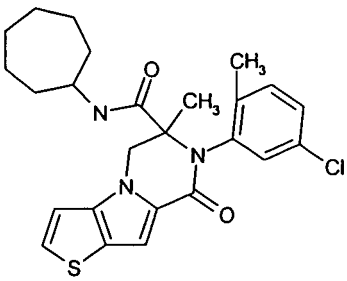 | 470,0378 | 471 | |

(continued)

| № | Formula | Mol.w. | LC MS, m/z (M+1) | 1H NMR |
|---|---|---|---|---|
| 83 | 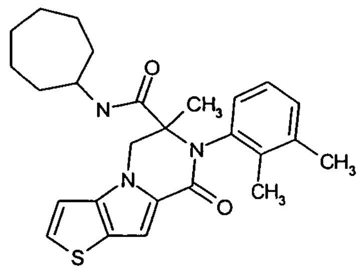 | 449,6199 | 450 | |
| 84 | 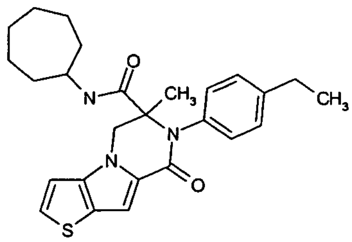 | 449,6199 | 450 | |
| 85 | 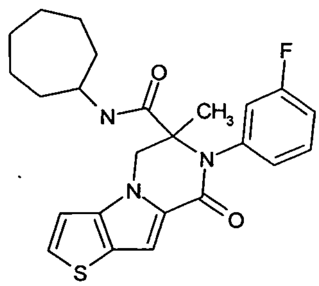 | 439,5561 | 440 | |
| 86 | 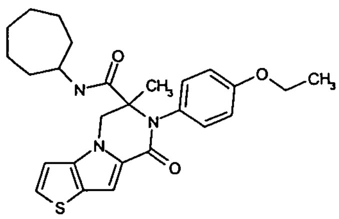 | 465,6193 | 466 | |
| 87 | 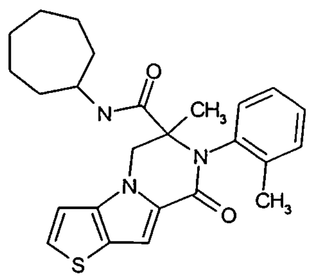 | 435,5928 | 436 | |
| 88 | 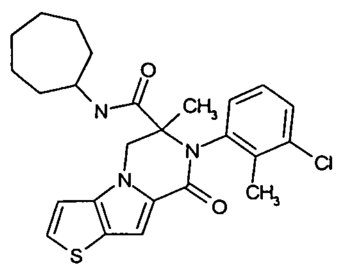 | 470,0378 | 471 | |

(continued)

| № | Formula | Mol.w. | LC MS, m/z (M+1) | 1H NMR |
|---|---|---|---|---|
| 89 | 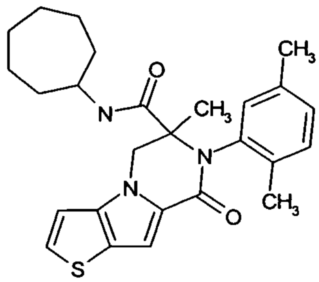 | 449,6199 | 450 | |
| 90 | 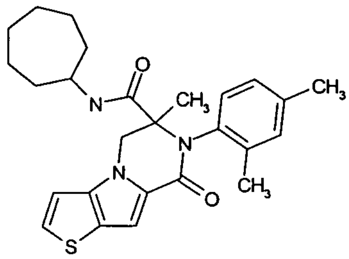 | 449,6199 | 450 | |
| 91 | 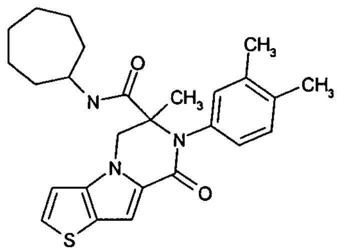 | 449,6199 | 450 | |
| 92 | 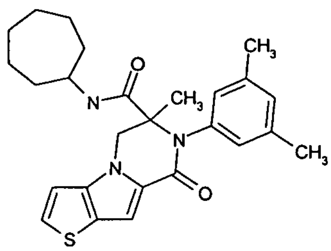 | 449,6199 | 450 | |
| 93 | 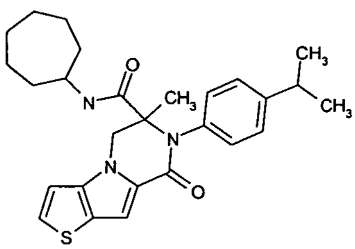 | 463,647 | 464 | |

(continued)

| № | Formula | Mol.w. | LC MS, m/z (M+1) | 1H NMR |
|---|---|---|---|---|
| 94 | 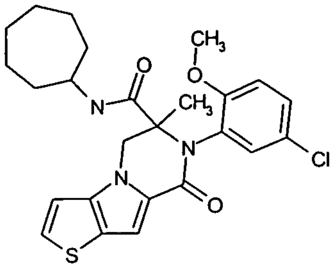 | 486,0372 | 487 | |
| 95 | 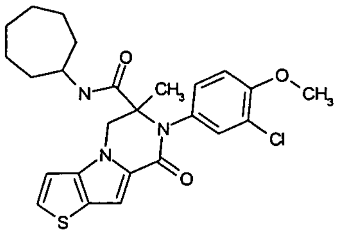 | 486,0372 | 487 | |
| 96 | 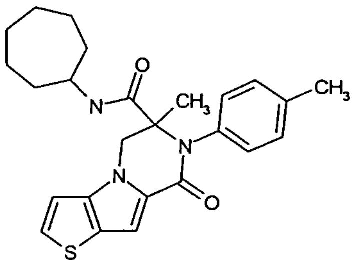 | 435,5928 | 436 | |
| 97 | 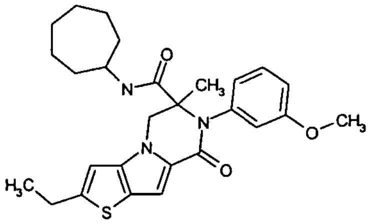 | 479,6464 | 480 | |
| 98 | 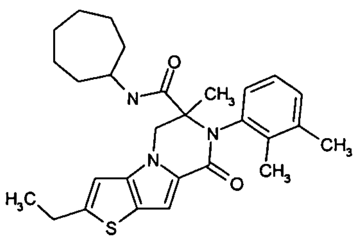 | 477,6741 | 478 | |
| 99 | 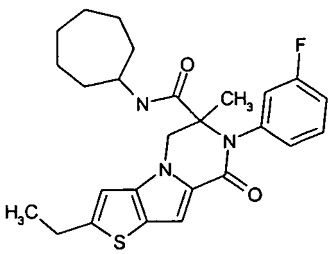 | 467,6103 | 468 | |

(continued)

| № | Formula | Mol.w. | LC MS, m/z (M+1) | 1H NMR |
|---|---|---|---|---|
| 100 | 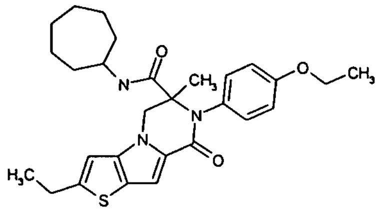 | 493,6735 | 494 | |
| 101 | 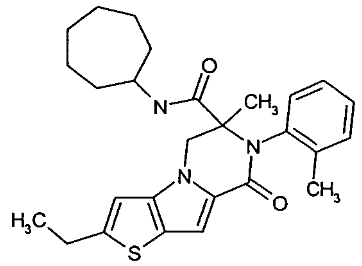 | 463,647 | 464 | |
| 102 | 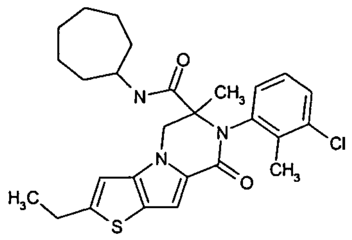 | 498,092 | 499 | |
| 103 | 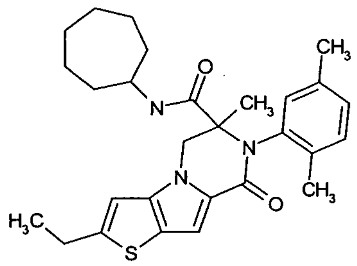 | 477,6741 | 478 | |
| 104 | 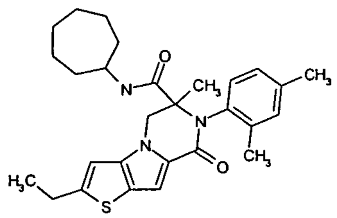 | 477,6741 | 478 | |
| 105 | 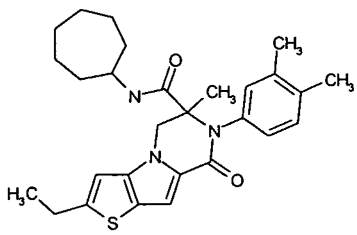 | 477,6741 | 478 | |

(continued)

| № | Formula | Mol.w. | LC MS, m/z (M+1) | 1H NMR |
|---|---|---|---|---|
| 106 | 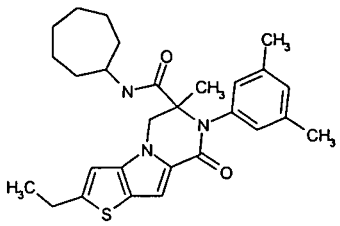 | 477,6741 | 478 | |
| 107 | 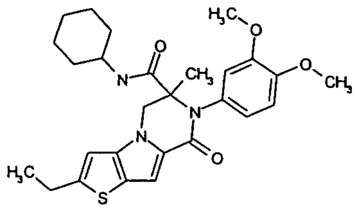 | 495,6458 | 496 | |
| 108 | 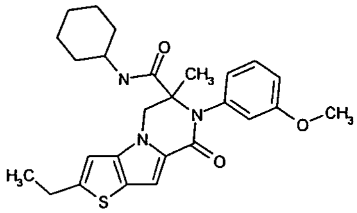 | 465,6193 | 466 | |
| 109 | 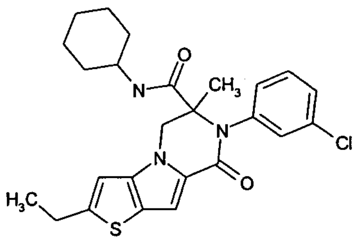 | 470,0378 | 471 | |
| 110 | 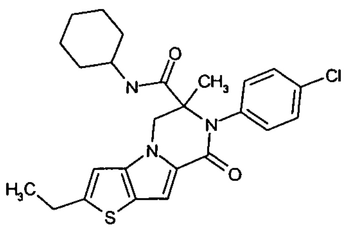 | 470,0378 | 471 | |
| 111 | 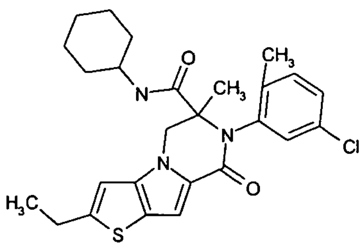 | 484,0649 | 485 | |

(continued)

| № | Formula | Mol.w. | LC MS, m/z (M+1) | 1H NMR |
|---|---|---|---|---|
| 112 | 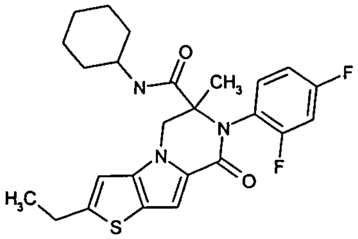 | 471,5736 | 472 | |
| 113 | 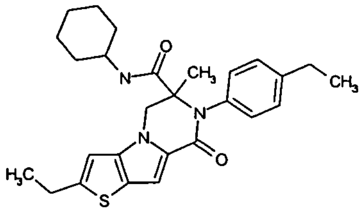 | 463,647 | 464 | |
| 114 | 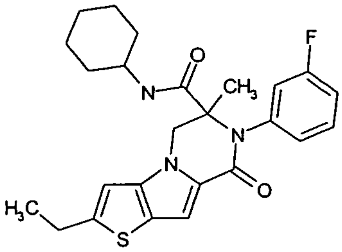 | 453,5832 | 454 | |
| 115 | 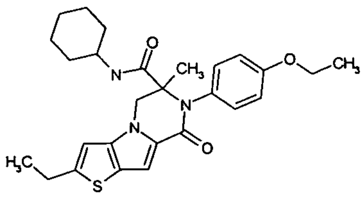 | 479,6464 | 480 | |
| 116 | 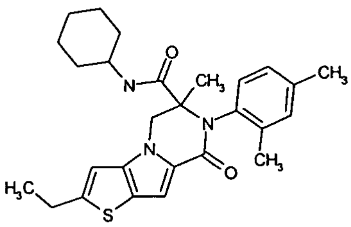 | 463,647 | 464 | |
| 117 | 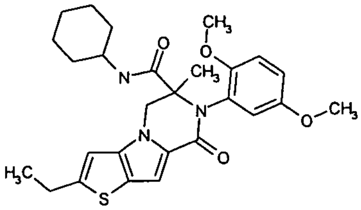 | 495,6458 | 496 | |

(continued)

| № | Formula | Mol.w. | LC MS, m/z (M+1) | 1H NMR |
|---|---|---|---|---|
| 118 | 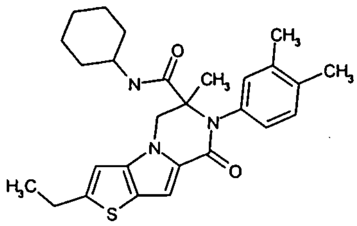 | 463,647 | 464 | |
| 119 | 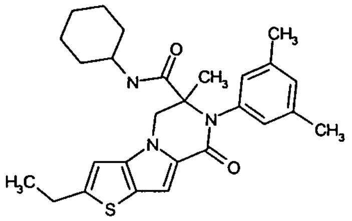 | 463,647 | 464 | |
| 120 | 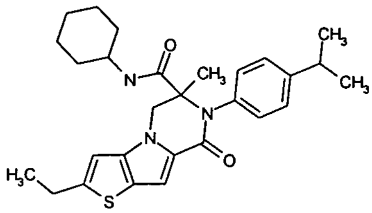 | 477,6741 | 478 | |
| 121 | 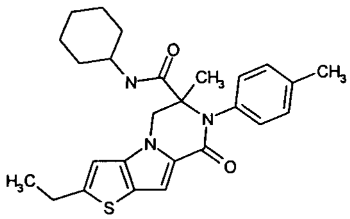 | 449,6199 | 450 | |
| 122 | 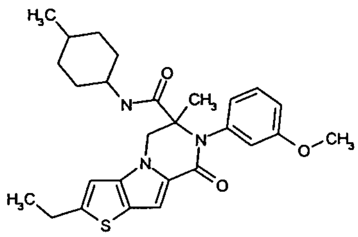 | 479,6464 | 480 | |
| 123 | 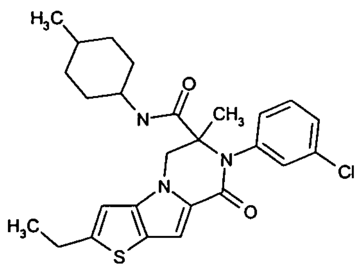 | 484,0649 | 485 | |

(continued)

| № | Formula | Mol.w. | LC MS, m/z (M+1) | 1H NMR |
|---|---|---|---|---|
| 124 | 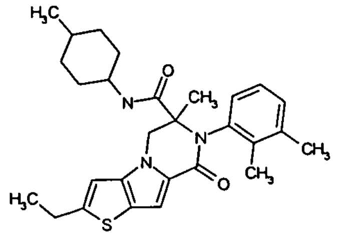 | 477,6741 | 478 | |
| 125 | 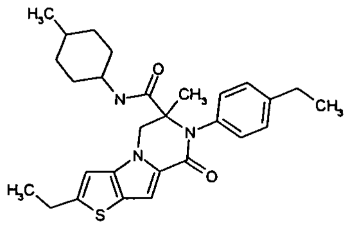 | 477,6741 | 478 | |
| 126 | 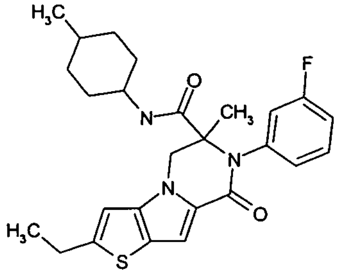 | 467,6103 | 468 | |
| 127 | 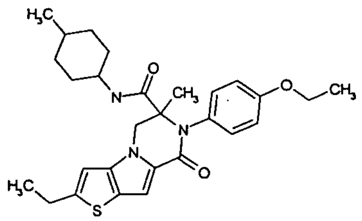 | 493,6735 | 494 | |
| 128 | 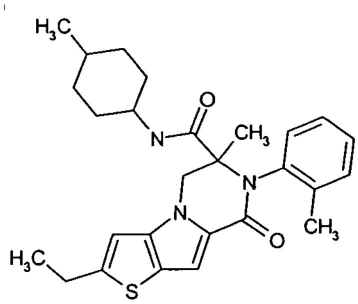 | 463,647 | 464 | |

(continued)

| № | Formula | Mol.w. | LC MS, m/z (M+1) | 1H NMR |
|---|---|---|---|---|
| 129 | 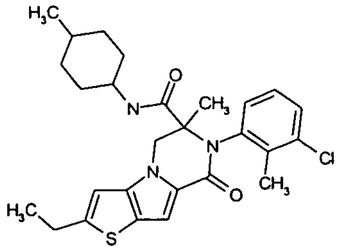 | 498,092 | 499 | |
| 130 | 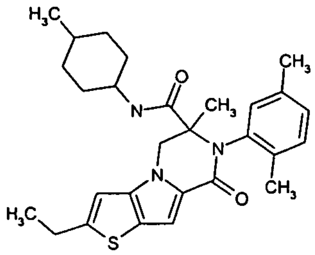 | 477,6741 | 478 | |

Table 1.16(3)

| № | Formula | Mol. w. | LC MS, m/z (M+1) | 1H NMR |
|---|---|---|---|---|
| 1 | 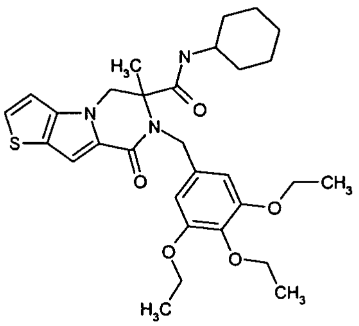 | 553,72643 | 554 | |
| 2 | 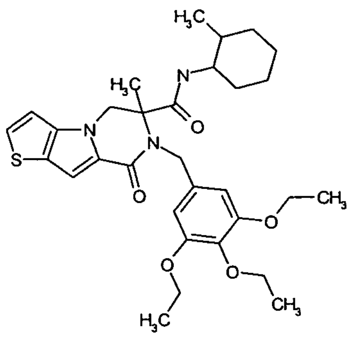 | 567,75352 | 568 | |

(continued)

| № | Formula | Mol. w. | LC MS, m/z (M+1) | 1H NMR |
|---|---|---|---|---|
| 3 | 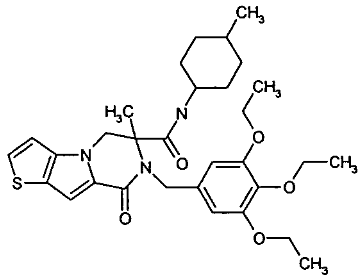 | 567,75352 | 568 | |
| 4 | 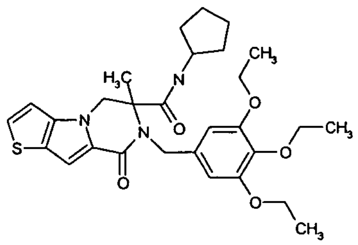 | 539,69934 | 540 | |
| 5 | 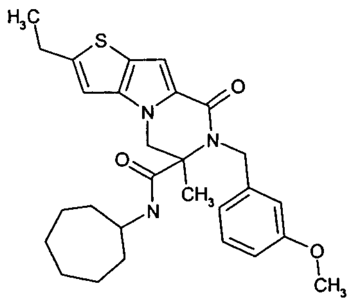 | 493,67345 | 494 | |
| 6 | 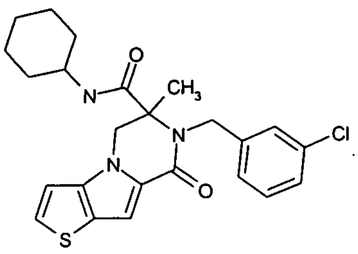 | 456,01072 | 457 | |
| 7 | 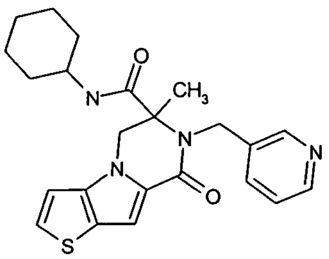 | 422,55327 | 423 | |

(continued)

| № | Formula | Mol. w. | LC MS, m/z (M+1) | 1H NMR |
|---|---|---|---|---|
| 8 | 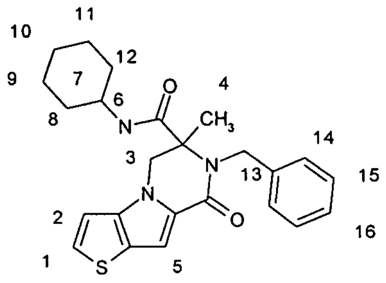 | 421,56569 | 422 | 0.86 - 1.66 (8, 9, 10, 11, 12; 10H; m.), 1.48 (4; 3H, s.), 3.41 (7; 1 H; b.m.), 4.06 + 4.87 (3; 2H; AB; 13.3 Hz), 4.27 + 5.31 (13; 2H; AB; 15.6 Hz), 6.98 (2, 5; 2H; m.), 7.20 (6; 1 H; d.; 8.0 Hz), 7.30 (1, 14, 15, 16; 6H ; m.) |
| 9 | 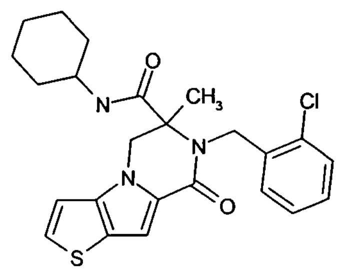 | 456,01072 | 457 | |
| 10 | 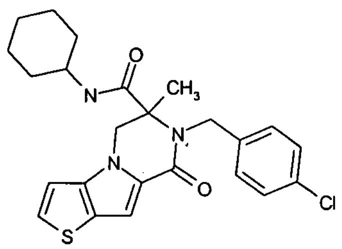 | 456,01072 | 457 | |
| 11 | 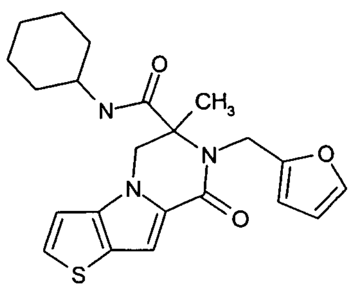 | 411,52685 | 412 | |
| 12 | 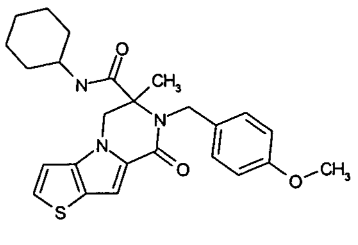 | 451,59218 | 452 | |

(continued)

| № | Formula | Mol. w. | LC MS, m/z (M+1) | 1H NMR |
|---|---|---|---|---|
| 13 | 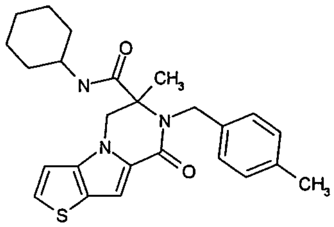 | 435,59278 | 436 | |
| 14 | 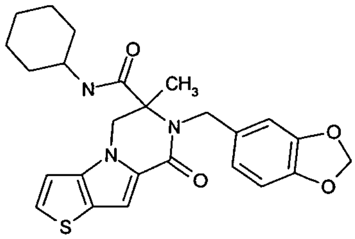 | 465,57564 | 466 | |
| 15 | 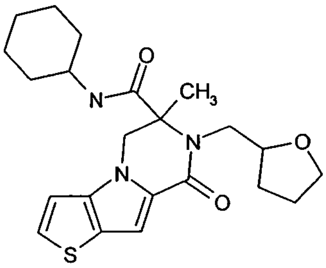 | 415,55873 | 416 | |
| 16 | 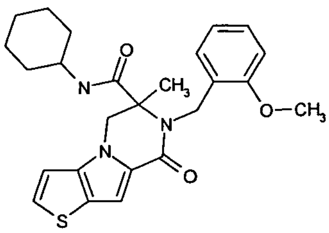 | 451,59218 | 452 | |
| 17 | 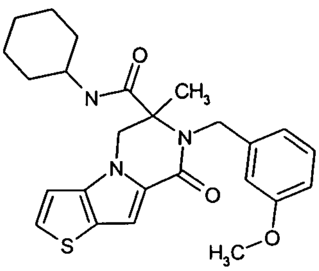 | 451,59218 | 452 | |
| 18 | 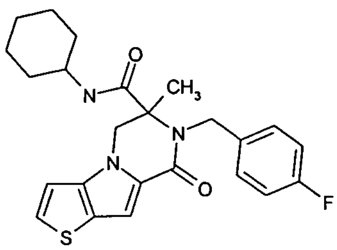 | 439,55612 | 440 | |

(continued)

| № | Formula | Mol. w. | LC MS, m/z (M+1) | 1H NMR |
|---|---|---|---|---|
| 19 | 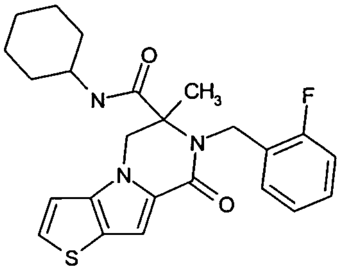 | 439,55612 | 440 | |
| 20 | 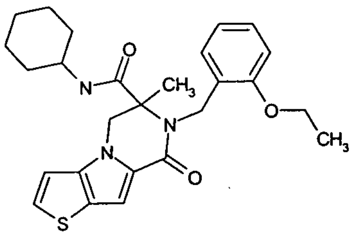 | 465,61927 | 466 | |
| 21 | 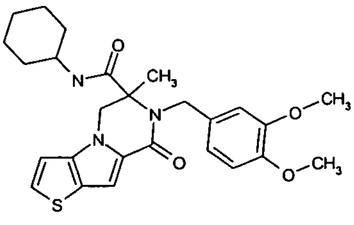 | 481,61867 | 482 | |
| 22 | 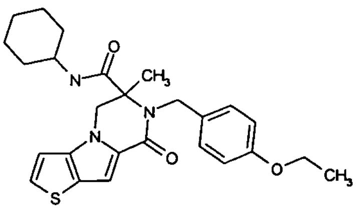 | 465,61927 | 466 | |
| 23 | 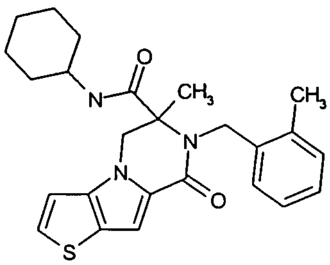 | 435,59278 | 436 | |
| 24 | 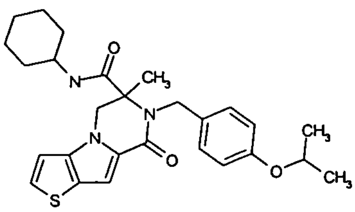 | 479,64636 | 480 | |

(continued)

| № | Formula | Mol. w. | LC MS, m/z (M+1) | 1H NMR |
|---|---|---|---|---|
| 25 | 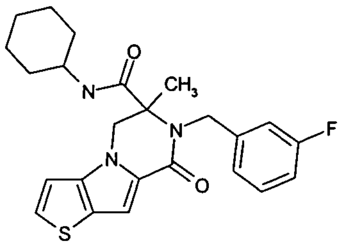 | 439,55612 | 440 | |
| 26 | 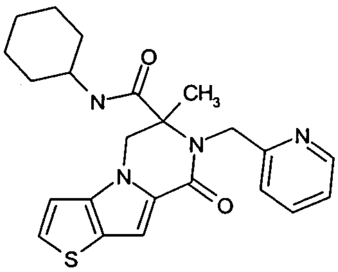 | 422,55327 | 423 | |
| 27 | 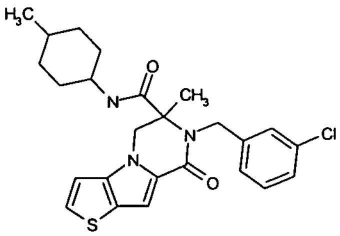 | 470,03781 | 471 | |
| 28 | 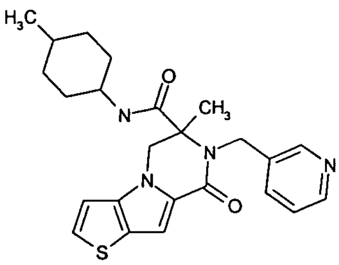 | 436,58036 | 437 | |
| 29 | 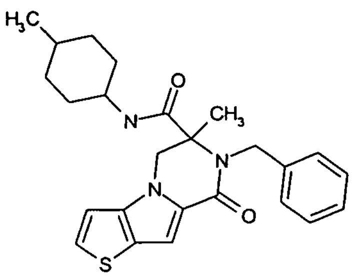 | 435,59278 | 436 | |

(continued)

| № | Formula | Mol. w. | LC MS, m/z (M+1) | 1H NMR |
|---|---|---|---|---|
| 30 | 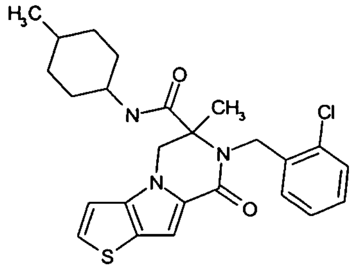 | 470,03781 | 471 | |
| 31 | 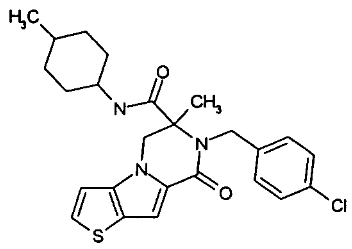 | 470,03781 | 471 | |
| 32 | 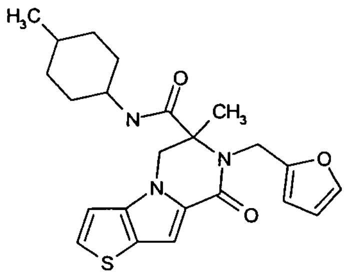 | 425,55394 | 426 | |
| 33 | 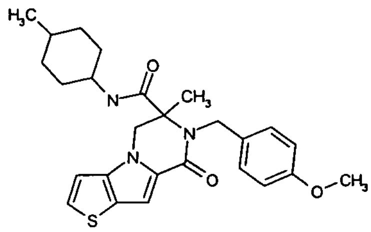 | 465,61927 | 466 | |
| 34 | 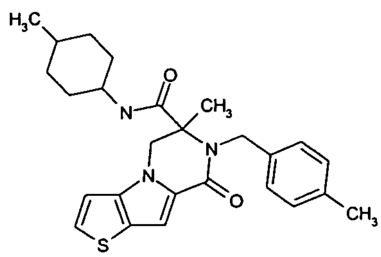 | 449,61987 | 450 | |
| 35 | 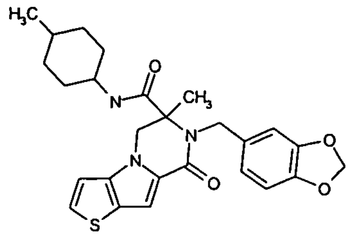 | 479,60273 | 480 | |

(continued)

| № | Formula | Mol. w. | LC MS, m/z (M+1) | 1H NMR |
|---|---|---|---|---|
| 36 | 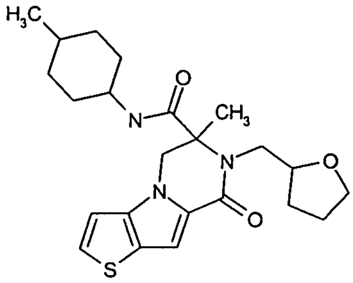 | 429,58582 | 430 | |
| 37 | 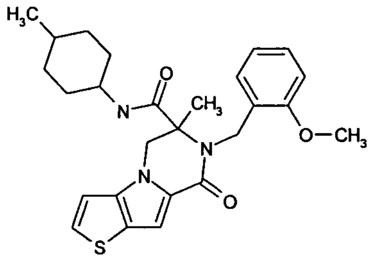 | 465,61927 | 466 | |
| 38 | 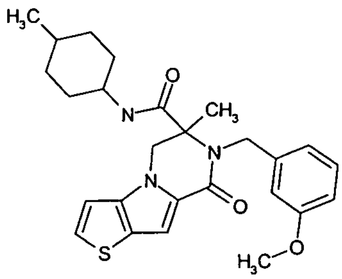 | 465,61927 | 466 | |
| 39 | 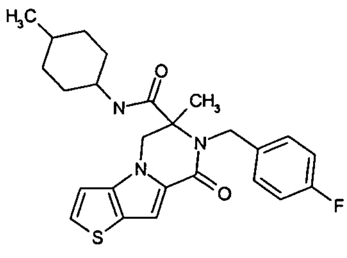 | 453,58321 | 454 | |
| 40 | 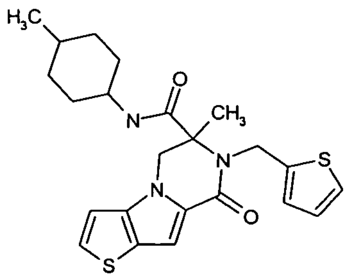 | 441,61854 | 442 | |

(continued)

| № | Formula | Mol. w. | LC MS, m/z (M+1) | 1H NMR |
|---|---|---|---|---|
| 41 | 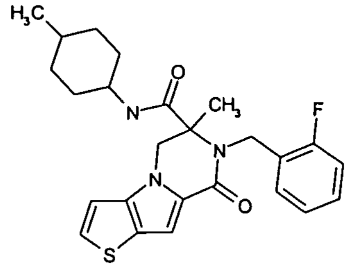 | 453,58321 | 454 | |
| 42 | 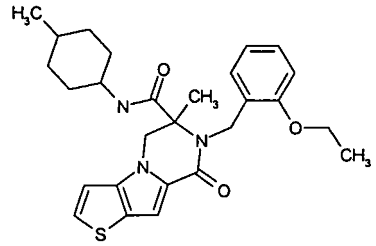 | 479,64636 | 480 | |
| 43 | 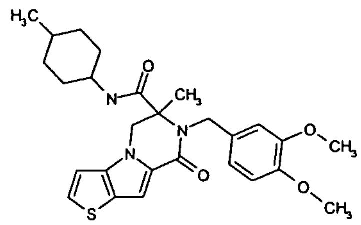 | 495,64576 | 496 | |
| 44 | 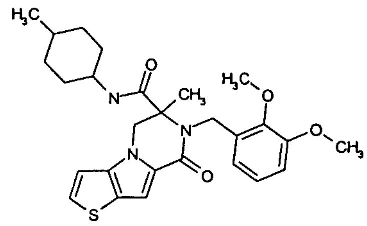 | 495,64576 | 496 | |
| 45 | 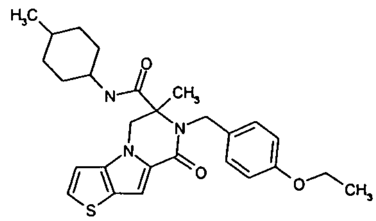 | 479,64636 | 480 | |
| 46 | 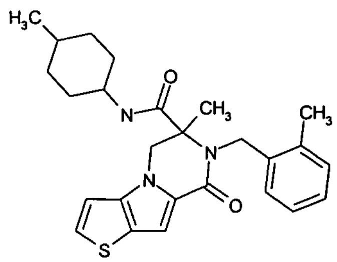 | 449,61987 | 450 | |

(continued)

| № | Formula | Mol. w. | LC MS, m/z (M+1) | 1H NMR |
|---|---|---|---|---|
| 47 | 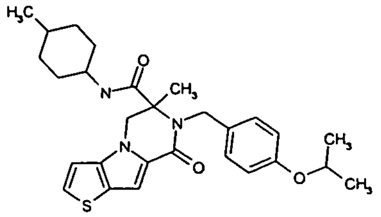 | 493,67345 | 494 | |
| 48 | 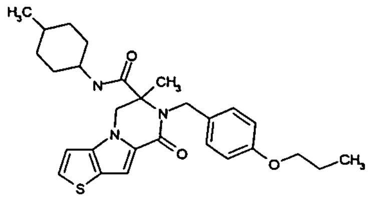 | 493,67345 | 494 | |
| 49 | 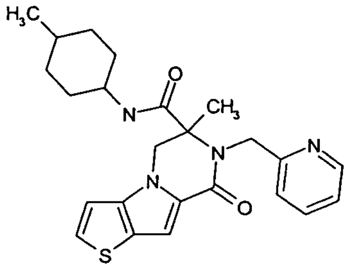 | 436,58036 | 437 | |
| 50 | 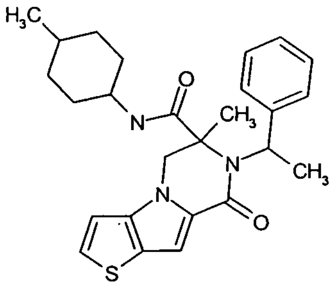 | 449,61987 | 450 | |
| 51 | 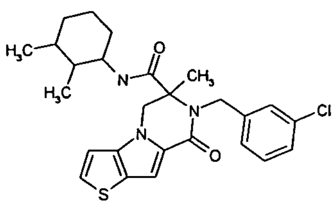 | 484,0649 | 485 | |
| 52 | 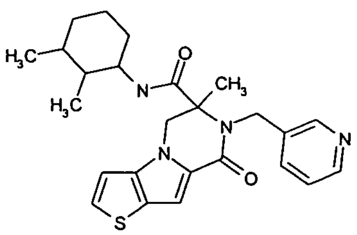 | 450,60745 | 451 | |

(continued)

| № | Formula | Mol. w. | LC MS, m/z (M+1) | 1H NMR |
|---|---|---|---|---|
| 53 | 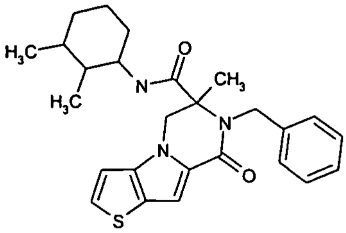 | 449,61987 | 450 | |
| 54 | 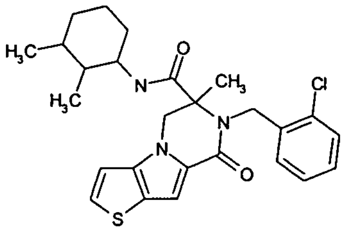 | 484,0649 | 485 | |
| 55 | 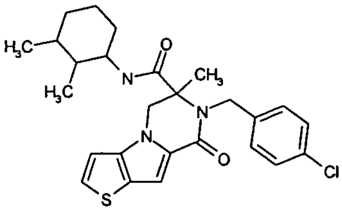 | 484,0649 | 485 | |
| 56 | 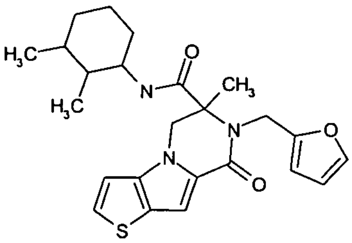 | 439,58103 | 440 | |
| 57 | 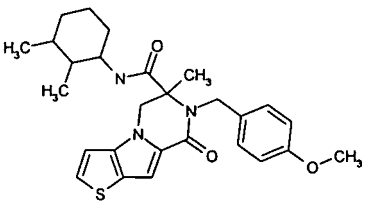 | 479,64636 | 480 | |
| 58 | 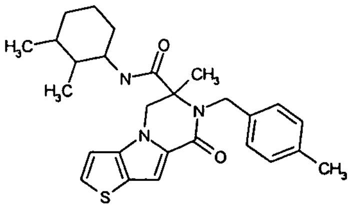 | 463,64696 | 464 | |

(continued)

| № | Formula | Mol. w. | LC MS, m/z (M+1) | 1H NMR |
|---|---|---|---|---|
| 59 | 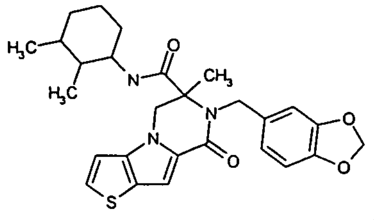 | 493,62982 | 494 | |
| 60 | 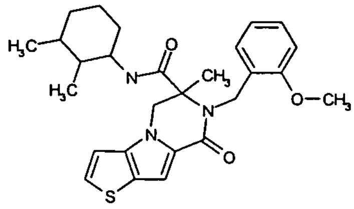 | 479,64636 | 480 | |
| 61 | 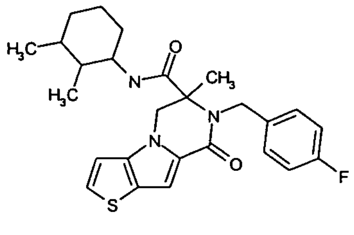 | 467,6103 | 468 | |
| 62 | 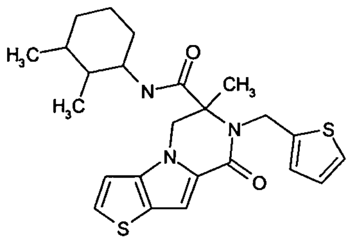 | 455,64563 | 456 | |
| 63 | 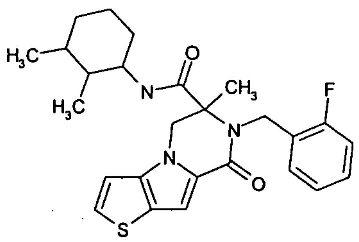 | 467,6103 | 468 | |
| 64 | 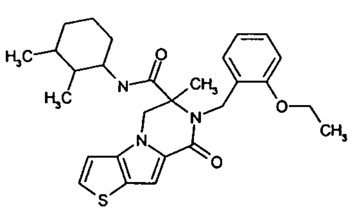 | 493,67345 | 494 | |

(continued)

| № | Formula | Mol. w. | LC MS, m/z (M+1) | 1H NMR |
|---|---|---|---|---|
| 65 | 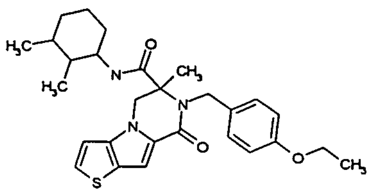 | 493,67345 | 494 | |
| 66 | 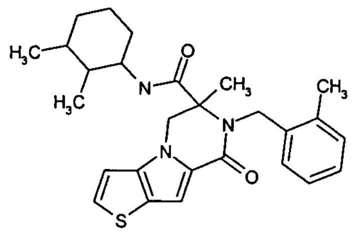 | 463,64696 | 464 | |
| 67 | 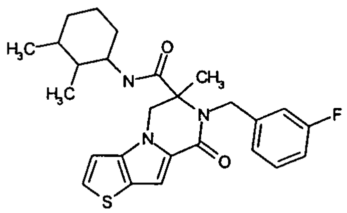 | 467,6103 | 468 | |
| 68 | 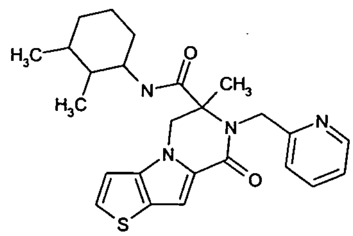 | 450,60745 | 451 | |
| 69 | 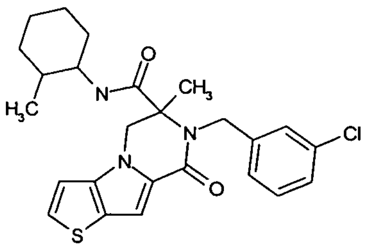 | 470,03781 | 471 | |
| 70 | 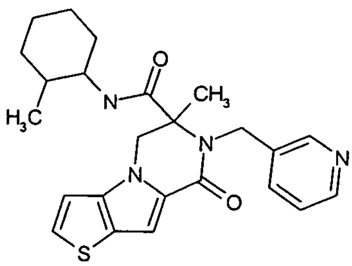 | 436,58036 | 437 | |

(continued)

| № | Formula | Mol. w. | LC MS, m/z (M+1) | 1H NMR |
|---|---|---|---|---|
| 71 | 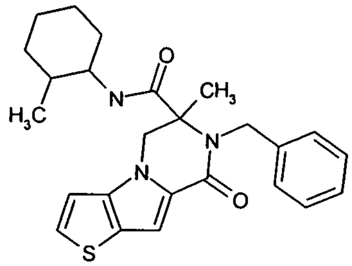 | 435,59278 | 436 | |
| 72 | 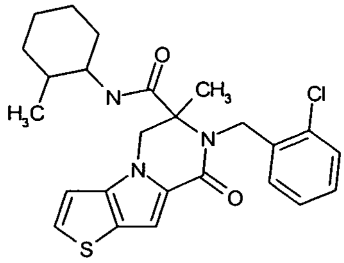 | 470,03781 | 471 | |
| 73 | 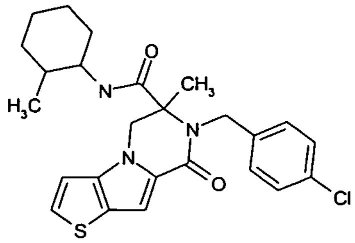 | 470,03781 | 471 | |
| 74 | 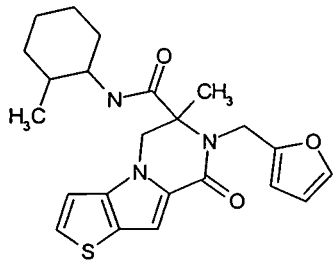 | 425,55394 | 426 | |
| 75 | 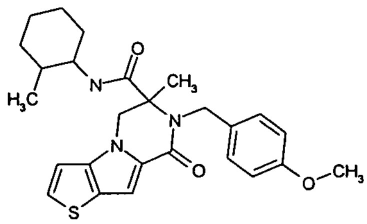 | 465,61927 | 466 | |
| 76 | 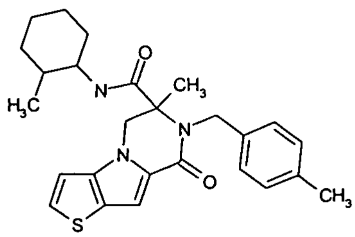 | 449,61987 | 450 | |

(continued)

| № | Formula | Mol. w. | LC MS, m/z (M+1) | 1H NMR |
|---|---|---|---|---|
| 77 | 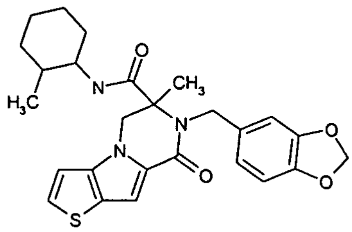 | 479,60273 | 480 | |
| 78 | 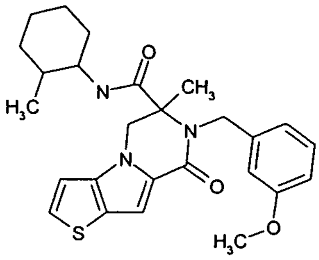 | 465,61927 | 466 | |
| 79 | 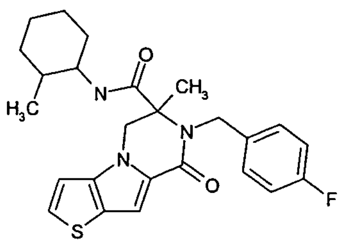 | 453,58321 | 454 | |
| 80 | 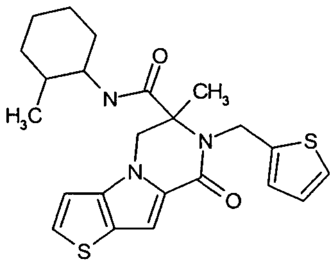 | 441,61854 | 442 | |
| 81 | 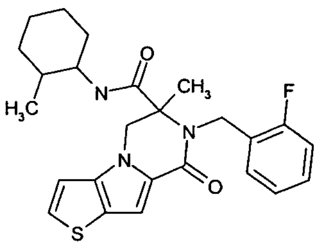 | 453,58321 | 454 | |
| 82 | 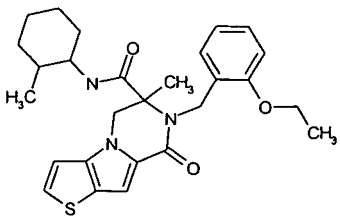 | 479,64636 | 480 | |

(continued)

| № | Formula | Mol. w. | LC MS, m/z (M+1) | 1H NMR |
|---|---|---|---|---|
| 83 | 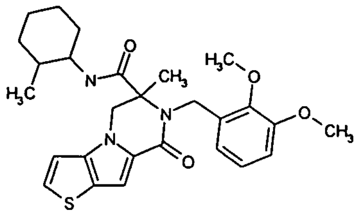 | 495,64576 | 496 | |
| 84 | 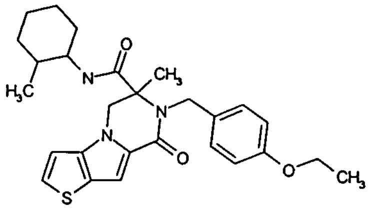 | 479,64636 | 480 | |
| 85 | 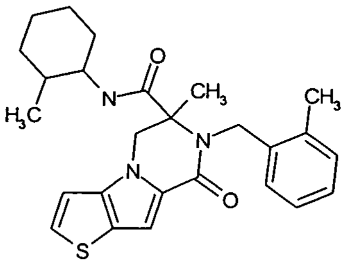 | 449,61987 | 450 | |
| 86 | 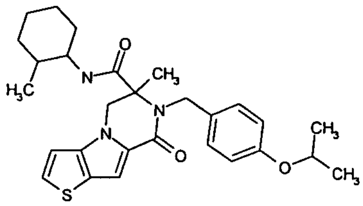 | 493,67345 | 494 | |
| 87 | 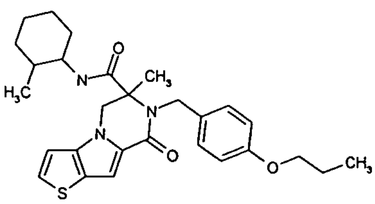 | 493,67345 | 494 | |
| 88 | 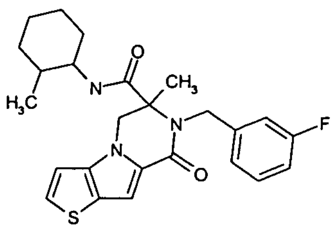 | 453,58321 | 454 | |

(continued)

| № | Formula | Mol. w. | LC MS, m/z (M+1) | 1H NMR |
|---|---|---|---|---|
| 89 | 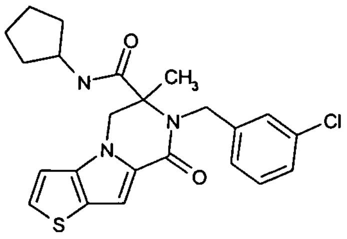 | 441,98363 | 442 | |
| 90 | 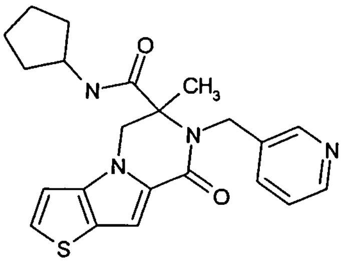 | 408,52618 | 409 | |
| 91 | 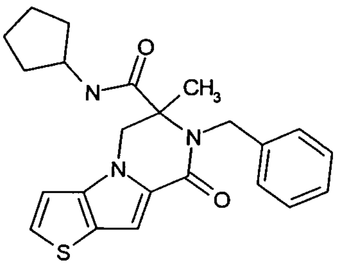 | 407,5386 | 408 | |
| 92 | 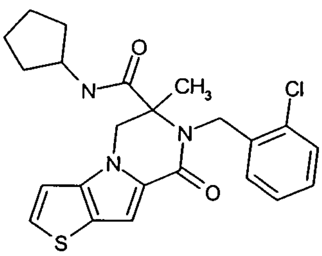 | 441,98363 | 442 | |
| 93 | 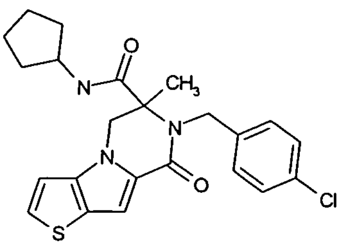 | 441,98363 | 442 | |
| 94 | 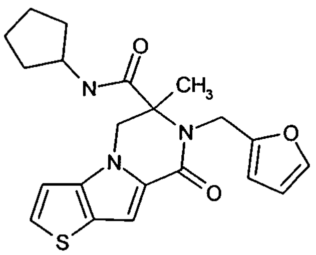 | 397,49976 | 397 | |

(continued)

| № | Formula | Mol. w. | LC MS, m/z (M+1) | 1H NMR |
|---|---|---|---|---|
| 95 | 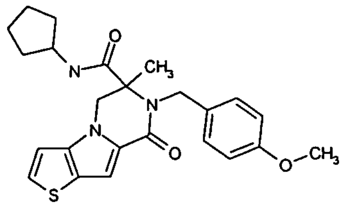 | 437,56509 | 438 | |
| 96 | 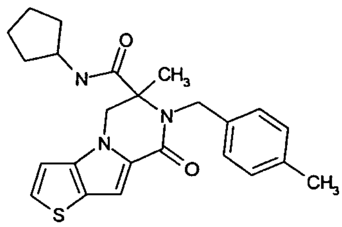 | 421,56569 | 422 | |
| 97 | 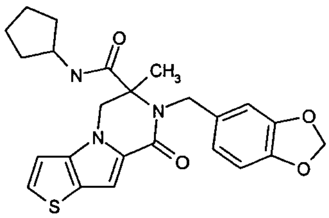 | 451,54855 | 452 | |
| 98 | 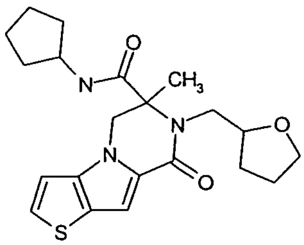 | 401,53164 | 402 | |
| 99 | 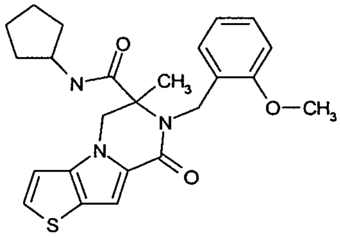 | 437,56509 | 438 | |
| 100 | 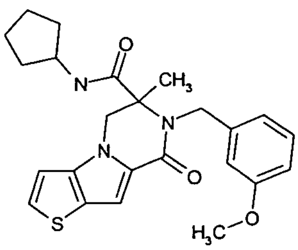 | 437,56509 | 438 | |

(continued)

| № | Formula | Mol. w. | LC MS, m/z (M+1) | 1H NMR |
|---|---|---|---|---|
| 101 | 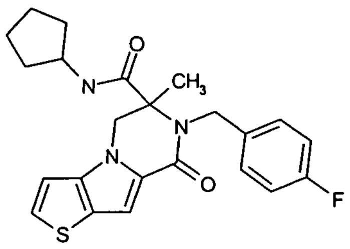 | 425,52903 | 426 | |
| 102 | 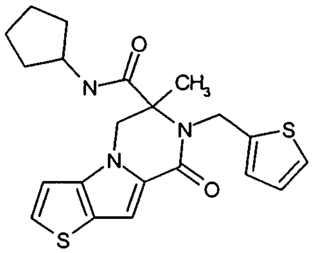 | 413,56436 | 414 | |
| 103 | 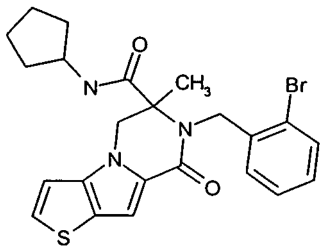 | 486,43463 | 487 | |
| 104 | 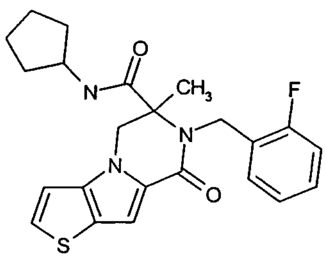 | 425,52903 | 426 | |
| 105 | 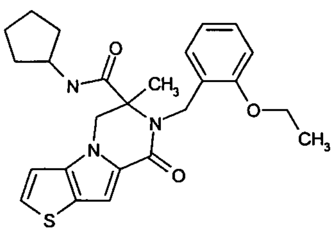 | 451,59218 | 452 | |
| 106 | 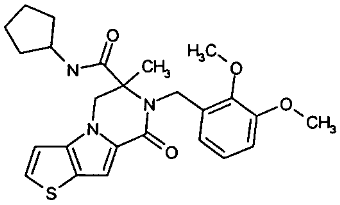 | 467,59158 | 468 | |

(continued)

| № | Formula | Mol. w. | LC MS, m/z (M+1) | 1H NMR |
|---|---|---|---|---|
| 107 | 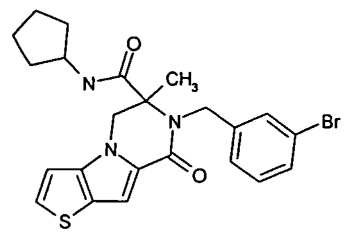 | 486,43463 | 487 | |
| 108 | 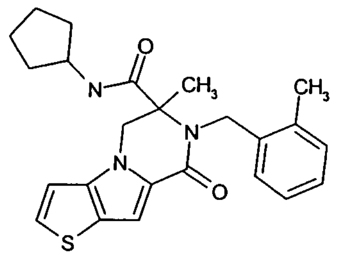 | 421,56569 | 422 | |
| 109 | 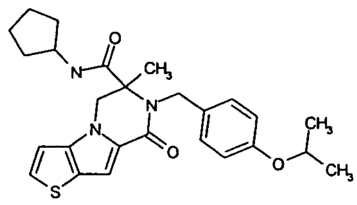 | 465,61927 | 466 | |
| 110 | 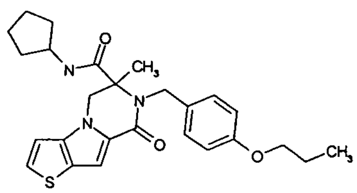 | 465,61927 | 466 | |
| 111 | 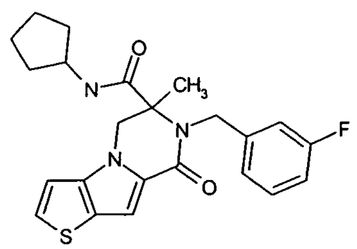 | 425,52903 | 426 | |
| 112 | 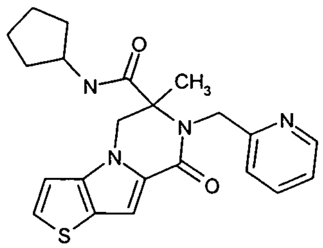 | 408,52618 | 409 | |

(continued)

| № | Formula | Mol. w. | LC MS, m/z (M+1) | 1H NMR |
|---|---|---|---|---|
| 113 | 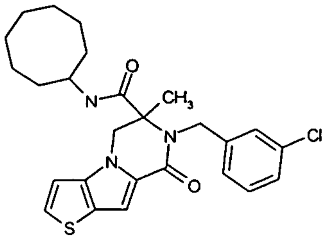 | 484,0649 | 485 | |
| 114 | 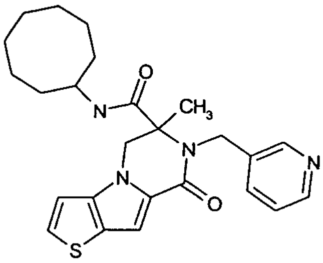 | 450,60745 | 451 | |
| 115 | 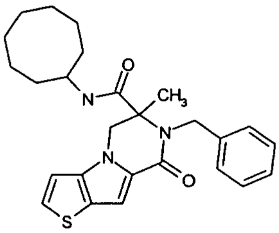 | 449,61987 | 450 | |
| 116 | 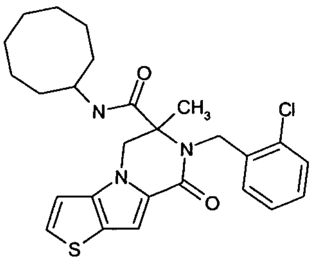 | 484,0649 | 485 | |
| 117 | 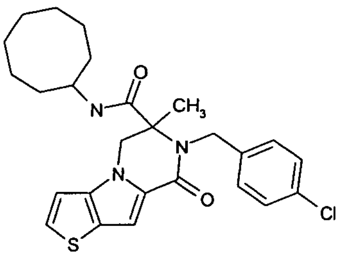 | 484,0649 | 485 | |
| 118 | 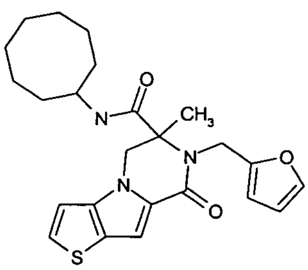 | 439,58103 | 440 | |

(continued)

| № | Formula | Mol. w. | LC MS, m/z (M+1) | 1H NMR |
|---|---|---|---|---|
| 119 | 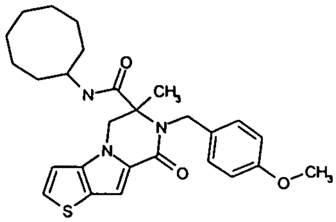 | 479,64636 | 480 | |
| 120 | 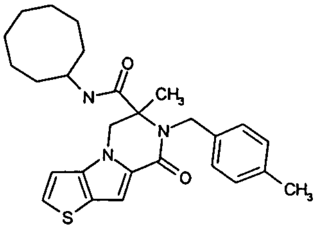 | 463,64696 | 464 | |
| 121 | 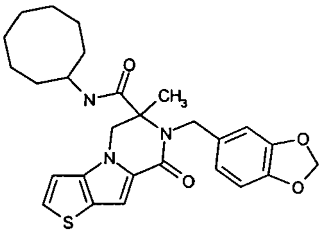 | 493,62982 | 494 | |
| 122 | 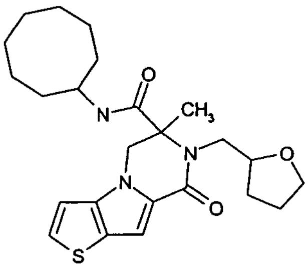 | 443,61291 | 444 | |
| 123 | 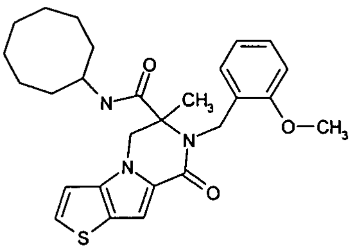 | 479,64636 | 480 | |
| 124 | 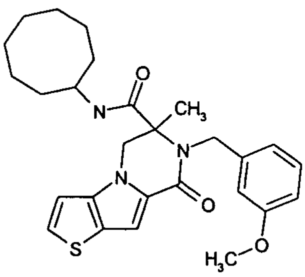 | 479,64636 | 480 | |

(continued)

| № | Formula | Mol. w. | LC MS, m/z (M+1) | 1H NMR |
|---|---|---|---|---|
| 125 | 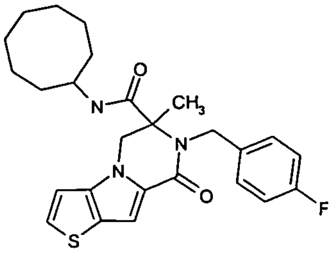 | 467,6103 | 468 | |
| 126 | 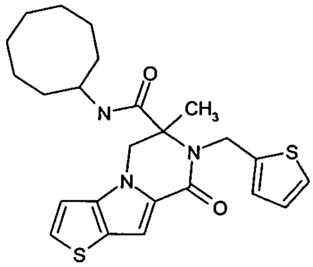 | 455,64563 | 456 | |
| 127 | 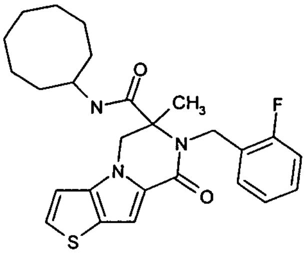 | 467,6103 | 468 | |
| 128 | 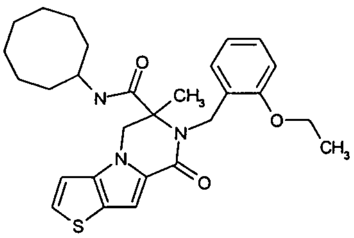 | 493,67345 | 494 | |
| 129 | 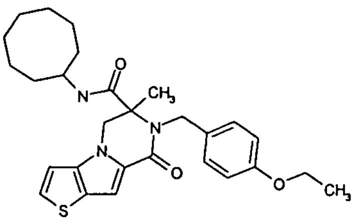 | 493,67345 | 494 | |
| 130 | 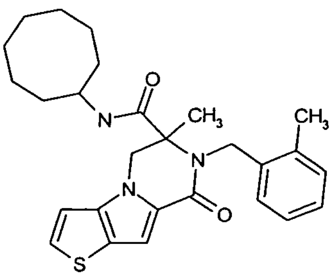 | 463,64696 | 464 | |

(continued)

| № | Formula | Mol. w. | LC MS, m/z (M+1) | 1H NMR |
|---|---|---|---|---|
| 131 | 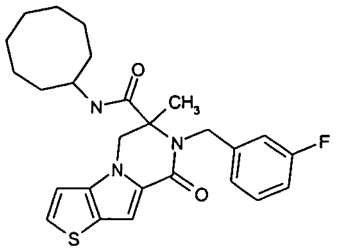 | 467,6103 | 468 | |
| 132 | 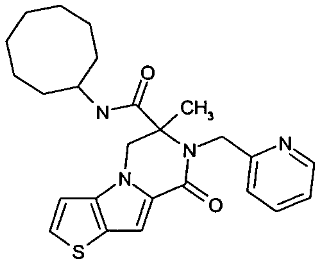 | 450,60745 | 451 | |
| 133 | 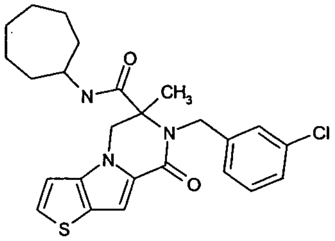 | 470,03781 | 471 | |
| 134 | 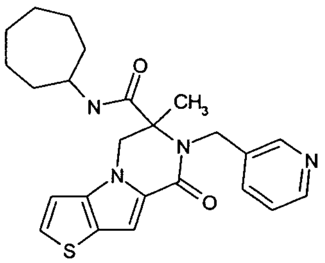 | 436,58036 | 437 | |
| 135 | 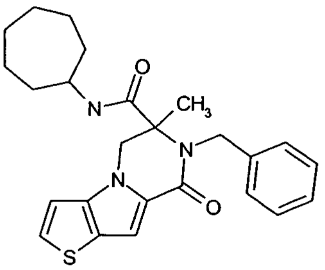 | 435,59278 | 436 | |

(continued)

| № | Formula | Mol. w. | LC MS, m/z (M+1) | 1H NMR |
|---|---|---|---|---|
| 136 | 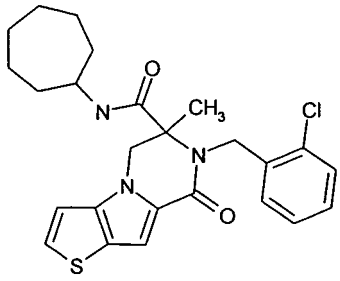 | 470,03781 | 471 | |
| 137 | 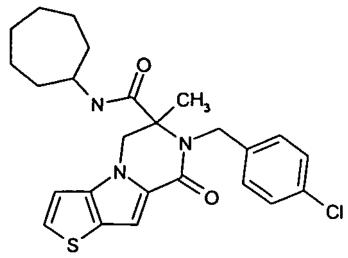 | 470,03781 | 471 | |
| 138 | 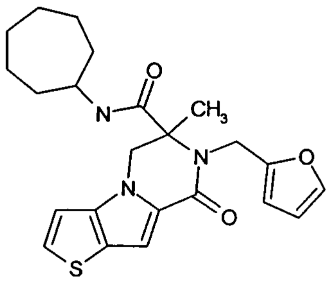 | 425,55394 | 426 | |
| 139 | 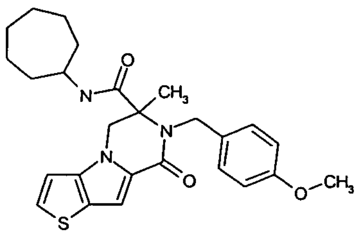 | 465,61927 | 466 | |
| 140 | 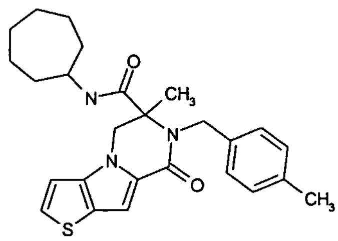 | 449,61987 | 450 | |
| 141 | 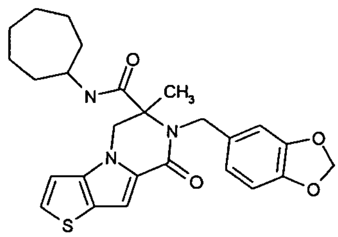 | 479,60273 | 480 | |

(continued)

| № | Formula | Mol. w. | LC MS, m/z (M+1) | 1H NMR |
|---|---|---|---|---|
| 142 | 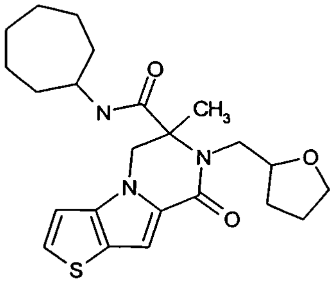 | 429,58582 | 430 | |
| 143 | 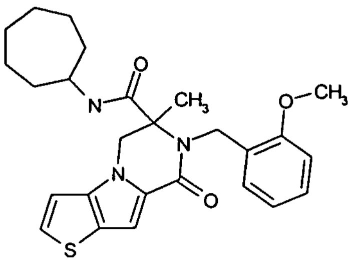 | 465,61927 | 466 | |
| 144 | 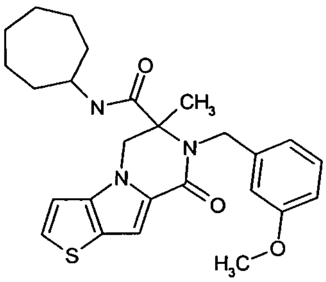 | 465,61927 | 466 | |
| 145 | 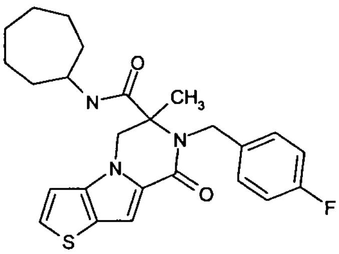 | 453,58321 | 454 | |
| 146 | 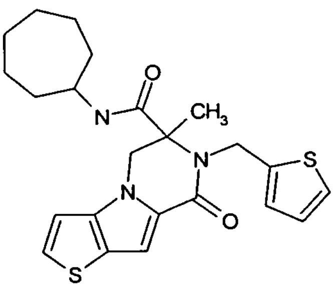 | 441,61854 | 442 | |

(continued)

| № | Formula | Mol. w. | LC MS, m/z (M+1) | 1H NMR |
|---|---|---|---|---|
| 147 | 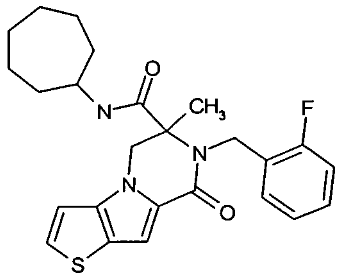 | 453,58321 | 454 | |
| 148 | 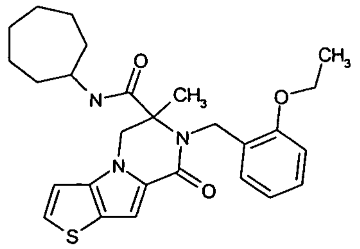 | 479,64636 | 480 | |
| 149 | 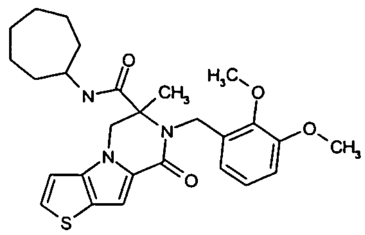 | 495,64576 | 496 | |
| 150 | 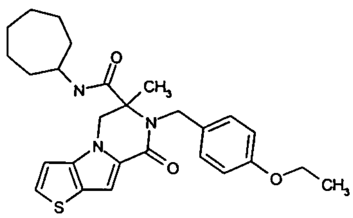 | 479,64636 | 480 | |
| 151 | 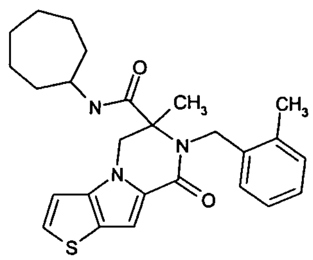 | 449,61987 | 450 | |
| 152 | 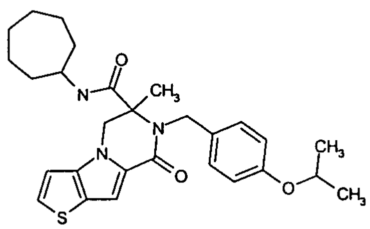 | 493,67345 | 494 | |

(continued)

| № | Formula | Mol. w. | LC MS, m/z (M+1) | 1H NMR |
|---|---|---|---|---|
| 153 | 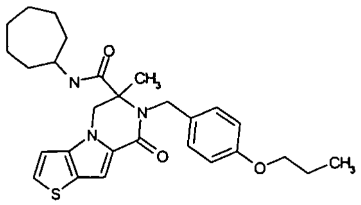 | 493,67345 | 494 | |
| 154 | 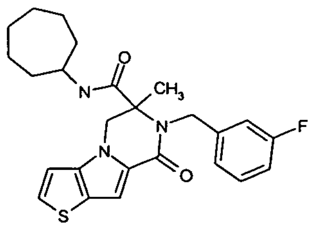 | 453,58321 | 454 | |
| 155 | 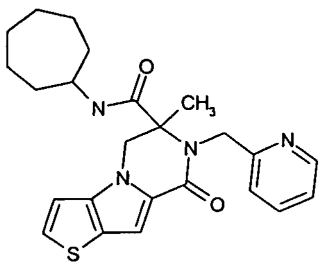 | 436,58036 | 437 | |
| 156 | 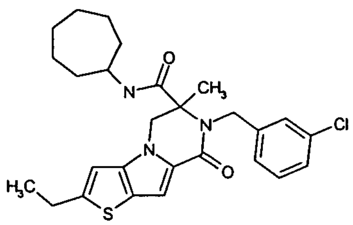 | 498,09199 | 499 | |
| 157 | 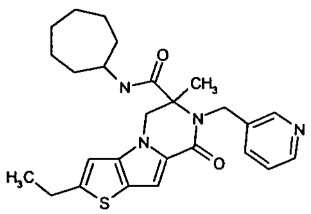 | 464,63454 | 465 | |
| 158 | 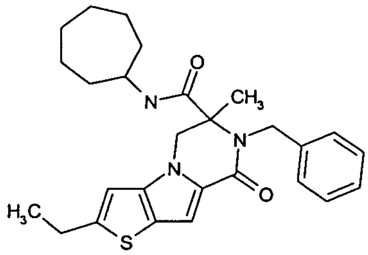 | 463,64696 | 464 | |

(continued)

| № | Formula | Mol. w. | LC MS, m/z (M+1) | 1H NMR |
|---|---|---|---|---|
| 159 | 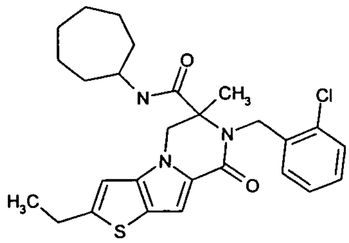 | 498,09199 | 499 | |
| 160 | 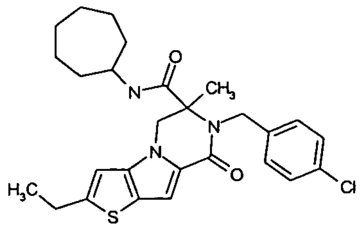 | 498,09199 | 499 | |
| 161 | 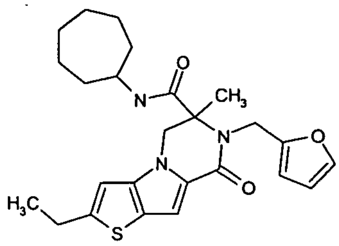 | 453,60812 | 454 | |
| 162 | 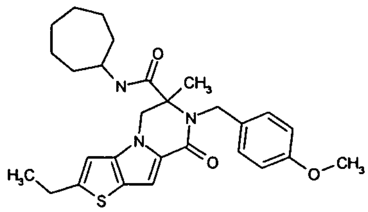 | 493,67345 | 494 | |
| 163 | 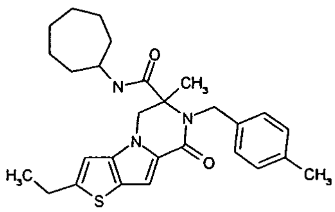 | 477,67405 | 478 | |
| 164 | 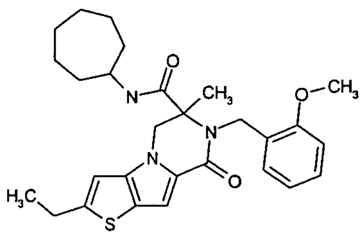 | 493,67345 | 494 | |

(continued)

| № | Formula | Mol. w. | LC MS, m/z (M+1) | 1H NMR |
|---|---|---|---|---|
| 165 | 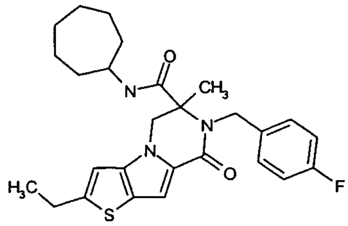 | 481,63739 | 482 | |
| 166 | 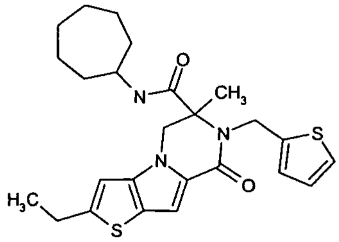 | 469,67272 | 470 | |
| 167 | 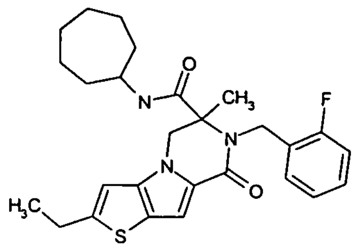 | 481,63739 | 482 | |
| 168 | 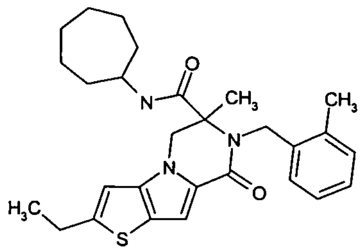 | 477,67405 | 478 | |
| 169 | 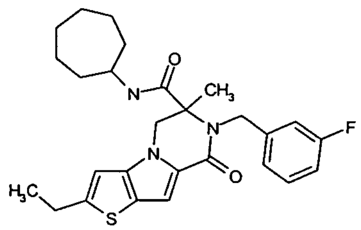 | 481,63739 | 482 | |
| 170 | 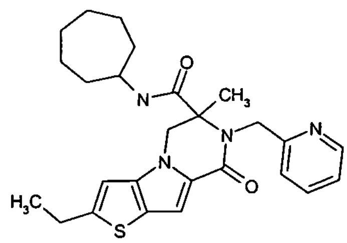 | 464,63454 | 465 | |

(continued)

| № | Formula | Mol. w. | LC MS, m/z (M+1) | 1H NMR |
|---|---|---|---|---|
| 171 | 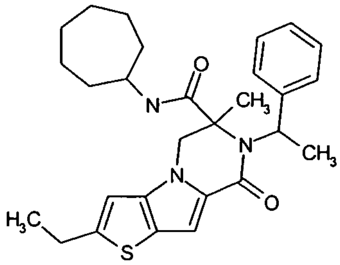 | 477,67405 | 478 | |
| 172 | 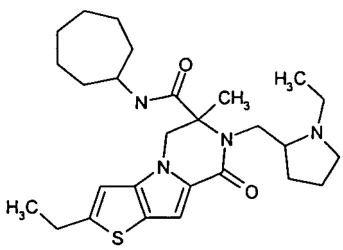 | 484,70945 | 485 | |
| 173 | 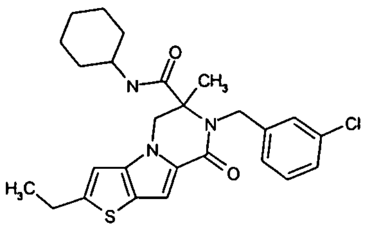 | 484,0649 | 485 | |
| 174 | 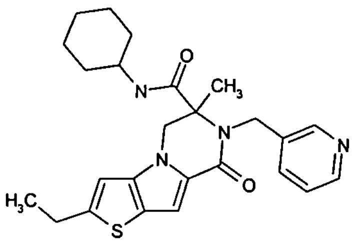 | 450,60745 | 451 | |
| 175 | 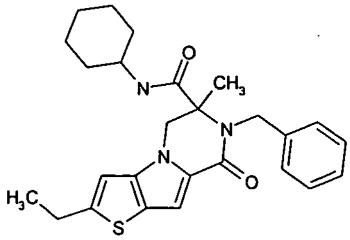 | 449,61987 | 450 | |
| 176 | 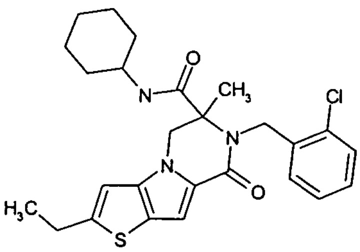 | 484,0649 | 485 | |

(continued)

| № | Formula | Mol. w. | LC MS, m/z (M+1) | 1H NMR |
|---|---|---|---|---|
| 177 | 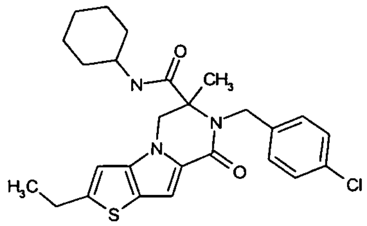 | 484,0649 | 485 | |
| 178 | 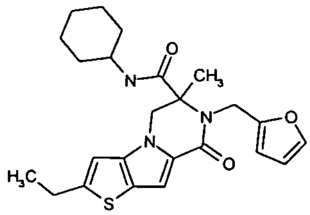 | 439,58103 | 440 | |
| 179 | 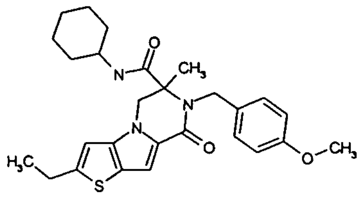 | 479,64636 | 480 | |
| 180 | 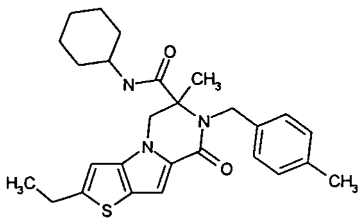 | 463,64696 | 464 | |
| 181 | 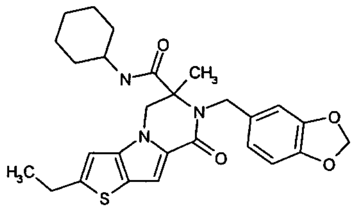 | 493,62982 | 494 | |
| 182 | 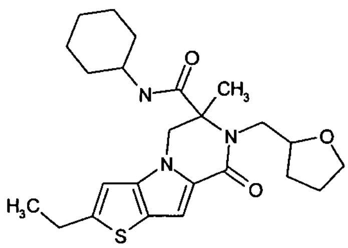 | 443,61291 | 444 | |

(continued)

| № | Formula | Mol. w. | LC MS, m/z (M+1) | 1H NMR |
|---|---|---|---|---|
| 183 | 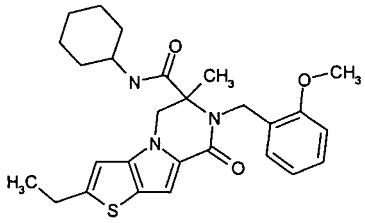 | 479,64636 | 480 | |
| 184 | 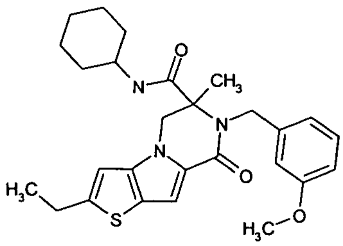 | 479,64636 | 480 | |
| 185 | 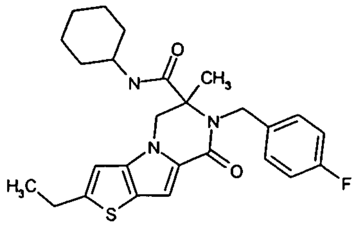 | 467,6103 | 468 | |
| 186 | 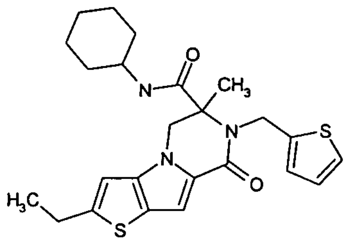 | 455,64563 | 456 | |
| 187 | 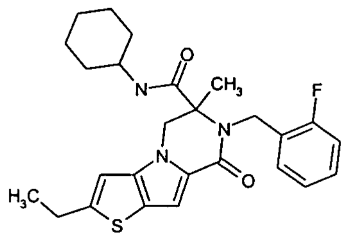 | 467,6103 | 468 | |
| 188 | 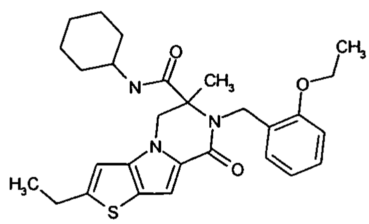 | 493,67345 | 494 | |

(continued)

| № | Formula | Mol. w. | LC MS, m/z (M+1) | 1H NMR |
|---|---|---|---|---|
| 189 | 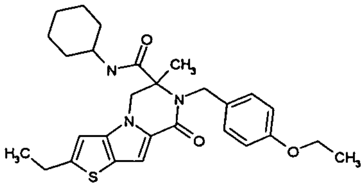 | 493,67345 | 494 | |
| 190 | 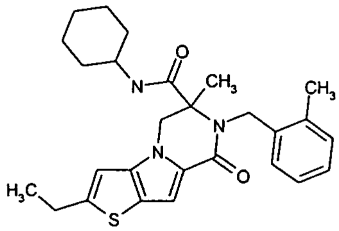 | 463,64696 | 464 | |
| 191 | 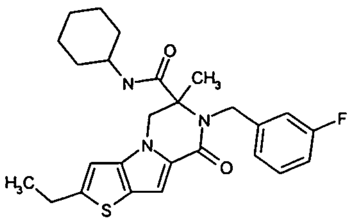 | 467,6103 | 468 | |
| 192 | 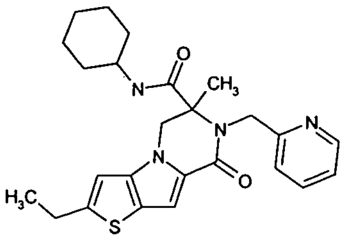 | 450,60745 | 451 | |
| 193 | 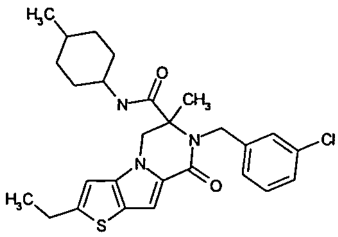 | 498,09199 | 499 | |
| 194 | 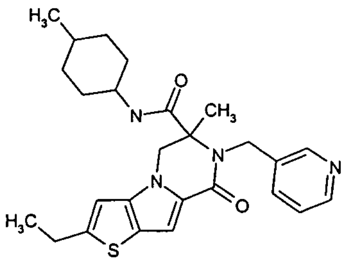 | 464,63454 | 465 | |

(continued)

| № | Formula | Mol. w. | LC MS, m/z (M+1) | 1H NMR |
|---|---|---|---|---|
| 195 | 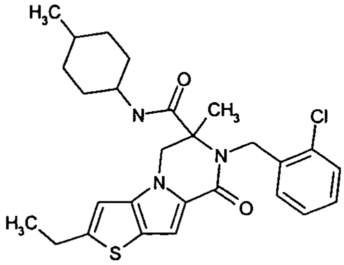 | 498,09199 | 499 | |
| 196 | 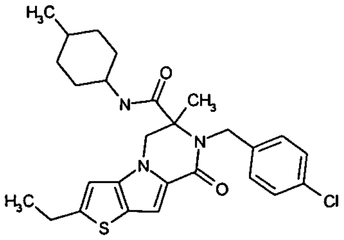 | 498,09199 | 499 | |
| 197 | 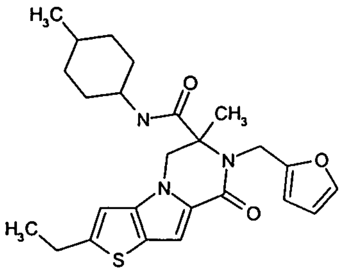 | 453,60812 | 454 | |
| 198 | 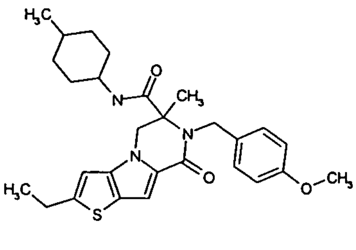 | 493,67345 | 494 | |
| 199 | 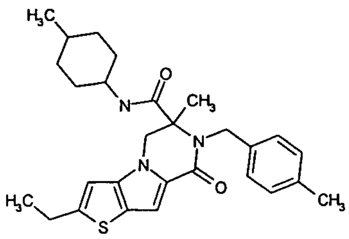 | 477,67405 | 478 | |

(continued)

| № | Formula | Mol. w. | LC MS, m/z (M+1) | 1H NMR |
|---|---|---|---|---|
| 200 | 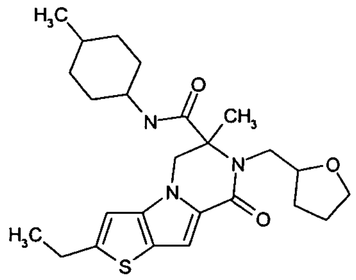 | 457,64 | 458 | |
| 201 | 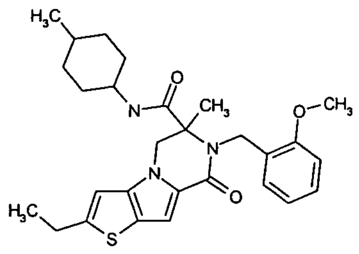 | 493,67345 | 494 | |
| 202 | 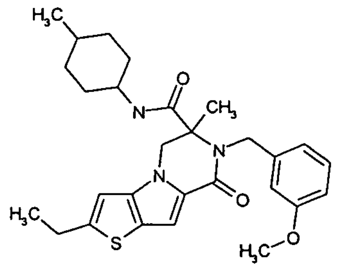 | 493,67345 | 494 | |
| 203 | 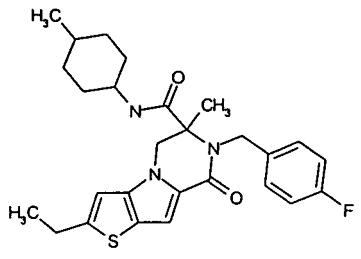 | 481,63739 | 482 | |
| 204 | 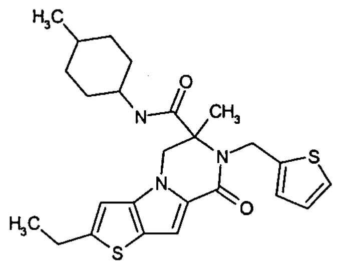 | 469,67272 | 470 | |

(continued)

| № | Formula | Mol. w. | LC MS, m/z (M+1) | 1H NMR |
|---|---|---|---|---|
| 205 | 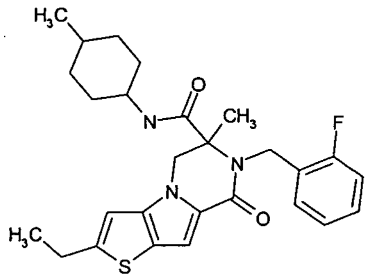 | 481,63739 | 482 | |
| 206 | 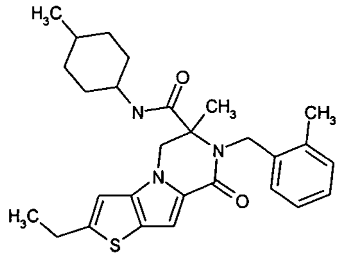 | 477,67405 | 478 | |
| 207 | 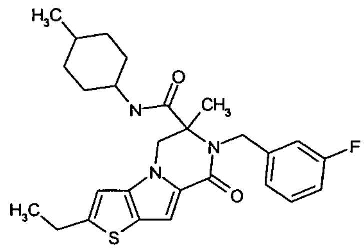 | 481,63739 | 482 | |
| 208 | 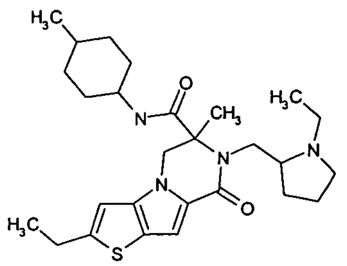 | 484,70945 | 485 | |

Table 1.16(4)

| № | Formula | Mol. w. | LC MS, m/z (M+1) | 1H NMR |
|---|---|---|---|---|
| 1 | 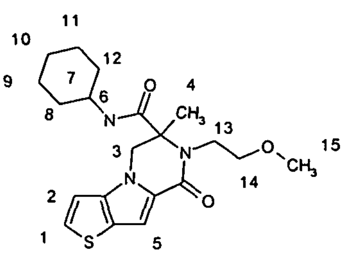 | 389,5205 | 390 | 0.95 - 1.74 (8, 9, 10, 11, 12; 10H; m.), 1.60 (4; 3H, s.), 3.37 (15; 3H; s.), 3.44 (7; 1 H; b.m.), 3.56 + 3.75 (13; 2H; m.m.), 3.59 (14; 2H; m.), 4.03 + 4.76 (3; 2H; AB; 12.1 Hz), 6.90 (5; 1H; s.), 7.00 (2; 1 H; d.; 5.7 Hz), 7.28 (1; 1 H ; d.; 5.7 Hz),... |

(continued)

| № | Formula | Mol. w. | LC MS, m/z (M+1) | 1H NMR |
|---|---|---|---|---|
| 2 | 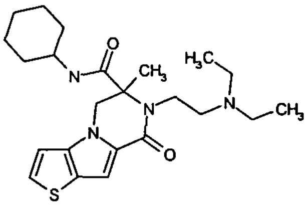 | 430,617 | 431 | |
| 3 | 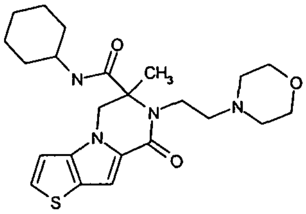 | 444,6005 | 445 | |
| 4 | 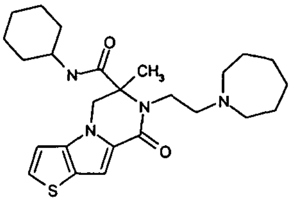 | 456,6553 | 457 | |
| 5 | 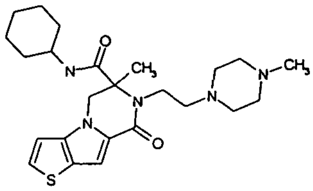 | 457,6429 | 458 | |
| 6 | 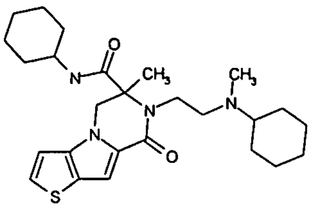 | 470,6824 | 471 | |
| 7 | 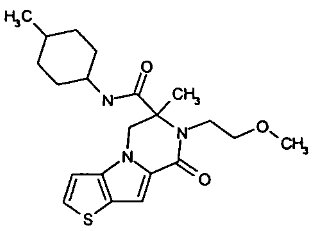 | 403,5476 | 404 | |
| 8 | 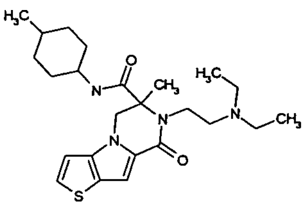 | 444,6441 | 445 | |

(continued)

| № | Formula | Mol. w. | LC MS, m/z (M+1) | 1H NMR |
|---|---|---|---|---|
| 9 | 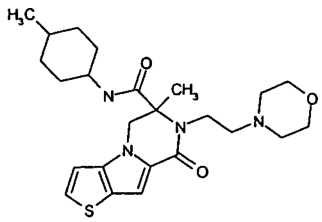 | 458,6276 | 459 | |
| 10 | 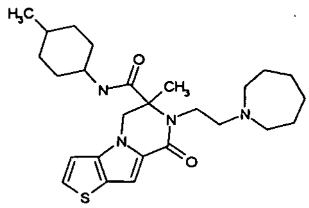 | 470,6824 | 471 | |
| 11 | 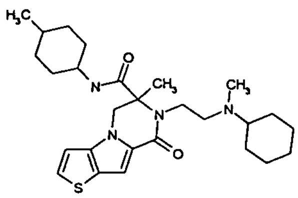 | 484,7095 | 485 | |
| 12 | 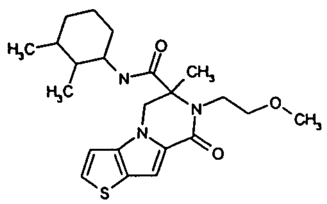 | 417,5747 | 418 | |
| 13 | 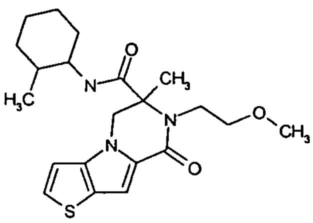 | 403,5476 | 404 | |
| 14 | 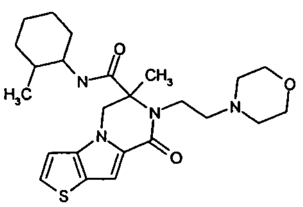 | 458,6276 | 459 | |
| 15 | 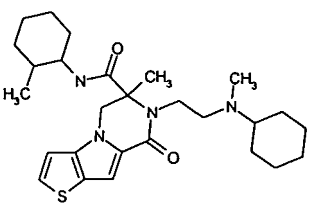 | 484,7095 | 485 | |

(continued)

| № | Formula | Mol. w. | LC MS, m/z (M+1) | 1H NMR |
|---|---|---|---|---|
| 16 | 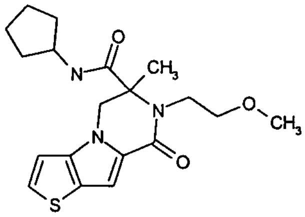 | 375,4934 | 376 | |
| 17 | 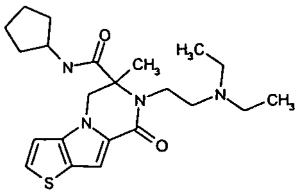 | 416,5899 | 417 | |
| 18 | 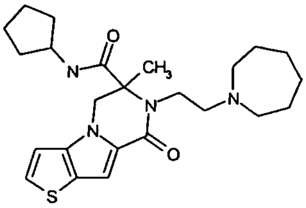 | 442,6282 | 443 | |
| 19 | 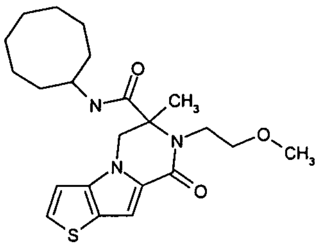 | 417,5747 | 418 | |
| 20 | 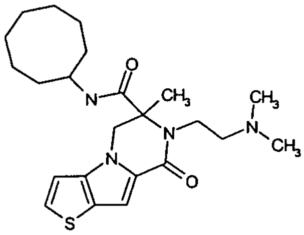 | 430,617 | 431 | |
| 21 | 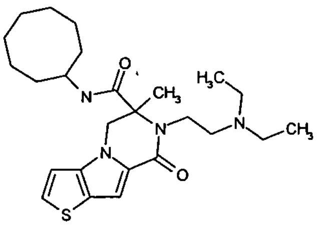 | 458,6712 | 459 | |

(continued)

| № | Formula | Mol. w. | LC MS, m/z (M+1) | 1H NMR |
|---|---|---|---|---|
| 22 | 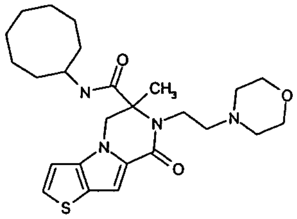 | 472,6547 | 473 | |
| 23 | 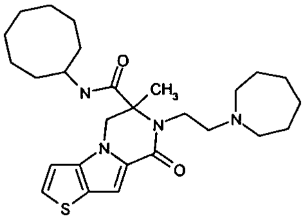 | 484,7095 | 485 | |
| 24 | 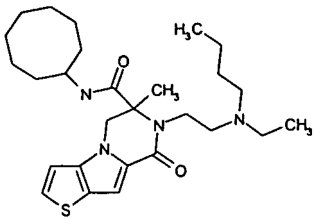 | 486,7254 | 487 | |
| 25 | 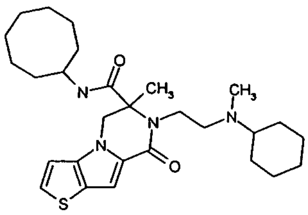 | 498,7365 | 499 | |
| 26 | 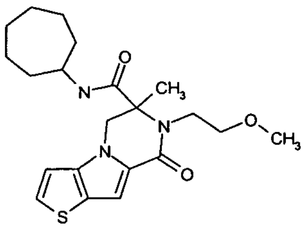 | 403,5476 | 404 | |
| 27 | 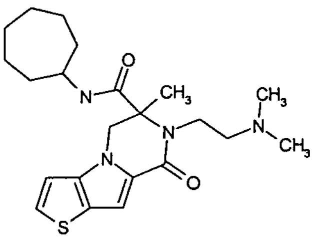 | 416,5899 | 417 | |

(continued)

| № | Formula | Mol. w. | LC MS, m/z (M+1) | 1H NMR |
|---|---|---|---|---|
| 28 | 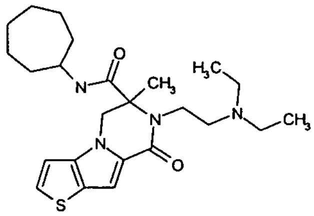 | 444,6441 | 445 | |
| 29 | 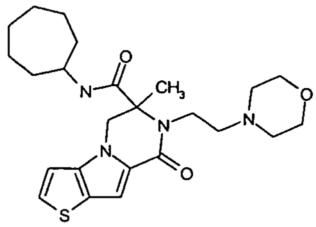 | 458,6276 | 459 | |
| 30 | 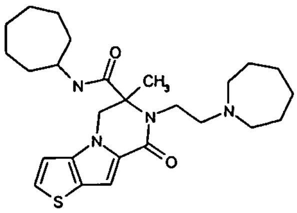 | 470,6824 | 471 | |
| 31 | 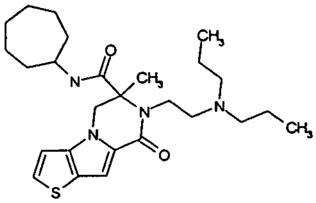 | 472,6983 | 473 | |
| 32 | 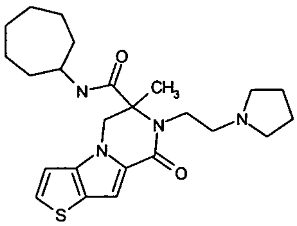 | 442,6282 | 443 | |
| 33 | 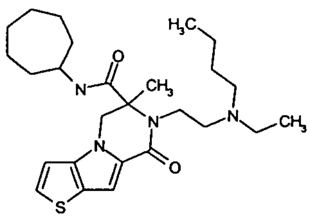 | 472,6983 | 473 | |

(continued)

| № | Formula | Mol. w. | LC MS, m/z (M+1) | 1H NMR |
|---|---|---|---|---|
| 34 | 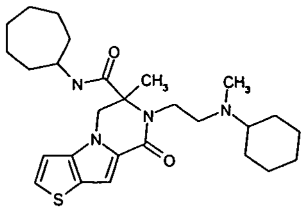 | 484,7095 | 485 | |
| 35 | 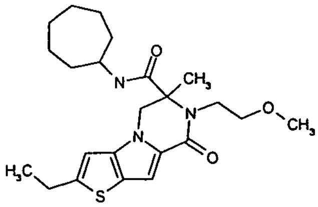 | 431,6018 | 432 | |
| 36 | 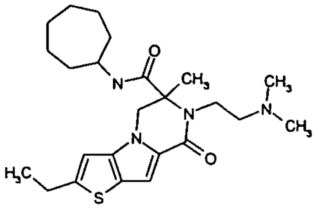 | 444,6441 | 445 | |
| 37 | 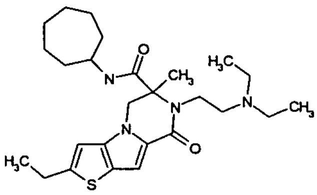 | 472,6983 | 473 | |
| 38 | 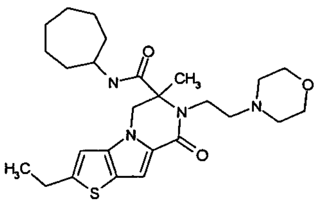 | 486,6818 | 487 | |
| 39 | 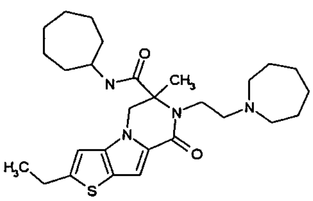 | 498,7365 | 499 | |
| 40 | 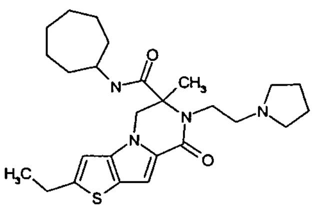 | 470,6824 | 471 | |

(continued)

| № | Formula | Mol. w. | LC MS, m/z (M+1) | 1H NMR |
|---|---|---|---|---|
| 41 | 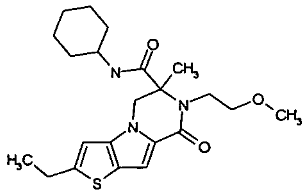 | 417,5747 | 418 | |
| 42 | 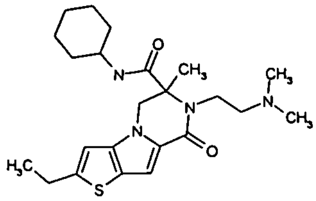 | 430,617 | 431 | |
| 43 | 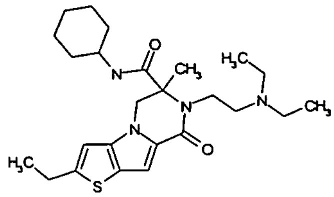 | 458,6712 | 459 | |
| 44 | 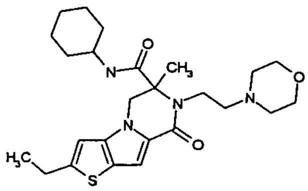 | 472,6547 | 473 | |
| 45 | 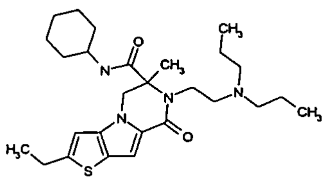 | 486,7254 | 487 | |
| 46 | 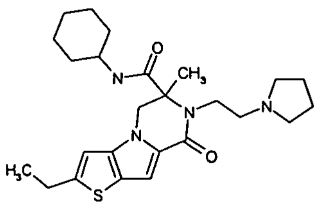 | 456,6553 | 457 | |
| 47 | 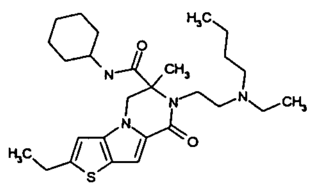 | 486,7254 | 487 | |

(continued)

| № | Formula | Mol. w. | LC MS, m/z (M+1) | 1H NMR |
|---|---|---|---|---|
| 48 | 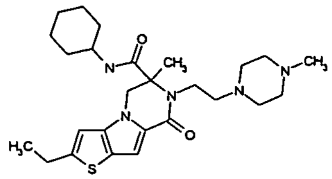 | 485,697 | 486 | |
| 49 | 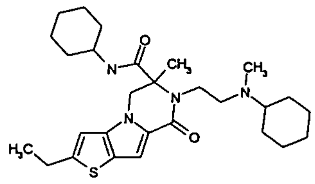 | 498,7365 | 499 | |
| 50 | 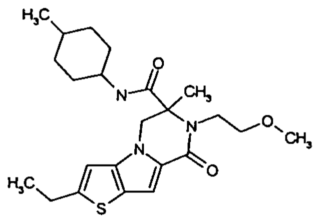 | 431,6018 | 432 | |
| 51 | 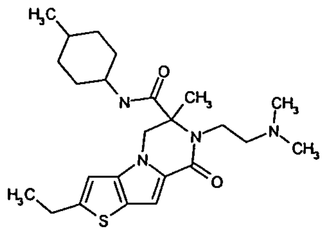 | 444,6441 | 445 | |
| 52 | 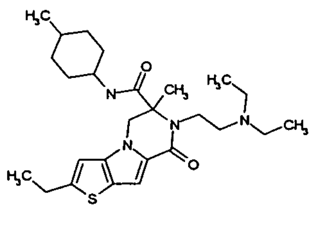 | 472,6983 | 473 | |
| 53 | 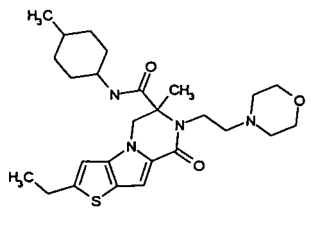 | 486,6818 | 487 | |
| 54 | 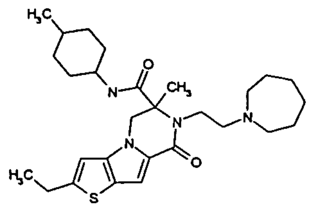 | 498,7365 | 499 | |

(continued)

| № | Formula | Mol. w. | LC MS, m/z (M+1) | 1H NMR |
|---|---|---|---|---|
| 55 | 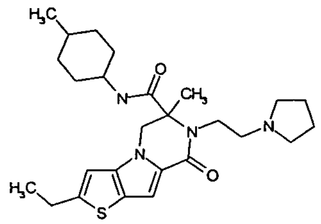 | 470,6824 | 471 | |

Table 1.16(5).

| № | Formula | Mol. w. | LC MS, m/z (M+1) | 1H NMR |
|---|---|---|---|---|
| 1 | 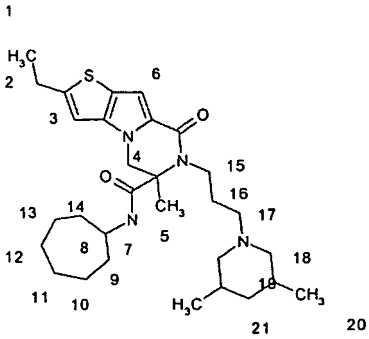 | 526,7907 | 527 | 0.82 (20; 6H; d.; 7.1 Hz), 1.07-1.99 (9, 10, 11, 12, 13, 14, 16, 18, 19, 21; 20H; m.), 1.36 (1; 3H, t.; 7.6 Hz), 1.58 (5; 3H, s.), 2.31 (18; 2H; m.), 2.81 (17; 2H; b.m.), 2.89 (2; 2H, d.; 7.6 Hz), 3.19 + 3.52 (15; 2H; m., m.), 3.79 (8; 1 H; b.m.) .... |
| 2 | 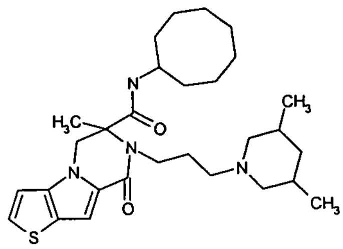 | 512,7636 | 513 | |
| 3 | 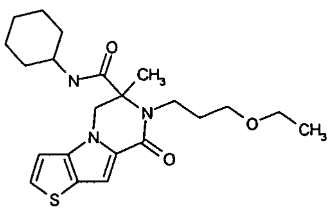 | 417,5747 | 418 | |
| 4 | 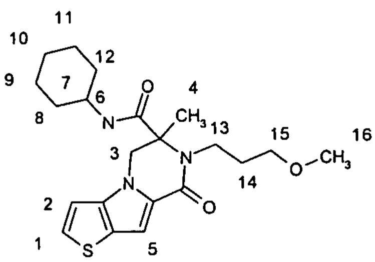 | 403,5476 | 404 | 0.98 - 1.74 (8, 9, 10, 11, 12; 10H; m.), 1.58 (4; 3H, s.), 1.86 (14; 2H; m.), 3.28 (16; 3H; s.), 3.28 + 3.59 (13; 2H; m.m.), 3.39 (15; 2H; t.; 6.1 Hz), 3.47 (7; 1 H; b.m.), 4.08 + 4.72 (3; 2H; AB; 13.3 Hz), 6.88 (5; 1 H; s.), 6.97 (2; 1 H; d.; 5.3 Hz),. |

(continued)

| № | Formula | Mol. w. | LC MS, m/z (M+1) | 1H NMR |
|---|---|---|---|---|
| 5 | 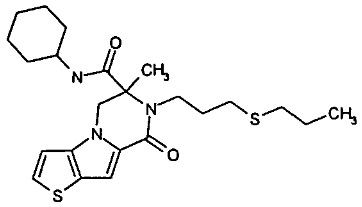 | 447,6664 | 448 | |
| 6 | 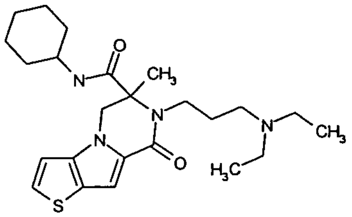 | 444,6441 | 445 | |
| 7 | 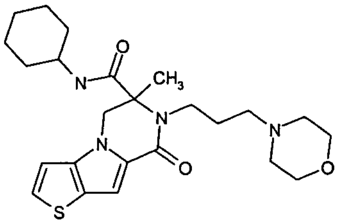 | 458,6276 | 459 | |
| 8 | 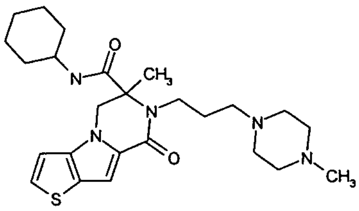 | 471,6699 | 472 | |
| 9 | 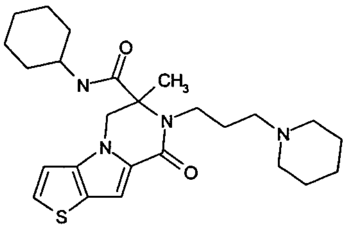 | 456,6553 | 457 | |
| 10 | 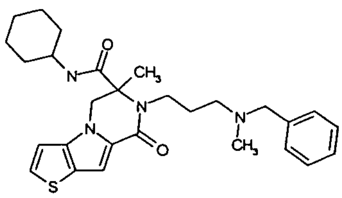 | 492,6887 | 493 | |

(continued)

| № | Formula | Mol. w. | LC MS, m/z (M+1) | 1H NMR |
|---|---|---|---|---|
| 11 | 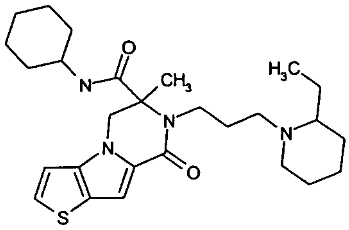 | 484,7095 | 485 | |
| 12 | 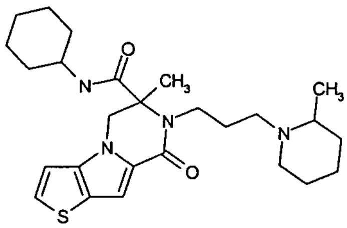 | 470,6824 | 471 | |
| 13 | 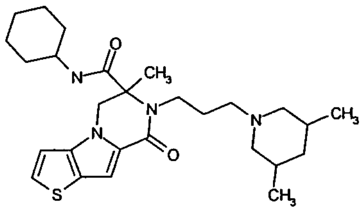 | 484,7095 | 485 | |
| 14 | 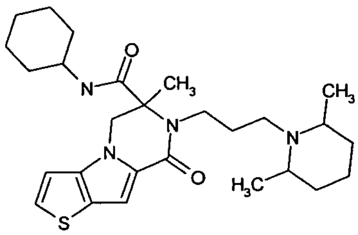 | 484,7095 | 485 | |
| 15 | 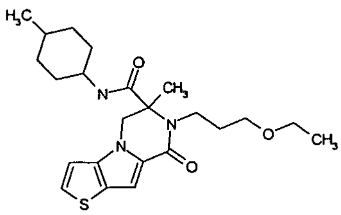 | 431,6018 | 432 | |
| 16 | 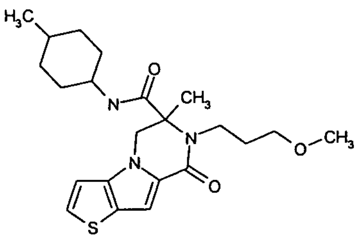 | 417,5747 | 418 | |

(continued)

| № | Formula | Mol. w. | LC MS, m/z (M+1) | 1H NMR |
|---|---|---|---|---|
| 17 | 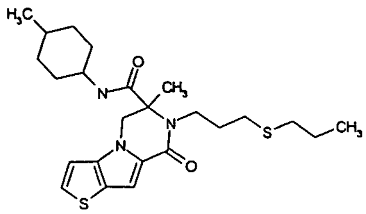 | 461,6935 | 462 | |
| 18 | 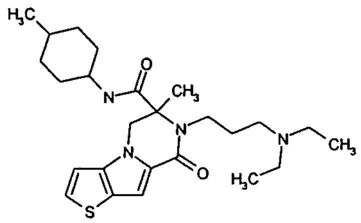 | 458,6712 | 459 | |
| 19 | 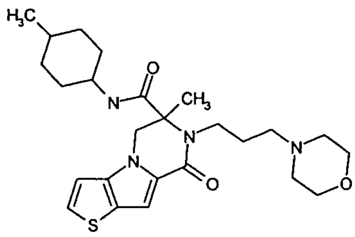 | 472,6547 | 473 | |
| 20 | 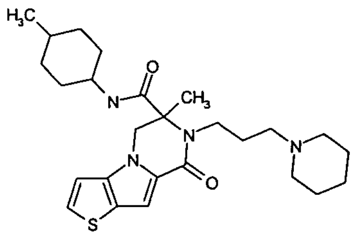 | 470,6824 | 471 | |
| 21 | 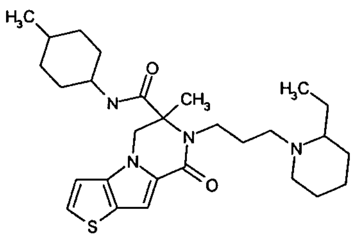 | 498,7365 | 499 | |
| 22 | 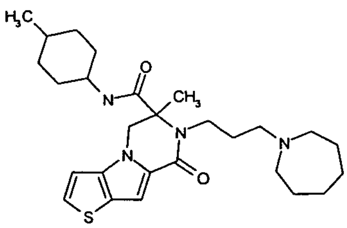 | 484,7095 | 485 | |

(continued)

| № | Formula | Mol. w. | LC MS, m/z (M+1) | 1H NMR |
|---|---|---|---|---|
| 23 | 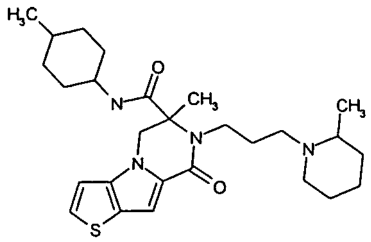 | 484,7095 | 485 | |
| 24 | 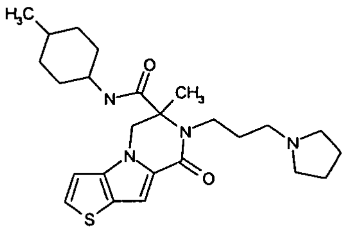 | 456,6553 | 457 | |
| 25 | 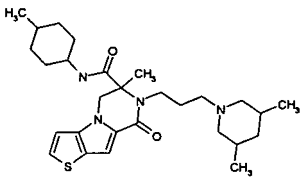 | 498,7365 | 499 | |
| 26 | 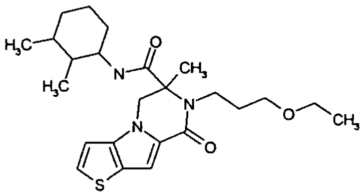 | 445,6289 | 446 | |
| 27 | 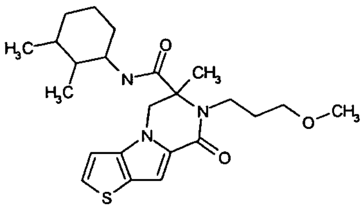 | 431,6018 | 432 | |
| 28 | 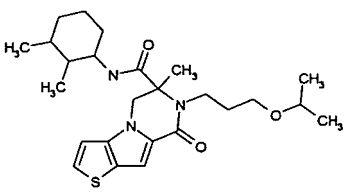 | 459,6559 | 460 | |
| 29 | 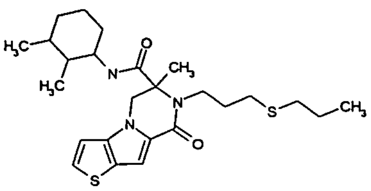 | 475,7205 | 476 | |

(continued)

| № | Formula | Mol. w. | LC MS, m/z (M+1) | 1H NMR |
|---|---|---|---|---|
| 30 | 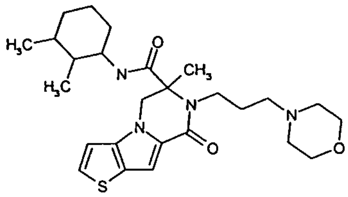 | 486,6818 | 487 | |
| 31 | 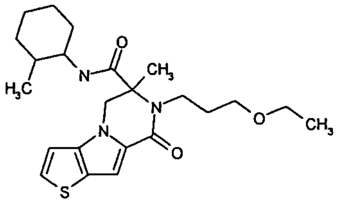 | 431,6018 | 432 | |
| 32 | 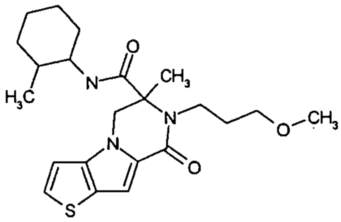 | 417,5747 | 418 | |
| 33 | 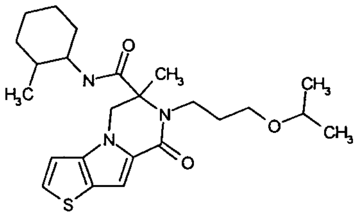 | 445,6289 | 446 | |
| 34 | 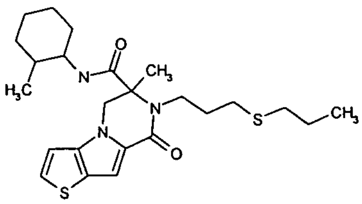 | 461,6935 | 462 | |
| 35 | 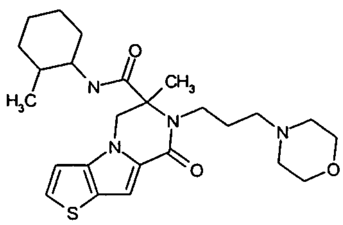 | 472,6547 | 473 | |
| 36 | 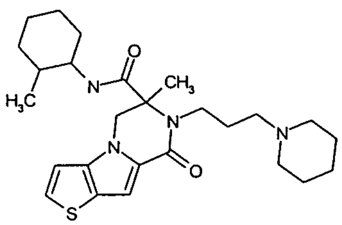 | 470,6824 | 471 | |

(continued)

| № | Formula | Mol. w. | LC MS, m/z (M+1) | 1H NMR |
|---|---|---|---|---|
| 37 | 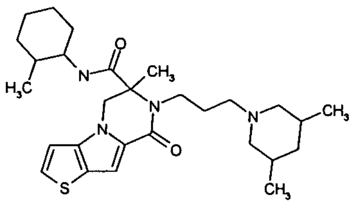 | 498,7365 | 499 | |
| 38 | 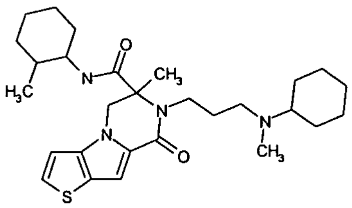 | 498,7365 | 499 | |
| 39 | 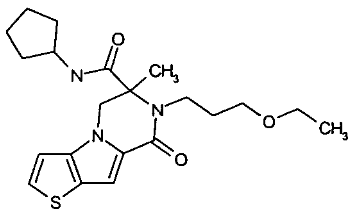 | 403,5476 | 404 | |
| 40 | 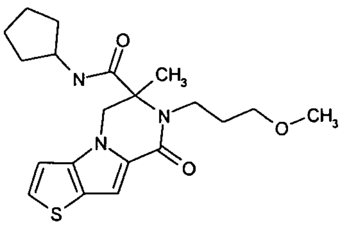 | 389,5205 | 390 | |
| 41 | 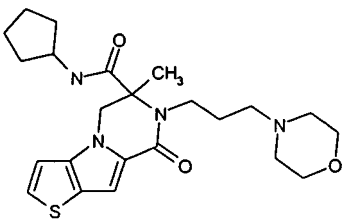 | 444,6005 | 445 | |
| 42 | 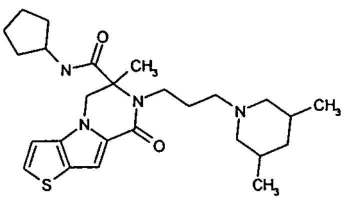 | 470,6824 | 471 | |

(continued)

| № | Formula | Mol. w. | LC MS, m/z (M+1) | 1H NMR |
|---|---|---|---|---|
| 43 | 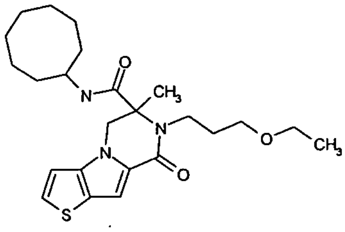 | 445,6289 | 446 | |
| 44 | 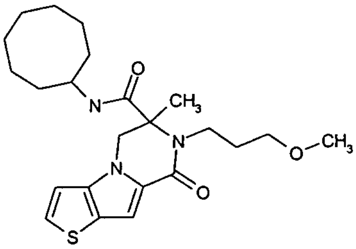 | 431,6018 | 432 | |
| 45 | 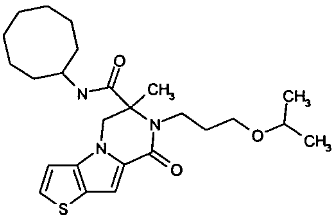 | 459,6559 | 460 | |
| 46 | 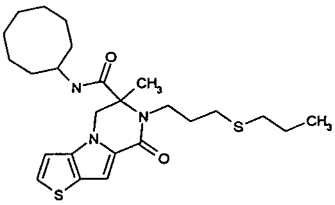 | 475,7205 | 476 | |
| 47 | 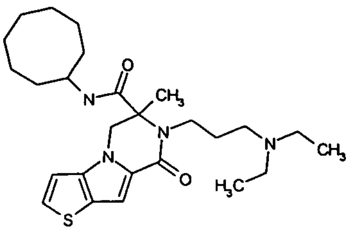 | 472,6983 | 473 | |
| 48 | 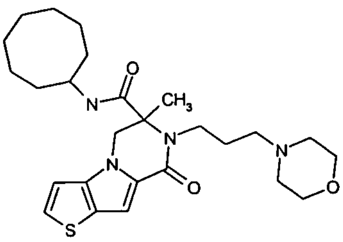 | 486,6818 | 487 | |

(continued)

| № | Formula | Mol. w. | LC MS, m/z (M+1) | 1H NMR |
|---|---|---|---|---|
| 49 | 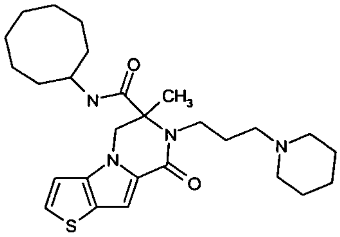 | 484,7095 | 485 | |
| 50 | 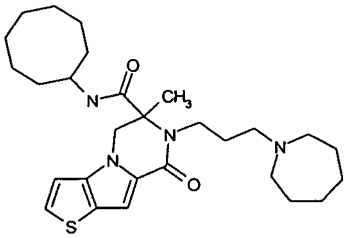 | 498,7365 | 499 | |
| 51 | 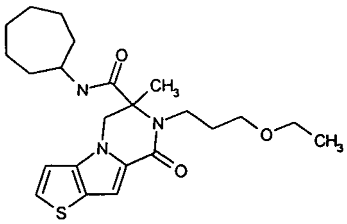 | 431,6018 | 432 | |
| 52 | 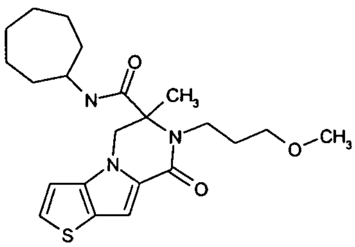 | 417,5747 | 418 | |
| 53 | 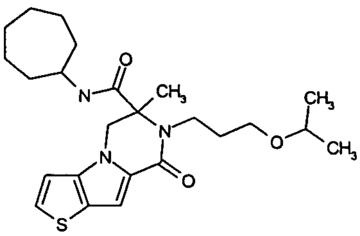 | 445,6289 | 446 | |
| 54 | 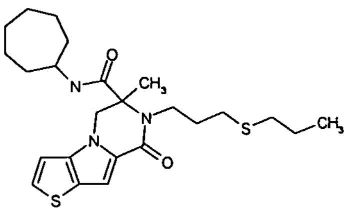 | 461,6935 | 462 | |

(continued)

| № | Formula | Mol. w. | LC MS, m/z (M+1) | 1H NMR |
|---|---|---|---|---|
| 55 | 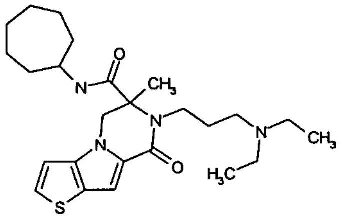 | 458,6712 | 459 | |
| 56 | 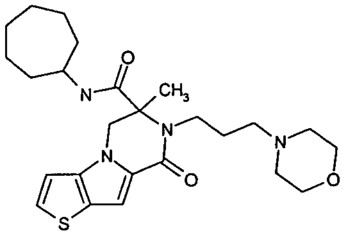 | 472,6547 | 473 | |
| 57 | 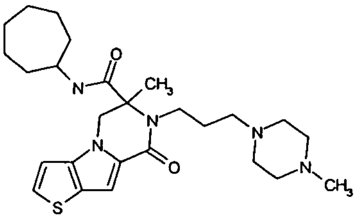 | 485,697 | 486 | |
| 58 | 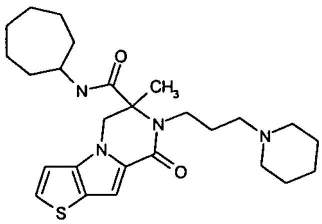 | 470,6824 | 471 | |
| 59 | 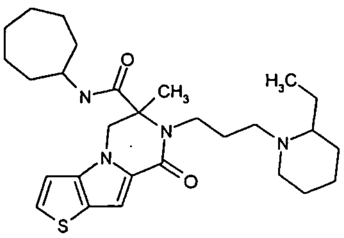 | 498,7365 | 499 | |
| 60 | 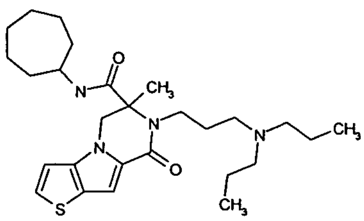 | 486,7254 | 487 | |

(continued)

| № | Formula | Mol. w. | LC MS, m/z (M+1) | 1H NMR |
|---|---|---|---|---|
| 61 | 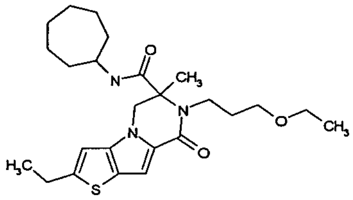 | 459,6559 | 460 | |
| 62 | 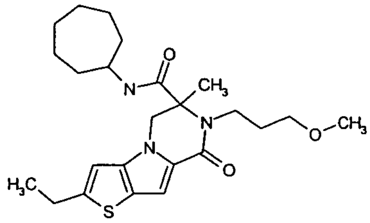 | 445,6289 | 446 | |
| 63 | 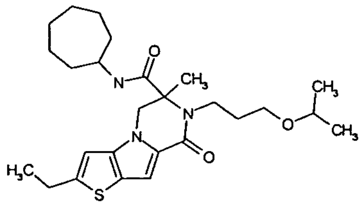 | 473,683 | 474 | |
| 64 | 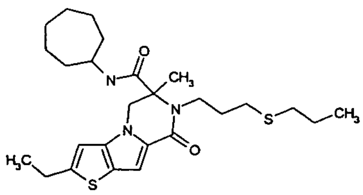 | 489,7476 | 490 | |
| 65 | 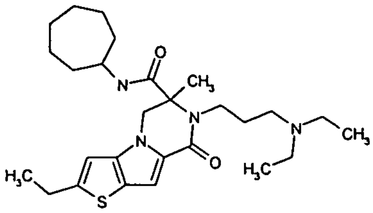 | 486,7254 | 487 | |
| 66 | 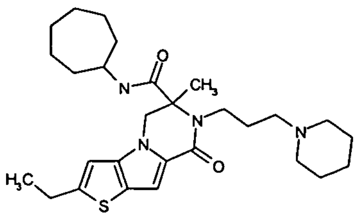 | 498,7365 | 499 | |

(continued)

| № | Formula | Mol. w. | LC MS, m/z (M+1) | 1H NMR |
|---|---|---|---|---|
| 67 | 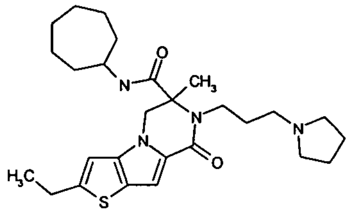 | 484,7095 | 485 | |
| 68 | 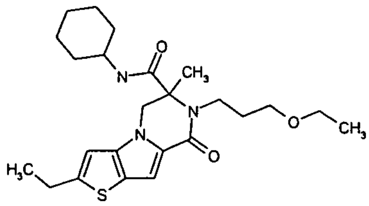 | 445,6289 | 446 | |
| 69 | 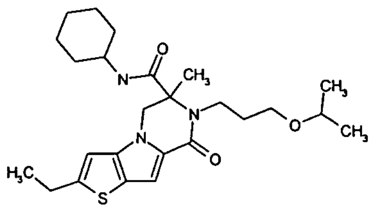 | 459,6559 | 460 | |
| 70 | 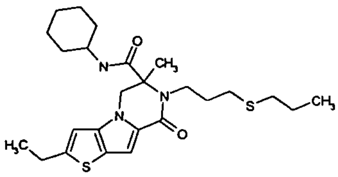 | 475,7205 | 476 | |
| 71 | 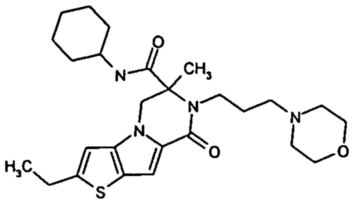 | 486,6818 | 487 | |
| 72 | 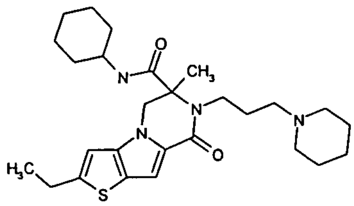 | 484,7095 | 485 | |
| 73 | 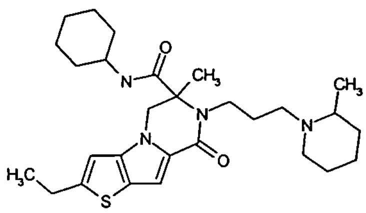 | 498,7365 | 499 | |

(continued)

| № | Formula | Mol. w. | LC MS, m/z (M+1) | 1H NMR |
|---|---|---|---|---|
| 74 | 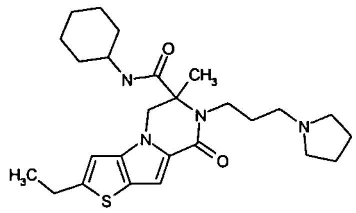 | 470,6824 | 471 | |
| 75 | 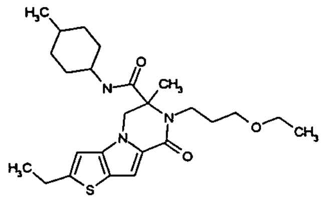 | 459,6559 | 460 | |
| 76 | 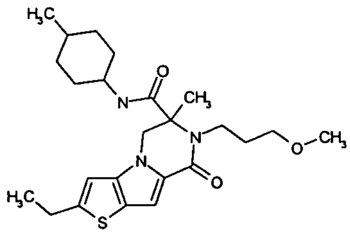 | 445,6289 | 446 | |
| 77 | 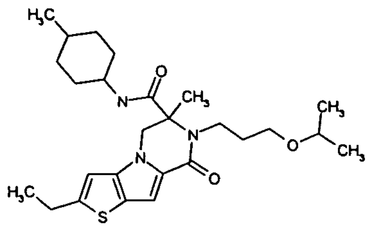 | 473,683 | 474 | |
| 78 | 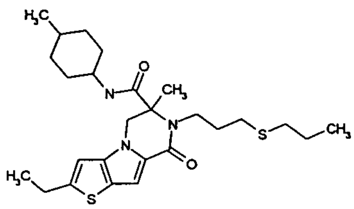 | 489,7476 | 490 | |
| 79 | 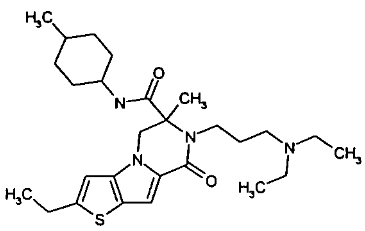 | 486,7254 | 487 | |

(continued)

| № | Formula | Mol. w. | LC MS, m/z (M+1) | 1H NMR |
|---|---|---|---|---|
| 80 | 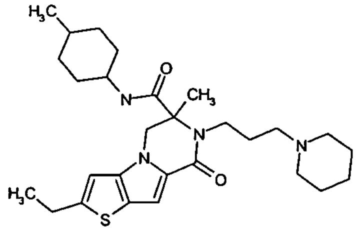 | 498,7365 | 499 | |
| 81 | 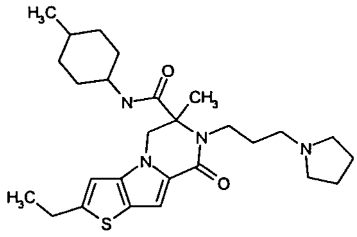 | 484,7095 | 485 | |

| | Table 1.16(6) | | | |
|---|---|---|---|---|
| № | **Formula** | **Mol. w.** | **LC MS, m/z (M+1)** | **1H NMR** |
| 1 | 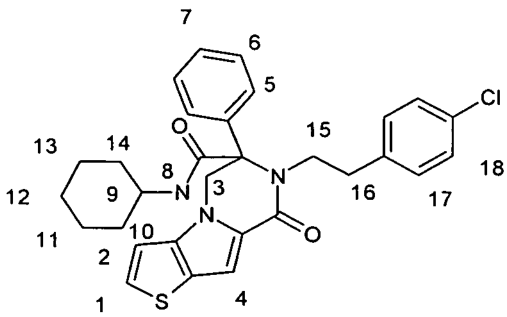 | 532,1095 | 533 | 0.86 - 1.79 (10, 11, 12, 13, 14; 10H; m.), 2.36 + 3.73 (15; 2H; AB; 13.3 Hz), 2.83 (16; 2H; m.), 3.58 (9; 1 H; m.), 4.84 + 5.24 (3; 2H; AB; 12.9 Hz), 6.54 (17; 2H; d.; 7.7 Hz), 6.95 (2, 4; 2H; m.), 7.09 (18; 2H; d.; 7.7 Hz), 7.25 (1; 1H; d.; 5.3 Hz), ... |
| 2 | 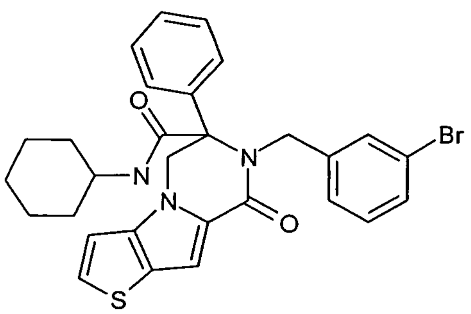 | 562,5334 | 563 | |

(continued)

| | Table 1.16(6) | | | |
|---|---|---|---|---|
| № | Formula | Mol. w. | LC MS, m/z (M+1) | 1H NMR |
| 3 | 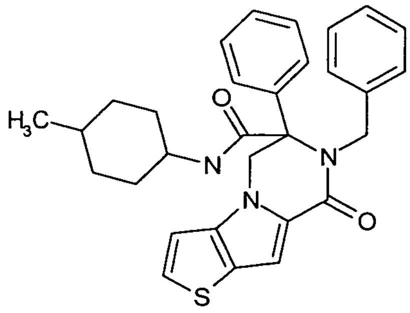 | 497,6645 | 498 | |
| 4 | 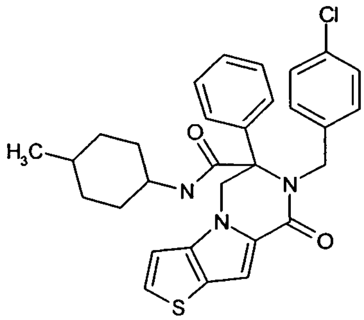 | 532,1095 | 533 | |
| 5 | 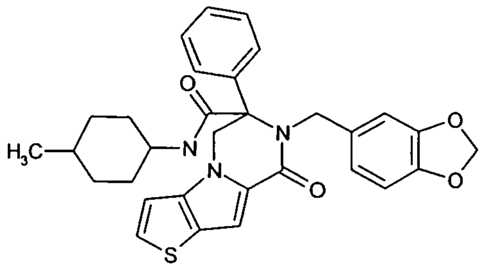 | 541,6744 | 542 | |
| 6 | 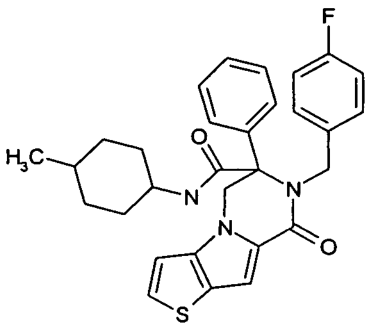 | 515,6549 | 516 | |
| 7 | 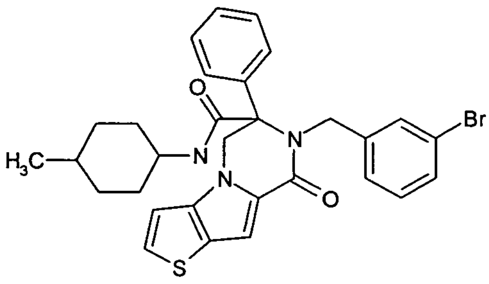 | 576,5605 | 577 | |

(continued)

| | Table 1.16(6) | | | |
|---|---|---|---|---|
| № | Formula | Mol. w. | LC MS, m/z (M+1) | 1H NMR |
| 8 | 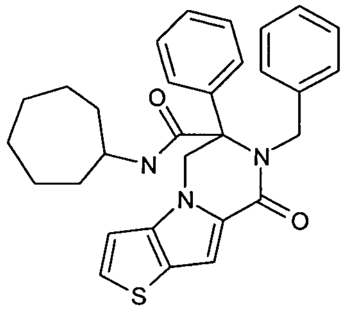 | 497,6645 | 498 | |
| 9 | 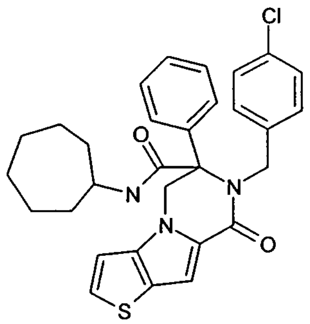 | 532,1095 | 533 | |
| 10 | 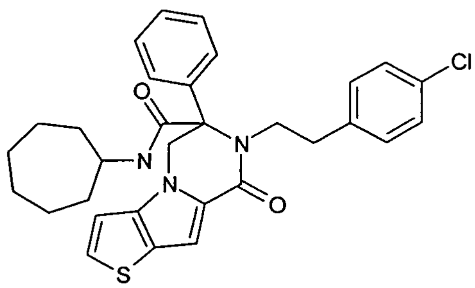 | 546,1366 | 547 | |
| 11 | 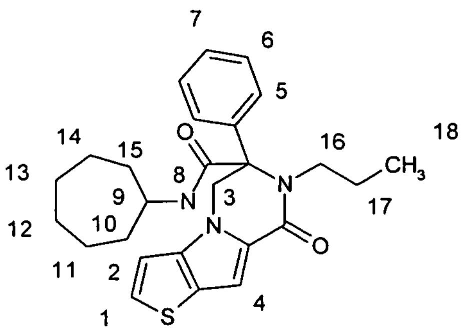 | 449,6199 | 450 | 0.63 (18; 3H; t; 7.1Hz), 1.05 - 1.72 (10, 11, 12, 13, 14, 15; 12H; m.), 2.73 + 3.47 (16; 2H; m.), 3.78 (9; 1H; m.), 4.73 + 5.14 (3; 2H; AB; 12.6 Hz), 6.90 (4; 1H; s.), 6.94 (2; 1 H; d.; 4.5 Hz), 7.14 (8; 1 H; d.; 7.8 Hz), 7.22 (1; 1H; d.; 4.5 Hz), 7.34.. |

(continued)

| | Table 1.16(6) | | | |
|---|---|---|---|---|
| № | Formula | Mol. w. | LC MS, m/z (M+1) | 1H NMR |
| 12 | 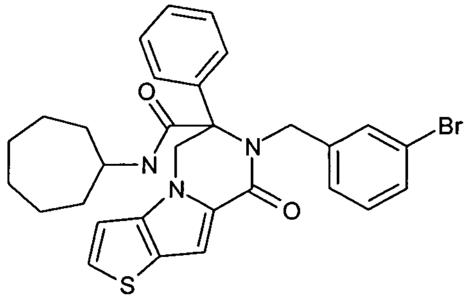 | 576,5605 | 577 | |
| 13 | 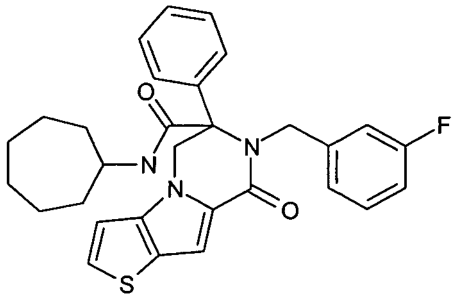 | 515,6549 | 516 | |
| 14 | 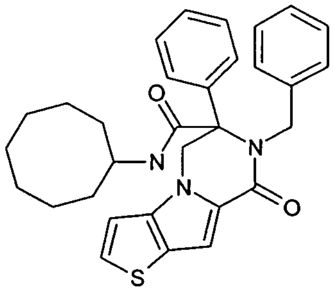 | 511,6916 | 512 | |
| 15 | 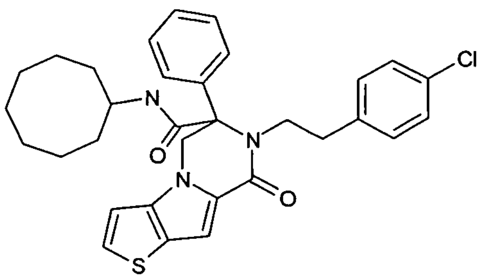 | 560,1637 | 561 | |
| 16 | 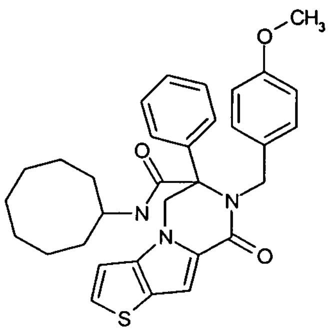 | 541,7181 | 542 | |

(continued)

| | Table 1.16(6) | | | |
|---|---|---|---|---|
| № | Formula | Mol. w. | LC MS, m/z (M+1) | 1H NMR |
| 17 | 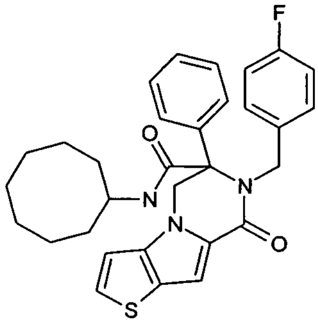 | 529,682 | 530 | |
| 18 | 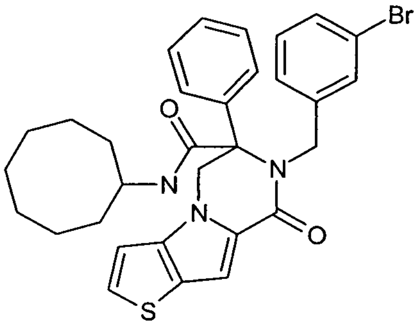 | 590,5876 | 591 | |
| 19 | 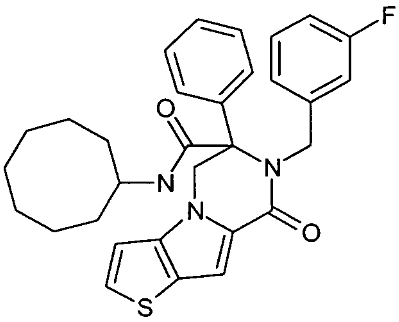 | 529,682 | 530 | |
| 20 | 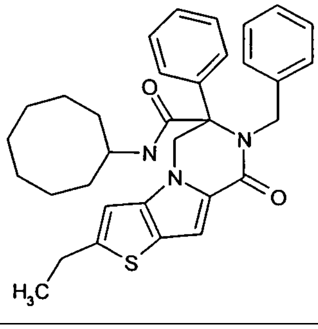 | 539,7457 | 540 | |

(continued)

| | Table 1.16(6) | | | |
|---|---|---|---|---|
| № | Formula | Mol. w. | LC MS, m/z (M+1) | 1H NMR |
| 21 | 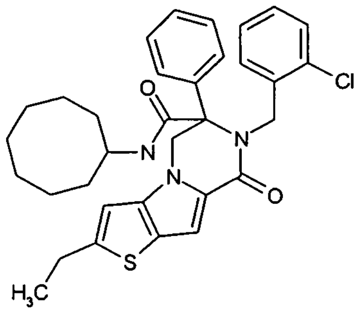 | 574,1908 | 575 | |
| 22 | 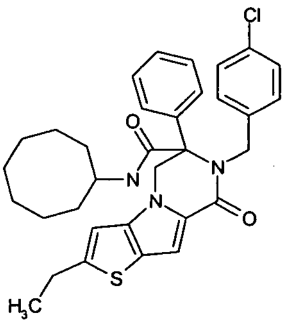 | 574,1908 | 575 | |
| 23 | 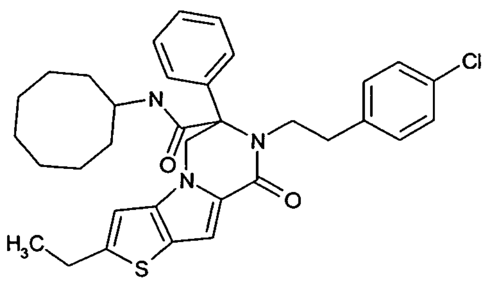 | 588,2179 | 589 | |
| 24 | 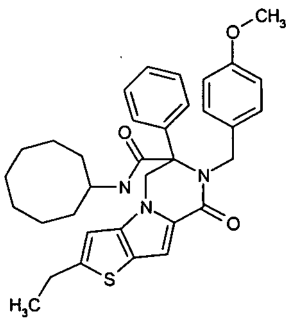 | 569,7722 | 570 | |
| 25 | 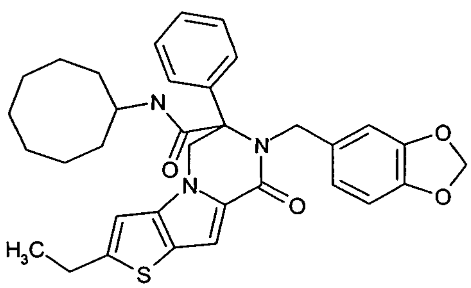 | 583,7557 | 584 | |

(continued)

| | Table 1.16(6) | | | |
|---|---|---|---|---|
| № | Formula | Mol. w. | LC MS, m/z (M+1) | 1H NMR |
| 26 | 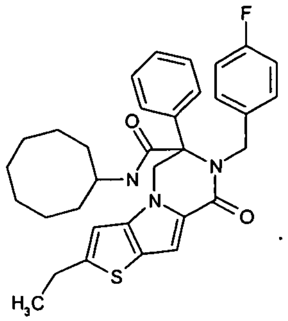 | 557,7362 | 558 | |
| 27 | 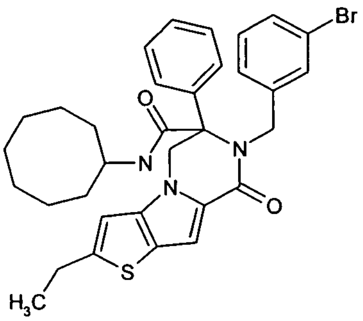 | 618,6418 | 619 | |
| 28 | 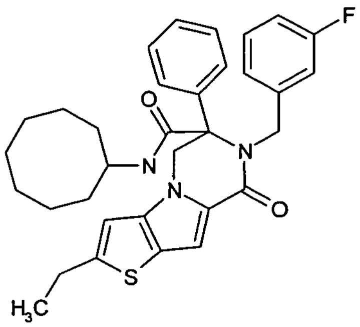 | 557,7362 | 558 | |
| 29 | 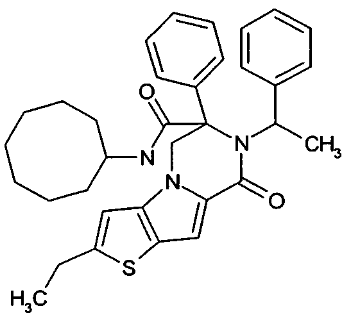 | 553,7728 | 554 | |

(continued)

| | Table 1.16(6) | | | |
|---|---|---|---|---|
| № | Formula | Mol. w. | LC MS, m/z (M+1) | 1H NMR |
| 30 | 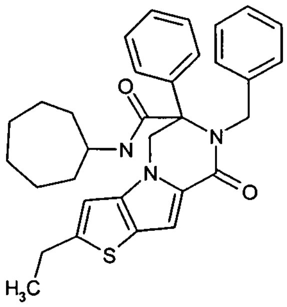 | 525,7187 | 526 | |
| 31 | 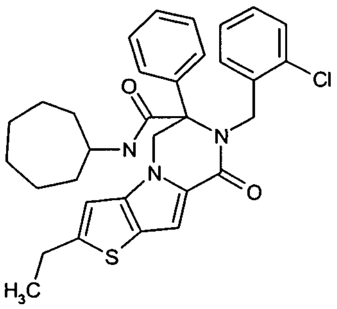 | 560,1637 | 561 | |
| 32 | 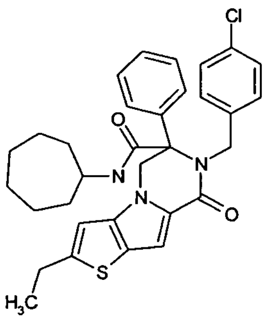 | 560,1637 | 561 | |
| 33 | 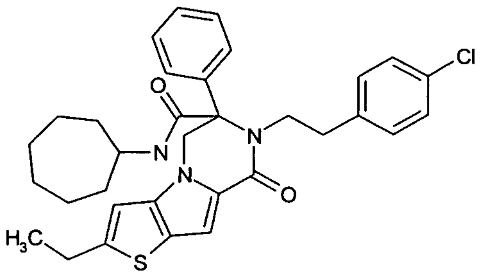 | 574,1908 | 575 | |

(continued)

| | Table 1.16(6) | | | |
|---|---|---|---|---|
| № | Formula | Mol. w. | LC MS, m/z (M+1) | 1H NMR |
| 34 | 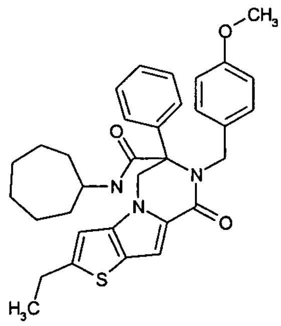 | 555,7451 | 556 | |
| 35 | 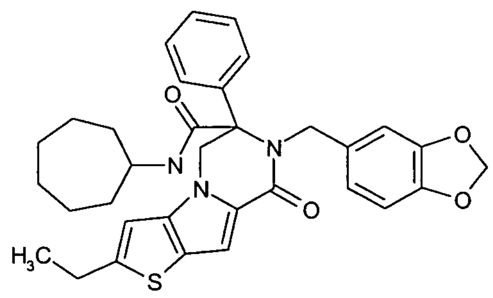 | 569,7286 | 570 | |
| 36 | 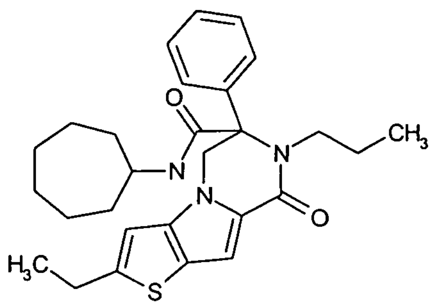 | 477,6741 | 478 | |
| 37 | 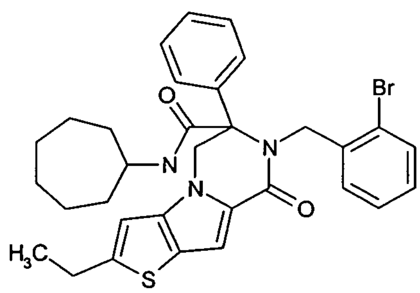 | 604,6147 | 605 | |
| 38 | 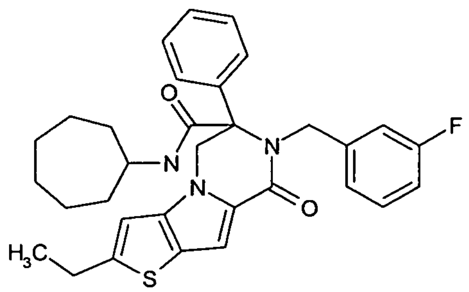 | 543,7091 | 544 | |

(continued)

| | Table 1.16(6) | | | |
|---|---|---|---|---|
| № | Formula | Mol. w. | LC MS, m/z (M+1) | 1H NMR |
| 39 | 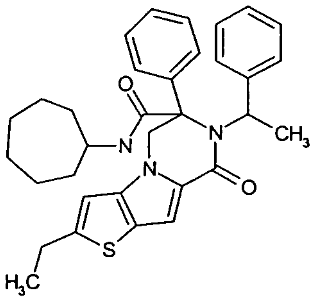 | 539,7457 | 540 | |
| 40 | 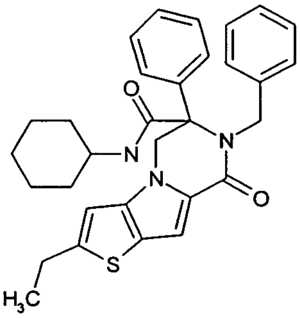 | 511,6916 | 512 | |
| 41 | 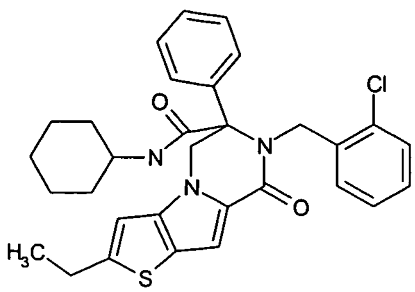 | 546,1366 | 545 | |
| 42 | 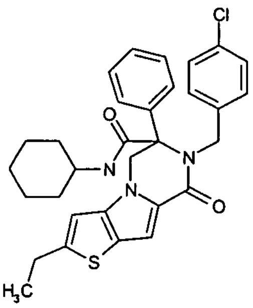 | 546,1366 | 545 | |
| 43 | 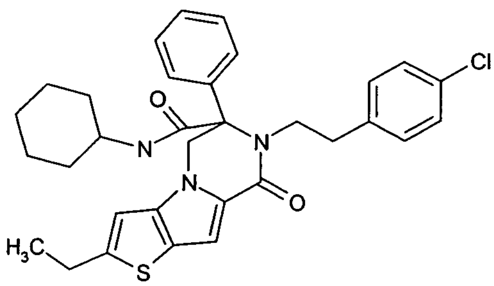 | 560,1637 | 561 | |

(continued)

| | Table 1.16(6) | | | |
|---|---|---|---|---|
| № | Formula | Mol. w. | LC MS, m/z (M+1) | 1H NMR |
| 44 | 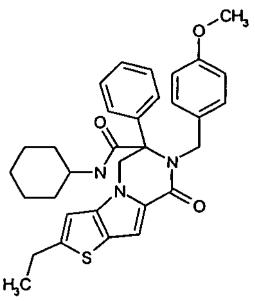 | 541,7181 | 542 | |
| 45 | 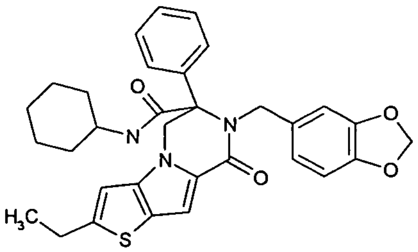 | 555,7015 | 556 | |
| 46 | 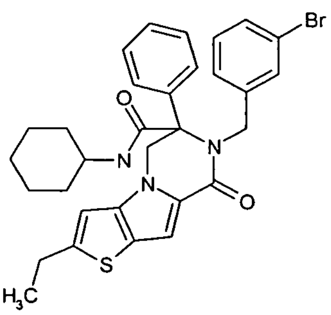 | 590,5876 | 591 | |

| | Table 1.17(1) | | | |
|---|---|---|---|---|
| № | Formula | Mol.w. | m/z (M+1) | 1H NMR |
| 1 | 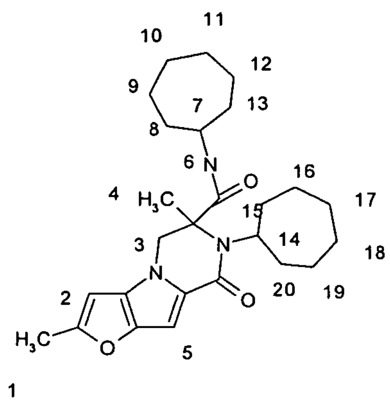 | 439,6031 | 440 | 1.02- 1.99 (8, 9, 10, 11, 12, 13, 15, 16, 17, 18, 19, 20; 24H; m.), 1.40 (4; 3H, s.), 2.41 (1; 3H, s.), 2.79 (14; 1H; b.m.), 3.79 (7; 1 H; b.m.), 3.81 + 4.41 (3; 2H; AB; 12.4 Hz), 6.11 (2; 1H; s.), 6.45 (5; 1 H; s.), 7.88 (6; 1H; d.; 7.2 Hz) |

(continued)

| | Table 1.17(1) | | | |
|---|---|---|---|---|
| № | Formula | Mol.w. | m/z (M+1) | 1H NMR |
| 2 | 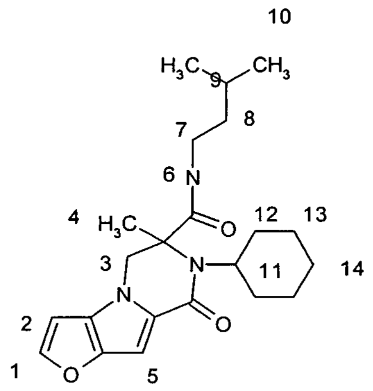 | 385,5107 | 386 | 0.89 (10; 6H; m.), 1.05 - 1.45 (8, 9, 14; 5H; m.), 1.45 (4; 3H, s.), 1.50 - 1.91, 2.50 - 2.89, 3.05 - 3.28 (7, 11, 12, 13; 11H; m.m.), 3.95 + 4.41 (3; 2H; AB; 12.4 Hz), 6.45 (2; 1 H; d., 1.4 Hz), 6.52 (5; 1H; s.), 7.42 (1; 1H; d., 1.4 Hz), 8.15 (6; 1 H... |
| 3 | 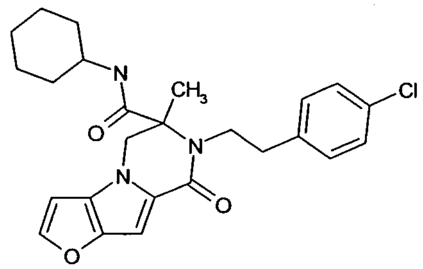 | 453,9732 | 454 | |
| 4 | 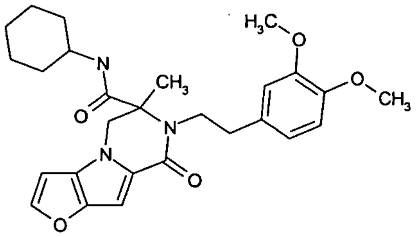 | 479,5812 | 480 | |
| 5 | 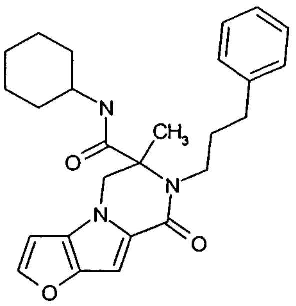 | 433,5553 | 434 | |
| 6 | 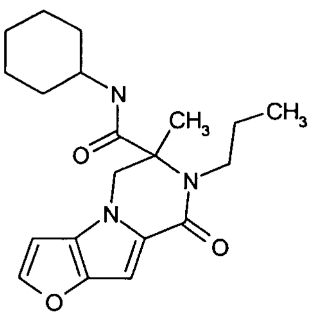 | 357,4565 | 358 | |

(continued)

| | Table 1.17(1) | | | |
|---|---|---|---|---|
| № | Formula | Mol.w. | m/z (M+1) | 1H NMR |
| 7 | 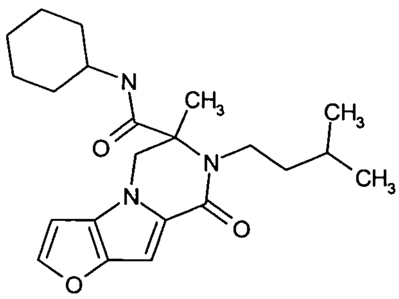 | 385,5107 | 386 | |
| 8 | 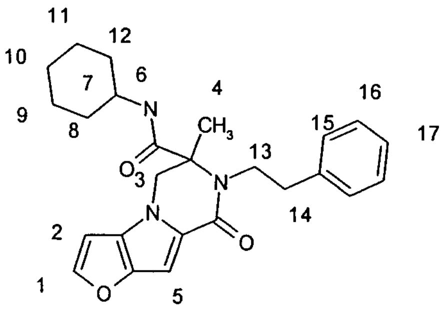 | 419,5282 | 420 | 1.03 - 1.72 (8, 9, 10, 11, 12; 10H; m.), 1.44 (4; 3H, s.), 2.87 (14; 2H, m.), 3.27 + 3.73 (13; 2H; AB; m.m.), 3.45 (7; 1 H; b.m.), 3.92 + 4.61 (3; 2H; AB; 12.6 Hz), 6.51 (2; 1H; d.; 2.1 Hz), 6.58 (5; 1H; s.), 7.08 - 7.33 (6, 15, 16, 17; 6H, m.), 7.48 ... |
| 9 | 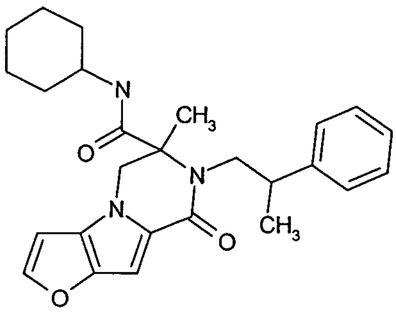 | 433,5553 | 434 | |
| 10 | 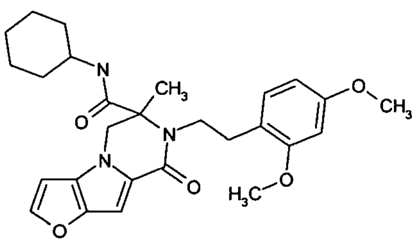 | 479,5812 | 480 | |
| 11 | 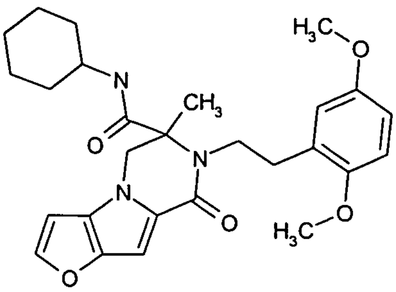 | 479,5812 | 480 | |

(continued)

| | Table 1.17(1) | | | |
|---|---|---|---|---|
| № | Formula | Mol.w. | m/z (M+1) | 1H NMR |
| 12 | 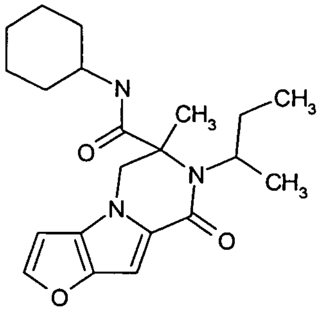 | 371,4836 | 372 | |
| 13 | 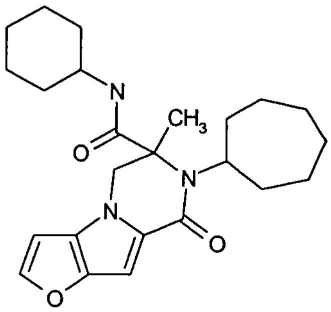 | 411,5489 | 412 | |
| 14 | 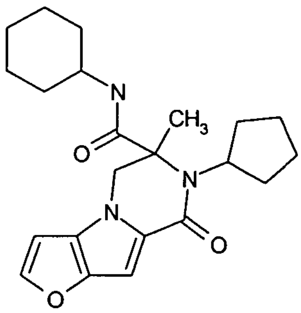 | 383,4947 | 384 | |
| 15 | 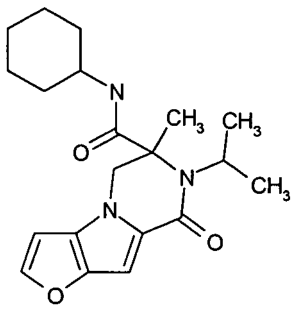 | 357,4565 | 358 | |

(continued)

| | Table 1.17(1) | | | |
|---|---|---|---|---|
| № | Formula | Mol.w. | m/z (M+1) | 1H NMR |
| 16 | 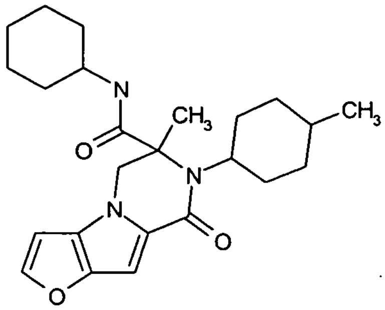 | 411,5489 | 412 | |
| 17 | 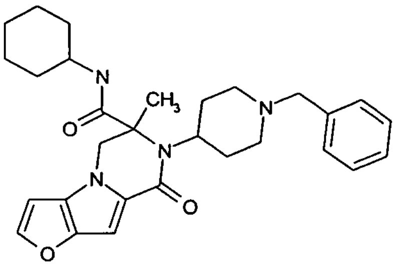 | 488,6353 | 489 | |
| 18 | 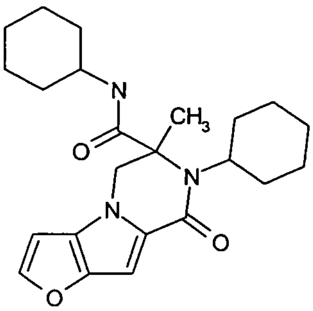 | 397,5218 | 398 | |
| 19 | 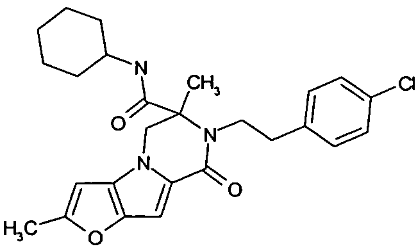 | 468,0003 | 469 | |
| 20 | 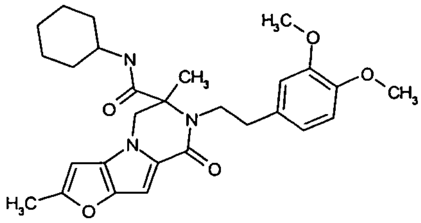 | 493,6083 | 494 | |

(continued)

| | Table 1.17(1) | | | |
|---|---|---|---|---|
| № | Formula | Mol.w. | m/z (M+1) | 1H NMR |
| 21 | 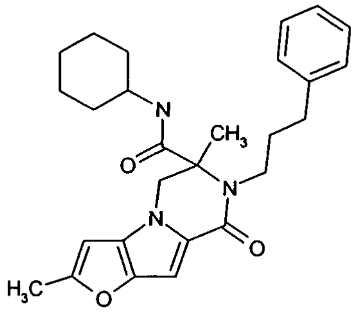 | 447,5824 | 448 | |
| 22 | 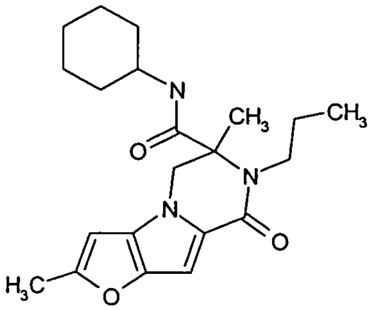 | 371,4836 | 372 | |
| 23 | 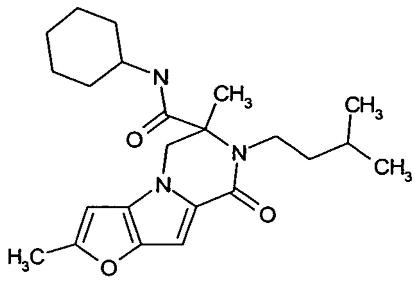 | 399,5378 | 400 | |
| 24 | 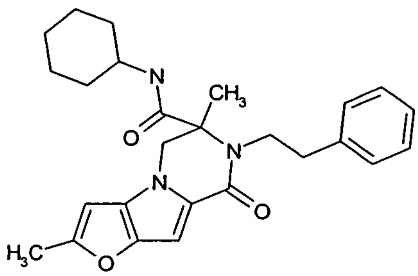 | 433,5553 | 434 | |
| 25 | 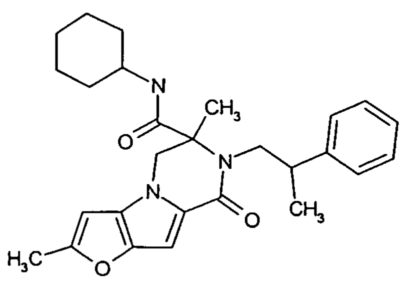 | 447,5824 | 448 | |

(continued)

| | Table 1.17(1) | | | |
|---|---|---|---|---|
| № | Formula | Mol.w. | m/z (M+1) | 1H NMR |
| 26 | 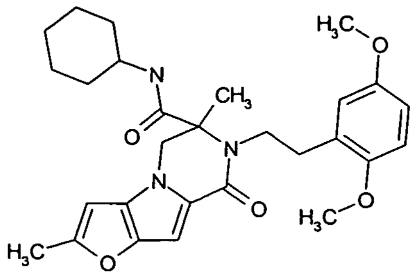 | 493,6083 | 494 | |
| 27 | 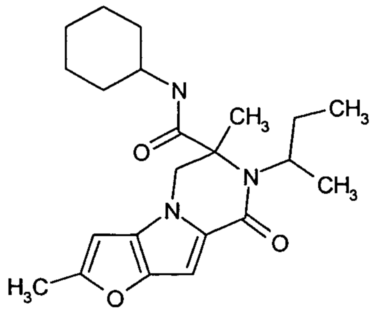 | 385,5107 | 386 | |
| 28 | 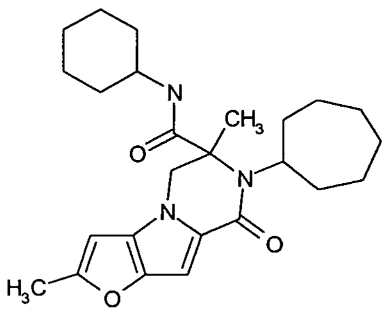 | 425,576 | 426 | |
| 29 | 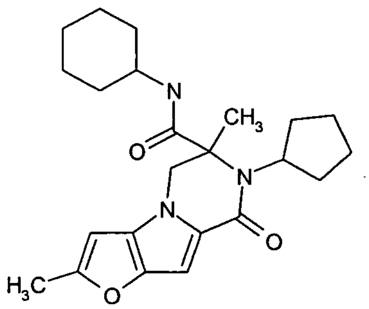 | 397,5218 | 398 | |
| 30 | 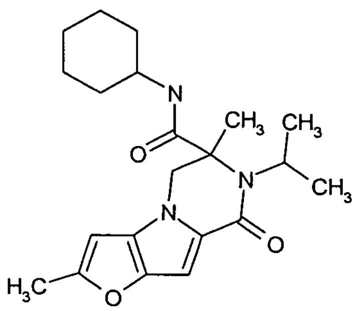 | 371,4836 | 372 | |

(continued)

| | Table 1.17(1) | | | |
|---|---|---|---|---|
| № | Formula | Mol.w. | m/z (M+1) | 1H NMR |
| 31 | 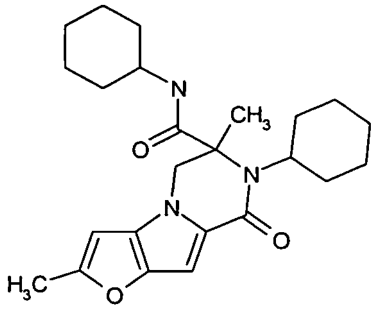 | 411,5489 | 412 | |
| 32 | 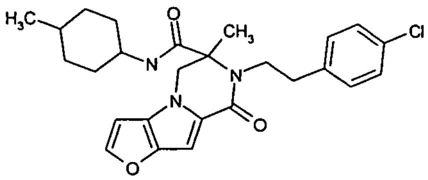 | 468,0003 | 469 | |
| 33 | 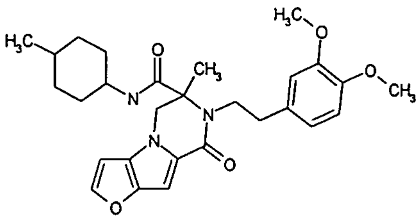 | 493,6083 | 494 | |
| 34 | 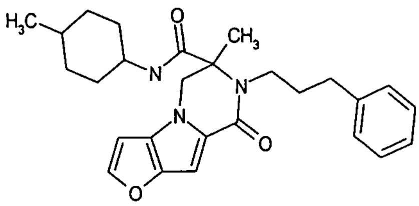 | 447,5824 | 448 | |
| 35 | 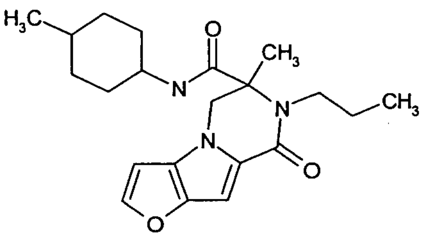 | 371,4836 | 372 | |
| 36 | 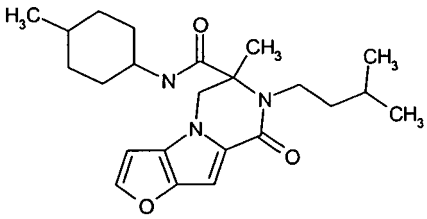 | 399,5378 | 400 | |

(continued)

| | Table 1.17(1) | | | |
|---|---|---|---|---|
| № | Formula | Mol.w. | m/z (M+1) | 1H NMR |
| 37 | 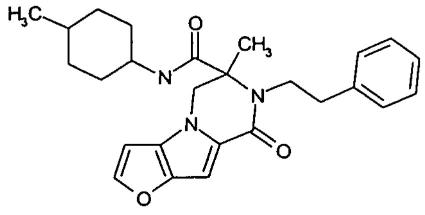 | 433,5553 | 434 | |
| 38 | 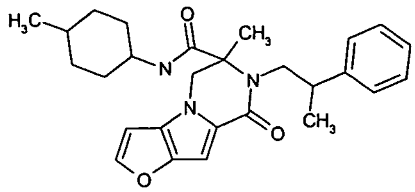 | 447,5824 | 448 | |
| 39 | 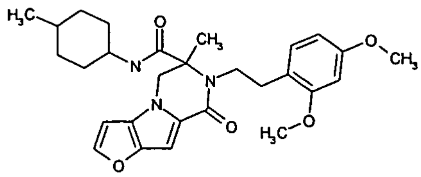 | 493,6083 | 494 | |
| 40 | 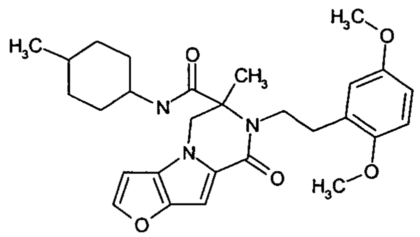 | 493,6083 | 494 | |
| 41 | 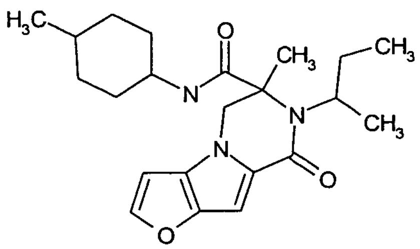 | 385,5107 | 386 | |
| 42 | 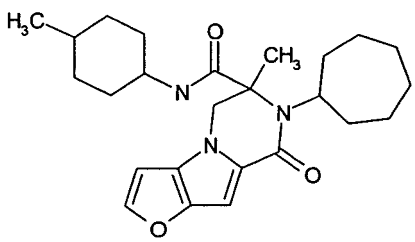 | 425,576 | 426 | |

(continued)

| | Table 1.17(1) | | | |
|---|---|---|---|---|
| № | Formula | Mol.w. | m/z (M+1) | 1H NMR |
| 43 | 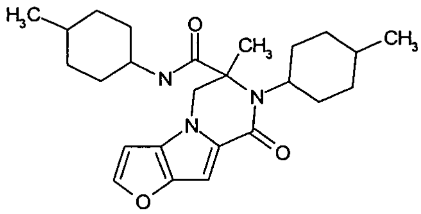 | 425,576 | 426 | |
| 44 | 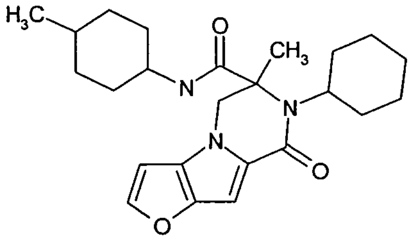 | 411,5489 | 412 | |
| 45 | 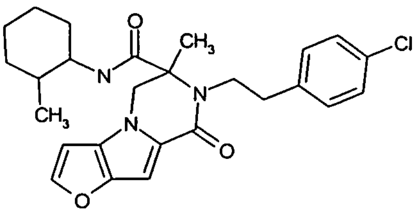 | 468,0003 | 469 | |
| 46 | 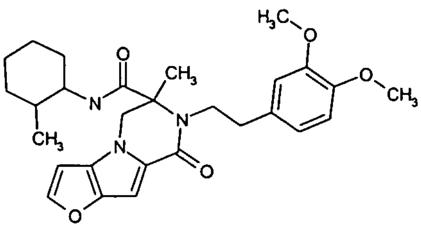 | 493,6083 | 494 | |
| 47 | 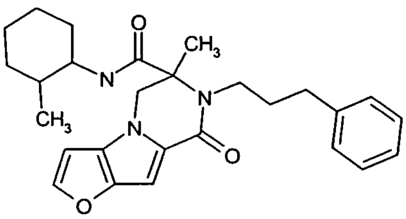 | 447,5824 | 448 | |
| 48 | 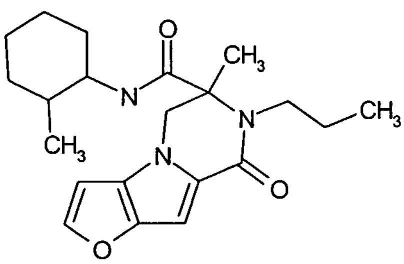 | 371,4836 | 372 | |

(continued)

| | Table 1.17(1) | | | |
|---|---|---|---|---|
| № | Formula | Mol.w. | m/z (M+1) | 1H NMR |
| 49 | 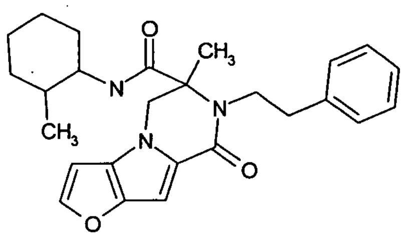 | 433,5553 | 434 | |
| 50 | 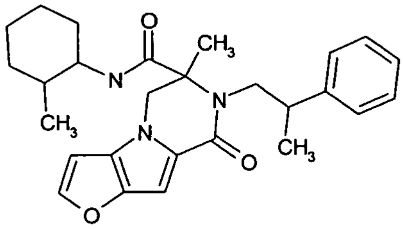 | 447,5824 | 448 | |
| 51 | 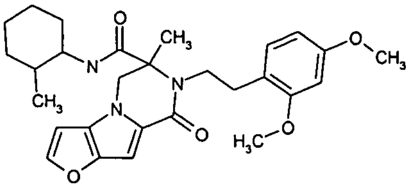 | 493,6083 | 494 | |
| 52 | 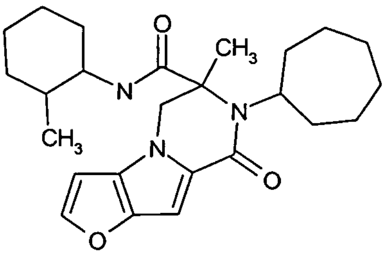 | 425,576 | 426 | |
| 53 | 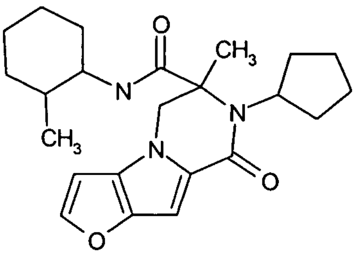 | 397,5218 | 398 | |
| 54 | 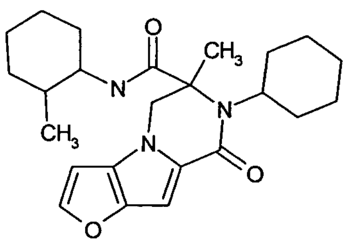 | 411,5489 | 412 | |

(continued)

| | Table 1.17(1) | | | |
|---|---|---|---|---|
| № | Formula | Mol.w. | m/z (M+1) | 1H NMR |
| 55 | 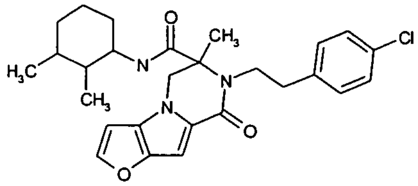 | 482,0274 | 483 | |
| 56 | 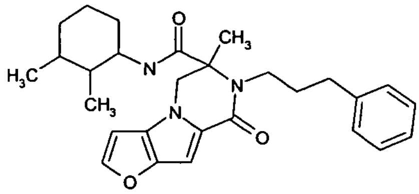 | 461,6095 | 462 | |
| 57 | 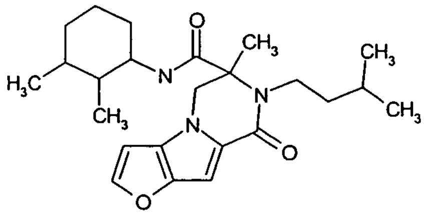 | 413,5649 | 414 | |
| 58 | 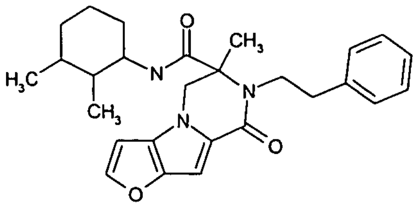 | 447,5824 | 448 | |
| 59 | 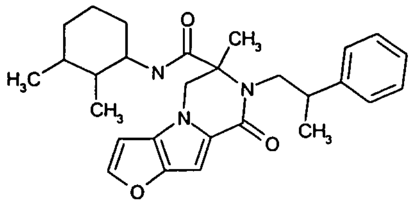 | 461,6095 | 462 | |
| 60 | 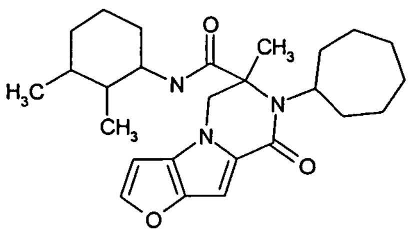 | 439,6031 | 440 | |

(continued)

| | Table 1.17(1) | | | |
|---|---|---|---|---|
| № | Formula | Mol.w. | m/z (M+1) | 1H NMR |
| 61 | 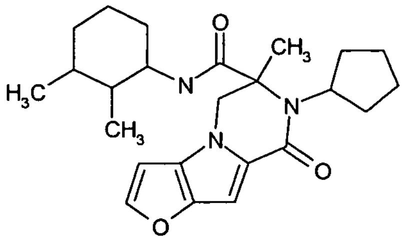 | 411,5489 | 412 | |
| 62 | 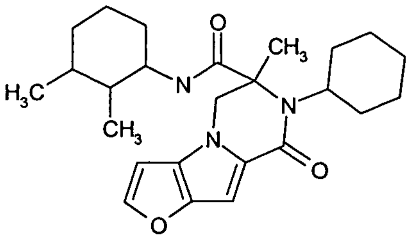 | 425,576 | 426 | |
| 63 | 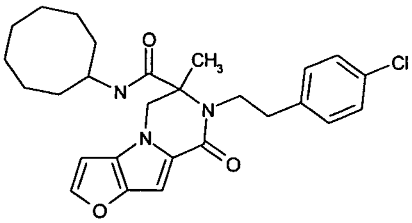 | 482,0274 | 483 | |
| 64 | 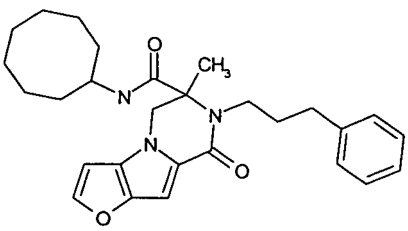 | 461,6095 | 462 | |
| 65 | 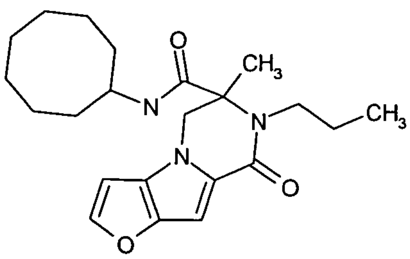 | 385,5107 | 386 | |
| 66 | 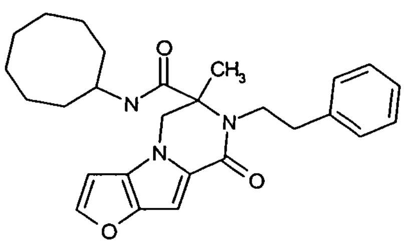 | 447,5824 | 448 | |

(continued)

| | Table 1.17(1) | | | |
|---|---|---|---|---|
| № | Formula | Mol.w. | m/z (M+1) | 1H NMR |
| 67 | 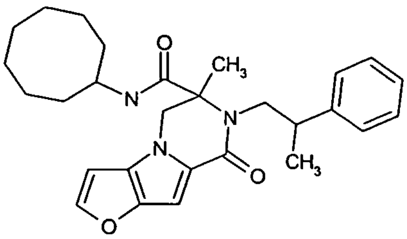 | 461,6095 | 462 | |
| 68 | 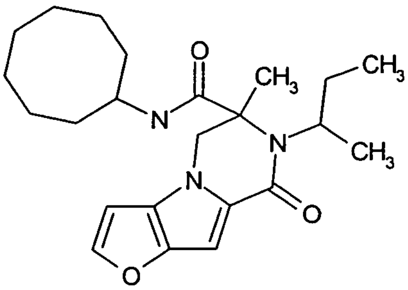 | 399,5378 | 400 | |
| 69 | 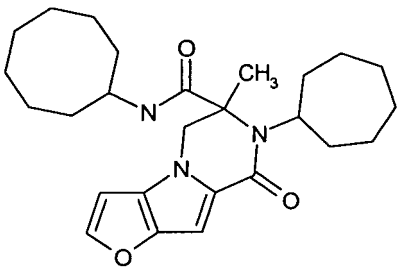 | 439,6031 | 440 | |
| 70 | 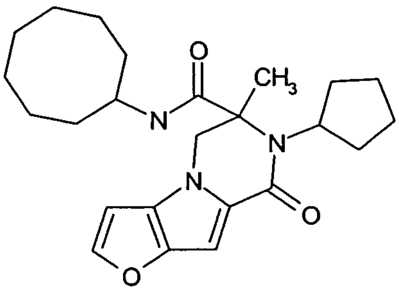 | 411,5489 | 412 | |
| 71 | 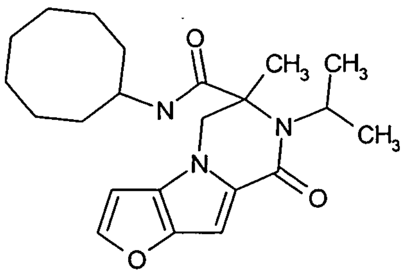 | 385,5107 | 386 | |

(continued)

| | Table 1.17(1) | | | |
|---|---|---|---|---|
| № | Formula | Mol.w. | m/z (M+1) | 1H NMR |
| 72 | 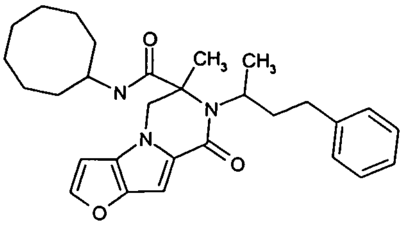 | 475,6365 | 476 | |
| 73 | 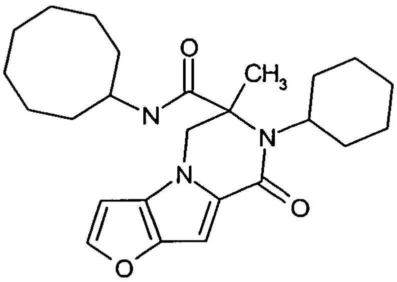 | 425,576 | 426 | |
| 74 | 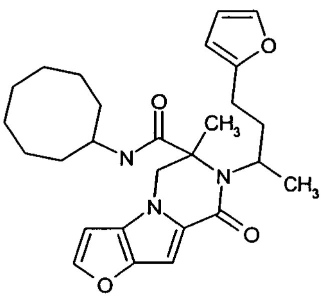 | 465,5977 | 466 | |
| 75 | 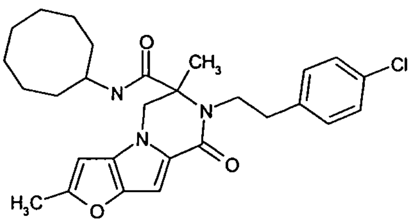 | 496,0545 | 497 | |
| 76 | 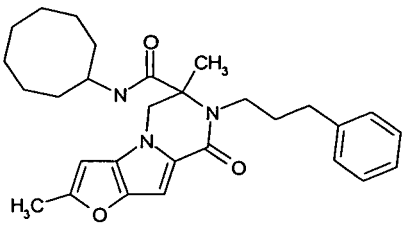 | 475,6365 | 476 | |

(continued)

| | Table 1.17(1) | | | |
|---|---|---|---|---|
| № | Formula | Mol.w. | m/z (M+1) | 1H NMR |
| 77 | 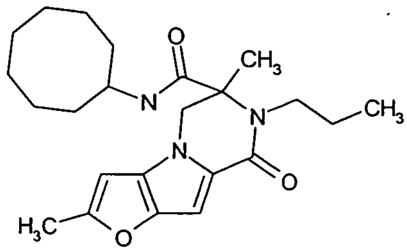 | 399,5378 | 400 | |
| 78 | 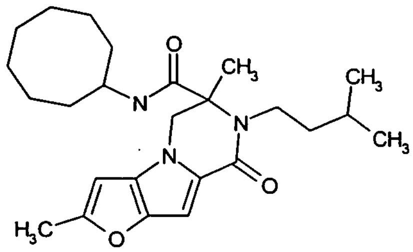 | 427,5919 | 428 | |
| 79 | 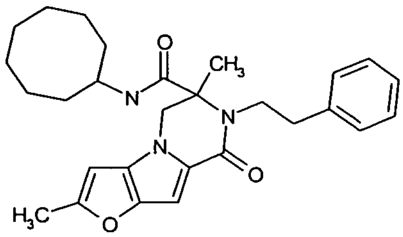 | 461,6095 | 462 | |
| 80 | 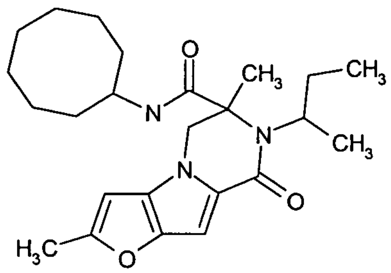 | 413,5649 | 414 | |
| 81 | 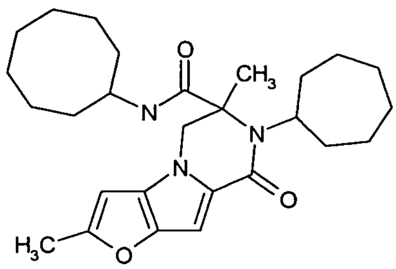 | 453,6302 | 454 | |

(continued)

| | Table 1.17(1) | | | |
|---|---|---|---|---|
| № | Formula | Mol.w. | m/z (M+1) | 1H NMR |
| 82 | 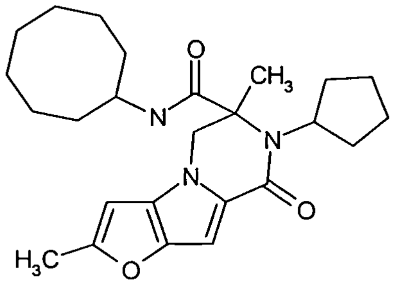 | 425,576 | 426 | |
| 83 | 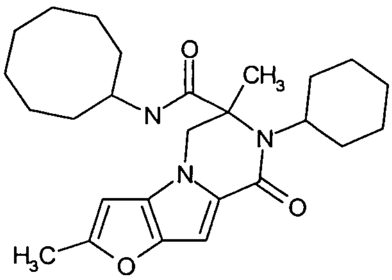 | 439,6031 | 440 | |
| 84 | 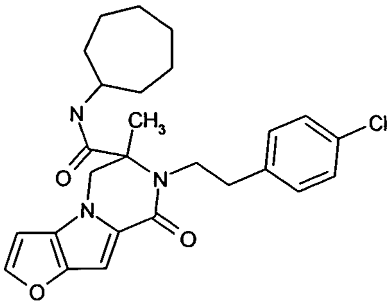 | 468,0003 | 469 | |
| 85 | 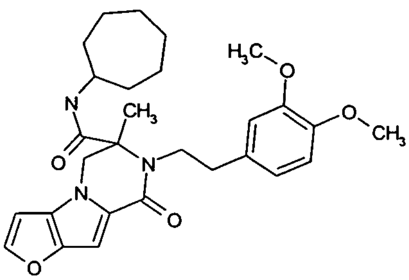 | 493,6083 | 494 | |
| 86 | 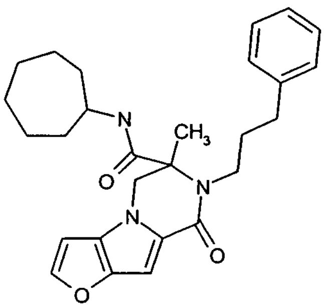 | 447,5824 | 448 | |

(continued)

| | Table 1.17(1) | | | |
|---|---|---|---|---|
| № | Formula | Mol.w. | m/z (M+1) | 1H NMR |
| 87 | 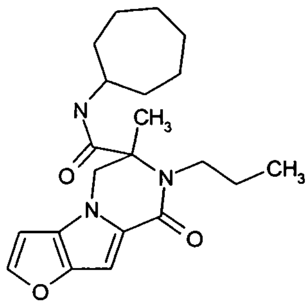 | 371,4836 | 372 | |
| 88 | 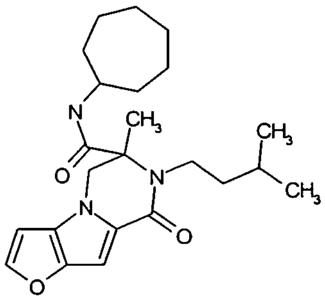 | 399,5378 | 400 | |
| 89 | 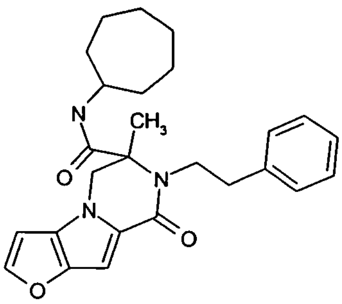 | 433,5553 | 434 | |
| 90 | 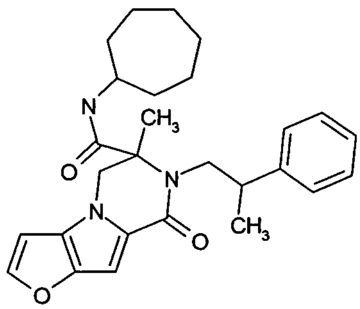 | 447,5824 | 448 | |
| 91 | 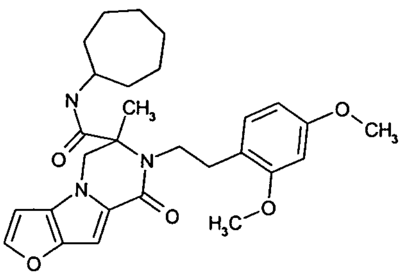 | 493,6083 | 494 | |

(continued)

| | Table 1.17(1) | | | |
|---|---|---|---|---|
| № | Formula | Mol.w. | m/z (M+1) | 1H NMR |
| 92 | 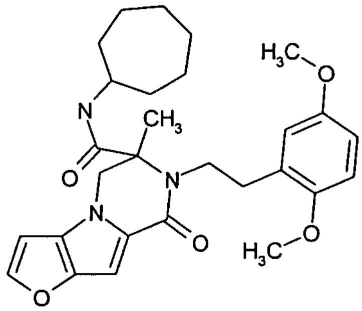 | 493,6083 | 494 | |
| 93 | 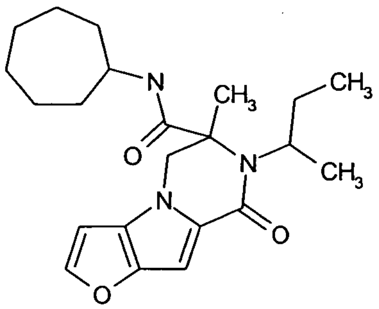 | 385,5107 | 386 | |
| 94 | 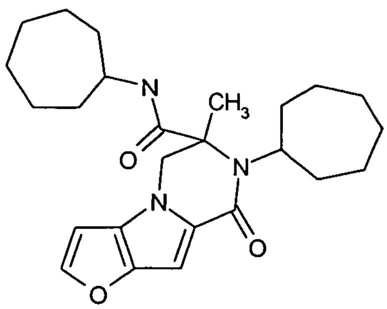 | 425,576 | 426 | |
| 95 | 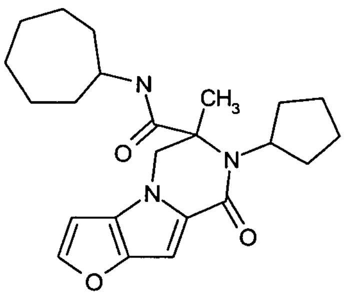 | 397,5218 | 398 | |

(continued)

| | Table 1.17(1) | | | |
|---|---|---|---|---|
| Nº | Formula | Mol.w. | m/z (M+1) | 1H NMR |
| 96 | 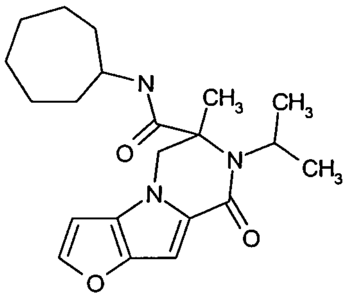 | 371,4836 | 371 | |
| 97 | 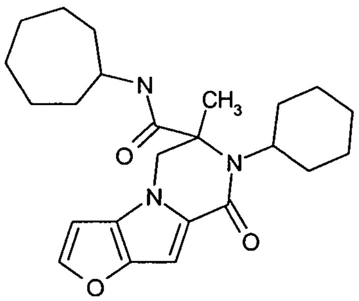 | 411,5489 | 412 | |
| 98 | 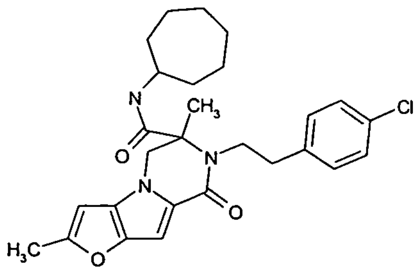 | 482,0274 | 483 | |
| 99 | 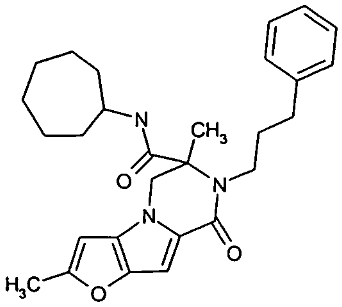 | 461,6095 | 462 | |
| 100 | 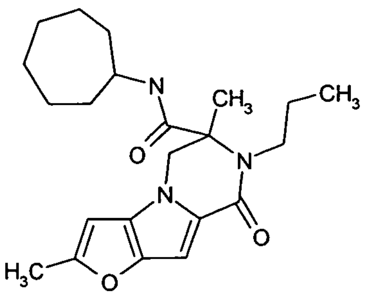 | 385,5107 | 386 | |

(continued)

| | Table 1.17(1) | | | |
|---|---|---|---|---|
| № | Formula | Mol.w. | m/z (M+1) | 1H NMR |
| 101 | 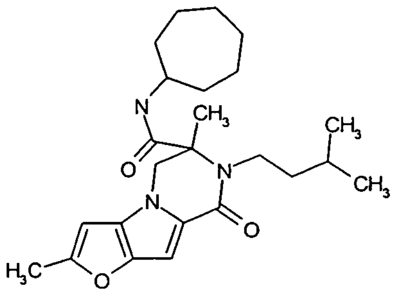 | 413,5649 | 414 | |
| 102 | 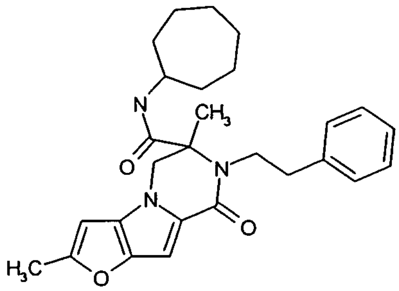 | 447,5824 | 448 | |
| 103 | 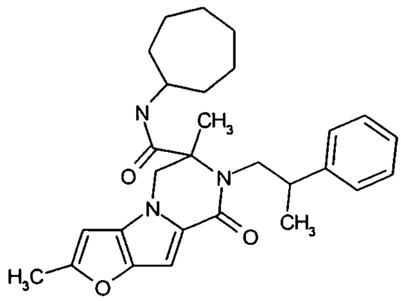 | 461,6095 | 462 | |
| 104 | 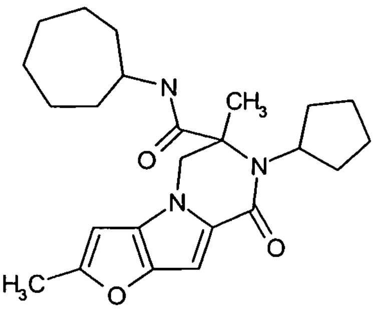 | 411,5489 | 412 | |
| 105 | 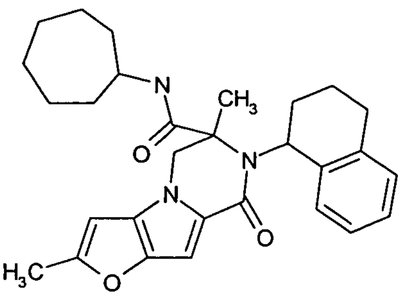 | 473,6206 | 474 | |

(continued)

| | Table 1.17(1) | | | |
|---|---|---|---|---|
| № | Formula | Mol.w. | m/z (M+1) | 1H NMR |
| 106 | 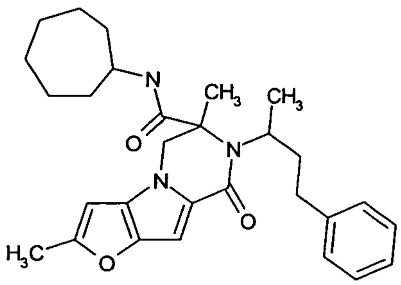 | 475,6365 | 476 | |
| 107 | 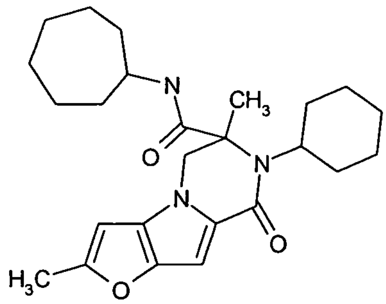 | 425,576 | 426 | |

| | Table 1.17(2) | | | |
|---|---|---|---|---|
| № | Formula | Mol.w. | m/z (M+1) | 1H NMR |
| 1 | 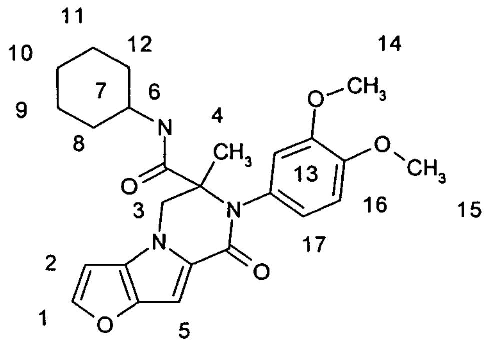 | 451,527 | 452 | 1.01 - 1.69 (8, 9, 10, 11, 12; 10H; m.), 1.29 (4; 3H, s.), 3.49 (7; 1H; b.m.), 3.76; 3.84 (14, 15; 6H, s., s.), 4.18 + 4.77 (3; 2H; AB; 13.6 Hz), 6.48 (2; 1 H; d.; 2.1 Hz), 6.54 (5; 1H; s.), 6.81 (16, 17; 2H, m.), 6.94 (13; 1H, s.), 7.29 (6; 1 H; b.d.,... |
| 2 | 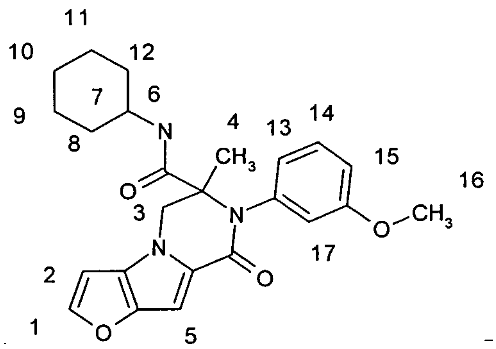 | 421,5005 | 422 | 1.00 - 1.74 (8, 9, 10, 11, 12; 10H; m.), 1.29 (4; 3H, s.), 3.49 (7; 1H; b.m.), 3.76 (16; 3H, s.), 4.21 + 4.77 (3; 2H; AB; 13.6 Hz), 6.52 (2; 1H; d.; 2.0 Hz), 6.58 (5; 1H; s.), 6.81 - 6.96 (13, 15, 17; 3H, m.), 7.22 (14; 1H, t.; 3.1 Hz), 7.36 (6; 1H; ... |

(continued)

| | Table 1.17(2) | | | |
|---|---|---|---|---|
| № | Formula | Mol.w. | m/z (M+1) | 1H NMR |
| 3 | 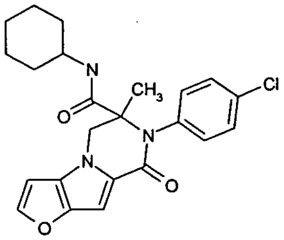 | 425,919 | 426 | |
| 4 | 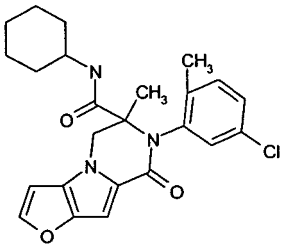 | 439,9461 | 440 | |
| 5 | 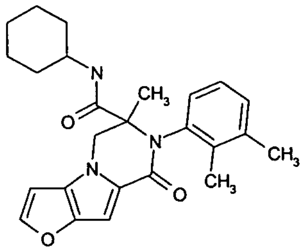 | 419,5282 | 420 | |
| 6 | 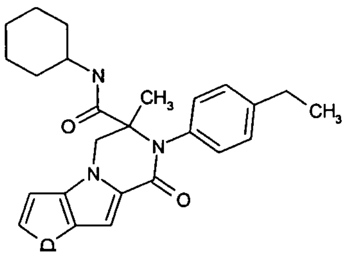 | 419,5282 | 420 | |
| 7 | 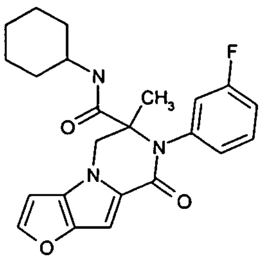 | 409,4644 | 410 | |
| 8 | 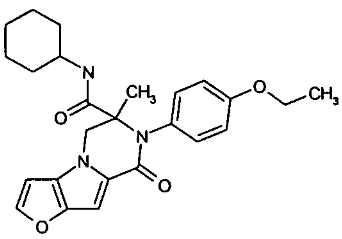 | 435,5276 | 436 | |

(continued)

| | Table 1.17(2) | | | |
|---|---|---|---|---|
| № | Formula | Mol.w. | m/z (M+1) | 1H NMR |
| 9 | 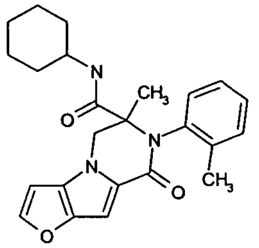 | 405,5011 | 406 | |
| 10 | 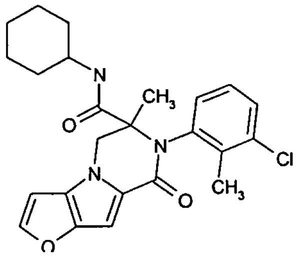 | 439,9461 | 440 | |
| 11 | 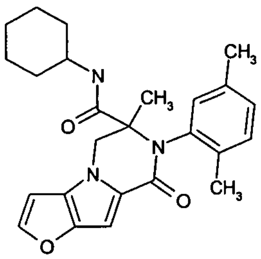 | 419,5282 | 420 | |
| 12 | 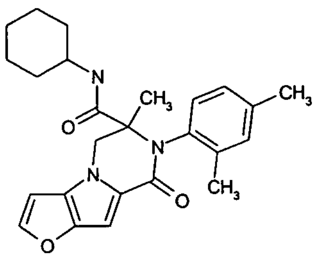 | 419,5282 | 420 | |
| 13 | 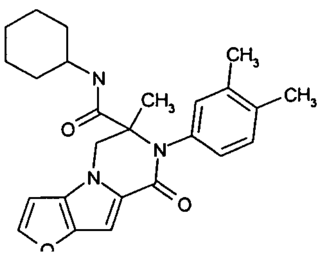 | 419,5282 | 420 | |
| 14 | 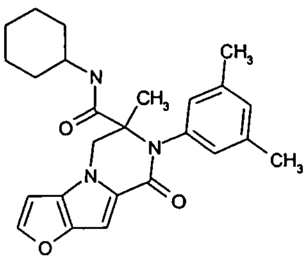 | 419,5282 | 420 | |

(continued)

| | Table 1.17(2) | | | |
|---|---|---|---|---|
| № | Formula | Mol.w. | m/z (M+1) | 1H NMR |
| 15 | 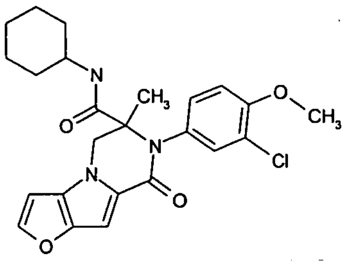 | 455,9455 | 456 | |
| 16 | 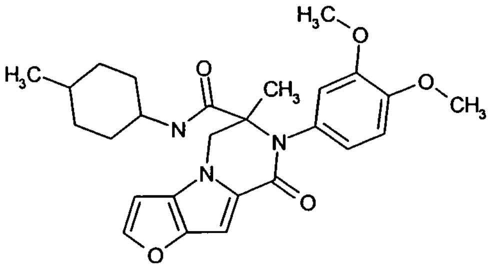 | 465,5541 | 466 | |
| 17 | 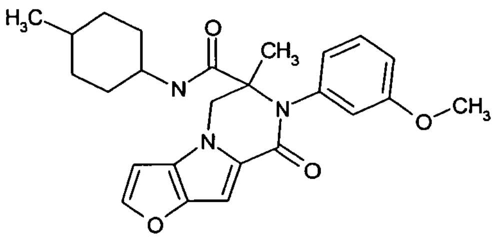 | 435,5276 | 436 | |
| 18 | 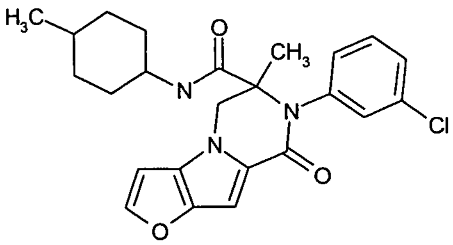 | 439,9461 | 440 | |
| 19 | 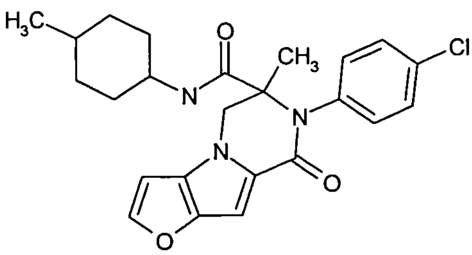 | 439,9461 | 440 | |
| 20 | 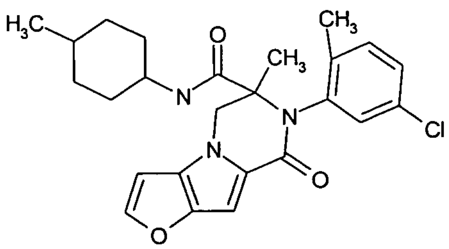 | 453,9732 | 454 | |

(continued)

| | Table 1.17(2) | | | |
|---|---|---|---|---|
| № | Formula | Mol.w. | m/z (M+1) | 1H NMR |
| 21 | 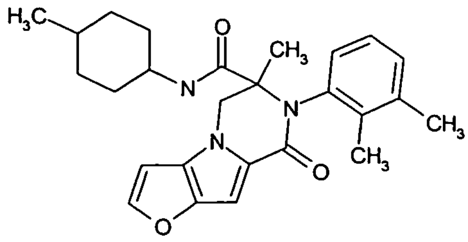 | 433,5553 | 434 | |
| 22 | 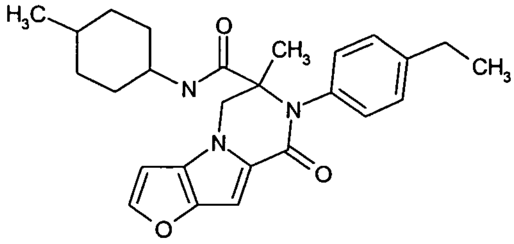 | 433,5553 | 434 | |
| 23 | 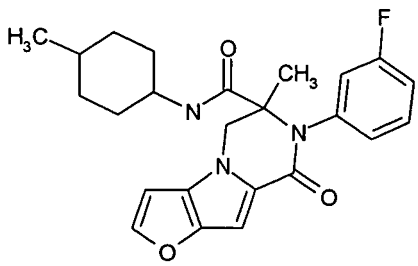 | 423,4915 | 424 | |
| 24 | 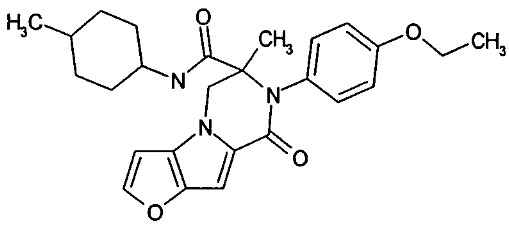 | 449,5547 | 450 | |
| 25 | 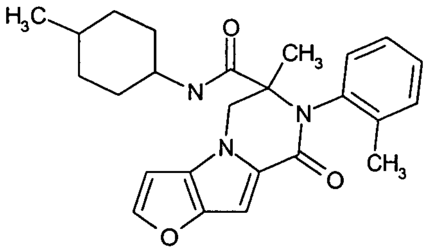 | 419,5282 | 420 | |
| 26 | 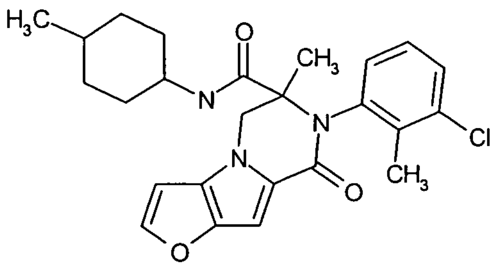 | 453,9732 | 454 | |

(continued)

| | Table 1.17(2) | | | |
|---|---|---|---|---|
| № | Formula | Mol.w. | m/z (M+1) | 1H NMR |
| 27 | 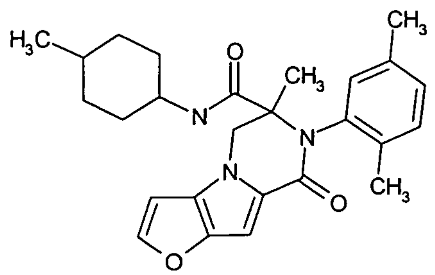 | 433,5553 | 434 | |
| 28 | 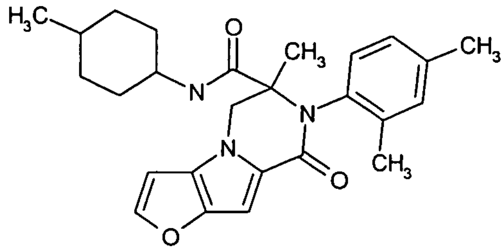 | 433,5553 | 434 | |
| 29 | 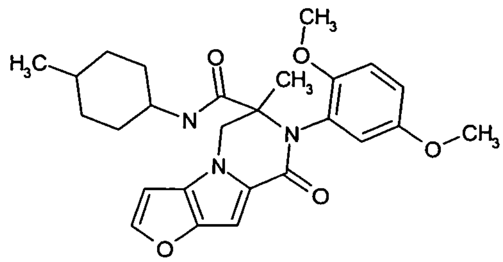 | 465,5541 | 466 | |
| 30 | 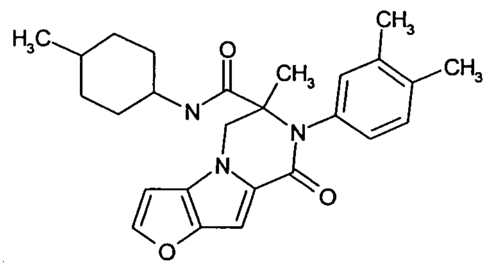 | 433,5553 | 434 | |
| 31 | 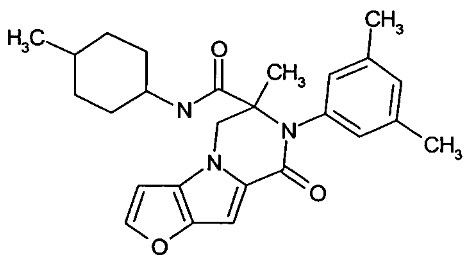 | 433,5553 | 434 | |

(continued)

| | Table 1.17(2) | | | |
|---|---|---|---|---|
| Nº | Formula | Mol.w. | m/z (M+1) | 1H NMR |
| 32 | 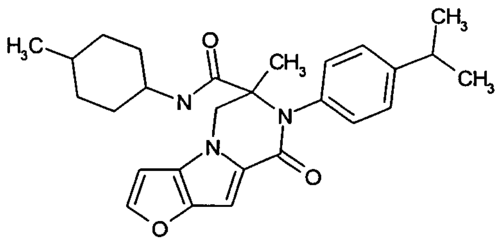 | 447,5824 | 448 | |
| 33 | 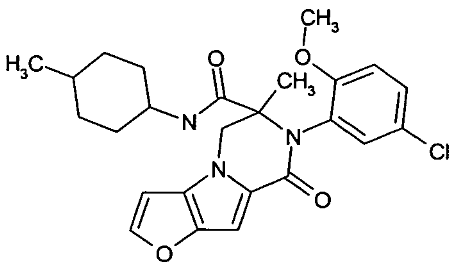 | 469,9726 | 470 | |
| 34 | 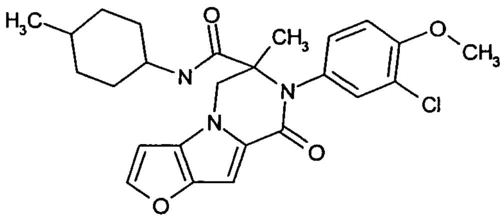 | 469,9726 | 470 | |
| 35 | 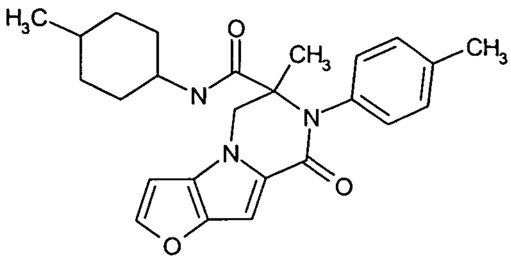 | 419,5282 | 420 | |
| 36 | 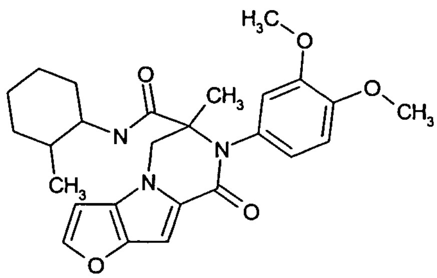 | 465,5541 | 466 | |
| 37 | 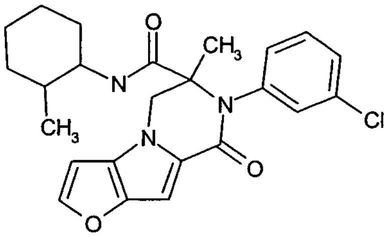 | 439,9461 | 440 | |

(continued)

| | Table 1.17(2) | | | |
|---|---|---|---|---|
| № | Formula | Mol.w. | m/z (M+1) | 1H NMR |
| 38 | 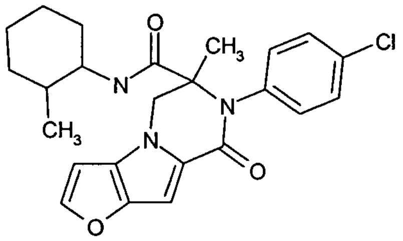 | 439,9461 | 440 | |
| 39 | 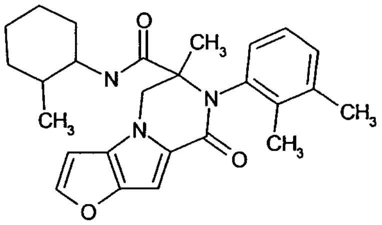 | 433,5553 | 434 | |
| 40 | 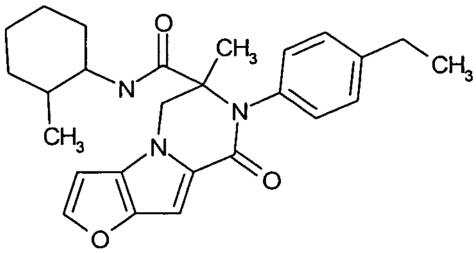 | 433,5553 | 434 | |
| 41 | 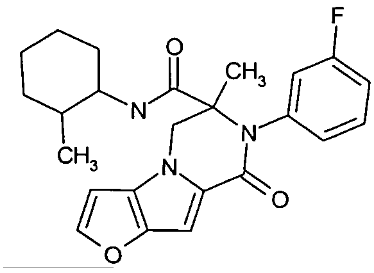 | 423,4915 | 424 | |
| 42 | 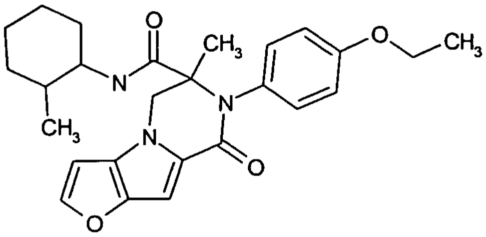 | 449,5547 | 450 | |
| 43 | 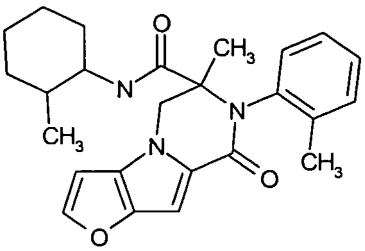 | 419,5282 | 420 | |

(continued)

| | Table 1.17(2) | | | |
|---|---|---|---|---|
| № | Formula | Mol.w. | m/z (M+1) | 1H NMR |
| 44 | 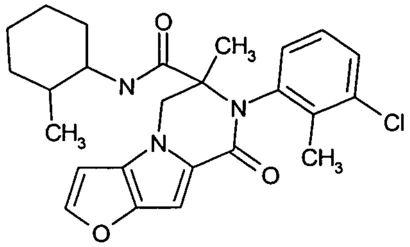 | 453,9732 | 454 | |
| 45 | 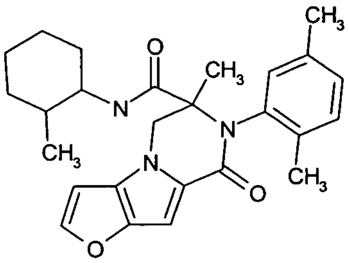 | 433,5553 | 434 | |
| 46 | 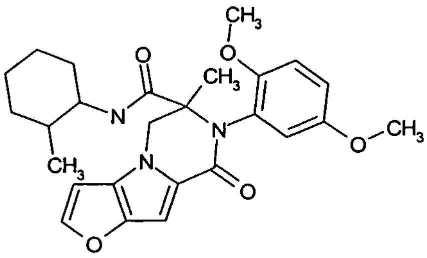 | 465,5541 | 466 | |
| 47 | 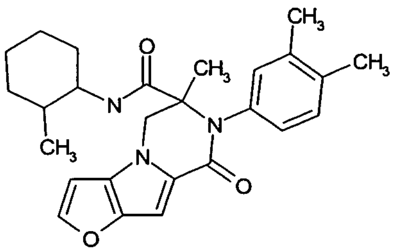 | 433,5553 | 434 | |
| 48 | 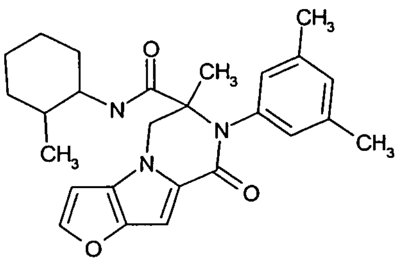 | 433,5553 | 434 | |

(continued)

| | Table 1.17(2) | | | |
|---|---|---|---|---|
| № | Formula | Mol.w. | m/z (M+1) | 1H NMR |
| 49 | 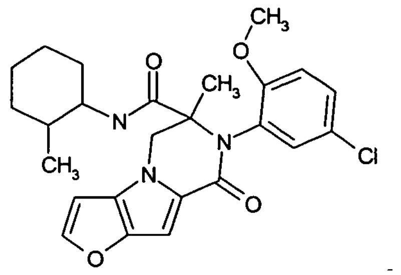 | 469,9726 | 470 | |
| 50 | 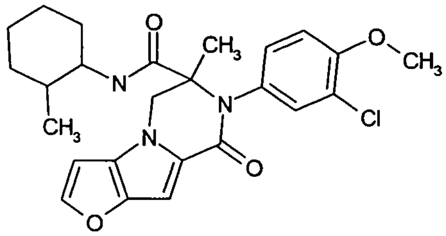 | 469,9726 | 470 | |
| 51 | 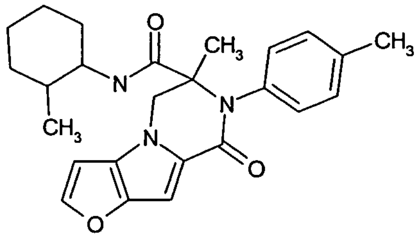 | 419,5282 | 420 | |
| 52 | 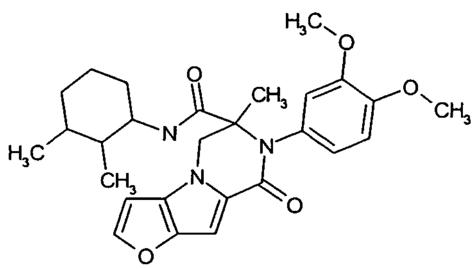 | 479,5812 | 480 | |
| 53 | 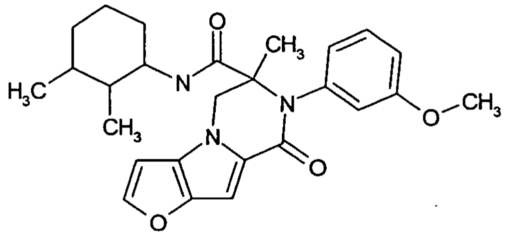 | 449,5547 | 450 | |
| 54 | 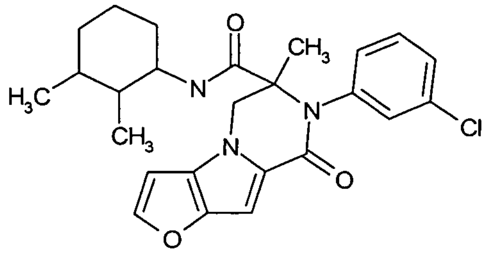 | 453,9732 | 454 | |

(continued)

| | Table 1.17(2) | | | |
|---|---|---|---|---|
| № | Formula | Mol.w. | m/z (M+1) | 1H NMR |
| 55 | 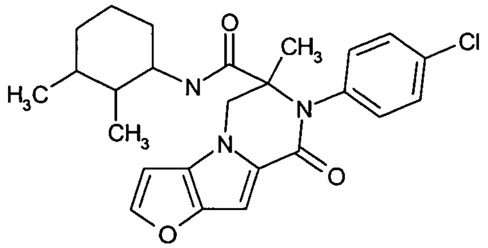 | 453,9732 | 454 | |
| 56 | 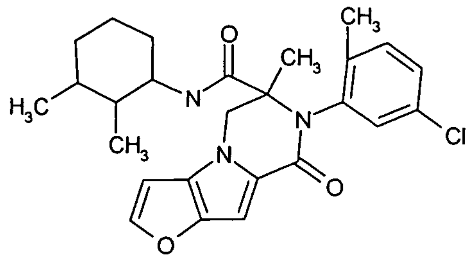 | 468,0003 | 469 | |
| 57 | 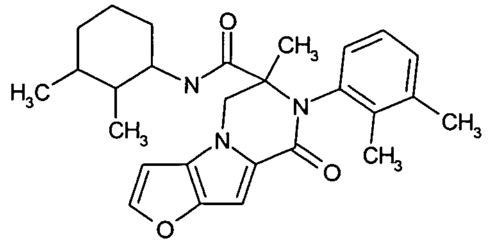 | 447,5824 | 448 | |
| 58 | 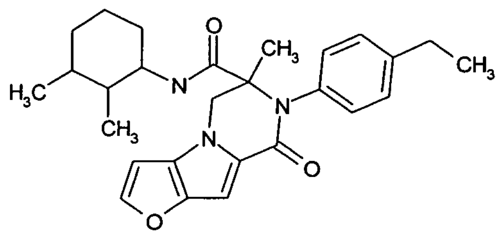 | 447,5824 | 448 | |
| 59 | 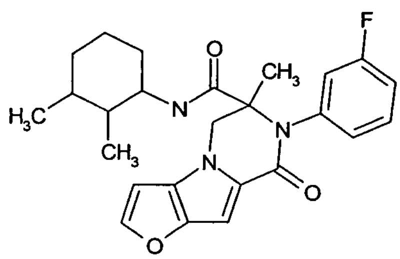 | 437,5186 | 438 | |
| 60 | 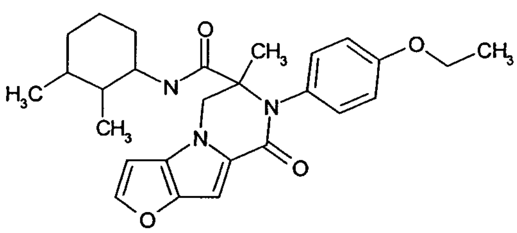 | 463,5818 | 464 | |

(continued)

| | Table 1.17(2) | | | |
|---|---|---|---|---|
| № | Formula | Mol.w. | m/z (M+1) | 1H NMR |
| 61 | 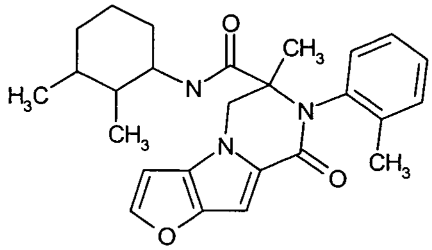 | 433,5553 | 434 | |
| 62 | 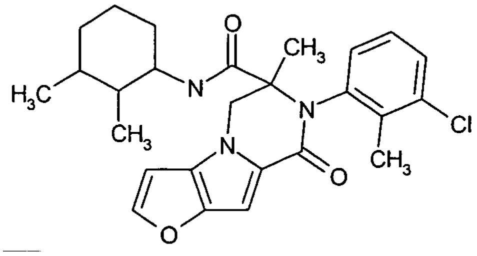 | 468,0003 | 469 | |
| 63 | 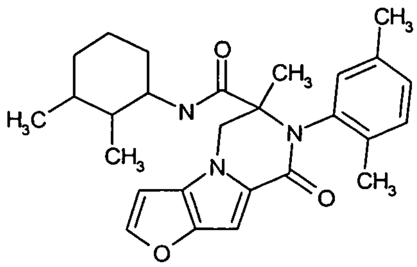 | 447,5824 | 448 | |
| 64 | 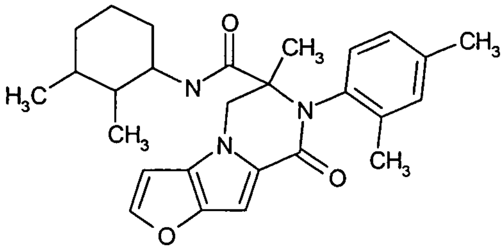 | 447,5824 | 448 | |
| 65 | 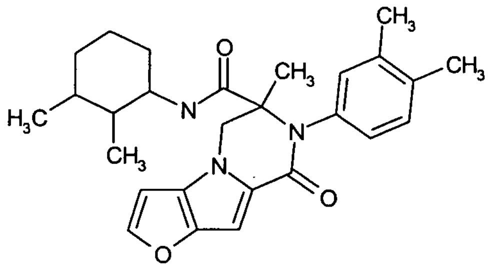 | 447,5824 | 448 | |

(continued)

| | Table 1.17(2) | | | |
|---|---|---|---|---|
| № | Formula | Mol.w. | m/z (M+1) | 1H NMR |
| 66 | 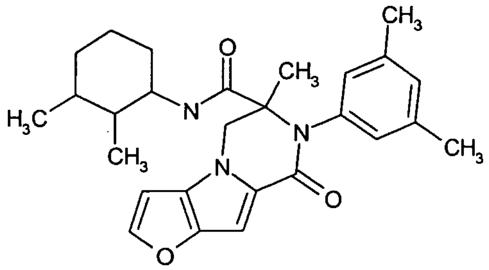 | 447,5824 | 448 | |
| 67 | 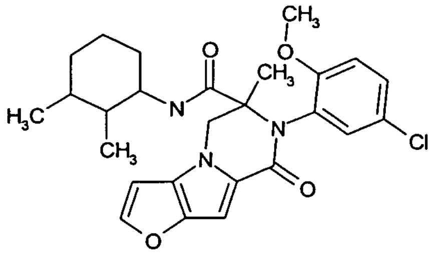 | 483,9997 | 484 | |
| 68 | 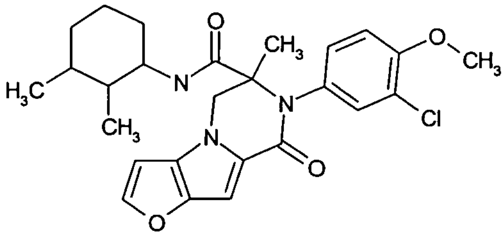 | 483,9997 | 484 | |
| 69 | 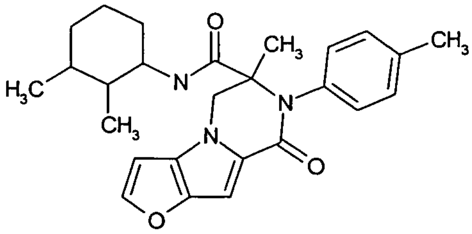 | 433,5553 | 434 | |
| 70 | 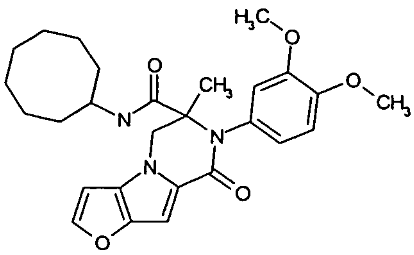 | 479,5812 | 480 | |
| 71 | 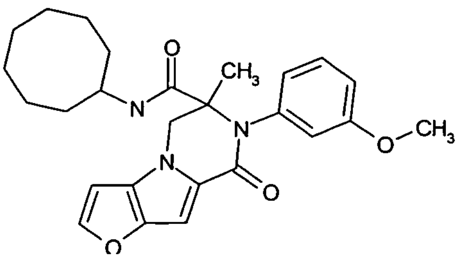 | 449,5547 | 450 | |

(continued)

| | Table 1.17(2) | | | |
|---|---|---|---|---|
| № | Formula | Mol.w. | m/z (M+1) | 1H NMR |
| 72 | 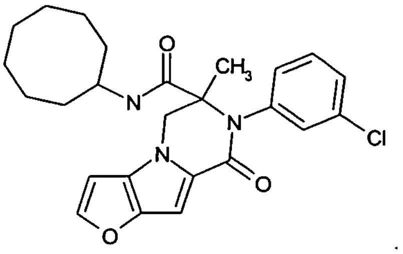 | 453,9732 | 454 | |
| 73 | 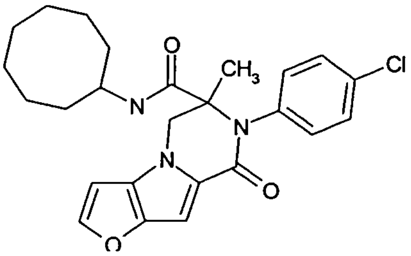 | 453,9732 | 454 | |
| 74 | 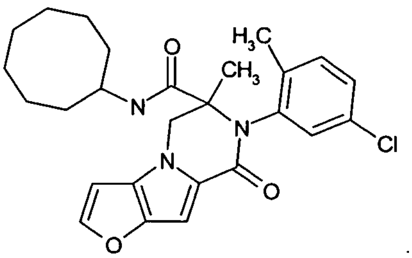 | 468,0003 | 469 | |
| 75 | 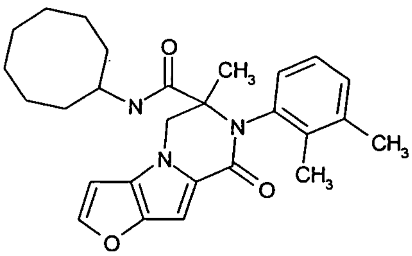 | 447,5824 | 448 | |
| 76 | 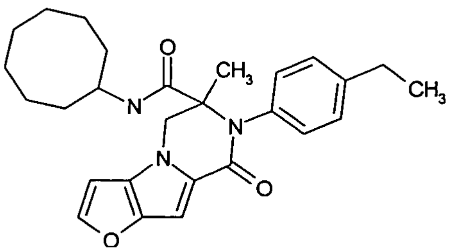 | 447,5824 | 448 | |

(continued)

| | Table 1.17(2) | | | |
|---|---|---|---|---|
| № | Formula | Mol.w. | m/z (M+1) | 1H NMR |
| 77 | 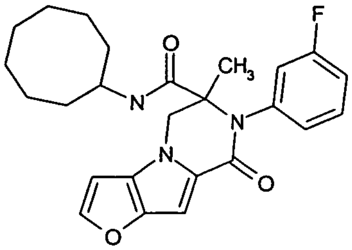 | 437,5186 | 438 | |
| 78 | 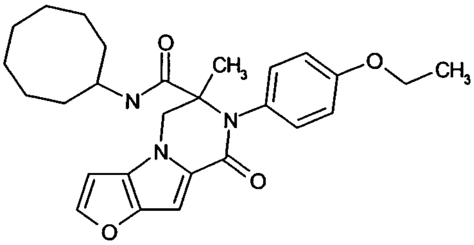 | 463,5818 | 464 | |
| 79 | 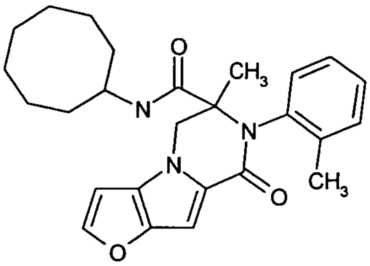 | 433,5553 | 434 | |
| 80 | 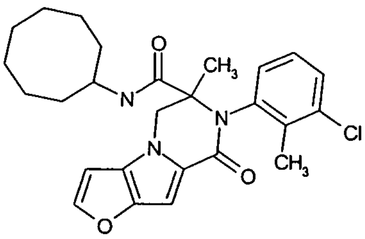 | 468,0003 | 469 | |
| 81 | 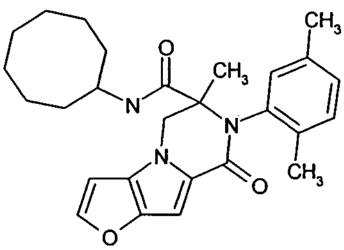 | 447,5824 | 448 | |
| 82 | 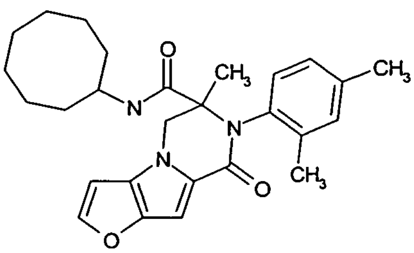 | 447,5824 | 448 | |

(continued)

| | Table 1.17(2) | | | |
|---|---|---|---|---|
| № | Formula | Mol.w. | m/z (M+1) | 1H NMR |
| 83 | 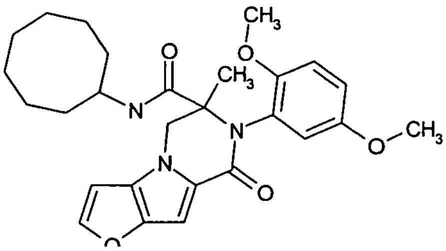 | 479,5812 | 480 | |
| 84 | 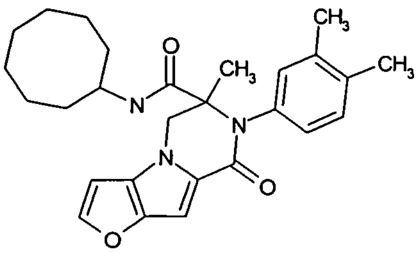 | 447,5824 | 448 | |
| 85 | 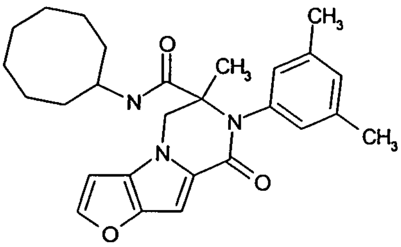 | 447,5824 | 448 | |
| 86 | 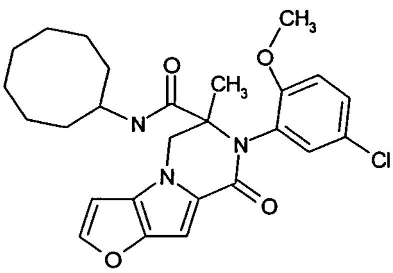 | 483,9997 | 484 | |
| 87 | 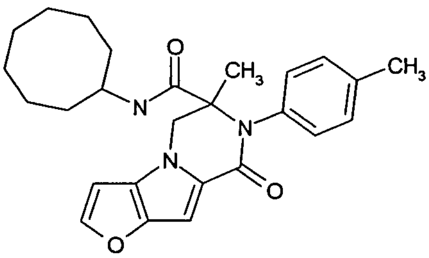 | 433,5553 | 434 | |

(continued)

| | Table 1.17(2) | | | |
|---|---|---|---|---|
| № | Formula | Mol.w. | m/z (M+1) | 1H NMR |
| 88 | 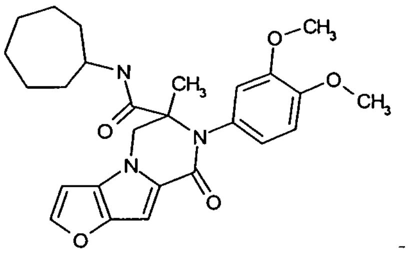 | 465,5541 | 466 | |
| 89 | 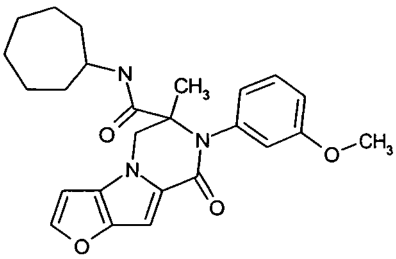 | 435,5276 | 436 | |
| 90 | 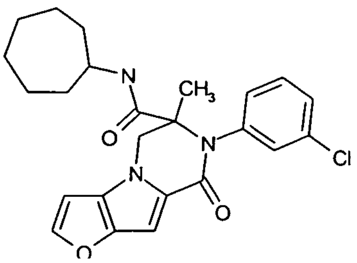 | 439,9461 | 440 | |
| 91 | 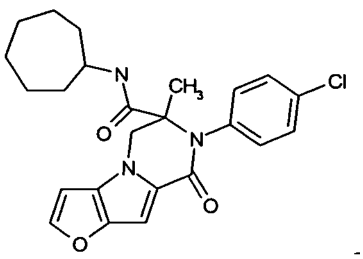 | 439,9461 | 440 | |
| 92 | 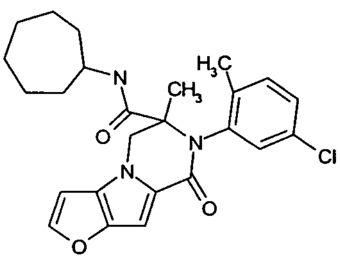 | 453,9732 | 454 | |

(continued)

| | Table 1.17(2) | | | |
|---|---|---|---|---|
| № | Formula | Mol.w. | m/z (M+1) | 1H NMR |
| 93 | 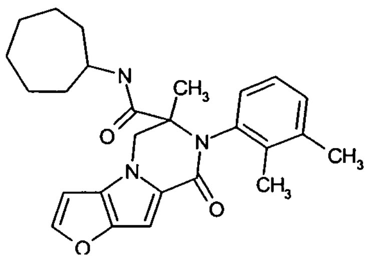 | 433,5553 | 434 | |
| 94 | 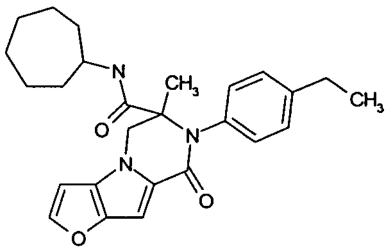 | 433,5553 | 434 | |
| 95 | 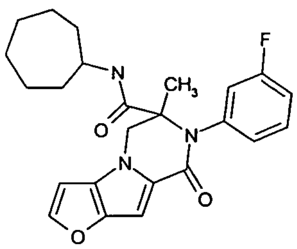 | 423,4915 | 424 | |
| 96 | 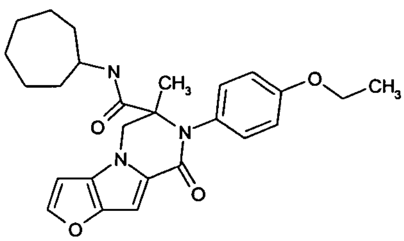 | 449,5547 | 450 | |
| 97 | 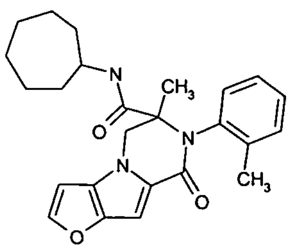 | 419,5282 | 420 | |

(continued)

| | Table 1.17(2) | | | |
|---|---|---|---|---|
| № | Formula | Mol.w. | m/z (M+1) | 1H NMR |
| 98 | 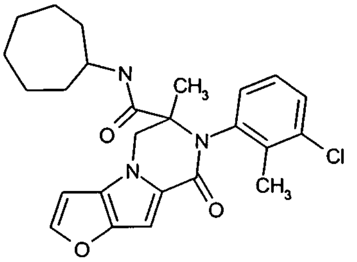 | 453,9732 | 454 | |
| 99 | 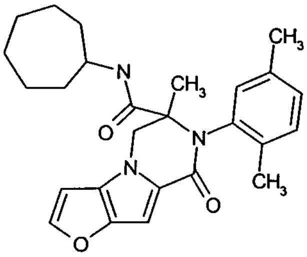 | 433,5553 | 434 | |
| 100 | 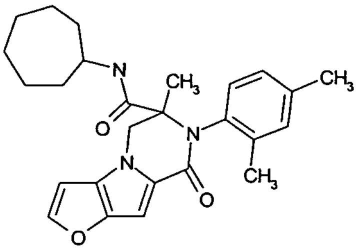 | 433,5553 | 434 | |
| 101 | 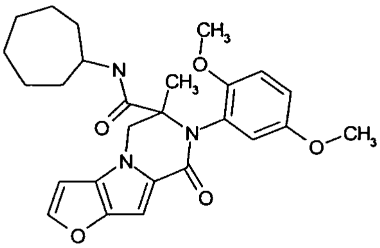 | 465,5541 | 466 | |
| 102 | 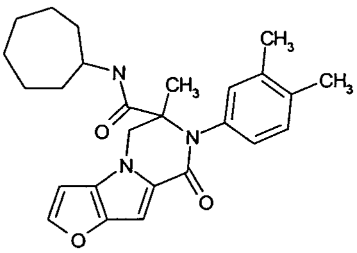 | 433,5553 | 434 | |

(continued)

| | Table 1.17(2) | | | |
|---|---|---|---|---|
| № | Formula | Mol.w. | m/z (M+1) | 1H NMR |
| 103 | 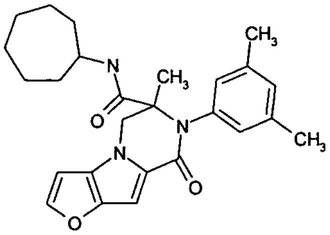 | 433,5553 | 434 | |
| 104 | 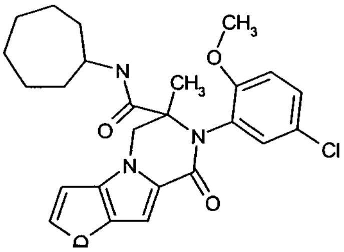 | 469,9726 | 470 | |
| 105 | 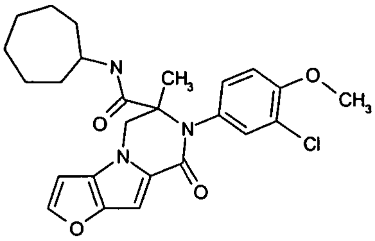 | 469,9726 | 470 | |
| 106 | 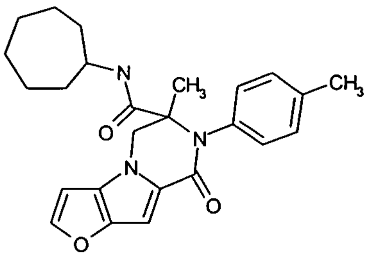 | 419,5282 | 420 | |
| 107 | 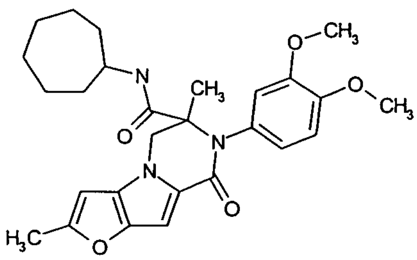 | 479,5812 | 480 | |
| 108 | 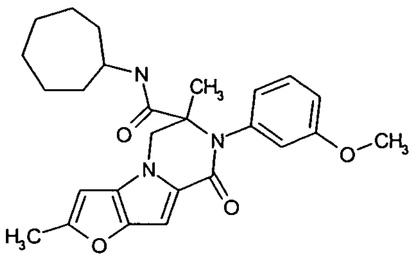 | 449,5547 | 450 | |

(continued)

| | Table 1.17(2) | | | |
|---|---|---|---|---|
| № | Formula | Mol.w. | m/z (M+1) | 1H NMR |
| 109 | 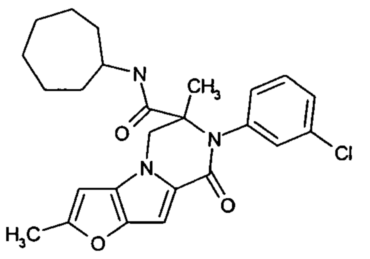 | 453,9732 | 454 | |
| 110 | 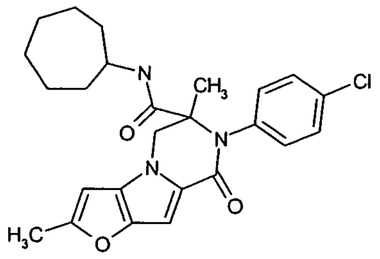 | 453,9732 | 454 | |
| 111 | 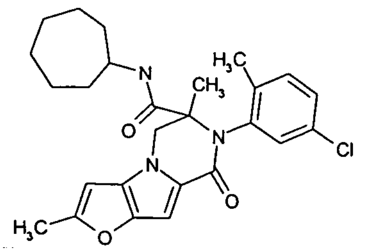 | 468,0003 | 469 | |
| 112 | 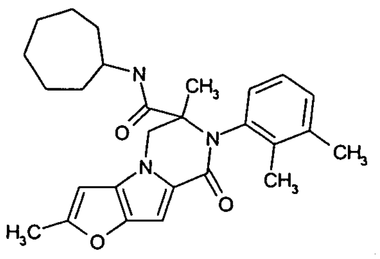 | 447,5824 | 448 | |
| 113 | 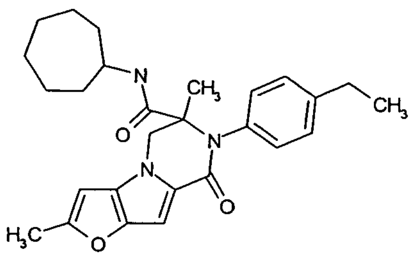 | 447,5824 | 448 | |
| 114 | 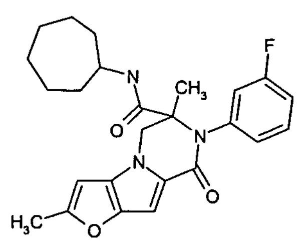 | 437,5186 | 438 | |

(continued)

| | Table 1.17(2) | | | |
|---|---|---|---|---|
| № | Formula | Mol.w. | m/z (M+1) | 1H NMR |
| 115 | 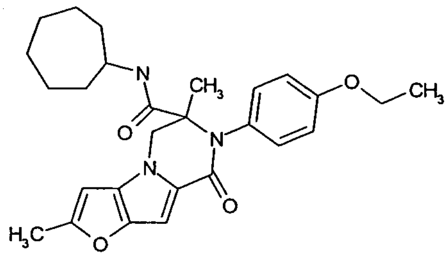 | 463,5818 | 464 | |
| 116 | 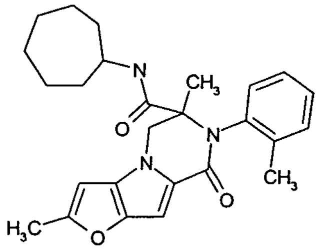 | 433,5553 | 434 | |
| 117 | 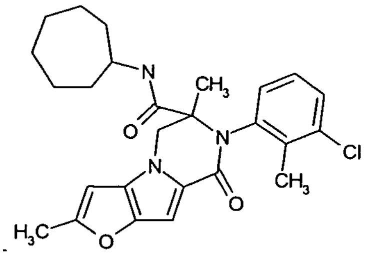 | 468,0003 | 469 | |
| 118 | 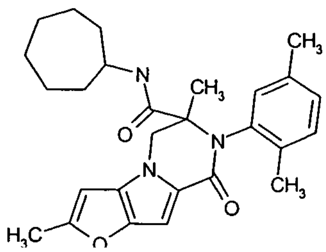 | 447,5824 | 448 | |
| 119 | 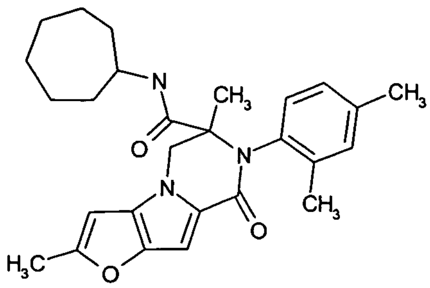 | 447,5824 | 448 | |

(continued)

| | Table 1.17(2) | | | |
|---|---|---|---|---|
| № | Formula | Mol.w. | m/z (M+1) | 1H NMR |
| 120 | 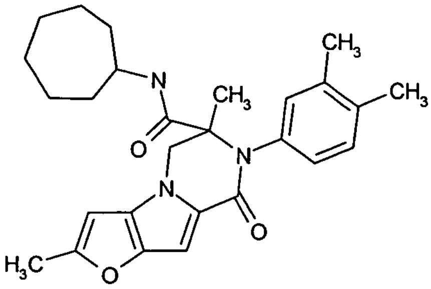 | 447,5824 | 448 | |
| 121 | 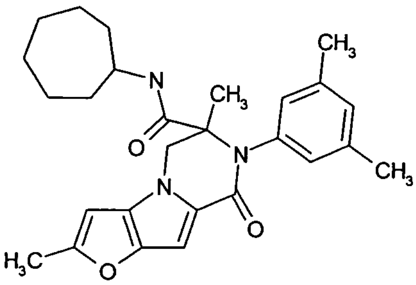 | 447,5824 | 448 | |
| 122 | 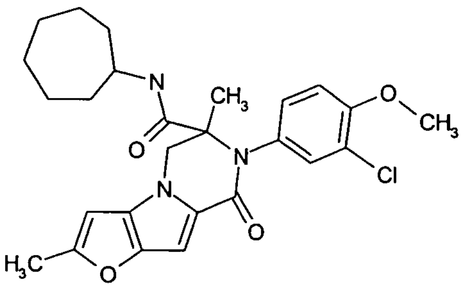 | 483,9997 | 484 | |
| 123 | 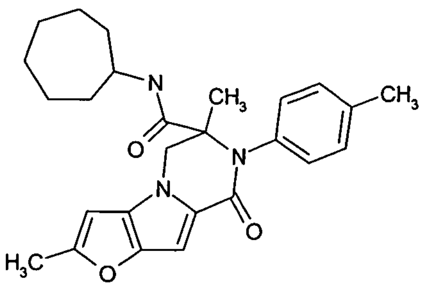 | 433,5553 | 434 | |

Table 1.17(3).

| № | Formula | Mol.w. | m/z (M+1) | 1H NMR |
|---|---|---|---|---|
| 1 | 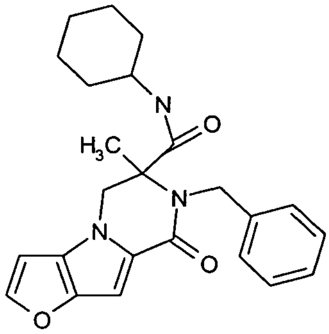 | 405,50109 | 406 | |
| 2 | 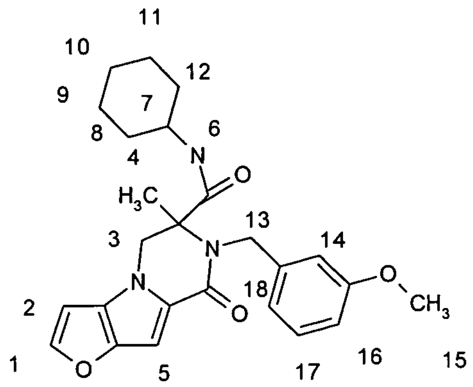 | 435,52758 | 436 | 0.92 - 1.74 (8, 9, 10, 11, 12; 10H; m.), 1.53 (4; 3H, s.), 3.47 (7; 1 H; b.m.), 3.77 (16; 3H; s.), 4.01 + 4.78 (3; 2H; AB; 12.4 Hz), 4.23 + 5.23 (13; 2H; AB; 16.1 Hz), 6.56 (5; 1H; s.), 6.61 (2; 1 H; d.; 1.1 Hz), 6.72 (16; 1 H; d.; 3.4 Hz), 6.88 (14, 1... |
| 3 | 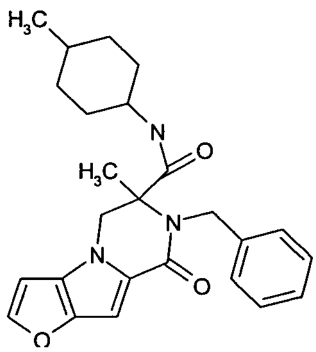 | 419,52818 | 420 | |
| 4 | 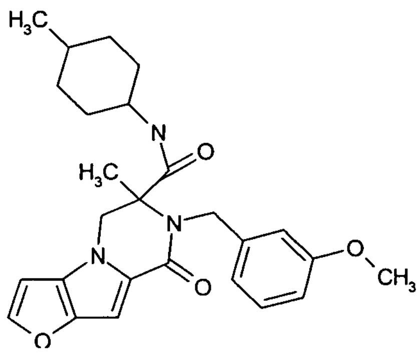 | 449,55467 | 450 | |

(continued)

| № | Formula | Mol.w. | m/z (M+1) | 1H NMR |
|---|---|---|---|---|
| 5 | 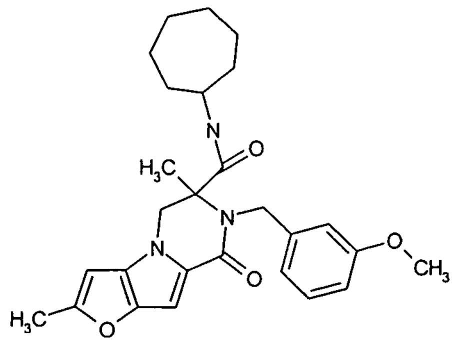 | 463,58176 | 464 | |
| 6 | 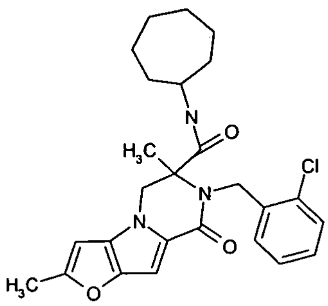 | 468,0003 | 469 | |
| 7 | 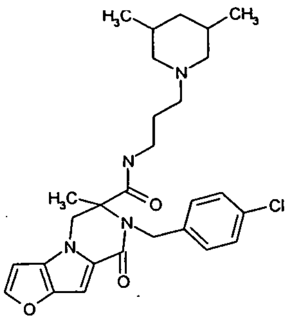 | 511,06915 | 512 | |
| 8 | 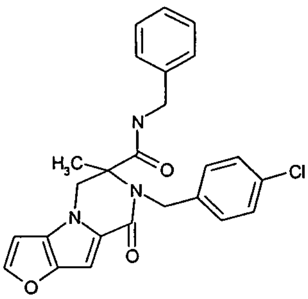 | 447,92539 | 448 | |
| 9 | 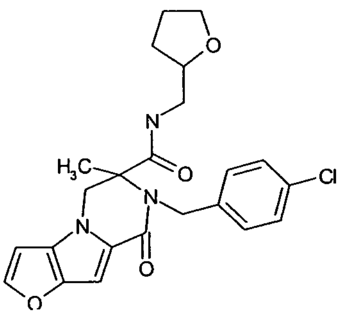 | 441,91843 | 442 | |

(continued)

| № | Formula | Mol.w. | m/z (M+1) | 1H NMR |
|---|---|---|---|---|
| 10 | 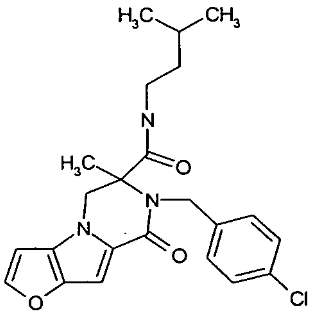 | 427,93497 | 428 | |
| 11 | 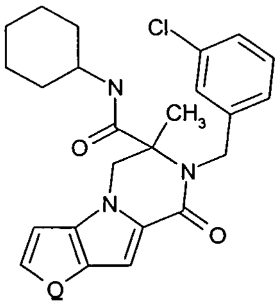 | 439,94612 | 440 | |
| 12 | 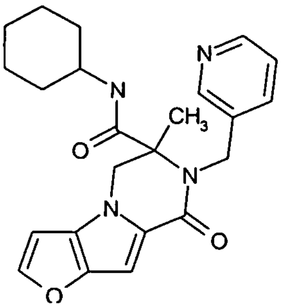 | 406,48867 | 407 | |
| 13 | 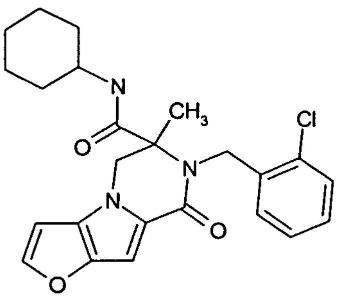 | 439,94612 | 440 | |
| 14 | 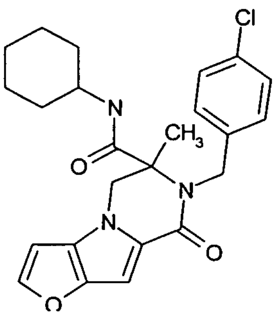 | 439,94612 | 440 | |

(continued)

| № | Formula | Mol.w. | m/z (M+1) | 1H NMR |
|---|---|---|---|---|
| 15 | 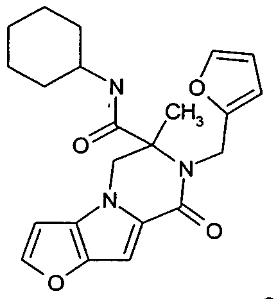 | 395,46225 | 396 | |
| 16 | 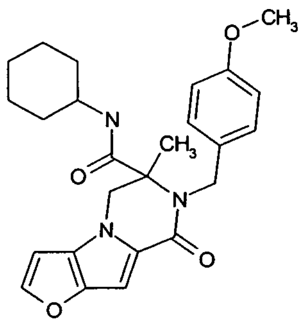 | 435,52758 | 436 | |
| 17 | 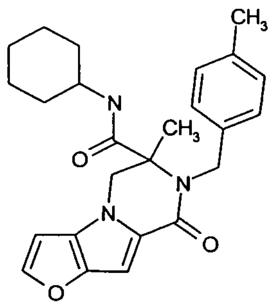 | 419,52818 | 420 | |
| 18 | 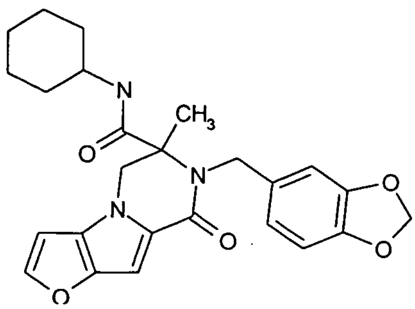 | 449,51104 | 450 | |
| 19 | 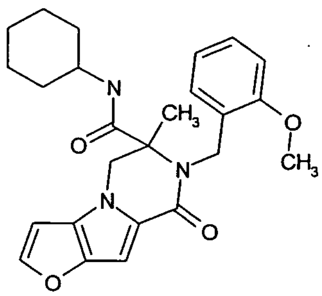 | 435,52758 | 436 | |

(continued)

| № | Formula | Mol.w. | m/z (M+1) | 1H NMR |
|---|---|---|---|---|
| 20 | 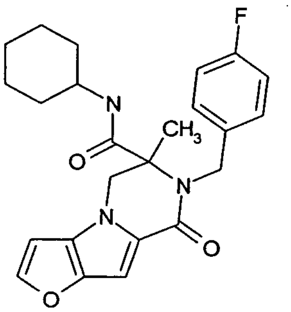 | 423,49152 | 424 | |
| 21 | 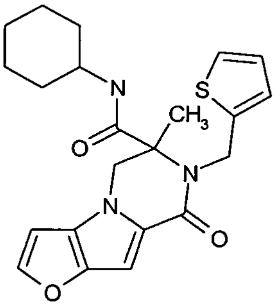 | 411,52685 | 412 | |
| 22 | 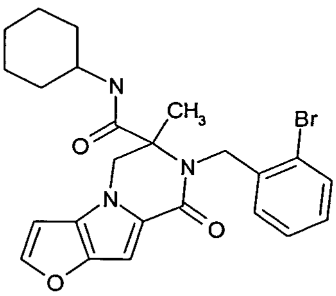 | 484,39712 | 485 | |
| 23 | 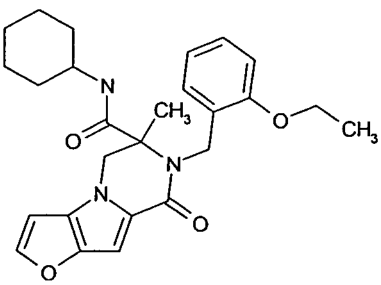 | 449,55467 | 450 | |
| 24 | 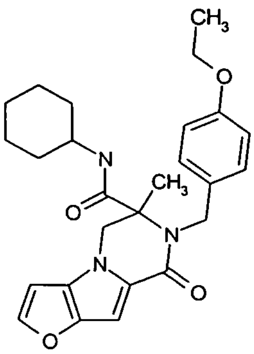 | 449,55467 | 450 | |

(continued)

| № | Formula | Mol.w. | m/z (M+1) | 1H NMR |
|---|---|---|---|---|
| 25 | 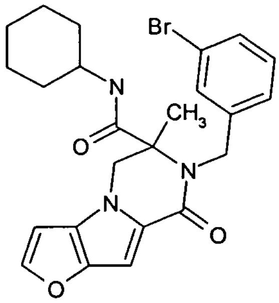 | 484,39712 | 485 | |
| 26 | 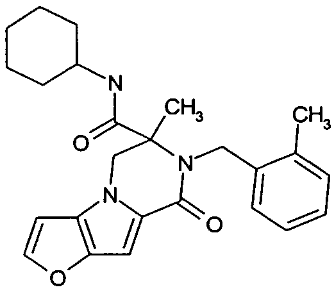 | 419,52818 | 420 | |
| 27 | 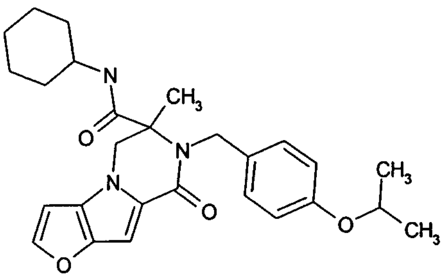 | 463,58176 | 464 | |
| 28 | 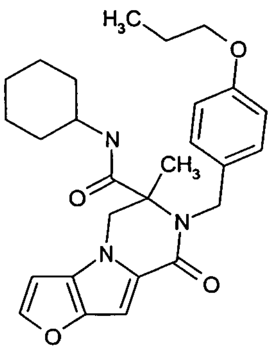 | 463,58176 | 464 | |

(continued)

| № | Formula | Mol.w. | m/z (M+1) | 1H NMR |
|---|---|---|---|---|
| 29 | 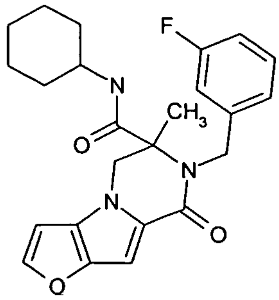 | 423,49152 | 424 | |
| 30 | 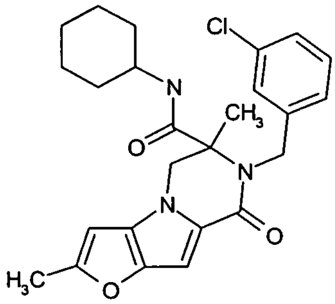 | 453,97321 | 454 | |
| 31 | 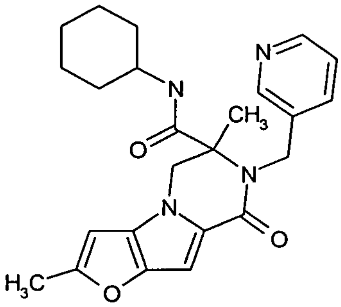 | 420,51576 | 421 | |
| 32 | 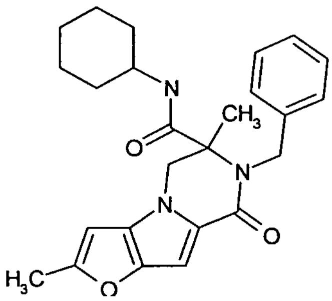 | 419,52818 | 420 | |
| 33 | 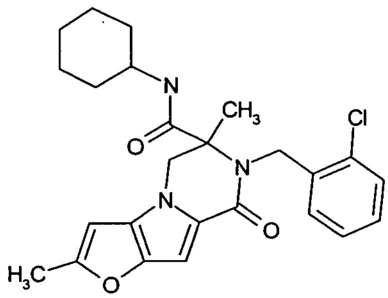 | 453,97321 | 454 | |

(continued)

| № | Formula | Mol.w. | m/z (M+1) | 1H NMR |
|---|---|---|---|---|
| 34 | 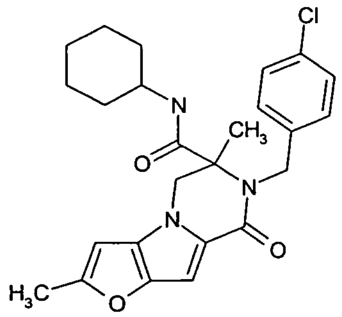 | 453,97321 | 454 | |
| 35 | 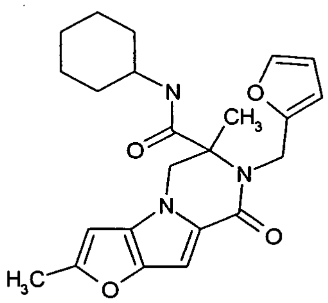 | 409,48934 | 410 | |
| 36 | 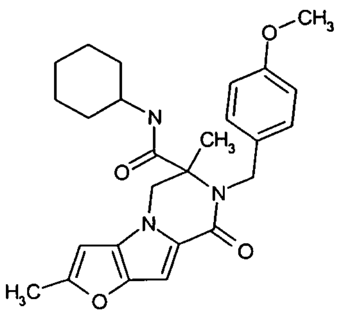 | 449,55467 | 450 | |
| 37 | 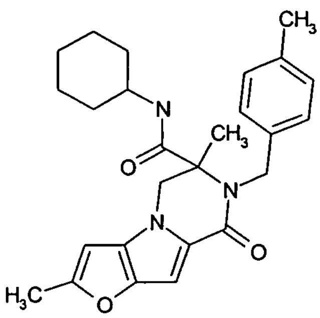 | 433,55527 | 434 | |
| 38 | 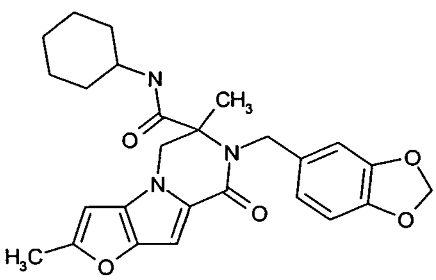 | 463,53813 | 464 | |

(continued)

| № | Formula | Mol.w. | m/z (M+1) | 1H NMR |
|---|---|---|---|---|
| 39 | 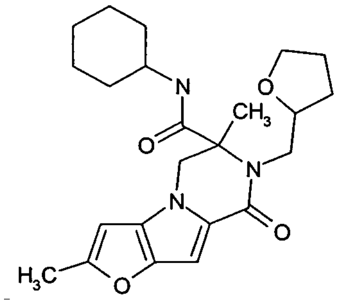 | 413,52122 | 414 | |
| 40 | 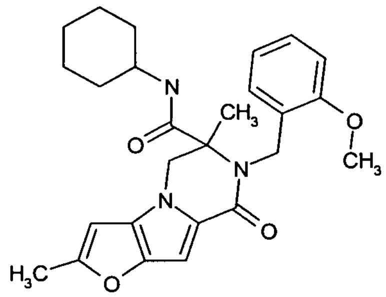 | 449,55467 | 450 | |
| 41 | 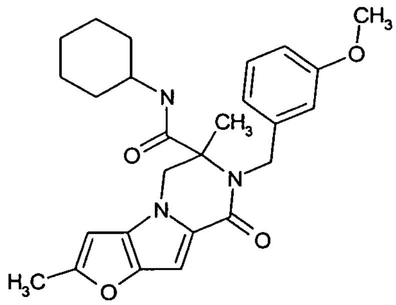 | 449,55467 | 450 | |
| 42 | 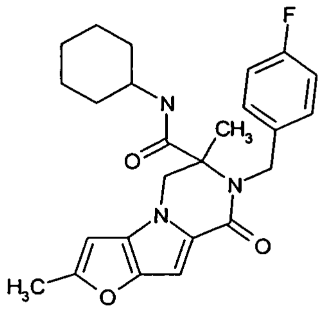 | 437,51861 | 438 | |
| 43 | 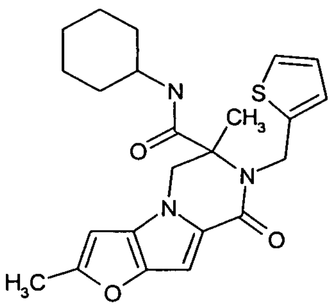 | 425,55394 | 426 | |

(continued)

| № | Formula | Mol.w. | m/z (M+1) | 1H NMR |
|---|---|---|---|---|
| 44 | 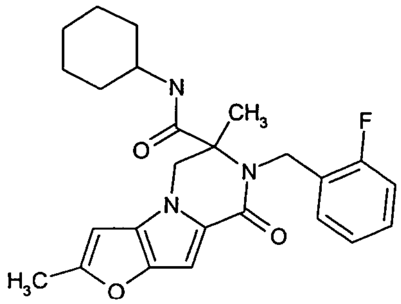 | 437,51861 | 438 | |
| 45 | 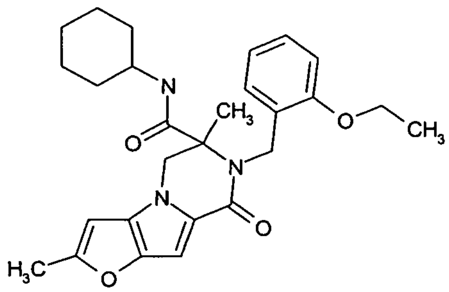 | 463,58176 | 464 | |
| 46 | 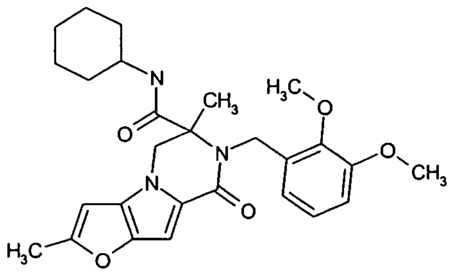 | 479,58116 | 480 | |
| 47 | 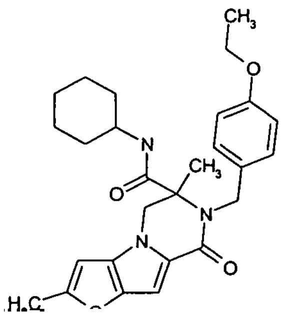 | 463,58176 | 464 | |
| 48 | 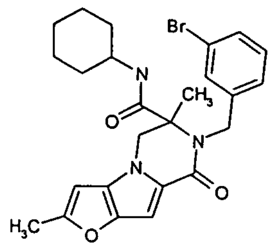 | 498,42421 | 499 | |

(continued)

| № | Formula | Mol.w. | m/z (M+1) | 1H NMR |
|---|---|---|---|---|
| 49 | 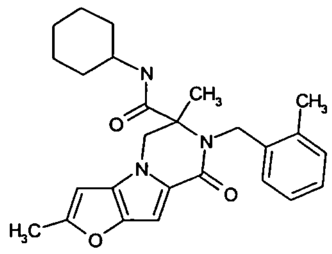 | 433,55527 | 434 | |
| 50 | 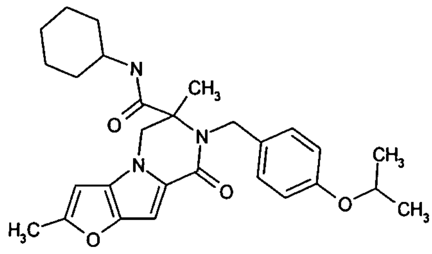 | 477,60885 | 478 | |
| 51 | 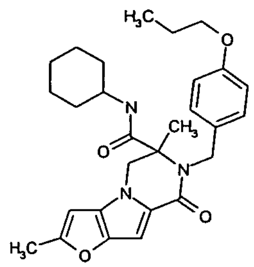 | 477,60885 | 478 | |
| 52 | 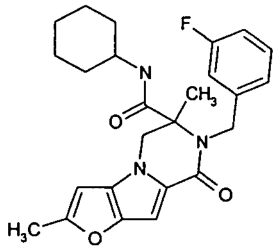 | 437,51861 | 438 | |
| 53 | 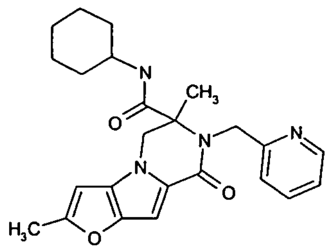 | 420,51576 | 421 | |
| 54 | 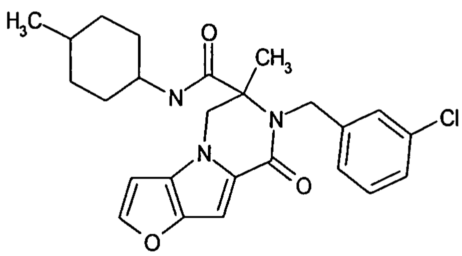 | 453,97321 | 454 | |

(continued)

| № | Formula | Mol.w. | m/z (M+1) | 1H NMR |
|---|---|---|---|---|
| 55 | 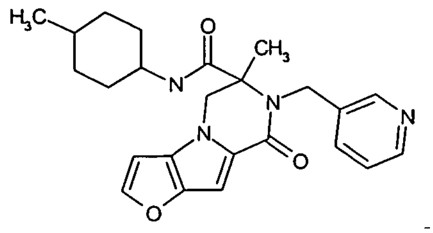 | 420,51576 | 421 | |
| 56 | 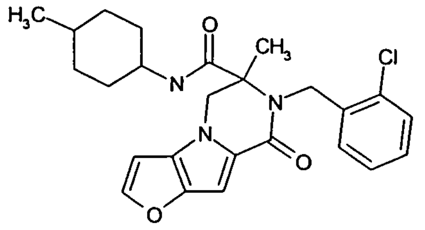 | 453,97321 | 454 | |
| 57 | 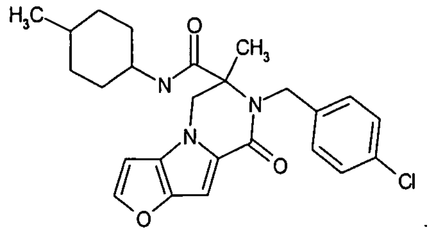 | 453,97321 | 454 | |
| 58 | 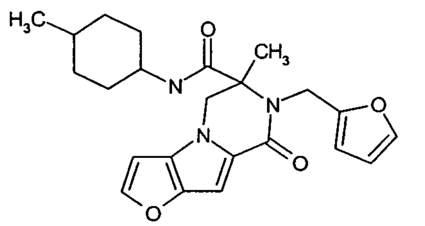 | 409,48934 | 410 | |
| 59 | 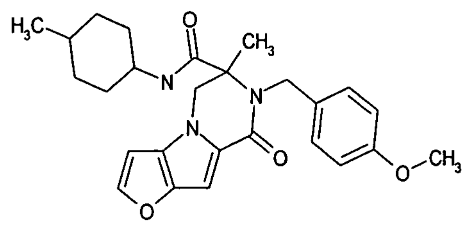 | 449,55467 | 450 | |
| 60 | 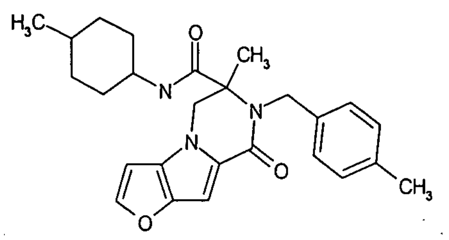 | 433,55527 | 434 | |

(continued)

| № | Formula | Mol.w. | m/z (M+1) | 1H NMR |
|---|---|---|---|---|
| 61 | 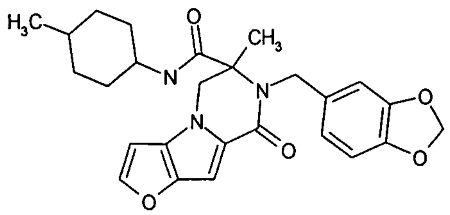 | 463,53813 | 464 | |
| 62 | 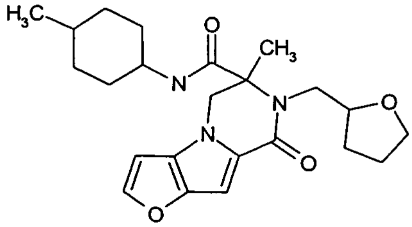 | 413,52122 | 414 | |
| 63 | 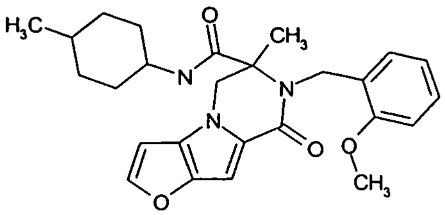 | 449,55467 | 450 | |
| 64 | 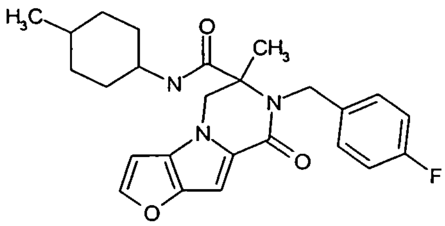 | 437,51861 | 438 | |
| 65 | 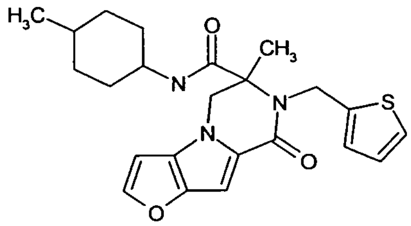 | 425,55394 | 426 | |
| 66 | 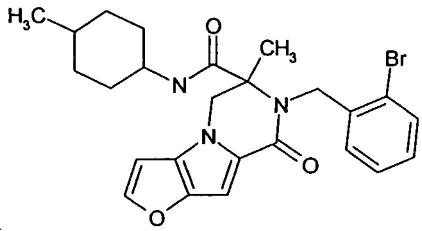 | 498,42421 | 499 | |

(continued)

| № | Formula | Mol.w. | m/z (M+1) | 1H NMR |
|---|---|---|---|---|
| 67 | 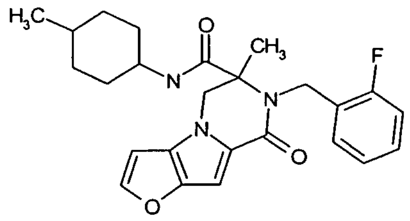 | 437,51861 | 438 | |
| 68 | 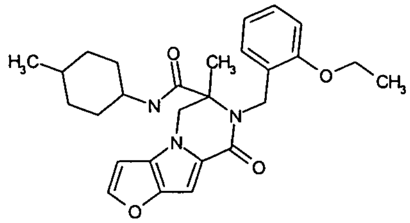 | 463,58176 | 464 | |
| 69 | 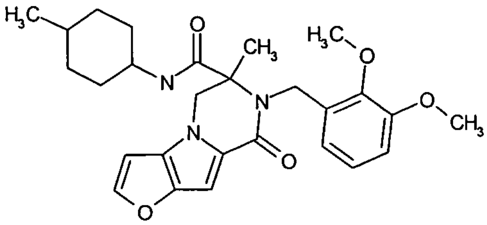 | 479,58116 | 480 | |
| 70 | 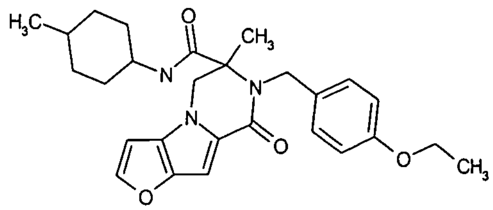 | 463,58176 | 464 | |
| 71 | 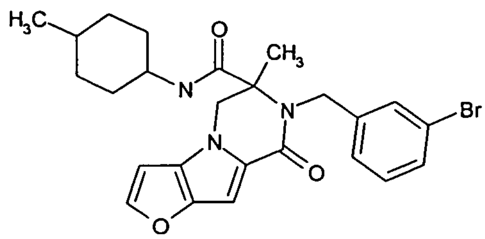 | 498,42421 | 499 | |
| 72 | 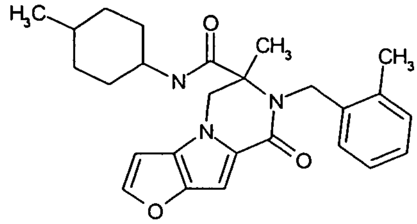 | 433,55527 | 434 | |

(continued)

| № | Formula | Mol.w. | m/z (M+1) | 1H NMR |
|---|---|---|---|---|
| 73 | 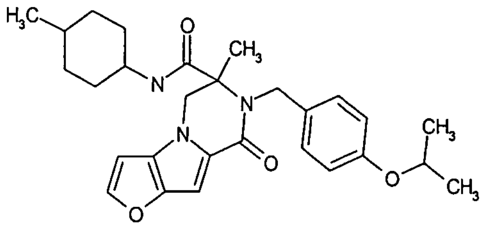 | 477,60885 | 478 | |
| 74 | 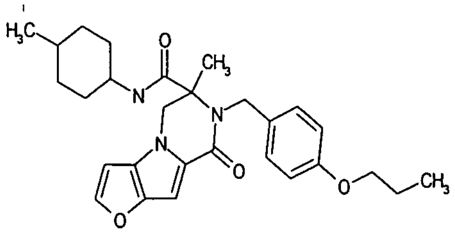 | 477,60885 | 478 | |
| 75 | 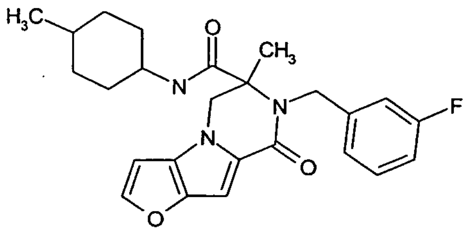 | 437,51861 | 438 | |
| 76 | 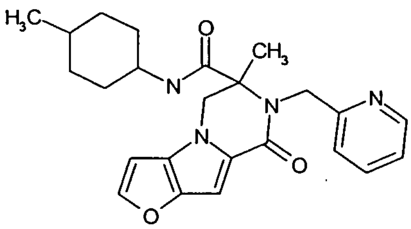 | 420,51576 | 421 | |
| 77 | 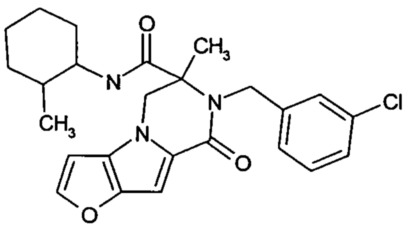 | 453,97321 | 454 | |
| 78 | 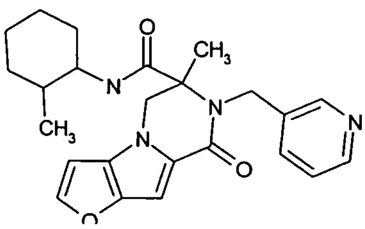 | 420,51576 | 421 | |

(continued)

| № | Formula | Mol.w. | m/z (M+1) | 1H NMR |
|---|---|---|---|---|
| 79 | 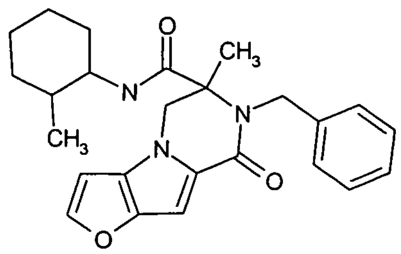 | 419,52818 | 420 | |
| 80 | 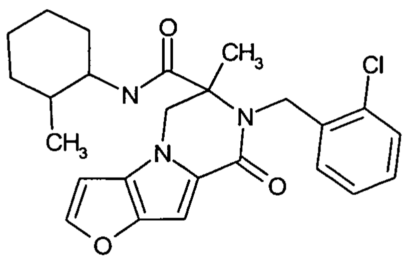 | 453,97321 | 454 | |
| 81 | 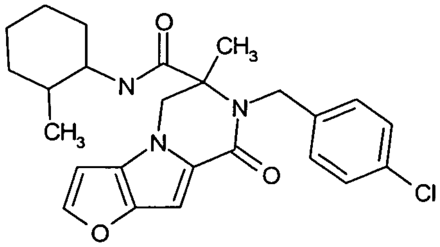 | 453,97321 | 454 | |
| 82 | 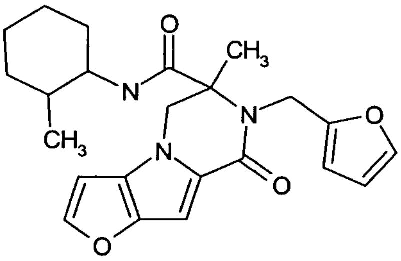 | 409,48934 | 410 | |
| 83 | 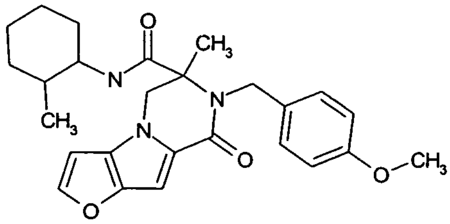 | 449,55467 | 450 | |
| 84 | 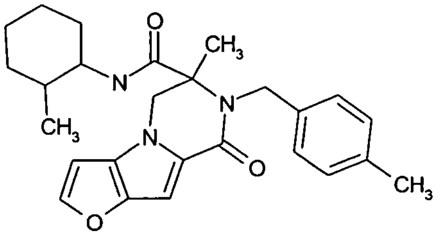 | 433,55527 | 434 | |

(continued)

| № | Formula | Mol.w. | m/z (M+1) | 1H NMR |
|---|---|---|---|---|
| 85 | 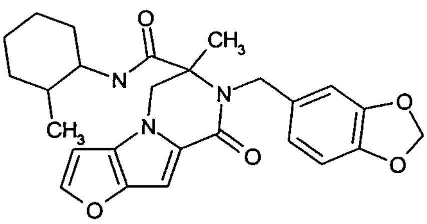 | 463,53813 | 464 | |
| 86 | 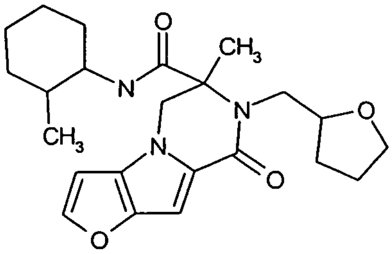 | 413,52122 | 414 | |
| 87 | 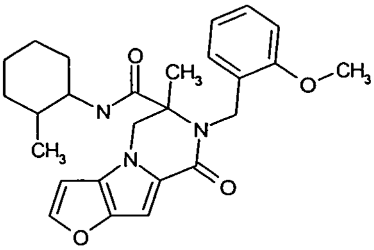 | 449,55467 | 450 | |
| 88 | 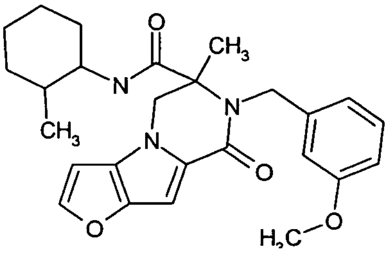 | 449,55467 | 450 | |
| 89 | 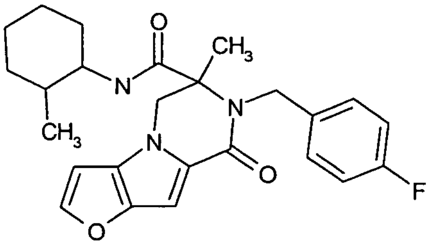 | 437,51861 | 438 | |
| 90 | 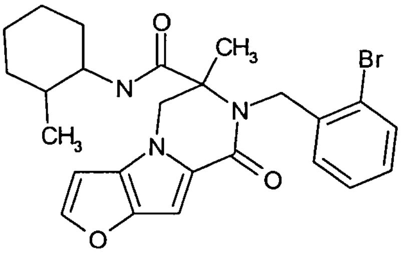 | 498,42421 | 499 | |

(continued)

| № | Formula | Mol.w. | m/z (M+1) | 1H NMR |
|---|---|---|---|---|
| 91 | 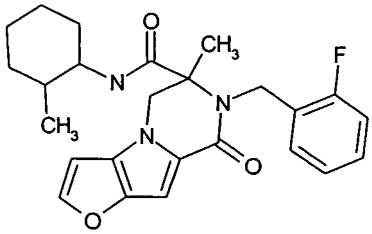 | 437,51861 | 438 | |
| 92 | 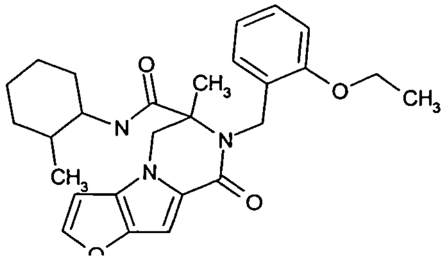 | 463,58176 | 464 | |
| 93 | 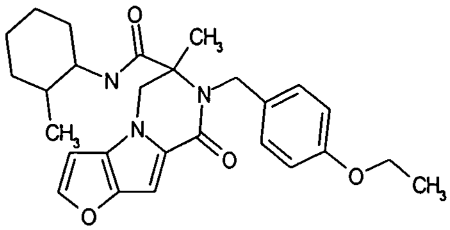 | 463,58176 | 464 | |
| 94 | 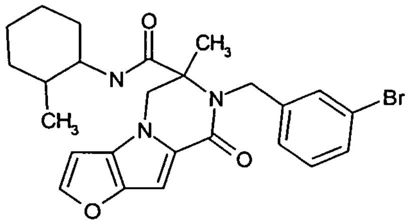 | 498,42421 | 499 | |
| 95 | 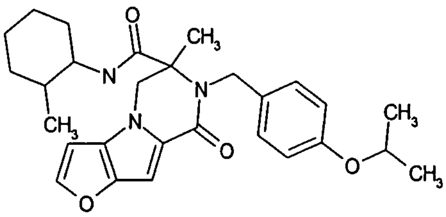 | 477,60885 | 478 | |
| 96 | 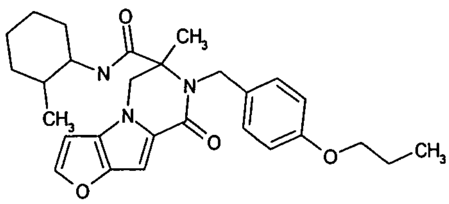 | 477,60885 | 478 | |

(continued)

| № | Formula | Mol.w. | m/z (M+1) | 1H NMR |
|---|---|---|---|---|
| 97 | 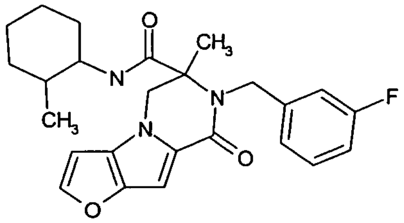 | 437,51861 | 438 | |
| 98 | 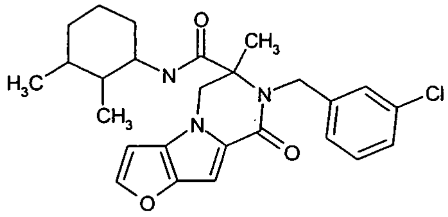 | 468,0003 | 469 | |
| 99 | 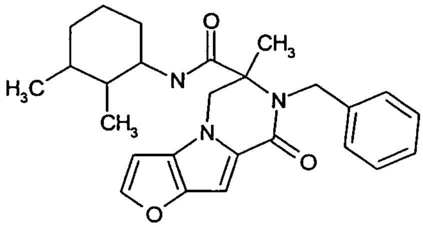 | 433,55527 | 434 | |
| 100 | 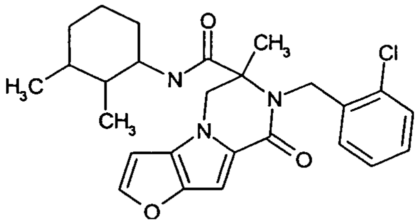 | 468,0003 | 469 | |
| 101 | 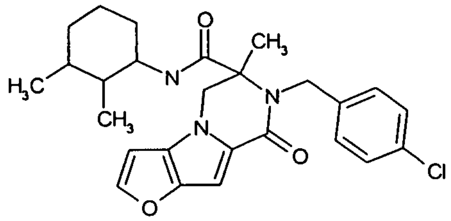 | 468,0003 | 469 | |
| 102 | 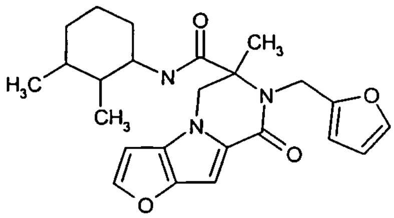 | 423,51643 | 424 | |

(continued)

| № | Formula | Mol.w. | m/z (M+1) | 1H NMR |
|---|---|---|---|---|
| 103 | 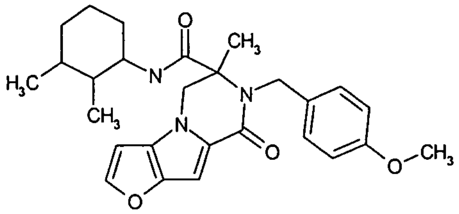 | 463,58176 | 464 | |
| 104 | 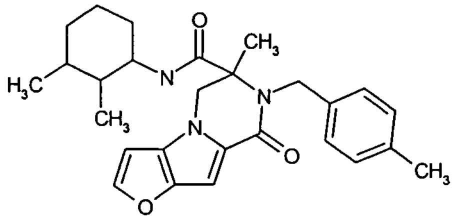 | 447,58236 | 448 | |
| 105 | 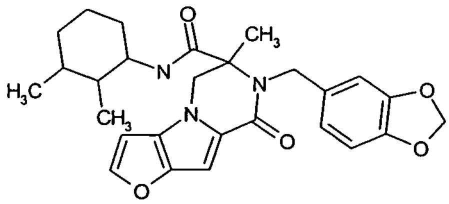 | 477,56522 | 478 | |
| 106 | 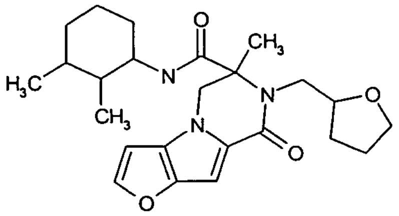 | 427,54831 | 428 | |
| 107 | 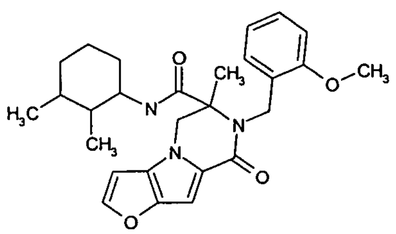 | 463,58176 | 464 | |
| 108 | 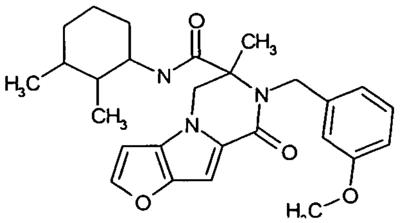 | 463,58176 | 464 | |
| 109 | 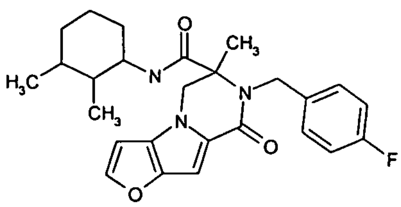 | 451,5457 | 452 | |

(continued)

| № | Formula | Mol.w. | m/z (M+1) | 1H NMR |
|---|---|---|---|---|
| 110 | 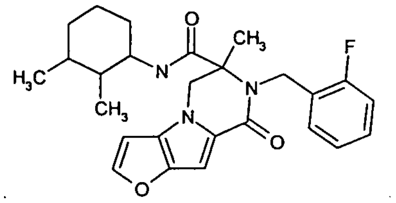 | 451,5457 | 452 | |
| 111 | 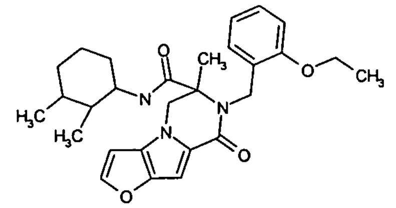 | 477,60885 | 478 | |
| 112 | 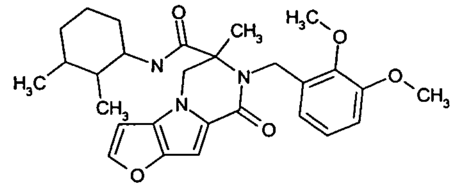 | 493,60825 | 494 | |
| 113 | 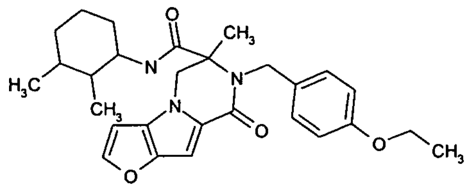 | 477,60885 | 478 | |
| 114 | 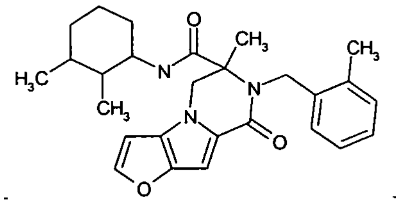 | 447,58236 | 448 | |
| 115 | 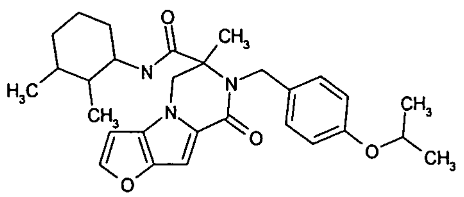 | 491,63594 | 492 | |
| 116 | 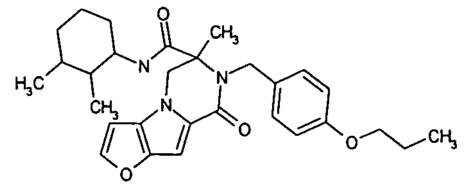 | 491,63594 | 492 | |

(continued)

| № | Formula | Mol.w. | m/z (M+1) | 1H NMR |
|---|---|---|---|---|
| 117 | 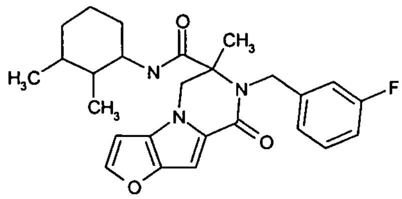 | 451,5457 | 452 | |
| 118 | 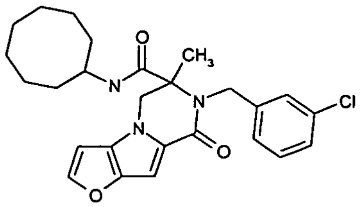 | 468,0003 | 469 | |
| 119 | 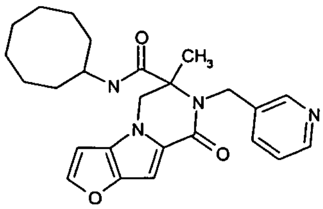 | 434,54285 | 435 | |
| 120 | 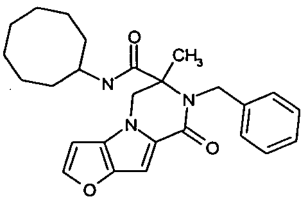 | 433,55527 | 434 | |
| 121 | 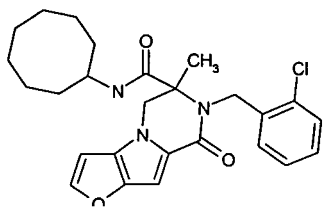 | 468,0003 | 469 | |
| 122 | 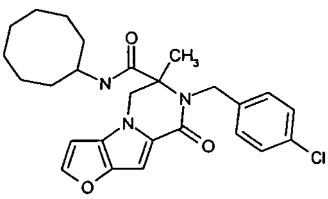 | 468,0003 | 469 | |
| 123 | 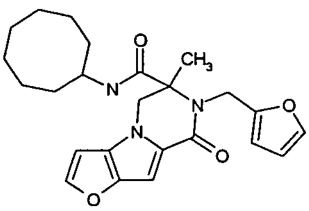 | 423,51643 | 424 | |

(continued)

| № | Formula | Mol.w. | m/z (M+1) | 1H NMR |
|---|---|---|---|---|
| 124 | 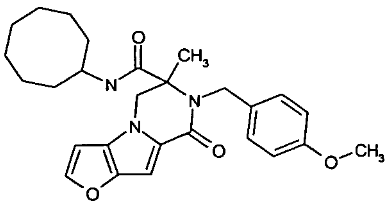 | 463,58176 | 464 | |
| 125 | 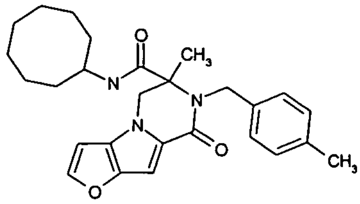 | 447,58236 | 448 | |
| 126 | 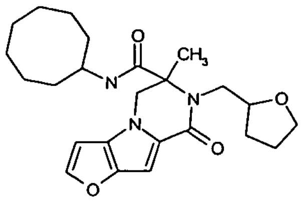 | 427,54831 | 428 | |
| 127 | 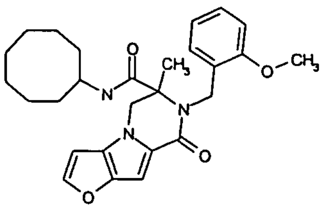 | 463,58176 | 464 | |
| 128 | 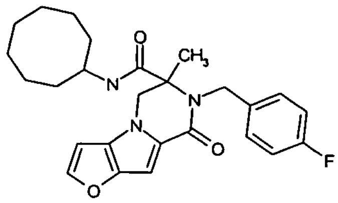 | 451,5457 | 452 | |
| 129 | 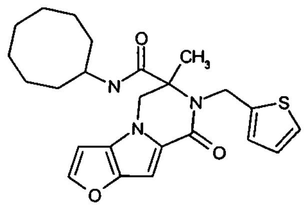 | 439,58103 | 440 | |
| 130 | 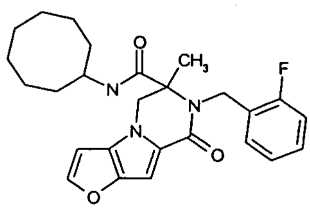 | 451,5457 | 452 | |

(continued)

| № | Formula | Mol.w. | m/z (M+1) | 1H NMR |
|---|---|---|---|---|
| 131 | 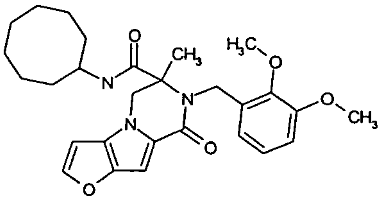 | 493,60825 | 494 | |
| 132 | 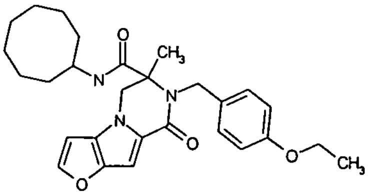 | 477,60885 | 478 | |
| 133 | 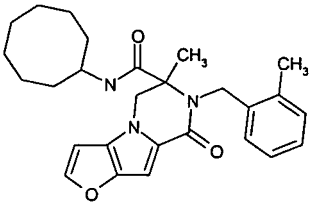 | 447,58236 | 448 | |
| 134 | 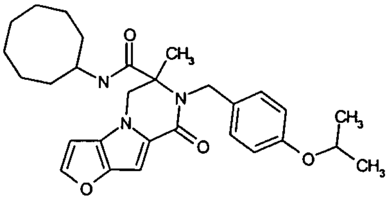 | 491,63594 | 492 | |
| 135 | 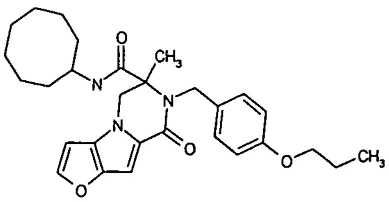 | 491,63594 | 492 | |
| 136 | 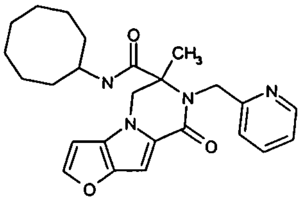 | 434,54285 | 435 | |
| 137 | 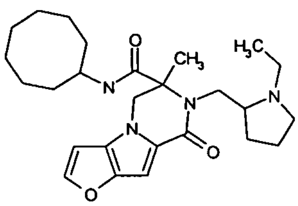 | 454,61776 | 455 | |

(continued)

| № | Formula | Mol.w. | m/z (M+1) | 1H NMR |
|---|---|---|---|---|
| 138 | 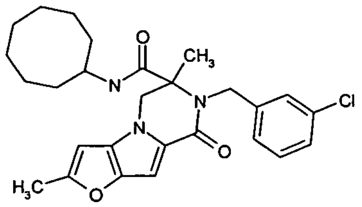 | 482,02739 | 483 | |
| 139 | 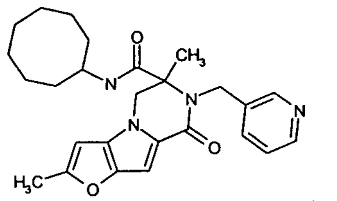 | 448,56994 | 449 | |
| 140 | 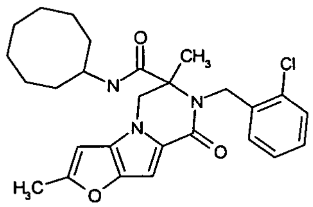 | 482,02739 | 483 | |
| 141 | 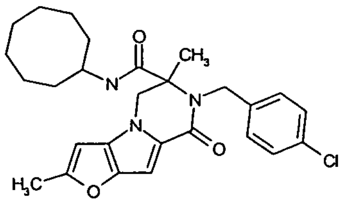 | 482,02739 | 483 | |
| 142 | 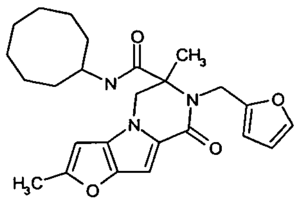 | 437,54352 | 438 | |
| 143 | 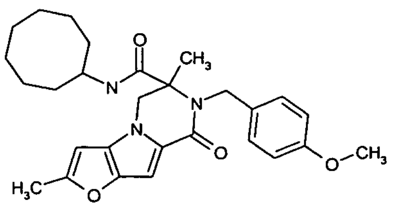 | 477,60885 | 478 | |
| 144 | 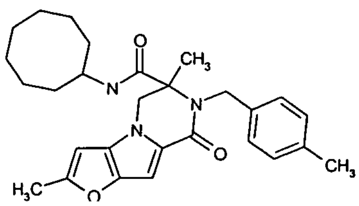 | 461,60945 | 462 | |

(continued)

| № | Formula | Mol.w. | m/z (M+1) | 1H NMR |
|---|---|---|---|---|
| 145 | 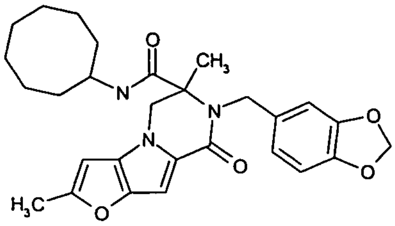 | 491,59231 | 492 | |
| 146 | 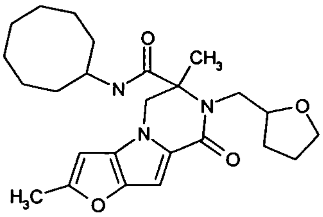 | 441,5754 | 442 | |
| 147 | 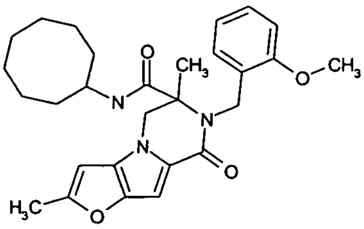 | 477,60885 | 478 | |
| 148 | 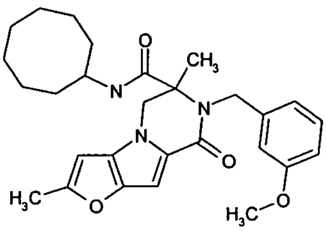 | 477,60885 | 478 | |
| 149 | 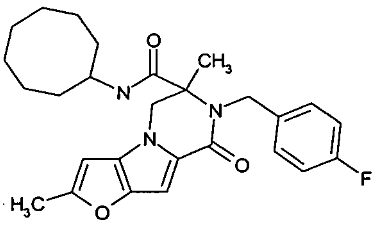 | 465,57279 | 466 | |
| 150 | 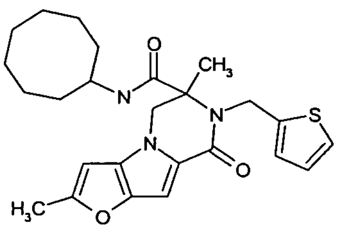 | 453,60812 | 454 | |

(continued)

| № | Formula | Mol.w. | m/z (M+1) | 1H NMR |
|---|---|---|---|---|
| 151 | 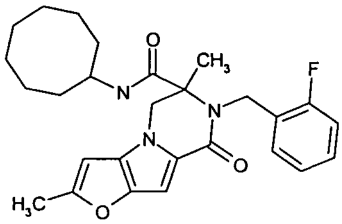 | 465,57279 | 466 | |
| 152 | 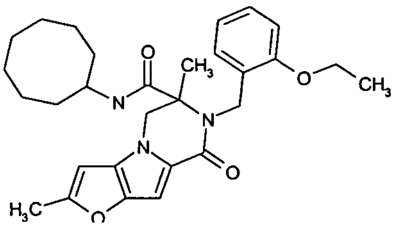 | 491,63594 | 492 | |
| 153 | 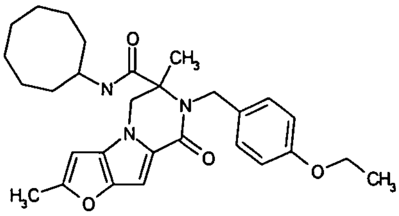 | 491,63594 | 492 | |
| 154 | 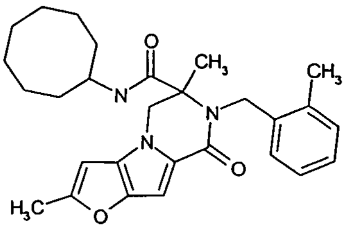 | 461,60945 | 462 | |
| 155 | 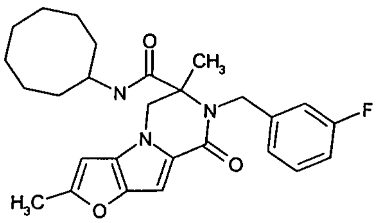 | 465,57279 | 466 | |
| 156 | 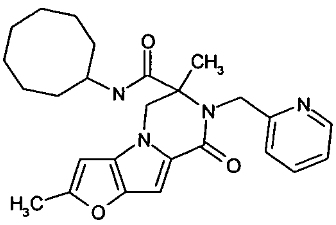 | 448,56994 | 449 | |

(continued)

| № | Formula | Mol.w. | m/z (M+1) | 1H NMR |
|---|---|---|---|---|
| 157 | 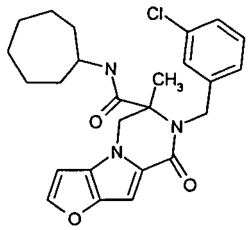 | 453,97321 | 454 | |
| 158 | 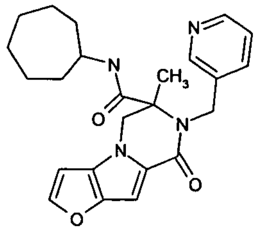 | 420,51576 | 421 | |
| 159 | 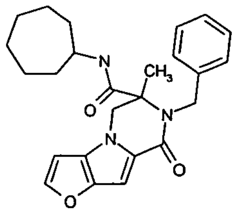 | 419,52818 | 420 | |
| 160 | 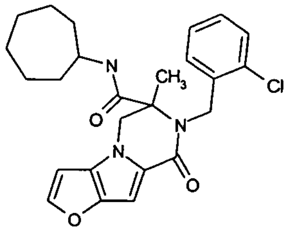 | 453,97321 | 454 | |
| 161 | 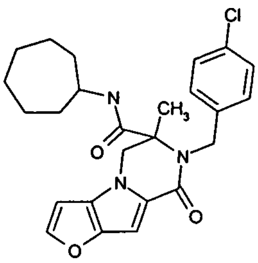 | 453,97321 | 454 | |
| 162 | 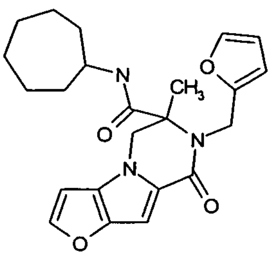 | 409,48934 | 410 | |

(continued)

| № | Formula | Mol.w. | m/z (M+1) | 1H NMR |
|---|---|---|---|---|
| 163 | 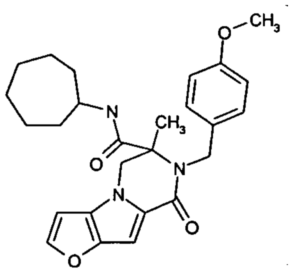 | 449,55467 | 450 | |
| 164 | 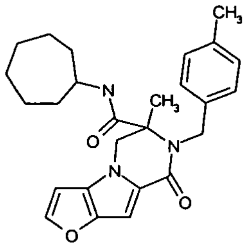 | 433,55527 | 434 | |
| 165 | 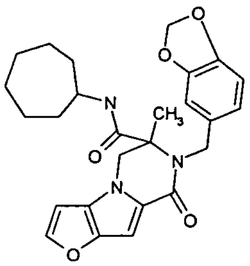 | 463,53813 | 464 | |
| 166 | 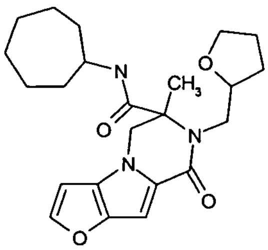 | 413,52122 | 414 | |
| 167 | 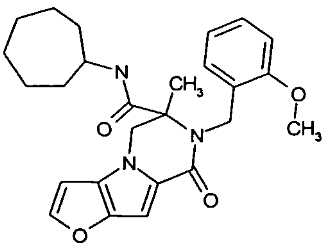 | 449,55467 | 450 | |
| 168 | 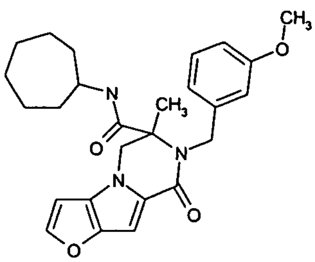 | 449,55467 | 450 | |

(continued)

| № | Formula | Mol.w. | m/z (M+1) | 1H NMR |
|---|---|---|---|---|
| 169 | 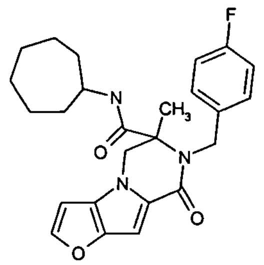 | 437,51861 | 438 | |
| 170 | 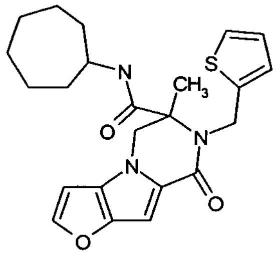 | 425,55394 | 426 | |
| 171 | 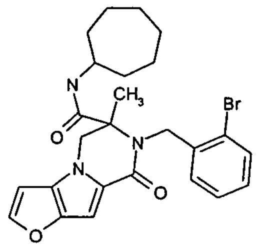 | 498,42421 | 499 | |
| 172 | 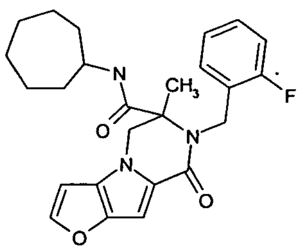 | 437,51861 | 438 | |
| 173 | 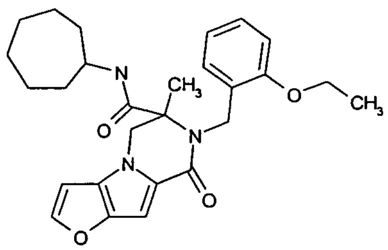 | 463,58176 | 464 | |
| 174 | 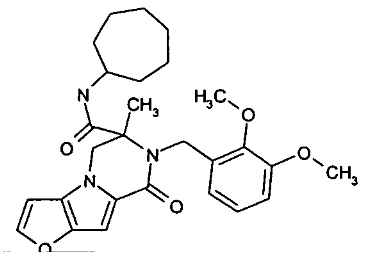 | 479,58116 | 480 | |

(continued)

| № | Formula | Mol.w. | m/z (M+1) | 1H NMR |
|---|---|---|---|---|
| 175 | 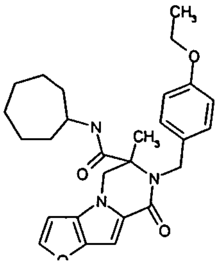 | 463,58176 | 464 | |
| 176 | 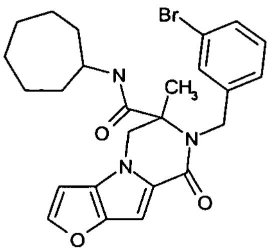 | 498,42421 | 499 | |
| 177 | 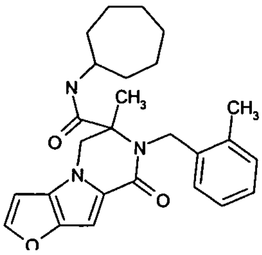 | 433,55527 | 434 | |
| 178 | 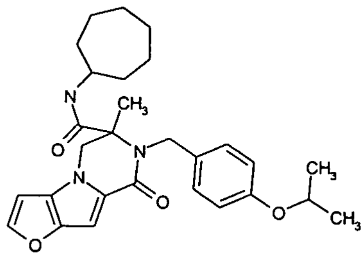 | 477,60885 | 478 | |
| 179 | 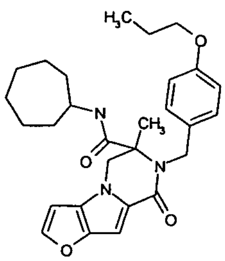 | 477,60885 | 478 | |
| 180 | 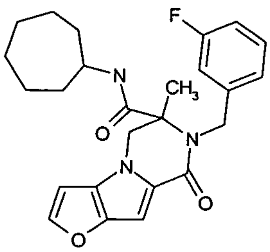 | 437,51861 | 438 | |

(continued)

| № | Formula | Mol.w. | m/z (M+1) | 1H NMR |
|---|---|---|---|---|
| 181 | 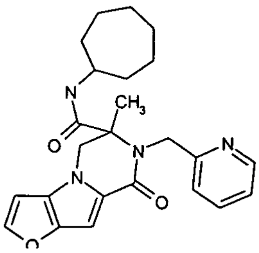 | 420,51576 | 421 | |
| 182 | 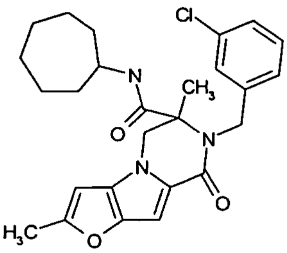 | 468,0003 | 469 | |
| 183 | 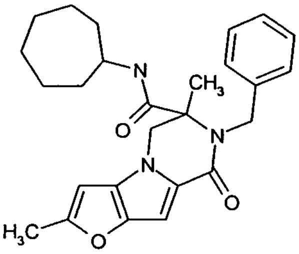 | 433,55527 | 434 | |
| 184 | 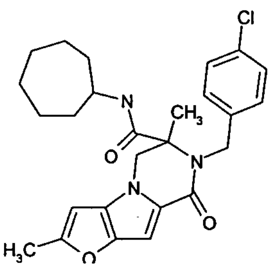 | 468,0003 | 469 | |
| 185 | 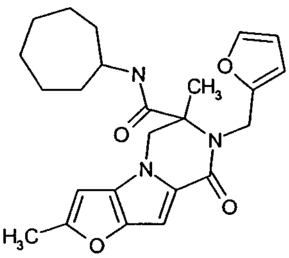 | 423,51643 | 424 | |
| 186 | 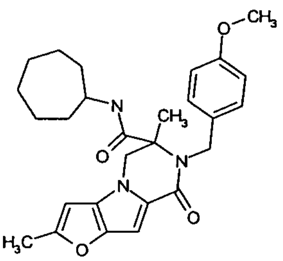 | 463,58176 | 464 | |

(continued)

| № | Formula | Mol.w. | m/z (M+1) | 1H NMR |
|---|---|---|---|---|
| 187 | 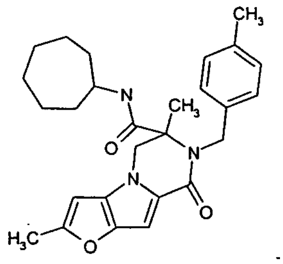 | 447,58236 | 448 | |
| 188 | 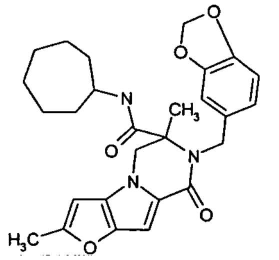 | 477,56522 | 478 | |
| 189 | 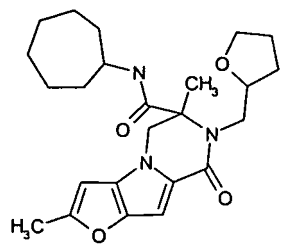 | 427,54831 | 428 | |
| 190 | 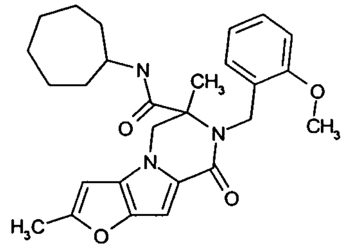 | 463,58176 | 464 | |
| 191 | 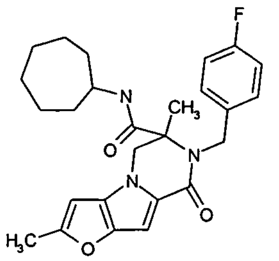 | 451,5457 | 452 | |
| 192 | 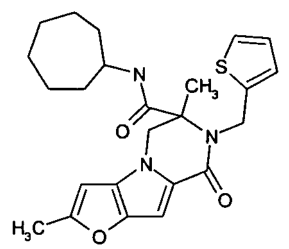 | 439,58103 | 440 | |

(continued)

| № | Formula | Mol.w. | m/z (M+1) | 1H NMR |
|---|---|---|---|---|
| 193 | 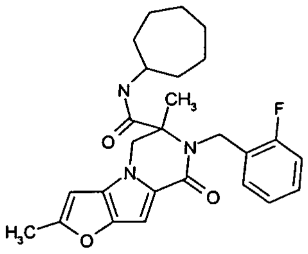 | 451,5457 | 452 | |
| 194 | 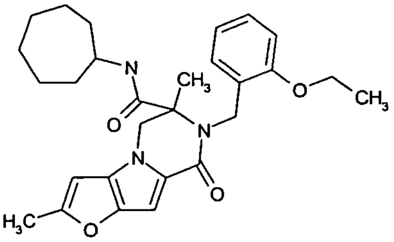 | 477,60885 | 478 | |
| 195 | 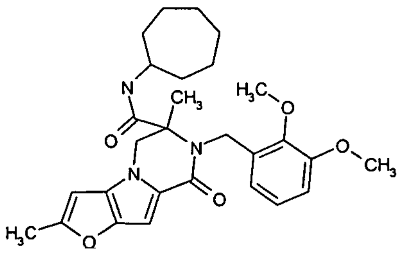 | 493,60825 | 494 | |
| 196 | 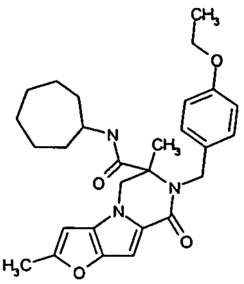 | 477,60885 | 478 | |
| 197 | 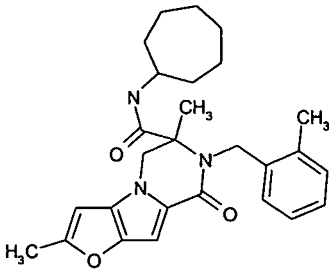 | 447,58236 | 448 | |

(continued)

| № | Formula | Mol.w. | m/z (M+1) | 1H NMR |
|---|---|---|---|---|
| 198 | 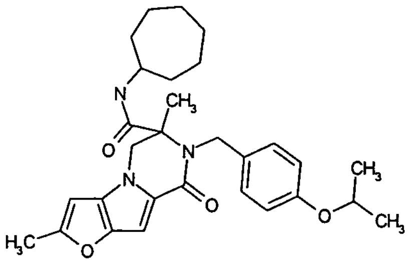 | 491,63594 | 492 | |
| 199 | 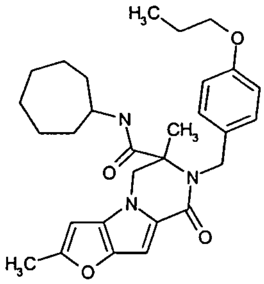 | 491,63594 | 492 | |
| 200 | 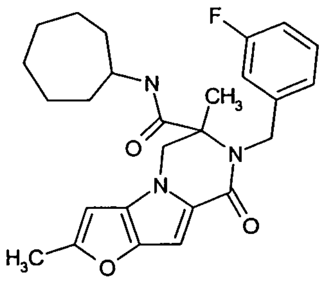 | 451,5457 | 452 | |
| 201 | 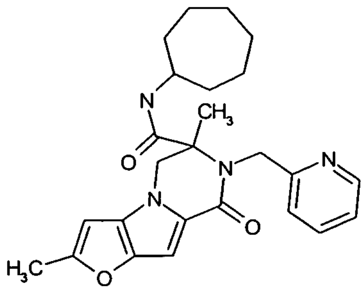 | 434,54285 | 435 | |

| | Table 1.17(4) | | | |
|---|---|---|---|---|
| № | Formula | Mol.w. | LC MS, m/z (M+1) | 1H NMR |
| 1 | 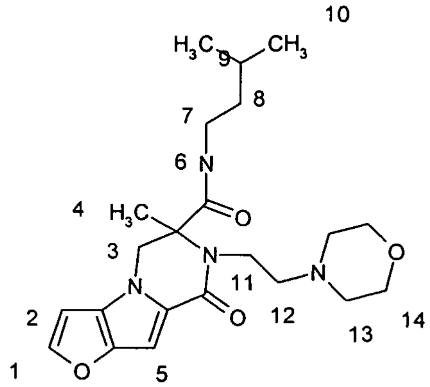 | 416,5247 | 417 | 0.56, 0.65 (10; 6H; d.,d., 6.4 Hz), 0.82 - 1.15 (8, 9; 3H; m.), 1.63 (4; 3H, s.), 2.25, 2.41, 2.61, 2.69, 2.86, 3.27, 3.37, 3.59, 4.14 (7, 11, 12, 13, 14; 14H; m.m.), 3.86 + 4.66 (3; 2H; AB; 12.2 Hz), 6.47 (2; 1H; d., 1.7 Hz), 6.52 (5; 1 H; s.), 7.44 ... |
| 2 | 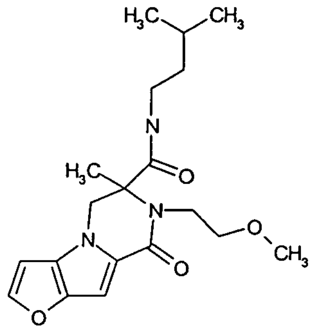 | 361,4447 | 362 | |
| 3 | 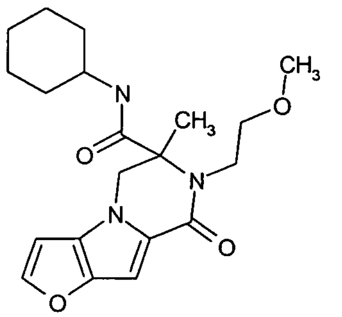 | 373,4559 | 374 | |
| 4 | 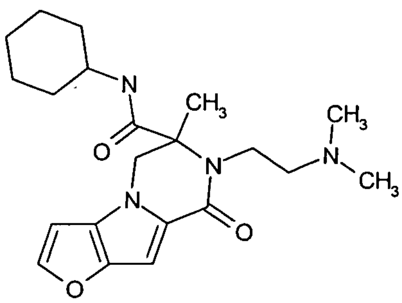 | 386,4983 | 387 | |

(continued)

| | Table 1.17(4) | | | |
|---|---|---|---|---|
| № | Formula | Mol.w. | LC MS, m/z (M+1) | 1H NMR |
| 5 | 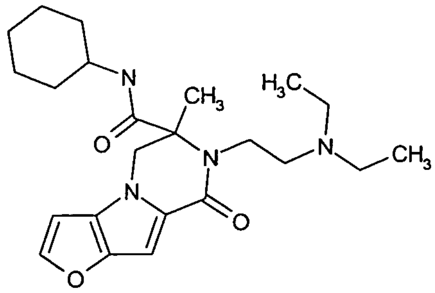 | 414,5524 | 415 | |
| 6 | 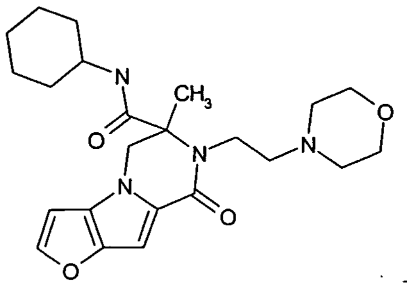 | 428,5359 | 429 | |
| 7 | 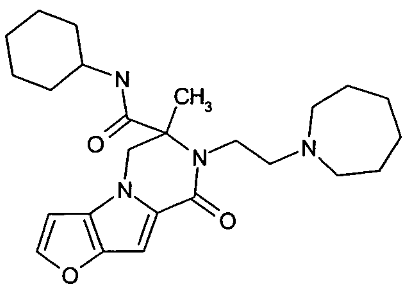 | 440,5907 | 441 | |
| 8 | 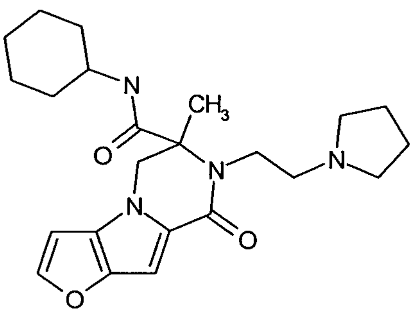 | 412,5365 | 413 | |
| 9 | 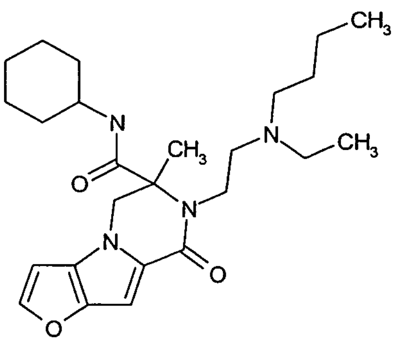 | 442,6066 | 443 | |

(continued)

| | Table 1.17(4) | | | |
|---|---|---|---|---|
| № | Formula | Mol.w. | LC MS, m/z (M+1) | 1H NMR |
| 10 | 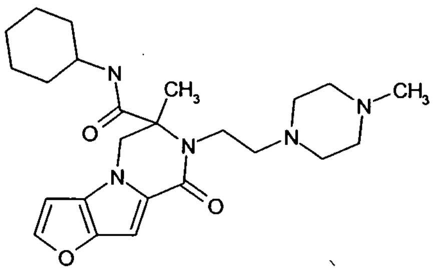 | 441,5783 | 442 | |
| 11 | 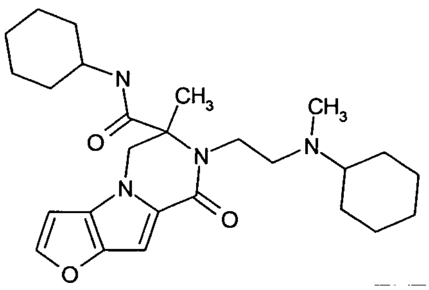 | 454,6178 | 455 | |
| 12 | 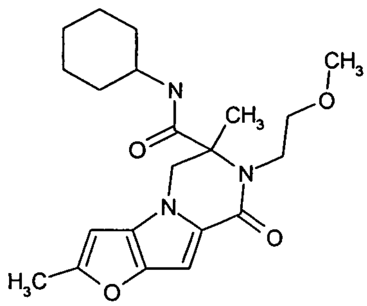 | 387,483 | 388 | |
| 13 | 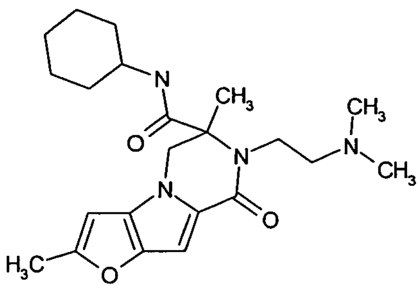 | 400,5253 | 401 | |
| 14 | 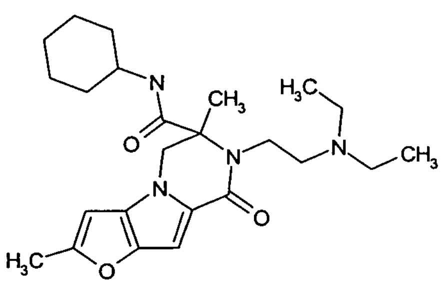 | 428,5795 | 429 | |

(continued)

| | Table 1.17(4) | | | |
|---|---|---|---|---|
| № | Formula | Mol.w. | LC MS, m/z (M+1) | 1H NMR |
| 15 | 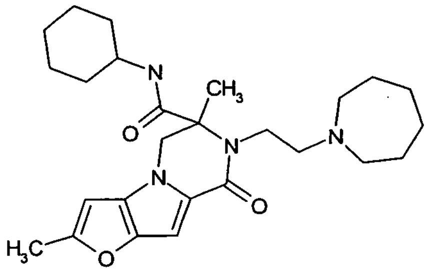 | 454,6178 | 455 | |
| 16 | 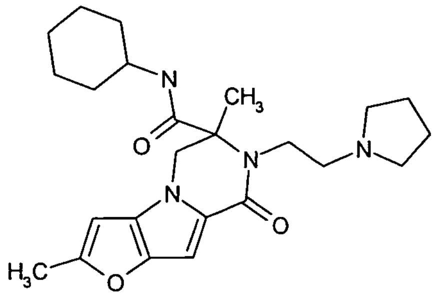 | 426,5636 | 427 | |
| 17 | 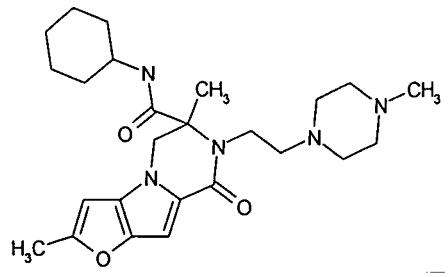 | 455,6053 | 456 | |
| 18 | 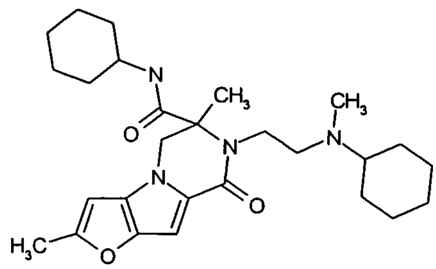 | 468,6449 | 469 | |
| 19 | 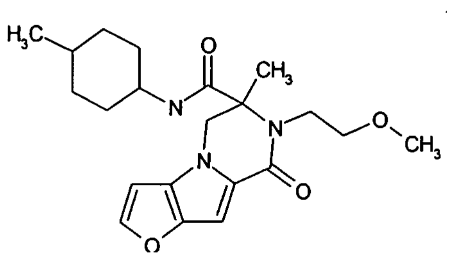 | 387,483 | 388 | |

(continued)

| | Table 1.17(4) | | | |
|---|---|---|---|---|
| № | Formula | Mol.w. | LC MS, m/z (M+1) | 1H NMR |
| 20 | 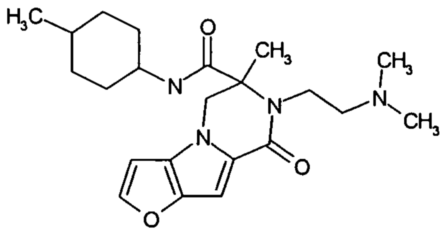 | 400,5253 | 401 | |
| 21 | 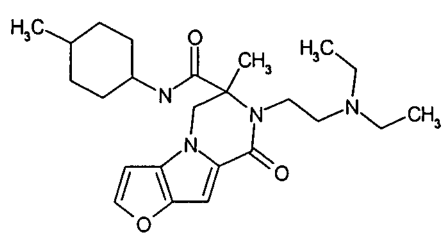 | 428,5795 | 429 | |
| 22 | 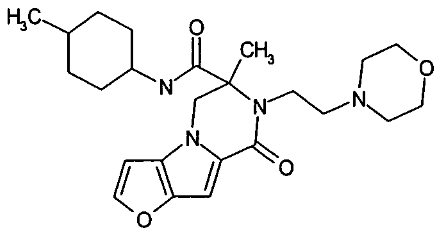 | 442,563 | 443 | |
| 23 | 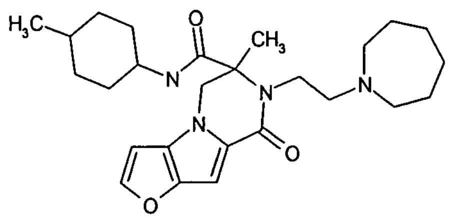 | 454,6178 | 455 | |
| 24 | 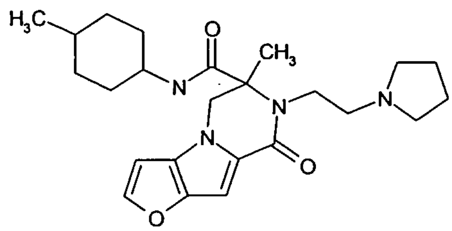 | 426,5636 | 427 | |
| 25 | 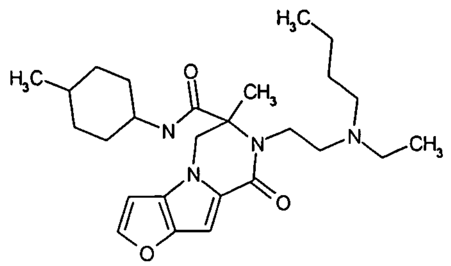 | 456,6337 | 457 | |

(continued)

| | Table 1.17(4) | | | |
|---|---|---|---|---|
| № | Formula | Mol.w. | LC MS, m/z (M+1) | 1H NMR |
| 26 | 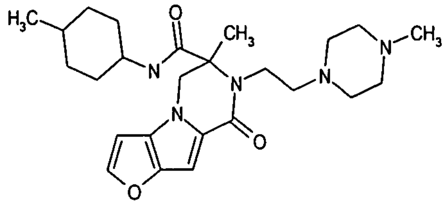 | 455,6053 | 456 | |
| 27 | 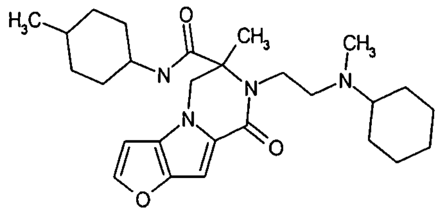 | 468,6449 | 469 | |
| 28 | 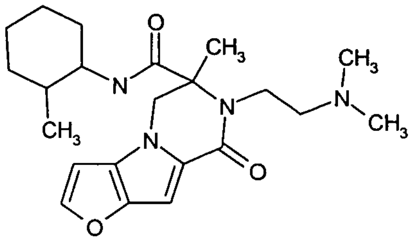 | 400,5253 | 401 | |
| 29 | 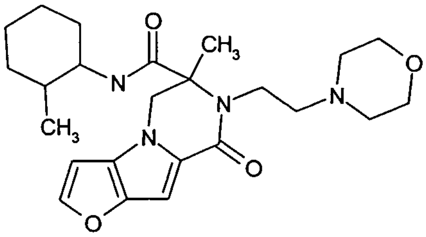 | 442,563 | 443 | |
| 30 | 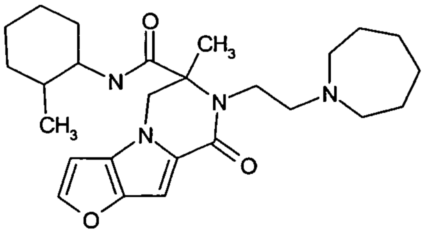 | 454,6178 | 455 | |
| 31 | 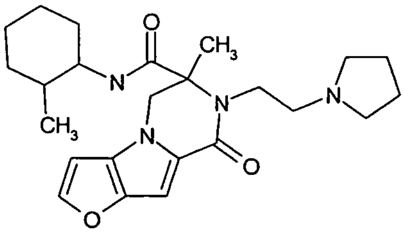 | 426,5636 | 427 | |

(continued)

| | Table 1.17(4) | | | |
|---|---|---|---|---|
| № | Formula | Mol.w. | LC MS, m/z (M+1) | 1H NMR |
| 32 | 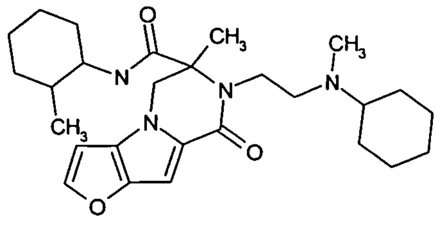 | 468,6449 | 469 | |
| 33 | 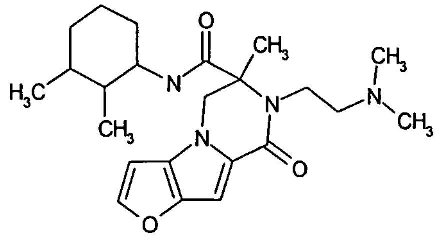 | 414,5524 | 415 | |
| 34 | 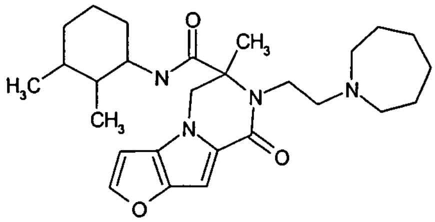 | 468,6449 | 469 | |
| 35 | 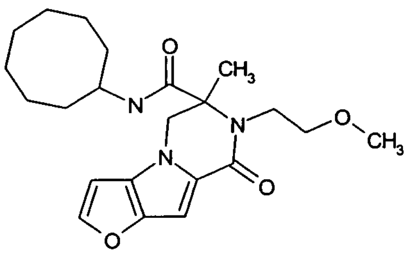 | 401,5101 | 402 | |
| 36 | 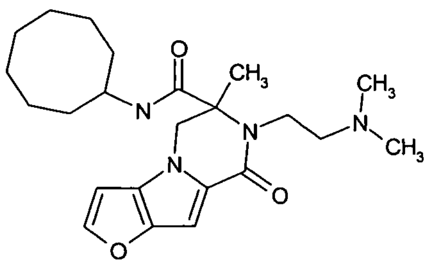 | 414,5524 | 415 | |
| 37 | 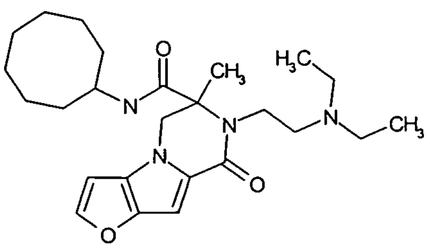 | 442,6066 | 443 | |

(continued)

| | Table 1.17(4) | | | |
|---|---|---|---|---|
| № | Formula | Mol.w. | LC MS, m/z (M+1) | 1H NMR |
| 38 | 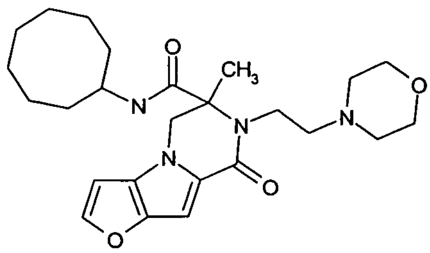 | 456,5901 | 457 | |
| 39 | 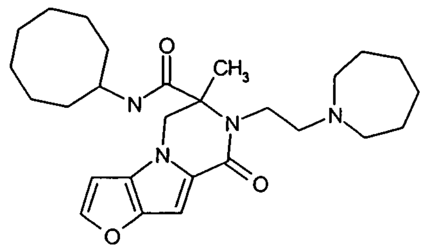 | 468,6449 | 469 | |
| 40 | 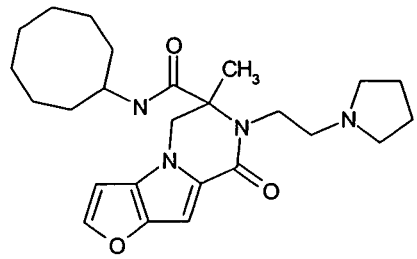 | 440,5907 | 441 | |
| 41 | 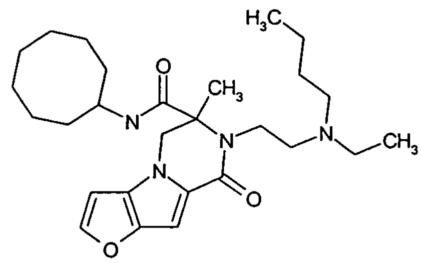 | 470,6608 | 471 | |
| 42 | 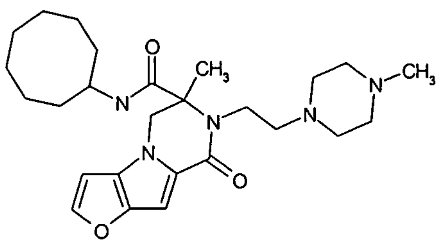 | 469,6324 | 470 | |

(continued)

| | Table 1.17(4) | | | |
|---|---|---|---|---|
| № | Formula | Mol.w. | LC MS, m/z (M+1) | 1H NMR |
| 43 | 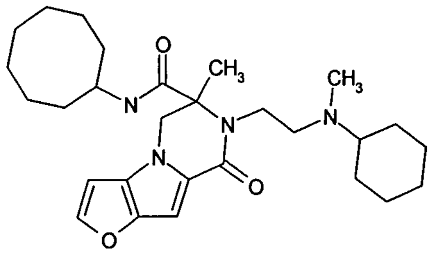 | 482,6719 | 483 | |
| 44 | 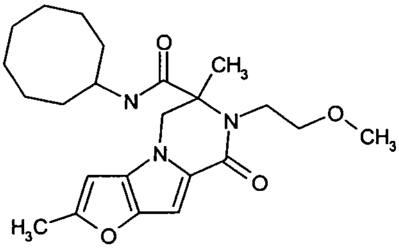 | 415,5372 | 416 | |
| 45 | 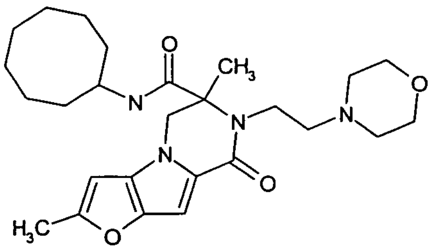 | 470,6172 | 471 | |
| 46 | 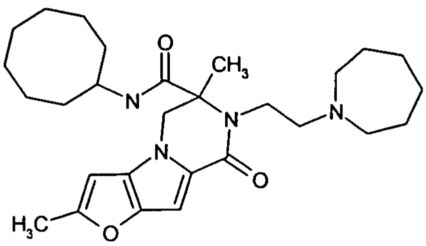 | 482,6719 | 483 | |
| 47 | 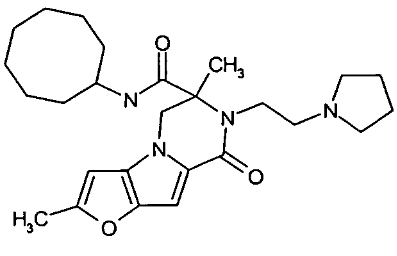 | 454,6178 | 455 | |

(continued)

| | Table 1.17(4) | | | |
|---|---|---|---|---|
| № | Formula | Mol.w. | LC MS, m/z (M+1) | 1H NMR |
| 48 | 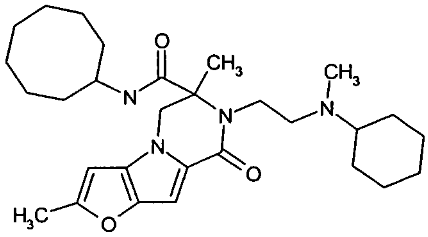 | 496,699 | 497 | |
| 49 | 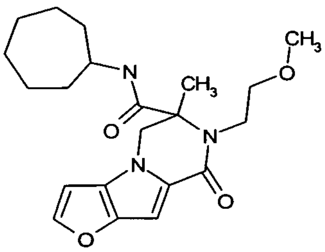 | 387,483 | 388 | |
| 50 | 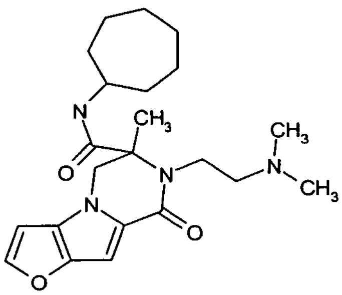 | 400,5253 | 401 | |
| 51 | 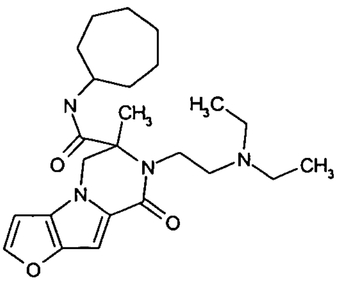 | 428,5795 | 429 | |
| 52 | 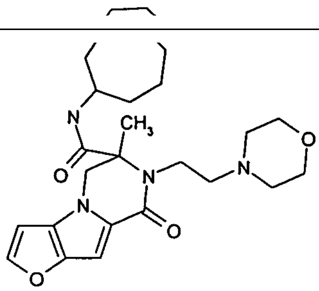 | 442,563 | 443 | |

(continued)

| | Table 1.17(4) | | | |
|---|---|---|---|---|
| № | Formula | Mol.w. | LC MS, m/z (M+1) | 1H NMR |
| 53 | 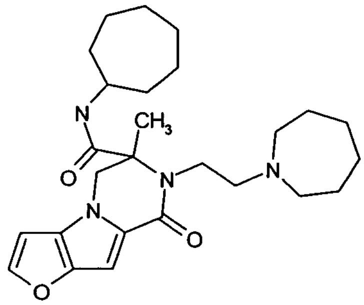 | 454,6178 | 455 | |
| 54 | 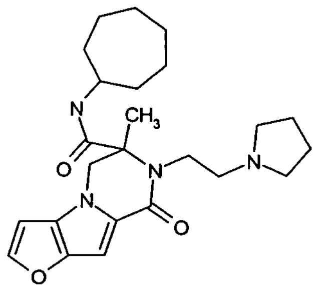 | 426,5636 | 427 | |
| 55 | 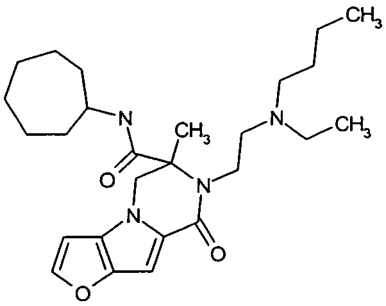 | 456,6337 | 457 | |
| 56 | 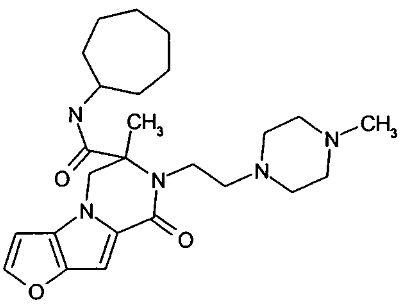 | 455,6053 | 456 | |
| 57 | 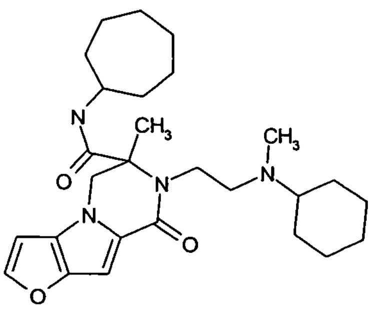 | 468,6449 | 469 | |

(continued)

| | Table 1.17(4) | | | |
|---|---|---|---|---|
| № | Formula | Mol.w. | LC MS, m/z (M+1) | 1H NMR |
| 58 | 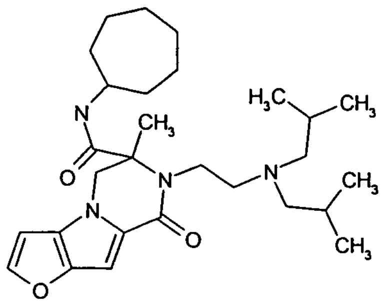 | 484,6879 | 485 | |
| 59 | 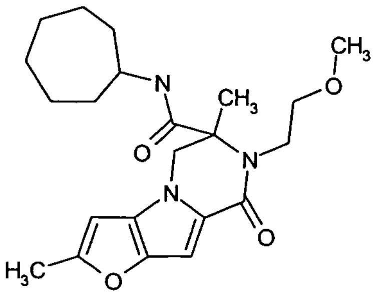 | 401,5101 | 402 | |
| 60 | 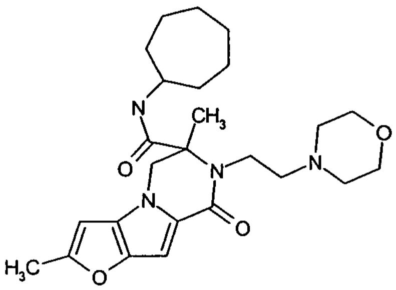 | 456,5901 | 457 | |
| 61 | 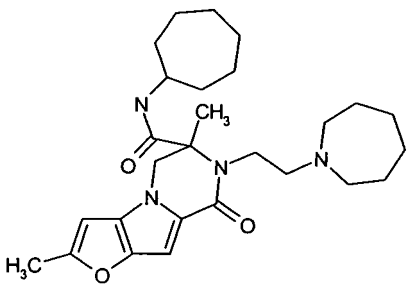 | 468,6449 | 469 | |
| 62 | 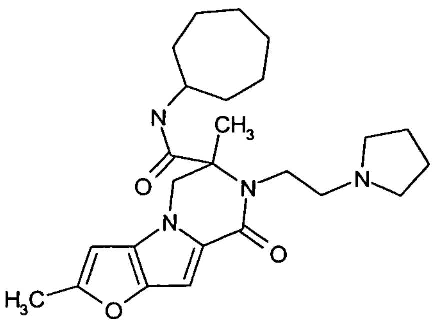 | 440,5907 | 441 | |

(continued)

| | Table 1.17(4) | | | |
|---|---|---|---|---|
| № | Formula | Mol.w. | LC MS, m/z (M+1) | 1H NMR |
| 63 | 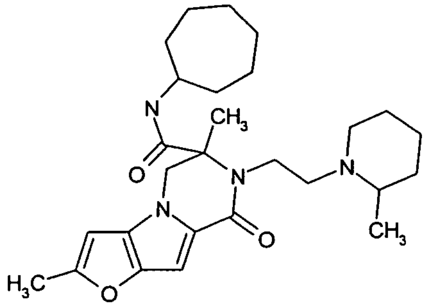 | 468,6449 | 469 | |
| 64 | 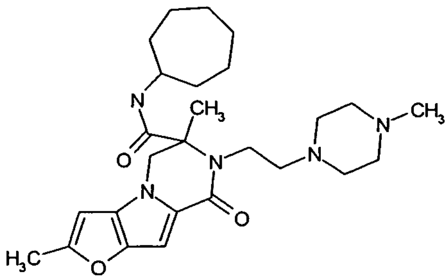 | 469,6324 | 470 | |
| 65 | 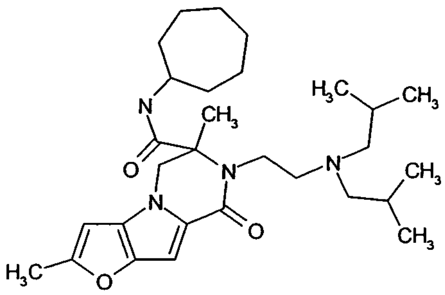 | 498,715 | 499 | |

| | Table 1.17(5) | | | |
|---|---|---|---|---|
| № | Formula | Mol.w. | LC MS, m/z (M+1) | 1H NMR |
| 1 | 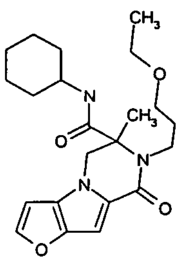 | 401,51007 | 402 | |
| 2 | 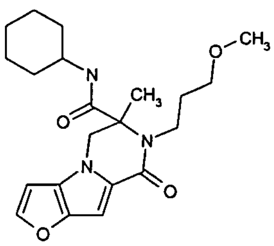 | 387,48298 | 388 | |

(continued)

| | Table 1.17(5) | | | |
|---|---|---|---|---|
| № | Formula | Mol.w. | LC MS, m/z (M+1) | 1H NMR |
| 3 | 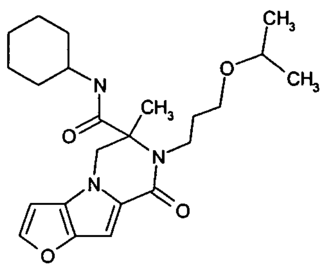 | 415,53716 | 416 | |
| 4 | 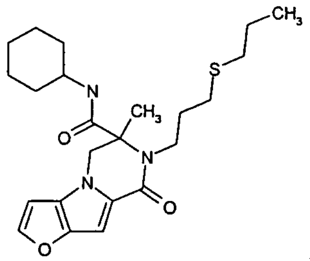 | 431,60176 | 432 | |
| 5 | 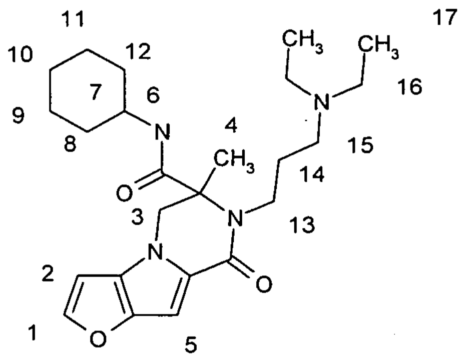 | 428,57952 | 429 | 0.99 (17; 6H; t.; 6.2 Hz), 1.03 - 1.82 (8, 9, 10, 11, 12, 14; 12H; m.), 1.57 (4; 3H, s.), 2.45 (15, 16; 6H; m.), 3.18 (7; 1 H; b.m.), 3.52 (13; 2H; m.), 3.96 + 4.60 (3; 2H; AB; 12.3 Hz), 6.52 (2; 1H; b.s.), 6.55 (5; 1H; s.), 7.15 (6; 1 H; d.; 8.3 Hz),... |
| 6 | 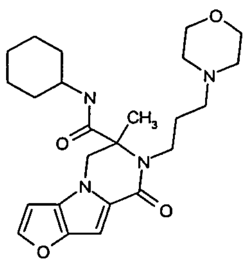 | 442,56298 | 443 | |
| 7 | 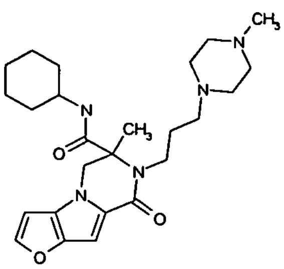 | 455,60534 | 456 | |

(continued)

| | Table 1.17(5) | | | |
|---|---|---|---|---|
| № | Formula | Mol.w. | LC MS, m/z (M+1) | 1H NMR |
| 8 | 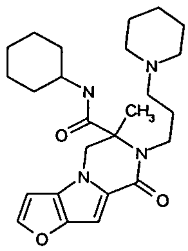 | 440,59067 | 441 | |
| 9 | 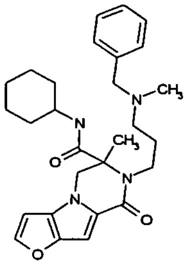 | 476,62412 | 477 | |
| 10 | 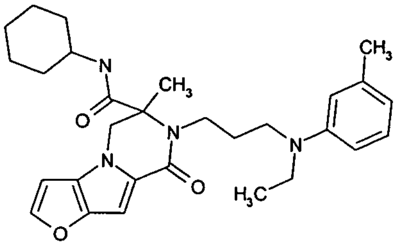 | 490,65121 | 491 | |
| 11 | 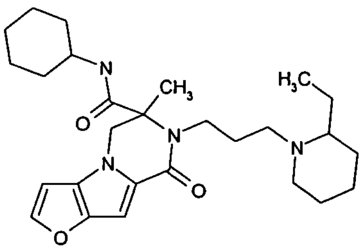 | 468,64485 | 469 | |
| 12 | 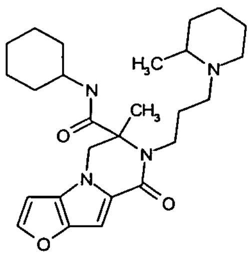 | 454,61776 | 455 | |
| 13 | 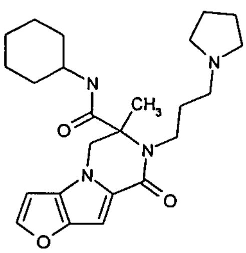 | 426,56358 | 427 | |

(continued)

| | Table 1.17(5) | | | |
|---|---|---|---|---|
| № | Formula | Mol.w. | LC MS, m/z (M+1) | 1H NMR |
| 14 | 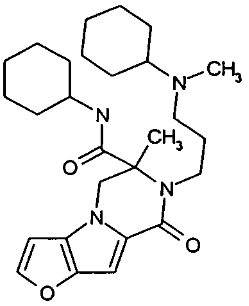 | 468,64485 | 469 | |
| 15 | 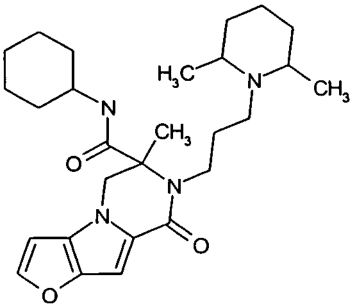 | 468,64485 | 469 | |
| 16 | 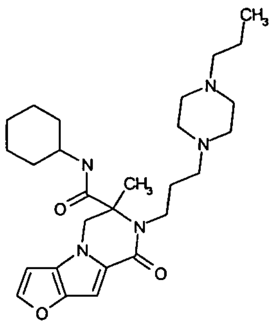 | 483,65952 | 484 | |
| 17 | 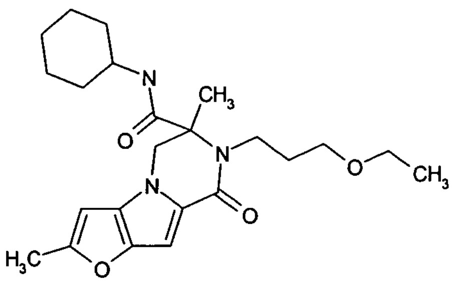 | 415,53716 | 416 | |
| 18 | 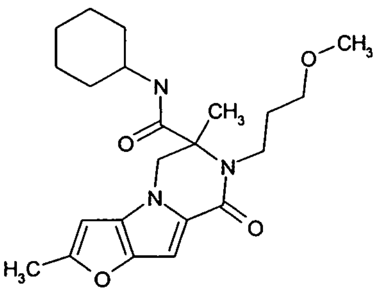 | 401,51007 | 402 | |

(continued)

| | Table 1.17(5) | | | |
|---|---|---|---|---|
| Nº | Formula | Mol.w. | LC MS, m/z (M+1) | 1H NMR |
| 19 | 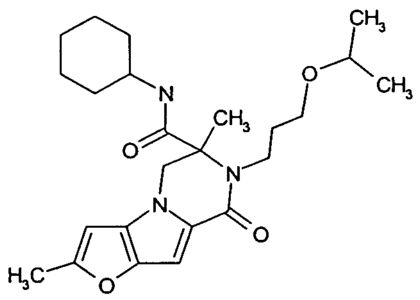 | 429,56425 | 430 | |
| 20 | 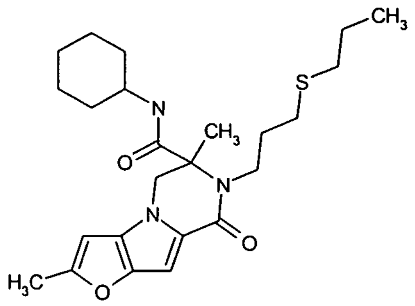 | 445,62885 | 446 | |
| 21 | 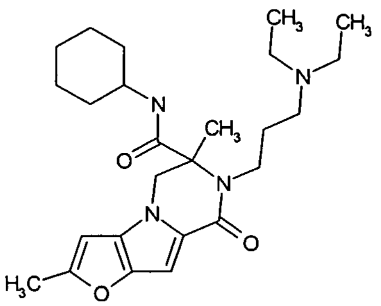 | 442,60661 | 443 | |
| 22 | 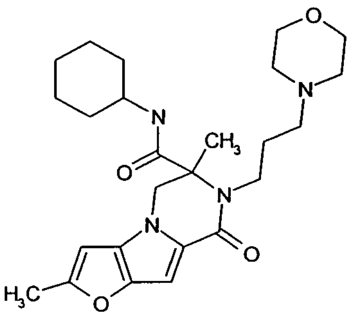 | 456,59007 | 457 | |
| 23 | 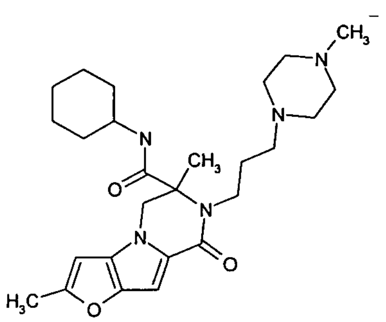 | 469,63243 | 470 | |

(continued)

| | Table 1.17(5) | | | |
|---|---|---|---|---|
| № | Formula | Mol.w. | LC MS, m/z (M+1) | 1H NMR |
| 24 | 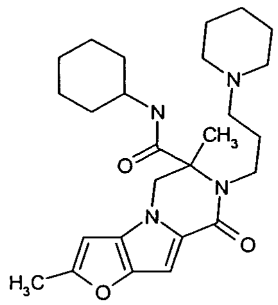 | 454,61776 | 455 | |
| 25 | 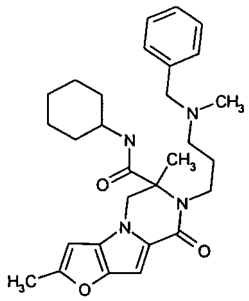 | 490,65121 | 491 | |
| 26 | 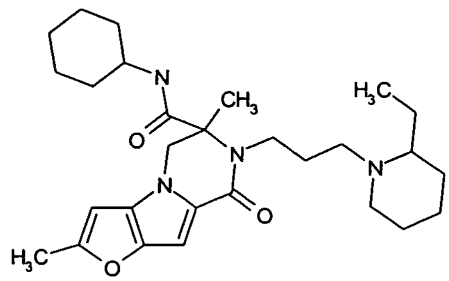 | 482,67194 | 483 | |
| 27 | 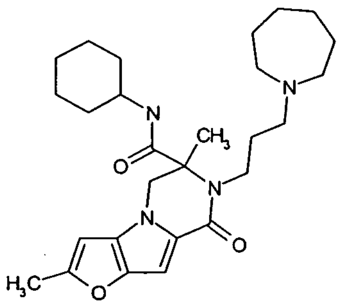 | 468,64485 | 469 | |
| 28 | 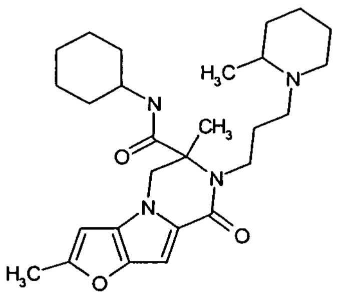 | 468,64485 | 469 | |

(continued)

| | Table 1.17(5) | | | |
|---|---|---|---|---|
| № | Formula | Mol.w. | LC MS, m/z (M+1) | 1H NMR |
| 29 | 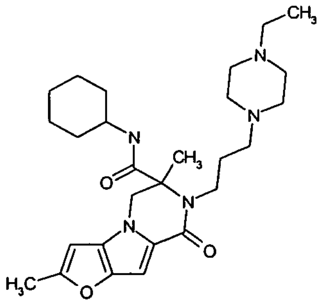 | 483,65952 | 484 | |
| 30 | 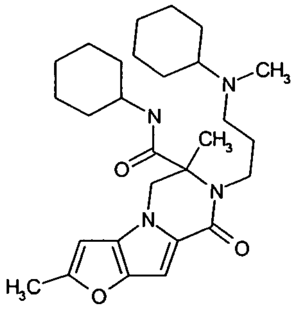 | 482,67194 | 483 | |
| 31 | 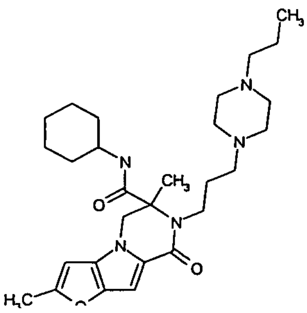 | 497,68661 | 498 | |
| 32 | 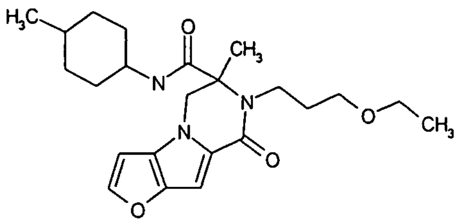 | 415,53716 | 416 | |
| 33 | 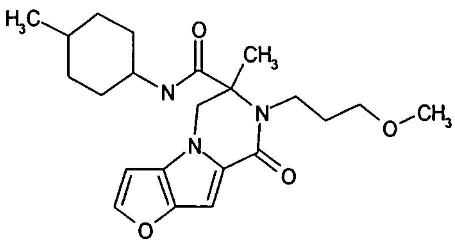 | 401,51007 | 402 | |

(continued)

| | Table 1.17(5) | | | |
|---|---|---|---|---|
| № | Formula | Mol.w. | LC MS, m/z (M+1) | 1H NMR |
| 34 | 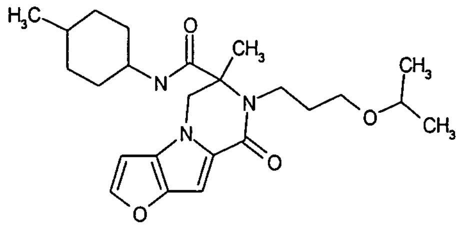 | 429,56425 | 430 | |
| 35 | 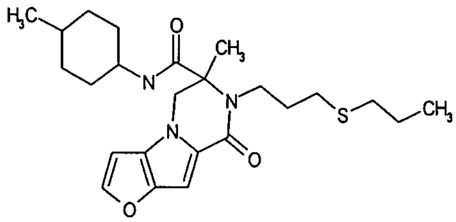 | 445,62885 | 446 | |
| 36 | 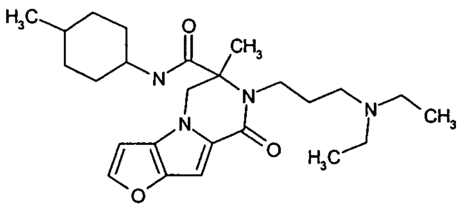 | 442,60661 | 443 | |
| 37 | 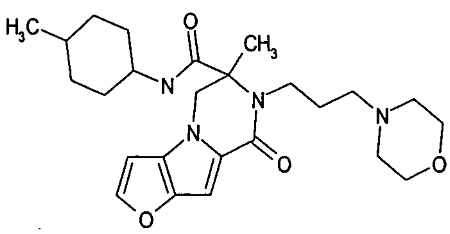 | 456,59007 | 457 | |
| 38 | 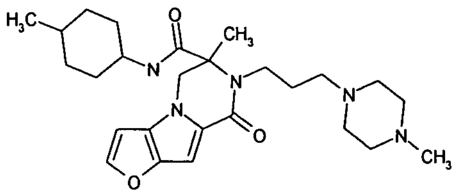 | 469,63243 | 470 | |
| 39 | 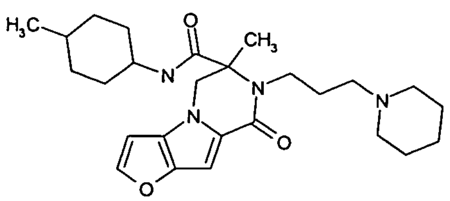 | 454,61776 | 455 | |

(continued)

| | Table 1.17(5) | | | |
|---|---|---|---|---|
| № | Formula | Mol.w. | LC MS, m/z (M+1) | 1H NMR |
| 40 | 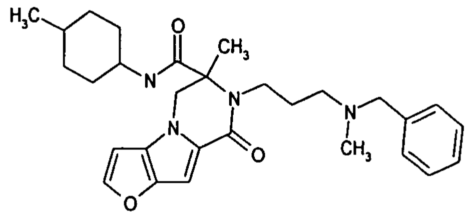 | 490,65121 | 491 | |
| 41 | 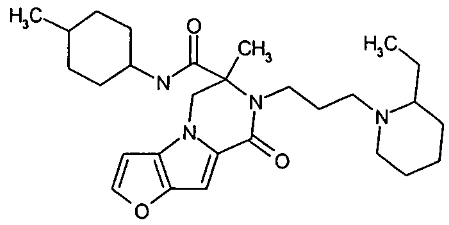 | 482,67194 | 483 | |
| 42 | 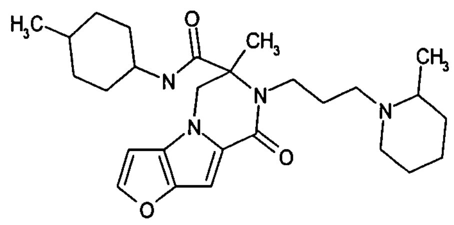 | 468,64485 | 469 | |
| 43 | 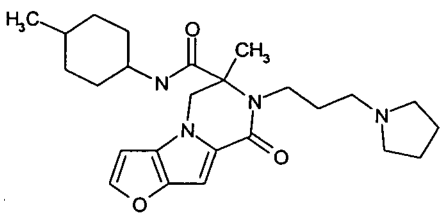 | 440,59067 | 441 | |
| 44 | 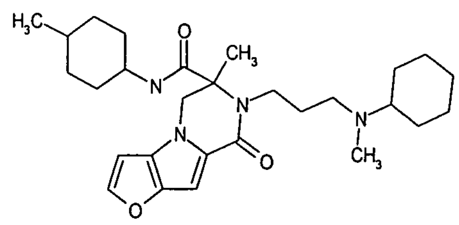 | 482,67194 | 483 | |
| 45 | 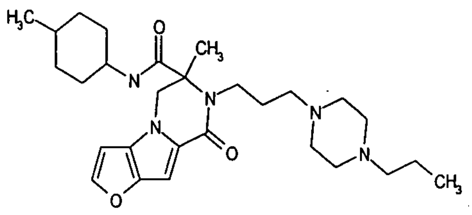 | 497,68661 | 498 | |

(continued)

| | Table 1.17(5) | | | |
|---|---|---|---|---|
| № | Formula | Mol.w. | LC MS, m/z (M+1) | 1H NMR |
| 46 | 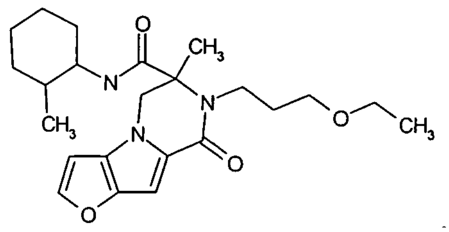 | 415,53716 | 416 | |
| 47 | 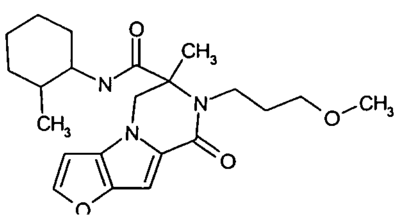 | 401,51007 | 402 | |
| 48 | 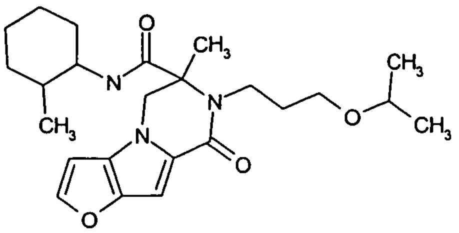 | 429,56425 | 430 | |
| 49 | 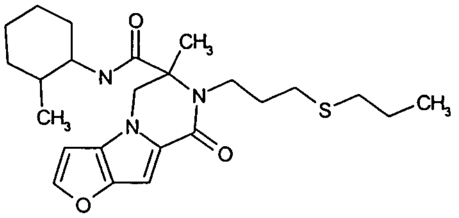 | 445,62885 | 446 | |
| 50 | 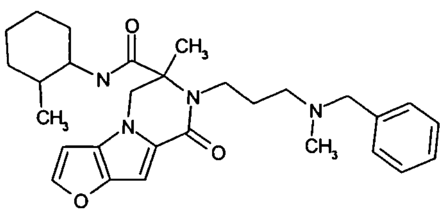 | 490,65121 | 491 | |
| 51 | 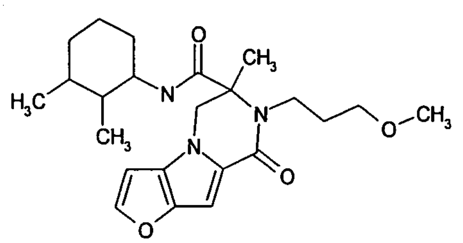 | 415,53716 | 416 | |

(continued)

| | Table 1.17(5) | | | |
|---|---|---|---|---|
| № | Formula | Mol.w. | LC MS, m/z (M+1) | 1H NMR |
| 52 | 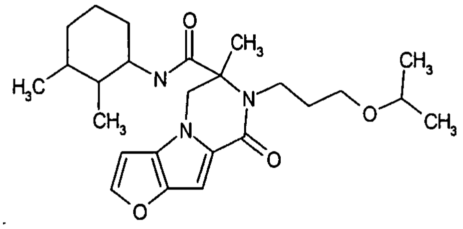 | 443,59134 | 444 | |
| 53 | 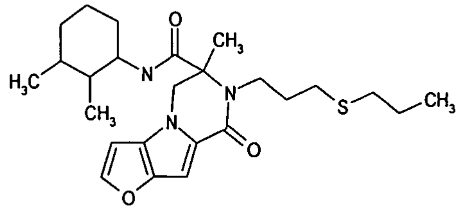 | 459,65594 | 460 | |
| 54 | 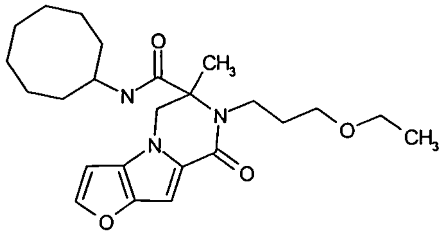 | 429,56425 | 430 | |
| 55 | 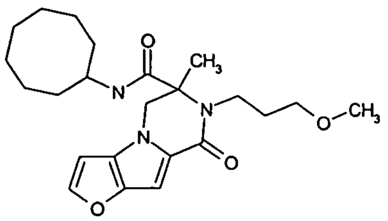 | 415,53716 | 416 | |
| 56 | 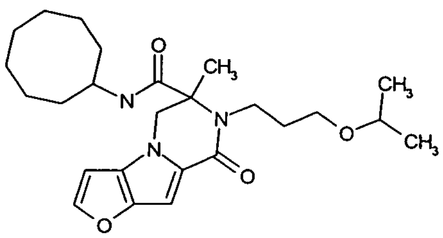 | 443,59134 | 444 | |
| 57 | 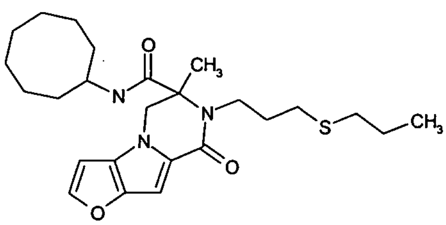 | 459,65594 | 460 | |

(continued)

| | Table 1.17(5) | | | |
|---|---|---|---|---|
| № | Formula | Mol.w. | LC MS, m/z (M+1) | 1H NMR |
| 58 | 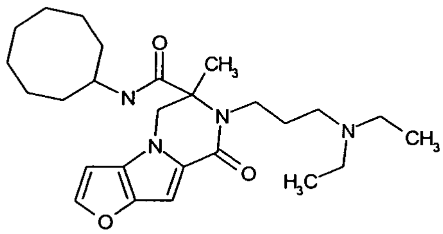 | 456,6337 | 457 | |
| 59 | 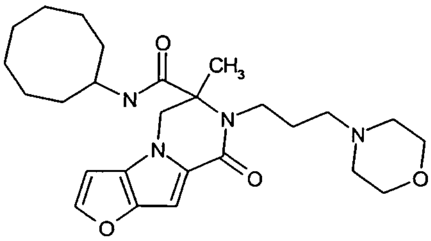 | 470,61716 | 471 | |
| 60 | 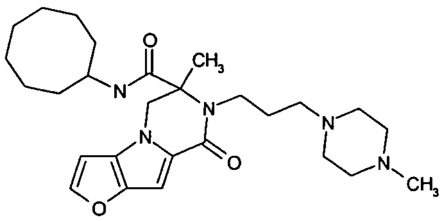 | 483,65952 | 484 | |
| 61 | 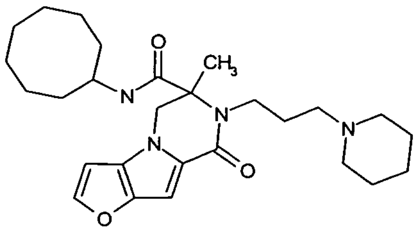 | 468,64485 | 469 | |
| 62 | 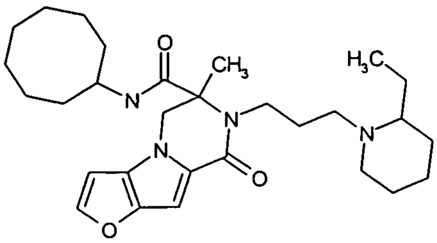 | 496,69903 | 497 | |
| 63 | 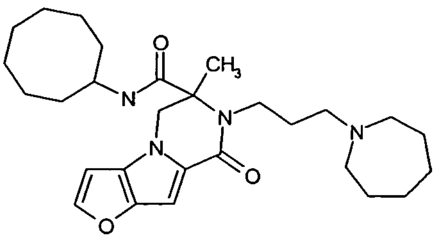 | 482,67194 | 483 | |

(continued)

| | Table 1.17(5) | | | |
|---|---|---|---|---|
| № | Formula | Mol.w. | LC MS, m/z (M+1) | 1H NMR |
| 64 | 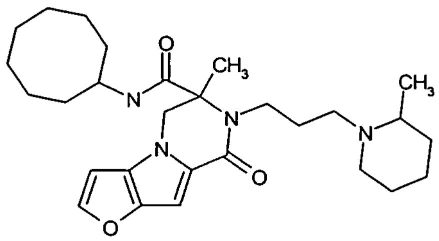 | 482,67194 | 483 | |
| 65 | 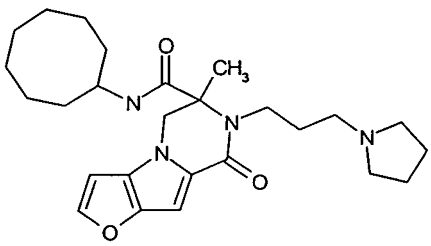 | 454,61776 | 455 | |
| 66 | 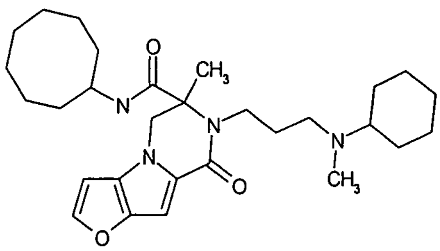 | 496,69903 | 497 | |
| 67 | 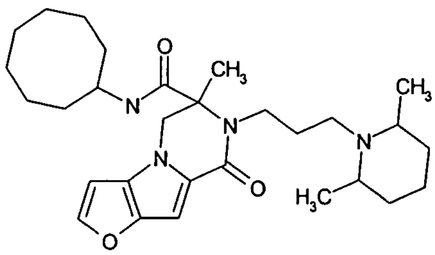 | 496,69903 | 497 | |
| 68 | 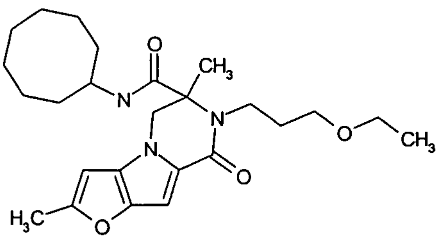 | 443,59134 | 444 | |
| 69 | 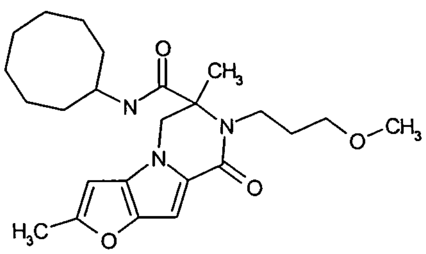 | 429,56425 | 430 | |

(continued)

| | Table 1.17(5) | | | |
|---|---|---|---|---|
| № | Formula | Mol.w. | LC MS, m/z (M+1) | 1H NMR |
| 70 | 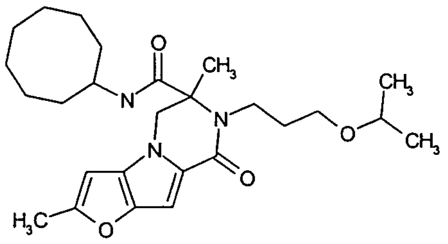 | 457,61843 | 458 | |
| 71 | 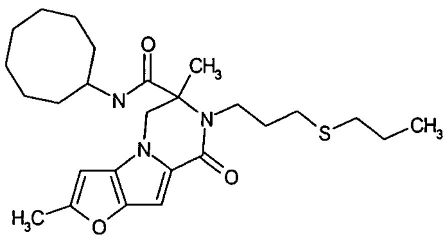 | 473,68303 | 474 | |
| 72 | 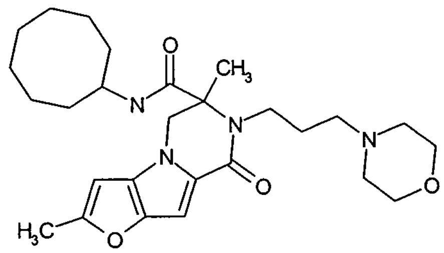 | 484,64425 | 485 | |
| 73 | 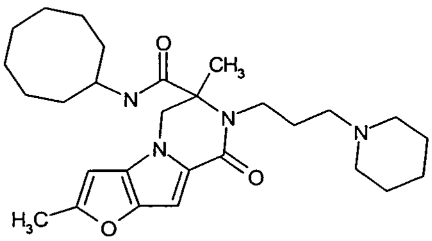 | 482,67194 | 483 | |
| 74 | 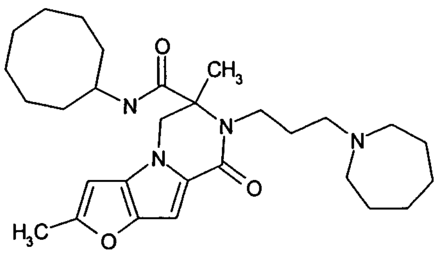 | 496,69903 | 497 | |
| 75 | 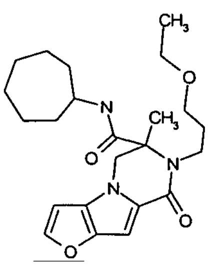 | 415,53716 | 416 | |

(continued)

| | Table 1.17(5) | | | |
|---|---|---|---|---|
| № | Formula | Mol.w. | LC MS, m/z (M+1) | 1H NMR |
| 76 | 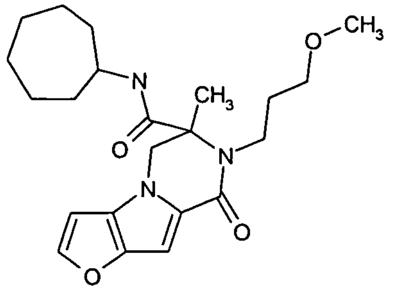 | 401,51007 | 402 | |
| 77 | 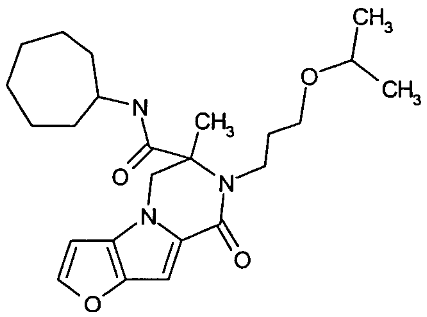 | 429,56425 | 430 | |
| 78 | 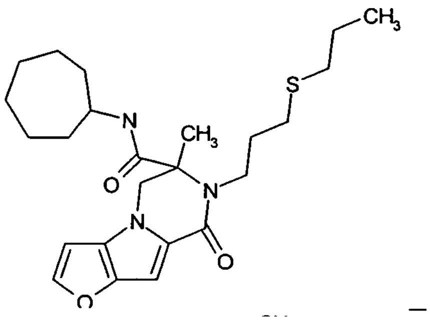 | 445,62885 | 446 | |
| 79 | 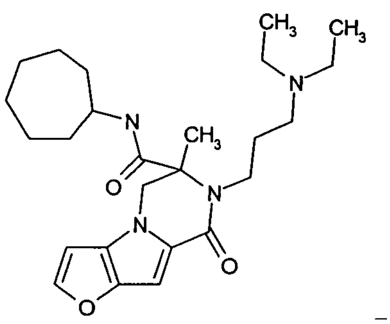 | 442,60661 | 443 | |
| 80 | 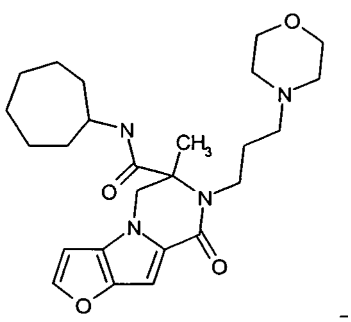 | 456,59007 | 457 | |

(continued)

| | Table 1.17(5) | | | |
|---|---|---|---|---|
| № | Formula | Mol.w. | LC MS, m/z (M+1) | 1H NMR |
| 81 | 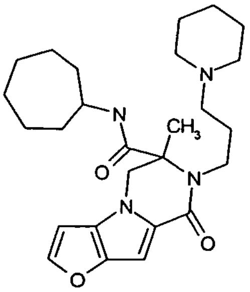 | 454,61776 | 455 | |
| 82 | 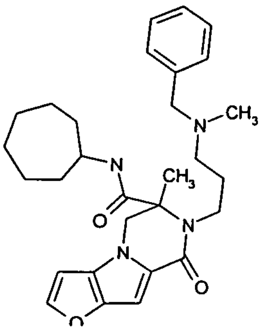 | 490,65121 | 491 | |
| 83 | 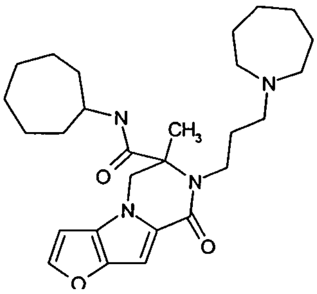 | 468,64485 | 469 | |
| 84 | 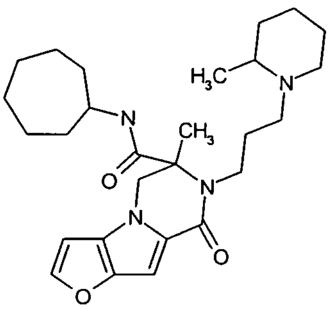 | 468,64485 | 469 | |
| 85 | 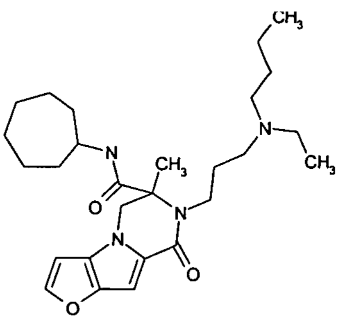 | 470,66079 | 471 | |

(continued)

| | Table 1.17(5) | | | |
|---|---|---|---|---|
| № | Formula | Mol.w. | LC MS, m/z (M+1) | 1H NMR |
| 86 | 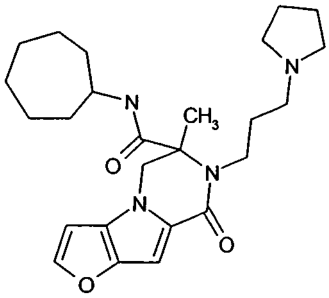 | 440,59067 | 441 | |
| 87 | 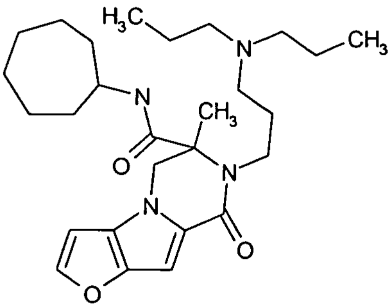 | 470,66079 | 471 | |
| 88 | 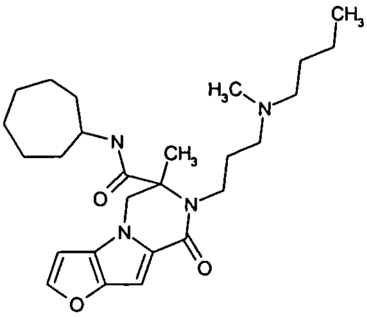 | 456,6337 | 457 | |
| 89 | 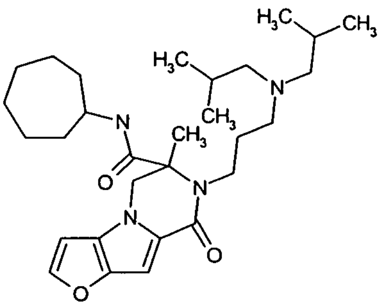 | 498,71497 | 499 | |
| 90 | 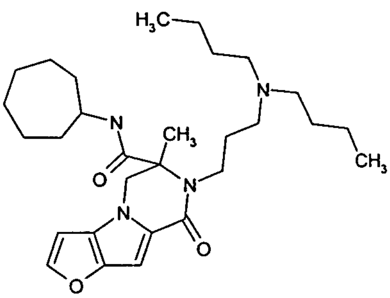 | 498,71497 | 499 | |

(continued)

| | Table 1.17(5) | | | |
|---|---|---|---|---|
| № | Formula | Mol.w. | LC MS, m/z (M+1) | 1H NMR |
| 91 | 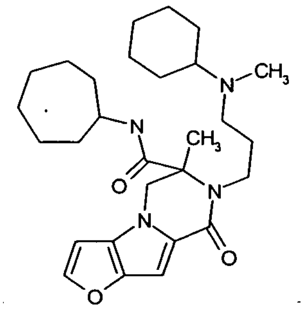 | 482,67194 | 483 | |
| 92 | 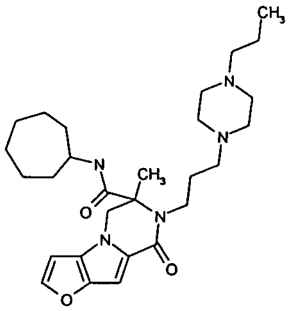 | 497,68661 | 498 | |
| 93 | 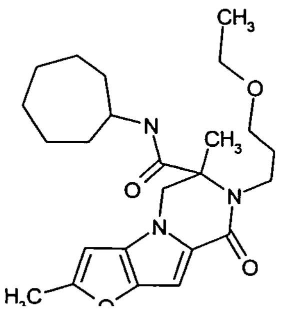 | 429,56425 | 430 | |
| 94 | 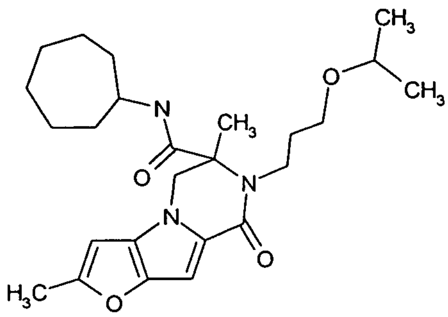 | 443,59134 | 444 | |
| 95 | 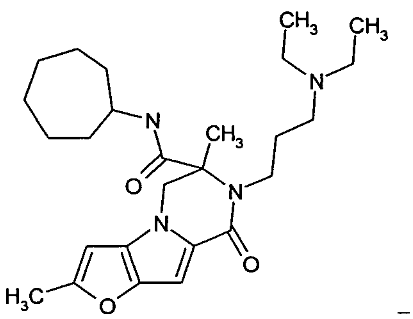 | 456,6337 | 457 | |

(continued)

| | Table 1.17(5) | | | |
|---|---|---|---|---|
| № | Formula | Mol.w. | LC MS, m/z (M+1) | 1H NMR |
| 96 | 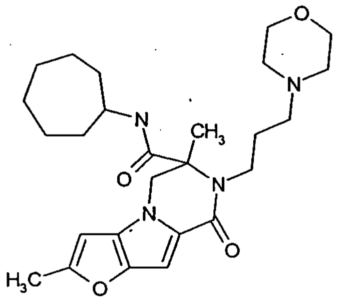 | 470,61716 | 471 | |
| 97 | 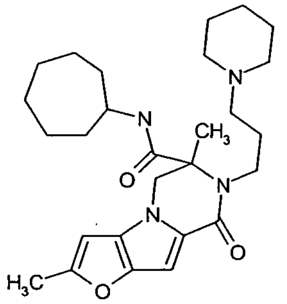 | 468,64485 | 469 | |
| 98 | 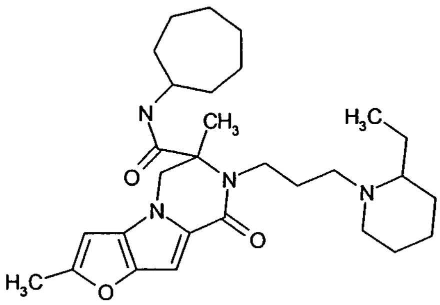 | 496,69903 | 497 | |
| 99 | 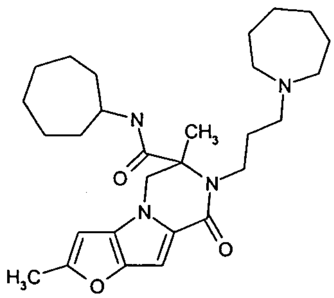 | 482,67194 | 483 | |

(continued)

| | Table 1.17(5) | | | |
|---|---|---|---|---|
| № | Formula | Mol.w. | LC MS, m/z (M+1) | 1H NMR |
| 100 | 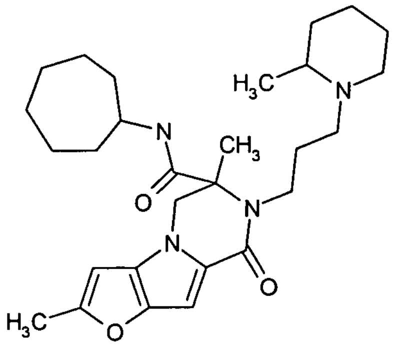 | 482,67194 | 483 | |
| 101 | 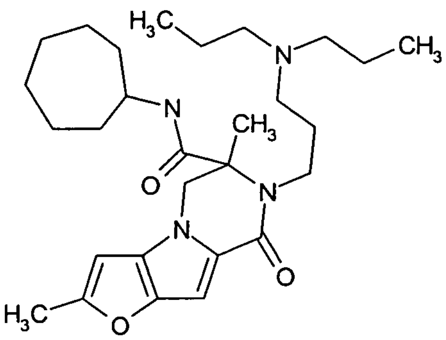 | 484,68788 | 485 | |
| 102 | 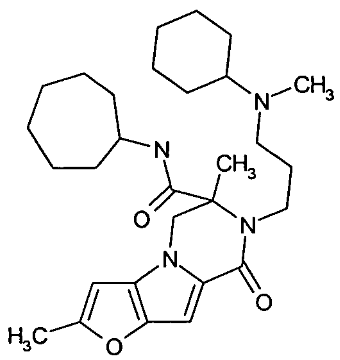 | 496,69903 | 497 | |

| | Table 1.17(6) | | |
|---|---|---|---|
| № | Formula | Mol.w. | LC MS,m/z (M+1) |
| 1 | 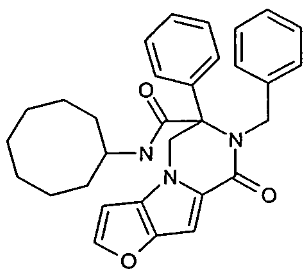 | 495,62696 | 496 |

(continued)

| | Table 1.17(6) | | |
|---|---|---|---|
| № | Formula | Mol.w. | LC MS,m/z (M+1) |
| 2 | 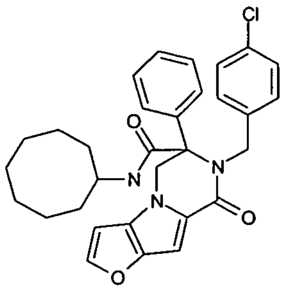 | 530,07199 | 531 |
| 3 | 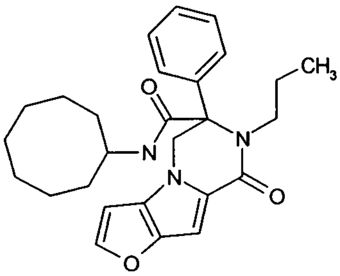 | 447,58236 | 448 |
| 4 | 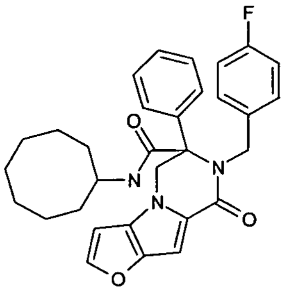 | 513,61739 | 514 |
| 5 | 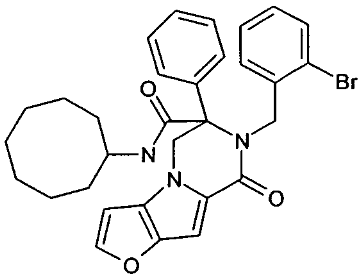 | 574,52299 | 575 |
| 6 | 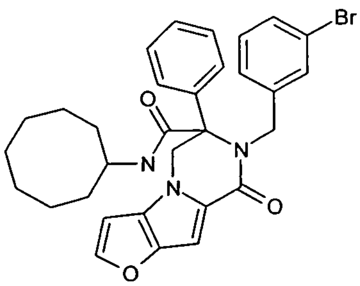 | 574,52299 | 575 |

(continued)

| | Table 1.17(6) | | |
|---|---|---|---|
| № | Formula | Mol.w. | LC MS,m/z (M+1) |
| 7 | 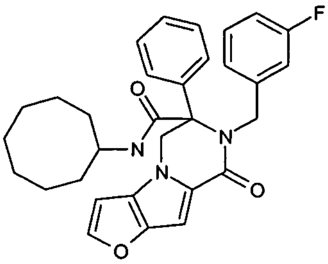 | 513,61739 | 514 |
| 8 | 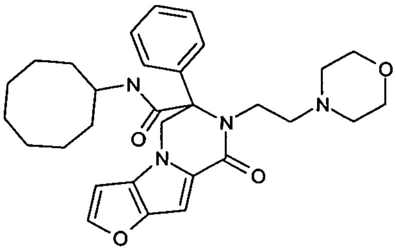 | 518,66176 | 519 |
| 9 | 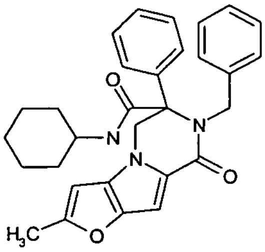 | 481,59987 | 482 |
| 10 | 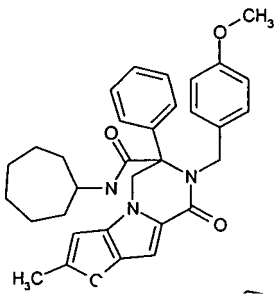 | 525,65345 | 526 |
| 11 | 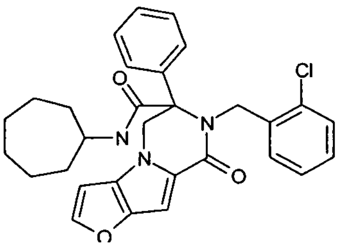 | 516,0449 | 517 |

(continued)

| | Table 1.17(6) | | |
|---|---|---|---|
| № | Formula | Mol.w. | LC MS,m/z (M+1) |
| 12 | 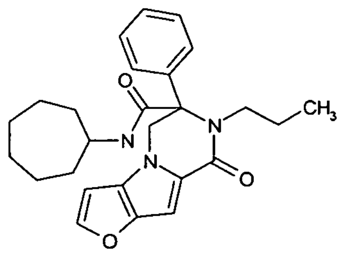 | 433,55527 | 434 |
| 13 | 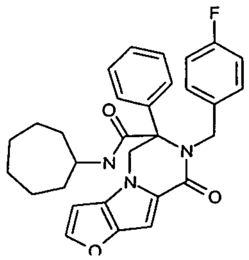 | 499,5903 | 500 |
| 14 | 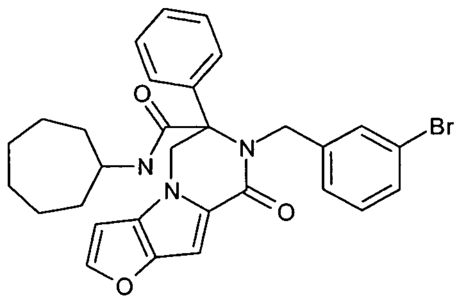 | 560,4959 | 561 |
| 15 | 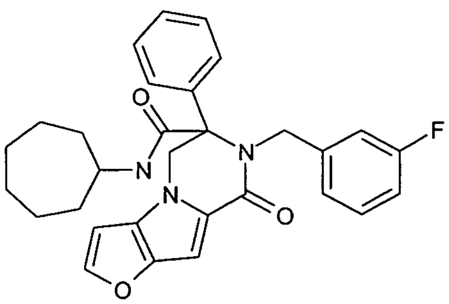 | 499,5903 | 500 |
| 16 | 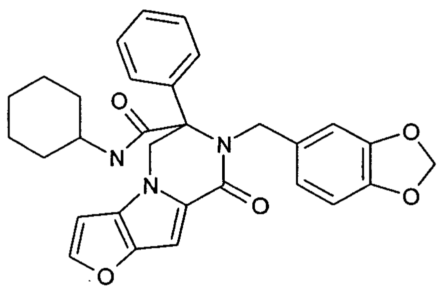 | 511,58273 | 512 |

(continued)

| | Table 1.17(6) | | |
|---|---|---|---|
| № | Formula | Mol.w. | LC MS,m/z (M+1) |
| 17 | 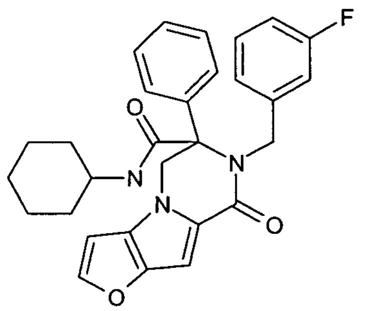 | 485,56321 | 486 |

**Table 1.18.**

| № | Formula | Spectral data |
|---|---|---|
| 1 | 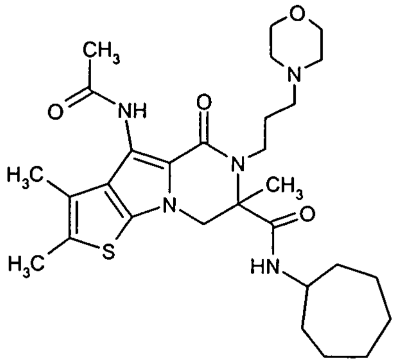 | $^1$H-NMR (400 MHz, DMSO-*d*$_6$+$CCl_4$): δ 9.0 (s, 1H), 7.3 (d, *J* = 6.4 Hz, 1H), 4.48 (d, *J* = 5.4 Hz, 1H), 3.81 (d, *J* = 5.4 Hz, 1H), 3.7-3.4 (m, 6H), 3.22-3.1 (m, 1H), 2.4-2.2 (m, 5H), 2.3 (s, 3H), 2.12 (s, 3H), 2.01 (s, 3H), 1.8-1.3 (m, 15H), 1.5 (s, 3H); $^{13}$C-NMR (400 MHz, DMSO-*d*$_6$): δ 169.0, 159.6, 130.8, 127.8, 126.1, 123.7, 119.0, 117.8, 66.1, 64.3, 55.9, 53.2, 51.9, 50.5, 34.0, 33.9, 27.6, 27.5, 26.5, 23.7, 22.8, 20.5, 13.4, 11.7; HRMS (*m*/*z*): $[M]^+$ calcd for $C_{29}H_{43}N_5O_4S$, 557.7612; found, 557.7608. |
| 2 | 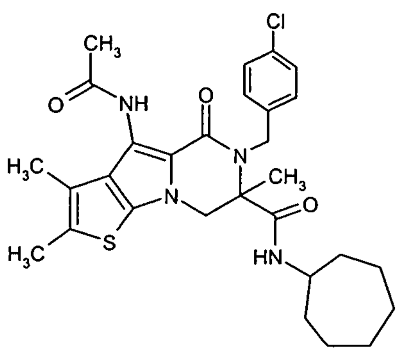 | $^1$H-NMR (400 MHz, DMSO-*d*$_6$+$CCl_4$): δ 9.12 (s, 1H), 7.26-7.1 (m, 5H), 5.12 (d, *J*= 5.6 Hz, 1H), 4.50 (d, *J* = 5.6 Hz, 1H), 4.15 (d, *J* = 5.4 Hz, 1H), 3.93 (d, *J* = 5.4 Hz, 1H), 3.61-3.58 (m, 1H), 2.38 (s, 3H), 2.13 (s, 3H), 2.0 (s, 3H), 1.76-1.27 (m, 12H), 1.47 (s, 3H); $^{13}$C-NMR (400 MHz, DMSO-*d*$_6$): δ 169.0, 160.1, 139.5, 131.3, 130.8, 128.5, 128.0, 126.3, 123.8, 118.8, 118.3, 64.7, 51.9, 50.5, 45.0, 34.06, 33.9, 27.7, 27.6, 23.7, 23.6, 22.8, 20.8, 13.5, 11.8; HRMS (*m*/*z*): $[M]^+$ calcd for $C_{29}H_{35}ClN_4O_3S$, 555.1443; found, 555.1437. |
| 3 | 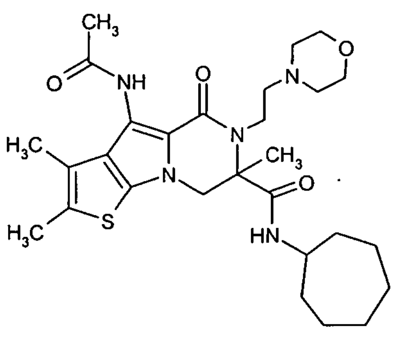 | $^1$H-NMR (400 MHz, DMSO-*d*$_6$+$CCl_4$): δ 9.1 (s, 1H), 7.38 (br s, 1H), 4.49 (d, *J* = 5.6 Hz, 1H), 3.83 (d, *J* = 5.6 Hz, 1H), 3.62-3.34 (m, 8H), 2.3 (s, 3H), 2.11 (s, 3H), 2.01 (s, 3H), 1.63-1.28 (m, 12H), 1.51 (s, 3H); $^{13}$C-NMR (400 MHz, DMSO-*d*$_6$): δ 169.1, 159.6, 130.9, 127.8, 126.1, 123.7, 119.0, 117.9, 66.1, 64.2, 57.7, 53.6, 51.9, 50.6, 34.0, 27.5, 23.8, 22.8, 20.4, 13.5, 11.7; HRMS (*m*/*z*): $[M]^+$ calcd for $C_{28}H_{41}N_5O_4S$, 543.7341; found, 543.73471. |

(continued)

| № | Formula | Spectral data |
|---|---|---|
| 4 | 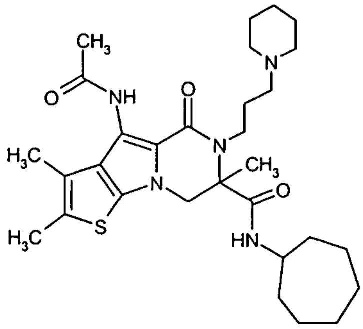 | : $^{1}$H-NMR (400 MHz, DMSO-$d_6$+$CCl_4$): δ 9.0 (s, 1H), 7.3 (d, $J$ = 6.2 Hz, 1H), 4.48 (d, $J$ = 5.4 Hz, 1H), 3.82 (d, $J$ = 5.4 Hz, 1H), 3.71-3.6 (m, 1H), 3.55-3.42 (m, 1H), 3.22-3.1 (m, 1H), 2.4-1.9 (m, 6H), 2.31 (s, 3H), 2.13 (s, 3H), 2.04 (s, 3H), 1.91-1.33 (m, 20H), 1.56 (s, 3H); $^{13}$C-NMR (400 MHz, DMSO-$d_6$): δ 169.0, 159.7, 130.9, 127.8, 126.1, 123.7, 119.0, 117.8, 64.3, 55.9, 53.7, 51.9, 50.6, 34.0, 33.9, 27.6, 26.6, 25.3, 25.2, 23.7, 22.8, 20.5, 13.5, 11.8; HRMS ($m/z$): [M]$^+$ calcd for $C_{30}H_{45}N_5O_3S$, 555.7889; found, 555.7882. |
| 5 | 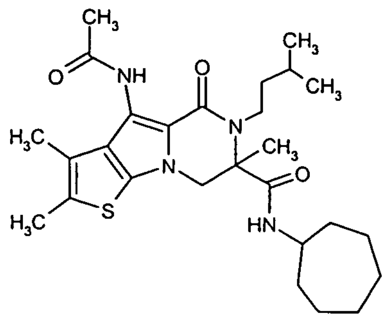 | : $^{1}$H-NMR (400 MHz, DMSO-$d_6$+$CCl_4$): δ 9.04 (s, 1H), 7.31 (d, $J$ = 4.6 Hz, 1H), 4.49 (d, $J$ = 5.6 Hz, 1H), 3.84 (d, $J$= 5.6 Hz, 1H), 3.75-3.6 (m, 1H), 3.55-3.44 (m, 1H), 3.23-3.1 (m, 1H), 2.3 (s, 3H), 2.12 (s, 3H), 2.02 (s, 3H), 1.71-1.32 (m, 15H), 1.55 (s, 3H), 1.00 (s, 6H); $^{13}$C-NMR (400 MHz, DMSO-$d_6$): δ 169.0, 159.4, 130.8, 127.8, 126.0, 123.8, 119.0, 117.8, 64.3, 51.9, 50.5, 34.0, 33.9, 27.6,27.6, 26.1, 23.7, 22.8, 22.5, 22.4, 20.4, 13.5, 11.8; HRMS ($m/z$): [M]$^+$ calcd for $C_{27}H_{40}N_4O_3S$, 500.7089; found, 500.7094. |
| 6 | 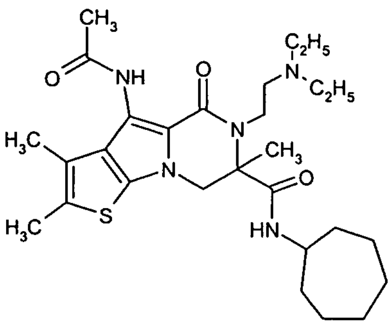 | $^{1}$H-NMR (400 MHz, DMSO-$d_6$+$CCl_4$): δ 9.00 (s, 1H), 7.33 (d, $J$ = 4.4 Hz, 1H), 4.50 (d, $J$ = 5.6 Hz, 1H), 3.82 (d, $J$= 5.6 Hz, 1H), 3.71-3.52 (m, 2H), 3.24-3.16 (m, 1H), 2.61-2.48 (m, 7H), 2.31 (s, 3H), 2.12 (s, 3H), 2.00 (s, 3H), 1.73-1.34 (m, 12H), 1.53 (s, 3H), 1.02 (t, $J$=4.2 Hz, 6H); $^{13}$C-NMR (400 MHz, DMSO-$d_6$): δ 169.0, 159.6, 130.9, 127.8, 126.1, 123.8, 118.9, 117.9, 64.2, 51.9, 51.8, 50.6, 46.9, 41.2, 34.0, 27.6, 27.5, 23.8, 22.8, 20.5, 13.5, 12.0, 11.8; HRMS ($m/z$): [M]$^+$ calcd for $C_{28}H_{43}N_5O_3S$, 529.7506; found, 529.7497. |
| 7 | 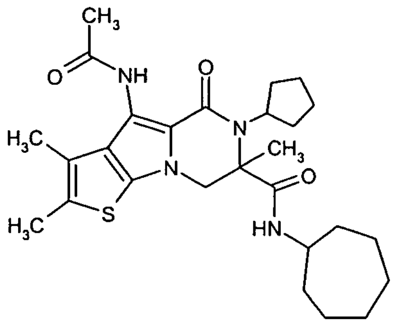 | $^{1}$H-NMR (400 MHz, DMSO-$d_6$+$CCl_4$): δ 9.00 (s, 1H), 7.32 (d, $J$= 4.4 Hz, 1H), 4.48 (d, $J$= 5.4 Hz, 1H), 3.77 (d, $J$ = 5.4 Hz, 1H), 3.22-3.13 (m, 1H), 2.32 (s, 3H), 2.13 (s, 3H), 2.02 (s, 3H), 2.00-11.4 (m, 20H), 1.41 (s, 3H); $^{13}$C-NMR (400 MHz, DMSO-$d_6$): δ 168.7, 158.2, 131.0, 128.2, 126.3, 123.7, 120.4, 119.7, 118.1, 65.9, 58.9, 57.5, 51.2, 50.6, 33.9, 33.8, 29.5, 27.8, 27.7, 25.4, 24.8, 23.8, 23.7, 22.9, 20.6, 13.5, 11.7; HRMS ($m/z$): [M]$^+$ calcd for $C_{27}H_{38}N_4O_3S$, 498.6929; found, 498.6922. |

(continued)

| № | Formula | Spectral data |
|---|---|---|
| 8 | 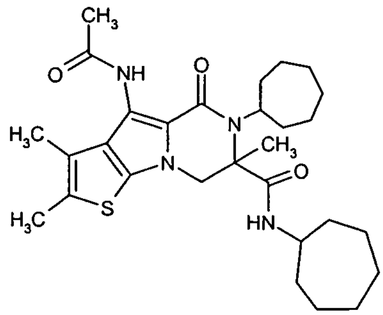 | $^1$H-NMR (400 MHz, DMSO-$d_6$+$CCl_4$): δ 9.0 (s, 1H), 7.31 (d, $J$ = 4.0 Hz, 1H), 4.49 (d, $J$ = 5.6 Hz, 1H), 3.82 (d, $J$ = 5.6 Hz, 1H), 3.71-3.6 (m, 1H), 3.6-3.49 (m, 1H), 3.23-3.16 (m, 1H), 2.61-2.5 (m, 6H), 2.30 (s, 3H), 2.12 (s, 3H), 2.02 (s, 3H), 1.86-1.31 (m, 20H), 1.53 (s, 3H); $^{13}$C-NMR (400 MHz, DMSO-$d_6$): δ 169.1, 159.6, 130.9, 127.8, 126.1, 123.7, 118.9, 117.9, 64.2, 56.8, 55.1, 51.8, 50.6, 34.0, 27.9, 27.6, 27.5, 26.5, 23.8, 22.8, 20.5, 13.5, 11.8; HRMS ($m/z$): $[M]^+$ calcd for $C_{30}H_{45}N_5O_3S$, 555.7889; found, 555.7895. |
| 9 | 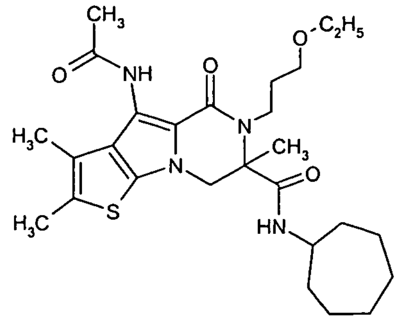 | $^1$H-NMR (400 MHz, DMSO-$d_6$+$CCl_4$): δ 9.04 (s, 1H), 7.28 (d, $J$ = 4.2 Hz, 1H), 4.48 (d, $J$= 5.6 Hz, 1H), 3.82 (d, $J$ = 5.6 Hz, 1H), 3.70-3.6 (m, 1H), 3.59-3.49 (m, 1H), 3.49-3.3 (q, $J$= 4.6 Hz, 2H), 3.24-3.12 (m, 1H), 2.33 (s, 3H), 2.14 (s, 3H), 2.03 (s, 3H), 1.91-1.30 (m, 14H), 1.51 (s, 3H), 1.13 (t, $J$ = 4.6 Hz, 3H); $^{13}$C-NMR (400 MHz, DMSO-$d_6$): δ 169.0, 159.7, 130.8, 127.8, 126.1, 123.7, 119.0, 117.8, 67.8, 65.2, 64.4, 51.9, 50.5, 34.0, 33.9, 29.9, 27.6, 27.6, 23.7, 22.8, 20.5, 15.1, 13.5, 11.8; HRMS ($m/z$): $[M]^+$ calcd for $C_{27}H_{40}N_4O_4S$, 516.7083; found, 516.7078. |

**Table 1.19-20**.

| № | Formula | Mol. w. | Spectral data |
|---|---|---|---|
| 1.19(1) | 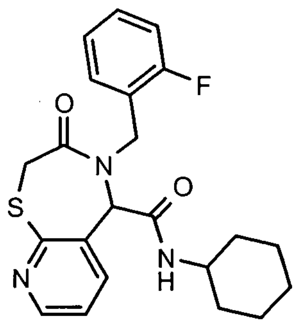 | 413,52 | Масс-спектр: m/z 414 (M+1) |
| 1.20(1) | 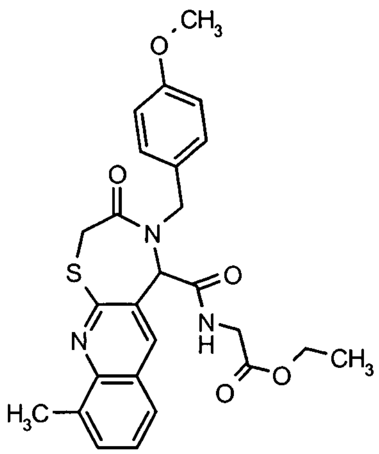 | 493,59 | $^1$H ЯМР (DMSO $d_6$, δ): 8.22 t ($J$ = 5.9 Hz, 1H), 7.89 s (1H), 7.53-7.56 m (2H), 7.36 t ($J$= 7.3 Hz, 1H), 7.12-7.14 m (2H), 6.60-6.62 m (2H), 5.57 s (1H), 5.13 d ($J$ = 14.6 Hz, 1H), 4.52 d ($J$ = 15.0, 1H), 4.26 d ($J$ = 14.6 Hz, 1H), 4.10 q ($J$ = 6.9 Hz, 2H), 3.88 dd ($J$ = 17.2, 5.9 Hz, 1H), 3.75 dd ($J$ = 17.2, 5.9 Hz, 1H), 3.50 s (3H), 3.33 d ($J$ = 15.0 Hz, 1H), 2.58 s (3H), 1.18 t ($J$ = 6.9 Hz, 3H); $^{13}$C ЯМР (DMSO $d_6$, δ): 169.8, 169.7, 169.3, 158.5, 157.0, 145.6, 140.6, 134.4, 130.7, 129.5 (2C), 129.4, 125.9, 125.8, 125.5, 125.2, 113.6 (2C), 65.4, 60.9, 55.0, 51.7, 41.8,31.8,17.5, 14.2. |

(continued)

| № | Formula | Mol. w. | Spectral data |
|---|---|---|---|
| 1.20(2) | 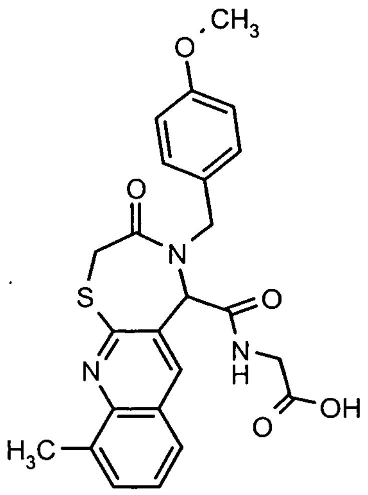 | 465,53 | Масс-спектр: m/z 466 (M+1) |

**Table 1.21-22.**

| № | Formula | Mol. w. | Spectral data |
|---|---|---|---|
| 1. | 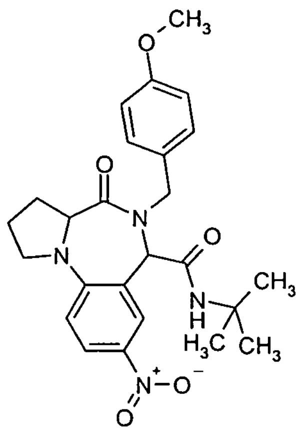 | 466,564 | Масс-спектр: m/z 467(M+H). |
| 1. | 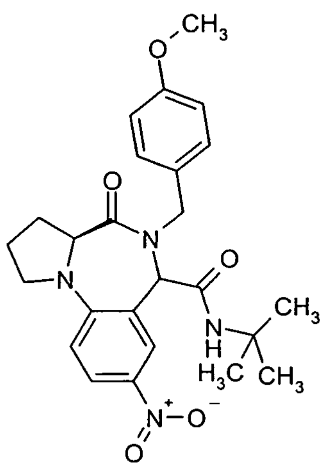 | 466,564 | Масс-спектр: m/z 467(M+H). |

(continued)

| № | Formula | Mol. w. | Spectral data |
|---|---|---|---|
| 1. | 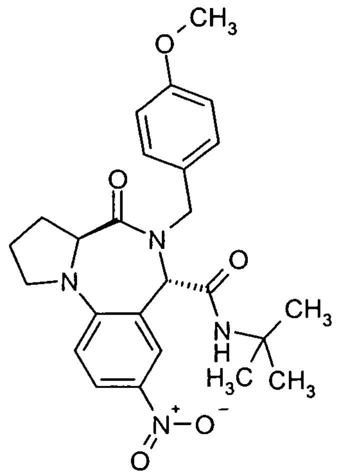 | 466,564 | NMR-$^1$H ($CDCl_3$): dd 8.06 (1H, J=9.5 Hz, J=2.9 Hz), d 7.82 (1H, J=2.9 Hz), d 7.33 (2H, J=8.8 Hz), d 6.90 (2H, J=8.8 Hz), d 6.53 (1H, J=9.5 Hz), d 5.17 (1H, J=14.3), br.s 5.11 (1H), s 4.64 (1H), m 4.41-4.45 (1H), d 4.09 (1H, J=14.3), s 3.60 (3H), m 3.46-3.52 (1H), m 3.34-3.44 (1H), m 2.68-2.76 (1H), m 1.96-2.16 (2H), s 1.08 (9H). Масс-спектр: m/z 467(M+H). |
| | 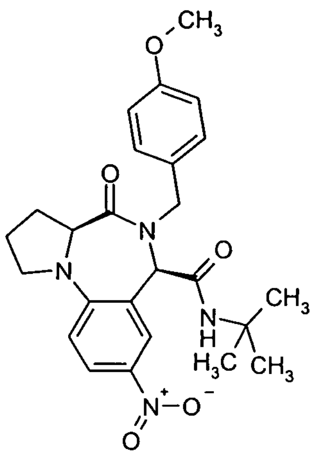 | 466,564 | NMR-$^1$H ($CDCl_3$): dd 8.20 (1H, J=8.8 Hz, J=2.6 Hz), d 7.70 (1H, J=2.6 Hz), d 7.24 (2H, J=8.4 Hz), d 7.01 (1H, J=8.8 Hz), d 6.90 (2H, J=8.4 Hz), d 5.16 (1H, J=14.7), br.s 4.94 (1H), s 4.68 (1H), d 4.43 (1H, J=14.7), s 3.83 (3H), m 3.68-3.77 (1H), m 3.27-3.34 (1H), m 3.18-3.24 (1H), m 2.51-2.62 (1H), m 2.31-2.44 (1H), m 1.87-2.05 (2H), s 1.14 (9H). Масс-спектр: m/z 467(M+H). |
| | 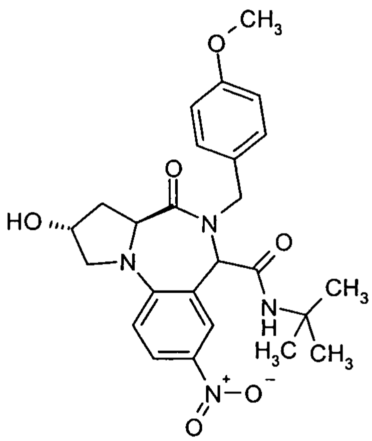 | 482,54 | Масс-спектр: m/z 483(M+H). |

| | Table 1.23 | | |
|---|---|---|---|
| № | Formula | Mol.w. | LC MS, m/z (M+1), 1H NMR |
| 1 | 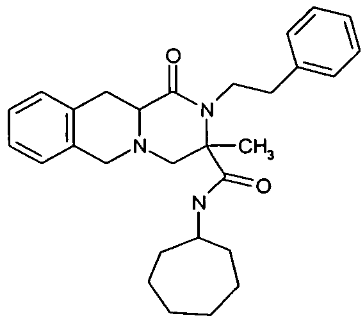 | 459,63714 | 460 |
| 2 | 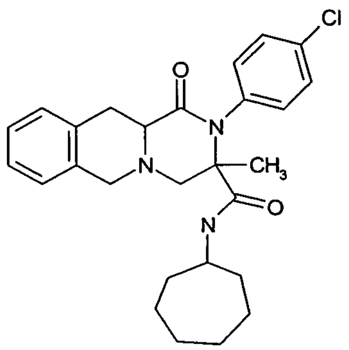 | 466,02799 | 467 |
| 3 | 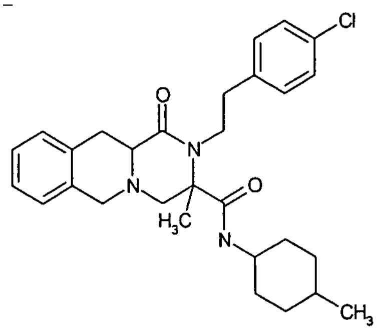 | 494,08217 | LC MS, m/z: 495 (M+1). 1H NMR: 0.79 -1.08 (m, 4H, H and CH3 at -CH-Me of 4-Me-cyclohexil ring); 1.10 - 1.88 (m, 11 H, CH3 of N-C-Me and 4 - CH2 - of 4-Me-cyclohexil ring); 2.68 - 2.93 (m, 6H, H at C11 of 1,3,4,6,11,11 a-hexahydro-2H-pyrazino[1,2-b] isoquinoline , 4H of N-C... |
| 4 | 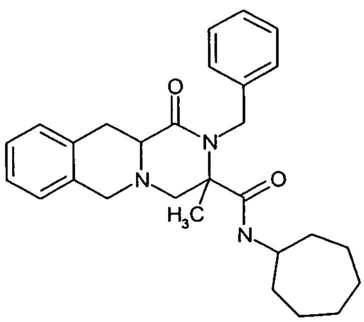 | 445,61005 | LC MS, m/z: 446 (M+1). 1H NMR: 1.25 (s, 3H, CH3); 1.30 - 1.70 (m, 12H, 6 -CH2-, cycloheptyl); 2.68 (d, 1H, H at C11 of 1,3,4,6,11,11a-hexahydro-2H-pyrazino [1,2-b]isoquinoline, J=14.0 Hz); 2.82 (t, 1H, H at C11 of 1,3,4,6,11,11 a-hexahydro-2H-pyrazino[1,2-b] isoquinoline, J=14... |

(continued)

| | Table 1.23 | | |
|---|---|---|---|
| № | Formula | Mol.w. | LC MS, m/z (M+1), 1H NMR |
| 5 | 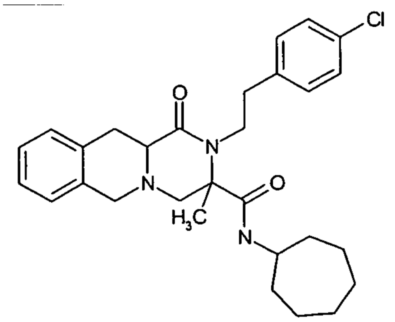 | 494,08217 | LC MS, m/z: 495 (M+1). 1H NMR: 1.10-1.80 (m, 15H, 6 -CH2-, cycloheptyl and CH3); 2.64 - 3.00 (m, 6H, H2 at C11 of 1,3,4,6,11,11 a-hexahydro-2H-pyrazino[1,2-b]isoquinoline and 4H of N-CH2-CH2-Ar); 3.05 - 3.48 (m, 4H, H2 at C4 of 1,3,4,6,11,11a-hexahydro-2H-pyrazino[1,2-b]i... |
| 6 | 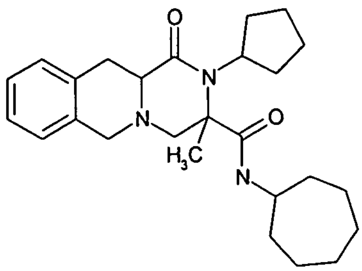 | 423,60369 | LC MS, m/z: 424 (M+1). 1H NMR: 1.10 - 1.95 (m, 23H, 4 -CH2-, cyclopentyl, 6 -CH2 cycloheptyl and CH3); 2.30 (br. s, 1H, CH of cyclopentyl); 2.62 - 2.78 (m, 3H, H at C11a of 1,3,4,6,11,11a-hexahydro-2H-pyrazino [1,2-b]isoquinoline, 2H at C11 of 1,3,4,6,11,11a-hexahydro-... |
| 7 | 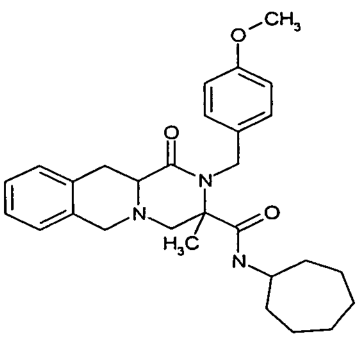 | 475,63654 | LC MS, m/z: 476 (M+1). 1H NMR: 1.15 -1.80 (m, 15H, 6 -CH2-, cycloheptyl and CH3); 2.64 (d, 1 H, H at C11 of 1,3,4,6,11,11 a-hexahydro-2H-pyrazino[1,2-b] isoquinoline, J=14.3 Hz); 2.78 (t, 1H, H at C11 of 1,3,4,6,11,11a-hexah ydro-2H-pyrazino[1,2-b] isoquinoline , J=14.3 Hz); ... |
| 8 | 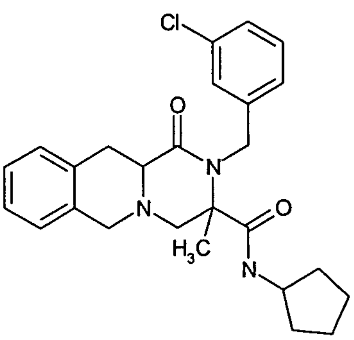 | 452,0009 | LC MS, m/z: 453 (M+1). 1H NMR: 1.05 -1.82 (m, 11H, 4 -CH2-, cyclopentyl and CH3); 2.62 - 2.83 (m, 2H, H at C11 of 1,3,4,6,11,11a-hexahydro-2H-pyrazino[1,2-b]isoquinoline and H at C11a of 1,3,4,6,11,11a-hexahydro-2H-pyrazi no[1,2-b]isoquinoline); 3.10 - 3.41 (m, 3H, H at C... |

(continued)

| | Table 1.23 | | |
|---|---|---|---|
| № | Formula | Mol.w. | LC MS, m/z (M+1), 1H NMR |
| 9 | 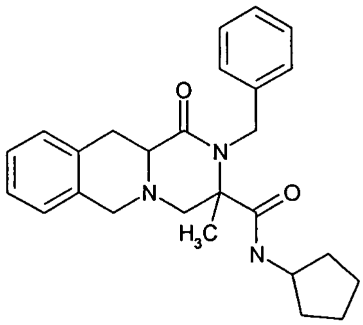 | 417,55587 | LC MS, m/z: 418 (M+1). 1H NMR: 1.10 -1.80 (m, 11H, 4 -CH2-, cyclopentyl and CH3); 2.71 (m, 2H, 2H at C11 of 1,3,4,6,11,11 a-hexahydro-2H-pyrazino[1,2-b] isoquinoline); 3.11 - 3.53 (m, 4H, H at C11a of 1,3,4,6,11,11a-hexahydro-2H-pyrazino[1,2-b] isoquinoline, H at C4 of 1... |

**Table 24.** Percent of inhibition of cell proliferation of three cell lines by the compounds tested.

| № comp. | Formula | Клеточные культуры | | |
|---|---|---|---|---|
| | | DLD-1 | DU-145 | T-47D |
| **1.5**(2){105} | 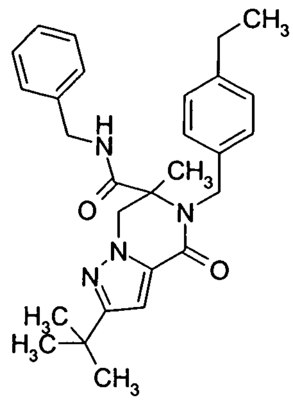 | 46 | 59 | 75 |
| **1.5**(3){44} | 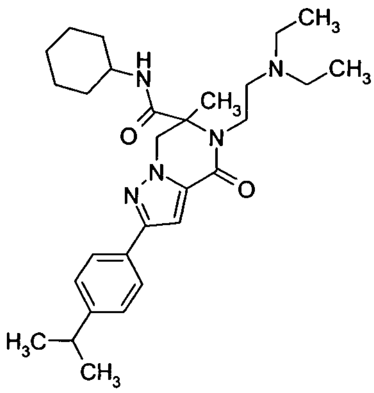 | 98 | 94 | 97 |

(continued)

| № comp. | Formula | Клеточные культуры | | |
|---|---|---|---|---|
| | | DLD-1 | DU-145 | T-47D |
| **1.5**(3){50} | 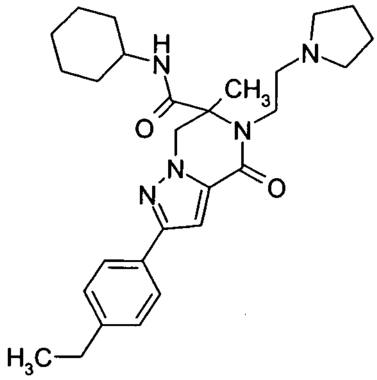 | 98 | 92 | 97 |
| **1.5**(4){130} | 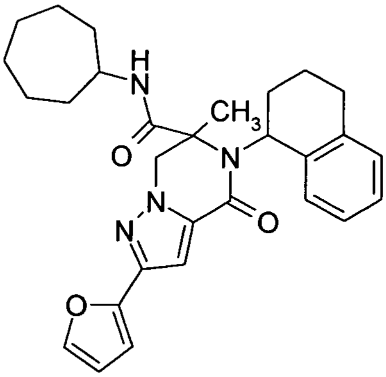 | 18 | 48 | 80 |
| **1.10**(1){18} | 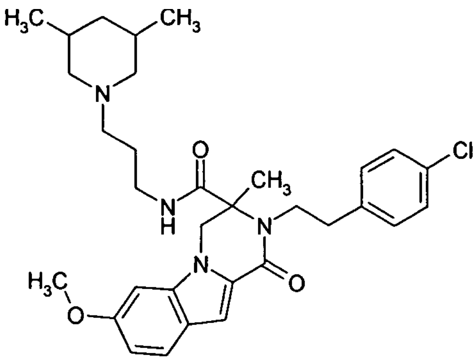 | 97 | 96 | 97 |
| **1.10**(1){19} | 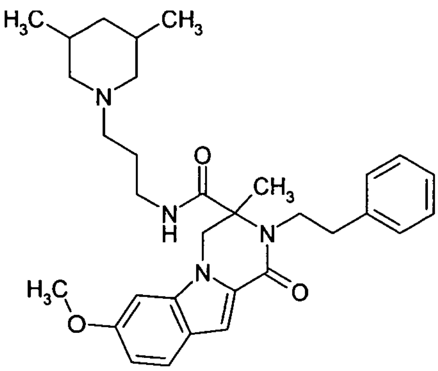 | 33 | 68 | 80 |

(continued)

| № comp. | Formula | Клеточные культуры | | |
|---|---|---|---|---|
| | | DLD-1 | DU-145 | T-47D |
| **1.10**(1){20} | 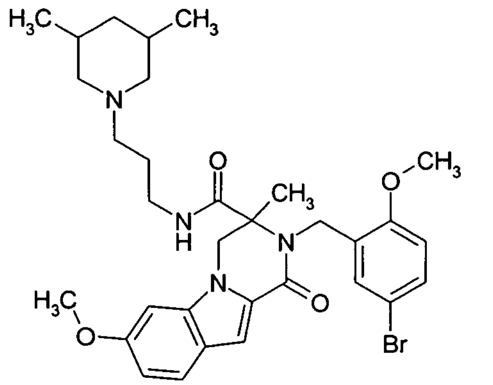 | 78 | 73 | 97 |
| **1.16**(2){28} | 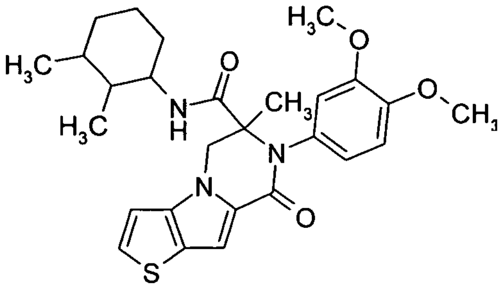 | 97 | 98 | 97 |
| **1.16**(4){51} | 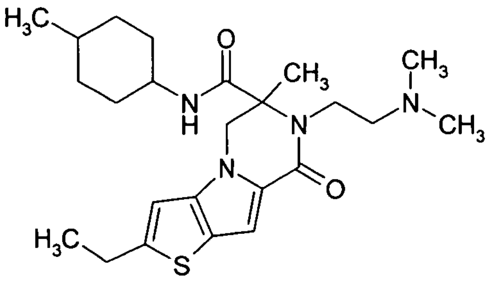 | 62 | 88 | 98 |
| **1.16**(5){38} | 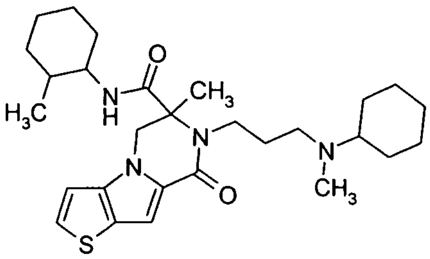 | 3 | 0 | 66 |
| **1.16**(5){66} | 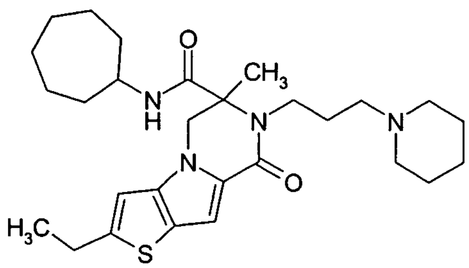 | 5 | 74 | 70 |

(continued)

| № comp. | Formula | Клеточные культуры | | |
|---|---|---|---|---|
| | | DLD-1 | DU-145 | T-47D |
| **1.17**(3){7} | 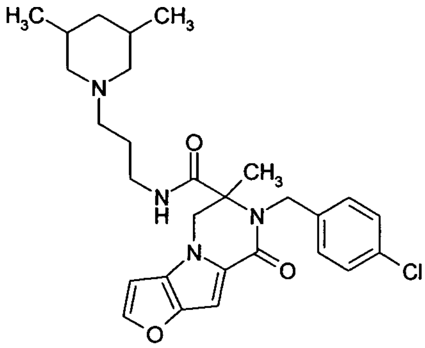 | -2 | 31 | 61 |
| **1.17**(4){46} | 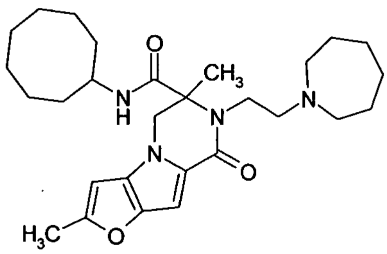 | 7 | 3 | 64 |
| **1.17**(4){47} | 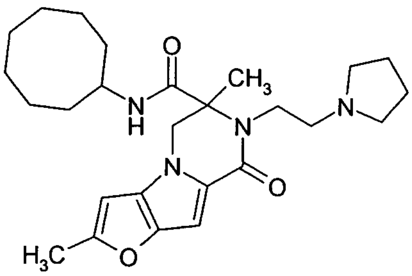 | 5 | 6 | 66 |
| **1.17**(5){102} | 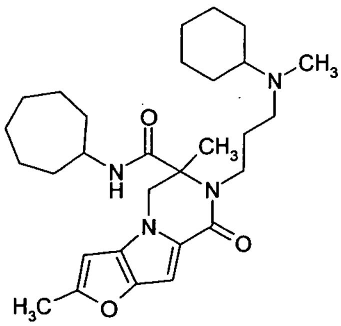 | 8 | 2 | 72 |

## Claims

**1.** Annellated carbamyl-aza-heterocyclic compounds of general formula **1:**

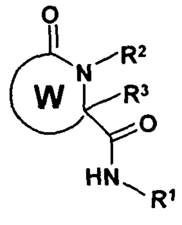

1

wherein:

**W** is a 6-oxopiperazine, [1,4]diazepan, [1,4]thiazepan, or [1,4]oxazepan cyclic compound annellated by at least one optionally substituted and optionally condensed heterocyclic compound **Q**;
**$R^1$, $R^2$** and **$R^3$** are, independently of one another, a hydrogen atom, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl; and
Q is a pyrrole, pyrazole, imidazole, thiazole, pyrrolidine, indole, benzofuran, 4,5,6,7-tetrahydro-benzothiophene, thieno[3,2-b]pyrrole, furo[3,2-b]pyrrole, thieno[2,3-b]pyrrole, benzimidazole, pyridine, quinoline, or 1,2,3,4-tetrahydro-isoquinoline cyclic compound.

**2.** Compounds of claim 1, which are substituted 1-oxo-1,2,3,4-tetrahydropyrrolo[1,2-a]pyrazine-3-carboxamides of general formula **1.1** and 3-oxo-2,3,4,6-tetrahydro-1*H*-pyrrolo[1,2-a]pyrazine-1-carboxamides of general formula **1.2,** 6-oxo-5,6-dihydro-4*H*-benzo[f]pyrrolo[1,2-a][1,4]diazepine-4-carboxamides of general formula **1.3,** 5-oxo-1,3,4,5,6,7,8,9-octahydro-2*H*-10-thia-6,9-diaza-benzo[a]-cyclopenta[e]azulene-7-carboxamides of general formula 1.4:

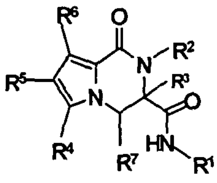

1.1

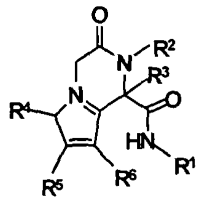

1.2

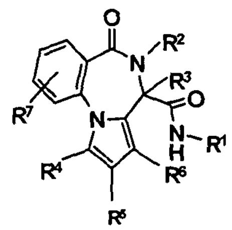

1.3

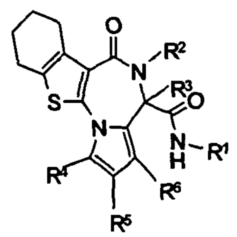

1.4

wherein:

**$R^1$, $R^2$** and **$R^3$** are as above;
**$R^4$, $R^5$** and **$R^6$** are, independently of one another, a hydrogen atom, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl; and
**$R^7$** is a hydrogen atom or an optionally substituted $C_1$-$C_6$ alkyl.

**3.** Compounds of claim 1, which are substituted 4-oxo-4,5,6,7-tetrahydropyrazolo[1,5-a]pyrazine-6-carboxamides of general formula **1.5,** 4-oxo-1,4,5,6-tetrahydro-1,2,5,9a-tetra-azacyclopenta[e]azulene-6-carboxamides of general formula 1.6 and 6-oxo-4,5,6,7-tetrahydro-1*H*-8-thia-1,2,5-triaza-azulene-4-carboxamides of general formula 1.7:

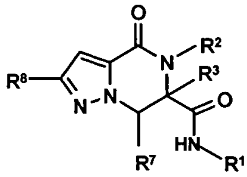

1.5

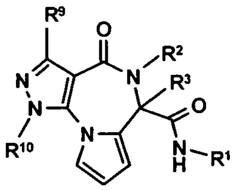

1.6

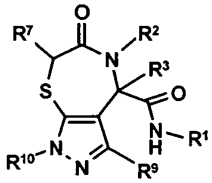

1.7

wherein:

**$R^1$, $R^2$, $R^3$,** and **$R^7$** are as above;
**$R^8$** and **$R^9$** are, independently of one another, a hydrogen atom, carboxyalkyl, carboxy, or an optionally substituted carbamyl; and
**$R^8$, $R^9$** and **$R^{10}$** are, independently of one another, an inert substituent, an optionally substituted $C_1$-$C_6$ alkyl, an optionally substituted $C_3$-$C_8$ cycloalkyl, an optionally substituted phenyl, an optionally substituted aryl, or an optionally substituted heterocyclyl.

**4.** Compounds of claim 1, which are substituted 8-oxo-5,6,7,8-tetrahydro-imidazo[1,5-a]pyrazine-6-carboxamides of general formula 1.8:

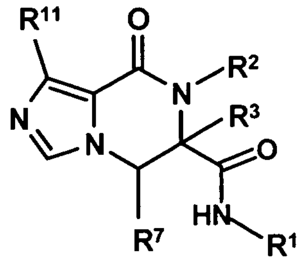

**1.8**

wherein:

**$R^1$, $R^2$, $R^3$** and **$R^7$** are as above; and
**$R^{11}$** is a hydrogen atom, carboxyalkyl, carboxy, or an optionally substituted carbamyl.

**5.** Compounds of claim 1, which are substituted 8-oxo-5,6,7,8-tetrahydro-4-oxa-1-thia-3,7-diaza-azulene-6-carboxamides of general formula 1.9:

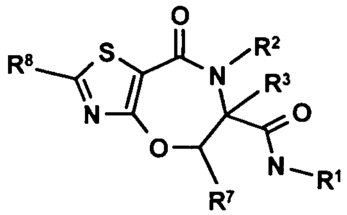

**1.9**

where: **$R^1$, $R^2$, $R^3$, $R^7$** and **$R^8$** are as above.

**6.** Compounds of claim 1, which are substituted 1-oxo-1,2,3,4-tetrahydropyrazine[1,2-a]indole-3-carboxamides of general formula **1.10,** 7-oxo-5,7,8,10-tetrahydro-6*H*-9-thia-6,10-diaza-benzo[a]azulene-5-carboxamides of general

formula **1.11,** 7-oxo-7,8,9,10-tetrahydro-6*H*-5-thia-8,10-diaza-benzo[a]azulene-9-carboxamides of general formula **1.12,** and 9-oxo-7,8,9,10-tetrahydro-6*H*-5-thia-8,10-diaza-benzo[a]azulene-7-carboxamides of general formula 1.13:

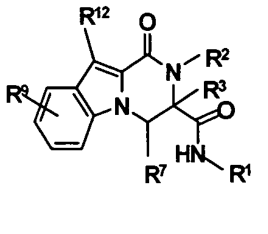

**1.10**

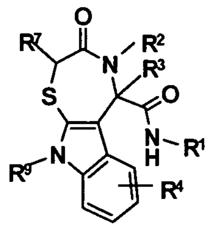

**1.11**

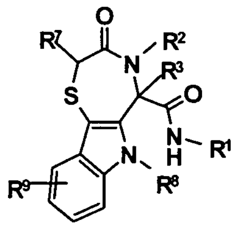

**1.12**

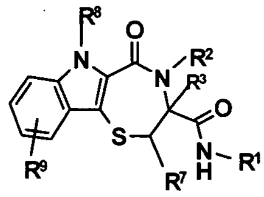

**1.13**

wherein:

**$R^1$, $R^2$, $R^3$, $R^7$, $R^8$** and **$R^9$** are as above; and
**$R^{12}$** is a hydrogen atom, an inert substituent, a substituted amino group, or pyrrol-1-yl.

**7.** Compounds of claim 1, which are substituted 9-oxo-6,7,8,9-tetrahydro-10-oxa-5-thia-8-aza-benzo[a]azulene-7-carboxamides of general formula **1.14:**

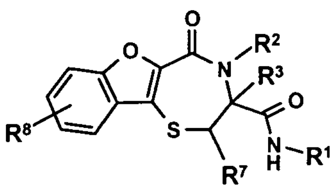

**1.14**

wherein: **$R^1$, $R^2$, $R^3$, $R^7$** and **$R^8$** are as above.

**8.** Compounds of claim 1, which are substituted 1-oxo-1,2,3,4-tetrahydrobenzo[4,5]imidazo[1,2-a]pyrazine-3-carboxamides of general formula 1.15:

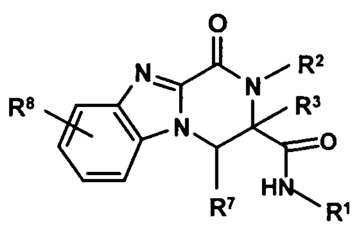

**1.15**

wherein: **$R^1$, $R^2$, $R^3$, $R^7$** and **$R^8$** are as above.

**9.** Compounds of claim 1, which are substituted 7-oxo-4,5,6,7-tetrahydro-1-thia-3b,6-diaza-cyclopenta[a]indene-5-carboxamides of general formula **1.16** and 7-oxo-4,5,6,7-tetrahydro-1-oxa-3b,6-diaza-cyclopenta[a]indene-5-carboxamides of general formula **1.17:**

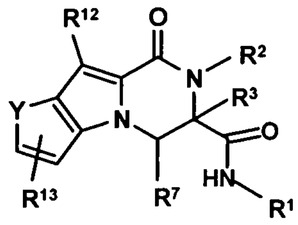

**1.16 (Y=S), 1.17 (Y=O)**

wherein:

**$R^1$, $R^2$, $R^3$, $R^7$** and **$R^{12}$** are as above; and
**$R^{13}$** is a hydrogen atom or an optionally substituted $C_1$-$C_6$ alkyl.

**10.** Compounds of claim 1, which are substituted 7-oxo-4,5,6,7-tetrahydro-3-thia-3b,6-diaza-cyclopenta[a]indene-5-carboxamides of general formula **1.18:**

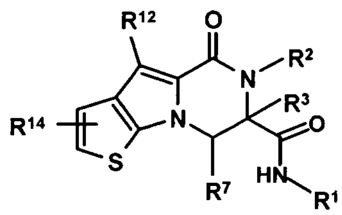

**1.18**

wherein:

**$R^1$, $R^2$, $R^3$**, **$R^7$**and **$R^{12}$** as above; and
**$R^{14}$** is a hydrogen atom or an optionally substituted $C_1$-$C_6$ alkyl.

**11.** Compounds of claim 1, which are substituted 7-oxo-5,6,7,8-tetrahydro-9-thia-1,6-diaza-benzocycloheptane-5-carboxamides of general formula **1.19** and 8-oxo-7,8,9,10-tetrahydro-6-thia-5,9-diaza-cyclopenta[b]naphthalene-10-carboxamides of general formula 1.20:

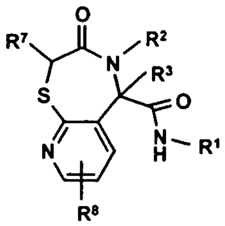

**1.19**

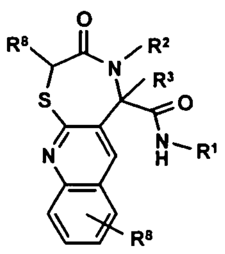

**1.20**

wherein: **$R^1$, $R^2$, $R^3$, $R^7$** and **$R^8$** are as above.

**12.** Compounds of claim 1, which are substituted 4-oxo-2,3,3a,4,5,6-hexahydro-1*H*-benzo[f]pyrrolo[1,2-a][1,4]diazepin-6-carboxamides of general formula **1.21,** (3a*S*)-4-oxo-2,3,3a,4,5,6-hexahydro-1*H*-benzo[f]pyrrolo[1,2-a][1,4]diazepin-6-carboxamides of general formula **1.22,** (3a*S*,6*S*)-4-oxo-2,3,3a,4,5,6-hexahydro-1*H*-benzo[f]pyrrolo[1,2-a][1,4]diazepin-6- carboxamides of general formula **1.22a,** and (3a*S*,6*R*)-4-oxo-2,3,3a,4,5,6-hexahydro-1*H*-benzo[f]pyrrolo[1,2-a][1,4]diazepin-6-carboxamides of general formula **1.22b:**

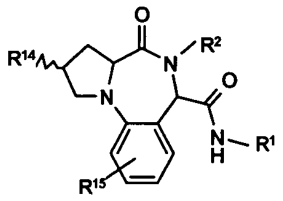

1.21

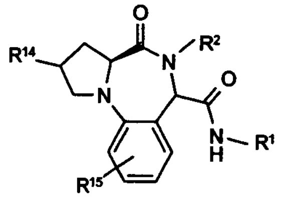

1.22

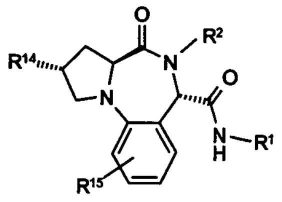

1.22a

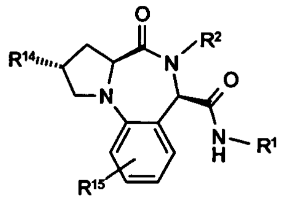

1.22b

wherein:

**$R^1$** and **$R^2$** are as above;
**$R^{14}$** is a hydrogen atom or an optionally substituted hydroxyl group; and
**$R^{15}$** is a hydrogen atom, $NO_2$, $CF_3$, CN, carboxyalkyl, carboxy, an optionally substituted carbamyl, or an optionally substituted amino group.

**13.** Compounds of claim 1, which are substituted 1-oxo-1,3,4,6,11,11a-hexahydro-2*H*-pyrazino[1,2-b]isoquinoline-3-carboxamides of general formula **1.23:**

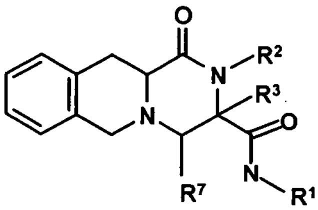

**1.23**

wherein: **$R^1$, $R^2$, $R^3$** and **$R^7$** are as above.

**14.** A method of producing annellated carbamyl-aza-heterocyclic compounds of general formula 1 by three-component condensation of a suitable isonitrile of general formula 2, a suitable primary amine of general formula 3, and a suitable heterocyclic bifunctional reagent of general formula 4:

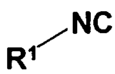

**2**

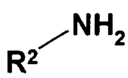

**3**

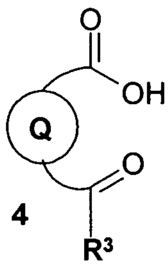

**4**

wherein: **Q, $R^1$, $R^2$** and **$R^3$** are as above.

**15.** A method of producing compounds of claim 14, wherein bifunctional reagents 4 are heterocyclic compounds containing a carboxyl and a carbonyl groups of general formula **4.1-4.23:**

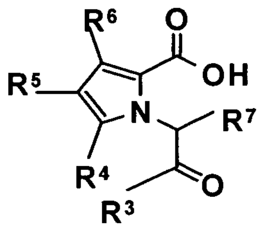

4.1

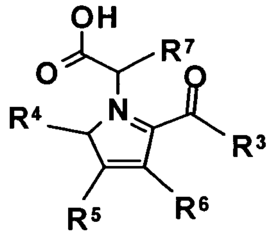

4.2

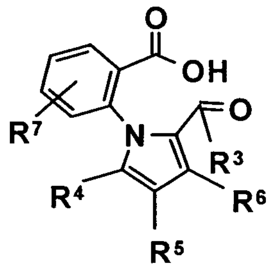

4.3

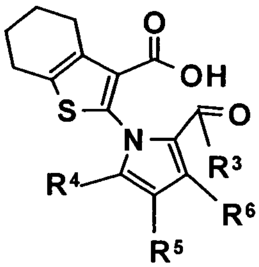

4.4

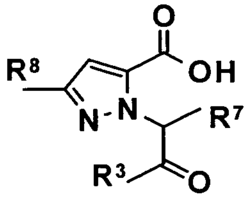

4.5

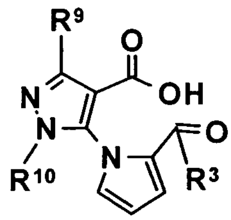

4.6

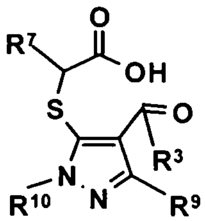

4.7

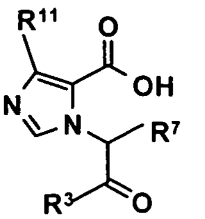

4.8

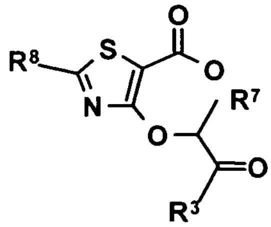

4.9

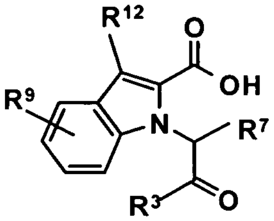

4.10

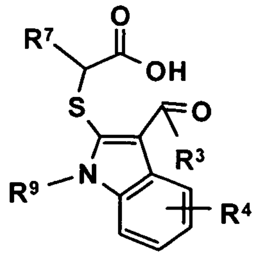

4.11

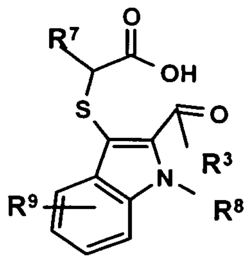

4.12

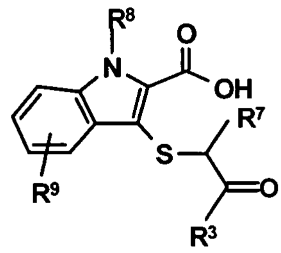
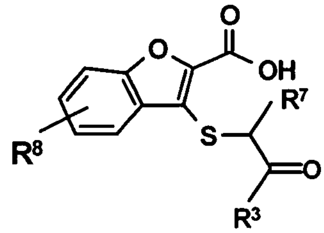
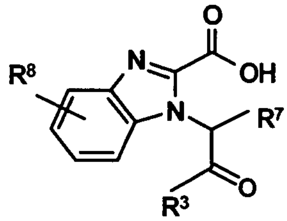

4.13 4.14 4.15

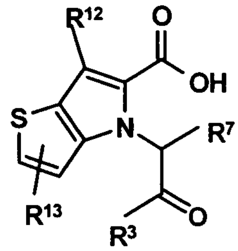
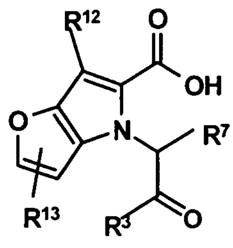
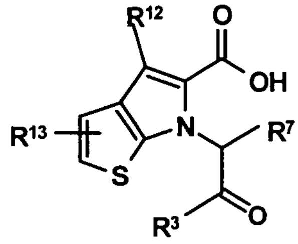

4.16 4.17 4.18

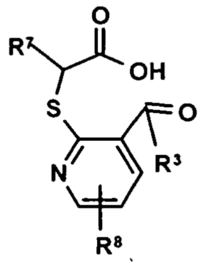
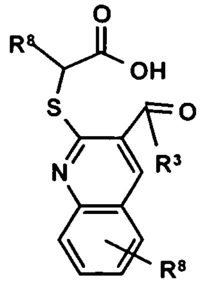
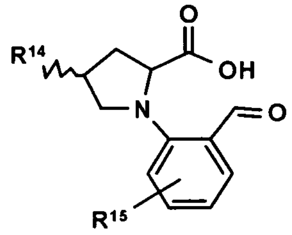

4.19 4.20 4.21

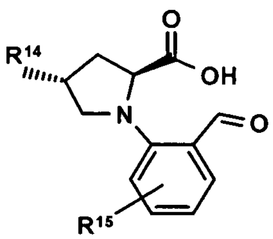
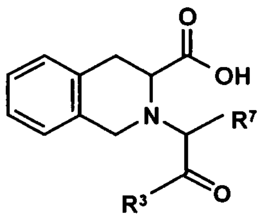

4.22 4.23

wherein: **R^3** to **R^15** are as in claims 2 to 12.

**15.** A focused library for determining leader compounds, comprising at least one compound of general formula 1

according to claim 1.

**16.** A pharmaceutical composition exhibiting anticancer activity, comprising at least one annellated carbamyl-aza-heterocyclic compound of general formula 1 according to claim 1 or a pharmaceutically acceptable salt thereof.

**INTERNATIONAL SEARCH REPORT**

International application No. PCT/RU 2005/000235

| | |
|---|---|
| A. CLASSIFICATION OF SUBJECT MATTER | C07D 487/04, 487/14, 498/04, 498/14, 513/14, 513/04, C40B 40/04, A61K 31/4985, 31/5517, 31/5513, 31/553, 31/554, A61P 35/00 |

According to International Patent Classification (IPC) or to both national classification and IPC

B. FIELDS SEARCHED

Minimum documentation searched (classification system followed by classification symbols)

C07D 487/04, 487/14, 498/04, 498/14, 513/14, 513/04, 519/06, C40B 40/04, C07B 61/00, 61/02, A61K 31/4985, 31/5517, 31/5513, 31/553, 31/554, A61P 35/00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P, X | ILYN ALEXEY P. et al. "New four-component Ugi-type reaction. Synthesis of heterocyclyc structures containing a pyrrolo [1,2-a][1,4]diazepine fragment" Journal of Organic Chemistry, 18.02.2005, 70(4), 1478-1481 | 1-3, 14-15 |
| A | SU 725565 A (SANDOS AG) 30.03.1980 | 1-4, 8, 15 |
| A | SU 936815 A (SIONOGI AND CO. LTD.) 15.06.1982 | 1, 3, 5-7, 11, 14, 17 |
| A | EA 003061 B1 (AVENTIS FARMACEUTICALS INC.) 26.12.2002 | 1, 13-15 |
| A | GB 1441554 A (MERCK PATENT GESELLSCHAFT MIT BESCHRANKTER HAFTUNG) 07.07.1976, the claims | 1-3, 12, 14-15 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| (22.08.2005) | (15.09.2005) |
| Name and mailing address of the ISA/ RU | Authorized officer |
| Facsimile No. | Telephone No. |

Form PCT/ISA/210 (second sheet) (July 1992)

**INTERNATIONAL SEARCH REPORT**

International application No.
PCT/RU 2005/000235

C (Continuation). DOCUMENTS CONSIDERED TO BE RELEVANT

| Category* | Citation of document, with indication, where appropriate, of the relevant passages | Relevant to claim No. |
|---|---|---|
| A | TEMPEST PAUL et al. "MMC/SNAr methodology. Part 2: Novel three-step solution phase access to libraries of benzodiazepines", Tetrahedron Letters (English) 2003, 44(9), 1947-1950 [on-line] Found on: ACS on STN, no. 139:36509 | 1-3, 12, 14-16 |
| A | NEFZI ADEL et al. "Solid phase synthesis of 1,4-benzothiazepin-5-one derivatives". Tetrahedron Letters (English) 1999, 40(27), 4939-4942 | 1, 3, 6-7, 11, 14-15 |
| A | ZHANG JUNDONG et al. "Unique Structures Generated by Ugi 3CC Reactions Using Bifynctional Starting Materials Containing Aldehyde and Carboxylic Acid" Journal of Organic Chemistry (English) 1999, 64(3), 1074-1076 | 1, 5, 14 |
| A | SAYER WOLFGANG H.B. et al. "Molecular Shape Diversity of Combinatorial Libraries: A Prerequisite for Broad Bioactivity". Journal of Chemical Information and Computer Sciences (English) 2003, 43(3), 987-1003. [on-line] Found on: ACS on STN, no. 138:362606 | 1, 6-7, 14-16 |

Form PCT/ISA/210 (continuation of second sheet) (July 1992)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8705022 A
- WO 9748706 A


### Non-patent literature cited in the description


- **CAFIERI, F. et al.** *J. Nat. Prod.,* 1998, vol. 61, 122
- **UMEYAMA, A. et al.** *J. Nat. Prod.,* 1998, vol. 61, 1433
- **POULLENNEC, K. ; ROMO, D.** *J. Am. Chem. Soc.,* 2003, vol. 125, 6344
- **ASKEW, B.C. et al.** *J. Med. Chem.,* 1997, vol. 40 (12), 1779
- **KWON O.** *J. Antibiot.,* 2001, vol. 54 (2), 179
- **IKEMOTO, T. et al.** *Tetrahedron,* 2000, vol. 56 (40), 7915
- **TAPIA, R. et al.** *Eur. J. Org. Chem.,* 2002, vol. 23, 4005
- **LOPEZ-RODRIGUEZ, M. et al.** *J. Med. Chem.,* 2001, vol. 44 (2), 186
- **GREENE, T.W. ; WUTS, P.G.M.** Protective Groups in Organic Synthesis. John Wiley & Sons, Inc, 1999, 494-653
- **JOHN O'BRIEN ; IAN WILSON ; TERRY ORTON ; FRANÇOIS POGNAN.** Investigation of the Alamar Blue (resazurin) Fluorescent Dye for the Assessment of Mammalian Cell Cytotoxicity. *Eur. J. Biochem.,* 2000, vol. 267, 5421-5426